(19) Europäisches Patentamt
European Patent Office
Office européen des brevets

(11) EP 1 704 248 B1

(12) EUROPEAN PATENT SPECIFICATION

(45) Date of publication and mention of the grant of the patent:
**05.05.2010 Bulletin 2010/18**

(21) Application number: **04803064.7**

(22) Date of filing: **23.12.2004**

(51) Int Cl.:
***C12Q 1/68*** *(2006.01)*

(86) International application number:
**PCT/DK2004/000914**

(87) International publication number:
**WO 2005/064009 (14.07.2005 Gazette 2005/28)**

(54) **CLASSIFICATION OF COLON CANCER**
KLASSIFIZIERUNG VON KOLONKREBS
CLASSIFICATION DU CANCER DU COLON

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **27.12.2003 DK 200301940**
**24.01.2004 DK 200400096**
**07.04.2004 DK 200400586**
**26.11.2004 DK 200401843**

(43) Date of publication of application:
**27.09.2006 Bulletin 2006/39**

(73) Proprietor: **AROS APPLIED BIOTECHNOLOGY A/S**
**8200 Aarhus N. (DK)**

(72) Inventors:
- **ØRNTOFT, Torben, Falck**
**8230 Aabyhøj (DK)**
- **JENSEN, Jens, Ledet**
**8240 Risskov (DK)**
- **KRUHØFFER, Mogens**
**8240 Risskov (DK)**
- **LAIHO, Päivi**
**00500 Helsinki (FI)**
- **AALTONEN, Lauri, A.**
**02170 Espoo (FI)**

(74) Representative: **Høiberg A/S**
**European Patent & Trademark Attorneys**
**Store Kongensgade 59 A**
**1264 Copenhagen K (DK)**

(56) References cited:
**EP-A- 1 361 286**

- **MORI YURIKO ET AL: "The impact of microsatellite instability on the molecular phenotype of colorectal tumors." CANCER RESEARCH. 1 AUG 2003, vol. 63, no. 15, 1 August 2003 (2003-08-01), pages 4577-4582, XP002321908 ISSN: 0008-5472 cited in the application**
- **BOLAND C R ET AL: "A National Cancer Institute Workshop on microsatellite instability for cancer detection and familial predisposition: development of international criteria for the determination of microsatellite instability in colorectal cancer" CANCER RESEARCH, AMERICAN ASSOCIATION FOR CANCER RESEARCH, BALTIMORE, MD, US, vol. 58, no. 22, 15 November 1998 (1998-11-15), pages 5248-5257, XP001080197 ISSN: 0008-5472 cited in the application**
- **ALON U ET AL: "Broad patterns of gene expression revealed by clustering analysis of tumor and normal colon tissues probed by oligonucleotide arrays" PROCEEDINGS OF THE NATIONAL ACADEMY OF SCIENCES OF USA, NATIONAL ACADEMY OF SCIENCE. WASHINGTON, US, vol. 96, 1999, pages 6745-6750, XP000900484 ISSN: 0027-8424**
- **PELTOMAKI PAIVI: "Deficient DNA mismatch repair: A common etiologic factor for colon cancer" HUMAN MOLECULAR GENETICS, OXFORD UNIVERSITY PRESS, SURREY, GB, vol. 10, no. 7, 2001, pages 735-740, XP002204642 ISSN: 0964-6906**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 704 248 B1

- WOERNER S M ET AL: "Systematic identification of genes with coding microsatellites mutated ni DNA mismatch repair-deficient cancer cells" INTERNATIONAL JOURNAL OF CANCER, NEW YORK, NY, US, vol. 93, 6 April 2001 (2001-04-06), pages 12-19, XP001061477 ISSN: 0020-7136
- LINNEBACHER M ET AL: "Frameshilft peptide-derived T-cell epitopes: a source of novel tumor-specific antigens" INTERNATIONAL JOURNAL OF CANCER, NEW YORK, NY, US, vol. 93, 6 April 2001 (2001-04-06), pages 6-11, XP001061478 ISSN: 0020-7136
- THE MULTIPLE LEIOMYOMA CONSORTIUM: "Germline mutatiosn in FH predispose to dominantly inherited uterine fibroids, skin leiomyomata and papillary renal cell cancer" NATURE GENETICS, vol. 30, 25 February 2002 (2002-02-25), pages 406-410,
- OSTERGAARD ET AL: "Psoriasin (S100A7): a putative urinary marker for the follow-up of patients with bladder squamous cell carcinomas" ELECTROPHORESIS, vol. 20, 1999, pages 349-354,
- GAZZOLI ET AL: "A hereditary nonpolyposis colorectal carcinoma case associated with hypermethylation of the MLH1 gene in normal tissue and loss of heterozygosity of the unmethylated allele in the resulting microsatellite instability-high tumor" CANCER RESEARCH, vol. 62, 15 July 2002 (2002-07-15), pages 3925-3928, XP002437844
- MENIGATTI ET AL: "Methylation pattern of different regions of the MLH1 promoter and silencing of gene expression in hereditary and sporadic colorectal cancer." GENES, CHROMOSOMES AND CANCER, vol. 31, 2001, pages 357-361,

SEQUENCE LISTING

<110> Aros Applied Biotechnology Aps

<120> Classification of Colon Cancer

<130> P949PC00

<160> 139

<170> PatentIn version 3.1

<210> 1
<211> 1237
<212> DNA
<213> NM_002985.2| Homo sapiens chemokine (C-C motif) ligand 5 (CCL5), mRNA

<400> 1

gctgcagagg attcctgcag aggatcaaga cagcacgtgg acctcgcaca gcctctccca 60
caggtaccat gaaggtctcc gcggcagccc tcgctgtcat cctcattgct actgccctct 120
gcgctcctgc atctgcctcc ccatattcct cggacaccac accctgctgc tttgcctaca 180
ttgcccgccc actgccccgt gcccacatca aggagtattt ctacaccagt ggcaagtgct 240
ccaacccagc agtcgtcttt gtcacccgaa agaaccgcca agtgtgtgcc aacccagaga 300
agaaatgggt tcgggagtac atcaactctt tggagatgag ctaggatgga gagtccttga 360
acctgaactt acacaaattt gcctgtttct gcttgctctt gtcctagctt gggaggcttc 420
ccctcactat cctaccccac ccgctccttg aagggcccag attctaccac acagcagcag 480
ttacaaaaac cttccccagg ctggacgtgg tggctcacgc ctgtaatccc agcactttgg 540
gaggccaagg tgggtggatc acttgaggtc aggagttcga gaccagcctg gccaacatga 600
tgaaacccca tctctactaa aaatacaaaa aattagccgg gcgtggtagc gggcgcctgt 660
agtcccagct actcgggagg ctgaggcagg agaatggcgt gaacccggga ggcggagctt 720
gcagtgagcc gagatcgcgc cactgcactc cagcctgggc gacagagcga gactccgtct 780
caaaaaaaaa aaaaaaaaaa aaaatacaaa aattagccgg gcgtggtggc ccacgcctgt 840
aatcccagct actcgggagg ctaaggcagg aaaattgttt gaacccagga ggtggaggct 900
gcagtgagct gagattgtgc cacttcactc cagcctgggt gacaaagtga gactccgtca 960

caacaacaac aacaaaaagc ttccccaact aaagcctaga agagcttctg aggcgctgct 1020
ttgtcaaaag gaagtctcta ggttctgagc tctggctttg ccttggcttt gccagggctc 1080
tgtgaccagg aaggaagtca gcatgcctct agaggcaagg aggggaggaa cactgcactc 1140
ttaagcttcc gccgtctcaa cccctcacag gagcttactg gcaaacatga aaaatcggct 1200
taccattaaa gttctcaatg caaccataaa aaaaaaa 1237

<210> 2
<211> 2884
<212> DNA
<213> NM_004184.3| Homo sapiens tryptophanyl-tRNA synthetase (WARS), transcript variant 1, mRNA

<400> 2

tcgattctca agagggtttc attggtctca acctggcccc ccaggcaacc cacccctgat 60
tggacagtct catcaagaag gttggtcaag agctcaagtg tttctgagaa tctgggtgat 120
ttataagaaa cccttagctg aatgcagggt ggggagaacg aaagacaaaa gcatcttttt 180
tcagaaggga aactgaaaga aagaggggaa gagtattaaa gaccatttct ggctgggcag 240
ggcactctca gcagctcaac tgcccagcgt gaccagtggc cacctctgca gtgtcttcca 300
caacctggtc ttgactcgtc tgctgaacaa atcctctgac ctcaggccgg ctgtgaacgt 360
agttcctgag agatagcaaa catgcccaac agtgagcccg catctctgct ggagctgttc 420
aacagcatcg ccacacaagg ggagctcgta aggtccctca aagcgggaaa tgcgtcaaag 480
gatgaaattg attctgcagt aaagatgttg gtgtcattaa aaatgagcta caaagctgcc 540
gcgggggagg attacaaggc tgactgtcct ccagggaacc cagcacctac cagtaatcat 600
ggcccagatg ccacagaagc tgaagaggat tttgtggacc catggacagt acagacaagc 660
agtgcaaaag gcatagacta cgataagctc attgttcggt ttggaagtag taaaattgac 720
aaagagctaa taaaccgaat agagagagcc accggccaaa gaccacacca cttcctgcgc 780
agaggcatct tcttctcaca cagagatatg aatcaggttc ttgatgccta tgaaaataag 840
aagccatttt atctgtacac gggccggggc ccctcttctg aagcaatgca tgtaggtcac 900
ctcattccat ttattttcac aaagtggctc caggatgtat ttaacgtgcc cttggtcatc 960
cagatgacgg atgacgagaa gtatctgtgg aaggacctga ccctggacca ggcctatagc 1020
tatgctgtgg agaatgccaa ggacatcatc gcctgtggct ttgacatcaa caagactttc 1080
atattctctg acctggacta catggggatg agctcaggtt tctacaaaaa tgtggtgaag 1140
attcaaaagc atgttacctt caaccaagtg aaaggcattt tcggcttcac tgacagcgac 1200
tgcattggga agatcagttt tcctgccatc caggctgctc cctccttcag caactcattc 1260
ccacagatct tccgagacag gacggatatc cagtgcctta tcccatgtgc cattgaccag 1320

gatccttact ttagaatgac aagggacgtc gcccccagga tcggctatcc taaaccagcc 1380
ctgctgcact ccaccttctt cccagccctg cagggcgccc agaccaaaat gagtgccagc 1440
gaccccaact cctccatctt cctcaccgac acggccaagc agatcaaaac caaggtcaat 1500
aagcatgcgt tttctggagg gagagacacc atcgaggagc acaggcagtt tgggggcaac 1560
tgtgatgtgg acgtgtcttt catgtacctg accttcttcc tcgaggacga cgacaagctc 1620
gagcagatca ggaaggatta caccagcgga gccatgctca ccggtgagct caagaaggca 1680
ctcatagagg ttctgcagcc cttgatcgca gagcaccagg cccggcgcaa ggaggtcacg 1740
gatgagatag tgaaagagtt catgactccc cggaagctgt ccttcgactt tcagtagcac 1800
tcgttttaca tatgcttata aaagaagtga tgtatcagta atgtatcaat aatcccagcc 1860
cagtcaaagc accgccacct gtaggcttct gtctcatggt aattactggg cctggcctct 1920
gtaagcctgt gtatgttatc aatactgttt cttcctgtga gttccattat ttctatctct 1980
tatgggcaaa gcattgtggg taattggtgc tggctaacat tgcatggtcg gatagagaag 2040
tccagctgtg agtctctccc caaagcagcc ccacagtgga gcctttggct ggaagtccat 2100
gggccacccct gttcttgtcc atggaggact ccgagggttc caagtatact cttaagaccc 2160
actctgttta aaaatatata ttctatgtat gcgtatatgg aattgaaatg tcattattgt 2220
aacctagaaa gtgctttgaa atattgatgt ggggaggttt attgagcaca agatgtattt 2280
cagcccatgc cccctcccaa aaagaaattg ataagtaaaa gcttcgttat acatttgact 2340
aagaaatcac ccagctttaa agctgctttt aacaatgaag attgaacaga gttcagcaat 2400
tttgattaaa ttaagacttg ggggtgaaac tttccagttt actgaactcc agaccatgca 2460
tgtagtccac tccagaaatc atgctcgctt cccttggcac accagtgttc tcctgccaaa 2520
tgaccctaga ccctctgtcc tgcagagtca gggtggcttt tcccctgact gtgtccgatg 2580
ccaaggagtc ctggcctccg cagatgcttc attttgaccc ttggctgcag tggaagtcag 2640
cacagagcag tgccctggct gtgtccctgg acgggtggac ttagctaggg agaaagtcga 2700
ggcagcagcc ctcgaggccc tcacagatgt ctaggcaggc ctcatttcat cacgcagcat 2760
gtgcaggcct ggaagagcaa agccaaatct cagggaagtc cttggttgat gtatctgggt 2820
ctcctctgga gcactctgcc ctcctgtcac ccagtagagt aaataaactt ccttggctcc 2880
tgct 2884

<210> 3
<211> 1012
<212> DNA
<213> NM_006263.2| Homo sapiens proteasome (prosome, macropain) activator subunit 1 (PA28 alpha) (PSME1), transcript variant 1, mRNA

<400> 3

```
aggcggagct gggtgcgagc gccctaccgc tttcgctttc ccttcgcggt gcccactcca      60
ctccttgtgc ggcgctaggc cccccgtccc ggtcatggcc atgctcaggg tccagcccga     120
ggcccaagcc aaggtggatg tgtttcgtga agacctctgt accaagacag agaacctgct     180
cgggagctat ttccccaaga agatttctga gctggatgca tttttaaagg agccagctct     240
caatgaagcc aacttgagca atctgaaggc cccattggac atcccagtgc ctgatccagt     300
caaggagaaa gagaaagagg agcggaagaa acagcaggag aaggaagaca aggatgaaaa     360
gaagaagggg gaggatgaag acaaaggtcc tccctgtggc ccagtgaact gcaatgaaaa     420
gatcgtggtc cttctgcagc gcttgaagcc tgagatcaag gatgtcattg agcagctcaa     480
cctggtcacc acctggttgc agctgcagat acctcggatt gaggatggta acaattttgg     540
agtggctgtc caggagaagg tgtttgagct gatgaccagc ctccacacca agctagaagg     600
cttccacact caaatctcta agtatttctc tgagcgtggt gatgcagtga ctaaagcagc     660
caagcagccc catgtgggtg attatcggca gctggtgcac gagctggatg aggcagagta     720
ccgggacatc cggctgatgg tcatggagat ccgcaatgct tatgctgtgt tatatgacat     780
catcctgaag aacttcgaga agctcaagaa gcccagggga gaaacaaagg gaatgatcta     840
ttgagagccc tctctcccat tctgtgatga gtacagcaga gaccttcctg ctttttactg     900
gggactccag attttcccca aacttgcttc tgttgagatt tttccctcac cttgcctctc     960
aggcacaata aatatagtta taccactgcc catcaaaaaa aaaaaaaaaa aa            1012
```

<210> 4
<211> 983
<212> DNA
<213> NM_004335.2| Homo sapiens bone marrow stromal cell antigen 2 (BST2), mRNA

<400> 4

```
gtggaattca tggcatctac ttcgtatgac tattgcagag tgcccatgga agacggggat      60
aagcgctgta agcttctgct ggggatagga attctggtgc tcctgatcat cgtgattctg     120
ggggtgccct tgattatctt caccatcaag gccaacagcg aggcctgccg ggacggcctt     180
cgggcagtga tggagtgtcg caatgtcacc catctcctgc aacaagagct gaccgaggcc     240
cagaagggct ttcaggatgt ggaggcccag gccgccacct gcaaccacac tgtgatggcc     300
ctaatggctt ccctggatgc agagaaggcc caaggacaaa agaaagtgga ggagcttgag     360
ggagagatca ctacattaaa ccataagctt caggacgcgt ctgcagaggt ggagcgactg     420
agaagagaaa accaggtctt aagcgtgaga atcgcggaca agaagtacta ccccagctcc     480
caggactcca gctccgctgc ggcgccccag ctgctgattg tgctgctggg cctcagcgct     540
ctgctgcagt gagatcccag gaagctggca catcttggaa ggtccgtcct gctcggcttt     600
```

tcgcttgaac attcccttga tctcatcagt tctgagcggg tcatggggca acacggttag 660
cggggagagc acggggtagc cggagaaggg cctctggagc aggtctggag gggccatggg 720
gcagtcctgg gtgtggggac acagtcgggt tgacccaggg ctgtctccct ccagagcctc 780
cctccggaca atgagtcccc cctcttgtct cccaccctga gattgggcat ggggtgcggt 840
gtgggggca tgtgctgcct gttgttatgg gttttttttg cgggggggt tgcttttttc 900
tggggtcttt gagctccaaa aaataaacac ttcctttgag ggagagcaaa aaaaaaaaa 960
aaaaaaaaa aaaaaaaaa aaa 983

<210> 5
<211> 1260
<212> DNA
<213> NM_004223.3| Homo sapiens ubiquitin-conjugating enzyme E2L 6 (UBE2L6), transcript variant 1, mRNA

<400> 5

gggggtgggg tccccggggc ggggcggggc gcgctgtgtc gcgggtcgga gctcggtcct 60
gctggaggcc acgggtgcca cacactcggt cccgacatga tggcgagcat gcgagtggtg 120
aaggagctgg aggatcttca gaagaagcct cccccatacc tgcggaacct gtccagcgat 180
gatgccaatg tcctggtgtg gcacgctctc ctcctacccg accaacctcc ctaccacctg 240
aaagccttca acctgcgcat cagcttcccg ccggagtatc cgttcaagcc tcccatgatc 300
aaattcacaa ccaagatcta ccaccccaac gtggacgaga acggacagat ttgcctgccc 360
atcatcagca gtgagaactg gaagccttgc accaagactt gccaagtcct ggaggccctc 420
aatgtgctgg tgaatagacc gaatatcagg gagcccctgc ggatggacct cgctgacctg 480
ctgacacaga atccggagct gttcagaaag aatgccgaag agttcaccct ccgattcgga 540
gtggaccggc cctcctaact catgttctga ccctctgtgc actggatcct cggcatagcg 600
gacggacaca cctcatggac tgaggccaga gccccctgtg gcccattccc cattcatttt 660
tcccttctta ggttgttagt cattagtttg tgtgtgtgtg tggtggaggg aagggagcta 720
tgagtgtgtg tgttgtgtat ggactcactc ccaggttcac ctggccacag gtgcaccctt 780
cccacaccct ttacattccc cagagccaag ggagtttaag tttgcagtta caggccagtt 840
ctccagctct ccatcttaga gagacaggtc accttgcagg cctgcttgca ggaaatgaat 900
ccagcagcca actcgaatcc ccctagggct caggcactga gggcctgggg acagtggagc 960
atatgggtgg gagacagatg gagggtaccc tatttacaac tgagtcagcc aagccactga 1020
tgggaatata cagatttagg tgctaaaccg tttattttcc acggatgagt cacaatctga 1080
agaatcaaac ttccatcctg aaaatctata tgtttcaaaa ccacttgcca tcctgttaga 1140
ttgccagttc ctgggaccag gcctcagact gtgaagtata tatcctccag cattcagtcc 1200

agggggagcc acggaaacca tgttcttgct taagccatta aagtcagaga tgaattctgg 1260

<210> 6
<211> 3799
<212> DNA
<213> NM_003488.2| Homo sapiens A kinase (PRKA) anchor protein 1 (AKAP1), nuclear gene encoding mitochondrial protein, transcript variant 1, mRNA

<400> 6

ctgtgttcca cccgcctggg ctagcacgtg ggggagctgc ggaagcgcgg cgctgcgggc 60
cgggccgcgg ggcacagccg ggggccggcg gcggcgcgcg gactccgcat cccgcacccc 120
gatggtagcc gaggagctgg tgtaattact tcaagcctcc aggatggcaa tccagttccg 180
ttcgctcttc cccttggcat tgcctgggat gctggcgctc ctcggctggt ggtggttttt 240
ctctcgtaaa aaaggccatg tcagcagcca tgatgagcag caggtggagg ctggtgctgt 300
gcagctgagg gctgaccctg ccatcaagga acctctcccc gtggaagacg tctgtcccaa 360
agtagtgtcc acacccccca gtgtcacaga gcctccagaa aaggaactgt ccaccgtgag 420
caagctgcct gcagagcccc cagcattgct ccagacacac ccaccttgcc gaagatcaga 480
gtcctcgggc attcttccta acaccacaga catgagattg cgaccaggaa cacgcagaga 540
tgacagtaca aagctggagc tagccctgac aggtggtgaa gccaaatcga ttcctctaga 600
gtgcccccctt tcatccccaa agggtgtact attctccagc aaatcagctg aggtgtgtaa 660
gcaagattcc cccttcagca gggtgccaag gaaggtccag ccaggctacc ccgtagtccc 720
cgcagagaag cgtagctctg ggagagggc aagagagaca ggtggggccg aagggactgg 780
tgatgccgtg ttgggggaaa aggtgcttga agaagctctg ttgtctcggg agcatgtctt 840
ggaattggag aacagcaagg gccccagcct ggcctcttta gagggggaag aagataaggg 900
gaagagcagc tcatcccagg tggtggggcc agtgcaggag gaagagtatg tagcagagaa 960
gttgccaagt aggttcatcg agtcggctca cacagagctg gcaaaggacg atgcggcgcc 1020
agcacccccca gtcgcagacg ccaaagccca ggatagaggt gtcgagggag aactgggcaa 1080
tgaggagagc ttggatagaa atgaggaggg cttggataga aatgaggagg gcttggatag 1140
aaatgaggag agcttggata gaaatgagga gggcttggat agaaatgagg agattaagcg 1200
ggctgccttc cagataatct cccaagtgat ctcagaagca accgaacagg tgctggccac 1260
cacggttggc aaggttgcag gtcgtgtgtg tcaggccagt cagctccaag ggcagaagga 1320
agagagctgt gtcccagttc accagaaaac tgtcttgggc ccagacactg cggagcctgc 1380
cacagcagag gcagctgttg ccccgccgga tgctggcctc cccttgccag gcctaccagc 1440
agagggctca ccaccaccaa agacctacgt gagctgcctg aagagccttc tgtccagccc 1500
caccaaggac agtaagccaa atatctctgc acaccacatc tccctggcct cctgcctggc 1560

```
actgaccacc cccagtgaag agttgccgga ccgggcaggc atcctggtgg aagatgccac    1620
ctgtgtcacc tgcatgtcag acagcagcca aagtgtccct ttggtggctt ctccaggaca    1680
ctgctcagat tctttcagca cttcagggct tgaagactct tgcacagaga ccagctcgag    1740
ccccagggac aaggccatca ccccgccact gccagaaagt actgtgccct tcagcaatgg    1800
ggtgctgaag ggggagttgt cagacttggg ggctgaggat ggatggacca tggatgcgga    1860
agcagatcat tcaggaggtt ctgacaggaa cagcatggat tccgtggata gctgttgcag    1920
tctcaagaag actgagagct tccaaaatgc ccaggcaggc tccaacccta agaaggtcga    1980
cctcatcatc tgggagatcg aggtgccaaa gcacttagtc ggtcggctaa ttggcaagca    2040
ggggcgctat gtgagttttc tgaagcaaac atctggtgcc aagatctaca tttcaaccct    2100
gccttacacc cagagcgtcc agatctgcca catagaaggc tctcaacatc atgtagacaa    2160
agcgctgaac ttgattggga agaagttcaa agagctgaac ctcaccaata tctacgctcc    2220
cccattgcct tcactggcac tgccttctct gccgatgaca tcctggctca tgctgcctga    2280
tggcatcacc gtggaggtca ttgtggtcaa ccaggtcaat gccgggcacc tgttcgtgca    2340
gcagcacaca caccctacct tccacgcgct gcgcagcctc gaccagcaga tgtacctctg    2400
ttactctcag cctggaatcc ccaccttgcc caccccagtg gaaataacgg tcatctgtgc    2460
cgcccctggt gcggacgggg cctggtggcg agcccaagtg gttgcctcct acgaggagac    2520
caacgaagtg gagattcgat acgtggacta cggcggatat aagagggtga aagtagacgt    2580
gctccggcaa atcaggtctg actttgtcac cctgccgttt cagggagcag aagtccttct    2640
ggacagtgtg atgcccctgt cagacgatga ccagttttca ccggaagcag atgccgccat    2700
gagcgagatg acggggaata cagcactgct tgctcaggtg acaagttaca gtccaactgg    2760
tcttcctctg attcagctgt ggagtgtggt tggagatgaa gtggtgttga taaaccggtc    2820
cctggtggag cgaggccttg cccagtgggt agacagctac tacacaagcc tttgaccccc    2880
atgctgcttc ctgagagtct ttttttgcac tgttgaaatt gggcttggca ctcaagtcaa    2940
agatgaacat cggaataaca aacattgtcc tctccagaaa gtcctttctt tatccatact    3000
gtagtcctat tgagaagaca tttcgtctct gagaaaaaag gatggaacta tgggttctct    3060
tcgcaaagcc aaaggatagt gtttaacaag ccagctggct tatcctggtt ctcagctgtt    3120
taaaaaaaaa aaaaaaaagg aatagaaaca gtttcaacca gattgtccta ttccccctgt    3180
tccattcccc tcttcttcct tctatctcct tccccggcaa aaaccaaaca aactggcaga    3240
caggccaggg atgtatgttg cttgcttgag agggtttctt ttacttcaaa atctttcttc    3300
agggagcaag acatgaactg actaattggt atccactact tgtacagctt acataaatga    3360
gttgatgata tttaaccagt ttttataaac ttcatttagg tctctaaaca cagacttttt    3420
aaattgcaac tgtaaatatg aaatggtcat cacatctgac cttggtcagt ggggaggggga    3480
actggtatcc tgccaagcct ggttgtaatt tgtaaccatt ttctatttgt gcaaactctg    3540
taaatatgtg tttaaacaaa tgtaatattt tgtacaagat acactggaga acaaagggaa    3600
```

ctcaagattc ttccagccac atgtcacctg taggtagaag taaactctgc agtgcagctt 3660
ctgctcttgg cccctctggc cagggcccct gtggcttcct gcacactgga caggtgactg 3720
tatggtagag actgtgatct gggaactttt tgctgtacaa atctgtttaa aaaaaaaaaa 3780
aagtaactca ttgaattaa 3799

<210> 7
<211> 829
<212> DNA
<213> NM_002818.2| Homo sapiens proteasome (prosome, macropain) activator subunit 2 (PA28 beta) (PSME2), mRNA

<400> 7

tggggagtga aagcgaaagc ccgggcgact agccgggaga ccagagatct agcgactgaa 60
gcagcatggc caagccgtgt ggggtgcgcc tgagcgggga agcccgcaaa caggtggagg 120
tcttcagaca gaatcttttc caggaggctg aggaattcct ctacagattc ttgccacaga 180
aaatcatata cctgaatcag ctcttgcaag aggactccct caatgtggct gacttgactt 240
ccctccgggc cccactggac atccccatcc cagaccctcc acccaaggat gatgagatgg 300
aaacagataa gcaggagaag aaagaagtcc ataagtgtgg atttctccct gggaatgaga 360
aagtcctgtc cctgcttgcc ctggttaagc cagaagtctg gactctcaaa gagaaatgca 420
ttctggtgat tacatggatc caacacctga tccccaagat tgaagatgga aatgattttg 480
gggtagcaat ccaggagaag gtgctggaga gggtgaatgc cgtcaagacc aaagtggaag 540
ctttccagac aaccatttcc aagtacttct cagaacgtgg ggatgctgtg gccaaggcct 600
ccaaggagac tcatgtaatg gattaccggg ccttggtgca tgagcgagat gaggcagcct 660
atggggagct cagggccatg gtgctggacc tgagggcctt ctatgctgag ctttatcata 720
tcatcagcag caacctggag aaaattgtca acccaaaggg tgaagaaaag ccatctatgt 780
actgaacccg ggactagaag gaaaataaat gatctatatg ttgtgtgga 829

<210> 8
<211> 2974
<212> DNA
<213> NM_004363.1 Homo sapiens carcinoembryonic antigen-related cell adhesion molecule 5 (CEACAM5), mRNA

<400> 8

ctcagggcag agggaggaag gacagcagac cagacagtca cagcagcctt gacaaaacgt 60
tcctggaact caagctcttc tccacagagg aggacagagc agacagcaga gaccatggag 120

```
tctccctcgg cccctcccca cagatggtgc atcccctggc agaggctcct gctcacagcc   180
tcacttctaa ccttctggaa cccgcccacc actgccaagc tcactattga atccacgccg   240
ttcaatgtcg cagaggggaa ggaggtgctt ctacttgtcc acaatctgcc ccagcatctt   300
tttggctaca gctggtacaa aggtgaaaga gtggatggca accgtcaaat tataggatat   360
gtaataggaa ctcaacaagc taccccaggg cccgcataca gtggtcgaga gataatatac   420
cccaatgcat ccctgctgat ccagaacatc atccagaatg acacaggatt ctacacccta   480
cacgtcataa agtcagatct tgtgaatgaa gaagcaactg gccagttccg ggtataccccg   540
gagctgccca agccctccat ctccagcaac aactccaaac ccgtggagga caaggatgct   600
gtggccttca cctgtgaacc tgagactcag gacgcaacct acctgtggtg ggtaaacaat   660
cagagcctcc cggtcagtcc caggctgcag ctgtccaatg gcaacaggac cctcactcta   720
ttcaatgtca caagaaatga cacagcaagc tacaaatgtg aaacccagaa cccagtgagt   780
gccaggcgca gtgattcagt catcctgaat gtcctctatg gcccggatgc ccccaccatt   840
tcccctctaa acacatctta cagatcaggg gaaaatctga acctctcctg ccacgcagcc   900
tctaacccac ctgcacagta ctcttggttt gtcaatggga ctttccagca atccacccaa   960
gagctcttta tccccaacat cactgtgaat aatagtggat cctatacgtg ccaagcccat  1020
aactcagaca ctggcctcaa taggaccaca gtcacgacga tcacagtcta tgcagagcca  1080
cccaaaccct tcatcaccag caacaactcc aaccccgtgg aggatgagga tgctgtagcc  1140
ttaacctgtg aacctgagat tcagaacaca acctacctgt ggtgggtaaa taatcagagc  1200
ctcccggtca gtcccaggct gcagctgtcc aatgacaaca ggaccctcac tctactcagt  1260
gtcacaagga atgatgtagg accctatgag tgtggaatcc agaacgaatt aagtgttgac  1320
cacagcgacc cagtcatcct gaatgtcctc tatggcccag acgaccccac catttccccc  1380
tcatacacct attaccgtcc aggggtgaac ctcagcctct cctgccatgc agcctctaac  1440
ccacctgcac agtattcttg gctgattgat gggaacatcc agcaacacac acaagagctc  1500
tttatctcca acatcactga gaagaacagc ggactctata cctgccaggc caataactca  1560
gccagtggcc acagcaggac tacagtcaag acaatcacag tctctgcgga gctgcccaag  1620
ccctccatct ccagcaacaa ctccaaaccc gtggaggaca aggatgctgt ggccttcacc  1680
tgtgaacctg aggctcagaa cacaacctac ctgtggtggg taaatggtca gagcctccca  1740
gtcagtccca ggctgcagct gtccaatggc aacaggaccc tcactctatt caatgtcaca  1800
agaaatgacg caagagccta tgtatgtgga atccagaact cagtgagtgc aaaccgcagt  1860
gacccagtca ccctggatgt cctctatggg ccggacaccc ccatcatttc ccccccagac  1920
tcgtcttacc tttcgggagc gaacctcaac ctctcctgcc actcggcctc taacccatcc  1980
ccgcagtatt cttggcgtat caatgggata ccgcagcaac acacacaagt tctctttatc  2040
gccaaaatca cgccaaataa taacgggacc tatgcctgtt ttgtctctaa cttggctact  2100
ggccgcaata attccatagt caagagcatc acagtctctg catctggaac ttctcctggt  2160
```

ctctcagctg gggccactgt cggcatcatg attggagtgc tggttggggt tgctctgata 2220
tagcagccct ggtgtagttt cttcatttca ggaagactga cagttgtttt gcttcttcct 2280
taaagcattt gcaacagcta cagtctaaaa ttgcttcttt accaaggata tttacagaaa 2340
agactctgac cagagatcga gaccatccta gccaacatcg tgaaacccca tctctactaa 2400
aaatacaaaa atgagctggg cttggtggcg cgcacctgta gtcccagtta ctcgggaggc 2460
tgaggcagga gaatcgcttg aacccgggag gtggagattg cagtgagccc agatcgcacc 2520
actgcactcc agtctggcaa cagagcaaga ctccatctca aaaagaaaag aaaagaagac 2580
tctgacctgt actcttgaat acaagtttct gataccactg cactgtctga gaatttccaa 2640
aactttaatg aactaactga cagcttcatg aaactgtcca ccaagatcaa gcagagaaaa 2700
taattaattt catgggacta aatgaactaa tgaggattgc tgattcttta aatgtcttgt 2760
ttcccagatt tcaggaaact tttttctttt taagctatcc actcttacag caatttgata 2820
aaatatactt ttgtgaacaa aaattgagac atttacattt tctccctatg tggtcgctcc 2880
agacttggga aactattcat gaatatttat attgtatggt aatatagtta ttgcacaagt 2940
tcaataaaaa tctgctcttt gtataacaga aaaa 2974

<210> 9
<211> 5028
<212> DNA
<213> NM_005766.2| Homo sapiens FERM, RhoGEF (ARHGEF) and pleckstrin domain protein 1 (chondrocyte-derived) (FARP1), transcript variant 1, mRNA

<400> 9

gcggccgctg cccgctttgc gccgctcctc cctgcgcgag tagcgctggc cccggcgtcg 60
aggcggccat ggcgacccgg agcccgctcc ccacccaccc cgcctgctcc gccctcccct 120
ccgccccgcg ccacctttga tggctcggac ctcagccggc caccgccagc cctgctcgcg 180
cgcccgcgcc gccgccgccc gcgggtatta atagccggcg ccgccgcgcc ctcggccgcc 240
gggggcttgg gagccgccga tcccggagcc cgagccggga gagggagccg ccgcagccgc 300
cggcgctgtg gagatattct ctaagccgct ttcatcatgg gagaaataga gcagaggccg 360
accccaggat cacgactggg ggccccggaa aattcgggga tcagtacctt ggaacgtgga 420
cagaagccgc ccccaacacc ttcaggaaaa ctcgtgtcca tcaaaatcca gatgctggat 480
gacacccagg aggcatttga agttccacaa agagctcctg ggaaggtgct gctggatgca 540
gtttgcaacc acctcaacct cgtggaaggt gactattttg gcctcgagtt tcctgatcac 600
aaaaagatca cggtgtggct ggatctccta aaacccattg tgaaacagat tagaaggcca 660
aagcacgttg ttgttaagtt tgtggtgaaa ttctttccgc ctgaccacac acaactccaa 720
gaagaactca caaggtacct gttcgcgctg caggtgaagc aggacttggc tcaaggcagg 780

ttgacgtgta atgacaccag cgcagctctc ttgatttcac acattgtgca atctgagatt 840
ggggattttg atgaagcctt ggacagagag cacttagcaa aaaataaata catacctcag 900
caagacgcac tagaggacaa aatcgtggaa tttcaccata accacattgg acaaacacca 960
gcagaatcag atttccagct cctagagatt gcccgtcggc tagagatgta tggaatccgg 1020
ttgcacccgg ccaaggacag ggaaggcacg aagatcaatc tggccgttgc caacacggga 1080
attctagtgt ttcagggttt cactaagatc aatgccttca actgggccaa ggtgcggaag 1140
ctgagcttca agaggaagcg ctttctcatc aagctccggc cagatgccaa tagtgcgtac 1200
caggatacct tggaattcct gatggccagt cgggatttct gcaagtcctt ctggaaaatc 1260
tgtgttgaac atcatgcctt ctttagactt tttgaagagc ccaaaccaaa gcccaagccc 1320
gtcctcttta gccgggggtc atcatttcgg ttcagtggtc ggactcagaa gcaggttctc 1380
gactatgtta aagaaggagg acataagaag gtgcagtttg aaaggaagca cagcaagatt 1440
cattctatcc ggagccttgc ttcacagcct acagaactga attcggaagt gctggagcag 1500
tctcagcaga gcaccagcct tacatttgga gaaggtgccg aatctccagg gggccagagc 1560
tgccggcgag gaaaggaacc gaaggtttcc gccggggagc cggggtcgca cccgagccct 1620
gcgccgagga gaagccccgc gggtaacaag caggcggacg gagccgcctc ggcgcccacg 1680
gaggaagagg aggaggtcgt taaggatagg acccagcaga gtaaacctca gccccgcag 1740
ccaagcacag gctccctgac tggcagtcct cacctttccg agctgtctgt gaactcgcag 1800
gggggagtgg cccctgccaa cgtgaccttg tctcccaacc tgagccccga caccaagcag 1860
gcctctccct tgatcagccc gctgctgaat gaccaggcct gcccccggac ggacgatgag 1920
gatgagggcc ggaggaagag attcccaact gataaagcgt acttcatagc taaggaagtg 1980
tctaccaccg agcgaacata tctgaaggat ctcgaagtta tcacttcgtg gtttcagagc 2040
acagtgagca aagaggacgc catgccggaa gcactgaaaa gtctcatatt cccgaatttt 2100
gaacctttgc acaaatttca tactaatttt ctcaaggaaa ttgagcaacg acttgccctg 2160
tgggaaggcc gctcaaatgc ccaaatcaga gattaccaaa gaatcggcga tgtcatgctg 2220
aagaacattc agggcatgaa gcacctggcg gctcacctgt ggaagcacag cgaggccttg 2280
gaggccctgg agaatggaat caagagctcc cggcggctgg agaacttctg cagagacttt 2340
gagctgcaga aggtgtgtta cctaccgctc aacaccttcc tcctgcggcc actgcaccgg 2400
ctcatgcact acaagcaggt cctggagcgg ctgtgcaaac accacccgcc gagccacgcc 2460
gacttcaggg actgccgagc cgctttggca gagatcacgg agatggtggc acagctccac 2520
ggtacgatga tcaagatgga gaatttccag aagctgcacg aactcaagaa agatttgatt 2580
ggcattgaca atcttgtggt tccgggaagg gagttcatcc gtctgggcag cctcagcaag 2640
ctctcgggga aggggctcca gcagcgcatg ttcttcctgt tcaacgacgt cctgctatac 2700
acgagccggg ggctgacggc ctccaatcag tttaaagtcc acgggcagct cccgctctat 2760

ggcatgacga ttgaggagag cgaagacgag tggggggtgc cccactgcct gaccctccgg 2820
ggccagcggc agtccatcat cgtggccgcc agttctcggt ccgagatgga gaagtgggtt 2880
gaggacatcc agatggccat tgacctggcg gagaagagca gcagccccgc ccctgagttc 2940
ctggccagca gcccccctga caacaagtcc cctgatgaag ccaccgcggc tgaccaggag 3000
tcagaggatg acctgagcgc ctcgcgcaca tcgctggagc gccaggcccc gcaccgcggc 3060
aacacaatgg tgcacgtgtg ctggcaccgc aacaccagcg tctccatggt ggacttcagc 3120
atcgcagtgg agaatcagtt gtctggaaac ctgctgagga aattcaaaaa cagcaacggg 3180
tggcagaagc tgtgggtggt gttcacaaac ttctgcctgt tcttctacaa atcacaccag 3240
gacaatcatc cccttgccag cctgcctctg ctcggctact cgctcaccat cccctctgag 3300
tccgagaaca tccagaaaga ctacgtgttc aagctgcact tcaagtccca cgtctactac 3360
ttcagggcgg aaagcgagta cacgttcgaa aggtggatgg aagtgatccg cagtgccacc 3420
agctctgcct cgcgacccca cgtgttgagt cacaaagagt ctcttgtgta ttgatggccg 3480
gacacactcg tttccgcagt ggctgctttc ctggaagacg tttcctttct tctgtattaa 3540
tgaagcctgg taaaattaac acctgtctga aaatcaaaaa catggcttcc cagcagctct 3600
cctgtctcca cagccgcgtt ttttaacccc gacctctcag cgtctgaatg aacagcgctc 3660
ccacctccag tcctggcatc cgctgggggc gctgttcttt agctagtgcc agtattaaaa 3720
cattgtcatt acgagagtgc caaatgacat cttccctcca ccctgcccct gaaaaacagt 3780
acacacacat ccgttcaaca caagacaggg caagtgtttt tcttcctaaa aaaagttctt 3840
tcttttatta ttttcaccta ttggctgctg cattttacga agtggacttc ccggtgtttg 3900
tttgtttgtt tgcaatacac tcagtgcagc cttaagcaaa tgagatcatt ttcagatttc 3960
attttttttt tcagtctttc tacttttgta ataataggaa gttagtagga ctcacttctc 4020
tgattaataa gcaatttgca gcacacagcg ttccactgcg ggtttcacg ctcacctgaa 4080
aacacctgtt cccaacctac ttcttggtgc aagttgacca aatcgtttta agtggtaact 4140
ctttccaacc gtagcagggt tgttttctgt taagcaaagc cgagatccag tgcaatacct 4200
ggactgtcac cgtcctgtga gtggtgtaca caatgggaag ataataagcc gtggtgtttt 4260
gctgtctgtc tgtgtcacaa gcatgaaaac ccgtgtgtca ttgatcagca ccatttgtgg 4320
tatgttccgt gatgagcgtt tagtgagcct gctggctgca gagcactatg aaatcatggt 4380
acgtagtccc cggcacctgt cgttattcct atatcctcct gcaactgtgg tttgaaactg 4440
cgcattctct agtagtatat atcgtgcctg tcttcaaaaa catttccctt tttatactca 4500
ttccccccag gcatggggta gtgtcagtcg gactgcacag ggaacacggt ttccagtggc 4560
tttggcccct actcgggaaa cgtctgcctg ttctcgatgg tgatggggtg gctgccattc 4620
ccttggtttt cctaagccct ttctaacgag agtctcaaac aagcggaggc gagggccaat 4680
tcaaccccat tctttccagc gccccgcacc atagcacctg cccacctgag aaccaggaac 4740
gcaccctctc tgtggagctc tgactggtgt agctggaaac aaacagcaac ttgcaaacgg 4800

acgaagagcc tgccgtgtgt taatcatttg ccttacaaga tgtaccagac ggtttccagt 4860
actaacaaag ggaataaaaa tacctcacgc cacaatccag catattgatg ttttaaggca 4920
aaacaaaaaa aaaaaaaaaa aaaaaaaaaa aaaaaaaaaa aaaaaaaaaa aaaaaaaaaa 4980
aaaaaaaaaa aaaaaaaaaa aaaaaaaaaa aaaaaaaaaa aaaaaaaa 5028

<210> 10
<211> 7787
<212> DNA
<213> NM_012334.1| Homo sapiens myosin X (MYO10), mRNA

<400> 10

gagacaaagg ctgccgtcgg gacgggcgag ttagggactt gggtttgggc gaacaaaagg 60
tgagaaggac aagaagggac cgggcgatgg cagcagggga gccccgcggg cgcgcgtcct 120
cgggagtggc gccgtgacac gcatggtttc cccggacccg cggcggcgct gacttccgcg 180
agtcggagcg gcactcggcg agtccgggac tgcgctggaa caatggataa cttcttcacc 240
gagggaacac gggtctggct gagagaaaat ggccagcatt ttccaagtac tgtaaattcc 300
tgtgcagaag gcatcgtcgt cttccggaca gactatggtc aggtattcac ttacaagcag 360
agcacaatta cccaccagaa ggtgactgct atgcacccca cgaacgagga gggcgtggat 420
gacatggcgt ccttgacaga gctccatggc ggctccatca tgtataactt attccagcgg 480
tataagagaa atcaaatata tacctacatc ggctccatcc tggcctccgt gaacccctac 540
cagcccatcg ccgggctgta cgagcctgcc accatggagc agtacagccg gcgccacctg 600
ggcgagctgc cccgcacat cttcgccatc gccaacgagt gctaccgctg cctgtggaag 660
cgctacgaca accagtgcat cctcatcagt ggtgaaagtg gggcaggtaa aaccgaaagc 720
actaaattga tcctcaagtt tctgtcagtc atcagtcaac agtctttgga attgtcctta 780
aaggagaaga catcctgtgt tgaacgagct attcttgaaa gcagccccat catggaagct 840
ttcggcaatg cgaagaccgt gtacaacaac aactctagtc gctttgggaa gtttgttcag 900
ctgaacatct gtcagaaagg aaatattcag ggcgggagaa ttgtagatta tttattagaa 960
aaaaaccgag tagtaaggca aaatcccggg gaaaggaatt atcacatatt ttatgcactg 1020
ctggcagggc tggaacatga agaaagagaa gaatttttatt tatctacgcc agaaaactac 1080
cactacttga atcagtctgg atgtgtagaa gacaagacaa tcagtgacca ggaatccttt 1140
agggaagtta ttacggcaat ggacgtgatg cagttcagca aggaggaagt tcgggaagtg 1200
tcgaggctgc ttgctggtat actgcatctt gggaacatag aatttatcac tgctggtggg 1260
gcacaggttt ccttcaaaac agctttgggc agatctgcgg agttacttgg gctggaccca 1320
acacagctca cagatgcttt gacccagaga tcaatgttcc tcaggggaga agagatcctc 1380

acgcctctca atgttcaaca ggcagtagac agcagggact ccctggccat ggctctgtat 1440
gcgtgctgct ttgagtgggt aatcaagaag atcaacagca ggatcaaagg caatgaggac 1500
ttcaagtcta ttggcatcct cgacatcttt ggatttgaaa actttgaggt taatcacttt 1560
gaacagttca atataaacta tgcaaacgag aaacttcagg agtacttcaa caagcatatt 1620
ttttctttag aacaactaga atatagccgg gaaggattag tgtgggaaga tattgactgg 1680
atagacaatg gagaatgcct ggacttgatt gagaagaaac ttggcctcct agcccttatc 1740
aatgaagaaa gccattttcc tcaagccaca gacagcacct tattggagaa gctacacagt 1800
cagcatgcga ataaccactt ttatgtgaag cccagagttg cagttaacaa ttttggagtg 1860
aagcactatg ctggagaggt gcaatatgat gtccgaggta tcttggagaa gaacagagat 1920
acatttcgag atgaccttct caatttgcta agagaaagcc gatttgactt tatctacgat 1980
ctttttgaac atgtttcaag ccgcaacaac caggatacct tgaaatgtgg aagcaaacat 2040
cggcggccta cagtcagctc acagttcaag gactcactgc attccttaat ggcaacgcta 2100
agctcctcta atcctttctt tgttcgctgt atcaagccaa acatgcagaa gatgccagac 2160
cagtttgacc aggcggttgt gctgaaccag ctgcggtact cagggatgct ggagactgtg 2220
agaatccgca aagctgggta tgcggtccga agaccctttc aggactttta caaaaggtat 2280
aaagtgctga tgaggaatct ggctctgcct gaggacgtcc gagggaagtg cacgagcctg 2340
ctgcagctct atgatgcctc caacagcgag tggcagctgg ggaagaccaa ggtctttctt 2400
cgagaatcct tggaacagaa actggagaag cggagggaag aggaagtgag ccacgcggcc 2460
atggtgattc gggcccatgt cttgggcttc ttagcacgaa aacaatacag aaaggtcctt 2520
tattgtgtgg tgataataca gaagaattac agagcattcc ttctgaggag gagatttttg 2580
cacctgaaaa aggcagccat agttttccag aagcaactca gaggtcagat tgctcggaga 2640
gtttacagac aattgctggc agagaaaagg gagcaagaag aaaagaagaa acaggaagag 2700
gaagaaaaga agaaacggga ggaagaagaa agagaaagag agagagagcg aagagaagcc 2760
gagctccgcg cccagcagga agaagaaacg aggaagcagc aagaactcga agccttgcag 2820
aagagccaga aggaagctga actgacccgt gaactggaga aacagaagga aaataagcag 2880
gtggaagaga tcctccgtct ggagaaagaa atcgaggacc tgcagcgcat gaaggagcag 2940
caggagctgt cgctgaccga ggcttccctg cagaagctgc aggagcggcg ggaccaggag 3000
ctccgcaggc tggaggagga agcgtgcagg gcggcccagg agttcctcga gtccctcaat 3060
ttcgacgaga tcgacgagtg tgtccggaat atcgagcggt ccctgtcggt gggaagcgaa 3120
ttttccagcg agctggctga gagcgcatgc gaggagaagc ccaacttcaa cttcagccag 3180
ccctacccag aggaggaggt cgatgagggc ttcgaagccg acgacgacgc cttcaaggac 3240
tccccccaacc ccagcgagca cggccactca gaccagcgaa caagtggcat ccggaccagc 3300
gatgactctt cagaggagga cccatacatg aacgacacgg tggtgcccac cagccccagt 3360
gcggacagca cggtgctgct cgccccatca gtgcaggact ccgggagcct acacaactcc 3420

tccagcggcg agtccaccta ctgcatgccc cagaacgctg gggacttgcc ctccccagac 3480
ggcgactacg actacgacca ggatgactat gaggacggtg ccatcacttc cggcagcagc 3540
gtgaccttct ccaactccta cggcagccag tggtcccccg actaccgctg ctctgtgggg 3600
acctacaaca gctcgggtgc ctaccggttc agctctgagg gggcgcagtc ctcgtttgaa 3660
gatagtgaag aggactttga ttccaggttt gatacagatg atgagctttc ataccggcgt 3720
gactctgtgt acagctgtgt cactctgccg tatttccaca gctttctgta catgaaaggt 3780
ggcctgatga actcttggaa acgccgctgg tgcgtcctca aggatgaaac cttcttgtgg 3840
ttccgctcca agcaggaggc cctcaagcaa ggctggctcc acaaaaaagg ggggggctcc 3900
tccacgctgt ccaggagaaa ttggaagaag cgctggtttg tcctccgcca gtccaagctg 3960
atgtactttg aaaacgacag cgaggagaag ctcaagggca ccgtagaagt gcgaacggca 4020
aaagagatca tagataacac caccaaggag aatgggatcg acatcattat ggccgatagg 4080
actttccacc tgattgcaga gtccccagaa gatgccagcc agtggttcag cgtgctgagt 4140
caggtccacg cgtccacgga ccaggagatc caggagatgc atgatgagca ggcaaaccca 4200
cagaatgctg tgggcacctt ggatgtgggg ctgattgatt ctgtgtgtgc ctctgacagc 4260
cctgatagac ccaactcgtt tgtgatcatc acggccaacc gggtgctgca ctgcaacgcc 4320
gacacgccgg aggagatgca ccactggata accctgctgc agaggtccaa aggggacacc 4380
agagtggagg gccaggaatt catcgtgaga ggatggttgc acaaagaggt gaagaacagt 4440
ccgaagatgt cttcactgaa actgaagaaa cggtggtttg tactcaccca caattccctg 4500
gattactaca agagttcaga gaagaacgcg ctcaaactgg ggaccctggt cctcaacagc 4560
ctctgctctg tcgtcccccc agatgagaag atattcaaag agacaggcta ctggaacgtc 4620
accgtgtacg ggcgcaagca ctgttaccgg ctctacacca agctgctcaa cgaggccacc 4680
cggtggtcca gtgccattca aaacgtgact gacaccaagg ccccgatcga cacccccacc 4740
cagcagctga ttcaagatat caaggagaac tgcctgaact cggatgtggt ggaacagatt 4800
tacaagcgga acccgatcct tcgatacacc catcacccct tgcactcccc gctcctgccc 4860
cttccgtatg gggacataaa tctcaacttg ctcaaagaca aaggctatac caccccttcag 4920
gatgaggcca tcaagatatt caattccctg cagcaactgg agtccatgtc tgacccaatt 4980
ccaataatcc agggcatcct acagacaggg catgacctgc gacctctgcg ggacgagctg 5040
tactgccagc ttatcaaaca gaccaacaaa gtgccccacc ccggcagtgt gggcaacctg 5100
tacagctggc agatcctgac atgcctgagc tgcaccttcc tgccgagtcg agggattctc 5160
aagtatctca agttccatct gaaaaggata cgggaacagt ttccaggaac cgagatggaa 5220
aaatacgctc tcttcactta cgaatctctt aagaaaacca aatgccgaga gtttgtgcct 5280
tcccgagatg aaatagaagc tctgatccac aggcaggaaa tgacatccac ggtctattgc 5340
catggcggcg gctcctgcaa gatcaccatc aactcccaca ccactgctgg ggaggtggtg 5400

gagaagctga tccgaggcct ggccatggag gacagcagga acatgtttgc tttgtttgaa 5460
tacaacggcc acgtcgacaa agccattgaa agtcgaaccg tcgtagctga tgtcttagcc 5520
aagtttgaaa agctggctgc cacatccgag gttggggacc tgccatggaa attctacttc 5580
aaactttact gcttcctgga cacagacaac gtgccaaaag acagtgtgga gtttgcattt 5640
atgtttgaac aggcccacga agcggttatc catggccacc atccagcccc ggaagaaaac 5700
ctccaggttc ttgctgccct gcgactccag tatctgcagg gggattatac tctgcacgct 5760
gccatcccac ctctcgaaga ggtttattcc ctgcagagac tcaaggcccg catcagccag 5820
tcaaccaaaa ccttcacccc ttgtgaacgg ctggagaaga ggcggacgag cttcctagag 5880
gggaccctga ggcggagctt ccggacagga tccgtggtcc ggcagaaggt cgaggaggag 5940
cagatgctgg acatgtggat taaggaagaa gtctcctctg ctcgagccag tatcattgac 6000
aagtggagga aatttcaggg aatgaaccag gaacaggcca tggccaagta catggccttg 6060
atcaaggagt ggcctggcta tggctcgacg ctgtttgatg tggagtgcaa ggaaggtggc 6120
ttccctcagg aactctggtt gggtgtcagc gcggacgccg tctccgtcta caagcgtgga 6180
gagggaagac cactggaagt cttccagtat gaacacatcc tctcttttgg ggcacccctg 6240
gcgaatacgt ataagatcgt ggtcgatgag agggagctgc tctttgaaac cagtgaggtg 6300
gtggatgtgg ccaagctcat gaaagcctac atcagcatga tcgtgaagaa gcgctacagc 6360
acgacacgct ccgccagcag ccagggcagc tccaggtgaa ggcgggacag agcccacctg 6420
tctttgctac ctgaacgcac caccctctgg cctaggctgg ctccagtgtg ccatgcccag 6480
ccaaaacaaa cacagagctg cccaggcttt ctggaagctt ctggtctgag ggaggtgtct 6540
ccgaggatcc ttttgcctgc cgccttcatt gatcctgtat taagctgtca actttaacag 6600
tctgcacagt ttccaaagct ttactactct tagaggacac atgccttaaa aaaggagggg 6660
aggaaccacg ctgccaccaa agcagccgga agtgccttaa cttgtggaac caacactaat 6720
cgaccgtaac tgtgctactg aagggaactg cctttccccc ttctgggga gacttaacag 6780
agcgtggaag gggggcattc tctgtcaatg atgcactaac ctcccaacct gatttccccg 6840
aatctgaggg aaggtgaggg agtgggaagg gggatggaga gctcgagggg acagtgtgtt 6900
tgagctggag tgctgcgggc agcctttctc atggaatgac atgaatcaac tttttctttt 6960
gtttcatctt ttaagtgtac gtgcttgcct gttcgtgcat gtgttcataa actcaacact 7020
ttaatcatgg tttcatgagc attaaaaagc aaagggaaaa aggatgtgta atggtgtaca 7080
cagtctgtat attttaataa tgcagagcta tagtctcaat tgttacttta taaggtggtt 7140
ttattaacaa acccaaatcc tggattttcc tgtctttgct gtattttgaa aaacacgtgt 7200
tgactccatt gttttacatg tagcaaagtc tgccatctgt gtctgctgta ttataaacag 7260
ataagcagcc tacaagataa ctgtatttat aaaccactct tcaacagctg gctccagtgc 7320
tggttttaga acaagaatga agtcattttg gagtctttca tgtctaaaag atttaagtta 7380
aaaacaaagt gttacttgga aggttagctt ctatcattct ggatagatta cagatataat 7440

aaccatgttg actatggggg agagacgctg cattccagaa acgtcttaac acttgagtga 7500
atcttcaaag gaccctgaca ttaaatgctg aggctttaat acacacatat tttatcccaa 7560
gtttataatg gtggtctgaa caaggcacct gtaaataaat cagcatttat gaccagaaga 7620
aaaataatct ggtcttggac tttttatttt tatatggaaa agttttaagg acttgggcca 7680
actaagtcta cccacacgaa aaaagaaatt tgccttgtcc ctttgtgtac aaccatgcaa 7740
aactgtttgt tggctcacag aagttctgac aataaaagat actagct 7787

<210> 11
<211> 2033
<212> DNA
<213> NM_001533-1| Homo sapiens heterogeneous nuclear ribonucleoprotein L (HNRPL), mRNA

<400> 11

ggacgagcag cggaggcggt cgggagcgat ggtgaagatg gcggcggcgg gcggcggagg 60
cggcggtggc cgctactacg gcggcggcag tgagggcggc cgggccccta agcggctcaa 120
gactgacaac gccggcgacc agcacggagg cggcggcggt ggcggtggag gagccggggc 180
ggcgggcggc ggcggcggtg gggagaacta cgatgacccg cacaaaaccc ctgcctcccc 240
agttgtccac atcaggggcc tgattgacgg tgtggtggaa gcagaccttg tggaggcctt 300
gcaggagttt ggacccatca gctatgtggt ggtaatgcct aaaaagagac aagcactggt 360
ggagtttgaa gatgtgttgg gggcttgcaa cgcagtgaac tacgcagccg acaaccaaat 420
atacattgct ggtcacccag cttttgtcaa ctactctacc agccagaaga tctcccgccc 480
tggggactcg gatgactccc ggagcgtgaa cagtgtgctt ctctttacca tcctgaaccc 540
catttattcg atcaccacgg atgttcttta cactatctgt aatccttgtg gccctgtcca 600
gagaattgtc attttcagga agaatggagt tcaggcgatg gtggaatttg actcagttca 660
aagtgcccag cgggccaagg cctctctcaa tggggctgat atctattctg gctgttgcac 720
tctgaagatc gaatacgcaa agcctacacg cttgaatgtg ttcaagaatg atcaggatac 780
ttgggactac acaaacccca atctcagtgg acaaggtgac cctggcagca accccaacaa 840
acgccagagg cagcccctc tcctgggaga tcaccccgca gaatatggag ggccccacgg 900
tgggtaccac agccattacc atgatgaggg ctacgggccc cccccacctc actacgaagg 960
gagaaggatg ggtccaccag tgggggtca ccgtcggggc ccaagtcgct acggccccca 1020
gtatgggcac cccccacccc ctcccccacc acccgagtat ggccctcacg ccgacagccc 1080
tgtgctcatg gtctatggct tggatcaatc taagatgaac ggtgaccgag tcttcaatgt 1140
cttctgctta tatggcaatg tggagaaggt gaaattcatg aaaagcaagc cgggggccgc 1200
catggtggag atggctgatg gctacgctgt agaccgggcc attacccacc tcaacaacaa 1260

```
cttcatgttt gggcagaagc tgaatgtctg tgtctccaag cagccagcca tcatgcctgg   1320
tcagtcatac gggttggaag acgggtcttg cagttacaaa gacttcagtg aatcccggaa   1380
caatcggttc tccaccccag agcaggcagc caagaaccgc atccagcacc ccagcaacgt   1440
gctgcacttc ttcaacgccc cgctggaggt gaccgaggag aacttctttg agatctgcga   1500
tgagctggga gtgaagcggc catcttctgt gaaagtattc tcaggcaaaa gtgagcgcag   1560
ctcctctgga ctgctggagt gggaatccaa gagcgatgcc ctggagactc tgggcttcct   1620
gaaccattac cagatgaaaa acccaaatgg tccataccct tacactctga agttgtgttt   1680
ctccactgct cagcacgcct cctaattagg tgcctaggaa gagtcccatc tgagcaggaa   1740
gacatttctc tttcctttat gccatttttt gtttttgtta tttgcaaaag atcttgtatt   1800
cctttttttt tttttttttt tttaaatgct aggtttgtag aggcttactt aaccttaatg   1860
gaaacgctgg aaatctgcag gggagggag aggggaactg ttatctccca agattaacct   1920
tcacttttaa aaaattattg tacatgtgat tttttttttt cctgttcata catttgtgct   1980
gcccatgtac tcttggcaca tttcaataaa attgtttgga aaataaacac agc          2033
```

<210> 12
<211> 3453
<212> DNA
<213> NM_001144.3| Homo sapiens autocrine motility factor receptor (AMFR), transcript variant 1, mRNA

<400> 12

```
gggccgccgc agaggcccgg ccgcagcgca gggaagcctg ggggccagag gtcgccgctg     60
ccgccatgcc gctgctcttc ctcgagcgct tcccctggcc cagcctccgc acctacacgg    120
gcctcagcgg cctggccctg ctgggcacca tcatcagcgc ctaccgcgcg ctcagccagc    180
ccgaggccgg ccccggcgag ccggaccagc taacggcctc gctgcagcct gagccgccgg    240
cgcccgcccg gccgagcgcc gggggacccc gggcccgcga tgtggcccag tacctgctct    300
cagacagcct cttcgtgtgg gttctagtaa ataccgcttg ctgtgttttg atgttggtgg    360
ctaagctcat ccagtgtatt gtgtttggcc ctcttcgagt gagtgagaga cagcatctca    420
aagacaaatt ttggaatttt attttctaca agttcatttt catctttggt gtgctgaatg    480
tccagacagt ggaagaggtg gtcatgtggt gcctctggtt tgccggactt gtctttctgc    540
acctgatggt tcagctctgc aaggatcgat ttgaatatct ttccttctcg cccaccacgc    600
cgatgagcag ccacggtcga gtcctgtccc tgttggttgc catgctgctt tcctgctgtg    660
gactggcggc cgtctgctcc atcaccggct acacccacgg aatgcacacc ttggctttca    720
tggctgcaga gtctcttctt gtgacagtga ggactgctca tgtgatttta cgatacgtaa    780
ttcacctctg ggacctcaac cacgaaggga cgtgggaagg aaaggggacg tatgtctatt    840
```

acacagactt tgtcatggag ctcactctcc tgtccctgga cctcatgcac catattcaca 900
tgttgttatt tggcaacatc tggttatcca tggccagcct ggtcatcttt atgcagctgc 960
gttacctgtt tcatgaggtg caacgtcgaa ttcgtcggca caagaactat ctacgtgtgg 1020
ttggaaacat ggaggccagg tttgcagttg caactccaga ggagctggct gtcaacaatg 1080
acgactgtgc catctgttgg gactccatgc aggctgcgcg gaaactgccc tgtggacatc 1140
ttttccacaa ctcctgtctt cgttcctggc tagaacaaga cacctcctgt ccaacatgca 1200
gaatgtctct taatattgcc gacaataatc gtgtcaggga agaacatcaa ggagagaact 1260
tggatgagaa tttggttcct gtagcagcag ccgaagggag acctcgctta aaccaacaca 1320
atcacttctt ccatttcgat gggtctcgga ttgcgagctg gctgccgagt ttttcggttg 1380
aagtgatgca caccaccaac attcttggca ttacgcaggc cagcaactcc cagctcaatg 1440
caatggctca tcagattcaa gagatgtttc cccaggttcc ataccatctg gtactgcagg 1500
acctccagct gacacgctca gttgaaataa caacagacaa tattttagaa ggacggattc 1560
aagtaccttt tcctacacag cggtcagata gcatcagacc tgcattgaac agtcctgtgg 1620
aaaggccaag cagtgaccag gaagagggag aaacttctgc tcagaccgag cgtgtgccac 1680
tggacctcag tcctcgcctg gaggagacgc tggacttcgg cgaggtggaa gtggagccca 1740
gtgaggtgga agacttcgag gctcgtggga gccgcttctc caagtctgct gatgagagac 1800
agcgcatgct ggtgcagcgt aaggacgaac tcctccagca agctcgcaaa cgtttcttga 1860
acaaaagttc tgaagatgat gcggcctcag agagcttcct cccctcggaa ggtgcgtcct 1920
ctgaccccgt gaccctgcgt cgaaggatgc tggctgccgc cgcggaacgg aggcttcaga 1980
agcagcagac ctcctagcgc tcccttgcct tcctcagctg cctcctgcgc cctgtgcccg 2040
actgactgga ggaggcctgt cccaattctg cccgctccat ggaaaagcgg gcttgactgc 2100
attgccgctg tataaagcat gtggtcttat agtgtttgga cagctgataa atttaatcct 2160
tctttgtaat actttctatg tgacatttct cttcccctta gaaacactgc aaattttaac 2220
tgtaggtatg atctcttctg gtgttgactg gactgcttgg ggtgggggac gatcaggagg 2280
aagtgagcag tcgcctgcct gcagcaggca gcttctactc ctgcctcatg catacgtccc 2340
acaaatgcag gtgtcctgag caccacaccc agtgggaaga gtgtggggga ggcgcacagt 2400
gtgagcccgc ccccacgtcg tggggtaaca tctgttatca aactgctgtc gttgttgtgg 2460
aagcatgtag actgtgccag aggccagacc cacgggctca tgcacccctg agccagcagg 2520
gcatcttgga aaaggaactc ttggttcgat acctggagca gaggagggga aagtccaggg 2580
ctatagggtg tgatgaagtc accccttttct gtcccactac atctgggact gactttccga 2640
gcctccagtc caaagccggc ttgatttccg tgaactctgg tgctcctgca tctcatgagt 2700
gtgccccatg ggtcccctcc cctctcagca tttccttgtc ccgtctggac ctggggagtg 2760
gttaggcagc aagctttggt ttatggtttt cattcattgg tgaagtaaat taggcagtgc 2820

taaagcctgt gggtttggtc cttgaacaag atgtgggcct tgcaagatgg gagagtaaac 2880
cttgaagggc tttattaaag aaataaaaaa gaacttttgt atcttttatc ctgggagcac 2940
tgcgttttcc tagctgtgtt attcctggtt taattcagca gagaaggtaa ggtgtgaacc 3000
tacctgcctt ggagagggcc caggtcccaa atctcttcaa attcttcaca tgtttaactt 3060
taaggatttg aaccatgaag tcataggtta cagacctcag ttttatgccc cattggatta 3120
cttttttttt tttttttttt tttttttttt tactctttga aagctttgtt ttgtggtagt 3180
ccttttggga agaatccagt attatctaca attattggca aagtttaaat gtattttaca 3240
taacggaaag tttttagaat gttgaaaagt aattgaaaaa ggtgataggt aaatttttag 3300
gcaaagataa tttatttcaa taaatctttc aaaagcctta ccttgaaatg ctgttagtaa 3360
atttctgtga tttttttttt taatttgttt tgctgagagc atagctattt gtttttattg 3420
taaaacaata ataataataa aaagcaaact cta 3453

<210> 13
<211> 1351
<212> DNA
<213> NM_013974.1| Homo sapiens dimethylarginine dimethylaminohydrolase 2 (DDAH2), mRNA

<400> 13

ccgcttagac aatgccccgg agccgccaga ccgtcgcgcc cctgccccat cgtagtatat 60
gagctcgcct acacaaggac ccccgctaaa agccagagct cccagtcccc gaggcttgaa 120
gacggggact cccttctcca ccaactctgt cctcgggggg tggggcccca gccgagatca 180
cagcgcgaca ggagtggggg tggccgctgg agacaggtga agaaacaaga aaactaagaa 240
atccgagcgg ttggaggggg agtctgtgtg gatgggatgg ggacgccggg ggaggggctg 300
ggccgctgct cccatgccct gatccgggga gtcccagaga gcctggcgtc gggggaaggt 360
gcgggggctg gccttcccgc tctggatctg gccaaagctc aaagggagca cggggtgctg 420
ggaggtaaac tgaggcaacg actggggcta cagctgctag aactgccacc tgaggagtca 480
ttgccgctgg gaccgctgct tggcgacacg gccgtgatcc aaggggacac ggccctaatc 540
acgcggccct ggagccccgc tcgtaggcca gaggtcgatg gagtccgcaa agccctgcaa 600
gacctggggc tccgaattgt ggaaatagga gacgagaacg cgacgctgga tggcactgac 660
gttctcttca ccggccggga gtttttcgta ggcctctcca aatggaccaa tcaccgagga 720
gctgagatcg tggcggacac gttccgggac ttcgccgtct ccactgtgcc agtctcgggt 780
ccctcccacc tgcgcggtct ctgcggcatg gggggacctc gcactgttgt ggcaggcagc 840
agcgacgctg cccaaaaggc tgtccgggca atggcagtgc tgacagatca cccatatgcc 900
tccctgaccc tcccagatga cgcagctgct gactgcctct ttcttcgtcc tgggttgcct 960

ggtgtgcccc ctttcctcct gcaccgtgga ggtggggatc tgcccaacag ccaggaggca 1020
ctgcagaagc tctctgatgt caccctggta cctgtgtcct gctcagaact ggagaaggct 1080
ggcgccgggc tcagctccct ctgcttggtg ctcagcacac gcccccacag ctgagggcct 1140
ggccttgggg tactgctggc caggggtagg atagtatagg aagtagaagg ggaaggaggg 1200
ttagatagag aatgctgaat aggcagtagt tgggagagag cctcaatatt gggggagggg 1260
agagtgtagg gaaaaggatc cactgggtga atcctccctc tcagaaccaa taaaatagaa 1320
ttgacctttt aaaaaaaaaa aaaaaaaaaa a 1351

<210> 14
<211> 4180
<212> DNA
<213> NM_006291.2| Homo sapiens tumor necrosis factor, alpha-induced protein 2 (TNFAIP2), mRNA

<400> 14

ccagggtgat gctgaagatg atgaccttct tccaaggcct ctagagccat cagcctgtgc 60
caggcaccct cgacttgcct agaggccccc aaaagttgca gtccacatca gaggcagagt 120
cagaggcctc catgtcggag gcctcctctg aggacctggt gccacccctg gaggctgggg 180
cagccccata tagggaggag gaagaggcgg cgaagaagaa gaaggagaag aagaagaagt 240
ccaaaggcct ggccaatgtg ttctgcgtct tcaccaaagg gaagaagaag aagggtcagc 300
ccagctcagc ggagcccgag gacgcagccg ggtccaggca ggggctggat ggcccgcccc 360
ccacagtgga ggagctgaag gcggcgctgg agcgcgggca gctggaggcg gcgcggccgc 420
tgctggcgct ggagcgggag ctggcggcgg cggcggcggc gggcggtgtg agcgaggagg 480
agctggtgcg gcgccagagc aaggtggagg cgctgtacga gctgctgcgc gaccaggtgc 540
tgggcgtgct gcggcggccg ctggaggcgc cgcccgagcg gctgcgccag gcgctggccg 600
tggtggcgga gcaggagcgc gaggaccgcc aggcggcggc ggcggggccg gggacctcgg 660
ggctggcggc cacgcgcccg cggcgctggc tgcagctgtg gcggcgcggc gtggcggagg 720
cggccgagga gcgcatgggc cagcggccgg ccgcgggcgc cgaggtcccc gagagcgtct 780
ttctgcactt gggccgcacc atgaaggagg acctggaggc cgtggtggag cggctgaagc 840
cgctgttccc cgccgagttc ggcgtcgtgg cggcctacgc cgagagctac caccagcact 900
tcgcggccca cctggccgcc gtggcgcagt tcgagctgtg cgagcgcgac acctacatgc 960
tgctgctctg ggtgcagaac ctctacccca atgacatcat caacagcccc aagctggtgg 1020
gtgagctgca gggtatgggg ctcgggagcc tcctgccccc caggcagatc cgactgctgg 1080
aggccacatt cctgtccagt gaggcggcca atgtgaggga gttgatggac cgagctctgg 1140
agctagaggc acggcgctgg gctgaggatg tgcctcccca gaggctggac ggccactgcc 1200

acagcgagct ggccatcgac atcatccaga tcacctccca ggcccaggcc aaggccgaga 1260
gcatcacgct ggacttgggc tcacagataa agcgggtgct gctggtggag ctgcctgcgt 1320
tcctgaggag ctaccagcgc gcctttaatg aatttctgga gagaggcaag cagctgacga 1380
attacagggc caatgttatt gccaacatca acaactgcct gtccttccgg atgtccatgg 1440
agcagaattg gcaggtaccc caggacaccc tgagcctcct gctgggcccc ctgggtgagc 1500
tcaagagcca cggctttgac accctgctcc agaacctgca tgaggacctg aagccactgt 1560
tcaagaggtt cacgcacacc cgctgggcgg cccctgtgga gaccctggaa aacatcatcg 1620
ccactgtaga cacgaggctg cctgagttct cagagctgca gggctgtttc cgggaggagc 1680
tcatggaggc cttgcacctg cacctggtga aggagtacat catccaactc agcaaggggc 1740
gcctggtcct caagacggcc gagcagcagc agcagctggc tgggtacatc ctggccaatg 1800
ctgacaccat ccagcacttc tgcacccagc acggctcccc ggcgacctgg ctgcagcctg 1860
ctctccctac gctggccgag atcattcgcc tgcaggaccc cagtgccatc aagattgagg 1920
tggccactta tgccacctgc taccctgact tcagcaaagg ccacctgagc gctatcctgg 1980
ccatcaaggg gaacctatcc aacagtgagg tcaagcgcat ccggagcatc ttggacgtca 2040
gcatgggggc gcaggagccc tcccggcccc tattttccct tataaaggtt ggttagcttt 2100
tcctgtggcc tgacctgcct gtgagtgccc agcaagcctt gggcacaccc cgctgggagc 2160
tgttaagagc agcgctggtt ctcggttcct cccgggtctc ctgtgctctg atgctacttc 2220
tgcctagccc tggcggaggt gcaggccctg tcagctggaa ctggacagac cttggtttgt 2280
ttacatgtcc gatgggggca ggagctccca tcctgggcag ccaaccaggc aacaccaagg 2340
actctttgta aacgatagct gatcgtgtgc acgcaaggaa agaaccagga gggagagtgc 2400
agccaggctc agggatcccc ggacacctct gtccagagcc cctccacagt cggcctcatg 2460
actgtcctcc tcgtgggtgg ggccgagggc cctcttcagc tctctggaga caggggccga 2520
gcctcaccca tctgccctct gcagcccagg gccgccgtga gcgggattca gcaatggtgg 2580
aatggaagac agaactggaa gagaaagaag gaaaagatga gctctcgtct ggcaggggct 2640
tttagggtcc tgtggcgagc tgtgagcacc gccagcatta gacgtcacat ccaggtggcc 2700
ccacggcccc tacaggctgg ccctgcaatg gggccctgag ccctccctct tcatccccca 2760
aggcctcaac tagagggtgg tcccccgagg gcttggtgtc tactaccgaa gggcccaaga 2820
cctcctgggt cctctcaggc tccccccttcc ccaaggcagg gacaggccct gggggtgcca 2880
ccgtgggccc tgccacccag aagtctggct gaggtctggg caggggcagg gcaagcttga 2940
cctctcactg ttgacccttt ggcctctgta tttgtttcct attgccgtga caggtttcca 3000
caaacttcgt ggatcaaaac gaggtcttcc agttctgcgg gtcagaaggc tgacccgggg 3060
ctcaaatctg ggtgtcggca gtcctgcact ccttctggag gctctagggg agaattcatt 3120
tctggccttt tcatttttag aggctgaccg taattcttga cttcaggctc ctccatcttc 3180
agagccagct gtgggtagtt gaatcttttt cccgtcacct cattgaggcc tcccctctcc 3240

```
tgcctccctc caccactttt tttttttttt ttttgagaca gggtcttgct gtgttgccca    3300
ggctggagtg cagtggcctg gtcatggcat caaggctcac tgcagcctgg acctcctggt    3360
tcaagtgatc ctcttgtctc agtcccctga gacaatcccc cacgcccagc tacatatttt    3420
ttgtggatac agggtctcat tctgttgcct aggcttgtct ggaactcctg ggctcaaggg    3480
atcttgtagc cttagcctcc taaagtgctg ggattatagg catgagtcac tgtacccggc    3540
ctgctctacc gcttttaagg acgcttatga tcacattgcg cctacccaga gaacccaggt    3600
cgtctttcta ttttcaggtc agctgattag ccaccttagt tccatctgca actttagttc    3660
ccactggctg tgtaacctaa catagtcaca ggctctgggg actgtcacgt ggacatcttt    3720
gggaggccgt tattctgccc accgcaccct ccgttcatcc cctgccctgc cgggcacctc    3780
gctctacccc aggaaaatgt gagctcgttt tcctgctcgg catgtgctcc ccctaaggct    3840
ctgctcctcc ctgggcctga aagttccttc tcagcctgag agggggccct tcggactcag    3900
gcatgactca gcccggctga tgcctctgca gtgctgagtc aggatttggg gccggctctc    3960
ttgggtccgt ccccttttcc caggtactgc cttacaaagc tgtggccagg aagtggccgg    4020
tataaaggat gcccaaggtc tttgtacgtg tgtaggagtt agcgtgtttg atattgttaa    4080
tataataata attatttttt agagtactgc ttttgtatgt atgttgaaca ggatccaggt    4140
ttttatagct tgatataaaa cagaattcaa aagtgaaaaa                          4180
```

<210> 15
<211> 2524
<212> DNA
<213> NM_000249.2| Homo sapiens mutL homolog 1, colon cancer, nonpolyposis type 2 (E. coli) (MLH1), mRNA

<400> 15

```
attggctgaa ggcacttccg ttgagcatct agacgtttcc ttggctcttc tggcgccaaa      60
atgtcgttcg tggcaggggt tattcggcgg ctggacgaga cagtggtgaa ccgcatcgcg     120
gcgggggaag ttatccagcg gccagctaat gctatcaaag agatgattga gaactgttta     180
gatgcaaaat ccacaagtat tcaagtgatt gttaaagagg gaggcctgaa gttgattcag     240
atccaagaca atggcaccgg gatcaggaaa gaagatctgg atattgtatg tgaaaggttc     300
actactagta aactgcagtc ctttgaggat ttagccagta tttctaccta tggctttcga     360
ggtgaggctt tggccagcat aagccatgtg gctcatgtta ctattacaac gaaaacagct     420
gatggaaagt gtgcatacag agcaagttac tcagatggaa aactgaaagc ccctcctaaa     480
ccatgtgctg gcaatcaagg gacccagatc acggtggagg accttttta caacatagcc      540
acgaggagaa aagctttaaa aaatccaagt gaagaatatg ggaaaatttt ggaagttgtt     600
ggcaggtatt cagtacacaa tgcaggcatt agtttctcag ttaaaaaaca aggagagaca     660
```

gtagctgatg ttaggacact acccaatgcc tcaaccgtgg acaatattcg ctccatcttt 720
ggaaatgctg ttagtcgaga actgatagaa attggatgtg aggataaaac cctagccttc 780
aaaatgaatg gttacatatc caatgcaaac tactcagtga agaagtgcat cttcttactc 840
ttcatcaacc atcgtctggt agaatcaact tccttgagaa aagccataga aacagtgtat 900
gcagcctatt tgcccaaaaa cacacaccca ttcctgtacc tcagtttaga aatcagtccc 960
cagaatgtgg atgttaatgt gcaccccaca aagcatgaag ttcacttcct gcacgaggag 1020
agcatcctgg agcgggtgca gcagcacatc gagagcaagc tcctgggctc caattcctcc 1080
aggatgtact tcacccagac tttgctacca ggacttgctg gcccctctgg ggagatggtt 1140
aaatccacaa caagtctgac ctcgtcttct acttctggaa gtagtgataa ggtctatgcc 1200
caccagatgg ttcgtacaga ttcccgggaa cagaagcttg atgcatttct gcagcctctg 1260
agcaaacccc tgtccagtca gccccaggcc attgtcacag aggataagac agatatttct 1320
agtggcaggg ctaggcagca agatgaggag atgcttgaac tcccagcccc tgctgaagtg 1380
gctgccaaaa atcagagctt ggagggggat acaacaaagg ggacttcaga aatgtcagag 1440
aagagaggac ctacttccag caaccccaga aagagacatc gggaagattc tgatgtggaa 1500
atggtggaag atgattcccg aaaggaaatg actgcagctt gtaccccccg gagaaggatc 1560
attaacctca ctagtgtttt gagtctccag gaagaaatta atgagcaggg acatgaggtt 1620
ctccgggaga tgttgcataa ccactccttc gtgggctgtg tgaatcctca gtgggccttg 1680
gcacagcatc aaaccaagtt ataccttctc aacaccacca agcttagtga agaactgttc 1740
taccagatac tcatttatga ttttgccaat tttggtgttc tcaggttatc ggagccagca 1800
ccgctctttg accttgccat gcttgcctta gatagtccag agagtggctg gacagaggaa 1860
gatggtccca aagaaggact tgctgaatac attgttgagt ttctgaagaa gaaggctgag 1920
atgcttgcag actatttctc tttggaaatt gatgaggaag ggaacctgat tggattaccc 1980
cttctgattg acaactatgt gccccctttg gagggactgc ctatcttcat tcttcgacta 2040
gccactgagg tgaattggga cgaagaaaag gaatgttttg aaagcctcag taaagaatgc 2100
gctatgttct attccatccg gaagcagtac atatctgagg agtcgaccct ctcaggccag 2160
cagagtgaag tgcctggctc cattccaaac tcctggaagt ggactgtgga acacattgtc 2220
tataaagcct tgcgctcaca cattctgcct cctaaacatt tcacagaaga tggaaatatc 2280
ctgcagcttg ctaacctgcc tgatctatac aaagtctttg agaggtgtta aatatggtta 2340
tttatgcact gtgggatgtg ttcttctttc tctgtattcc gatacaaagt gttgtatcaa 2400
agtgtgatat acaaagtgta ccaacataag tgttggtagc acttaagact tatacttgcc 2460
ttctgatagt attcctttat acacagtgga ttgattataa ataaatagat gtgtcttaac 2520
ataa 2524

<210> 16
<211> 1536
<212> DNA

<213> NM_001071.1| Homo sapiens thymidylate synthetase (TYMS), mRNA

<400> 16

```
gggggggggg ggaccacttg gcctgcctcc gtcccgccgc gccacttggc ctgcctccgt      60
cccgccgcgc cacttcgcct gcctccgtcc cccgcccgcc gcgccatgcc tgtggccggc     120
tcggagctgc cgcgccggcc cttgcccccc gccgcacagg agcgggacgc cgagccgcgt     180
ccgccgcacg gggagctgca gtacctgggg cagatccaac acatcctccg ctgcggcgtc     240
aggaaggacg accgcacggg caccggcacc ctgtcggtat tcggcatgca ggcgcgctac     300
agcctgagag atgaattccc tctgctgaca accaaacgtg tgttctggaa gggtgttttg     360
gaggagttgc tgtggtttat caagggatcc acaaatgcta aagagctgtc ttccaaggga     420
gtgaaaatct gggatgccaa tggatcccga gactttttgg acagcctggg attctccacc     480
agagaagaag gggacttggg cccagtttat ggcttccagt ggaggcattt tggggcagaa     540
tacagagata tggaatcaga ttattcagga cagggagttg accaactgca aagagtgatt     600
gacaccatca aaaccaaccc tgacgacaga agaatcatca tgtgcgcttg gaatccaaga     660
gatcttcctc tgatggcgct gcctccatgc catgccctct gccagttcta tgtggtgaac     720
agtgagctgt cctgccagct gtaccagaga tcgggagaca tgggcctcgg tgtgcctttc     780
aacatcgcca gctacgccct gctcacgtac atgattgcgc acatcacggg cctgaagcca     840
ggtgacttta tacacacttt gggagatgca catatttacc tgaatcacat cgagccactg     900
aaaattcagc ttcagcgaga acccagacct ttcccaaagc tcaggattct tcgaaaagtt     960
gagaaaattg atgacttcaa agctgaagac tttcagattg aagggtacaa tccgcatcca    1020
actattaaaa tggaaatggc tgtttagggt gctttcaaag gagcttgaag gatattgtca    1080
gtctttaggg gttgggctgg atgccgaggt aaaagttctt tttgctctaa aagaaaaagg    1140
aactaggtca aaaatctgtc cgtgacctat cagttattaa tttttaagga tgttgccact    1200
ggcaaatgta actgtgccag ttctttccat aataaaaggc tttgagttaa ctcactgagg    1260
gtatctgaca atgctgaggt tatgaacaaa gtgaggagaa tgaaatgtat gtgctcttag    1320
caaaaacatg tatgtgcatt tcaatcccac gtacttataa agaaggttgg tgaatttcac    1380
aagctatttt tggaatattt ttagaatatt ttaagaattt cacaagctat tccctcaaat    1440
ctgagggagc tgagtaacac catcgatcat gatgtagagt gtggttatga actttatagt    1500
tgttttatat gttgctataa taaagaagtg ttctgc                              1536
```

<210> 17
<211> 2986
<212> DNA
<213> NM_000201.1| Homo sapiens intercellular adhesion molecule 1 (CD54), human rhinovirus receptor (ICAM1), mRNA

<400> 17

```
gcgccccagt cgacgctgag ctcctctgct actcagagtt gcaacctcag cctcgctatg     60
gctcccagca gcccccggcc cgcgctgccc gcactcctgg tcctgctcgg ggctctgttc    120
ccaggacctg gcaatgccca gacatctgtg tccccctcaa aagtcatcct gccccgggga    180
ggctccgtgc tggtgacatg cagcacctcc tgtgaccagc ccaagttgtt gggcatagag    240
accccgttgc ctaaaaagga gttgctcctg cctgggaaca accggaaggt gtatgaactg    300
agcaatgtgc aagaagatag ccaaccaatg tgctattcaa actgccctga tgggcagtca    360
acagctaaaa ccttcctcac cgtgtactgg actccagaac gggtggaact ggcacccctc    420
ccctcttggc agccagtggg caagaacctt accctacgct gccaggtgga gggtggggca    480
ccccgggcca acctcaccgt ggtgctgctc cgtggggaga aggagctgaa acgggagcca    540
gctgtggggg agcccgctga ggtcacgacc acggtgctgg tgaggagaga tcaccatgga    600
gccaatttct cgtgccgcac tgaactggac ctgcggcccc aagggctgga gctgtttgag    660
aacacctcgg cccctacca gctccagacc tttgtcctgc cagcgactcc cccacaactt    720
gtcagccccc gggtcctaga ggtggacacg caggggaccg tggtctgttc cctggacggg    780
ctgttcccag tctcggaggc ccaggtccac ctggcactgg gggaccagag gttgaacccc    840
acagtcacct atggcaacga ctccttctcg gccaaggcct cagtcagtgt gaccgcagag    900
gacgagggca cccagcggct gacgtgtgca gtaatactgg ggaaccagag ccaggagaca    960
ctgcagacag tgaccatcta cagctttccg gcgcccaacg tgattctgac gaagccagag   1020
gtctcagaag ggaccgaggt gacagtgaag tgtgaggccc accctagagc caaggtgacg   1080
ctgaatgggg ttccagccca gccactgggc ccgagggccc agctcctgct gaaggccacc   1140
ccagaggaca acgggcgcag cttctcctgc tctgcaaccc tggaggtggc cggccagctt   1200
atacacaaga accagacccg ggagcttcgt gtcctgtatg gcccccgact ggacgagagg   1260
gattgtccgg gaaactggac gtggccagaa aattcccagc agactccaat gtgccaggct   1320
tgggggaacc cattgcccga gctcaagtgt ctaaaggatg gcactttccc actgcccatc   1380
ggggaatcag tgactgtcac tcgagatctt gagggcacct acctctgtcg ggccaggagc   1440
actcaagggg aggtcacccg cgaggtgacc gtgaatgtgc tctccccccg gtatgagatt   1500
gtcatcatca ctgtggtagc agccgcagtc ataatgggca ctgcaggcct cagcacgtac   1560
ctctataacc gccagcggaa gatcaagaaa tacagactac aacaggccca aaaagggacc   1620
cccatgaaac cgaacacaca agccacgcct ccctgaacct atcccgggac agggcctctt   1680
cctcggcctt cccatattgg tggcagtggt gccacactga acagagtgga agacatatgc   1740
```

```
catgcagcta cacctaccgg ccctgggacg ccggaggaca gggcattgtc ctcagtcaga    1800
tacaacagca tttggggcca tggtacctgc acacctaaaa cactaggcca cgcatctgat    1860
ctgtagtcac atgactaagc caagaggaag gagcaagact caagacatga ttgatggatg    1920
ttaaagtcta gcctgatgag aggggaagtg gtgggggaga catagcccca ccatgaggac    1980
atacaactgg gaaatactga aacttgctgc ctattgggta tgctgaggcc cacagactta    2040
cagaagaagt ggccctccat agacatgtgt agcatcaaaa cacaaaggcc cacacttcct    2100
gacggatgcc agcttgggca ctgctgtcta ctgaccccaa cccttgatga tatgtattta    2160
ttcatttgtt attttaccag ctatttattg agtgtctttt atgtaggcta aatgaacata    2220
ggtctctggc ctcacggagc tcccagtcca tgtcacattc aaggtcacca ggtacagttg    2280
tacaggttgt acactgcagg agagtgcctg gcaaaaagat caaatggggc tgggacttct    2340
cattggccaa cctgcctttc cccagaagga gtgatttttc tatcggcaca aaagcactat    2400
atggactggt aatggttcac aggttcagag attacccagt gaggccttat tcctcccttc    2460
cccccaaaac tgacaccttt gttagccacc tccccaccca catacatttc tgccagtgtt    2520
cacaatgaca ctcagcggtc atgtctggac atgagtgccc agggaatatg cccaagctat    2580
gccttgtcct cttgtcctgt ttgcatttca ctgggagctt gcactattgc agctccagtt    2640
tcctgcagtg atcagggtcc tgcaagcagt ggggaagggg gccaaggtat tggaggactc    2700
cctcccagct ttggaagggt catccgcgtg tgtgtgtgtg tgtatgtgta gacaagctct    2760
cgctctgtca cccaggctgg agtgcagtgg tgcaatcatg gttcactgca gtcttgacct    2820
tttgggctca agtgatcctc ccacctcagc ctcctgagta gctgggacca taggctcaca    2880
acaccacacc tggcaaattt gatttttttt tttttttca gagacggggt ctcgcaacat    2940
tgcccagact tcctttgtgt tagttaataa agctttctca actgcc                   2986
```

<210> 18
<211> 736
<212> DNA
<213> NM_004492.1| Homo sapiens general transcription factor IIA, 2 (12kD subunit) (GTF2A2), mRNA

<400> 18

```
cgagctggag aggtggtcgg agaagtagga acctcctgcc gggctcgtgg cggcttctgt      60
ccgctccgcg gagggaagcg ccttccccac aggacatcaa tgcaagcttg aataagaaaa     120
acaaattctt cctcctaagc catggcatat cagttataca gaaatactac tttgggaaac     180
agtcttcagg agagcctaga tgagctcata cagtctcaac agatcacccc ccaacttgcc     240
cttcaagttc tacttcagtt tgataaggct ataaatgcag cactggctca gagggtcagg     300
aacagagtca atttcagggg ctctctaaat acgtacagat tctgcgataa tgtgtggact     360
```

tttgtactga atgatgttga attcagagag gtgacagaac ttattaaagt ggataaagtg 420
aaaattgtag cctgtgatgg taaaaatact ggctccaata ctacagaatg aatagaaaaa 480
atatgacttt tttacaccat cttctgttat tcattgcttt tgaagagaag catagaagag 540
actttttatt tattctagaa ttgcagaaat gactacactg tgctatacca gagaattcca 600
gtagaaagaa acttgtaact ctgtagcctc ttacatcacc tttattatac agcatgaaaa 660
accataactt tttttaagg acaaaagttg ttgccttcct aagaaccttc tttaataaac 720
tcattttaaa actctg 736

<210> 19
<211> 6401
<212> DNA
<213> NM_004850.3| Homo sapiens Rho-associated, coiled-coil containing protein kinase 2 (ROCK2), mRNA

<400> 19

caaggcggcc ggcggcgacc atggcagcgg gccggcggcg gccgtagtgg cccaggcctg 60
ggcttcagcc tcccggggcc ccagagggcg gggcggtccg ggccgcggcg gtggcggcgc 120
cacttccctg ctcccgcccg aggactcctg cgggcactcg ctgaggacca gcggaccggc 180
ggcgcgaatc tgactgaggg gcggggacgc cgtctgttcc ccgccgctcc cggcagggcc 240
gggccgggct gggccgggct gggccgggcg ggcccctggg agcagccccc aggcggggga 300
ccgccttgga gacccgaagc cggagctaga ggcaggcggt gggcccgggt ggagtcccgg 360
ccggagctgg tggttcgggg gcggtgctag gccccgaggc tgcgggacct gagcgcgagg 420
agcctgagtg cgggtccagc ggtggcggca tgagccggcc cccgccgacg gggaaaatgc 480
ccggcgcccc cgagaccgcg ccgggggacg gggcaggcgc gagccgccag aggaagctgg 540
aggcgctgat ccgagaccct cgctccccca tcaacgtgga gagcttgctg gatggcttaa 600
attccttggt ccttgattta gattttcctg ctttgaggaa aaacaagaac atagataatt 660
tcttaaatag atatgagaaa attgtgaaaa aaatcagagg tctacagatg aaggcagaag 720
actatgatgt tgtaaaagtt attggaagag gtgcttttgg tgaagtgcag ttggttcgtc 780
acaaggcatc gcagaaggtt tatgctatga agcttcttag taagtttgaa atgataaaaa 840
gatcagattc tgcctttttt tgggaagaaa gagatattat ggcctttgcc aatagcccct 900
gggtggttca gctttttat gcctttcaag atgataggta tctgtacatg gtaatggagt 960
acatgcctgg tggagacctt gtaaacctta tgagtaatta tgatgtgcct gaaaaatggg 1020
ccaaatttta cactgctgaa gttgttcttg ctctggatgc aatacactcc atgggtttaa 1080
tacacagaga tgtgaagcct gacaacatgc tcttggataa acatggacat ctaaaattag 1140
cagattttgg cacgtgtatg aagatggatg aaacaggcat ggtacattgt gatacagcag 1200

ttggaacacc ggattatata tcacctgagg ttctgaaatc acaagggggt gatggtttct 1260
atgggcgaga atgtgattgg tggtctgtag gtgttttcct ttatgagatg ctagtggggg 1320
atactccatt ttatgcggat tcacttgtag gaacatatag caaaattatg gatcataaga 1380
attcactgtg tttccctgaa gatgcagaaa tttccaaaca tgcaaagaat ctcatctgtg 1440
ctttcttaac agatagggag gtacgacttg ggagaaatgg ggtggaagaa atcagacagc 1500
atcctttctt taagaatgat cagtggcatt gggataacat aagagaaacg gcagctcctg 1560
tagtacctga actcagcagt gacatagaca gcagcaattt cgatgacatt gaagatgaca 1620
aaggagatgt agaaaccttc ccaattccta aagcttttgt tggaaatcag ctgcctttca 1680
tcggatttac ctactataga gaaaatttat tattaagtga ctctccatct tgtagagaaa 1740
ctgattccat acaatcaagg aaaaatgaag aaagtcaaga gattcagaaa aaactgtata 1800
cattagaaga acatcttagc aatgagatgc aagccaaaga ggaactggaa cagaagtgca 1860
aatctgttaa tactcgccta gaaaaaacag caaaggagct agaagaggag attaccttac 1920
ggaaaagtgt ggaatcagca ttaagacagt tagaaagaga aaaggcgctt cttcagcaca 1980
aaaatgcaga atatcagagg aaagctgatc atgaagcaga caaaaaacga aatttggaaa 2040
atgatgttaa cagcttaaaa gatcaacttg aagatttgaa aaaaagaaat caaaactctc 2100
aaatatccac tgagaaagtg aatcaactcc agagacaact ggatgaaacc aatgctttac 2160
tgcgaacaga gtctgatact gcagcccggt taaggaaaac ccaggcagaa agttcaaaac 2220
agattcagca gctggaatct aacaatagag atctacaaga taaaaactgc ctgctggaga 2280
ctgccaagtt aaaacttgaa aaggaattta tcaatcttca gtcagctcta gaatctgaaa 2340
ggagggatcg aacccatgga tcagagataa ttaatgattt acaaggtaga atatgtggcc 2400
tagaagaaga tttaaagaac ggcaaaatct tactagcgaa agtagaactg gagaagagac 2460
aacttcagga gagatttact gatttggaaa aggaaaaaag caacatggaa atagatatga 2520
cataccaact aaaagttata cagcagagcc tagaacaaga agaagctgaa cataaggcca 2580
caaaggcacg actagcagat aaaaataaga tctatgagtc catcgaagaa gccaaatcag 2640
aagccatgaa agaaatggag aagaagctct tggaggaaag aactttaaaa cagaaagtgg 2700
agaacctatt gctagaagct gagaaaagat gttctctatt agactgtgac ctcaaacagt 2760
cacagcagaa aataaatgag ctccttaaac agaaagatgt gctaaatgag gatgttagaa 2820
acctgacatt aaaaatagag caagaaactc agaagcgctg ccttacacaa aatgacctga 2880
agatgcaaac acaacaggtt aacacactaa aaatgtcaga aaagcagtta aagcaagaaa 2940
ataaccatct catggaaatg aaaatgaact tggaaaaaca aaatgctgaa cttcgaaaag 3000
aacgtcagga tgcagatggg caaatgaaag agctccagga tcagctcgaa gcagaacagt 3060
atttctcaac cctttataaa acacaagtta gggagcttaa agaagaatgt gaagaaaaga 3120
ccaaacttgg taaagaattg cagcagaaga aacaggaatt acaggatgaa cgggactctt 3180

tggctgccca actggagatc accttgacca aagcagattc tgagcaactg gctcgttcaa 3240
ttgctgaaga acaatattct gatttggaaa aagagaagat catgaaagag ctggagatca 3300
aagagatgat ggctagacac aaacaggaac ttacggaaaa agatgctaca attgcttctc 3360
ttgaggaaac taataggaca ctaactagtg atgttgccaa tcttgcaaat gagaaagaag 3420
aattaaataa caaattgaaa gatgttcaag agcaactgtc aagattgaaa gatgaagaaa 3480
taagcgcagc agctattaaa gcacagtttg agaagcagct attaacagaa agaacactca 3540
aaactcaagc tgtgaataag ttggctgaga tcatgaatcg aaaagaacct gtcaagcgtg 3600
gtaatgacac agatgtgcgg agaaaagaga aggagaatag aaagctacat atggagctta 3660
aatctgaacg tgagaaattg acccagcaga tgatcaagta tcagaaagaa ctgaatgaaa 3720
tgcaggcaca aatagctgaa gagagccaga ttcgaattga actgcagatg acattggaca 3780
gtaaagacag tgacattgag cagctgcggt cacaactcca agccttgcat attggtctgg 3840
atagttccag tataggcagt ggaccagggg atgctgaggc agatgatggg tttccagaat 3900
caagattaga aggatggctt tcattgcctg tacgaaacaa cactaagaaa tttggatggg 3960
ttaaaaagta tgtgattgta agcagtaaga agattctttt ctatgacagt gaacaagata 4020
aagaacaatc caatccttac atggttttag atatagacaa gttatttcat gtccgaccag 4080
ttacacagac agatgtgtat agagcagatg ctaaagaaat tccaaggata ttccagattc 4140
tgtatgccaa tgaaggagaa agtaagaagg aacaagaatt tccagtggag ccagttggag 4200
aaaaatctaa ttatatttgc cacaagggac atgagtttat tcctactctt tatcatttcc 4260
caaccaactg tgaggcttgt atgaagcccc tgtggcacat gtttaagcct cctcctgctt 4320
tggagtgccg ccgttgccat attaagtgtc ataaagatca tatggacaaa aaggaggaga 4380
ttatagcacc ttgcaaagta tattatgata tttcaacggc aaagaatctg ttattactag 4440
caaattctac agaagagcag cagaagtggg ttagtcggtt ggtgaaaaag atacctaaaa 4500
agcccccagc tccagaccct tttgcccgat catctcctag aacttcaatg aagatacagc 4560
aaaaccagtc tattagacgg ccaagtcgac agcttgcccc aaacaaacct agctaactgc 4620
cttctatgaa agcagtcatt attcaaggtg atcgtattct tccagtgaaa acaagactga 4680
aatatgatgg cccaaaattt attaaaaagc tatattttcc tgagagactg atacatacac 4740
tcatacatat atgtgttccc cttttccctg taatataaat tacaaatctg ggctcctttg 4800
aagcaacagg ttgaaccaac aatgattggt tgatagacta aggatatatg caactcttcc 4860
agacttttcc ataaagctct ctcggcagtc gctcacacta caatgcacac aaggattgag 4920
aagagttaaa ggctaaagaa aacatctttt ctagcttcaa cagagaggtt tcaccagcac 4980
atttaccaga agaatctggg aatggattcc actacagtga tattgactgc atctttaaga 5040
agtgaccatt atactgtgta tatatatata aacacacaca catatatata tatatatata 5100
gtactctaat actgcaagaa ggtttttttaa acttcccact ttattttttta tacacattaa 5160
tcagatatca ttacttgctg cagttgcaac tatgcacttg tataaagcca taatgttgga 5220

gtttatatca ctcattcctg tgtacctgat ggaagttgca tgttcatgtt taagcagtta 5280
ctgtaacaag aagtttaaag ttaattatat cagtttccta atgcttcatg ataggcaact 5340
ttacccattt tgaatgcctt aatttaattt ttttcaaagt ctcagccctg tctgtattaa 5400
aaaacaaaaa aagcgtttac cagctcttag gatgtaaact agctttgtgg aagataaatc 5460
gtgcactatt tttacacata aatagttata tcaatgtcag cctattttga ttaacaaatg 5520
tttttaaagt attattggtt atagaaacaa taatggatgg tgttggaact aatatatcct 5580
tgatgtctgt ctattattca ttcaactctt tttacagacc tcagtattag tctgtgacta 5640
caaaatattt tatttgcttt aaatttgctg gctaccctag atgtgttttt attcctggta 5700
aagacatttg tgattacatt ttcacactta agattcaaaa tttttcccaa atataaagaa 5760
aactaagaca gactgtagat gcattttaaa tatttaaata tgatcctcag acatgcagct 5820
gtgtgtggca gtattttagt accgggttaa gaaaactggc aactgggaag aagtggcctc 5880
aaaggcactt aatttgattt ttattttta aatgctgtca aagttacagt ttacgcagga 5940
cattcttgcc gtattctcat gatcccagat aagtgtgtgt tttatactgc aacaatatgc 6000
agcaatggta agcgtaaagt tttttttttg tttttgtttt tttttatatt atgaagtctt 6060
ttaacagtct ctctttatat aaatacacag agtttggtat gatatttaaa tacatcatct 6120
ggccaggcat ggtggcttac gcctgtaatc ctagcacttt gggaggccaa gacgggcgga 6180
tcacctgagg tgaggagttc aagaccagcc tgcccaacat agtgaaactc cgtctctacc 6240
aatatacaaa aattagccgg gcatgatggt ggtggcctgt aatcccagct acttgggagg 6300
ctgagacagg agaatcgctt gaacccagga gacggtggtt gcagtgagcg aagatcgagc 6360
cactgcactc cagcctgggc agctgaacaa gactccgtct c 6401

<210> 20
<211> 1556
<212> DNA
<213> NM_005783.3| Homo sapiens thioredoxin domain containing 9 (TXNDC9), mRNA

<400> 20

ggcgtccaag gtgatatcgc gcgaggttcg cagccaataa ggaggcggat gtgacggccc 60
gtttgcagcc gccggcagct actgcaaggc aaaagccgga gtggacgtgt cttttgaaac 120
tgctgctctt tcacttctca ggcgtcaccg agagctcagc acccaggctg aactctgtac 180
catttggaag aatggaagct gatgcatctg ttgacatgtt ttccaaagtc ctggagcatc 240
agctgcttca gactaccaaa ctggtggaag aacatttgga ttctgaaatt caaaaactgg 300
atcagatgga tgaggatgaa ttggaacgcc ttaaagaaaa gagactccag gcactaagga 360
aagctcaaca gcagaaacaa gaatggcttt ctaaaggaca tggggaatac agagaaatcc 420

ctagtgaaag agactttttt caagaagtca aggagagtga aaatgtggtt tgccatttct 480
acagagactc cacattcagg tgtaaaatac tagacagaca tctggcaata ttgtccaaga 540
aacacctcga gaccaaattt ttgaagctga atgtggaaaa agcacctttc ctttgtgaga 600
gactgcatat caaagtcatt cccacactag cactgctaaa agatgggaaa acacaagatt 660
atgttgttgg gtttactgac ctaggaaata cagatgactt caccacagaa actttagaat 720
ggaggctcgg ttcttctgac attcttaatt acagtggaaa tttaatggag ccaccatttc 780
agaaccaaaa gaaatttgga acaaacttca caaagctgga aaagaaaact atccgaggaa 840
agaaatatga ttcagactct gatgatgatt agagctcaat aattctttgt aaattgtctt 900
tttttttctg cttcagattt aaatgtgttt ttaaaattct attaatgtct atacattggt 960
cacctaaata ctcatattct cgagttttat acagttgtat cacatcgaaa agtgtcttta 1020
ctgttttctg tgtggccatc atgtttaagt tgaggaaaac tcagttctta aattatctgg 1080
gaagggtctg gattctctat ttttgagatt gactttatca caatatgatt cttacatctt 1140
tataccattt acaattgtgt tttagatcta cagagttaga aattcgaaaa ctattccagg 1200
actaattctt aatcggcatt atttatacaa gaggtcaagt aacatttact agcgcaatac 1260
tgcacttgta aatgaattat aaacgctctt ctggaatata tttaaataac cattaaagaa 1320
ctgcttattc attctggaca ctgcatgttg atgttgaatc aactgatgcc agcagaaagc 1380
tattttgatt tgtgaacata ctgccttatt taaagggtcc tgattgcttg tattttaaga 1440
cattcattaa aaagaaacca ggaaacactt ttgaaataac agcataagga acttcactgt 1500
ctctgctcaa taaaatacct gtaactggaa aaaaaaaaaa aaaaaaaaaa aaaaaa 1556

<210> 21
<211> 1276
<212> DNA
<213> NM_003581.1| Homo sapiens NCK adaptor protein 2 (NCK2), mRNA

<400> 21

gtgccaaaga aggactccat gaaagatgac agaagaagtt attgtgatag ccaagtggga 60
ctacaccgcc cagcaggacc aggagctgga catcaagaag gtgaacgagc ggctgtggtt 120
gctggacgac tccaagacgt ggtggcgggt gaggaacgcg gccaacagga cgggctatgt 180
accgtccaac tacgtggagc ggaagaacag cctgaagaag ggctccctcg tgaagaacct 240
gaaggacaca ctaggcctcg gcaagacgcg caggaagacc agcgcgcggg atgcgtcccc 300
cacgcccagc acggacgccg agtaccccgc caatggcagc ggcgccgacc gcatctacga 360
cctcaacatc ccggccttcg tcaagttcgc ctatgtggcc gagcgggagg atgagttgtc 420
cctggtgaag ggtcgcgcg tcaccgtcat ggagaagtgc agcgacggtt ggtggcgggg 480
cagctacaac gggcagatcg gctggttccc ctccaactac gtcttggagg aggtggacga 540

```
ggcggctgcg gagtccccaa gcttcctgag cctgcgcaag ggcgcctcgc tgagcaatgg    600
ccagggctcc cgcgtgctgc atgtggtcca gacgctgtac cccttcagct cagtcaccga    660
ggaggagctc aacttcgaga agggggagac catggaggtg attgagaagc cggagaacga    720
ccccgagtgg tggaaatgca aaaatgcccg gggccaggtg ggcctcgtcc ccaaaaacta    780
cgtggtggtc ctcagtgacg ggcctgccct gcaccctgcg cacgccccac agataagcta    840
caccgggccc tcgtccagcg ggcgcttcgc gggcagagag tggtactacg ggaacgtgac    900
gcggcaccag gcgcagtgcg ccctcaacga gcggggcgtg gagggcgact tcctcattag    960
ggacagcgag tcctcgccca gcgacttctc cgtgtccctt aaagcgtcag ggaagaacaa   1020
acacttcaag gtgcagctcg tggacaatgt ctactgcatt gggcagcggc gcttccacac   1080
catggacgag ctggtggaac actacaaaaa ggcgcccatc ttcaccagcg agcacgggga   1140
gaagctctac ctcgtcaggg ccctgcagtg acggcgcccc ggccccacac tcgcctcccg   1200
ggccccacgg tggagctgcc cgcccggcct tgtggcagag gctcctcccg cggggacggc   1260
cccgacggct tctctg                                                   1276
```

<210> 22
<211> 1577
<212> DNA
<213> NM_006214.2| Homo sapiens phytanoyl-CoA hydroxylase (Refsum disease) (PHYH), mRNA

<400> 22

```
gcccgctgcg gtaaatgggg cagaggccgg gaggggtggg ggttccccgc gccgcagcca     60
tggagcagct tcgcgccgcc gcccgtctgc agattgttct gggccacctc ggccgcccct    120
cggccggggc tgtcgtagct catcccactt cagggactat ttcctctgcc agtttccatc    180
ctcaacaatt ccagtatact ctggataata atgttctaac cctggaacag agaaaatttt    240
atgaagaaaa tgggtttcta gtaatcaaaa atcttgtacc tgatgccgat attcaacgct    300
ttcggaatga gtttgaaaaa atctgcagaa aggaggtgaa accattagga ttaacagtaa    360
tgagagatgt gaccatttcg aaatccgaat atgctccaag tgagaagatg atcacgaagg    420
tccaggattt ccaggaagat aaggagctct tcagatactg cactctcccc gagattctga    480
aatatgtgga gtgcttcact ggacctaata ttatggccat gcacacaatg ttgataaaca    540
aacctccaga ttctggcaag aagacgtccc gtcacccccct gcaccaggac ctgcactatt    600
tccccttcag gcccagcgat ctcatcgttt gcgcctggac ggcgatggag cacatcagcc    660
ggaacaacgg ctgtctggtt gtgctcccag gcacacacaa gggctccctg aagccccacg    720
attaccccaa gtgggagggg ggagttaaca aaatgttcca cgggatccag gactacgagg    780
aaaacaaggc ccgggtgcac ctggtgatgg agaagggcga cactgtttc ttccatcctt    840
```

tgctcatcca cggatctggt cagaataaaa cccagggatt ccggaaggca atttcctgcc 900
atttcgccag tgccgattgc cactacattg acgtgaaggg caccagtcaa gaaaacatcg 960
agaaggaagt tgtaggaata gcacataaat tctttggagc tgaaaatagc gtgaacttga 1020
aggatatttg gatgtttcga gctcgacttg tgaaaggaga aagaaccaat ctttgaaata 1080
gccatctgct ataactcttt caacagaaaa ccaaaaccaa acgaaatgtc taaggaaaat 1140
gttttcttaa tgagatgatg taaccttttc tatcacttgt taaaagcaga aaacatgtat 1200
caggtactta attgcataga gttagttttg cagcacaatg gtgttgcttt aatggaaaaa 1260
aaaaacagta aaagtgaaat attactgttt taaggaaaac taatttaggg tggcagccaa 1320
taaaggtggt tggtgtctaa tttaagtgtt aaatcaattt ctttcattca gttagctctt 1380
tacccaagaa gaagtgaatg atttggagct tagggtatgt tttgtatccc ctttctgata 1440
aacccattcc ctaccaattt tatgtcataa gagatttttt tcccccaaat ctagaacaat 1500
gtataataca ttcacatcta gtcaagggca taggaacggt gtcatggagt ccaaataaag 1560
tggatattcc tgctcgg 1577

<210> 23
<211> 3060
<212> DNA
<213> NM_004739.2| Homo sapiens metastais-associated gene family, member 2 (MTA2), mRNA

<400> 23

tccggaagga ggcgaaccct gaggcgggcc cggcaagcct tccctgcggc cggcagagcc 60
caacgactag tgggactccg cgggggcggg ggtagctgga gcctggctct ggcctggcag 120
gagccgagct tgttccggaa gaagccgagc ggacgggggc cagcctcagc gtcccgggag 180
tgaggcgata gctgcggcgg cgacagcgcg ggccgggatg aaccgcgacg gctgaggcag 240
cggaggtgcc ggctgcgcgg gccccagtga gactccctcg aagcggcagc ccaccgttcg 300
gggctttgcc tcgagccgag ccctgccccc gcgagcctcc cggacccctt tgtgcggccg 360
gaggcggcgg cgggaacggc catggcggcc aacatgtacc gggtgggaga ttacgtctat 420
tttgagaact cttccagcaa tccttacctg gttagacgga ttgaggagct caacaagact 480
gcaaatggaa atgtggaggc aaaggttgtc tgtcttttcc ggcgcaggga catttctagt 540
agcctcaaca gcctggctga tagtaatgcc agggagtttg aagaggaatc aaagcagcca 600
ggggtgtctg agcagcagcg ccatcaactg aagcaccggg aactttttct ttctcggcaa 660
tttgaatcat taccagccac ccacatacgg gggaaatgca gtgtgaccct cttgaatgag 720
acagatatct tgagccagta cctggaaaag gaggactgct ttttttactc actggtgttt 780
gaccccgtgc agaagacact tctcgctgat cagggcgaga ttagagttgg ttgcaaatac 840

```
caagctgaga tcccagatcg cctagtagag ggagaatctg ataatcggaa ccagcagaag    900
atggagatga aggtctggga cccagacaac cctctcacag accggcagat cgaccagttt    960
cttgtggtgg cccgagctgt gggaaccttt gcaagagccc tagattgtag cagctccatt   1020
cggcagccaa gcttgcacat gagtgcagct gctgcctccc gagatatcac tctgtttcac   1080
gccatggata ccttgcaaag gaacggctac gacctggcta aggccatgtc gaccctggta   1140
ccccagggag gcccggtgct gtgtcgggat gagatggagg aatggtcagc ctcagaggcc   1200
atgctatttg aggaggccct agagaagtat gggaaggact tcaatgatat tcgccaggat   1260
tttctaccct ggaagtcact tgccagcata gtccagtttt attacatgtg gaaaaccaca   1320
gaccggtata ttcagcagaa aaggttgaaa gctgctgaag cagacagcaa actgaaacag   1380
gtctacattc ccacctacac taagccaaac cctaaccaga tcatttctgt gggttcaaaa   1440
cctggcatga atggggctgg atttcagaag ggcctgactt gtgagagttg ccacaccaca   1500
cagtctgctc agtggtatgc ctggggccca cctaacatgc agtgccgcct ctgtgcttcc   1560
tgttggatct actggaagaa gtatggggga ctgaagaccc caactcagct tgaggggggcc  1620
actcggggca ccacggagcc acactcaagg ggtcatttat ccagacctga agctcaaagt   1680
ctctctcctt acacaaccag cgccaacagg gccaagctac tggctaagaa cagacaaact   1740
ttcctgcttc agaccacaaa gctgacccgt cttgccagac gcatgtgcag ggacctatta   1800
cagccaagga gggccgcccg acggccttat gctcctatca atgccaatgc catcaaagca   1860
gagtgctcca ttcgacttcc taaggccgcc aagactccat tgaagattca ccctctggtg   1920
cggctgcccc tggcaactat cgtcaaagat ctggtggccc aggcacccct gaaaccaaaa   1980
acacctcggg gtaccaagac accgatcaac agaaaccagc tgtcccagaa ccggggactg   2040
gggggcatta tggtgaaacg ggcctatgag actatggcag gggcaggggt tcctttctct   2100
gccaatggaa ggcctctggc ttcagggatt cgttcaagct cacagccagc agccaagcgt   2160
cagaaactaa acccagctga tgcccccaat cctgtggtgt ttgtggccac aaaggatacc   2220
agggccctac ggaaggctct gacccatctg gaaatgcggc gagctgctcg ccgacccaac   2280
ttgcccctga aggtgaagcc aacgctgatt gcagtgcggc cccctgtccc tctacctgca   2340
ccctcacatc ctgccagcac caatgagcct attgtcctgg aggactgagc acctgtgggg   2400
aagggaggtg ggctgagagg tagagggtgg atgcccaggg cacccaaacc tcccttccct   2460
ttcgtgtcga agggagtgag gagtgaatta aggaagagag caagtgagtg tgtgtccctg   2520
gaggggttgg gcgccctctg gtgttaccac ctcgagactt gtctcatgcc tccatgcttg   2580
ccgatggagg acagactgca ggaacttggc ccatgtggga acctagcctg ttttgggggg   2640
taggacccac agatgtcttg gacagttttg gggggagggt tttttaattt tttaaaagtt   2700
ttgcctccct ttgtgaaagg ggatggggag gggaagagta aacagataac aggtggtggt   2760
acctggttgg gggagggggg cgtgcactgc catgtctttt tttttttttt tttttttttt   2820
```

tttcctaatt gggggtttct ctttctgtcc ggtgtccgga ctttcctaat tggagtttga 2880
ggcccctaag ctggcatcaa ccccaggcca cgctcgctct ttccttccct cccctccccc 2940
tctgcctttt gtacgccagt tctcagaaat aaagatcttt tgtccgtttt tttaacctcg 3000
gattctgtaa ttggttctta tagtaacaaa taaaaagctg ttttcttcag cttctcctgg 3060

<210> 24
<211> 2407
<212> DNA
<213> NM_001091.1| Homo sapiens amiloride binding protein 1 (amine oxidase (copper-containing)) (ABP1), mRNA

<400> 24

gatcagctta agggcaaagg ctggaagcag agcgaactgg gagcagagca cacagagccg 60
tggagcgaga gatgccggcc ctgggctggg ccgtggctgc catcctgatg ctgcagacgg 120
ccatggcgga gccctccccg ggactctgc ccaggaaggc aggggtgttt tcagacctaa 180
gcaaccaaga gctgaaggca gtgcacagct tcctctggtc caagaaggag ctgaggctgc 240
agccctccag taccaccacc atggccaaga acaccgtgtt tctcatcgag atgctgctgc 300
ccaagaagta ccatgtgctg aggtttctgg ataaaggtga aaggcatcct gtgcgggaag 360
cccgtgccgt catcttcttt ggtgaccagg agcatcccaa tgtcaccgag tttgctgtgg 420
ggcccctgcc agggccctgc tacatgcgag cactgtcccc caggcctggg taccagtcct 480
cctgggcatc gaggcccatc tccacagcag agtatgccct cctctaccac accctgcagg 540
aagccaccaa gcccctgcat cagttcttcc tcaataccac aggcttctca ttccaagact 600
gccatgacag atgcctggcc ttcaccgatg tggccccccg ggtgtggct tctggccagc 660
gccgcagttg gcttatcata cagcgctatg tagaaggcta ctttctgcac cccactgggc 720
tggagctcct cgtggatcat gggagcacag atgctgggca ctgggccgtg gagcaggtgt 780
ggtacaacgg gaagttctat gggagcccag aggaactggc tcggaagtat gcagatggag 840
aggtggacgt ggtggtcctg gaggacccgc tgcctggggg caaggggcat gacagcacag 900
aggagccgcc cctcttctcc tcccacaagc cccgcgggga cttccccagc cccatccatg 960
tgagcggccc ccgcttggtc cagccccacg gccctcgctt caggctggag ggcaacgctg 1020
tgctctacgg cggctggagc tttgccttcc ggctgcgctc ctcctccggg ctgcaggtcc 1080
tgaacgtgca cttcggcgga gagcgcattg cctatgaggt cagcgtgcaa gaggcagtgg 1140
cgctgtatgg aggacacaca cctgcaggca tgcagaccaa gtacctcgat gtcggctggg 1200
gcctgggcag cgtcactcat gagttagccc ccggcatcga ctgcccggag accgccacct 1260
tcctggacac tttccactac tatgatgccg atgacccggt ccattatccc cgagccctct 1320
gcctctttga aatgcccaca ggggtgcccc ttcggcggca ctttaattcc aactttaaag 1380

gtggcttcaa cttctatgcg gggctgaagg gccaggtgct ggtgctgcgg acaacttcaa 1440
ctgtctacaa ttatgattac atttgggact ttatcttcta ccccaacggg gtgatggagg 1500
ccaagatgca tgccactggc tacgtccacg ccaccttcta cacccccgag gggctgcgcc 1560
acggcactcg cctgcacacc cacctgattg gcaacataca cactcacttg gtgcactacc 1620
gcgtagacct ggatgtggca ggcaccaaga acagcttcca gacactgcag atgaagctag 1680
aaaacatcac caacccctgg agcccaagac accgcgtggt ccagccaact ctggagcaga 1740
cgcagtactc ctgggagcgc caggcggcct tccgcttcaa aaggaagctg cccaagtacc 1800
tgctctttac cagcccccag gagaacccct ggggccacaa gcgcagctac cgcctgcaga 1860
tccactccat ggccgaccag gtgctgcccc caggctggca ggaggagcag gccatcacct 1920
gggcaaggta ccccctggca gtgaccaagt accgggagtc ggagctgtgc agcagcagca 1980
tctaccacca gaacgacccc tggcacccgc ccgtggtctt tgagcagttt cttcacaaca 2040
acgagaacat tgaaaatgag gacctggtgg cctgggtgac ggtgggcttc ctgcacatcc 2100
cccactcaga ggacattccc aacacagcca cacctgggaa ctccgtgggc ttcctgctcc 2160
ggccattcaa cttcttccca gaggacccct ccctggcatc cagagacact gtgatcgtgt 2220
ggcctcggga caacggcccc aactacgtcc agcgctggat ccctgaggac agggactgct 2280
cgatgcctcc cccttttagc tacaatggga cctatagacc tgtgtgacca gcccccagtt 2340
cctcccccag ttcctcccag gaagcccagg agcctcactg gggcagacaa taaactctca 2400
gagcctc 2407

<210> 25
<211> 1094
<212> DNA
<213> NM_000712.3| Homo sapiens biliverdin reductase A (BLVRA), mRNA

<400> 25

ccgccccggt ccgcaaagcc ggtggcgccc ggaggctgca cggagagcgg tgcccgcgtc 60
agtgaccgaa ggaagagacc aagatgaatg cagagcccga gaggaagttt ggcgtggtgg 120
tggttggtgt tggccgagcc ggctccgtgc ggatgaggga cttgcggaat ccacaccctt 180
cctcagcgtt cctgaacctg attggcttcg tgtcgagaag ggagctcggg agcattgatg 240
gagtccagca gatttctttg gaggatgctc tttccagcca agaggtggag gtcgcctata 300
tctgcagtga gagctccagc catgaggact acatcaggca gttccttaat gctggcaagc 360
acgtccttgt ggaatacccc atgacactgt cattggcggc cgctcaggaa ctgtgggagc 420
tggctgagca gaaaggaaaa gtcttgcacg aggagcatgt tgaactcttg atggaggaat 480
tcgctttcct gaaaaaagaa gtggtgggga aagacctgct gaaagggtcg ctcctcttca 540
cagctggccc gttggaagaa gagcggtttg gcttccctgc attcagcggc atctctcgcc 600

```
tgacctggct ggtctccctc tttggggagc tttctcttgt gtctgccact ttggaagagc   660
gaaaggaaga tcagtatatg aaaatgacag tgtgtctgga gacagagaag aaaagtccac   720
tgtcatggat tgaagaaaaa ggacctggtc taaaacgaaa cagatattta agcttccatt   780
tcaagtctgg gtccttggag aatgtgccaa atgtaggagt gaataagaac atatttctga   840
aagatcaaaa tatatttgtc cagaaactct tgggccagtt ctctgagaag gaactggctg   900
ctgaaaagaa acgcatcctg cactgcctgg ggcttgcaga agaaatccag aaatattgct   960
gttcaaggaa gtaagaggag gaggtgatgt agcacttcca agatggcacc agcatttggt  1020
tcttctcaag agttgaccat tatctctatt cttaaaatta aacatgttgg ggaaacaaga  1080
aaaaaaaaaa aaaa                                                    1094
```

<210> 26
<211> 5546
<212> DNA
<213> NM_000933.2| Homo sapiens phospholipase C, beta 4 (PLCB4), transcript variant 1, mRNA

<400> 26

```
aggaaaagac aatttctctc tgattcagaa tcctgaaaat gtgatctccc ttaaaaagag     60
gacagtgctg ctgtgagttt gacgaagtgg acatcacctg cagtcagtcc agagctgccc    120
agtcttgaat ataatcatgg ccaaacctta tgaatttaac tggcagaagg aagttccctc    180
ctttttgcaa gaaggaacag tttttgacag atacgaggag gaatcctttg tgtttgaacc    240
caactgcctc ttcaaagtgg atgagtttgg cttctttctg acatggagaa gtgaaggcaa    300
ggaaggacag gtgctagaat gctccctcat caacagtatt cggtcgggag ccataccaaa    360
ggatcccaaa atcttggctg ctcttgaagc tgttggaaaa tcagaaaatg atctggaagg    420
gcggatagtt tgtgtctgca gtggcacaga tctagtgaac attagtttta cctacatggt    480
ggctgaaaat ccagaagtaa ctaagcaatg ggtagaaggc ctgagatcaa tcatacacaa    540
cttcagggcc aacaacgtca gtccaatgac atgcctcaag aaacactgga tgaaattggc    600
atttatgacc aacacaaatg gtaaaattcc agttaggagt attactagaa catttgcatc    660
gggaaaaaca gaaaaggtga tctttcaagc actcaaggag ttaggtcttc ccagtggaaa    720
gaatgatgaa attgagccca cagcattttc ttatgaaaag ttctatgaac tgacacaaaa    780
gatttgtcct cggacagata tagaagatct tttcaaaaaa atcaatggag acaaaactga    840
ttatttaacg gtagaccaat tagtgagctt tctaaatgaa catcaacgag atcctcgatt    900
gaatgaaatt ttatttccat tttatgatgc caaaagggca atgcagatca ttgagatgta    960
tgaacctgat gaagatttga agaaaaaagg ccttatatca agtgatgggt tttgcagata   1020
tctgatgtca gatgaaaacg ccccagtctt cctagatcgt ttagaacttt accaagaaat   1080
```

ggaccatcct ctggctcact acttcatcag ttcttcccat aacacttatc tcactggcag 1140
acagttcggc gggaagtctt cggtagaaat gtacagacag gttctcctgg ctggttgcag 1200
atgtgttgaa cttgactgct gggatggaaa aggtgaagac caagaaccaa taataactca 1260
tggaaaagca atgtgtacag atatcctttt taaggatgta attcaagcca tcaaggaaac 1320
tgcatttgtc acatcagaat atcctgtaat tctctccttt gaaaatcact gcagcaaata 1380
tcaacagtac aagatgtcca aatattgcga agatctattt ggggatctcc tgttgaaaca 1440
agcacttgaa tcacatccac ttgaaccagg cagggctttg ccatccccca atgacctcaa 1500
aagaaaaata ctcataaaaa acaagcggct gaaacctgaa gttgaaaaaa aacagctgga 1560
agctttgaga agcatgatgg aagctggaga atctgcctcc ccagcaaaca tcttagagga 1620
cgataatgaa gaggagatcg aaagtgctga ccaagaggag gaagctcacc ccgaattcaa 1680
atttggaaat gaactttctg ctgatgactt gggtcacaag gaagctgttg caaatagcgt 1740
caagaagggc ctggtcactg tagaagatga gcaggcgtgg atggcatctt ataaatatgt 1800
aggtgctacc actaatatcc atccatattt gtccacaatg atcaactacg cccagcctgt 1860
aaagtttcaa ggtttccatg tggcagaaga acgcaatatt cattataaca tgtcttcttt 1920
taatgaatca gtcggtcttg gctacttgaa gacacatgca attgaatttg tcaattataa 1980
caaacggcaa atgagtcgca tttaccccaa gggaggccga gtcgattcca gtaattacat 2040
gcctcagatt ttctggaacg ctggctgcca gatggtttca ctgaactatc aaaccccaga 2100
tttagcgatg caattgaatc agggaaaatt tgagtataat ggatcgtgcg ggtaccttct 2160
caaaccagat ttcatgaggc ggcctgatcg aacatttgac cccttctctg aaactcctgt 2220
tgatggtgtt attgcagcca cttgctcagt gcaggttata tcaggtcaat tcttatcaga 2280
taagaaaatt ggcacctacg tagaggtgga tatgtatggg ttgcccactg acaccatacg 2340
taaggaattc cgaactcgca tggttatgaa taatggactc aatccagttt acaatgaaga 2400
gtcatttgta tttcggaagg tgatcctgcc ggacctggct gtcttgagaa tagctgtgta 2460
tgatgataac aacaagctga ttggccagag gatcctcccg cttgatggcc tccaagccgg 2520
atatcgacac atttcccttc gaaatgaggg aaataaacca ttatcactac caacaatttt 2580
ctgcaatatt gttcttaaaa catatgtgcc tgatggattt ggagatatcg tggatgcttt 2640
atcagatcca aagaaatttc tctcaattac agaaaagaga gcagaccaaa tgagagctat 2700
gggcattgaa actagtgaca tagccgacgt gcccagtgac acttccaaaa atgacaagaa 2760
aggaaaggcc aacaccgcca aagcaaatgt gacccctcag agtagctctg agctcagacc 2820
aaccaccacg gctgccctgg cctctggtgt ggaagccaag aaaggtattg aacttatccc 2880
tcaagtaagg atagaagact taaagcagat gaaggcttac ttgaagcatt taaagaaaca 2940
gcagaaggag ctaaattctt taaagaagaa acatgcaaag gaacacagta ccatgcagaa 3000
gttacactgc acgcaagttg acaaaattgt ggcacagtat gacaaagaga agtcgactca 3060

tgagaaaatc ctagagaagg caatgaagaa gaagggggga agtaattgtc tcgaaatgaa 3120
aaaagaaaca gaaatcaaaa ttcagacgct gacatcagat cacaaatcta aggtcaaaga 3180
gattgtagca cagcacacaa aggaatggtc agaaatgatc aatacccaca gtgctgagga 3240
gcaagaaatc cgagacctgc acctcagcca gcagtgtgag ctgctgaaaa agctactcat 3300
caatgcccac gagcagcaaa cccagcagct gaaactgtcc catgacaggg aaagcaagga 3360
aatgcgagca caccaggcta agatttctat ggaaaatagc aaagccatca gccaagataa 3420
atctatcaag aataaagcag aacgggaaag gcgagtcagg gagttaaaca gcagcaacac 3480
taaaaagttt ctggaagaaa gaaagagact tgccatgaag cagtccaaag aaatggatca 3540
gttgaaaaaa gtccagcttg aacatctaga attcctagag aaacagaatg agcagctttt 3600
gaaatcctgt catgcagtgt cccaaacgca aggcgaagga gatgcagcag atggtgaaat 3660
tggaagccga gatggaccgc agaccagcaa cagtagtatg aaactccaaa atgcaaactg 3720
aagcagcaaa cccacaaagc atcaaaagac tcactcacaa acttctgaac acaaactcca 3780
tggatgaaag ctgtttattt tgtttccttt atgtgtaaac aagatgatat ctgaaaccag 3840
agagacttgg aatgtctgac tgacttctat ttaacagctt gagtattgca tttccttggc 3900
caaacaaaaa tagctacaaa tccacaaaaa tttactattc cagtaaggca gagtccaacc 3960
attgataata caacttaaac atgtttgcta taaaatacca tcacaagtaa atgagcttgg 4020
tgtgaacaac tctcctttgt gatgccttag gacatgtttg aactgcagca aaaaacaaaa 4080
acaaaaaaca gtgcattagc aatttcatag caagtgcatg cactaggaaa agaaaactct 4140
gtctacaagt ttattagcag aagtggtggt ctgctagaca aataattttg caaaattttt 4200
ctacatctaa gttacctcat cagtaagtgc catgtctcta ccatgccatc agaggctaat 4260
ttcctgtaaa agttgtggaa attgttagaa caatagaaaa atagagcagt gtatgtgtgc 4320
caaaactcat cattactcaa aggagaactg tgttaggcac atttaagaaa gtttacatct 4380
gacattgctt tataggaatt gtttctgcag attccggata ttataattca caccataaag 4440
attgtgaagt ggttattggc aaacgtttgt aaatgtgacc atgtataaag tatttatact 4500
ctttaattca cactgttaga gagcaaaatc atctaagtat tgccacatga caagattagt 4560
aaacaggaat actagaacta tgtttgcatg atacacaagc accaataaag actaatccat 4620
acacagttaa cctaatgcca aataaatact ggttaaataa atgtatggca cagaatataa 4680
tttgactatc aagactttta gcataatgaa aaaccctctc tctatatata tatgtgtata 4740
tgaattatgt gggcattctt gatacttcaa gttctagttt gaaaaaaata cataactaat 4800
ttaattttac acaaaaatat ttatgcagat tttcagaatt tcatatcagg aaatgacctt 4860
tttatgtctg ttaaatatca aaacaatttg ctacagtgtt aatctgcatg gtctttaagc 4920
ctgctgtagt tgagttgcag acagtgcatg aaaaagtatt ccgctgggaa ttgagccatg 4980
ccaccaaagc caagaggagc gcatggaaac ccggtagtct agaactaatc agattactga 5040
ttttagggca cagcaccaga tgaattgttg tatatgcttg taaaaattga ttctgtgtgt 5100

tcctctgaac aaagcggaga aaatgatgat accatcaata ttgaaattaa acttccaact 5160
tctctaataa aaaattaaaa cacgcataac actcgtcaag agtatttgct cccaagacac 5220
attctagcaa atgttttgcc tttttcatat acatgatatc atcgttattt tcaaaggggg 5280
cttattaata ccctcagcat gtttttcacc caaatgatgc aaaacatgca gattctagtt 5340
gacttcagtt gtaatagact tgtttttctc ctatttatga tttgaagtgg attctgtaaa 5400
atatctcttg ttcttagttt ccttatctgt aaaacagtgg agttagacta catatctttt 5460
ggcactaaca tctcatgaaa aattatggtt aataaaatat caccacattt ggattgccaa 5520
ttttcaaaaa aaaaaaaaaa aaaaaa 5546

<210> 27
<211> 2545
<212> DNA
<213> NM_002416.1| Homo sapiens chemokine (C-X-C motif) ligand 9 (CXCL9), mRNA

<400> 27

atccaataca ggagtgactt ggaactccat tctatcacta tgaagaaaag tggtgttctt 60
ttcctcttgg gcatcatctt gctggttctg attggagtgc aaggaacccc agtagtgaga 120
aagggtcgct gttcctgcat cagcaccaac caagggacta tccacctaca atccttgaaa 180
gaccttaaac aatttgcccc aagcccttcc tgcgagaaaa ttgaaatcat tgctacactg 240
aagaatggag ttcaaacatg tctaaaccca gattcagcag atgtgaagga actgattaaa 300
aagtgggaga aacaggtcag ccaaaagaaa aagcaaaaga atgggaaaaa acatcaaaaa 360
aagaaagttc tgaaagttcg aaaatctcaa cgttctcgtc aaaagaagac tacataagag 420
accacttcac caataagtat tctgtgttaa aaatgttcta ttttaattat accgctatca 480
ttccaaagga ggatggcata taatacaaag gcttattaat ttgactagaa aatttaaaac 540
attactctga aattgtaact aaagttagaa agttgatttt aagaatccaa acgttaagaa 600
ttgttaaagg ctatgattgt ctttgttctt ctaccaccca ccagttgaat ttcatcatgc 660
ttaaggccat gattttagca atacccatgt ctacacagat gttcacccaa ccacatccca 720
ctcacaacag ctgcctggaa gagcagccct aggcttccac gtactgcagc ctccagagag 780
tatctgaggc acatgtcagc aagtcctaag cctgttagca tgctggtgag ccaagcagtt 840
tgaaattgag ctggacctca ccaagctgct gtggccatca acctctgtat ttgaatcagc 900
ctacaggcct cacacacaat gtgtctgaga gattcatgct gattgttatt gggtatcacc 960
actggagatc accagtgtgt ggctttcaga gcctcctttc tggctttgga agccatgtga 1020
ttccatcttg cccgctcagg ctgaccactt tatttctttt tgttcccctt tgcttcattc 1080
aagtcagctc ttctccatcc taccacaatg cagtgccttt cttctctcca gtgcacctgt 1140

```
catatgctct gatttatctg agtcaactcc tttctcatct tgtccccaac accccacaga    1200
agtgctttct tctcccaatt catcctcact cagtccagct tagttcaagt cctgcctctt    1260
aaataaacct ttttggacac acaaattatc ttaaaactcc tgtttcactt ggttcagtac    1320
cacatgggtg aacactcaat ggttaactaa ttcttgggtg tttatcctat ctctccaacc    1380
agattgtcag ctccttgagg gcaagagcca cagtatattt ccctgtttct tccacagtgc    1440
ctaataatac tgtggaacta ggttttaata attttttaat tgatgttgtt atgggcagga    1500
tggcaaccag accattgtct cagagcaggt gctggctctt tcctggctac tccatgttgg    1560
ctagcctctg gtaacctctt acttattatc ttcaggacac tcactacagg gaccagggat    1620
gatgcaacat ccttgtcttt ttatgacagg atgtttgctc agcttctcca acaataagaa    1680
gcacgtggta aaacacttgc ggatattctg gactgttttt aaaaaatata cagtttaccg    1740
aaaatcatat aatcttacaa tgaaaaggac tttatagatc agccagtgac caaccttttc    1800
ccaaccatac aaaaattcct tttcccgaag gaaaagggct ttctcaataa gcctcagctt    1860
tctaagatct aacaagatag ccaccgagat ccttatcgaa actcatttta ggcaaatatg    1920
agttttattg tccgtttact tgtttcagag tttgtattgt gattatcaat taccacacca    1980
tctcccatga agaaagggaa cggtgaagta ctaagcgcta gaggaagcag ccaagtcggt    2040
tagtggaagc atgattggtg cccagttagc ctctgcagga tgtggaaacc tccttccagg    2100
ggaggttcag tgaattgtgt aggagaggtt gtctgtggcc agaatttaaa cctatactca    2160
ctttcccaaa ttgaatcact gctcacactg ctgatgattt agagtgctgt ccggtggaga    2220
tcccacccga acgtcttatc taatcatgaa actccctagt tccttcatgt aacttccctg    2280
aaaaatctaa gtgtttcata aatttgagag tctgtgaccc acttaccttg catctcacag    2340
gtagacagta tataactaac aaccaaagac tacatattgt cactgacaca cacgttataa    2400
tcatttatca tatatataca tacatgcata cactctcaaa gcaaataatt tttcacttca    2460
aaacagtatt gacttgtata ccttgtaatt tgaaatattt tctttgttaa aatagaatgg    2520
tatcaataaa tagaccatta atcag                                          2545
```

<210> 28
<211> 1144
<212> DNA
<213> NM_005859.2| Homo sapiens purine-rich element binding protein A (PURA), mRNA

<400> 28

```
cgactgaggc ggcgggcgga gcggcaggcg gcggcggcgc ggcagcggag cgcagcatca      60
tggcggaccg agacagcggc agcgagcagg gtggtgcggc gctgggttcg ggcggctccc     120
tggggcaccc cggctcgggc tcaggctccg gcgggggcgg tggtggcggc gggggcggcg     180
```

```
gcggcagtgg cggcggcggc ggcggggccc cagggggct gcagcacgag acgcaggagc 240
tggcctccaa gcgggtggac atccagaaca agcgcttcta cctggacgtg aagcagaacg 300
ccaagggccg cttcctgaag atcgccgagg tgggcgcggg cggcaacaag agccgcctta 360
ctctctccat gtcagtggcc gtggagttcc gcgactacct gggcgacttc atcgagcact 420
acgcgcagct gggccccagc cagccgccgg acctggccca ggcgcaggac gagccgcgcc 480
gggcgctcaa aagcgagttc ctggtgcgcg agaaccgcaa gtactacatg gatctcaagg 540
agaaccagcg cggccgcttc ctgcgcatcc gccagacggt caaccggggg cctggcctgg 600
gctccacgca gggccagacc attgcgctgc ccgcgcaggg gctcatcgag ttccgtgacg 660
ctctggccaa gctcatcgac gactacggag tggaggagga gccggccgag ctgcccgagg 720
gcacctcctt gactgtggac aacaagcgct tcttcttcga tgtgggctcc aacaagtacg 780
gcgtgtttat gcgagtgagc gaggtgaagc ccacctatcg caactccatc accgtgccct 840
acaaggtgtg ggccaagttc ggacacacct tctgcaagta ctcggaggag atgaagaaga 900
ttcaagagaa gcagagggag aagcgggctg cctgtgagca gcttcaccag cagcaacagc 960
agcagcagga ggagaccgcc gctgccaccc tgctactgca gggtgaggaa gaaggggaag 1020
aagattgatc aaactgaatg aaacccccac acacacacac atgcatacac acacacacac 1080
agccacacac acagaaaata tactgtaaag aaagagagaa aataaaaagt taaaaagtta 1140
aaaa                                                              1144
```

<210> 29
<211> 1575
<212> DNA
<213> NM_014298.3| Homo sapiens quinolinate phosphoribosyltransferase (nicotinate-nucleotide pyrophosphorylase (carboxylating)) (QPRT), RNA

<400> 29

```
tcccaccccc agcctggggc ctctgggagc cttggtcctg agcagccaac acaccagccc 60
agacagctgc aagtcaccat ggacgctgaa ggcctggcgc tgctgctgcc gcccgtcacc 120
ctggcagccc tggtggacag ctggctccga gaggactgcc cagggctcaa ctacgcagcc 180
ttggtcagcg gggcaggccc ctcgcaggcg gcgctgtggg ccaaatcccc tggggtactg 240
gcagggcagc ctttcttcga tgccatattt acccaactca actgccaagt ctcctggttc 300
ctccccgagg gatcgaagct ggtgccggtg gccagagtgg ccgaggtccg gggccctgcc 360
cactgcctgc tgctggggga acgggtggcc ctcaacacgc tggcccgctg cagtggcatt 420
gccagtgctg ccgccgctgc agtggaggcc gccagggggg ccggctggac tgggcacgtg 480
gcaggcacga ggaagaccac gccaggcttc cggctggtgg agaagtatgg gctcctggtg 540
ggcggggccg cctcgcaccg ctacgacctg ggagggctgg tgatggtgaa ggataaccat 600
```

gtggtggccg ccggtggcgt ggagaaggcg gtgcgggcgg ccagacaggc ggctgacttc 660
gctctgaagg tggaagtgga atgcagcagc ctgcaggagg ccgtgcaggc agctgaggct 720
ggtgccgacc ttgtcctgct ggacaacttc aagccagagg agctgcaccc cacggccacc 780
gtgctgaagg cccagttccc gagtgtggct gtggaagcca gtgggggcat caccctggac 840
aacctccccc agttctgcgg gccgcacata gacgtcatct ccatggggat gctgacccag 900
gcggccccag cccttgattt ctccctcaag ctgtttgcca aagaggtggc tccagtgccc 960
aaaatccact agtcctaaac cggaagagga tgacaccggc catgggttaa cgtggctcct 1020
caggaccctc tgggtcacac atctttaggg tcagtggcca atggggcaca tttggcacta 1080
gcttgagccc aactctggct ctgccacctg ctgctcctgt gacctgtcag ggctgacttc 1140
acctctgctc atctcagttt cctaatctgt aaaatgggtc taataaagga tcaaccacat 1200
ggggttctgc ggtgataatg agcacatagt gaggggtcag caaatgtcag aagttacctg 1260
ggacagccgg gcacgatggc tcacacctgt aatcccagca ctttgggagg ctgaggcggg 1320
aagatcactt gagttcagga gtttgagacc agcctggcca acatggtgaa accccatctc 1380
taccaaaaat agaagaatta gctgggtgtg gtggcacgcg cctgtaatcc cagctactta 1440
ggaggctgag gcaggagaat cgcttgaacc caggaagtgg aggttgcagt gagctgatgg 1500
tgccactgca ctccagcctg ggtgatagag cgagactctg tctccaaaga agaaaaaaaa 1560
aaaaaaaaaa aaaaa 1575

<210> 30
<211> 768
<212> DNA
<213> NM_004585.2| Homo sapiens retinoic acid receptor responder (tazarotene induced) 3 (RARRES3), mRNA

<400> 30

ccttcagcat aaaagctgat ccacaaacaa gaggagcacc agacctcctc ttggcttcga 60
gatggcttcg ccacaccaag agcccaaacc tggagacctg attgagattt tccgccttgg 120
ctatgagcac tgggccctgt atataggaga tggctacgtg atccatctgg ctcctccaag 180
tgagtacccc ggggctggct cctccagtgt cttctcagtc ctgagcaaca gtgcagaggt 240
gaaacggggg cgcctggaag atgtggtggg aggctgttgc tatcgggtca acaacagctt 300
ggaccatgag taccaaccac ggcccgtgga ggtgatcatc agttctgcga aggagatggt 360
tggtcagaag atgaagtaca gtattgtgag caggaactgt gagcactttg tcgcccagct 420
gagatatggc aagtcccgct gtaaacaggt ggaaaaggcc aaggttgaag tcggtgtggc 480
cacggcgctt ggaatcctgg ttgttgctgg atgctctttt gcgattagga gataccaaaa 540
aaaagcaaca gcctgaagca gccacaaaat cctgtgttag aagcagctgt gggggtccca 600

gtggagatga gcctccccca tgcctccagc agcctgaccc tcgtgccctg tctcaggcgt 660
tctctagatc ctttcctctg tttccctctc tcgctggcaa aagtatgatc taattgaaac 720
aagactgaag gatcaataaa cagccatctg ccccttcaaa aaaaaaa 768

<210> 31
<211> 696
<212> DNA
<213> NM_002994.1| Homo sapiens chemokine (C-C motif) ligand 4 (CCL4), mRNA

<400> 31

ttcccccccc cccccccccc ccccgcccga gcacaggaca cagctgggtt ctgaagcttc 60
tgagttctgc agcctcacct ctgagaaaac ctcttttcca ccaataccat gaagctctgc 120
gtgactgtcc tgtctctcct catgctagta gctgccttct gctctccagc gctctcagca 180
ccaatgggct cagaccctcc caccgcctgc tgcttttctt acaccgcgag gaagcttcct 240
cgcaactttg tggtagatta ctatgagacc agcagcctct gctcccagcc agctgtggta 300
ttccaaacca aaagaagcaa gcaagtctgt gctgatccca gtgaatcctg ggtccaggag 360
tacgtgtatg acctggaact gaactgagct gctcagagac aggaagtctt cagggaaggt 420
cacctgagcc cggatgcttc tccatgagac acatctcctc catactcagg actcctctcc 480
gcagttcctg tcccttctct taatttaatc tttttatgt gccgtgttat tgtattaggt 540
gtcatttcca ttatttatat tagtttagcc aaaggataag tgtcctatgg ggatggtcca 600
ctgtcactgt ttctctgctg ttgcaaatac atggataaca catttgattc tgtgtgtttt 660
ccataataaa actttaaaat aaaatgcaga cagtta 696

<210> 32
<211> 3338
<212> DNA
<213> NM_001455.2| Homo sapiens forkhead box O3A (FOXO3A), transcript variant 1, mRNA

<400> 32

gcgcgaggcc gtcgattcgc tcgcggctcc atcgcggcct ggccgggggg cggtgtctgc 60
tgcgccaggt tcgctggccg cacgtcttca ggtcctcctg ttcctgggag gcgggcgcgg 120
caggactggg aggtggcggc agcgggcgag gactcgccga ggacggggct ccggcccggg 180
ataaccaact ctccttctct cttctttggt gcttccccag gcggcggcgg cggcgcccgg 240
gagccggagc cttcgcggcg tccacgtccc tcccccgctg caccccgccc cggcgcgaga 300
ggagagcgcg agagccccag ccgcgggcgg gcgggcggcg aagatggcag aggcaccggc 360

```
ttccccggcc ccgctctctc cgctcgaagt ggagctggac ccggagttcg agccccagag    420
ccgtccgcga tcctgtacgt ggcccctgca aaggccggag ctccaagcga gccctgccaa    480
gccctcgggg gagacggccg ccgactccat gatccccgag gaggaggacg atgaagacga    540
cgaggacggc gggggacggg ccggctcggc catggcgatc ggcggcggcg gcgggagcgg    600
cacgctgggc tccgggctgc tccttgagga ctcggcccgg gtgctggcac ccggagggca    660
agaccccggg tctgggccag ccaccgcggc gggcgggctg agcgggggta cacaggcgct    720
gctgcagcct cagcaaccgc tgccaccgcc gcagccgggg gcggctgggg gctccgggca    780
gccgaggaaa tgttcgtcgc ggcggaacgc ctggggaaac ctgtcctacg cggacctgat    840
cacccgcgcc atcgagagct ccccggacaa acggctcact ctgtcccaga tctacgagtg    900
gatggtgcgt tgcgtgccct acttcaagga taagggcgac agcaacagct ctgccggctg    960
gaagaactcc atccggcaca acctgtcact gcatagtcga ttcatgcggg tccagaatga   1020
gggaactggc aagagctctt ggtggatcat caaccctgat ggggggaaga gcggaaaagc   1080
cccccggcgg cgggctgtct ccatggacaa tagcaacaag tataccaaga gccgtggccg   1140
cgcagccaag aagaaggcag ccctgcagac agcccccgaa tcagctgacg acagtccctc   1200
ccagctctcc aagtggcctg gcagccccac gtcacgcagc agtgatgagc tggatgcgtg   1260
gacggacttc cgttcacgca ccaattctaa cgccagcaca gtcagtggcc gcctgtcgcc   1320
catcatggca agcacagagt tggatgaagt ccaggacgat gatgcgcctc tctcgcccat   1380
gctctacagc agctcagcca gcctgtcacc ttcagtaagc aagccgtgca cggtggaact   1440
gccacggctg actgatatgg caggcaccat gaatctgaat gatgggctga ctgaaaacct   1500
catggacgac ctgctggata acatcacgct cccgccatcc cagccatcgc ccactggggg   1560
actcatgcag cggagctcta gcttcccgta taccaccaag ggctcgggcc tgggctcccc   1620
aaccagctcc tttaacagca cggtgttcgg accttcatct ctgaactccc tacgccagtc   1680
tcccatgcag accatccaag agaacaagcc agctaccttc tcttccatgt cacactatgg   1740
taaccagaca ctccaggacc tgctcacttc ggactcactt agccacagcg atgtcatgat   1800
gacacagtcg gaccccttga tgtctcaggc cagcaccgct gtgtctgccc agaattcccg   1860
ccggaacgtg atgcttcgca atgatccgat gatgtccttt gctgcccagc ctaaccaggg   1920
aagtttggtc aatcagaact tgctccacca ccagcaccaa acccagggcg ctcttggtgg   1980
cagccgtgcc ttgtcgaatt ctgtcagcaa catgggcttg agtgagtcca gcagccttgg   2040
gtcagccaaa caccagcagc agtctcctgt cagccagtct atgcaaaccc tctcggactc   2100
tctctcaggc tcctccttgt actcaactag tgcaaacctg cccgtcatgg gccatgagaa   2160
gttccccagc gacttggacc tggacatgtt caatgggagc ttggaatgtg acatggagtc   2220
cattatccgt agtgaactca tggatgctga tgggttggat tttaactttg attccctcat   2280
ctccacacag aatgttgttg gtttgaacgt ggggaacttc actggtgcta agcaggcctc   2340
atctcagagc tgggtgccag gctgaaggat cactgaggaa ggggaagtgg gcaaagcaga   2400
```

ccctcaaact gacacaagac ctacagagaa aacccctttgc caaatctgct ctcagcaagt 2460
ggacagtgat accgtttaca gcttaacacc tttgtgaatc ccacgccatt ttcctaaccc 2520
agcagagact gttaatggcc ccttaccctg ggtgaagcac ttacccttgg aacagaactc 2580
taaaaagtat gcaaaatctt ccttgtacag ggtggtgagc cgcctgccag tggaggacag 2640
cacccctcag caccacccac cctcattcag agcacaccgt gagccccgt cggccattct 2700
gtggtgtttt aatattgcga tggtttatgg gacgttttaa gtgttgttct tgtgtttgtt 2760
ttcctttgac tttctgagtt tttcacatgc attaacttgc ggtatttttc tgttaaaatg 2820
ttaaccgtcc ttcccctagc aaatttaaaa acagaaagaa aatgttgtac cagttaccat 2880
tccgggttcg agcatcacaa gcttttgagc gcatggaact ccataaacta acaaattaca 2940
taaactaaag gggattttc tttcttcttt tgtttggtag aaaattatcc ttttctaaaa 3000
actgaacaat ggcacaattg tttgctatgt gcacccgtcc aggacagaac cgtgcatagg 3060
caaaaggagt ggagcacagc gtccggccca gtgtgtttcc ggttctgagt cagggtgatc 3120
tgtggacggg accccagcac caagtctacg ggtgccagat cagtagggcc tgtgatttcc 3180
tgtcagtgtc ctcagctaat gtgaacagtg ttggtctgct ggttagaaac tagaatattg 3240
atattttcag gaaagaaatc agctcagctc tccactcatt gccaaatgtc actaaagggt 3300
ttagttttaa ggagaaagaa aaggaaaaaa aaaaaaa 3338

<210> 33
<211> 2646
<212> DNA
<213> NM_152873.1| Homo sapiens tumor necrosis factor receptor superfamily, member 6 (TNFRSF6), transcript variant 4, mRNA

<400> 33

cctacccgcg cgcaggccaa gttgctgaat caatggagcc ctccccaacc cgggcgttcc 60
ccagcgaggc ttccttccca tcctcctgac caccggggct tttcgtgagc tcgtctctga 120
tctcgcgcaa gagtgacaca caggtgttca aagacgcttc tggggagtga gggaagcggt 180
ttacgagtga cttggctgga gcctcagggg cgggcactgg cacggaacac accctgaggc 240
cagccctggc tgcccaggcg gagctgcctc ttctcccgcg ggttggtgga cccgctcagt 300
acggagttgg ggaagctctt tcacttcgga ggattgctca acaaccatgc tgggcatctg 360
gaccctccta cctctggttc ttacgtctgt tgctagatta tcgtccaaaa gtgttaatgc 420
ccaagtgact gacatcaact ccaagggatt ggaattgagg aagactgtta ctacagttga 480
gactcagaac ttggaaggcc tgcatcatga tggccaattc tgccataagc cctgtcctcc 540
aggtgaaagg aaagctaggg actgcacagt caatggggat gaaccagact gcgtgccctg 600
ccaagaaggg aaggagtaca cagacaaagc ccatttttct tccaaatgca gaagatgtag 660

```
attgtgtgat gaaggacatg atgtgaacat ggaatcatca aggaatgcac actcaccagc   720
aacaccaagt gcaaagagga aggatccaga tctaacttgg ggtggctttg tcttcttctt   780
ttgccaattc cactaattgt ttgggtgaag agaaaggaag tacagaaaac atgcagaaag   840
cacagaaagg aaaaccaagg ttctcatgaa tctccaacct taaatcctga aacagtggca   900
ataaatttat ctgatgttga cttgagtaaa tatatcacca ctattgctgg agtcatgaca   960
ctaagtcaag ttaaaggctt tgttcgaaag aatggtgtca atgaagccaa aatagatgag  1020
atcaagaatg acaatgtcca agacacagca gaacagaaag ttcaactgct tcgtaattgg  1080
catcaacttc atggaaagaa agaagcgtat gacacattga ttaaagatct caaaaaagcc  1140
aatctttgta ctcttgcaga gaaaattcag actatcatcc tcaaggacat tactagtgac  1200
tcagaaaatt caaacttcag aaatgaaatc caaagcttgg tctagagtga aaaacaacaa  1260
attcagttct gagtatatgc aattagtgtt tgaaaagatt cttaatagct ggctgtaaat  1320
actgcttggt tttttactgg gtacatttta tcatttatta gcgctgaaga gccaacatat  1380
ttgtagattt ttaatatctc atgattctgc ctccaaggat gtttaaaatc tagttgggaa  1440
aacaaacttc atcaagagta aatgcagtgg catgctaagt acccaaatag gagtgtatgc  1500
agaggatgaa agattaagat tatgctctgg catctaacat atgattctgt agtatgaatg  1560
taatcagtgt atgttagtac aaatgtctat ccacaggcta accccactct atgaatcaat  1620
agaagaagct atgacctttt gctgaaatat cagttactga acaggcaggc cactttgcct  1680
ctaaattacc tctgataatt ctagagattt taccatattt ctaaactttg tttataactc  1740
tgagaagatc atatttatgt aaagtatatg tatttgagtg cagaatttaa ataaggctct  1800
acctcaaaga cctttgcaca gtttattggt gtcatattat acaatatttc aattgtgaat  1860
tcacatagaa aacattaaat tataatgttt gactattata tatgtgtatg cattttactg  1920
gctcaaaact acctacttct ttctcaggca tcaaaagcat tttgagcagg agagtattac  1980
tagagctttg ccacctctcc atttttgcct tggtgctcat cttaatggcc taatgcaccc  2040
ccaaacatgg aaatatcacc aaaaaatact taatagtcca ccaaaaggca agactgccct  2100
tagaaattct agcctggttt ggagatacta actgctctca gagaaagtag ctttgtgaca  2160
tgtcatgaac ccatgtttgc aatcaaagat gataaaatag attcttattt ttcccccacc  2220
cccgaaaatg ttcaataatg tcccatgtaa aacctgctac aaatggcagc ttatacatag  2280
caatggtaaa atcatcatct ggatttagga attgctcttg tcatacccccc aagtttctaa  2340
gatttaagat tctccttact actatcctac gtttaaatat ctttgaaagt ttgtattaaa  2400
tgtgaatttt aagaaataat atttatattt ctgtaaatgt aaactgtgaa gatagttata  2460
aactgaagca gatacctgga accacctaaa gaacttccat ttatggagga tttttttgcc  2520
ccttgtgttt ggaattataa aatataggta aaagtacgta attaaataat gtttttggta  2580
aaaaaaaaaa aaaaaaaaaa aaaaaaaaaa aaaaaaaaaa aaaaaaaaaa aaaaaaaaaa  2640
```

aaaaaa 2646

<210> 34
<211> 817
<212> DNA
<213> NM_002038.2| Homo sapiens interferon, alpha-inducible protein (clone IFI-6-16) (G1P3), transcript variant 1, mRNA

<400> 34

```
gaaccgttta ctcgctgctg tgcccatcta tcagcaggct ccgggctgaa gattgcttct     60
cttctctcct ccaaggtcta gtgacggagc ccgcgcgcgg cgccaccatg cggcagaagg    120
cggtatcgct ttttcttgtgc tacctgctgc tcttcacttg cagtggggtg gaggcaggta   180
agaaaaagtg ctcggagagc tcggacagcg gctccgggtt ctggaaggcc ctgaccttca    240
tggccgtcgg aggaggactc gcagtcgccg ggctgcccgc gctgggcttc accggcgccg    300
gcatcgcggc caactcggtg gctgcctcgc tgatgagctg gtctgcgatc ctgaatgggg    360
gcggcgtgcc cgccgggggg ctagtggcca cgctgcagag cctcggggct ggtggcagca    420
gcgtcgtcat aggtaatatt ggtgccctga tgggctacgc cacccacaag tatctcgata    480
gtgaggagga tgaggagtag ccagcagctc ccagaacctc ttcttccttc ttggcctaac    540
tcttccagtt aggatctaga actttgcctt tttttttttt tttttttttt tttgagatgg    600
gttctcacta tattgtccag gctagagtgc agtggctatt cacagatgcg aacatagtac    660
actgcagcct ccaactccta gcctcaagtg atcctcctgt ctcaacctcc caagtaggat    720
tacaagcatg cgccgacgat gcccagaatc cagaactttg tctatcactc tccccaacaa    780
cctagatgtg aaaacagaat aaacttcacc cagaaaa                              817
```

<210> 35
<211> 1172
<212> DNA
<213> NM_001565.1| Homo sapiens chemokine (C-X-C motif) ligand 10 (CXCL10), mRNA

<400> 35

```
gagacattcc tcaattgctt agacatattc tgagcctaca gcagaggaac ctccagtctc     60
agcaccatga atcaaactgc gattctgatt tgctgcctta tctttctgac tctaagtggc    120
attcaaggag tacctctctc tagaaccgta cgctgtacct gcatcagcat tagtaatcaa    180
cctgttaatc caaggtcttt agaaaaactt gaaattattc ctgcaagcca attttgtcca    240
cgtgttgaga tcattgctac aatgaaaaag aagggtgaga agagatgtct gaatccagaa    300
tcgaaggcca tcaagaattt actgaaagca gttagcaagg aaatgtctaa aagatctcct    360
```

taaaaccaga ggggagcaaa atcgatgcag tgcttccaag gatggaccac acagaggctg 420
cctctcccat cacttcccta catggagtat atgtcaagcc ataattgttc ttagtttgca 480
gttacactaa aaggtgacca atgatggtca ccaaatcagc tgctactact cctgtaggaa 540
ggttaatgtt catcatccta agctattcag taataactct accctggcac tataatgtaa 600
gctctactga ggtgctatgt tcttagtgga tgttctgacc ctgcttcaaa tatttccctc 660
acctttccca tcttccaagg gtactaagga atctttctgc tttggggttt atcagaattc 720
tcagaatctc aaataactaa aaggtatgca atcaaatctg ctttttaaag aatgctcttt 780
acttcatgga cttccactgc catcctccca aggggcccaa attctttcag tggctaccta 840
catacaattc caaacacata caggaaggta gaaatatctg aaaatgtatg tgtaagtatt 900
cttatttaat gaaagactgt acaaagtata agtcttagat gtatatattt cctatattgt 960
tttcagtgta catggaataa catgtaatta agtactatgt atcaatgagt aacaggaaaa 1020
ttttaaaaat acagatagat atatgctctg catgttacat aagataaatg tgctgaatgg 1080
ttttcaaata aaaatgaggt actctcctgg aaatattaag aaagactatc taaatgttga 1140
aagatcaaaa ggttaataaa gtaattataa ct 1172

<210> 36
<211> 396
<212> DNA
<213> NM_005950.1| Homo sapiens metallothionein 1G (MT1G), mRNA

<400> 36

actccgcctt ccacgtgcac ccactgcctc ttcccttctc gcttgggaac tctagtctcg 60
cctcgggttg caatggaccc caactgctcc tgtgccgctg gtgtctcctg cacctgcgcc 120
agctcctgca agtgcaaaga gtgcaaatgc acctcctgca agaagagctg ctgctcctgc 180
tgccctgtgg gctgtgccaa gtgtgcccaa ggctgcatct gcaaaggggc atcggagaag 240
tgcagctgct gcgcctgatg tcgggacagc cctgctccca agtacaaata gagtgacccg 300
taaaatctag gatttttgt tttttgctac aatcttgacc cctttgctac attccctttt 360
ttctgtgaaa tatgtgaata ataattaaac acttag 396

<210> 37
<211> 2755
<212> DNA
<213> NM_000043.3| Homo sapiens tumor necrosis factor receptor superfamily, member 6 (TNFRSF6), transcript variant 1, mRNA

<400> 37

cctacccgcg cgcaggccaa gttgctgaat caatggagcc ctccccaacc cgggcgttcc 60
ccagcgaggc ttccttccca tcctcctgac caccggggct tttcgtgagc tcgtctctga 120
tctcgcgcaa gagtgacaca caggtgttca aagacgcttc tggggagtga gggaagcggt 180
ttacgagtga cttggctgga gcctcagggg cgggcactgg cacggaacac accctgaggc 240
cagccctggc tgcccaggcg gagctgcctc ttctcccgcg ggttggtgga cccgctcagt 300
acggagttgg ggaagctctt tcacttcgga ggattgctca acaaccatgc tgggcatctg 360
gaccctccta cctctggttc ttacgtctgt tgctagatta tcgtccaaaa gtgttaatgc 420
ccaagtgact gacatcaact ccaagggatt ggaattgagg aagactgtta ctacagttga 480
gactcagaac ttggaaggcc tgcatcatga tggccaattc tgccataagc cctgtcctcc 540
aggtgaaagg aaagctaggg actgcacagt caatggggat gaaccagact gcgtgccctg 600
ccaagaaggg aaggagtaca cagacaaagc ccatttttct tccaaatgca gaagatgtag 660
attgtgtgat gaaggacatg gcttagaagt ggaaataaac tgcacccgga cccagaatac 720
caagtgcaga tgtaaaccaa actttttttg taactctact gtatgtgaac actgtgaccc 780
ttgcaccaaa tgtgaacatg gaatcatcaa ggaatgcaca ctcaccagca acaccaagtg 840
caaagaggaa ggatccagat ctaacttggg gtggctttgt cttcttcttt tgccaattcc 900
actaattgtt tgggtgaaga gaaaggaagt acagaaaaca tgcagaaagc acagaaagga 960
aaaccaaggt tctcatgaat ctccaacctt aaatcctgaa acagtggcaa taaatttatc 1020
tgatgttgac ttgagtaaat atatcaccac tattgctgga gtcatgacac taagtcaagt 1080
taaaggcttt gttcgaaaga atggtgtcaa tgaagccaaa atagatgaga tcaagaatga 1140
caatgtccaa gacacagcag aacagaaagt tcaactgctt cgtaattggc atcaacttca 1200
tggaaagaaa gaagcgtatg acacattgat taaagatctc aaaaaagcca atctttgtac 1260
tcttgcagag aaaattcaga ctatcatcct caaggacatt actagtgact cagaaaattc 1320
aaacttcaga aatgaaatcc aaagcttggt ctagagtgaa aaacaacaaa ttcagttctg 1380
agtatatgca attagtgttt gaaaagattc ttaatagctg gctgtaaata ctgcttggtt 1440
ttttactggg tacattttat catttattag cgctgaagag ccaacatatt tgtagatttt 1500
taatatctca tgattctgcc tccaaggatg tttaaaatct agttgggaaa acaaacttca 1560
tcaagagtaa atgcagtggc atgctaagta cccaaatagg agtgtatgca gaggatgaaa 1620
gattaagatt atgctctggc atctaacata tgattctgta gtatgaatgt aatcagtgta 1680
tgttagtaca aatgtctatc cacaggctaa ccccactcta tgaatcaata gaagaagcta 1740
tgaccttttg ctgaaatatc agttactgaa caggcaggcc actttgcctc taaattacct 1800
ctgataattc tagagatttt accatatttc taaactttgt ttataactct gagaagatca 1860
tatttatgta aagtatatgt atttgagtgc agaatttaaa taaggctcta cctcaaagac 1920
ctttgcacag tttattggtg tcatattata caatatttca attgtgaatt cacatagaaa 1980

acattaaatt ataatgtttg actattatat atgtgtatgc attttactgg ctcaaaacta 2040
cctacttctt tctcaggcat caaaagcatt ttgagcagga gagtattact agagctttgc 2100
cacctctcca tttttgcctt ggtgctcatc ttaatggcct aatgcacccc caaacatgga 2160
aatatcacca aaaaatactt aatagtccac caaaaggcaa gactgccctt agaaattcta 2220
gcctggtttg gagatactaa ctgctctcag agaaagtagc tttgtgacat gtcatgaacc 2280
catgtttgca atcaaagatg ataaaataga ttcttatttt tcccccaccc ccgaaaatgt 2340
tcaataatgt cccatgtaaa acctgctaca aatggcagct tatacatagc aatggtaaaa 2400
tcatcatctg gatttaggaa ttgctcttgt catacccccca agtttctaag atttaagatt 2460
ctccttacta ctatcctacg tttaaatatc tttgaaagtt tgtattaaat gtgaatttta 2520
agaaataata tttatatttc tgtaaatgta aactgtgaag atagttataa actgaagcag 2580
atacctggaa ccacctaaag aacttccatt tatggaggat ttttttgccc cttgtgtttg 2640
gaattataaa atataggtaa aagtacgtaa ttaaataatg tttttggtaa aaaaaaaaaa 2700
aaaaaaaaaa aaaaaaaaaa aaaaaaaaaa aaaaaaaaaa aaaaaaaaaa aaaaa 2755

<210> 38
<211> 1600
<212> DNA
<213> NM_001953.2| Homo sapiens endothelial cell growth factor 1 (platelet-derived) (ECGF1), mRNA

<400> 38

gccccgccgc cggcagtgga ccgctgtgcg cgaaccctga accctacggt cccgacccgc 60
gggcgaggcc gggtacctgg gctgggatcc ggagcaagcg ggcgagggca gcgccctaag 120
caggcccgga gcgatggcag ccttgatgac cccgggaacc ggggccccac ccgcgcctgg 180
tgacttctcc ggggaaggga gccagggact tcccgaccct tcgccagagc ccaagcagct 240
cccggagctg atccgcatga agcgagacgg aggccgcctg agcgaagcgg acatcagggg 300
cttcgtggcc gctgtggtga atgggagcgc gcagggcgca cagatcgggg ccatgctgat 360
ggccatccga cttcggggca tggatctgga ggagacctcg gtgctgaccc aggccctggc 420
tcagtcggga cagcagctgg agtggccaga ggcctggcgc cagcagcttg tggacaagca 480
ttccacaggg ggtgtgggtg acaaggtcag cctggtcctc gcacctgccc tggcggcatg 540
tggctgcaag gtgccaatga tcagcggacg tggtctgggg cacacaggag gcaccttgga 600
taagctggag tctattcctg gattcaatgt catccagagc ccagagcaga tgcaagtgct 660
gctggaccag gcgggctgct gtatcgtggg tcagagtgag cagctggttc ctgcggacgg 720
aatcctatat gcagccagag atgtgacagc caccgtggac agcctgccac tcatcacagc 780
ctccattctc agtaagaaac tcgtggaggg gctgtccgct ctggtggtgg acgttaagtt 840

```
cggaggggcc gccgtcttcc ccaaccagga gcaggcccgg gagctggcaa agacgctggt    900
tggcgtggga gccagcctag ggcttcgggt cgcggcagcg ctgaccgcca tggacaagcc    960
cctgggtcgc tgcgtgggcc acgccctgga ggtggaggag gcgctgctct gcatggacgg   1020
cgcaggcccg ccagacttaa gggacctggt caccacgctc gggggcgccc tgctctggct   1080
cagcggacac gcggggactc aggctcaggg cgctgcccgg gtggccgcgg cgctggacga   1140
cggctcggcc cttggccgct tcgagcggat gctggcggcg cagggcgtgg atcccggtct   1200
ggcccgagcc ctgtgctcgg gaagtcccgc agaacgccgg cagctgctgc ctcgcgcccg   1260
ggagcaggag gagctgctgg cgcccgcaga tggcaccgtg gagctggtcc gggcgctgcc   1320
gctggcgctg gtgctgcacg agctcggggc cgggcgcagc cgcgctgggg agccgctccg   1380
cctgggggtg ggcgcagagc tgctggtcga cgtgggtcag aggctgcgcc gtgggacccc   1440
ctggctccgc gtgcaccggg acggccccgc gctcagcggc ccgcagagcc gcgccctgca   1500
ggaggcgctc gtactctccg accgcgcgcc attcgccgcc ccctcgccct tcgcagagct   1560
cgttctgccg ccgcagcaat aaagctcctt tgccgcgaaa                         1600
```

<210> 39
<211> 931
<212> DNA
<213> NM_005138.1| Homo sapiens SCO cytochrome oxidase deficient homolog 2 (yeast) (SCO2), nuclear gene encoding mitochondrial protein, mRNA

<400> 39

```
gcagagccca gggagctgga ggtcggcgct tcctctcgtg cttggtccac tgacgcgcgg     60
ccccgccgcg aggagcatca gatccatgct gctgctgact cggagcccca cagcttggca    120
caggctctct cagctcaagc ctccggtcct ccctgggacc ctgggaggcc aggccctgca    180
tctgaggtcc tggcttttgt caaggcaggg ccctgcagag acaggtgggc agggccagcc    240
ccagggccct gggcttcgaa cccggctgct gatcacaggc ctgttcgggg ctggactcgg    300
tggggcctgg ctggccctga gggctgagaa ggagaggctg cagcagcaaa agcgaacaga    360
agccctgcgc caggcagctg tgggccaggg cgacttccac ctgctggatc acagaggccg    420
ggctcgctgc aaggctgact tccggggcca gtgggtgctg atgtactttg gcttcactca    480
ctgccctgac atctgcccag acgagctgga gaagctggtg caggtggtgc ggcagctgga    540
agcagagcct ggtttgcctc cagtgcagcc tgtcttcatc actgtggacc ccgagcggga    600
cgacgttgaa gccatggccc gctacgtcca ggacttccac ccaagactgt tgggtctgac    660
cggctccacc aaacaggttg cccaggctag tcacagttac cgcgtgtact acaatgccgg    720
ccccaaggat gaggaccagg actacatcgt ggaccactcc attgccatct acctgctcaa    780
ccctgacggc ctcttcacgg attactacgg ccggagcaga tcggctgagc agatctcaga    840
```

cagtgtgcgg cggcacatgg cggctttccg cagtgtcctg tcttgagcca ctgcagtctg 900
ggccccatca ttaaacgggc tgcgtttaaa a 931

<210> 40
<211> 1216
<212> DNA
<213> NM_006419.1| Homo sapiens chemokine (C-X-C motif) ligand 13 (B-cell chemoattractant) (CXCL13), mRNA

<400> 40

ttcggcactt gggagaagat gtttgaaaaa actgactctg ctaatgagcc tggactcaga 60
gctcaagtct gaactctacc tccagacaga atgaagttca tctcgacatc tctgcttctc 120
atgctgctgg tcagcagcct ctctccagtc caaggtgttc tggaggtcta ttacacaagc 180
ttgaggtgta gatgtgtcca agagagctca gtctttatcc ctagacgctt cattgatcga 240
attcaaatct tgccccgtgg gaatggttgt ccaagaaaag aaatcatagt ctggaagaag 300
aacaagtcaa ttgtgtgtgt ggaccctcaa gctgaatgga tacaaagaat gatggaagta 360
ttgagaaaaa gaagttcttc aactctacca gttccagtgt ttaagagaaa gattccctga 420
tgctgatatt tccactaaga acacctgcat tcttccctta tccctgctct ggattttagt 480
tttgtgctta gttaaatctt ttccagggag aaagaacttc cccatacaaa taaggcatga 540
ggactatgtg aaaaataacc ttgcaggagc tgatggggca aactcaagct tcttcactca 600
cagcacccta tatacacttg gagtttgcat tcttattcat cagggaggaa agtttctttg 660
aaaatagtta ttcagttata agtaatacag gattattttg attatatact tgttgtttaa 720
tgtttaaaat ttcttagaaa acaatggaat gagaatttaa gcctcaaatt tgaacatgtg 780
gcttgaatta agaagaaaat tatggcatat attaaaagca ggcttctatg aaagactcaa 840
aaagctgcct gggaggcaga tggaacttga gcctgtcaag aggcaaagga atccatgtag 900
tagatatcct ctgcttaaaa actcactacg gaggagaatt aagtcctact tttaaagaat 960
ttctttataa aatttactgt ctaagattaa tagcattcga agatccccag acttcataga 1020
atactcaggg aaagcattta aagggtgatg tacacatgta tcctttcaca catttgcctt 1080
gacaaacttc tttcactcac atcttttca ctgacttttt ttgtgggggc ggggccgggg 1140
ggactctggt atctaattct ttaatgattc ctataaatct aatgacattc aataaagttg 1200
agcaaacatt ttactt 1216

<210> 41
<211> 738
<212> DNA
<213> NM_006433.2| Homo sapiens granulysin (GNLY), transcript variant NKG5, mRNA

<400> 41

gtatctgtgg taaacccagt gacacggggg agatgacata caaaaagggc aggacctgag 60
aaagattaag ctgcaggctc cctgcccata aaacagggtg tgaaaggcat ctcagcggct 120
gccccaccat ggctacctgg gccctcctgc tccttgcagc catgctcctg ggcaacccag 180
gtctggtctt ctctcgtctg agccctgagt actacgacct ggcaagagcc cacctgcgtg 240
atgaggagaa atcctgcccg tgcctggccc aggagggccc ccagggtgac ctgttgacca 300
aaacacagga gctgggccgt gactacagga cctgtctgac gatagtccaa aaactgaaga 360
agatggtgga taagcccacc cagagaagtg tttccaatgc tgcgacccgg gtgtgtagga 420
cggggaggtc acgatggcgc gacgtctgca gaaatttcat gaggaggtat cagtctagag 480
ttacccaggg cctcgtggcc ggagaaactg cccagcagat ctgtgaggac ctcaggttgt 540
gtataccttc tacaggtccc ctctgagccc tctcaccttg tcctgtggaa gaagcacagg 600
ctcctgtcct cagatcccgg gaacctcagc aacctctgcc ggctcctcgc ttcctcgatc 660
cagaatccac tctccagtct ccctcccctg actccctctg ctgtcctccc ctctcacgag 720
aataaagtgt caagcaag 738

<210> 42
<211> 1579
<212> DNA
<213> NM_001767.2| Homo sapiens CD2 antigen (p50), sheep red blood cell receptor (CD2), mRNA

<400> 42

accaacccct aagatgagct ttccatgtaa atttgtagcc agcttccttc tgattttcaa 60
tgtttcttcc aaaggtgcag tctccaaaga gattacgaat gccttggaaa cctggggtgc 120
cttgggtcag gacatcaact tggacattcc tagtttttcaa atgagtgatg atattgacga 180
tataaaatgg gaaaaaactt cagacaagaa aaagattgca caattcagaa aagagaaaga 240
gactttcaag gaaaaagata catataagct atttaaaaat ggaactctga aaattaagca 300
tctgaagacc gatgatcagg atatctacaa ggtatcaata tatgatacaa aaggaaaaaa 360
tgtgttggaa aaaatatttg atttgaagat tcaagagagg gtctcaaaac caaagatctc 420
ctggacttgt atcaacacaa ccctgacctg tgaggtaatg aatggaactg accccgaatt 480
aaacctgtat caagatggga aacatctaaa actttctcag agggtcatca cacacaagtg 540
gaccaccagc ctgagtgcaa aattcaagtg cacagcaggg aacaaagtca gcaaggaatc 600
cagtgtcgag cctgtcagct gtccagagaa aggtctggac atctatctca tcattggcat 660

```
atgtggagga ggcagcctct tgatggtctt tgtggcactg ctcgttttct atatcaccaa    720
aaggaaaaaa cagaggagtc ggagaaatga tgaggagctg gagacaagag cccacagagt    780
agctactgaa gaaaggggcc ggaagcccca acaaattcca gcttcaaccc ctcagaatcc    840
agcaacttcc caacatcctc ctccaccacc tggtcatcgt tcccaggcac ctagtcatcg    900
tcccccgcct cctggacacc gtgttcagca ccagcctcag aagaggcctc ctgctccgtc    960
gggcacacaa gttcaccagc agaaaggccc gcccctcccc agacctcgag ttcagccaaa   1020
acctccccat ggggcagcag aaaactcatt gtccccttcc tctaattaaa aaagatagaa   1080
actgtctttt tcaataaaaa gcactgtgga tttctgccct cctgatgtgc atatccgtac   1140
ttccatgagg tgttttctgt gtgcagaaca ttgtcacctc ctgaggctgt gggccacagc   1200
cacctctgca tcttcgaact cagccatgtg gtcaacatct ggagtttttg gtctcctcag   1260
agagctccat cacaccagta aggagaagca atataagtgt gattgcaaga atggtagagg   1320
accgagcaca gaaatcttag agatttcttg tcccctctca ggtcatgtgt agatgcgata   1380
aatcaagtga ttggtgtgcc tgggtctcac tacaagcagc ctatctgctt aagagactct   1440
ggagtttctt atgtgccctg gtggacactt gcccaccatc ctgtgagtaa aagtgaaata   1500
aaagctttga ctagaaaaaa aaaaaaaaaa aaaaaaaaaa aaaaaaaaaa aaaaaaaaaa   1560
aaaaaaaaaa aaaaaaaaa                                                1579
```

<210> 43
<211> 3738
<212> DNA
<213> NM_006275.4| Homo sapiens splicing factor, arginine/serine-rich 6 (SFRS6), mRNA

<400> 43

```
ctggcgcgcg cgcgcgccat tgtgtggctg gactcggccg cccctgtggt gtgaggcgcg     60
tgttcgggct cttgccgtcc ccgcacccgc accgcggtta ctggcttgcg gtccgccgtt    120
cgacaaccag cccttgggtc cccgcccgcc acggacatgc cgcgcgtcta cataggacgc    180
ctgagctaca acgtccggga gaaggacatc cagcgctttt tcagtggcta tggccgcctc    240
ctcgaagtag acctcaaaaa tgggtacggc ttcgtggagt tcgaggactc ccgcgacgcc    300
gacgacgccg tttacgagct gaacggcaag gagctctgcg gcgagcgcgt gatcgtagag    360
cacgcccggg gcccgcgtcg cgatcgcgac ggctacagct acggaagccg cagtggtgga    420
ggtggataca gcagtcggag aacatctggc agagacaaat acggaccacc tgttcgtaca    480
gaatacaggc ttattgtaga aaatctttct agtcggtgca gttggcaaga tttaaaggat    540
tttatgcgac aagcaggtga agtaacctat gcggatgccc acaaggaacg aacaaatgag    600
ggtgtaattg agtttcgctc ctactctgac atgaagcgtg ctttggacaa actggatggc    660
```

```
acagaaataa atggcagaaa tattaggctt attgaagata agccacgcac aagccatagg      720
cgatcttact ctggaagcag atccaggtct cgatctagaa gacggtcacg aagtaggagt      780
cgcaggagca gccgcagtag atctcgaagt atctcaaaaa gtcgctcccg ttccaggtcg      840
cggagcaaag gtcgatcacg ttctcgatca aaaggcagga aatctagatc aaagagcaaa      900
tctaagccca agtctgatcg gggctcccat tcacattctc gaagcagatc taaggatgag      960
tatgagaaat ctcgaagcag gtctcggtcc cgatccccca aagaaaatgg aaagggtgat     1020
ataaagtcaa aatccagatc aaggagccag tcccgttcca attcgccgct acctgttcca     1080
ccctcaaagg cccgttctgt gtcccctcca ccaaaaagag ctacttcaag atcccgttct     1140
agatctcgct caaagtcaag atcaaggtcc aggtcgagtt ccagagatta actcagaact     1200
ccttgtttgc acattattat ggaacacttt cctacttagg cagttactct tccatgttta     1260
tacttggcct cttctgcaag aggaatctct tgaaaacagg ggcacacaga aatttgattt     1320
gtggccaaat tggatgaaaa agatgaggct ctaaggaaat ggtggcatga agaccctctc     1380
ccttctttgt agaattaaga taactttgat tttatagctt ttgagctaac gtaacttttg     1440
taaagattaa gctcatttag tgttgttttt tttttttttt tttttttttt tttttagtat     1500
ttcagcagga tctgctggca gggttttttt gttttatttg tttgcttatt tttaaattaa     1560
ctgttttgag ctttgaatac ttaaggcttt agagggagaa cccaattttc aattatgttg     1620
gctttttata aagcttgagt tatgtaagat ttaaataaaa gtttgctacc aagatgattg     1680
ccttattgaa taggtcacta ttaaattcct ttaaatgttg atatctgcca tttgtggaaa     1740
caacgtaaat tctacttaag tgtaaacaag gcaagcctca gaccagcaat aaattactca     1800
gtttggataa cattattttg tgcagttaat caaatttgcc aaagtcttta tctgcccctt     1860
taacaagttg agtaaaaata aaaggtattt tttagtcaat gtgttccatg attttgctta     1920
aattaatact tttaagtaat ggaacttttt tcaaaggcaa atttaaacta tttaagaaat     1980
agctcctaat acttgggatc ttgtttagag aatccacttt ctggaagttc tcagcataat     2040
tagtgttgag agtggttcag ttgtctttaa tgtttgtcat gtggaaatgg aagtagcctc     2100
tttttgttct gaaattgagt ttattcaaag tgtaaaagca catactgcat tttctgctga     2160
aagatcatta tgtttaacag gcacttaatc tcagtaaagt cagttgccag ttaagttcca     2220
cccagtagtc agtccccttt gtagttagtg ggattatttg ataattggtt agatcatact     2280
tgtaaatttt aatgctttgt gtaattggtt tgaaaaacag tgaaatgggt aaacgcaaaa     2340
cttttgtact ttattacgag taaagtgtaa tgagtactgt ggaaaccaaa tttgaatact     2400
gcaaatttgt aggagttact aggttagcaa ttagtccata catccataag cctgatgagt     2460
tgaaattgca gtttgagaag tgaattaacc ttacatccct ttgttcagat accttaaaag     2520
ttactttatt taaaagcatt tattaatctt agtctgaaat caaaatatag attaattggc     2580
tcagctttaa tacctttcta ggaggtgtca caatgtaggg taccaagggt tggattgtga     2640
tggggcatgg tcgtacactg ctcattgtgc cacaggtgtg actggaaagc atgatattct     2700
```

agggttggtt tgtagattca aataatccag aaatatacct aataagattg agtgaaaaat 2760
ttgagtcaaa tatctagggc attcacagag tagctgtgag ttcttggtaa tgtgaaaaag 2820
gccttgtttt tcagaaattc ctgggtttcc tgttaaaaaa tcttaaagcc caaccttagg 2880
aatatagtgc cccaaaaggc ggatgcttct tccattatct tattttcttt gatactttat 2940
ttaattagat gtttataaag aaatgggttt atttttccag cataaacctc agaatttaag 3000
gaaagaaaat gatgtctgtt gttatagttc attgttttgc ctactcagca gaagtgatga 3060
ctcttaaaaa ttggctttga ccaaagttct cttgttttca gggaaagaac ataaaagctt 3120
tttgaactac agccttttta aaagagggat gggaggatat tacagtaaga aattaggctt 3180
tctaaaagta tgaaacatcc ttcaactggg ctctcttgtt aataggacat catatggtaa 3240
tagactggtt tgactatatt gttagctgcc acagtaagca ggtcattgta taggtaaatg 3300
cctgcaccca taattttcta gtaatagcca cgaccaattt attaacagtc agggcctatc 3360
cttgcctgta gttctcagtc actggatgca caaaatcact gtgtaacatt ggctcacttg 3420
gtgagcatag ggttgactga taaaatgttt aattcccttg ctagcttgtg agaagaatga 3480
gttgatgaca tgctccatac cagtggctag atggagtatt aaggtggagc agaaaagaag 3540
tgagaacatc ttgattcccc tttcttttac ttgatggtgt ttatgaacat gccgtagtgc 3600
ctttatggcc agtttgagtc ctgcctactt tgacttttac gttcccattc ctgtgttacc 3660
accttcctcc cgatttgttc acctattttg tgctttaaat ctcaataaaa tacttactga 3720
ggaaaaaaaa aaaaaaaa 3738

<210> 44
<211> 2033
<212> DNA
<213> NM_003212.1| Homo sapiens teratocarcinoma-derived growth factor 1 (TDGF1), mRNA

<400> 44

ggagaatccc cggaaaggct gagtctccag ctcaaggtca aaacgtccaa ggccgaaagc 60
cctccagttt cccctggacg ccttgctcct gcttctgcta cgaccttctg gggaaaacga 120
atttctcatt ttcttcttaa attgccattt tcgctttagg agatgaatgt ttcctttgg 180
ctgttttggc aatgactctg aattaaagcg atgctaacgc ctcttttccc cctaattgtt 240
aaaagctatg gactgcagga agatggcccg cttctcttac agtgtgattt ggatcatggc 300
catttctaaa gtctttgaac tgggattagt tgccgggctg ggccatcagg aatttgctcg 360
tccatctcgg ggatacctgg ccttcagaga tgacagcatt tggccccagg aggagcctgc 420
aattcggcct cggtcttccc agcgtgtgcc gcccatgggg atacagcaca gtaaggagct 480
aaacagaacc tgctgcctga atgggggaac ctgcatgctg gggtcctttt gtgcctgccc 540

tccctccttc tacggacgga actgtgagca cgatgtgcgc aaagagaact gtgggtctgt 600
gccccatgac acctggctgc ccaagaagtg ttccctgtgt aaatgctggc acggtcagct 660
ccgctgcttt cctcaggcat ttctacccgg ctgtgatggc cttgtgatgg atgagcacct 720
cgtggcttcc aggactccag aactaccacc gtctgcacgt actaccactt ttatgctagt 780
tggcatctgc ctttctatac aaagctacta ttaatcgaca ttgacctatt tccagaaata 840
caattttaga tatcatgcaa atttcatgac cagtaaaggc tgctgctaca atgtcctaac 900
tgaaagatga tcatttgtag ttgccttaaa ataatgaata caatttccaa aatggtctct 960
aacatttcct tacagaacta cttcttactt ctttgccctg ccctctccca aaaaactact 1020
tcttttttca aaagaaagtc agccatatct ccattgtgcc taagtccagt gtttcttttt 1080
ttttttttt ttgagacgga gtctcactct gtcacccagg ctggactgca atgacgcgat 1140
cttggttcac tgcaacctcc gcatccgggg ttcaagccat tctcctgcct aagcctccca 1200
agtaactggg attacaggca tgtgtcacca tgcccagcta atttttttgt attttagtag 1260
agatgggggt ttcaccatat tggccagtct ggtctcgaac tctgaccttg tgatccatcg 1320
atcagcctct cgagtgctga gattacacac gtgagcaact gtgcaaggcc tggtgtttct 1380
tgatacatgt aattctacca aggtcttctt aatatgttct tttaaatgat tgaattatat 1440
gttcagatta ttggagacta attctaatgt ggaccttaga atacagtttt gagtagagtt 1500
gatcaaaatc aattaaaata gtctctttaa aaggaaagaa aacatcttta aggggaggaa 1560
ccagagtgct gaaggaatgg aagtccatct gcgtgtgtgc agggagactg ggtaggaaag 1620
aggaagcaaa tagaagagag aggttgaaaa acaaaatggg ttacttgatt ggtgattagg 1680
tggtggtaga gaagcaagta aaaaggctaa atggaagggc aagtttccat catctataga 1740
aagctatata agacaagaac tccccttttt ttcccaaagg cattataaaa agaatgaagc 1800
ctccttagaa aaaaaattat acctcaatgt ccccaacaag attgcttaat aaattgtgtt 1860
tcctccaagc tattcaattc ttttaactgt tgtagaagac aaaatgttca caatatattt 1920
agttgtaaac caagtgatca aactacatat tgtaaagccc atttttaaaa tacattgtat 1980
atatgtgtat gcacagtaaa aatggaaact atattgacct aaaaaaaaaa aaa 2033

<210> 45
<211> 367
<212> DNA
<213> NM_005951.1| Homo sapiens metallothionein 1H (MT1H), mRNA

<400> 45

ctccagtctc acctcggctt gcaatggacc ccaactgctc ctgcgaggct ggtggctcct 60
gcgcctgcgc cggctcctgc aagtgcaaaa agtgcaaatg cacctcctgc aagaagagct 120

gctgctcctg ttgcccctg ggctgtgcca agtgtgccca gggctgcatc tgcaaagggg 180
cgtcagagaa gtgcagctgc tgtgcctgat gtcgggacag ccctgctgtc agatgaaaac 240
agaatgacac gtaaaatccg aggtttttt tttctacaac tccgactcat ttgctacatt 300
cctttttttc tgtgaaatat gtgaataata attaaacact tagacttgaa aaaaaaaaaa 360
aaaaaaa 367

<210> 46
<211> 3052
<212> DNA
<213> NM_000767.4| Homo sapiens cytochrome P450, family 2, subfamily B, polypeptide 6 (CYP2B6), mRNA

<400> 46

caggaccatg gaactcagcg tcctcctctt ccttgcactc ctcacaggac tcttgctact 60
cctggttcag cgccacccta acacccatga ccgcctccca ccagggcccc gccctctgcc 120
ccttttggga aaccttctgc agatggatag aagaggccta ctcaaatcct ttctgaggtt 180
ccgagagaaa tatggggacg tcttcacggt acacctggga ccgaggcccg tggtcatgct 240
gtgtggagta gaggccatac gggaggccct tgtggacaag gctgaggcct tctctggccg 300
gggaaaaatc gccatggtcg acccattctt ccggggatat ggtgtgatct ttgccaatgg 360
aaaccgctgg aaggtgcttc ggcgattctc tgtgaccact atgagggact tcgggatggg 420
aaagcggagt gtggaggagc ggattcagga ggaggctcag tgtctgatag aggagcttcg 480
gaaatccaag ggggccctca tggaccccac cttcctcttc cagtccatta ccgccaacat 540
catctgctcc atcgtctttg gaaaacgatt ccactaccaa gatcaagagt tcctgaagat 600
gctgaacttg ttctaccaga ctttttcact catcagctct gtattcggcc agctgtttga 660
gctcttctct ggcttcttga aatactttcc tggggcacac aggcaagttt acaaaaacct 720
gcaggaaatc aatgcttaca ttggccacag tgtggagaag caccgtgaaa ccctggaccc 780
cagcgccccc aaggacctca tcgacaccta cctgctccac atggaaaaag agaaatccaa 840
cgcacacagt gaattcagcc accagaacct caacctcaac acgctctcgc tcttctttgc 900
tggcactgag accaccagca ccactctccg ctacggcttc ctgctcatgc tcaaataccc 960
tcatgttgca gagagagtct acagggagat tgaacaggtg attggcccac atcgccctcc 1020
agagcttcat gaccgagcca aaatgccata cacagaggca gtcatctatg agattcagag 1080
attttccgac cttctcccca tgggtgtgcc ccacattgtc acccaacaca ccagcttccg 1140
agggtacatc atccccaagg acacagaagt atttctcatc ctgagcactg ctctccatga 1200
cccacactac tttgaaaaac cagacgcctt caatcctgac cactttctgg atgccaatgg 1260
ggcactgaaa aagactgaag cttttatccc cttctcctta gggaagcgga tttgtcttgg 1320

tgaaggcatc gcccgtgcgg aattgttcct cttcttcacc accatcctcc agaacttctc 1380
catggccagc cccgtggccc cagaagacat cgatctgaca ccccaggagt gtggtgtggg 1440
caaaataccc ccaacatacc agatccgctt cctgccccgc tgaaggggct gagggaaggg 1500
ggtcaaagga ttccagggtc attcagtgtc cccgcctctg tagacaatgg ctctgactcc 1560
ccgcaacttc ctgcctctga gagacctgct acaagccagc ttccttcccc tccatggcac 1620
cagttgtctg aggtcacatt gcaagtgagt gcaggagtga gattatcgaa aattataata 1680
tacaaaatca tatatatata tatgttcttg tttttgaga cagagtctca cactgttgcc 1740
caggctggag tgcagtggcg tgatctcggc tcactgcaac ctccaccccc ggggatcaag 1800
caactctcct gcctcagcct ccctagtagc tgggattaca ggcatgcact accacgcttg 1860
gctaattttt gtatttttag tagagatggg gtttcactgt gtaggccagg ctggtctcga 1920
actcctgaac tcaagtgatt cacccacctt agcctcccaa agtgctggga ttacaggcgt 1980
gagtcaccgt gcccagccat gtatatatat aattttaaaa attaagctga aattcacata 2040
acataaaatt agctgtttta aagtgtaaaa tttagtggcg tgtggttcat tcacaaagct 2100
gtacaaccac caccatctag ttccaaacat tttcttttt tctgagatgg agtctcactc 2160
tgtcacccag gttcgagttc agtggtgcca tctctgtcca ctgcaacctc cacatcctgg 2220
gttcaagtga ttctcctgcc tcagcctctg gaggagctgg tatcacaggc gtcccccacc 2280
acgcctggct aaattttgta tttttaggtg gtcttgaact cctgatgtca ggtgattctc 2340
ctagctccaa atgttttcat tatctctccc ccaacaaaac ccatacctat caagctgtca 2400
ctccccatac cccattctct ttttcatctc ggcccctgtc aatctggttt ttgtcactat 2460
ggacttacca attctgaata tttcccataa acagaatcat acaatatttg atttttttt 2520
ttttttgaa actaagcctt gctctgtctc ccaggctgga gtgctatggt gcaatttttg 2580
ttcactgcaa cctctgcctt ccaagatcaa gagattctcc agtctcagct cccaagtagc 2640
tgggattaca ggcatgtact accatgcctg gctaattttc ttgtagtttt agtagggaca 2700
tgttggccag gctggtggtg agctcctggc ctcaggtgat ccacccacct cagtgttcca 2760
aagtgctgat attacaggca taatatgtga tcttttgtgt ctggttgctt tcatgttgaa 2820
tgctattttt gaggttcatg cctgttgtag accacagtca cacactgctg tagtcttccc 2880
cagtcctcat tcccagctgc ctcttcctac tgcttccgtc tatcaaaaag ccccccttggc 2940
ccaggttccc tgagctgtgg gattctgcac tggtgctttg gattccctga tatgttcctt 3000
caaatctgct gagaattaaa taaacatctc taaagcctga cctccccacg tc 3052

<210> 47
<211> 1645
<212> DNA
<213> NM_003811.2| Homo sapiens tumor necrosis factor (ligand) superfamily, member 9 (TNFSF9), mRNA

<400> 47

```
agtctctcgt catggaatac gcctctgacg cttcactgga ccccgaagcc ccgtggcctc     60
ccgcgccccg cgctcgcgcc tgccgcgtac tgccttgggc cctggtcgcg gggctgctgc    120
tgctgctgct gctcgctgcc gcctgcgccg tcttcctcgc ctgcccctgg gccgtgtccg    180
gggctcgcgc ctcgcccggc tccgcggcca gcccgagact ccgcgagggt cccgagcttt    240
cgcccgacga tcccgccggc ctcttggacc tgcggcaggg catgtttgcg cagctggtgg    300
cccaaaatgt tctgctgatc gatgggcccc tgagctggta cagtgaccca ggcctggcag    360
gcgtgtccct gacgggggc ctgagctaca aagaggacac gaaggagctg gtggtggcca    420
aggctggagt ctactatgtc ttctttcaac tagagctgcg gcgcgtggtg gccggcgagg    480
gctcaggctc cgtttcactt gcgctgcacc tgcagccact gcgctctgct gctggggccg    540
ccgccctggc tttgaccgtg gacctgccac ccgcctcctc cgaggctcgg aactcggcct    600
tcggtttcca gggccgcttg ctgcacctga gtgccggcca gcgcctgggc gtccatcttc    660
acactgaggc cagggcacgc catgcctggc agcttaccca gggcgccaca gtcttgggac    720
tcttccgggt gacccccgaa atcccagccg gactcccttc accgaggtcg gaataacgcc    780
cagcctgggt gcagcccacc tggacagagt ccgaatccta ctccatcctt catggagacc    840
cctggtgctg ggtccctgct gctttctcta cctcaagggg cttggcaggg gtccctgctg    900
ctgacctccc cttgaggacc ctcctcaccc actccttccc caagttggac cttgatattt    960
attctgagcc tgagctcaga taatatatta tatatattat atatatatat atatttctat   1020
ttaaagagga tcctgagttt gtgaatggac tttttagag gagttgtttt gggggggggg   1080
tcttcgacat tgccgaggct ggtcttgaac tcctggactt agacgatcct cctgcctcag   1140
cctcccaagc aactgggatt catcctttct attaattcat tgtacttatt tgcctatttg   1200
tgtgtattga gcatctgtaa tgtgccagca ttgtgcccag gctagggggc tatagaaaca   1260
tctagaaata gactgaaaga aaatctgagt tatggtaata cgtgaggaat ttaaagactc   1320
atccccagcc tccacctcct gtgtgatact tgggggctag ctttttttctt tctttctttt   1380
ttttgagatg gtcttgttct gtcaaccagg ctagaatgca gcggtgcaat catgagtcaa   1440
tgcagcctcc agcctcgacc tcccgaggct caggtgatcc tcccatctca gcctctcgag   1500
tagctgggac cacagttgtg tgccaccaca cttggctaac tttttaattt ttttgcggag   1560
acggtattgc tatgttgcca aggttgttta catgccagta caatttataa taaacactca   1620
tttttcctca aaaaaaaaaa aaaaa                                         1645
```

<210> 48
<211> 6640
<212> DNA
<213> NM_006047.4| Homo sapiens RNA binding motif protein 12 (RBM12), transcript variant 1, mRNA

<400> 48

gagcgggccg acggccattt tgtgaagcgg cgaaggaggt ggtggctgcg ttgggctccg 60
ggaagccgtt cgggctgggg ctgtcggccg cggggcggag gcactcgcgc ggggggtaat 120
tcggggtctg ggttctggtg ccgcgcagct ttccccgtct aaaagttggt tttaattggt 180
tgcccacagg attgacttgg cctctacttc ttgttaagga aattcatctc ttgttttatc 240
aggtgtgtgt ggtttcagcg cagcatggct gtggtcatcc gtttgcaagg tctcccaatt 300
gtggcgggga ccatggacat tcgccacttc ttctctggat tgaccattcc tgatgggggc 360
gtgcatattg taggggggtga actgggtgag gctttcatcg tttttgccac tgatgaagat 420
gcaaggcttg gtatgatgcg cacaggtggt acaattaaag ggtcaaaagt aacactattg 480
ttgagtagta agacggaaat gcagaatatg attgaactga gtcgtaggcg ttttgaaact 540
gccaacttag atataccacc agcaaatgcc agtagatcag gaccaccacc tagctcagga 600
atgagtagca gggtaaactt gcccacaaca gtatccaact ttaataatcc atcacccagt 660
gtagttactg ccaccacttc tgttcatgaa agcaacaaaa acatacagac attttccaca 720
gccagcgtag gaacagctcc tccaaatatg gggcttcct ttgggagccc aacgtttagc 780
tcaactgttc caagcacagc ctctccaatg aacacagtcc cgccgccacc aattcctcca 840
attccagcga tgccatctct gccaccaatg ccatccattc ccccaattcc agttcctcct 900
ccagtaccta cattgcctcc tgtgcctcct gtgcccccga ttcccccagt tccttctgtg 960
ccacccatga ccccactgcc acccatgtcg ggcatgccgc ccttgaatcc gccacctgtg 1020
gcacctctac ctgctggaat gaatggctct ggagcaccta tgaatttgaa caataatctg 1080
aatcctatgt ttcttggtcc gttgaatcct gttaacccta tccagatgaa ctctcagagc 1140
agtgtgaagc cactccccat caaccctgat gatctgtatg tcagtgtgca tggaatgccc 1200
ttttctgcaa tggaaaatga tgtcagagat ttttttcatg ggctccgtgt tgatgcagtg 1260
catttgttga aagatcatgt aggtcgaaat aatgggaatg gattggttaa gtttctctcc 1320
cctcaagata catttgaagc tttgaaacga aacagaatgc tgatgattca acgctatgtg 1380
gaagttagcc ctgccacaga aagacagtgg gtagctgctg gaggccatat cacttttaag 1440
caaaatatgg gaccttctgg acaaactcat ccccctcctc agacacttcc caggtcaaaa 1500
tcgcccagtg ggcagaaaag atcaaggtca agatcaccac atgaggctgg tttttgtgtt 1560
tacttgaaag ggctaccatt tgaagcagaa aacaaacatg tcattgattt ttttaaaaag 1620
ctggatattg tggaagatag tatttatata gcttatggac ccaatgggaa agcaactggc 1680
gaaggctttg tagagttcag aaatgaggct gactataagg ctgctctgtg tcgtcataaa 1740
cagtacatgg gcaatcgctt tattcaagtt catccaatta ctaagaaagg tatgctagaa 1800

aagatagata tgattcgaaa aagactgcag aacttcagct atgaccagag ggaaatgata 1860
ctaaatccag agggggatgt caactctgcc aaagtctgtg cccacataac aaatattcca 1920
ttcagcatta caaagatgga tgttcttcag ttcctagaag gaatcccagt ggatgaaaat 1980
gctgtacatg ttcttgttga taacaatggg caaggtctag gacaggcatt ggttcagttt 2040
aaaaatgaag atgatgcacg taagtctgaa cgcttacacc gtaaaaaact taatgggaga 2100
gaagcttttg ttcatgtagt taccctagaa gatatgagag agattgagaa aaatcccccct 2160
gcccaaggaa aaaagggatt aaagatgcct gtgccaggta atcctgcagt tccaggaatg 2220
cccaatgcgg gactgcccgg tgtgggactg cccagtgcag gacttcccgg tgcaggcctg 2280
cccagcacag gactgcctgg ttcagcaata accagtgcag gactgcctgg tgcgggaatg 2340
cccagtgcag gaatacctag tgcaggaggt gaagagcatg ccttcctgac tgtaggatca 2400
aaggaagcca ataatgggcc tccatttaac tttcctggta attttggtgg atcaaatgcc 2460
tttgggccac caatccctcc tccaggatta ggaggcgggg cctttggtga tgctaggcct 2520
ggtatgcctt cagttggaaa cagtggtttg cctggtctag gactggatgt tccgggtttt 2580
ggaggtggac caaacaattt aagtgggcca tcgggatttg gagggggccc tcagaatttt 2640
ggaaatggcc ctggtagctt aggcggtccc ccggggtttg gaagtggccc tcctggtctt 2700
ggaagtgccc ctgggcattt gggtgggcca ccagcttttg ggcctggccc cggccccggc 2760
cccggccctg gcccaatcca tattggtggt ccccctggct ttgcatctag ttctggaaaa 2820
ccaggaccga cagtaattaa agtgcaaaac atgcccttta ctgtgtctat tgatgagatt 2880
ttagatttct tttatggcta tcaagtaatc ccaggctcag tgtgtttaaa atacaatgaa 2940
aaaggtatgc ccacaggtga agccatggtg gcctttgagt ctcgggatga agccacagct 3000
gctgtcattg acttaaatga caggcctata ggttcaagaa aagtaaaact tgtattaggg 3060
tagccattca catcattttt tatagggtag atcttcatat tgctgtgatt aatgcatcca 3120
gattgttttc ctagtatttc caggttagaa cctgtggatt gtttcaattg catatagctt 3180
ggtttccata acatagagca ttggttgact gtttacagaa gactcactca ccaggataaa 3240
cattgctgta tgttacagta aagctatctg gagagaacac ataaatgatt ttggcatacc 3300
attagagaaa ccatttgtaa aactcaaatg accacataaa gcttatcaag gagtctagat 3360
tggttttgtt ttataccata tgggatgaag aaaatagaaa tgtcagtaga actcattgag 3420
ggtgctcttg ccagctgctg aaaatagaag ttggctactc tcagaatttg gtttaaagct 3480
ggacagattt gctttgttat agggtaaagc tttgtctaaa gtcctcattt tcttttaaaa 3540
ttgaataaaa tttctgtata cagattcatt gtatgtacct ttattgcttc ttaagggtcc 3600
ttgctgtata gacagtcctg cttcagaagt tgctgctttg tttgtctaat tgactcattt 3660
gtaaatgagc agaactgttt tgttggtttt tttccctaaa tataaaagtc cacacttcgt 3720
ttgtgctata acctcaaact ttgatttcta atgtcacact taaaactgtg tggaataaga 3780

cttttgccat aaaaataaac tatggagtcc tttatctacc agagcctttt tggtttgacc 3840
gccacgattt aggttagtca gtttaaaaat tgttcatgtt gtttggatgg tatcgaaaac 3900
cagaaaccac ttttaataat gtgtttaaga tacttgattt gaagtccttt tcatatggac 3960
taattgtagt atcaatttcc tcctgtcccg attatgtgaa attttggcct ttaaacaaag 4020
aggggcccat tcataagaaa gtgttatatc taggttttta aaactgaagt tgaaattatc 4080
tttgttagca gtagtagtat agaataaaag atccgtatgc tggttcgtag attgatacgt 4140
gttagtcctg ttatttggag gctttttggc atagttgttc gatcaggagc ctgtttacta 4200
aaagtcttca tacagagtac aagtgcagcc gccagaggag aaaattgaga ttcttgaccc 4260
tttcatactc ttttctttgg tattcaggac actaaggcag gaggaccaca tgagttctgg 4320
ttggtaagtg tccttgtcat gaaaacactt gccttacaag gctctaatta tgatttccct 4380
agtcagtgct ctgaagatgt gtcacattat attataaaca atacggaagg ggagataggt 4440
gagatgatat gaaaaacaaa ttttctcact gtcataaaag gctctaatta tggttacttt 4500
ccttgtgatg aaaaacttgg ccttacaggg ctctagttat ggttactttc cctagtcaat 4560
gctctgaaaa tgtgtcacat tataaacaat acagaaggag agatagatga gagatcatga 4620
aaaccaaatt ttatctttta catggcccct ttgtcttcgt ttgaaacatc cccaacattt 4680
cctacaaatc agcgtagtta caaaggggtc agtcttttaa aataagtatt tcctattaaa 4740
ctatatatat atacagtgcc tttttggtgt tgtgagtcag tggaacactg aaatacagcg 4800
gttgtgtaat ttaagagtgg caacagtttc atttgataag atttgaaaag gctttttatc 4860
actacaatct tagaggattg acagtacagg atttttgttt aagagaagga ttgtttagac 4920
tcaagaggtg actatgttgt gggtcttttg ataattcatg aatacagatt tgctttgacc 4980
gatcactaga tactgcctcc tcaatttcaa aagcaatata acgtttgtat atgctgttta 5040
atttaagtta atgttaagta atcatttctc attccaaaga caatgcaaaa aacttcagca 5100
ctgcttgaga gttgtatttc agccacagat attttctcca ttggaaagct attttcattt 5160
tagaaaaatg ggttgtttga agatgaaagt cttttatctt ttttcacaat ttattttggt 5220
tatatgttcg tacattctta ttaaatattt catacacttt attgcaacta ctttgttatt 5280
tctacatctt agataaatgc tgaaaaggaa aacgatttca ttgttcatct aattaaataa 5340
attaaaagag cggtttgtgt agaaattgga gagaactatg ttttatatga atcacaacag 5400
tgctcgttgt ggactgtaat taaatgcttt gccctctgga acttgatttt tgtgtatctg 5460
agatttatta acagtgctaa ctgctaagga tactgtttat cttgttctgg gcattggagt 5520
ttcagttttt aaaaaattct tgaaaatgta ttctgtgaaa ctactcatac ctctcttcct 5580
gcgaattttc tcctaagaac taggtttggg gtagaaatga attgacatta ttttctcatt 5640
tgctttgtat ggtatgaagg atttgtaaag ctttgctcaa agttgtgtgc atttgtaaac 5700
actatcatga tattttcaat tttatgtgta aattttattg tctgtttttg gtgactctga 5760
cattaatgga agagaatatt ttccattaga tttaattttt tttcctctcc cttctgattt 5820

tttttttatt ggtgttcatt tttcttttga tttaaaggat tagagaaatc tacaaatgta 5880
tgtcataaat aagcaaattt gtaaactttt ctgaacttta gtgaaacatt tagttcacaa 5940
gcataatttg gaggtgtgtt ctgtgtttac acagtagttt ggatgtacaa ttattattag 6000
tggcttttta aaaaatgaaa cagtgttaag tgaaatgtag ttcctagctt tgtactccaa 6060
gttgtcaaag catcaacaat gaaaattcga ttaggaaact ttatttaaaa tttcaggtag 6120
taatattcag tgtagttaag gccagtctta acccactgga tgaaaatcta ggactgtatg 6180
gaagtaagca aacattacat ttttaggtgg aaatagtcag ccttgcataa aaacaaggat 6240
gcgtgaaagc cttaaattcc agctcccttt tactggagtc tgtggttgtg tacaggtatg 6300
ggccaagtgt aaaatctcat caattttaag aacactcggg aaatgagtaa agaaaatgta 6360
aaattgctgc tagtcaaatc ttttggaaag aatttctgga agtggtcact ttaaaaatta 6420
ttttccaccc ttgcaaaatt gccacattta aattgtttta ctggcagttc tatagtagtc 6480
cagactttag aaaccaaaca caacaaaatg gcttgttgcc aatatggcca caacattgcc 6540
agcaaatact gccttggcat cactcagcag aggtttttgt ttataaagat gaagtcttga 6600
atactgttca ataaacttgt caaaaaataa aaaaaaaaaa 6640

<210> 49
<211> 3680
<212> DNA
<213> NM_006644.2| Homo sapiens heat shock 105kDa/110kDa protein 1 (HSPH1), mRNA

<400> 49

tgagtaaatg ccgcagattc tggaaagttc tgatcagtgc gatacataag gctgaggaag 60
tgggacctcc ccttttgggt cggtagttca gcgccggcgc cggtgtgcga gccgcggcag 120
agtgaggcag gcaacccgag gtgcggagcg acctgcggag gctgagcccc gctttctccc 180
agggtttctt atcagccagc cgccgctgtc cccgggggag taggaggctc ctgacaggcc 240
gcggctgtct gtgtgtcctt ctgagtgtca gaggaacggc cagaccccgc gggccggagc 300
agaacgcggc cagggcagaa agcggcggca ggagaagcag gcagggggcc ggaggacgca 360
gaccgagacc cgaggcggag gcggaccgcg agccggccat gtcggtggtg gggttggacg 420
tgggctcgca gagctgctac atcgcggtag cccgggccgg gggcatcgag accatcgcca 480
atgagttcag cgaccggtgc accccgtcag tcatatcatt tggatcaaaa aatagaacaa 540
tcggagttgc agccaaaaat cagcaaatca ctcatgcaaa caatacggtg tctaacttca 600
aaagatttca tggccgagca ttcaatgacc ccttcattca aaaggagaag gaaaacttga 660
gttacgattt ggttccattg aaaaatggtg gagttggaat aaaggtaatg tacatgggtg 720
aagaacatct atttagtgtg gagcagataa cagccatgtt gttgactaag ctgaaggaaa 780

ctgctgaaaa cagcctcaag aaaccagtaa cagattgtgt tatttcagtc ccctccttct 840
ttacagatgc tgagaggcga tctgtgttag atgctgcaca gattgttggc ctaaactgtt 900
taagacttat gaatgacatg acagctgttg ctttgaatta cggaatttat aagcaggatc 960
tcccaagcct ggatgagaaa cctcggatag tggtttttgt tgatatggga cattcagctt 1020
ttcaagtgtc tgcttgtgct tttaacaagg gaaaattgaa ggtactggga acagcttttg 1080
atcctttctt aggaggaaaa aacttcgatg aaaagttagt ggaacatttt tgtgcagaat 1140
ttaaaactaa gtacaagttg gatgcaaaat ccaaaatacg agcactccta cgtctgtatc 1200
aggaatgtga aaaactgaaa aagctaatga gctctaacag cacagacctt ccactgaata 1260
tcgaatgctt tatgaatgat aaagatgttt ccggaaagat gaacaggtca caatttgaag 1320
aactctgtgc tgaacttctg caaaagatag aagtacccct ttattcactg ttggaacaaa 1380
ctcatctcaa agtagaagat gtgagtgcag ttgagattgt tggaggcgct acacgaattc 1440
cagctgtgaa ggaaagaatt gccaaattct ttggaaaaga tattagcaca acactcaatg 1500
cagatgaagc agtagccaga ggatgtgcat tacagtgtgc aatactttcc ccggcattta 1560
aagttagaga attttccgtc acagatgcag ttcctttttcc aatatctctg atctggaacc 1620
atgattcaga agatactgaa ggtgttcatg aagtctttag tcgaaaccat gctgctcctt 1680
tctccaaagt tctcaccttt ctgagaaggg ggccttttga gctagaagct ttctattctg 1740
atccccaagg agttccatat ccagaagcaa aaataggccg ctttgtagtt cagaatgttt 1800
ctgcacagaa agatggagaa aaatctagag taaaagtcaa agtgcgagtc aacacccatg 1860
gcattttcac catctctacg gcatctatgg tggagaaagt cccaactgag gagaatgaaa 1920
tgtcttctga agctgacatg gagtgtctga atcagagacc accagaaaac ccagacactg 1980
ataaaaatgt ccagcaagac aacagtgaag ctggaacaca gccccaggta caaactgatg 2040
ctcaacaaac ctcacagtct ccccccttcac ctgaacttac ctcagaagaa aacaaaatcc 2100
cagatgctga caaagcaaat gaaaaaaaag ttgaccagcc tccagaagct aaaaagccca 2160
aaataaaggt ggtgaatgtt gagctgccta ttgaagccaa cttggtctgg cagttaggga 2220
aagaccttct taacatgtat attgagacag agggtaagat gataatgcaa gataaattgg 2280
aaaaagaaag gaatgatgct aaaaatgcag ttgaggaata tgtgtatgag ttcagagaca 2340
agctgtgtgg accatatgaa aaatttatat gtgagcagga tcatcaaaat tttttgagac 2400
tcctcacaga aactgaagac tggctgtatg aagaaggaga ggaccaagct aaacaagcat 2460
atgttgacaa gttggaagaa ttaatgaaaa ttggcactcc agttaaagtt cggtttcagg 2520
aagctgaaga acggccaaaa atgtttgaag aactaggaca gaggctgcag cattatgcca 2580
agatagcagc tgacttcaga aataaggatg agaaatacaa ccatattgat gagtctgaaa 2640
tgaaaaaagt ggagaagtct gttaatgaag tgatggaatg gatgaataat gtcatgaatg 2700
ctcaggctaa aaagagtctt gatcaggatc cagttgtacg tgctcaggaa attaaaacaa 2760

aaatcaagga attgaacaac acatgtgaac ccgttgtaac acaaccgaaa ccaaaaattg 2820
aatcacccaa actggaaaga actccaaatg gcccaaatat tgataaaaag gaagaagatt 2880
tagaagacaa aaacaatttt ggtgctgaac ctccacatca gaatggtgaa tgttacccta 2940
atgagaaaaa ttctgttaat atggacttgg actagataac cttaaattgg cctattcctt 3000
caattaataa aatatttttg ccatagtatg tgactctaca taacatactg aaactattta 3060
tattttcttt tttaaggata tttagaaatt ttgtgtatta tatggaaaaa gaaaaaaagc 3120
ttaagtctgt agtctttatg atcctaaaag ggaaaattgc cttggtaact ttcagattcc 3180
tgtggaattg tgaattcata ctaagctttc tgtgcagtct caccatttgc atcactgagg 3240
atgaaactga cttttgtctt ttggagaaaa aaaactgtac tgcttgttca agagggctgt 3300
gattaaaatc tttaagcatt tgttcctgcc aaggtagttt tcttgcattt tgctctccat 3360
tcagcatgtg tgtgggtgtg gatgtttata aacaagacta agtctgactt cataagggct 3420
ttctaaaacc atttctgtcc aagagaaaat gactttttgc tttgatatta aaaattcaat 3480
gagtaaaaca aaagctagtc aaatgtgtta gcagcatgca gaacaaaaac tttaaacttt 3540
ctctctcact atacagtata ttgtcatgtg aaagtgtgga atggaagaaa tgtcgatcct 3600
gttgtaactg attgtgaaca cttttatgag ctttaaaata aagttcatct tatggtgtca 3660
tttctaaaaa aaaaaaaaaa 3680

<210> 50
<211> 3349
<212> DNA
<213> NM_004602.1| Homo sapiens staufen, RNA binding protein (Drosophila) (STAU), transcript variant T4, mRNA

<400> 50

acttcctgcc gggctgcggg cgcctgagcg ctcttcagcg tttgcgcggc ggctgcgcgt 60
ctctctcggc tcccgcttcc tttgaccgcc tccccccccc ggcccggcgg cgcccgcctc 120
ctccacggcc actccgcctc ttccctccct tcgtcccttc ttcctctccc ttttttcctt 180
cttccttccc ctcctcgccg ccaccgccca ggaccgccgg ccgggggacg agtccggagc 240
agcagccagc agcagccagg tggagttttg ctcttgtcgc ccaggctgga gtgcagtggc 300
gtgatctcgg ctcactgcaa cctccacctc ccaggtcagc gattttccca cttcagcctc 360
ccgataagct gagattacag gagtttatta accacttaac ctctcagaac tgaacaaaga 420
caacattgtt cctggaacgc cctcttttta aaaaagaaag cataacccct actgtagaac 480
taaatgcact gtgcatgaaa cttggaaaaa aaccaatgta taagcctgtt gacccttact 540
ctcggatgca gtccacctat aactacaaca tgagaggagg tgcttatccc ccgaggtact 600
tttacccatt tccagttcca cctttacttt atcaagtgga actttctgtg ggaggacagc 660

aatttaatgg caaaggaaag acaagacagg ctgcgaaaca cgatgctgct gccaaagcgt 720
tgaggatcct gcagaatgag cccctgccag agaggctgga ggtgaatgga agagaatccg 780
aagaagaaaa tctcaataaa tctgaaataa gtcaagtgtt tgagattgca cttaaacgga 840
acttgcctgt gaatttcgag gtggcccggg agagtggccc accccacatg aagaactttg 900
tgaccaaggt ttcggttggg gagtttgtgg gggaaggtga agggaaaagc aagaagattt 960
caaagaaaaa tgccgccata gctgttcttg aggagctgaa gaagttaccg cccctgcctg 1020
cagttgaacg agtaaagcct agaatcaaaa agaaaacaaa acccatagtc aagccacaga 1080
caagcccaga atatggccag gggatcaatc cgattagccg actggcccag atccagcagg 1140
caaaaaagga gaaggagcca gagtacacgc tcctcacaga gcgaggcctc ccgcgccgca 1200
gggagtttgt gatgcaggtg aaggttggaa accacactgc agaaggaacg ggcaccaaca 1260
agaaggtggc caagcgcaat gcagccgaga acatgctgga gatccttggt ttcaaagtcc 1320
cgcagcggca gcccaccaaa cccgcactca agtcagagga gaagacaccc ataaagaaac 1380
caggggatgg aagaaaagta acctttttg aacctggctc tggggatgaa aatgggacta 1440
gtaataaaga ggatgagttc aggatgcctt atctaagtca tcagcagctg cctgctggaa 1500
ttcttcccat ggtgcccgag gtcgcccagg ctgtaggagt tagtcaagga catcacacca 1560
aagattttac cagggcagct ccgaatcctg ccaaggccac ggtaactgcc atgatagccc 1620
gagagttgtt gtatgggggc acctcgccca cagccgagac cattttaaag aataacatct 1680
cttcaggcca cgtaccccat ggacctctca cgagaccctc tgagcaactg gactatcttt 1740
ccagagtcca gggattccag gttgaataca aagacttccc caaaaacaac aagaacgaat 1800
ttgtatctct tatcaattgc tcctctcagc cacctctgat cagccatggt atcggcaagg 1860
atgtggagtc ctgccatgat atggctgcgc tgaacatctt aaagttgctg tctgagttgg 1920
accaacaaag tacagagatg ccaagaacag gaaacggacc aatgtctgtg tgtgggaggt 1980
gctgaacctt ttctggccat gaaccattat aaaatcccaa catatatact gaaaatactg 2040
aaactgcttt gaaaatttgg aatttctgat acctccagtg ggccgagaga cacggtgggt 2100
aaaggatgtg ggcagcagca gggaagacaa cagaaacaca aggaggcggc tgtggccggc 2160
tggactgtgc tggggtttgt tgtgatggcc actcggtgac ctggcggtcc ctacgcaata 2220
gcagctgcct gtggggaaga agggctgccc agccagctgg ttctcccggg acaccagcag 2280
atccacaccc tgggcacctc cgtgtttggt cttttttttc ccctgtgtga aagaagaaac 2340
ggcacgaccc cttctcaagc tggctcactc agacacattg ggacaaaccc tggacagcca 2400
tgccagagag aggcctttga ccggccccag agctaaaagc accagagaaa atcaaatgct 2460
tcctactcag cgtgacccaa cttttctagt gtgccacggc cccaccacct cctgcagtac 2520
ccacaccatc accactgctt tctcttccaa cagtgatctg tattcttagt ttcattattt 2580
tcttttgatt gatatgacac tatataaaat tttcatttga gaatttctca attgtatcta 2640
gttaaatagc acagtttgga aacttgtctg agactgactt tatcaataat ctaaccgaca 2700

aagatcatat ccatgtgtat gtggttagac atttttattt cattgactaa cccaggacag 2760
tttcagtgat gcaaattgtg tgccctctgg ttcagctgaa acagtcctgg actttcaaaa 2820
accttgaata agtctcccac agttgtataa attggacaat ttaggaattt taaactttag 2880
atgatcattt ggttccattt ttatttcatt tttatttttg ttaatgcaaa caggacttaa 2940
atgaactttg atctctgttt taaagattat taaaaaacat tgtgtatcta tacatatggc 3000
tcttgaggac ttagctttca ctacactaca ggatatgatc tccatgtagt ccatataaac 3060
ctgcagagtg attttccaga gtgctcgata ctgttaatta catctccatt agggctgaaa 3120
agaatgacct acgtttctgt atacagctgt gttgcttttg atgttgtgtt actgtacaca 3180
gaagtgtgtg cactgaggct ctgcgtgtgg tccgtatgga aaacctggta gccctgcgag 3240
ttaagtactg cttccattca ttgtttacgc tggaattttt ctccccatgg aatgtaagta 3300
aaacttaagt gtttgtcatc aataaatggt aatactaaaa aaaaaaaaa 3349

<210> 51
<211> 402
<212> DNA
<213> NM_021246.2| Homo sapiens lymphocyte antigen 6 complex, locus G6D (LY6G6D), transcript variant 1, mRNA

<400> 51

atgaaacccc agtttgttgg gatcttgctc agctccctgc taggggctgc cttgggaaac 60
cgaatgcggt gctacaactg tggtggaagc cccagcagtt cttgcaaaga ggccgtgacc 120
acctgtggcg agggcagacc ccagccaggc ctggaacaga tcaagctacc tggaaacccc 180
ccagtgacct tgattcacca acatccagcc tgcgtcgcag cccatcattg caatcaagtg 240
gagacagagt cggtgggaga cgtgacttat ccagcccaca gggactgcta cctgggagac 300
ctgtgcaaca gcgccgtggc aagccatgtg gcccctgcag gcattttggc tgcagcagct 360
accgccctga cctgtctctt gccaggactg tggagcggat ag 402

<210> 52
<211> 3248
<212> DNA
<213> NM_007236.3| Homo sapiens calcium binding protein P22 (CHP), mRNA

<400> 52

accacccctg ggttccctcc cgggtccgca gtggaaacac tgccctctcc cttcttgacc 60
cctagccctt ccttccctcc ctccttccct cctgtcgccg tctcttctgg cgccgctgct 120
cccggaggag ctcccggcac ggcgatgggt tctcgggcct ccacgttact gcgggacgaa 180

gagctcgagg agatcaagaa ggagaccggc ttttcccaca gtcaaatcac tcgcctctac 240
agccggttca ccagcctgga caaaggagag aatgggactc tcagccggga agatttccag 300
aggattccag aacttgccat caacccactg gggaccgga tcatcaatgc cttctttcca 360
gagggagagg accaggtaaa cttccgtgga ttcatgcgaa ctttggctca tttccgcccc 420
attgaggata atgaaaagag caaagatgtg aatggacccg aaccactcaa cagccgaagc 480
aacaaactgc actttgcttt tcgactatat gatttggata aagatgaaaa gatctcccgt 540
gatgagctgt tacaggtgct acgcatgatg gtcggagtaa atatctcaga tgagcagctg 600
ggcagcatcg cagacaggac cattcaggag gctgatcagg atggggacag tgccatatct 660
ttcacagaat ttgttaaggt tttggagaag gtggatgtag aacagaaaat gagcatccga 720
tttcttcact aaaggagacc aaactgttcc ttgcggtcta gtatttaaga actggaactt 780
gaaagtcctc cttctaccaa ctccacctcc acccctcat tcccttctc ccaaagtact 840
actgctgttg catgacaacc ccaaatatgt tctgtcaaca caaacctgcc tttggtgtat 900
aaacagggca ttacagaatg gtacacccta tatatttctg ttcagtatcc attcactagt 960
tcttcattta taaatatcat cttccccatt ctgctgctga atgccacaca tccatccagt 1020
ctgagaaagt gagagaggca atcatgccaa gaacaagcca gcaaagctct ttcaccagat 1080
gtagactgta gccctgctgc cttccctcca gcgagtctgc cagcatgctt cttcatcctt 1140
tttatatgtt ctttgcttcc tacttccctg tcttccaaca tactgttcac ttactctggc 1200
agtctttctg cttttcatta agcctcaaaa tctcctctgt tctacttggc accacaagct 1260
atgtcctata tatgtatttc tgacttggca ggatagttca ggggtctggc agtttttatt 1320
taccttcatt attaaatggg cctctgggat gttgcctctt caggagcttt ttggtaatca 1380
atacttctct cagaagtatg agaccatcct ctgcactctg ctctgtcatc aaaggctgct 1440
gggtggagat acccttttg aaaggtggcc ttggtgagag gtatggagcc aagtcttcta 1500
ggttgcttgc ccacatcact ctatctctgg cctctgattc tcaactttgt acctgtgtgg 1560
ctcctcttgt tagtgcaatg ttgactgttg aaaaagcagc agtatgctta caggtttgct 1620
tagtttgggg acaccgttac caccagaatg gctgctctga caatatgcct agggactttc 1680
tcatggcttt tatttaataa ggaggctggg caccctataa agcctcatgc attcacacct 1740
ttgcagcatg gtttatgcct cagtgttatg tgcactggaa tgttttccac ttcacatttc 1800
caagtagaaa tattagtgtt acggaagtgc ctaatatccc agtccaaatt tttttttttt 1860
tttttttttt tttttgagac agagtcttgc tctgtcaccc aggctggagt gcagtggtgc 1920
gatcgctcac tgcaacctca gcctcctgga tttaagtgat tctcctgcct cagcctccca 1980
agtagctggg attacaggtg tgcaccacca tgcccggcta attttttgta tttttagtgg 2040
agacagggtt tcaccatgtt ggccaggctg gtctcgaact cctgacctcg tgatccgcct 2100
gcctcagcct cccaaagtgc tgggattaca ggtgtgagcc accacgcctg gccccagtcc 2160

aaaatattta aagattgttt ccttagtgtc ttgaagtttt gcacaaaatt cttttttttg 2220
agatggagtc tcactctgtc acccaggctg gagtgcagtg gcgtgatctt ggctcactgc 2280
aacctctgcc tcctgggttc aagcaattct cccacctcag cctcccaagt agctgggatt 2340
acagacgtgt gccaccatac ctgggtaatt tttgcatttt tagtggagag ggagtttcac 2400
catgttggcc aggttggtct tgaactcctg acctcaggtg atcctcctgc ctcggcctcc 2460
caaagtgctg ggattacagg catgagccac cgtgctcagc cgcaaaattc tttatgaatt 2520
ttacacttgg caaatgttaa tgacggaagc catagtctgc tcctaataca tgtccaaagc 2580
attgactgtt gtgtcattag ctgcctggtt acattagctc cctggcttct tgtttagacc 2640
actgctaatc ccttaaaaac aagaggtctg gcactagtag cacaacctaa ggtggcatta 2700
cagatctttg agcgagccac agcaactttt ctgccaagtc agcttagttt agacttcagt 2760
gaatcaggct attgctatcc taatgtatgt ctctatgagt gtatttagcc acacatctgc 2820
ccttggttga ctttctgact cattgcttgc ttgcttgttt ccttgctttg gaaaactatt 2880
gaagattgct aaaaaatacc actgcaaagt gatggaaaag ggtggagaac aggggagtag 2940
ccaggctgga tggctcaaat ataaatgaat gaggaattct ttatgaagta tcagtcagat 3000
tttatgatta agtgatgtaa tataggaatt atgtaaaagg gaagaatgtc tgatactgat 3060
ctattagaga ggtactttag aggcttcttg attggcataa agttcctaag gttatagatt 3120
ttcccccctt ttggctgtat agcaaagtgt tttaatccac ggttgtgcct tattgttcca 3180
ttaaaattgt atcttcgatc catcaataaa tacttgtggt tgaaacaaaa aaaaaaaaaa 3240
aaaaaaaa 3248

<210> 53
<211> 3098
<212> DNA
<213> NM_003671.2| Homo sapiens CDC14 cell division cycle 14 homolog B (S. cerevisiae) (cDCl4B), transcript variant 1, mRNA

<400> 53

cacggaacag ccctcctggg gtccccacga gccgcgtcct gctgtgcccc ggcgcctacg 60
cagcagcggc cgcggccgcg gtgggcacgc acggttaccc cgggcagctc cggccgccag 120
ctgcagcccc gtcgcctcgg ccgcgccagc cggctgcggg cacctggggg cgggctgggg 180
gcgccggccg cggcaggagg cgctgtagcg agggctgcgg cgccggtcct gcggcggccg 240
cgggaggcag cggggcaggc gctgtgggcc gggctcctcc tccggctcct gcgcgaccgc 300
ctcccgccgg gctctgccgg cgcccgccgt ccccgcagcg ccgctctgcg cccgccgccc 360
cgagcgcccg cgcggggctg gcgggagcct cggcgggcgc gcgggcgcgc ggggccatgg 420
tcgtggcccc ctgacgggcc gcggccgcct ccatgaagcg gaaaagcgag cggcggtcga 480

gctgggccgc cgcgcccccc tgctcgcggc gctgctcgtc gacctcgccg ggtgtgaaga 540
agatccgcag ctccacgcag caagacccgc gccgccggga cccccaggac gacgtgtacc 600
tggacatcac cgatcgcctt tgttttgcca ttctctacag cagaccaaag agtgcatcaa 660
atgtacatta tttcagcata gataatgaac ttgaatatga gaacttctac gcagattttg 720
gaccactcaa tctggcaatg gtttacagat attgttgcaa gatcaataag aaattaaagt 780
ccattacaat gttaaggaag aaaattgttc attttactgg ctctgatcag agaaaacaag 840
caaatgctgc cttccttgtt ggatgctaca tggttatata tttggggaga accccagaag 900
aagcatatag aatattaatc tttggagaga catcctatat tcctttcaga gatgctgcct 960
atggaagttg caatttctac attacacttc ttgactgttt tcatgcagta aagaaggcaa 1020
tgcagtatgg cttccttaat ttcaactcat ttaaccttga tgaatatgaa cactatgaaa 1080
aagcagaaaa tggagattta aattggataa taccagaccg atttattgcc ttctgtggac 1140
ctcattcaag agccagactt gaaagtggtt accaccaaca ttctcctgag acttatattc 1200
aatattttaa gaatcacaat gttactacca ttattcgtct gaataaaagg atgtatgatg 1260
ccaaacgctt tacggatgct ggcttcgatc accatgatct tttctttgcg gatggcagca 1320
cccctactga tgccattgtc aaagaattcc tagatatctg tgaaaatgct gagggtgcca 1380
ttgcagtaca ttgcaaagct ggccttggtc gcacgggcac tctgatagcc tgctacatca 1440
tgaagcatta caggatgaca gcagccgaga ccattgcgtg ggtcaggatc tgcagacctg 1500
gctcggtgat tgggcctcag cagcagtttt tggtgatgaa gcaaaccaac ctctggctgg 1560
aaggggacta ttttcgtcag aagttaaagg ggcaggagaa tggacaacac agagcagcct 1620
tctccaaact tctctctggc gttgatgaca tttccataaa tggggtcgag aatcaagatc 1680
agcaagaacc cgaaccgtac agtgatgatg acgaaatcaa tggagtgaca caaggtgata 1740
gacttcgggc cttgaaaagc agaagacaat ccaaaacaaa cgctattcct ctcactctct 1800
ccatttcaag gactaaaaca gtcttgcgtt aagtaaaaac ctgtgaccag agctgaagga 1860
agactctagg actgaaaact gcaacagaaa ttagcacaat ttgaaaacaa aacaaaattg 1920
caaaagcctt agttgctttt tccacctaag aagttgatca atggagaaaa tgtccactgg 1980
agtttgaata atgaactttg agtttgggtg caagcaaatg actcagagaa gggtccagct 2040
ctcaagctga atgacaaaca tgctgttgta aatttagtct caggtgtaaa tacccaagcc 2100
ctctggtacc cagggagctg gctggtctgt ggtgcatgtg tgtccctgtg atggcaatca 2160
ttgtagttgc tggccttcag aagaattgag gatctgatgg aggtttttta tgtatttatt 2220
ttctgttcac cttgtgaccc tgtgtcaaaa tttataaaga tacaaaaggc attactgaaa 2280
tggtactttc tgtaatttga tactatttgg cttaatcatc ttcacttgac tatttgtaat 2340
actgttgtaa tgttaactct gttaagtacc caagctgctt gtcttccacc aaagagtgct 2400
ttattaacaa gaatctgtga aaatcacatt taaacactgt tgcatgttgt aagaccaggt 2460
ggtaccttag taacctaaaa cttgcaagag aatattaatg gtagctttag aagactcagg 2520

aggagaaact gacttcagag ttggaagatg ttgcaagtcg ttcctttttc tgtccttcag 2580
ggactgaaga actgggaggc tgcccattgt ttggttgcca gtcatacaaa ttaaaatcat 2640
atttccttcc atgaatggaa gaaacacact attggttttt ccccttggaa acagcaatcc 2700
caaataatgt cggcttacaa aaaaaaaaag ttaccacttt tttagagtcc ttccctgtaa 2760
cattggattt tttttttccc ttatgagatc cacctaaggc cattgacgtg gcctgcgatc 2820
tcagtgacaa tgatctgctt ctggatctca ctgttgcctt tggttaggga acacagagtg 2880
cttctcccgc agccctactg gaacacagca gagtctgtgc catgaagcag ttacagaaac 2940
agaattgatg tgctgctaaa aaaaaaaaaa aaaatggggc ccgggggggc gtccgccggc 3000
cctgcgggcc gccggtgaaa taccactact ctgatcgttt tttcactgac ccggtgaggc 3060
gggggggcga gccccgaggg gctctcgctt ctggcgcg 3098

<210> 54
<211> 7850
<212> DNA
<213> XM_372063.2| PREDICTED: Homo sapiens similar to epiplakin (LOC389697), mRNA

<400> 54

atggcagcca cgctgggagc cggcacgccc cccaggcccc aggccaggag catagctggg 60
gtgtatgtgg aggcctcggg ccaggcccag agtgtctacg ccgccatgga gcagggcctc 120
ctgcctgctg ggctcgggca ggctctgcta gaggcccagg cagccactgg gggcctggtg 180
gacctcgccc ggggccagct gctccctgtg tccaaggccc tgcagcaggg tctggtgggg 240
ctggagctga aggagaagct gctggccgct gagcgtgcca ctacgggcta tcctgacccc 300
tacggcggtg agaagctggc cctctttcag gccatcggga aggaggttgt ggacagggcc 360
ctggggcaga gctggctgga ggtccaactg gccactgggg gcctggtgga ccccgcccag 420
ggagtgctcg tggcccctga gccagcctgc caccagggcc tcctggaccg ggagacatgg 480
cacaagctgt cagagcttga gcctggcaca ggtgacctgc gcttcctcga ccccaacacg 540
ctggagcggc tgacatacca ccagctgctg gaaaggtgtg tgcgtgcccc cggctcgggg 600
ctagccttgc tgcccctcaa gatcaccttc cgctccatgg gcggggcggt gagtgcagct 660
gagctgctgg aggtgggcat cctggacgag caggctgtgc agggtctgcg ggagggcagg 720
ctggccgcag tggacgtgag tgcacgtgcc gaggtgcggc gctacctgga gggtaccggc 780
agcgtggccg gggttgtcct gctgcccgaa ggccacaaga agagcttttt ccaggctgcc 840
accgagcacc tgctcccaat gggcaccgcg ctgccactcc tagaggccca ggctgccacc 900
cacaccctgg tggaccccat cacaggccag cggctgtggg tagacgaggc agtcagggcg 960
ggcctggtca gcccagagct ccatgagcag ctcctggtgg ccgagcaggc cgtgacaggg 1020

caccacgacc ccttcagtgg ctcccaaatc cccctttcc aggccatgaa gaaggggcta 1080
gtggacaggc cactggcact gcggctcttg gatgcccagc tggccacagg cgggctggtc 1140
tgtccagcac gcaggctccg gctgcccctg gaggccgccc tgcgctgcgg ctgcctggat 1200
gaagacactc agcggcagct ctcgcaggct ggcagcttct cagacggcac gcacggcggc 1260
ctgcgctatg aacagctgct ggccctctgt gtcaccgacc cagagaccgg gcttgccttc 1320
ctgccactct caggggacc ccggggaggg gagccccagg gacccccatt catcaagtac 1380
agcactcggc aggccctgag cacggccaca gccaccgtct ctgtggggaa gttccggggc 1440
cggcccgtgt ccctctggga gctgctcttc tctgaggcca tctcctcaga gcagagggcg 1500
atgctggccc agcagtacca ggaagggacc ctctccgtgg agaagctggc cgctaagctg 1560
agcgccaccc tcgagcaggc tgcagccact gccagggtca ccttttctgg gctgagggac 1620
accgtgacac caggagagct gctgaaagcc gagatcatcg accaggacct gtacgagcgg 1680
ctggagcatg gacaggccac agccaaggat gtgggcagcc tggcctcggt gcagaggtac 1740
ctgcagggta cgggctgcat tgctggcctg ctgctccctg gctcccagga acgcctgagc 1800
atctatgagg cccgatgcaa ggggctcctc cggcccggca ctgccctcat ccttctggag 1860
gcacaagctg ccacaggctt catcatcgac ccaaaagcaa acaaggggca ctccgttgag 1920
gaggcactga gggctgctgt cattgggcct gatgtgttcg cgaagctgct gtcggctgag 1980
cgcgctgtca ctggctacac tgacccctac accgggcagc agatctccct cttccaggcc 2040
atgcagaagg gcctcatcgt ccgggagcac ggcatccgcc tgctggaggc ccagatcgcc 2100
acgggcggcg tcatcgaccc cgtgcacagc caccgcgtgc ccgtggacgt ggcctaccgg 2160
cgcggctact tcgatcagat gctgaacttg atcctgttgg acccttctga cgacaccaag 2220
ggcttcttcg accccaacac gcacgagaac ctcacgtacc tgcagcttct ggagcgctgt 2280
gtgcgtgacc ccgagacggg cctgtacctc ctgccactca gcagcacgca gtccccgctg 2340
gtggacagtg ccacccagca ggccttccag aacctgctgc tctccgtgaa gtatggacgg 2400
tttcaggggc agagggtctc cgcgtgggag ctgatcaact ctgagtactt cagcgagggc 2460
cgcaggaggc agctgctgcg tcgctaccgg cagcgcgagg tcacgctggg gcaggtggca 2520
aagctgctgg aggcggagac gcagagacag gcggacatca tgctgcccgc actgcggagc 2580
cgggtcaccg tccaccagct cctggaggcc ggtatcattg accagcagct gttggaccaa 2640
gtgctggccg ggacaatcag cccggaggcc ctcctactca tggacggcgt ccgcaggtac 2700
ctgtgcggcc tgggagctgt gggcggtgtg cggctgctgc cctctggcca gcggctcagc 2760
ctctaccagg ccatgaggca gaagctgctg gggcccaggg tggccctggc cctgctggag 2820
gcccaggcgg ccaccggaac catcatggac cctcacagcc cagagagcct ctcggtggat 2880
gaggccgtgc gcaggggtgt ggtggggccg gagctgtatg gcaggctgaa gcgggctgag 2940
ggtgccattg ctggcttcag agacccttc tctgggaagc aggtgtctgt gttccaggcc 3000

atgaagaaag gtctcatccc ttgggagcaa gctgcccgcc tcctggaggc tcaagtggcc 3060
acaggaggga tcattgaccc caccagccac caccacctcc ccatgccagt ggccattcag 3120
cgtggctatg ttgaccagga gatggagaca gccttgtcca gctcctccga aaccttcccc 3180
acaccggacg gccaggggcg cacgagctat gcccagctcc tggaggagtg ccccagggat 3240
gagacttctg gccttcacct cctgcccctg ccagaaagtg ctcctgccct ccccaccgag 3300
gagcaggtcc agaggagcct gcaggccgtg ccgggggcca aggatggcac atccctctgg 3360
gacctgctca gctcctgcca cttcaccgag gagcaacgga ggggcctgct ggaggacgtg 3420
caggaggga ggaccactgt gccacagctg ctagcctctg tgcagaggtg ggtacaggag 3480
accaagctcc tggcccaggc ccgcgtcatg gtgcccggcc cacggggtga ggtacccgct 3540
gtctggctgc tggatgctgg catcatcacc caggagaccc ttgaggccct ggctcagggc 3600
acgcagtcgc ccgcccaggt cgccgagcag ccggcggtga aggcctgcct gtggggcaca 3660
ggctgcgtgg ccggtgtgct gctacagccc tctggggcca aggccagcat cgcccaggcc 3720
gtgagggatg gcctcctgcc cacaggcctg ggccagaggc tgctggaagc ccaggtggca 3780
tctggcttcc ttgttgaccc cctgaacaac cagagactgt cagtggagga cgcggttaag 3840
gtcggcctgg tgggcaggga gctgagtgag cagctcgggc aggccgagag ggcggcggcc 3900
gggtacccag atccctactc tagggcctcc ctctctctgt ggcaggccat ggagaagggg 3960
ctcgtgccac agaacgaggg cttgcccctc ctgcaggtgc agctggccac agggggtgtg 4020
gtggaccctg tccacggggt gcacctgccc caggcggcag cctgcagact cggccttctg 4080
gacacacaga cgagccaggt gctgactgca gttgacaagg acaacaagtt cttctttgac 4140
cccagtgcgc gggaccaggt gacctaccag cagctcaggg agcgctgcgt gtgcgactcc 4200
gagaccggat tgttgctgtt gccactgccc tcagacacag tgcttgaggt ggacgaccac 4260
accgcggtgg ctctgagggc catgaaggtg cccgtcagca cagggaggtt taaggggtgt 4320
agcgtgtcac tctgggacct gctgctctcc gaatacgttg gcgctgacaa gcggcgggag 4380
ctggtggcac tctgtcggtc tgggagggct gcggccctgc ggcaggtggt cagcgcagtc 4440
accaccctgg tcgaggctgc agagaggcag cccctgcagg ccaccttcag agggctccgg 4500
aagcaggtgt cagccaggga cctgttcagg gcgcagctga tcagcaggaa gacgctggac 4560
gagctgagcc aggggacaac gactgtgaag gaggtggcgg agatggacag cgtgaagcgg 4620
tccctggagg gaggcaactt cattgccggg gtccttatcc agggcaccca ggagaggatg 4680
agcatcccag aggccctgag gaggcacatc ctgcggcctg gcacagccct ggtgctgctg 4740
gaggcacagg cagctaccgg cttcatcatc gaccccgtgg agaaccggaa gctgaccgtg 4800
gaggaggcgt tcaaagcagg aatgttcggg aaagaaacct acgtgaagct gctgtcggcc 4860
gagcgcgccg tcaccggcta caccgacccc tataccgggc agcagatctc cctcttccag 4920
gccatgcaga aggacctcat cgtccgggag cacggcatcc gcctgctgga ggcccagatc 4980
gccacgggcg gcatcatcga ccccgtgcac agccaccgcg tgcccgtgga cgtggcctac 5040

cgctgcggct acttcgacga ggagatgaac cgcatcctgg cggaccccag cgacgacacc 5100
aagggcttct tcgaccccaa cacgcacgag aacctcacgt acctgcagct tctggagcgc 5160
tgtgtggagg accccgagac gggcctgtac ctgctacaaa tcataaagaa aggagaaaac 5220
tacgtgtaca tcaatgaggc cacgagacac gtgttgcaat ccagaactgc aaaaatgcgc 5280
gtggggaggt ttgctgacca ggtggtctct ttctgggacc tgctgtcctc tccatacttc 5340
acagaggaca ggaagcggga gctcatccag gagtatggag cccagagtgg gggcctggag 5400
aaattgctgg aaatcatcac cacgacaatt gaagaaacag agacgcaaaa ccaaggcatc 5460
aaagtggcgg ccatcagagg ggaggtgaca gctgcagacc tgttcaactc cagggtcatc 5520
gatcagaaga ccctgcacac acttcgtgtg gggaggactg ggggacaggc actcagcacg 5580
ctggagtgtg tgaagcccta tctggaaggc agcggctgca ttgcgggggt cacggtgccc 5640
tccaccaggg aggtcatgag cctccatgag gccagcagga aggagctcat ccctgcagca 5700
tttgcgactt ggctgctgga ggcgcaggcc gccaccgggt tcctcctgga cccctgcacc 5760
cgccagaagc tctctgtgga tgaggctgtg gatgtgggcc tggtgaacga ggagctgcgg 5820
gagaggctcc tgaaggctga aagagctgcc acgggctaca gggatccggc cacaggagac 5880
acgatcccgc tgttccaggc catgcagaag cagctcatcg agaaggcgga ggcactgagg 5940
ctgctggagg tgcaggtggc cacgggggt gtcatcgacc cacagcacca ccaccggctc 6000
ccactggaaa cagcctacag acggggctgt ctgcacaagg acatctatgc gctcatttcc 6060
gaccagaagc acatgaggaa acggtttgtg gacccgaaca cgcaagagaa ggtctcgtac 6120
cgagagctgc aggagaggtg ccgcccacaa gaggacacgg gctggctgct gttcccagtg 6180
aacaaggctg cacgggactc cgagcacatc gatgacgaga cgagaagggc cctggaggca 6240
gagcaagtgg aaatcacagt gggaaggttc agaggccaga aaccaacact gtgggcacta 6300
ctgaattccg aatacgtgac agaggagaag aagctccagc tggtgaggat gtatagaaca 6360
cacaccagac gggcactgca gacggtagcg cagctcatct tagagttgat cgagaagcag 6420
gaaaccagca acaaacacct gtggttccaa ggaattagac gacagatcac agcttctgaa 6480
ctcctcagct cagccataat cacggaggaa atgctccagg acctggaaac gggacggagc 6540
acgacgcaag agctcatgga ggacgaccgc gtcaagcgct acctggaggg caccagctgc 6600
atcgcgggcg tcctggtgcc cgccaaggac cagcccggcc gccaggagaa gatgagcatc 6660
taccaggcca tgtggaaggg cgtgctgcgg cccggcacgg ccctggtgct gctggaggcg 6720
caggcggcca ccggcttcgt catcgacccc gtgcgcaacc tgaggctgtc ggtggaggag 6780
gccgtggctg cgggcgtggt gggcggcgag atccaggaga agctgctgtc ggccgagcgc 6840
gccgtcaccg gctacaccga cccctacacc gggcagcaga tctccctctt ccaggccatg 6900
cagaaggacc tcatcgtccg ggagcacggc atccgcctgc tggaggccca gatcgccacg 6960
ggcggcgtca tcgaccccgt gcacagccac cgcgtgcccg tggacgtggc ctaccggcgc 7020

ggctacttcg acgaggagat gaaccgcgtc ctggccgacc ccagcgacga caccaagggc 7080
ttcttcgacc ccaacacgca cgagaacctc acgtacctgc agcttctgca gagggccacc 7140
ctggaccctg agacggggct cctatttctt tctctctctc tacagtgact gggcttcctc 7200
cgtgcagttt tctgcaactc tggagaagtg gaggcatact tgtgtgtctg ggttgttttt 7260
tttttgtttt tttttttgtc attctttaat tttgttgttt tacccattcg ttatctgtgg 7320
aaaacgtttt aagttgtcat gtgacagaaa cttttccttt gtccatcgag gtgtttcata 7380
agttttttgg tgtgttttct gggtcgtcta tgtgtcatat ggttttactt ttctctcctt 7440
tttcgttttc agaacatttt tctgtctgtt ttggattcac tgcttccatt ttacagaatg 7500
tcactcttta gactctcagt ccatcatgcc atcgggtact cttgttgcag tgtaattttt 7560
attacatgcg gttatttccc taacgatgtg ctattcacgt tcatcttcaa actcatttc 7620
catcagccag tgtctactat ttagtgccct ggctctattt cggtcctcct ccccgggctt 7680
tccctggctg ctgtgctggc caaaagcatg ggctttattc tctccattgg ctgctgctcc 7740
accttagagg tgtgacctca ctagcgttga ctgagcgagt ctgttgtgga gaagaacttt 7800
ttgtagtaat ttactaggaa aaattctgaa caagtaaaat atgaaggaaa 7850

<210> 55
<211> 454
<212> DNA
<213> NM_005952.2| Homo sapiens metallothionein 1x (MT1X), mRNA

<400> 55

tctgtcccgc tgcgtgtttt cctcttgatc gggaactcct gcttctcctt gcctcgaaat 60
ggaccccaac tgctcctgct cgcctgttgg ctcctgtgcc tgtgccggct cctgcaaatg 120
caaagagtgc aaatgcacct cctgcaagaa gagctgctgc tcctgctgcc ctgtgggctg 180
tgccaagtgt gcccagggct gcatctgcaa agggacgtca gacaagtgca gctgctgtgc 240
ctgatgccag gacagctgtg ctctcagatg taaatagagc aacctatata aacctggatt 300
tttttttttt tttttttgta caaccctgac ccgtttgcta catctttttt tctatgaaat 360
atgtgaatgg caataaattc atctagacta aaaaaaaaaa aaaaaaaaaa aaaaaaaaaa 420
aaaaaaaaaa aaaaaaaaaa aaaaaaaaaa aaaa 454

<210> 56
<211> 2090
<212> DNA
<213> NM_003242.3| Homo sapiens transforming growth factor, beta receptor II (70/80kDa) (TGFBR2), mRNA

<400> 56

gttggcgagg agtttcctgt ttcccccgca gcgctgagtt gaagttgagt gagtcactcg 60
cgcgcacgga gcgacgacac ccccgcgcgt gcacccgctc gggacaggag ccggactcct 120
gtgcagcttc cctcggccgc cgggggcctc cccgcgcctc gccggcctcc aggcccctcc 180
tggctggcga gcgggcgcca catctggccc gcacatctgc gctgccggcc cggcgcgggg 240
tccggagagg gcgcggcgcg gagcgcagcc aggggtccgg gaaggcgccg tccgtgcgct 300
gggggctcgg tctatgacga gcagcggggt ctgccatggg tcgggggctg ctcaggggcc 360
tgtggccgct gcacatcgtc ctgtggacgc gtatcgccag cacgatccca ccgcacgttc 420
agaagtcggt taataacgac atgatagtca ctgacaacaa cggtgcagtc aagtttccac 480
aactgtgtaa attttgtgat gtgagatttt ccacctgtga caaccagaaa tcctgcatga 540
gcaactgcag catcacctcc atctgtgaga agccacagga agtctgtgtg gctgtatgga 600
gaaagaatga cgagaacata acactagaga cagtttgcca tgaccccaag ctcccctacc 660
atgactttat tctggaagat gctgcttctc caaagtgcat tatgaaggaa aaaaaaaagc 720
ctggtgagac tttcttcatg tgttcctgta gctctgatga gtgcaatgac aacatcatct 780
tctcagaaga atataacacc agcaatcctg acttgttgct agtcatattt caagtgacag 840
gcatcagcct cctgccacca ctgggagttg ccatatctgt catcatcatc ttctactgct 900
accgcgttaa ccggcagcag aagctgagtt caacctggga aaccggcaag acgcggaagc 960
tcatggagtt cagcgagcac tgtgccatca tcctggaaga tgaccgctct gacatcagct 1020
ccacgtgtgc caacaacatc aaccacaaca cagagctgct gcccattgag ctggacaccc 1080
tggtggggaa aggtcgcttt gctgaggtct ataaggccaa gctgaagcag aacacttcag 1140
agcagtttga gacagtggca gtcaagatct ttccctatga ggagtatgcc tcttggaaga 1200
cagagaagga catcttctca gacatcaatc tgaagcatga gaacatactc cagttcctga 1260
cggctgagga gcggaagacg gagttgggga aacaatactg gctgatcacc gccttccacg 1320
ccaagggcaa cctacaggag tacctgacgc ggcatgtcat cagctgggag gacctgcgca 1380
agctgggcag ctccctcgcc cgggggattg ctcacctcca cagtgatcac actccatgtg 1440
ggaggcccaa gatgcccatc gtgcacaggg acctcaagag ctccaatatc ctcgtgaaga 1500
acgacctaac ctgctgcctg tgtgactttg ggctttccct gcgtctggac cctactctgt 1560
ctgtggatga cctggctaac agtgggcagg tgggaactgc aagatacatg gctccagaag 1620
tcctagaatc caggatgaat ttggagaatg ctgagtcctt caagcagacc gatgtctact 1680
ccatggctct ggtgctctgg gaaatgacat ctcgctgtaa tgcagtggga gaagtaaaag 1740
attatgagcc tccatttggt tccaaggtgc gggagcaccc ctgtgtcgaa agcatgaagg 1800
acaacgtgtt gagagatcga gggcgaccag aaattcccag cttctggctc aaccaccagg 1860
gcatccagat ggtgtgtgag acgttgactg agtgctggga ccacgaccca gaggcccgtc 1920

tcacagccca gtgtgtggca gaacgcttca gtgagctgga gcatctggac aggctctcgg 1980
ggaggagctg ctcggaggag aagattcctg aagacggctc cctaaacact accaaatagc 2040
tcttatgggg caggctgggc atgtccaaag aggctgcccc tctcaccaaa 2090

<210> 57
<211> 4568
<212> DNA
<213> NM_012408.3| Homo sapiens protein kinase C binding protein 1 (PRKCBP1), transcript variant 2, mRNA

<400> 57

gtgagaacta ggagcctgtc ctccatgttt tataagtatt gacattacac agtgttaaca 60
atgcatccac agagcttggc tgaagaggaa ataaaaacag aacaggaggt ggtagagggc 120
atggatatct ctactcgctc caaagatcct ggctctgcag agagaacagc ccagaaaaga 180
aagttcccca gccctccaca ttcttccaat ggccactcgc cgcaggacac atcaacaagc 240
cccattaaaa agaaaaagaa acctggctta ctgaacagta acaataagga gcagtcagaa 300
ctaagacatg gtccgtttta ctatatgaag cagccactca ccacagaccc tgttgatgtt 360
gtaccgcagg atggacggaa tgatttctac tgctgggttt gtcaccggga aggccaagtc 420
ctttgctgtg agctctgtcc ccgggtttat cacgctaagt gtctgagact gacatcggaa 480
ccagaggggg actggttttg tcctgaatgt gagaaaatta cagtagcaga atgcatcgag 540
acccagagta aagccatgac aatgctcacc attgaacagt tatcctacct gctcaagttt 600
gccattcaga aaatgaaaca gccagggaca gatgcattcc agaagcccgt tccattggaa 660
cagcaccctg actatgcgga atacatcttc catccaatgg acctttgtac attggaaaag 720
aatgcgaaaa agaaaatgta tggctgcaca gaagccttcc tggctgatgc aaagtggatt 780
ttgcacaact gcatcattta taatggggga aatcacaaat tgacgcaaat agcgaaagta 840
gtcatcaaaa tctgtgaaca tgagatgaat gaaatcgaag tatgtccaga atgttatcta 900
gctgcttgcc aaaaacgaga taactggttt tgtgagcctt gtagcaatcc acatcctttg 960
gtctgggcca aactgaaggg gtttccattc tggcctgcaa aagctctaag ggataaagac 1020
gggcaggtcg atgcccgatt ctttggacaa catgacaggg cctgggttcc aataaataat 1080
tgctacctca tgtctaaaga aattcctttt tctgtgaaaa agactaagag catcttcaac 1140
agtgccatgc aagagatgga ggtttacgtg gagaacatcc gcaggaagtt tggggttttt 1200
aattactctc catttaggac accctacaca cccaacagcc agtatcaaat gctgctcgat 1260
cccaccaacc ccagcgccgg cactgccaag atagacaagc aggagaaggt caagctcaac 1320
tttgacatga cggcatcccc caagatcctg atgagcaagc ctgtgctgag tgggggcaca 1380
ggccgccgga tttccttgtc ggatatgccg cgctccccca tgagcacaaa ctcttctgtg 1440

cacacgggct ccgacgtgga gcaggatgct gagaagaagg ccacgtcgag ccacttcagt 1500
gcgagcgagg agtccatgga cttcctggat aagagcacag cttcaccagc ctccaccaag 1560
acgggacaag cagggagttt atccggcagc ccaaagccct tctctcctca actgtcagct 1620
cctatcacga cgaaaacgga caaaacctcc accaccggca gcatcctgaa tcttaacctg 1680
gatcgaagca aagctgagat ggatttgaag gagctgagcg agtcggtcca gcaacagtcc 1740
acccctgttc ctctcatctc tcccaagcgc cagattcgta gcaggttcca gctgaatctt 1800
gacaagacca tagagagttg caaagcacaa ttaggcataa atgaaatctc ggaagatgtc 1860
tatacggccg tagagcacag cgattcggag gattctgaga agtcagatag tagcgatagt 1920
gagtatatca gtgatgatga gcagaagtct aagaacgagc cagaagacac agaggacaaa 1980
gaaggttgtc agatggacaa agagccatct gctgttaaaa aaaagcccaa gcctacaaac 2040
ccagtggaga ttaaagagga gctgaaaagc acgtcaccag ccagcgagaa ggcagaccct 2100
ggagcagtca aggacaaggc cagccctgag cctgagaagg acttttccga aaaggcaaaa 2160
ccttcacctc accccataaa ggataaactg aagggaaaag atgagacgga ttccccaaca 2220
gtccatttgg gcctggactc tgattcagag agcgaacttg tcatagattt aggagaagac 2280
cattctgggc gggagggtcg aaaaaataag aaggaaccca aagaaccatc tcccaaacag 2340
gatgttgtag gtaaaactcc accatccacg acggtgggca gccattctcc cccggaaaca 2400
ccggtgctca cccgctcttc cgcccaaact tccgcggctg gcgccacagc caccaccagc 2460
acgtcctcca cggtcaccgt cacggccccg gcccccgccg ccacaggaag cccagtgaaa 2520
aagcagaggc cgcttttacc gaaggagact gccccggccg tgcagcgggt cgtgtggaac 2580
tcatcaactg tccagcagaa ggagatcaca cagagcccat ccacgtccac catcaccctg 2640
gtgaccagca cacagtcatc gcccctggtc accagctcgg ggtccatgag caccccttgtg 2700
tcctcagtca acgctgacct gcccatcgcc actgcctcag ctgatgtcgc cgctgatatt 2760
gccaagtaca ctagcaaaat gatggatgca ataaaaggaa caatgacaga aatatacaac 2820
gatctttcta aaaacactac tggaagcaca atagctgaga ttcgcaggct gaggatcgag 2880
atagagaagc tccagtggct gcaccagcaa gagctctccg aaatgaaaca caacttagag 2940
ctgaccatgg cggagatgcg gcagagcctg gagcaggagc gggaccggct catcgccgag 3000
gtgaagaagc agctggagtt ggagaagcag caggcggtgg atgagaccaa gaagaagcag 3060
tggtgcgcca actgcaagaa ggaggccatc ttttactgct gttggaacac tagctactgt 3120
gactacccct gccagcaagc ccactggcct gagcacatga agtcctgcac ccagtcagct 3180
actgctcctc agcaggaagc ggatgctgag gtgaacacag aaacactaaa taagtcctcc 3240
caggggagct cctcgagcac acaatcagca ccttcagaaa cggccagcgc ctccaaagag 3300
aaggagacgt cagctgagaa aagcaaggag agtggctcga cccttgacct ttctggctcc 3360
agagagacgc cctcctccat tctcttaggc tccaaccaag gctctgacca ttcccggagt 3420
aataaatcca gttggagcag cagtgatgag aagaggggat cgacacgttc cgatcacaac 3480

accagtacca gcacgaagag cctcctcccg aaagagtctc ggctggacac cttctgggac 3540
tagcagtgaa tcgggacaca aaccacccac cccattggga gaaaaaccca gacgccagga 3600
aaagaagaaa caacaaaggc aggagaacag ccactttcag acttgaaaat gacaaaaccc 3660
tcagttgagc ctgagccccc ggcgcggggg ctgctacact acaggacacc cagcatcggc 3720
tttgactgca gactgttcac ccacacgagc cctgtgcttt tggtgtaaat aatgtacaat 3780
ttgtggatgt cattgaatct agaggacttt cccctttta tatttgtatt aactttaact 3840
tattaaaaaa aaaaaaagaa aaagaaaaac gatttaaaaa aaaaaaaaaa agcaaccaac 3900
cccaacaaca aaaaagaatg ttttggtatt ggagaaggga tggtcagtta gcctgtctgt 3960
cacacgacgg aatggatact gggcccgggg accactttca tactcacgtc ctcatccttg 4020
gatacccagg ggagggcgaa ccgttttcgc tcgtgtgtct gtacgcagca tgttgggatc 4080
gggagtttcg gcacagacta tcccatcaag ccgttggctc ctttcagcta ctacgttacc 4140
acgttcctaa aacgcaagct ctccggacca gacggacaca gggagaagct agtttctttc 4200
atgtgattga aatgatgact ctactcctaa aagggaaaaa acaatatcct tgtttacaga 4260
agagaaacaa acaagcccca ctcagctcag tcacaggaga gaacacagaa agtcttagga 4320
tcatgaactc tgaaaaaaag agaaacctta tctttgcttt gtggttcctt taaacacact 4380
cacacacact tggtcagaga tgctgtgctt cttggaagca aggactcaaa ggcaaggtgc 4440
acgcagagga cgtttgagtc tgggatgaag catgtacgta ttatttatat gatggaattt 4500
cacgttttta tgtaagcatg aaacacaggc agtatgagag aaagcaaggc ccgtcatgct 4560
gtccgtac 4568

<210> 58
<211> 2069
<212> DNA
<213> NM_003270.2| Homo sapiens transmembrane 4 superfamily member 6 (TM4SF6), mRNA

<400> 58

cgctcgtaag ttttcggcag tttccgggga gactcgggga ctccgcgtct cgctctctgt 60
gttccaatcg cccggtgcgg tggtgcaggg tctcgggcta gtcatggcgt ccccgtctcg 120
gagactgcag actaaaccag tcattacttg tttcaagagc gttctgctaa tctacacttt 180
tattttctgg atcactggcg ttatccttct tgcagttggc atttggggca aggtgagcct 240
ggagaattac ttttctcttt taaatgagaa ggccaccaat gtccccttcg tgctcattgc 300
tactggtacc gtcattattc ttttgggcac ctttggttgt tttgctacct gccgagcttc 360
tgcatggatg ctaaaactgt atgcaatgtt tctgactctc gtttttttgg tcgaactggt 420
cgctgccatc gtaggatttg ttttcagaca tgagattaag aacagcttta agaataatta 480

```
tgagaaggct ttgaagcagt ataactctac aggagattat agaagccatg cagtagacaa    540
gatccaaaat acgttgcatt gttgtggtgt caccgattat agagattgga cagatactaa    600
ttattactca gaaaaaggat ttcctaagag ttgctgtaaa cttgaagatt gtactccaca    660
gagagatgca gacaaagtaa acaatgaagg ttgttttata aaggtgatga ccattataga    720
gtcagaaatg ggagtcgttg caggaatttc ctttggagtt gcttgcttcc aactgattgg    780
aatctttctc gcctactgcc tctctcgtgc cataacaaat aaccagtatg agatagtgta    840
acccaatgta tctgtgggcc tattcctctc tacctttaag gacatttagg gtccccccctg    900
tgaattagaa agttgcttgg ctggagaact gacaacacta cttactgata gaccaaaaaa    960
ctacaccagt aggttgattc aatcaagatg tatgtagacc taaaactaca ccaataggct   1020
gattcaatca agatccgtgc tcgcagtggg ctgattcaat caagatgtat gtttgctatg   1080
ttctaagtcc accttctatc ccattcatgt tagatcgttg aaaccctgta tccctctgaa   1140
acactggaag agctagtaaa ttgtaaatga agtaatactg tgttcctctt gactgttatt   1200
tttcttagta gggggccttt ggaaggcact gtgaatttgc tattttgatg tagtgttaca   1260
agatggaaaa ttgattcctc tgactttgct attgatgtag tgtgatagaa aattcacccc   1320
tctgaactgg ctccttccca gtcaaggtta tctggtttga ttgtataatt tgcaccaaga   1380
agttaaaatg ttttatgact ctctgttctg ctgacaggca gagagtcaca ttgtgtaatt   1440
taatttcagt cagtcaatag atggcatccc tcatcagggt tgccagatgg tgataacagt   1500
gtaaggcctt gggtctaagg catccacgac tggaagggac tactgatgtt ctgtgataca   1560
tcaggtttca gcacacaact tacatttctt tgcctccaaa ttgaggcatt tattatgatg   1620
ttcatacttt ccctcttgtt tgaaagtttc taattattaa atggtgtcgg aattgttgta   1680
ttttccttag gaattcagtg gaacttatct tcattaaatt tagctggtac caggttgata   1740
tgacttgtca atattatggt caactttaag tcttagtttt cgtttgtgcc tttgattaat   1800
aagtataact cttatacaat aaatactgct ttcctctaaa aagatcgtgt ttaaattaac   1860
ttgtagaaaa tctgctggaa tggttgttgt tttccactga gaaagctaag ccctacattt   1920
ctattcagag tactgttttt agatgtgaaa tataagcctg cggccttaac tctgtattaa   1980
aaaaaatgtt tttgtttaaa aaaaactgtt cccataggtg cagcaaacca ccatggcaca   2040
tgtataccta tgtaacaaac ctgcacatt                                     2069
```

<210> 59
<211> 2402
<212> DNA
<213> NM_021200.1| Homo sapiens pleckstrin homology domain containing, family B (evectins) member 1 (PLEKHB1), mRNA

<400> 59

aagagaggaa ggcttaaaga gccagactgc gcagccagga ctggggtatg ggcgctgtcc 60
tgcaggccaa agaatgaaga tgtagccccg cccccaacct aggaggagga ccagcccggt 120
tcctgtcctg cccccgcaac ctcgccccga ttccactccg ggaacctcgg cgatgctgag 180
ccaagaccac ttctgaatca gggatgactt gtctagtgaa cgtagggtca gagccatcag 240
ttggaaaggc tgggaggagc ctggagaaag aggcgacctt ccttgggatc tgtgcgctcc 300
ctccttgcct ccccctccag cctcccactt ggtagcacct tcctgatccc cttatctcta 360
aggcgctcag ggaaatgccc cgctgcggga gccttctggg aaatgctgcc ctggccaccc 420
aggaaccatg agccctgcag ccccggtccc gcctgactcc gctctggaaa gtccttttga 480
agaaatggcc ctggtgaggg gcggctggct gtggagacag agctccatcc tccgccgctg 540
gaagcggaac tggtttgccc tgtggctgga cgggaccctg ggatactacc acgatgagac 600
agcgcaggac gaggaggacc gtgtgctcat ccacttcaat gtccgtgaca taaagatcgg 660
cccagagtgc catgatgtgc agcccccaga gggccggagc cgagatggcc tgctgactgt 720
gaacctacgg gaaggcggcc gcctgcacct ctgtgcggag accaaggatg atgccctagc 780
atggaagaca gcactgctgg aggcaaactc caccccggcc ccagctggag ccaccgtccc 840
tcccaggagc cgccgggttt gctccaaggt caggtgtgtg acccgctcgt ggagcccctg 900
taaggttgag aggcggatct gggtgcgcgt ctacagcccg taccaagact actacgaggt 960
ggtgcccccc aatgcacacg aggccacgta tgtccgcagc tactacggac cgccctacgc 1020
aggccctggc gtgacgcacg tgatagtgcg ggaggatccc tgctacagcg ccggcgcccc 1080
tctggccatg ggcatgcttg cgggagccgc cactggggcg gcgctgggct cgctcatgtg 1140
gtcgccctgc tggttctgag ccctgggact cggagcactg acccctgcgc ttggattgct 1200
agactcctct tcctcctgga ccccatcctc taccatccaa gccctgtccc actttggccc 1260
tatcctctcc attagctcct tccgggtttg gaccattccc cccactccct acccttaatc 1320
cccacatggg aagaagctat catcacaggt acaaacatcg cttgaagtct tcacatctac 1380
cactagacac ccccaaaatc tgttatagac atttatggat acatttcctc taaacacaac 1440
agggcacagc aaatacgact tcatttggct tcgagttccc caggcgctgt agacacaaca 1500
tgaatcgggc tctctgctct ctccttaggg agctcgagtc ctggtgggga gaacaggagt 1560
aaacaaggac ttgacaaagc tgaagagtta tcagtccttt gacaaggaca ggtggggcag 1620
ggagcaagac aggtaggctg gaagaacagt tattggcaag tatgcagagc cgtgaacgtc 1680
atggcatgtc caaggaatta aatgggagtt catttgggct ggggtggagg ctgggatcag 1740
accgtggtgg gccttcaagc taaggagctt cctaggtgaa aggggagatg tgagccttct 1800
ctggagggaa gtttcatgat tgcatctata atgaatatat tgcctgtttt gtgaatactg 1860
acacatgtcc atacctaaaa cactcctgag ttaagtccca tccttcccac aaacagcttc 1920
ctggctggta cccatgataa caattgagct gaacctgggg acccctggtt ggggaacagg 1980

tgagttctat ttgagacttc cagccctaga aagctgcctc cgtccagaaa tgcctctcac 2040
accaggagct cggccctctc tttatagctg tgactgtcac cctctcaggc tttgtctcat 2100
ccttcattct gaataagatg gcagtgttct cctctggggc ctgatccacc tctacaccag 2160
cccaggaagc cccatctgtg cctgccctca ggtggtccac cagtctcccc ctttggttcc 2220
cttccagtct cttcccctt tctatcccaa tcaccaatag aaatgctaac atccctgcct 2280
ggtagccaga ctagcccact aaagctcccc tgtaaatggg ggctccatta gttctgctgc 2340
cgagactaat aaagatttgg ttggttctag cagtaaaaaa aaaaaaaaaa aaaaaaaaaa 2400
aa 2402

<210> 60
<211> 2856
<212> DNA
<213> NM_003661-2| Homo sapiens apolipoprotein L, 1 (APOL1), transcript variant 1, mRNA

<400> 60

actttccctt tcgaattcct cggtatatct tggggactgg aggacctgtc tggttattat 60
acagacgcat aactggaggt gggatccaca cagctcagaa cagctggatc ttgctcagtc 120
tctgccaggg gaagattcct tggaggaggc cctgcagcga catggaggga gctgctttgc 180
tgagagtctc tgtcctctgc atctggatga gtgcactttt ccttggtgtg ggagtgaggg 240
cagaggaagc tggagcgagg gtgcaacaaa acgttccaag tgggacagat actggagatc 300
ctcaaagtaa gcccctcggt gactgggctg ctggcaccat ggacccagag agcagtatct 360
ttattgagga tgccattaag tatttcaagg aaaaagtgag cacacagaat ctgctactcc 420
tgctgactga taatgaggcc tggaacggat tcgtggctgc tgctgaactg cccaggaatg 480
aggcagatga gctccgtaaa gctctggaca accttgcaag acaaatgatc atgaaagaca 540
aaaactggca cgataaaggc cagcagtaca gaaactggtt tctgaaagag tttcctcggt 600
tgaaaagtga gcttgaggat aacataagaa ggctccgtgc ccttgcagat ggggttcaga 660
aggtccacaa aggcaccacc atcgccaatg tggtgtctgg ctctctcagc atttcctctg 720
gcatcctgac cctcgtcggc atgggtctgg caccettcac agagggaggc agccttgtac 780
tcttggaacc tgggatggag ttgggaatca cagccgcttt gaccgggatt accagcagta 840
ccatggacta cggaaagaag tggtggacac aagcccaagc ccacgacctg gtcatcaaaa 900
gccttgacaa attgaaggag gtgagggagt ttttgggtga gaacatatcc aactttcttt 960
ccttagctgg caatacttac caactcacac gaggcattgg gaaggacatc cgtgccctca 1020
gacgagccag agccaatctt cagtcagtac cgcatgcctc agcctcacgc ccccgggtca 1080
ctgagccaat ctcagctgaa agcggtgaac aggtggagag ggttaatgaa cccagcatcc 1140

```
tggaaatgag cagaggagtc aagctcacgg atgtggcccc tgtaagcttc tttcttgtgc   1200
tggatgtagt ctacctcgtg tacgaatcaa agcacttaca tgagggggca aagtcagaga   1260
cagctgagga gctgaagaag gtggctcagg agctggagga gaagctaaac attctcaaca   1320
ataattataa gattctgcag gcggaccaag aactgtgacc acagggcagg gcagccacca   1380
ggagagatat gcctggcagg ggccaggaca aaatgcaaac tttttttttt ttctgagaca   1440
gagtcttgct ctgtcgccaa gttggagtgc aatggtgcga tctcagctca ctgcaagctc   1500
tgcctcccgt gttcaagcga ttctcctgcc ttggcctccc aagtagctgg gactacaggc   1560
gcctaccacc atgcccagct aatttttgta tttttaatag agatggggtt tcaccatgtt   1620
ggccaggatg gtctcgatct cctgacctct tgatctgccc accttggcct cccaaagtgc   1680
tgggattaca ggcgtgagcc atcgcttttg acccaaatgc aaacatttta ttagggggat   1740
aaagagggtg aggtaaagtt tatggaactg agtgttaggg actttggcat ttccatagct   1800
gagcacagca ggggaggggt taatgcagat ggcagtgcag caaggagaag gcaggaacat   1860
tggagcctgc aataagggaa aaatgggaac tggagagtgt ggggaatggg aagaagcagt   1920
ttactttaga ctaaagaata tattgggggg ccgggtgtag tggctcatgc ctgtaatccg   1980
agcactttgg gaggccaagg cgggcggatc acgaggtcag gagatcgaga ccatcctggc   2040
taacacagtg aaaccccgtc tctactaaaa atacaaaaaa ttagccgggc atggtggcgg   2100
gcgcctgtag ttccagctaa ctgggcggct gaggcaggag aatggcgtga acctgggagg   2160
tggagcttgc agtgagccga gatatcgcca ctgcactcca gcctgggtga cagagcgaga   2220
ctccatctca aaaaaaaaaa aaaaaagaat atattgacgg aagaatagag aggaggcttg   2280
aaggaaccag caatgagaag gccaggaaaa gaaagagctg aaaatggaga aagcccaaga   2340
gttagaacag ttggatacag gagaagaaac agcggctcca ctacagaccc agccccaggt   2400
tcaatgtcct ccgaagaatg aagtctttcc ctggtgatgg tcccctgccc tgtctttcca   2460
gcatccactc tcccttgtcc tcctgggggc atatctcagt caggcagcgg cttcctgatg   2520
atggtcattg gggtggttgt catgtgatgg gtcccctcca ggttactaaa gggtgcatgt   2580
cccctgcttg aacactgaag ggcaggtggt gggccatggc catggtcccc agctgaggag   2640
caggtgtccc tgagaaccca aacttcccag agagtatgtg agaaccaacc aatgaaaaca   2700
gtcccatcgc tcttacccgg taagtaaaca gtcagaaaat tagcatgaaa gcagtttagc   2760
attgggagga agctcagatc tctagagctg tcttgtcgcc gcccaggatt gacctgtgtg   2820
taagtcccaa taaactcacc tactcatcaa gctgga                             2856
```

<210> 61
<211> 1655
<212> DNA
<213> NM_002164.3| Homo sapiens indoleamine-pyrrole 2,3 dioxygenase (INDO), mRNA

<400> 61

```
aatttctcac tgcccctgtg ataaactgtg gtcactggct gtggcagcaa ctattataag      60
atgctctgaa aactcttcag acactgaggg gcaccagagg agcagactac aagaatggca     120
cacgctatgg aaaactcctg gacaatcagt aaagagtacc atattgatga agaagtgggc     180
tttgctctgc caaatccaca ggaaaatcta cctgattttt ataatgactg gatgttcatt     240
gctaaacatc tgcctgatct catagagtct ggccagcttc gagaaagagt tgagaagtta     300
aacatgctca gcattgatca tctcacagac cacaagtcac agcgccttgc acgtctagtt     360
ctgggatgca tcaccatggc atatgtgtgg ggcaaaggtc atggagatgt ccgtaaggtc     420
ttgccaagaa atattgctgt tccttactgc caactctcca agaaactgga actgcctcct     480
attttggttt atgcagactg tgtcttggca aactggaaga aaaaggatcc taataagccc     540
ctgacttatg agaacatgga cgttttgttc tcatttcgtg atggagactg cagtaaagga     600
ttcttcctgg tctctctatt ggtggaaata gcagctgctt ctgcaatcaa agtaattcct     660
actgtattca aggcaatgca aatgcaagaa cgggacactt tgctaaaggc gctgttggaa     720
atagcttctt gcttggagaa agcccttcaa gtgtttcacc aaatccacga tcatgtgaac     780
ccaaaagcat ttttcagtgt tcttcgcata tatttgtctg gctggaaagg caacccccag     840
ctatcagacg gtctggtgta tgaagggttc tgggaagacc caaaggagtt tgcagggggc     900
agtgcaggcc aaagcagcgt ctttcagtgc tttgacgtcc tgctgggcat ccagcagact     960
gctggtggag gacatgctgc tcagttcctc caggacatga gaagatatat gccaccagct    1020
cacaggaact tcctgtgctc attagagtca aatccctcag tccgtgagtt tgtcctttca    1080
aaaggtgatg ctggcctgcg ggaagcttat gacgcctgtg tgaaagctct ggtctccctg    1140
aggagctacc atctgcaaat cgtgactaag tacatcctga ttcctgcaag ccagcagcca    1200
aaggagaata agacctctga agacccttca aaactggaag ccaaaggaac tggaggcact    1260
gatttaatga atttcctgaa gactgtaaga agtacaactg agaaatccct tttgaaggaa    1320
ggttaatgta acccaacaag agcacatttt atcatagcag agacatctgt atgcattcct    1380
gtcattaccc attgtaacag agccacaaac taatactatg caatgtttta ccaataatgc    1440
aatacaaaag acctcaaaat acctgtgcat ttcttgtagg aaaacaacaa aaggtaatta    1500
tgtgtaatta tactagaagt tttgtaatct gtatcttatc attggaataa aatgacattc    1560
aataaataaa aaaaaaaaaa aaaaaaaaaa aaaaaaaaaa aaaaaaaaaa aaaaaaaaaa    1620
aaaaaaaaaa aaaaaaaaaa aaaaaaaaaa aaaaa                               1655
```

<210> 62
<211> 2242
<212> DNA
<213> NM_021784.3| Homo sapiens forkhead box A2 (FOXA2), transcript variant 1, mRNA

<400> 62

```
cccgcccact tccaactacc gcctccggcc tgcccaggga gagagaggga gtggagccca     60
gggagaggga gcgcgagaga gggagggagg aggggacggt gctttggctg actttttttt    120
aaaagagggt gggggtgggg ggtgattgct ggtcgtttgt tgtggctgtt aaattttaaa    180
ctgccatgca ctcggcttcc agtatgctgg gagcggtgaa gatggaaggg cacgagccgt    240
ccgactggag cagctactat gcagagcccg agggctactc ctccgtgagc aacatgaacg    300
ccggcctggg gatgaacggc atgaacacgt acatgagcat gtcggcggcc gccatgggca    360
gcggctcggg caacatgagc gcgggctcca tgaacatgtc gtcgtacgtg ggcgctggca    420
tgagcccgtc cctggcgggg atgtcccccg gcgcgggcgc catggcgggc atgggcggct    480
cggccggggc ggccggcgtg gcgggcatgg ggccgcactt gagtcccagc ctgagcccgc    540
tcggggggca ggcggccggg gccatgggcg gcctggcccc ctacgccaac atgaactcca    600
tgagccccat gtacgggcag gcgggcctga gccgcgcccg cgaccccaag acctacaggc    660
gcagctacac gcacgcaaag ccgccctact cgtacatctc gctcatcacc atggccatcc    720
agcagagccc caacaagatg ctgacgctga gcgagatcta ccagtggatc atggacctct    780
tccccttcta ccggcagaac cagcagcgct ggcagaactc catccgccac tcgctctcct    840
tcaacgactg tttcctgaag gtgccccgct cgcccgacaa gcccggcaag ggctccttct    900
ggaccctgca ccctgactcg ggcaacatgt tcgagaacgg ctgctacctg cgccgccaga    960
agcgcttcaa gtgcgagaag cagctggcgc tgaaggaggc cgcaggcgcc gccggcagcg   1020
gcaagaaggc ggccgccgga gcccaggcct cacaggctca actcggggag gccgccgggc   1080
cggcctccga gactccggcg ggcaccgagt cgcctcactc gagcgcctcc ccgtgccagg   1140
agcacaagcg agggggcctg ggagagctga aggggacgcc ggctgcggcg ctgagccccc   1200
cagagccggc gccctctccc gggcagcagc agcaggccgc ggcccacctg ctgggcccgc   1260
cccaccaccc gggcctgccg cctgaggccc acctgaagcc ggaacaccac tacgccttca   1320
accacccgtt ctccatcaac aacctcatgt cctcggagca gcagcaccac cacagccacc   1380
accaccacca accccacaaa atggacctca aggcctacga acaggtgatg cactaccccg   1440
gctacggttc ccccatgcct ggcagcttgg ccatgggccc ggtcacgaac aaaacgggcc   1500
tggacgcctc gcccctggcc gcagatacct cctactacca gggggtgtac tcccggccca   1560
ttatgaactc ctcttaagaa gacgacggct tcaggcccgg ctaactctgg caccccggat   1620
cgaggacaag tgagagagca agtgggggtc gagactttgg ggagacggtg ttgcagagac   1680
gcaagggaga agaaatccat aacacccccc ccccaacacc cccaagacag cagtcttctt   1740
cacccgctgc agccgttccg tcccaaacag agggccacac agatacccca cgttctatat   1800
```

aaggaggaaa acgggaaaga atataaagtt aaaaaaaagc ctccggtttc cactactgtg 1860
tagactcctg cttcttcaag cacctgcaga ttctgatttt tttgttgttg ttgttctcct 1920
ccattgctgt tgttgcaggg aagtcttact taaaaaaaaa aaaaaatttt gtgagtgact 1980
cggtgtaaaa ccatgtagtt ttaacagaac cagagggttg tactattgtt taaaaacagg 2040
aaaaaaaata atgtaagggt ctgttgtaaa tgaccaagaa aaagaaaaaa aaagcattcc 2100
caatcttgac acggtgaaat ccaggtctcg ggtccgatta atttatggtt tctgcgtgct 2160
ttatttatgg cttataaatg tgtattctgg ctgcaagggc cagagttcca caaatctata 2220
ttaaagtgtt atacccggtt tt 2242

<210> 63
<211> 1047
<212> DNA
<213> NM_033423.2| Homo sapiens granzyme H (cathepsin G-like 2, protein h-CCPX) (GZMH), mRNA

<400> 63

ggagtcaaca ccaacagctc tgacctgggc agccttcctg agaaaatgca gccattcctc 60
ctcctgttgg cctttcttct gacccctggg gctgggacag aggagatcat cgggggccat 120
gaggccaagc cccactcccg cccctacatg gcctttgttc agtttctgca agagaagagt 180
cggaagaggt gtggcggcat cctagtgaga aaggactttg tgctgacagc tgctcactgc 240
cagggaagct ccataaatgt caccttgggg gcccacaata tcaaggaaca ggagcggacc 300
cagcagttta tccctgtgaa aagacccatc ccccatccag cctataatcc taagaacttc 360
tccaacgaca tcatgctact gcagctggag agaaaggcca agtggaccac agctgtgcgg 420
cctctcaggc tacctagcag caaggcccag gtgaagccag ggcagctgtg cagtgtggct 480
ggctggggtt atgtctcaat gagcacttta gcaaccacac tgcaggaagt gttgctgaca 540
gtgcagaagg actgccagtg tgaacgtctc ttccatggca attacagcag agccactgag 600
atttgtgtgg gggatccaaa gaagacacag accggtttca agggggactc cgggggcccc 660
ctcgtgtgta aggacgtagc ccaaggtatt ctctcctatg gaaacaaaaa agggacacct 720
ccaggagtct acatcaaggt ctcacacttc ctgccctgga taaagagaac aatgaagcgc 780
ctctaacagc aggcatgaga ctaaccttcc tctgggcctg accatctctg ggacagaggc 840
aagaatcccc aaggggtggg cagtcagggt tgcaggactg taataaatgg atctctggtg 900
tagaaaaaaa aaaaaaaaaa aaaaaaaaaa aaaaaaaaaa aaaaaaaaaa aaaaaaaaaa 960
aaaaaaaaaa aaaaaaaaaa aaaaaaaaaa aaaaaaaaaa aaaaaaaaaa aaaaaaaaaa 1020
aaaaaaaaaa aaaaaaaaaa aaaaaaa 1047

<210> 64
<211> 5243
<212> DNA

<213> NM_001165-3| Homo sapiens baculoviral IAP repeat-containing 3 (BIRC3), transcript variant 1, mRNA

<400> 64

```
agcgtgagac tcgcgccctc cggcacggaa aaggccaggc gacaggtgtc gcttgaaaag     60
actgggcttg tccttgctgg tgcatgcgtc gtcggcctct gggcagcagg tttacaaagg    120
aggaaaacga cttcttctag attttttttt cagtttcttc tataaatcaa aacatctcaa    180
aatggagacc taaaatcctt aaagggactt agtctaatct cgggaggtag ttttgtgcat    240
gggtaaacaa attaagtatt aactggtgtt ttactatcca aagaatgcta attttataaa    300
catgatcgag ttatataagg tataccataa tgagtttgat tttgaatttg atttgtggaa    360
ataaaggaaa agtgattcta gctggggcat attgttaaag catttttttc agagttggcc    420
aggcagtctc ctactggcac attctcccat tatgtagaat agaaatagta cctgtgtttg    480
ggaaagattt taaaatgagt gacagttatt tggaacaaag agctaataat caatccactg    540
caaattaaag aaacatgcag atgaaagttt tgacacatta aaatacttct acagtgacaa    600
agaaaaatca agaacaaagc tttttgatat gtgcaacaaa tttagaggaa gtaaaaagat    660
aaatgtgatg attggtcaag aaattatcca gttatttaca aggccactga tattttaaac    720
gtccaaaagt ttgtttaaat gggctgttac cgctgagaat gatgaggatg agaatgatgg    780
ttgaaggtta cattttagga aatgaagaaa cttagaaaat taatataaag acagtgatga    840
atacaaagaa gatttttata acaatgtgta aaatttttgg ccagggaaag gaatattgaa    900
gttagataca attacttacc tttgagggaa ataattgttg gtaatgagat gtgatgtttc    960
tcctgccacc tggaaacaaa gcattgaagt ctgcagttga aaagcccaac gtctgtgaga   1020
tccaggaaac catgcttgca aaccactggt aaaaaaaaaa aaaaaaaaaa aaaaaagcca   1080
cagtgacttg cttattggtc attgctagta ttatcgactc agaacctctt tactaatggc   1140
tagtaaatca taattgagaa attctgaatt ttgacaaggt ctctgctgtt gaaatggtaa   1200
atttattatt ttttttgtca tgataaattc tggttcaagg tatgctatcc atgaaataat   1260
ttctgaccaa aactaaattg atgcaatttg attatccatc ttagcctaca gatggcatct   1320
ggtaactttt gactgtttta aaaaataaat ccactatcag agtagatttg atgttggctt   1380
cagaaacatt tagaaaaaca aaagttcaaa aatgttttca ggaggtgata agttgaataa   1440
ctctacaatg ttagttcttt gagggggaca aaaaatttaa aatctttgaa aggtcttatt   1500
ttacagccat atctaaatta tcttaagaaa atttttaaca aagggaatga aatatatatc   1560
atgattctgt ttttccaaaa gtaacctgaa tatagcaatg aagttcagtt ttgttattgg   1620
tagtttgggc agagtctctt tttgcagcac ctgttgtcta ccataattac agaggacatt   1680
```

tccatgttct agccaagtat actattagaa taaaaaaact taacattgag ttgcttcaac 1740
agcatgaaac tgagtccaaa agaccaaatg aacaaacaca ttaatctctg attatttatt 1800
ttaaatagaa tatttaattg tgtaagatct aatagtatca ttatacttaa gcaatcatat 1860
tcctgatgat ctatgggaaa taactattat ttaattaata ttgaaaccag gttttaagat 1920
gtgttagcca gtcctgttac tagtaaatct ctttatttgg agagaaattt tagattgttt 1980
tgttctcctt attagaagga ttgtagaaag aaaaaaatga ctaattggag aaaaattggg 2040
gatatatcat atttcactga attcaaaatg tcttcagttg taaatcttac cattatttta 2100
cgtacctcta agaaataaaa gtgcttctaa ttaaaatatg atgtcattaa ttatgaaata 2160
cttcttgata acagaagttt taaaatagcc atcttagaat cagtgaaata tggtaatgta 2220
ttattttcct cctttgagtt aggtcttgtg cttttttttc ctggccacta aatttcacaa 2280
tttccaaaaa gcaaaataaa catattctga atatttttgc tgtgaaacac ttgacagcag 2340
agctttccac catgaaaaga agcttcatga gtcacacatt acatctttgg gttgattgaa 2400
tgccactgaa acattctagt agcctggaga agttgaccta cctgtggaga tgcctgccat 2460
taaatggcat cctgatggct taatacacat cactcttctg tgaagggttt taattttcaa 2520
cacagcttac tctgtagcat catgtttaca ttgtatgtat aaagattata caaaggtgca 2580
attgtgtatt tcttccttaa aatgtatcag tataggattt agaatctcca tgttgaaact 2640
ctaaatgcat agaaataaaa ataataaaaa atttttcatt ttggcttttc agcctagtat 2700
taaaactgat aaaagcaaag ccatgcacaa aactacctcc ctagagaaag gctagtccct 2760
tttcttcccc attcatttca ttatgaacat agtagaaaac agcatattct tatcaaattt 2820
gatgaaaagc gccaacacgt ttgaactgaa atacgacttg tcatgtgaac tgtaccgaat 2880
gtctacgtat tccacttttc ctgctggggt tcctgtctca gaaaggagtc ttgctcgtgc 2940
tggtttctat tacactggtg tgaatgacaa ggtcaaatgc ttctgttgtg gcctgatgct 3000
ggataactgg aaaagaggag acagtcctac tgaaaagcat aaaaagttgt atcctagctg 3060
cagattcgtt cagagtctaa attccgttaa caacttggaa gctacctctc agcctacttt 3120
tccttcttca gtaacaaatt ccacacactc attacttccg ggtacagaaa acagtggata 3180
tttccgtggc tcttattcaa actctccatc aaatcctgta aactccagag caaatcaaga 3240
tttttctgcc ttgatgagaa gttcctacca ctgtgcaatg aataacgaaa atgccagatt 3300
acttactttt cagacatggc cattgacttt tctgtcgcca acagatctgg caaaagcagg 3360
cttttactac ataggacctg gagacagagt ggcttgcttt gcctgtggtg gaaaattgag 3420
caattgggaa ccgaaggata atgctatgtc agaacacctg agacattttc ccaaatgccc 3480
atttatagaa aatcagcttc aagacacttc aagatacaca gtttctaatc tgagcatgca 3540
gacacatgca gcccgcttta aaacattctt taactggccc tctagtgttc tagttaatcc 3600
tgagcagctt gcaagtgcgg gtttttatta tgtgggtaac agtgatgatg tcaaatgctt 3660

ttgctgtgat ggtggactca ggtgttggga atctggagat gatccatggg ttcaacatgc 3720
caagtggttt ccaaggtgtg agtacttgat aagaattaaa ggacaggagt tcatccgtca 3780
agttcaagcc agttaccctc atctacttga acagctgcta tccacatcag acagcccagg 3840
agatgaaaat gcagagtcat caattatcca ttttgaacct ggagaagacc attcagaaga 3900
tgcaatcatg atgaatactc ctgtgattaa tgctgccgtg gaaatgggct ttagtagaag 3960
cctggtaaaa cagacagttc agagaaaaat cctagcaact ggagagaatt atagactagt 4020
caatgatctt gtgttagact tactcaatgc agaagatgaa ataagggaag aggagagaga 4080
aagagcaact gaggaaaaag aatcaaatga tttattatta atccggaaga atagaatggc 4140
actttttcaa catttgactt gtgtaattcc aatcctggat agtctactaa ctgccggaat 4200
tattaatgaa caagaacatg atgttattaa acagaagaca cagacgtctt tacaagcaag 4260
agaactgatt gatacgattt tagtaaaagg aaatattgca gccactgtat tcagaaactc 4320
tctgcaagaa gctgaagctg tgttatatga gcatttattt gtgcaacagg acataaaata 4380
tattcccaca gaagatgttt cagatctacc agtggaagaa caattgcgga gactacaaga 4440
agaaagaaca tgtaaagtgt gtatggacaa agaagtgtcc atagtgttta ttccttgtgg 4500
tcatctagta gtatgcaaag attgtgctcc ttctttaaga aagtgtccta tttgtaggag 4560
tacaatcaag ggtacagttc gtacatttct ttcatgaaga agaaccaaaa catcgtctaa 4620
actttagaat taatttatta aatgtattat aactttaact tttatcctaa tttggtttcc 4680
ttaaaatttt tatttattta caactcaaaa aacattgttt tgtgtaacat atttatatat 4740
gtatctaaac catatgaaca tatatttttt agaaactaag agaatgatag gcttttgttc 4800
ttatgaacga aaaagaggta gcactacaaa cacaatattc aatcaaaatt tcagcattat 4860
tgaaattgta agtgaagtaa aacttaagat atttgagtta acctttaaga attttaaata 4920
ttttggcatt gtactaatac cgggaacatg aagccaggtg tggtggtatg tgcctgtagt 4980
cccaggctga ggcaagagaa ttacttgagc ccaggagttt gaatccatcc tgggcagcat 5040
actgagaccc tgcctttaaa aacaaacaga acaaaaacaa aacaccaggg acacatttct 5100
ctgtcttttt tgatcagtgt cctatacatc gaaggtgtgc atatatgttg aatgacattt 5160
tagggacatg gtgtttttat aaagaattct gtgagaaaaa atttaataaa gcaacaaaaa 5220
ttactcttaa aaaaaaaaaa aaa 5243

<210> 65
<211> 3850
<212> DNA
<213> NM_005682.4| Homo sapiens G protein-coupled receptor 56 (GPR56), transcript variant 1, mRNA

<400> 65

agactgggtg cctgtggccc tgggaggagg tggaagggga ggagcaggcc acacaggcac 60
aggccggtga gggacctgcc cagacctgga gggtctcgct ctgtcacaca ggctggagtg 120
cagtggtgtg atcttggctc atcgtaacct ccacctcccg ggttcaagtg attctcatgc 180
ctcagcctcc cgagtagctg ggattacagg tggtgacttc caagagtgac tccgtcggag 240
gaaaatgact ccccagtcgc tgctgcagac gacactgttc ctgctgagtc tgctcttcct 300
ggtccaaggt gcccacggca ggggccacag ggaagacttt cgcttctgca gccagcggaa 360
ccagacacac aggagcagcc tccactacaa acccacacca gacctgcgca tctccatcga 420
gaactccgaa gaggccctca cagtccatgc ccctttccct gcagcccacc ctgcttcccg 480
atccttccct gaccccaggg gcctctacca cttctgcctc tactggaacc gacatgctgg 540
gagattacat cttctctatg gcaagcgtga cttcttgctg agtgacaaag cctctagcct 600
cctctgcttc cagcaccagg aggagagcct ggctcagggc cccccgctgt tagccacttc 660
tgtcacctcc tggtggagcc ctcagaacat cagcctgccc agtgccgcca gcttcacctt 720
ctccttccac agtcctcccc acacggccgc tcacaatgcc tcggtggaca tgtgcgagct 780
caaaagggac ctccagctgc tcagccagtt cctgaagcat ccccagaagg cctcaaggag 840
gccctcggct gcccccgcca gccagcagtt gcagagcctg gagtcgaaac tgacctctgt 900
gagattcatg gggacatgg tgtccttcga ggaggaccgg atcaacgcca cggtgtggaa 960
gctccagccc acagccggcc tccaggacct gcacatccac tcccggcagg aggaggagca 1020
gagcgagatc atggagtact cggtgctgct gcctcgaaca ctcttccaga ggacgaaagg 1080
ccggagcggg gaggctgaga agagactcct cctggtggac ttcagcagcc aagccctgtt 1140
ccaggacaag aattccagcc aagtcctggg tgagaaggtc ttggggattg tggtacagaa 1200
caccaaagta gccaacctca cggagcccgt ggtgctcact ttccagcacc agctacagcc 1260
gaagaatgtg actctgcaat gtgtgttctg ggttgaagac cccacattga gcagcccggg 1320
gcattggagc agtgctgggt gtgagaccgt caggagagaa acccaaacat cctgcttctg 1380
caaccacttg acctactttg cagtgctgat ggtctcctcg gtggaggtgg acgccgtgca 1440
caagcactac ctgagcctcc tctcctacgt gggctgtgtc gtctctgccc tggcctgcct 1500
tgtcaccatt gccgcctacc tctgctccag ggtgccccctg ccgtgcagga ggaaacctcg 1560
ggactacacc atcaaggtgc acatgaacct gctgctggcc gtcttcctgc tggacacgag 1620
cttcctgctc agcgagccgg tggccctgac aggctctgag gctggctgcc gagccagtgc 1680
catcttcctg cacttctccc tgctcacctg cctttcctgg atgggcctcg aggggtacaa 1740
cctctaccga ctcgtggtgg aggtctttgg cacctatgtc cctggctacc tactcaagct 1800
gagcgccatg ggctggggct tccccatctt tctggtgacg ctggtggccc tggtggatgt 1860
ggacaactat ggccccatca tcttggctgt gcataggact ccagagggcg tcatctaccc 1920
ttccatgtgc tggatccggg actccctggt cagctacatc accaacctgg gcctcttcag 1980
cctggtgttt ctgttcaaca tggccatgct agccaccatg gtggtgcaga tcctgcggct 2040

gcgcccccac acccaaaagt ggtcacatgt gctgacactg ctgggcctca gcctggtcct 2100
tggcctgccc tgggccttga tcttcttctc ctttgcttct ggcaccttcc agcttgtcgt 2160
cctctacctt ttcagcatca tcacctcctt ccaaggcttc ctcatcttca tctggtactg 2220
gtccatgcgg ctgcaggccc ggggtggccc ctcccctctg aagagcaact cagacagcgc 2280
caggctcccc atcagctcgg gcagcacctc gtccagccgc atctaggcct ccagcccacc 2340
tgcccatgtg atgaagcaga gattcggcct cgtcgcacac tgcctgtggc cccccgagccc 2400
ggcccagccc caggccagtc agccgcagac tttggaaagc ccaacgacca tggagagatg 2460
ggccgttgcc atggtggacg gactcccggg ctgggctttt gaattggcct tggggactac 2520
tcggctctca ctcagctccc acgggactca gaagtgcgcc gccatgctgc ctagggtact 2580
gtccccacat ctgtcccaac ccagctggag gcctggtctc tccttacaac ccctgggccc 2640
agccctcatt gctgggggcc aggccttgga tcttgagggt ctggcacatc cttaatcctg 2700
tgcccctgcc tgggacagaa atgtggctcc agttgctctg tctctcgtgg tcaccctgag 2760
ggcactctgc atcctctgtc attttaacct caggtggcac ccagggcgaa tggggcccag 2820
ggcagacctt cagggccaga gccctggcgg aggagaggcc ctttgccagg agcacagcag 2880
cagctcgcct acctctgagc ccaggccccc tccctccctc agccccccag tcctccctcc 2940
atcttccctg ggttctcct cctctcccag ggcctccttg ctccttcgtt cacagctggg 3000
ggtccccgat tccaatgctg tttttgggg agtggtttcc aggagctgcc tggtgtctgc 3060
tgtaaatgtt tgtctactgc acaagcctcg gcctgcccct gagccaggct cggtaccgat 3120
gcgtgggctg ggctaggtcc ctctgtccat ctgggccttt gtatgagctg cattgccctt 3180
gctcaccctg accaagcaca cgcctcagag gggccctcag cctctcctga agccctcttg 3240
tggcaagaac tgtggaccat gccagtcccg tctggtttcc atcccaccac tccaaggact 3300
gagactgacc tcctctggtg acactggcct agggcctgac actctcctaa gaggttctct 3360
ccaagccccc aaatagctcc aggcgccctc ggccgcccat catggttaat tctgtccaac 3420
aaacacacac gggtagattg ctggcctgtt gtaggtggta gggacacaga tgaccgacct 3480
ggtcactcct cctgccaaca ttcagtctgg tatgtgaggc gtgcgtgaag caagaactcc 3540
tggagctaca gggacaggga gccatcattc ctgcctggga atcctggaag acttcctgca 3600
ggagtcagcg ttcaatcttg accttgaaga tgggaaggat gttcttttta cgtaccaatt 3660
cttttgtctt ttgatattaa aaagaagtac atgttcattg tagagaattt ggaaactgta 3720
gaagagaatc aagaagaaaa ataaaaatca gctgttgtaa tcacctagca aactggaaaa 3780
aaaaaaaaaa aaaaaaaaaa aaaaaaaaaa aaaaaaaaaa aaaaaaaaaa aaaaaaaaaa 3840
aaaaaaaaaa 3850

<210> 66
<211> 372
<212> DNA
<213> NM_005953.2| Homo sapiens metallothionein 2A (MT2A), mRNA

<400> 66

agtcccagcg aacccgcgtg caacctgtcc cgactctagc cgcctcttca gcacgccatg 60
gatcccaact gctcctgcgc cgccggtgac tcctgcacct gcgccggttc ctgcaaatgc 120
aaagagtgca aatgcacttc gtgcaagaaa agctgctgct cctgctgccc tgtgggctgt 180
gccaagtgtg cccaaggctg catctgcaaa ggggcgtcgg acaagtgcag ctgctgcgcc 240
tgatgctggg acagccccgc tcccagatgt aaagaacgcg acttccacaa acctggattt 300
tttatgtaca accctgaccg tgaccgtttg ctatattcct tttctatga aataatgtga 360
atgataataa aa 372

<210> 67
<211> 4180
<212> DNA
<213> NM_015002.1| Homo sapiens F-box protein 21 (FBXO21), transcript variant 2, mRNA

<400> 67

gtacgcggac aagatggcgg cggcagcagt cgacagcgcg atggaggtgg tgccggcgct 60
ggcggaggag gccgcgccgg aggtagcggg cctcagctgc ctcgtcaacc tgccgggtga 120
ggtgctggag tacatcctgt gctgcggctc gctgacggcc gccgacatcg gccgtgtctc 180
cagcacctgc cggcggctgc gcgagctgtg ccagagcagc gggaaggtgt ggaaggagca 240
gttccgggtg aggtggcctt cccttatgaa acactacagc ccccaccgact acgtcaattg 300
gttggaagag tataaagttc ggcaaaaagc tgggttagaa gcgcggaaga ttgtagcctc 360
gttctcaaag aggttctttt cagagcacgt tccttgtaat ggcttcagtg acattgagaa 420
ccttgaagga ccagagattt tttttgagga tgaactggtg tgtatcctaa atatggaagg 480
aagaaaagct ttgacctgga aatactacgc aaaaaaaatt ctttactacc tgcggcaaca 540
gaagatctta aataatctta aggcctttct tcagcagcca gatgactatg agtcgtatct 600
tgaaggtgct gtatatattg accagtactg caatcctctc tccgacatca gcctcaaaga 660
catccaggcc caaattgaca gcatcgtgga gcttgtttgc aaaacccttc ggggcataaa 720
cagtcgccac cccagcttgg ccttcaaggc aggtgaatca tccatgataa tggaaataga 780
actccagagc caggtgctgg atgccatgaa ctatgtcctt tacgaccaac tgaagttcaa 840
ggggaatcga atggattact ataatgccct caacttatat atgcatcagg ttttgattcg 900
cagaacagga atcccaatca gcatgtctct gctctatttg acaattgctc ggcagttggg 960

agtcccactg gagcctgtca acttcccaag tcacttctta ttaaggtggt gccaaggcgc 1020
agaaggggcg accctggaca tctttgacta catctacata gatgcttttg ggaaaggcaa 1080
gcagctgaca gtgaaagaat gcgagtactt gatcggccag cacgtgactg cagcactgta 1140
tggggtggtc aatgtcaaga aggtgttaca gagaatggtg ggaaacctgt taagcctggg 1200
gaagcgggaa ggcatcgacc agtcatacca gctcctgaga gactcgctgg atctctatct 1260
ggcaatgtac ccggaccagg tgcagcttct cctcctccaa gccaggcttt acttccacct 1320
gggaatctgg ccagagaagg tgcttgacat cctccagcac atccaaaccc tagacccggg 1380
gcagcacggg gcggtgggct acctggtgca gcacactcta gagcacattg agcgcaaaaa 1440
ggaggaggtg ggcgtagagg tgaagctgcg ctccgatgag aagcacagag atgtctgcta 1500
ctccatcggg ctcattatga agcataagag gtatggctat aactgtgtga tctacggctg 1560
ggaccccacc tgcatgatgg gacacgagtg gatccggaac atgaacgtcc acagcctgcc 1620
gcacggccac caccagcctt tctataacgt gctggtggag gacggctcct gtcgatacgc 1680
agcccaagaa aacttggaat ataacgtgga gcctcaagaa atctcacacc ctgacgtggg 1740
acgctatttc tcagagttta ctggcactca ctacatccca aacgcagagc tggagatccg 1800
gtatccagaa gatctggagt ttgtctatga aacggtgcag aatatttaca gtgcaaagaa 1860
agagaacata gatgagtaaa gtctagagag gacattgcac ctttgctgct gctgctatct 1920
tccaagagaa cgggactccg gaagaagacg tctccacgga gccctcggga cctgctgcac 1980
caggaaagcc actccaccag tagtgctggt tgcctcctac taagtttaaa taccgtgtgc 2040
tcttccccag ctgcaaagac aatgttgctc tccgcctaca ctagtgaatt aatctgaaag 2100
gcactgtgtc agtggcatgg cttgtatgct tgtcctgtgg tgacagtttg tgacattctg 2160
tcttcatgag gtctcacagt cgacgctcct gtaatcattc tttgtattca ctccattccc 2220
ctgtctgtct gcatttgtct cagaacattt ccttggctgg acagatgggg ttatgcattt 2280
gcaataattt ccttctgatt tctctgtgga acgtgttcgg tcccgagtga ggactgtgtg 2340
tctttttacc ctgaagttag ttgcatattc agaggtaaag ttgtgtgcta tcttggcagc 2400
atcttagaga tggagacatt aacaagctaa tggtaattag aatcatttga atttattttt 2460
ttctaatatg tgaaacacag atttcaagtg ttttatcttt tttttttaaa tttaaatggg 2520
aatataacac agttttccct tccatattcc tctcttgagt ttatgcacat ctctataaat 2580
cattagtttt ctattttatt acataaaatt cttttagaaa atgcaaatag tgaactttgt 2640
gaatggattt ttccatactc atctacaatt cctccatttt aaatgactac ttttattttt 2700
taatttaaaa aatctacttc agtatcatga gtaggtctta catcagtgat gggttctttt 2760
tgtagtgaga catacaaatc tgatgttaat gtttgctctt agaagtcata ctccatggtc 2820
ttcaaagacc aaaaaatgag gttttgcctt tgtaatcagg aaaaaaaaaa attaatgaac 2880
cttaaaaaaa aaaaaaaagg ttttgaaggg aaaaaaagtg gtttcacacc tcttgttatt 2940

```
ccttagagtc acttcaaggc ctgtttgaat gtggcaggtt agaaagagag agaatgtctt    3000
tcatttgaag agtgttggac ttgtgtgaaa ggagatgtgc gtgttggaat ctgcttttcc    3060
aagccgccag ggtcctgacg gcagcaggac gaagcctgtt gtggcgtctt ctgggaaagc    3120
ctgaccgtgt gttcggacgg cactggctcc tttccgaagt tctcagtaac tgagcccaga    3180
gtaactgcac gcctttgtgc agctctggag ctccaccaac tctcggcctg ccagttctca    3240
agcgagctaa tcttgtcatt aatcgataga agctaacttc cgaagttagg acctagttac    3300
tttgctctca acatttaaaa taatgcagtt gctctagtga atggggcgtt aggggcctgt    3360
ctctgcacct gtctgtccat ctgcatgcag tattctcacc catgttgaat gcctgctgct    3420
tgtttaccct ttggaaaccc tggggtgacc aaggtttgga aagccacctg agaccacttc    3480
atagcaaggg aaggctttaa gcagttacta gaaagagatg gggatttggc ccctggctcc    3540
tccagcctga atgagctatt taatccactg tccatgttcc tcatcagtca aatccaaagt    3600
caaaggattt gaacctgcat ctggaaacgt aaccactcac agcacctggc ccgccaaggt    3660
tgggaggatt gtacactact ttcatttaaa ggggaaagtt tgataatacg gaattaatta    3720
atatgaatga gatgcattaa taagaacctg agcatgctga gagttgcaat tgttggtttt    3780
ctggtttgat tgatttcctt ttttcttaga cacatcaaag tcaagaaaga tggttttacc    3840
tttactgacc cagctgtaca tatgtatcta gactgttttt aaatgtcttt cttcatgaat    3900
gcttcatggg gctccaggaa gcctgtatca cctgtgtaag ttggtatttg ggcactttat    3960
atttttctaa aaacgtgttt tggatcctgt actctaataa atcataagtt tctttttaaa    4020
aattttccaa aacttttctc cattttaaaa agccctgtta taaacgttga actttcacaa    4080
tgttaaaatg ttaaatattt ggatatagca acttcttttc tcttcaaatg aatgccaaga    4140
ttttttgta caatgattaa taaatggaac ttatccagag                           4180
```

<210> 68
<211> 6276
<212> DNA
<213> NM_012156.2| Homo sapiens erythrocyte membrane protein band 4.1-like 1 (EPB41L1), transcript variant 1, mRNA

<400> 68

```
agtcggcatc catcagcggg cgggggtgtc gccgaacagg ctgctccgca gagcccgccg      60
cgaccccgcg ccgccccgcc ccgcggcctg cctgccagag gagccgaggg ggccgcccct     120
cgcccaacct gcccgacatg gggaaccccg ggcccaggcg tgctggtcac catgacaaca     180
gagacaggcc ccgactctga ggtgaagaaa gctcaggagg aggccccgca gcagcccgag     240
gctgctgccg ctgtgaccac ccctgtgacc cctgcaggcc acggccaccc agaggccaac     300
tccaatgaga agcatccatc ccagcaggac acgcggcctg ctgaacagag cctagacatg     360
```

```
gaggagaagg actacagtga ggccgatggc ctttcggaga ggaccacgcc cagcaaggcc      420
cagaaatcgc cccagaagat tgccaagaaa tacaagagtg ccatctgccg ggtcactctg      480
cttgatgcct cggagtatga gtgtgaggtg gagaaacatg gccggggcca ggtgctgttt      540
gacctggtct gtgaacacct caacctccta gagaaggact acttcggcct gaccttctgt      600
gatgctgaca gccagaagaa ctggctggac ccctccaagg agatcaagaa gcagatccgg      660
agtagcccct ggaattttgc cttcacagtc aagttctacc cgcctgatcc tgcccagctg      720
acagaagaca tcacaagata ctacctgtgc ctgcagctgc gggcagacat catcacgggc      780
cggctgccat gctcctttgt cacgcatgcc ctactgggct cctacgctgt gcaggctgag      840
ctgggtgact atgatgctga ggagcatgtg ggcaactatg tcagcgagct ccgcttcgcc      900
cctaaccaga cccgggagct ggaggagagg atcatggagc tgcataagac atataggggg      960
atgaccccgg gagaagcaga aatccacttc ttagagaatg ccaagaagct ttccatgtac     1020
ggagtagacc tgcaccatgc caaggactct gagggcatcg acatcatgtt aggcgtttgt     1080
gccaatggcc tgctcatcta ccgggaccgg ctgagaatca accgctttgc ctggcccaag     1140
atcctcaaga tctcctacaa gaggagtaac ttctatatca agatccggcc tggggagtat     1200
gagcaatttg agagcacaat tggctttaag ctcccaaacc accggtcagc caagagactg     1260
tggaaggtct gcatcgagca tcatacattc ttccggctgg tgtcccctga gcccccaccc     1320
aagggcttcc tggtgatggg ctccaagttc cggtacagtg ggaggaccca ggcacagact     1380
cgccaggcca gcgccctcat tgaccggcct gcacccttct ttgagcgttc ttccagcaaa     1440
cggtacacca tgtcccgcag ccttgatgga gcagagttct cccgcccagc ctcggtcagc     1500
gagaaccatg atgcagggcc tgacggtgac aagcgggatg aggatggcga gtctgggggg     1560
caacggtcag aggctgagga gggagaggtc aggactccaa ccaagatcaa ggagctaaag     1620
ccggagcagg aaaccacgcc gagacacaag caggagttct tagacaagcc agaagatgtc     1680
ttgctgaagc accaggccag catcaatgag ctcaaaagga ccctgaagga gcccaacagc     1740
aaactcatcc accgggatcg agactgggaa cgggagcgca ggctgccctc ctcccccgcc     1800
tcccccctcc ccaagggcac ccctgagaaa gccaatgaga gagcagggct gagggagggc     1860
tccgaggaga aagtcaaacc accacgtccc cgggccccag agagtgacac aggcgatgag     1920
gaccaggacc aggagaggga cacggtgttc ctgaaggaca accacctggc cattgagcgc     1980
aagtgctcca gcatcacggt cagctctacg tctagcctgg aggctgaggt ggacttcacg     2040
gtcattggtg actaccatgg cagcgccttc gaagacttct cccgcagcct gcctgagctc     2100
gaccgggaca aaagcgactc ggacactgag ggcctgctgt tctcccggga tctcaacaag     2160
ggggccccca gccaggatga tgagtctggg ggcattgagg acagcccgga tcgaggggcc     2220
tgctccaccc cggatatgcc ccagtttgag cccgtgaaaa cagaaaccat gactgtcagc     2280
agtctggcca ttagaaagaa gattgagccg gaggccgtac tgcagaccag agtctccgct     2340
atggataaca cccagcaggt tgatgggagt gcctcagtgg ggagggagtt catagcaacc     2400
```

actccctcca tcaccacgga gaccatatcg accaccatgg agaacagtct caagtccggg 2460
aagggggcag ctgccatgat cccaggccca cagacggtgg ccacggaaat ccgttctctt 2520
tctccgatca tcgggaaaga tgtcctcacc agcacctacg gcgccactgc ggaaaccctc 2580
tcaacctcca ccaccaccca tgtcaccaaa actgtgaaag gagggttttc tgagacaagg 2640
atcgagaagc gaatcatcat tactggggat gaagatgtcg atcaagacca ggccctggct 2700
ttggccatca aggaggccaa actgcagcat cctgatatgc tggtaaccaa agctgtcgta 2760
tacagagaaa cagacccatc cccagaggag agggacaaga agccacagga atcctgacct 2820
ctgtgaagag atcctggcat ttctggtcca acccaagcca gagaaccatt aagaaggggc 2880
cttcattctg gattctccga cgcaacactg acgtcccagc tgcgacgtac tgtcactgat 2940
gagagactgg gaagggaaaa gcatatatat atagatatat agagatatag atatatatac 3000
aggaaacacc gcatccttgc actgctgctg gggctggcag agcagttggc tgacagcaac 3060
aaccgacatc tgaacaccta catttccttt gcagacaaat tgaagaactg gtgggatttt 3120
tttcaagaaa aaaaattata taataactat aatcccttgc tcaccccttt cccccgccaa 3180
ataagaaacg caagccagac cacgatgatt gtagaagtcc ctcccgccct ggttctgcac 3240
gttacagtta gcagacgagc aattccattt gttcttctcc agcatctcta aggcccactt 3300
gaatgcaaag gaaaacactt gcacagcaaa gcaagagaag tcacagcagc aagacacgca 3360
cagtcaacca ttttccgaga aaaaaagaaa attccccact tggaaagaaa gaggaggaac 3420
actggattct tactttctgg atcttgacac tgggctgcaa aacctacctt cctctctccc 3480
gcctcccctc accctcaact ctcaatgtct tgctgtcatt ttctgtctcg gctccctcct 3540
ccccttccc ccttcccca ccccacaccc ttcaccctct gtgtcctggt ccttctgagg 3600
gccactgcag atgactctcc tttgaaatga gaaaaagaaa agaaagcaag aacagaaaac 3660
gaagccacag gaagggaagt agacattgta tgcttatggt ttctcattat gaaggtgcag 3720
cttgtaggag gtttgtacgg atgtgctttg aagttatgta tattacatat aacaggaaaa 3780
aatattaaaa taaacagtgc tggtaagtat gaagctgaca ttctaaaatt ataattatct 3840
gactgtgatt gatgtatcct gaggttccta gatctcactg aactggccca gctaaggaga 3900
cctggactct gggtgtgggt tggctcacag taggggctga cgggttcagt gtagtaatac 3960
tgtgtgtggt gtttgtaatt ggttgattgg tggggagggg tgggggccc taatggagag 4020
gtgtgggttt ggcaagaaag aagcaacaca gatgtcgtcc ccaaaatgcc agttcaagac 4080
accttctccc tgcccccctg gtagtaacag tcagggcctg gtctgtgctc aggtactggg 4140
tcccagtctg ggactctgct gctgaagttg ccacagtaga ggtccctggc ttagtcctta 4200
tctccctacg gggcttgcct tggttttcag tcttctctct ctttctctct tttttttttt 4260
tttgccacat tctgcccttc cctgacccca ttgtaataac caactccata tccaaaggga 4320
ggtggtgctc tcagccattg tagaagatgg tggctttaac ctgactgtct aaaaattccc 4380

agctaagcct tttcctctac tctcttcctt gttctgaatc atttcttctt ctcaggccaa 4440
agtagccatg gtaaggaggc ttcatggggc agaccctgaa agatcaaaac tgcatttgca 4500
aagccctccc ctgtcccagg acaaagctga gactgacggg tgatgttgct cataggctcc 4560
agctctgcat aagaccttgg cttggagacc tccctctcag tcaacagctg aactctgagc 4620
ttgtgcccag aaattacccc aagaccacag gaacccttca agaagctccc atcacaagct 4680
tggcattgct ctctgccaca cgtgggcttc ctcaggcttg tctgccacaa gctacttctc 4740
tgagctcaga aagtgcccct tgatgaggga aaatgtccca ctgcactgcg aatttctcag 4800
ttccatttta cctcccagtc ctccttctaa accagttaat aaattcattc cacaagtatt 4860
tactgattac ctgcttgtgc cagggactat tctcaggctg aagaaggtgg gaggggaggg 4920
cggaacctga ggagccacct gagccagctt tatatttcaa ccatggctgg cccatctgag 4980
agcatctccc cactctcgcc aacctatcgg ggcatagccc agggatgccc ccaggcggcc 5040
caggttagat gcgtcccttt ggcttgtcag tgatgacata caccttagct gcttagctgg 5100
tgctggcctg aggcagggca ggaaatcaga atagcatttg cttctctggg caaatgggaa 5160
gttcagcggg gcagcagaat cagtggcatt ccccctggtg caggccggtg ggtccactcc 5220
aactccccct gagtgtagca gcacactttc catacaccag gttctttcta caatcctggt 5280
ggaaaagcca cagaaccttc ttcctgccct tcttgagagt tccccctctt tctgggtcaa 5340
gagctggagt ggtggctcca tcctctctgg gccacttcgg tctaggaact catctttgca 5400
ggaaccagga gtcctgagca cactgaacac acctcagagg gaggatcctt gttgtggatt 5460
ttgcacctgg ctttggggca ggggtgaagt gaccaggctt agcttgtgga gtttatgggc 5520
caccagggtt tggggaaatc accatcccgc ggatgctgtg acctcccttc tacggagatg 5580
caggcagtgc cacgagggag gaggggacct gcaaagctag aatctagggc actgtttcct 5640
ccccatcctt ctctttgtag agaatagaga cgtttgtctt gtctgtcttc aacctacttt 5700
tccttttctc tttttgtttt ctcatcctct ctgtgccacc tctccaccca ggaggccatg 5760
tagcatagtg gaaaaagtcc ctgagggcgg ttaggagttc tgggtgacca tcctggctca 5820
gctcctaact caccatgtga catcaggcta tccccattcc ccctcttggg cctcagtttc 5880
ccgacttgca aaataagcag aaagaaccag atgctctcca gggtcttttt ctactttgct 5940
atctcatggg tcttcatttt ctcttatttt gttttctctg gatcttttcc atctgagggt 6000
acaggaagta ccaggacctg tttcagtttt tgaatcctgc aagcacattc caagactggc 6060
ctgaaactgc atgagcaaca tcactcgaaa taattttttt tttcaaaagc accttaacaa 6120
ccaattgcga tgctgtcctg ttccttttta ctcacaccct tctctccttt ctcgtcccca 6180
tgctccccca cctcagtgct ccgtgctgta tgcgtgtgct ctctgttctt gtatactcaa 6240
tataagtgaa ataaatgtgt ttgatgctga accata 6276

<210> 69
<211> 1209
<212> DNA
<213> NM_173834.2| Homo sapiens hypothetical protein MGC21416 (MGC21416), mRNA

<400> 69

```
gacccgggag aaggagggcc aagatggcgg aagcggagga gtctccagga gacccgggga     60
cagcatcgcc caggcccctg tttgcaggcc tttcagatat atccatctca caagacatcc    120
ccgtagaagg agaaatcacc attcctatga gatctcgcat ccgggagttt gacagctcca    180
cattaaatga atctgttcgc aataccatca tgcgtgatct aaaagctgtt gggaaaaaat    240
tcatgcatgt tttgtaccca aggaaaagta atactctttt gagagattgg gatttgtggg    300
gccctttgat cctttgtgtg acactcgcat taatgctgca aagagactct gcagatagtg    360
aaaaagatgg agggccccaa tttgcagagg tgtttgtcat tgtctggttt ggtgcagtta    420
ccatcacccct caactcaaaa cttcttggag ggaacatatc ttttttcag agcctctgtg    480
tgctgggtta ctgtatactt cccttgacag tagcaatgct gatttgccgg ctggtacttt    540
tggctgatcc aggacctgta aacttcatgg ttcggctttt tgtggtgatt gtgatgtttg    600
cctggtctat agttgcctcc acagctctcc ttgctgatag ccagcctcca aaccgcagag    660
ccctagctgt ttatcctgtt ttcctgtttt actttgtcat cagttggatg attctcacct    720
ttactcctca gtaaatcagg aatgggaaat taaaaaccag tgaattgaaa gcacatctga    780
aagatgcaat tcaccatgga gctttgtctc tggcccttat ttgtctaatt ttggaggtat    840
ttgataactg agtaggtgag gagattaaaa gggagccata tagcactgtc acccccttatt    900
tgaggaactg atgtttgaaa ggctgttctt ttctctctta atgtcatttc tttaaaaata    960
catgtgcata ctacacacag tatataatgc ctccttaagg catgatggag tcaccgtggt   1020
ccatttgggt gacaaccagt gacttgggaa gcacatagat acatcttaca agttgaatag   1080
agttgataac tattttcagt tttgagaata ccagttcagg tgcagctctt aaacacattg   1140
ccttatgact attagaatat gcctctcttt tcataaataa aaatacatgg tctaaaaaaa   1200
aaaaaaaaa                                                            1209
```

<210> 70
<211> 5249
<212> DNA
<213> NM_015352.1| Homo sapiens protein o-fucosyltransferase 1 (POFUT1), transcript variant 1, mRNA

<400> 70

```
cttccctccc cgactgtgcg ccgcggctgg ctcgggttcc cgggccgaca tgggcgccgc     60
cgcgtgggca cggccgctga gcgtgtcttt cctgctgctg cttctgccgc tcccggggat    120
```

gcctgcgggc tcctgggacc cggccggtta cctgctctac tgcccctgca tggggcgctt 180
tgggaaccag gccgatcact tcttgggctc tctggcattt gcaaagctgc taaaccgtac 240
cttggctgtc cctccttgga ttgagtacca gcatcacaag cctcctttca ccaacctcca 300
tgtgtcctac cagaagtact tcaagctgga gcccctccag gcttaccatc gggtcatcag 360
cttggaggat ttcatggaga agctggcacc cacccactgg ccccctgaga agcgggtggc 420
atactgcttt gaggtggcag cccagcgaag cccagataag aagacgtgcc ccatgaagga 480
aggaaacccc tttggcccat tctgggatca gtttcatgtg agtttcaaca agtcggagct 540
ttttacaggc atttccttca gtgcttccta cagagaacaa tggagccaga gattttctcc 600
aaaggaacat ccggtgcttg ccctgccagg agccccagcc cagttccccg tcctagagga 660
acacaggcca ctacagaagt acatggtatg gtcagacgaa atggtgaaga cgggagaggc 720
ccagattcat gcccaccttg tccggcccta tgtgggcatt catctgcgca ttggctctga 780
ctggaagaac gcctgtgcca tgctgaagga cgggactgca ggctcgcact tcatggcctc 840
tccgcagtgt gtgggctaca gccgcagcac agcggccccc ctcacgatga ctatgtgcct 900
gcctgacctg aaggagatcc agagggctgt gaagctctgg gtgaggtcgc tggatgccca 960
gtcggtctac gttgctactg attccgagag ttatgtgcct gagctccaac agctcttcaa 1020
agggaaggtg aaggtggtga gcctgaagcc tgaggtggcc caggtcgacc tgtacatcct 1080
cggccaagcc gaccacttta ttggcaactg tgtctcctcc ttcactgcct ttgtgaagcg 1140
ggagcgggac ctccagggga ggccgtcttc tttcttcggc atggacaggc cccctaagct 1200
gcgggacgag ttctgattct ggccggagca ccagaccctc tgatcctgga gggaccagag 1260
tctgagctgg tccttccagc caggcctggc agccagaggt gctccgggat tgcaaactcc 1320
tcttctcacc tgccaaagat ggagaagagt gccagggacc cctcaaggag ggagacgctc 1380
catatcccag ggcataggac ttgcaggttc ctaggagcag gagcatctcc catcgcacgt 1440
gctttctgct cttctgggaa tttctcacac tggcaaagca gtccagcctc cgtcttctgg 1500
tccactctgc tctgagcagc ctgggatgct gaactcttca gagagatttt tttatagaga 1560
gatttctata attttgatac aaggtcatga ctatcctaga actctctgtg gtttttgaaa 1620
atcattgaat tctattaatg taggtaccta aagtgacctt aactgaatgt ggatgaggct 1680
ggggctggtg tgggtctttt ggctgctttt caaggtgtcc cccaatgtgg ccctcaagag 1740
ccatccccac tgcctggcca gagccattgt tgtcccctac ttcctaggcc atttctgggg 1800
cttgggggat gaatgctgtc ctgtgctgta aacactatgc aaatggaagt tatcggttgt 1860
ggtgctgtgc agcgctctgt gggcgactaa gtgccactca cgcagcatgt tcctggcaag 1920
gagcacatac catcaagcca cactatcatg gtattgttct cacagtcttt tggtggttga 1980
tggccactgc aaacctggca ccatcagatc tcttctgatc tcttgcccca gtggggcctg 2040
gttggtagaa tgttggcatt cggttgatat ccaaagcctg ttctcccagc cgtcctcctg 2100

cagctggagc cttcaggccg tattctcacg agggaacgtt tgccaaggct ctgacctcac 2160
agaagatgcc cagggcccag aagccatcag aattatcagt ggagaagcac cttttgactc 2220
ttcccttcca atgtaatctc tgccaacacc atgaggctta aggtgctcta agtcatgagt 2280
gttttggtct caaatgctgc agttttaata atctgtgact cctgagagcc catggttttt 2340
tgaccttgtg gttctaaaat tccttgtctg acccctgtag atcttttcct tgccatgtca 2400
cctcccttgg cctttgatcc tggaaaggtg gcagagcctc cactgagcca ggcccagagc 2460
tccttgcagt gccttcttcc ttgtttacct gtgggaggaa acactttttt tgtcaggggc 2520
agcctggttc agagctcaga ggtcacactg tatcaaagat ctcaaacagc aaagtcagca 2580
tttgctgtat agagctgcca cccaactcta agcaggagaa actgtacaga aagggctttg 2640
ctatttttcc cttttgggaa aacaatgaag tgttttaagt cctgggtgga ctgagagatg 2700
gtttgcctgt ccagacttgc tctcaagcct catccagaga aggagctgca gatgagggag 2760
cccgtacact ccctgccacc actaggttgt aagcctgtag ctggctggct gatttcattt 2820
tggaattcat ttgccatcca cagccttaca ctaggcacac actttagagt ctggggctcc 2880
agtggggccc gcctaatttt ttttcccccc aagacagggc cttgctctgt ctcccaggct 2940
ggagtgcagt ggcatgatca tggcttactg cagccttgat ctcccaggct caagcgatcc 3000
ttctgcctca gcctctctgg tagctgagac tgcatgccca gctccaaatc accttgattc 3060
atatcagcag taataatcac ttgtgttctg aaagaaaggg caccagaagt tctagcaaaa 3120
ttcagttgtg ttctgtgagc tagcactttt tcctctgacc caattttctt acctataaaa 3180
tggtgataaa aaccgacagg ttgttcaaag gcccagatca gctaaagcat gtatataaga 3240
gcacgttgta aacttgaaag agacaaaggc acaaatgtgg ctgttgatta atttgactgc 3300
ttctcgttgc tcgtcacctc catgccaggc actgtgcttg ctaattgctt tatgggggca 3360
ttctcttatt tattccccag ccctgggaaa taggagctgt cattatcctt ctctttctgc 3420
acaaggaaaa attaatgccc tgagaattgt cataattttc ccaaggctgc ccagctggtg 3480
gtgttaagcc agaatttgac ctcccagagc cagtttccat tagctgccat gctctgctgc 3540
ctctaattca cagaatgcac tttctaccct gtgtgccatg gagacctcct atggaaaaat 3600
gatcagccac cttaccttct actgggtacc tgctgtgagt ctgcctatgc cagaaggatt 3660
aaggagggga ggttacccaa gaaacaaagc ctacatgccg cttacagccc ccgttggatg 3720
gttgctcagt acaacagtct tgcattcagc aggtgtttgt tcatcaccta ctatgtgtca 3780
ggctctatgc taggtactgg ggatacagga gagaatcaag cgtaaagtct ttgttctcaa 3840
ggaatttgca ttctagaaag tagaagatgt aataaatgta ctgtgggaca tgttaataag 3900
tgctataaag aaatataaag ggtttgggag caaaaagagg gagtggatct attttagatg 3960
agcccaggta agacctctct gaagagctgt catgaaggag ggagggagca cattcctggc 4020
agagaaaaca gcacgtgcaa aggccccgag actggagtgt gttcctgaag agcagccagg 4080
aggccagcat ggctggagag gcaggcatag gcagggaacc gagcagcagg tcagagcagg 4140

cgagctgaca ttctgcagcc tggacggcca tggcaggaag cttttagttg gagagataca 4200
ggaagcctcc tagggttctg agcagaagag gggcatgagc tgattcacat tctgaaggac 4260
ctctctagct ggccagtgct gaggaggttg gagagagaaa gggtgaaagc agagagacca 4320
gtgcagggct gttaacaggg ttgcaggcga gagactgggg tgctgggctc ccctagacta 4380
ggactccagt gccctcctct cccaagagac aaaggccatt gcattgaagg aggtgggaaa 4440
tgattagatt ctgaacatat gtaattattt ttcagtcttt ttcaaagata caaatattta 4500
catagtttta atcatgtaat atatacaatt taatgtccta gtgttttact taatagtgta 4560
tcatgttttc cctgttggta tgtagcctgg ataaatgctc ttaattataa aaaattctgt 4620
cgaggagtgt tccatagttt attgttttcc tattatgaga atttaggcca agtgtggtgg 4680
ctcatgcctg taatcccagc actttgcgag gccgaggtgg gcagatcact tgaggtgagg 4740
agttcaagac cagcctggcc aacatggtga attatctcta ctaaaaatac aaaaaaataa 4800
taataatagc caggcgtggt ggcacatgcc tgtattccca gctgcttggg aggctgaggc 4860
aggagaatgg cttgaacctg ggaggtggag gttgcagtga gccgagatgg tgccactgca 4920
ttccagcctg ggcaacagag cgagactcca tctcaaaaaa aaggagactt catgtgcccc 4980
caatttttca ctattgttat ttgaaaaaat atttttattt gtaagagttt ttctttattt 5040
aaaatgttca ttaataaagt tgttggacgg gaagcaaaaa aaaaaagttg tttaagataa 5100
attcccagaa gtgaatttgt tagatcaaac acttaaaact ttttgttatg gaagaattca 5160
aatataaata aaaaattgtg agtaataaaa tgaactcaca gtttcaacaa tgacccacaa 5220
aaaaaaaaaa aaaaaaaaaa aaaaaaaaa 5249

<210> 71
<211> 722
<212> DNA
<213> NM_175617.2| Homo sapiens metallothionein 1E (functional) (MT1E), mRNA

<400> 71

cttgttcgtc tcactggtgt gagctccagc atcccctttg ctcgaaatgg accccaactg 60
ctcttgcgcc actggtggct cctgcacgtg cgccggctcc tgcaagtgca aagagtgcaa 120
atgcacctcc tgcaagaaga gtgagtgcgg ggccatctcc aggaatctgg ggctgtggct 180
caggttggga gggaactcaa ggctggccct gagtgcatcc ttctggggaa ctgggctttc 240
tttgccctca ttgcccgtgt cattccctct ccaggctttc tgccctaaat tcagatgggg 300
caggacagca tttttctcgt gggacacaaa ccccaactgt accccctatg gtttcagaac 360
agagctgtgc cagacgaaaa aaagcatcct ctgggtctgg gttctgagct cgagccaggc 420
ttgctattag ggcagggagg tgcccggtca agtctactgc cacctctcac tctccccttc 480

ttccccaggc tgctgttcct gctgccccgt gggctgtgcc aagtgtgccc agggctgcgt 540
ctgcaaaggg gcatcggaga agtgcagctg ctgtgcctga tgtgggaaca gctcttctcc 600
cagatgtaaa tagaacaacc tgcacaacct ggattttttt aaaaatacaa cactgagcca 660
tttgctgcat ttctttttat actaaatatg tgactgacaa taaaaacaat tttgacttta 720
aa 722

<210> 72
<211> 980
<212> DNA
<213> NM_003283.3| Homo sapiens troponin T1, skeletal, slow (TNNT1), mRNA

<400> 72

agcaaggctc agcctcaaga ttcacagcat ctcagacgca gcctaggccg caccaggatg 60
tcggacaccg aggagcagga atatgaggag gagcagccgg aagaggaggc tgcggacgag 120
gaggaggaag cccccgaaga gccggagccg gtggcagagc cagaagagga acgccccaaa 180
ccaagccgcc ccgtggtgcc tcctttgatc ccgccaaaga tcccagaagg ggagcgcgtt 240
gacttcgatg acatccaccg caagcgcatg gagaaagacc tgctggagct gcagacactc 300
atcgatgtac atttcgagca gcggaagaag gaggaagagg agctggttgc cttgaaggag 360
cgcattgagc ggcgccggtc agagagagcc gagcaacagc gcttcagaac tgagaaggaa 420
cgcgaacgtc aggctaagct ggcggaggag aagatgagga aggaagagga agaggccaag 480
aagcgggcag aggatgatgc caagaaaaag aaggtgctgt ccaacatggg ggcccatttt 540
ggcggctacc tggtcaaggc agaacagaag cgtggtaagc ggcagacggg gcgggagatg 600
aaggtgcgca tcctctccga gcgtaagaag cctctggaca ttgactacat gggggaggaa 660
cagctccggg cccggtctgc ctggctgcct ccatcacagc cctcctgccc tgccagggag 720
aaagcccagg agctgtcgga ctggatccac cagctggagt ctgagaagtt cgacctgatg 780
gcgaagctga aacagcagaa atatgagatc aacgtgctgt acaaccgcat cagccacgcc 840
cagaagttcc ggaagggggc agggaagggc cgcgttggag gccgctggaa gtgaggatgc 900
cgccccggac agtggcacct gggaagcctg ggagtgtttg tcccatcggt agcttgaaat 960
aaacgctccc ctcagacacc 980

<210> 73
<211> 2213
<212> DNA
<213> NM_004067.1| Homo sapiens chimerin (chimaerin) 2 (CHN2), mRNA

<400> 73

gggcgtgcaa aggcgcggag cgggacggaa accacaaata aatagcggcg gcggcagcgc 60
gtcatctggt ggagcaggaa gtgcaggcag agtccggagg ctggtgcttt ctgcgcgtcc 120
ccaggacttt gccatgggct gggggccgcg gaggctgcga gcggccgggc gagggcagcg 180
gcggcggcgt ccccaccggg gctgagcgag cagcgacgcg aggggcgcgc ggagatggca 240
gcgtccagca actccagcct gtccggctcg tcggtgtcct ccgatgctga agaataccag 300
cctcctatat ggaaatcata cttatatcag ttacagcaag aggcacctcg tcccaagaga 360
atcatttgtc ctcgggaggt ggaaaacaga ccaaaatatt atggaagaga gtttcatggg 420
atcatctctc gggagcaggc ggatgagctt cttggaggcg tggagggtgc ctacatcctt 480
agagaaagcc agcggcaacc aggatgctac acgctggctc tcaggtttgg aaaccagacc 540
ttaaactaca ggctcttcca cgacgggaaa cactttgtgg gtgagaagag gtttgagtcg 600
attcatgatc tggtgacaga tggcttgata acactgtaca tagaaacaaa agctgccgag 660
tacatttcaa aaatgacaac taaccccatc tatgaacaca ttggatatgc caccctactc 720
agagaaaaag tatccagaag gctgagcagg tctaaaaatg aaccaagaaa aacaaacgtc 780
acacatgaag aacacacagc ggtggaaaag atctcctccc tggttcgaag ggctgccctc 840
acacacaacg acaaccactt caattatgag aagacacaca actttaaggt ccacacgttc 900
cgaggcccac actggtgtga atattgtgcc aatttcatgt gggggctcat cgcccaaggg 960
gtccggtgct cagactgtgg attgaacgta cacaaacagt gttccaagca cgttcccaat 1020
gactgccaac ctgatctcaa gaggatcaag aaagtgtact gttgtgacct cacaacactt 1080
gtgaaggctc acaacactca gagacccatg gtggtagaca tatgcattcg ggaaattgaa 1140
gcaagaggat taaaatcgga aggcctttac agagtctctg ggttcactga acacattgaa 1200
gatgtcaaaa tggcatttga cagagatggt gaaaaggccg atatatctgc caatgtctat 1260
ccagacataa acatcatcac tggagccctt aaactgtatt tcagagactt acccatccct 1320
gtcatcacat atgataccta ttccaaattt atagatgcag caaaaatctc caatgcagat 1380
gagaggctgg aagccgtcca tgaagtgctg atgctgctgc ctcctgccca ctatgaaacc 1440
ctccggtacc taatgatcca cctcaaaaag gttactatga atgaaaaaga caatttcatg 1500
aatgcagaaa atctggggat cgtgtttggg cccactctga tgaggccccc tgaggacagc 1560
accctgacca ccctgcatga tatgcggtac caaaagctga ttgtgcagat tttaatagaa 1620
aacgaagacg ttttattcta atccatcagg gaaatgagct gaatggccca gcaccatcaa 1680
gttgacacag ctaaggataa aacatttctt accacttgat ttgttttcca agcaagtgct 1740
agaatttgct ggactgcaga ggatcgctga gtggggtact gtgtctcata gacatgcgcc 1800
acctccacgt gagaacaagg gtgaaggtga gggaagcccc tcaggttggg tcttttgctg 1860
tgcctcctat gtatgtctgg tttgctggaa gagtgattaa tacatcttta atttattaaa 1920
aaacaatgta gacctttaaa cttcagtctt attgggaata aaagggaact taattcatac 1980

aggtacttga tacagttata cattttccac ttacaaaaag aagacaattc tgttaaatga 2040
aacgtgtatc gtaaaatgta attttattta cccacgagaa tgttgttatt ttagcaatag 2100
aactcaatgc agatgcattg gttattaccc tgtgtacctt gtccctcatt ttgctgtgac 2160
accctgaaaa agctgaccac aaatgcagta ttatcattga catacctctg tcc 2213

<210> 74
<211> 2201
<212> DNA
<213> NM_005520.1| Homo sapiens heterogeneous nuclear ribonucleoprotein H1 (H) (HNRPH1), mRNA

<400> 74

ttttttttt cgtcttagcc acgcagaagt cgcgtgtcta gtttgtttcg acgccggacc 60
gcgtaagaga cgatgatgtt gggcacggaa ggtggagagg gattcgtggt gaaggtccgg 120
ggcttgccct ggtcttgctc ggccgatgaa gtgcagaggt ttttttctga ctgcaaaatt 180
caaaatgggg ctcaaggtat tcgtttcatc tacaccagag aaggcagacc aagtggcgag 240
gcttttgttg aacttgaatc agaagatgaa gtcaaattgg ccctgaaaaa agacagagaa 300
actatgggac acagatatgt tgaagtattc aagtcaaaca acgttgaaat ggattgggtg 360
ttgaagcata ctggtccaaa tagtcctgac acggccaatg atggctttgt acggcttaga 420
ggacttccct ttggatgtag caaggaagaa attgttcagt tcttctcagg gttggaaatc 480
gtgccaaatg ggataacatt gccggtggac ttccagggga ggagtacggg ggaggccttc 540
gtgcagtttg cttcacagga aatagctgaa aaggctctaa agaaacacaa ggaaagaata 600
gggcacaggt atattgaaat ctttaagagc agtagagctg aagttagaac tcattatgat 660
ccaccacgaa agcttatggc catgcagcgg ccaggtcctt atgacagacc tggggctggt 720
agagggtata acagcattgg cagaggagct ggctttgaga ggatgaggcg tggtgcttat 780
ggtggaggct atggaggcta tgatgattac aatggctata atgatggcta tggatttggg 840
tcagatagat ttggaagaga cctcaattac tgtttttcag gaatgtctga tcacagatac 900
ggggatggtg gctctacttt ccagagcaca acaggacact gtgtacacat gcggggatta 960
ccttacagag ctactgagaa tgacatttat aatttttttt caccgctcaa ccctgtgaga 1020
gtacacattg aaattggtcc tgatggcaga gtaactggtg aagcagatgt cgagttcgca 1080
actcatgaag atgctgtggc agctatgtca aaagacaaag caaatatgca acacagatat 1140
gtagaactct tcttgaattc tacagcagga gcaagcggtg gtgcttacga acacagatat 1200
gtagaactct tcttgaattc tacagcagga gcaagcggtg gtgcttatgg tagccaaatg 1260
atgggaggca tgggcttgtc aaaccagtcc agctacgggg gcccagccag ccagcagctg 1320
agtgggggtt acggaggcgg ctacggtggc cagagcagca tgagtggata cgaccaagtt 1380

ttacaggaaa actccagtga ttttcaatca aacattgcat aggtaaccaa ggagcagtga 1440
acagcagcta ctacagtagt ggaagccgtg catctatggg cgtgaacgga atgggagggt 1500
tgtctagcat gtccagtatg agtggtggat ggggaatgta attgatcgat cctgatcact 1560
gactcttggt caaccttttt tttttttttt ttttctttaa gaaaacttca gtttaacagt 1620
ttctgcaata caagcttgtg atttatgctt actctaagtg gaaatcagga ttgttatgaa 1680
gacttaaggc ccagtatttt tgaatacaat actcatctag gatgtaacag tgaagctgag 1740
taaactataa ctgttaaact taagttccag cttttctcaa gttagttata ggatgtactt 1800
aagcagtaag cgtatttagg taaaagcagt tgaattatgt taaatgttgc cctttgccac 1860
gttaaattga acactgtttt ggatgcatgt tgaaagacat gcttttattt tttttgtaaa 1920
acaatatagg agctgtgtct actattaaaa gtgaaacatt ttggcatgtt tgttaattct 1980
agtttcattt aataacctgt aaggcacgta agtttaagct tttttttttt ttaagttaat 2040
gggaaaaatt tgagacgcaa taccaatact taggattttg gtcttggtgt ttgtatgaaa 2100
ttctgaggcc ttgatttaaa tctttcattg tattgtgatt tccttttagg tatattgcgc 2160
taagtgaaac ttgtcaaata aatcctcctt ttaaaaactg c 2201

<210> 75
<211> 1895
<212> DNA
<213> NM_004046.4| Homo sapiens ATP synthase, H+ transporting, mitochondrial F1 complex, alpha subunit, isoform 1, cardiac muscle (ATP5A1), nuclear gene encoding mitochondrial protein, transcript variant 2, mRNA

<400> 75

gtcttgacct tctttgcggc tcggccattt tgtcccagtc agtccggagg ctgcggctgc 60
agaagtaccg cctgcggagt aactgcaaag atgctgtccg tgcgcgttgc tgcggccgtg 120
gtccgcgccc ttcctcggcg ggccggactg gtctccagaa atgctttggg ttcatctttc 180
attgctgcaa ggaacttcca tgcctctaac actcatcttc aaaagactgg gactgctgag 240
atgtcctcta ttcttgaaga gcgtattctt ggagctgata cctctgttga tcttgaagaa 300
actgggcgtg tcttaagtat tggtgatggt attgcccgcg tacatgggct gaggaatgtt 360
caagcagaag aaatggtaga gttttcttca ggcttaaagg gtatgtcctt gaacttggaa 420
cctgacaatg ttggtgttgt cgtgtttgga aatgataaac taattaagga aggagatata 480
gtgaagagga caggagccat tgtggacgtt ccagttggtg aggagctgtt gggtcgtgta 540
gttgatgccc ttggtaatgc tattgatgga aagggtccaa ttggttccaa gacgcgtagg 600
cgagttggtc tgaaagcccc cggtatcatt cctcgaattt cagtgcggga accaatgcag 660
actggcatta aggctgtgga tagcttggtg ccaattggtc gtggtcagcg tgaactgatt 720
attggtgacc gacagactgg gaaaacctca attgctattg acacaatcat taaccagaaa 780

cgtttcaatg atggatctga tgaaaagaag aagctgtact gtatttatgt tgctattggt 840
caaaagagat ccactgttgc ccagttggtg aagagactta cagatgcaga tgccatgaag 900
tacaccattg tggtgtcggc tacggcctcg gatgctgccc cacttcagta cctggctcct 960
tactctggct gttccatggg agagtatttt agagacaatg gcaaacatgc tttgatcatc 1020
tatgacgact tatccaaaca ggctgttgct taccgtcaga tgtctctgtt gctccgccga 1080
ccccctggtc gtgaggccta tcctggtgat gtgttctacc tacactcccg gttgctggag 1140
agagcagcca aaatgaacga tgcttttggt ggtggctcct tgactgcttt gccagtcata 1200
gaaacacagg ctggtgatgt gtctgcttac attccaacaa atgtcatttc catcactgac 1260
ggacagatct tcttggaaac agaattgttc tacaaaggta tccgccctgc aattaacgtt 1320
ggtctgtctg tatctcgtgt cggatccgct gcccaaacca gggctatgaa gcaggtagca 1380
ggtaccatga agctggaatt ggctcagtat cgtgaggttg ctgcttttgc ccagttcggt 1440
tctgacctcg atgctgccac tcaacaactt ttgagtcgtg gcgtgcgtct aactgagttg 1500
ctgaagcaag gacagtattc tcccatggct attgaagaac aagtggctgt tatctatgcg 1560
ggtgtaaggg gatatcttga taaactggag cccagcaaga ttacaaagtt tgagaatgct 1620
ttcttgtctc atgtcgtcag ccagcaccaa gccttgttgg gcactatcag ggctgatgga 1680
aagatctcag aacaatcaga tgcaaagctg aaagagattg taacaaattt cttggctgga 1740
tttgaagctt aaactcctgt ggattcacat caaataccag ttcagttttg tcattgttct 1800
agtaaattag ttccatttgt aaaagggtta ctctcatact ccttatgtac agaaatcaca 1860
tgaaaaataa aggttccata atgcatagtt aaaaa 1895

<210> 76
<211> 1290
<212> DNA
<213> NM_001970.3| Homo sapiens eukaryotic translation initiation factor 5A (EIFSA), mRNA

<400> 76

gcggcggcgg cggtagaggc ggcggcggcg gcggcagcgg gctcggaggc agcggttggg 60
ctcgcggcga gcggacgggg tcgagtcagt gcgttcgcgc gagttggaat cgaagcctct 120
taaaatggca gatgacttgg acttcgagac aggagatgca ggggcctcag ccaccttccc 180
aatgcagtgc tcagcattac gtaagaatgg ctttgtggtg ctcaaaggcc ggccatgtaa 240
gatcgtcgag atgtctactt cgaagactgg caagcacggc cacgccaagg tccatctggt 300
tggtattgac atctttactg ggaagaaata tgaagatatc tgcccgtcaa ctcataatat 360
ggatgtcccc aacatcaaaa ggaatgactt ccagctgatt ggcatccagg atgggtacct 420
atcactgctc caggacagcg gggaggtacg agaggacctt cgtctccctg agggagacct 480

tggcaaggag attgagcaga agtacgactg tggagaagag atcctgatca cggtgctgtc 540
tgccatgaca gaggaggcag ctgttgcaat caaggccatg gcaaaataac tggctcccag 600
gatggcggtg gtggcagcag tgatcctctg aacctgcaga ggcccccctcc ccgagcctgg 660
cctggctctg gcccggtcct aagctggact cctcctacac aatttatttg acgttttatt 720
ttggttttcc ccacccccctc aatctgtcgg ggagcccctg cccttcacct agctcccttg 780
gccaggagcg agcgaagctg tggccttggt gaagctgccc tcctcttctc ccctcacact 840
acagccctgg tgggggagaa gggggtgggt gctgcttgtg gtttagtctt tttttttttt 900
tttttttttt ttttaaattc aatctggaat cagaaagcgg tggattctgg caaatggtcc 960
ttgtgccctc cccactcatc cctggtctgg tcccctgttg cccatagccc tttaccctga 1020
gcaccacccc aacagactgg ggaccagccc cctcgcctgc ctgtgtctct ccccaaaccc 1080
ctttagatgg ggagggaaga ggaggagagg ggaggggacc tgccccctcc tcaggcatct 1140
gggagggccc tgccccccatg ggctttaccc ttccctgcgg gctctctccc cgacacattt 1200
gttaaaatca aacctgaata aaactacaag tttaatatga aaaaaaaaaa aaaaaaaaaa 1260
aaaaaaaaaa aaaaaaaaaa aaaaaaaaaa 1290

<210> 77
<211> 2512
<212> DNA
<213> NM_005041.3| Homo sapiens perforin 1 (pore forming protein) (PRF1), mRNA

<400> 77

ggctggtgca aggagccaca gtgggctgcc tggggggctg atgccaccat tccaggagcc 60
tcggtgaaga gaggatatcc atctgtgtag ccgcttctct atacgggatt ccagctccat 120
ggcagcccgt ctgctcctcc tgggcatcct tctcctgctg ctgcccctgc ccgtccctgc 180
cccgtgccac acagccgcac gctcagagtg caagcgcagc cacaagttcg tgcctggtgc 240
atggctggcc ggggagggtg tggacgtgac cagcctccgc cgctcgggct ccttcccagt 300
ggacacacaa aggttcctgc ggcccgacgg cacctgcacc ctctgtgaaa atgccctaca 360
ggagggcacc ctccagcgcc tgcctctggc gctcaccaac tggcgggccc agggctctgg 420
ctgccagcgc catgtaacca gggccaaagt cagctccact gaagctgtgg cccgggatgc 480
ggctcgtagc atccgcaacg actggaaggt cgggctggac gtgactccta agcccaccag 540
caatgtgcat gtgtctgtgg ccggctcaca ctcacaggca gccaactttg cagcccagaa 600
gacccaccag gaccagtaca gcttcagcac tgacacggtg gagtgccgct tctacagttt 660
ccatgtggta cacactcccc cgctgcaccc tgacttcaag agggccctcg gggacctgcc 720
ccaccacttc aacgcctcca cccagcccgc ctacctcagg cttatctcca actacggcac 780

```
ccacttcatc cgggctgtgg agctgggtgg ccgcatatcg gccctcactg ccctgcgcac    840
ctgcgagctg gccctggaag ggctcacgga caacgaggtg gaggactgcc tgactgtcga    900
ggcccaggtc aacataggca tccacggcag catctctgcc gaagccaagg cctgtgagga    960
gaagaagaag aagcacaaga tgacggcctc cttccaccaa acctaccggg agcgccactc   1020
ggaagtggtt ggcggccatc acacctccat taacgacctg ctgttcggga tccaggccgg   1080
gcccgagcag tactcagcct gggtaaactc gctgcccggc agccctggcc tggtggacta   1140
caccctggaa cccctgcacg tgctgctgga cagccaggac ccgcggcggg aggcactgag   1200
gagggccctg agtcagtacc tgacggacag ggctcgctgg agggactgca gccggccgtg   1260
cccaccaggg cggcagaaga gcccccgaga cccatgccag tgtgtgtgcc atggctcagc   1320
ggtcaccacc caggactgct gccctcggca gaggggcctg gcccagctgg aggtgacctt   1380
catccaagca tggggcctgt ggggggactg gttcactgcc acggatgcct atgtgaagct   1440
cttctttggt ggccaggagc tgaggacgag caccgtgtgg gacaataaca accccatctg   1500
gtcagtgcgg ctggattttg gggatgtgct cctggccaca ggggggcccc tgaggttgca   1560
ggtctgggat caggactctg gcagggacga tgacctcctt ggcacctgtg atcaggctcc   1620
caagtctggt tcccatgagg tgagatgcaa cctgaatcat ggccacctaa aattccgcta   1680
tcatgccagg tgcttgcccc acctgggagg aggcacctgc ctggactatg tcccccaaat   1740
gcttctgggg gagcctccag gaaaccggag tggggccgtg tggtgagaac agtgagcttg   1800
gaaaggacca gtatgcttgg actgaagggg ttctcacagt gggagccagg gctgtcttcg   1860
tattcccatt agaccaagct tgtccaaccc gaggcccgca tgcggcccag gatggctttg   1920
aatgcggccc aacgcaaatt cgcaaacttt cttaaaacat tatgagtttc tttttgctat   1980
tttttttttt tttttagctc atcggctatc gttagtgcta gtggatttta catgtggccc   2040
aacacaattc ttcttccaac gtggcccaga gaagccaaaa gattggatac gcatcagaca   2100
gatggaaaag ggagattcag actgtttttc agggaggtgg ctgggtttac acgctaatcc   2160
cgattcaccc tgtccaaact gcctaagccc tccgccattc tcaagccctg cagtcacagc   2220
tacacagatc acagcttcag ccaggagctg ggcagaaggc caagaggctg ttcccaccag   2280
gctgctcagg gctggtcttt taggaccctt cccttgagcc ctctatggtg tggcaaagcc   2340
ttcattgcct taactggagc cccatcagct ccagctgctc tgtcttcttt gcccacaatg   2400
ctttgcccct gagacaaatg gaggcctgtc ctgacctgtc tcaccatgta catagcttga   2460
taaagggcca ataaatatga tgttatggtg aaaaaaaaaa aaaaaaaaaa aa           2512
```

<210> 78
<211> 4623
<212> DNA
<213> NN_014965.2| Homo sapiens OGT(O-Glc-NAC transferase)-interacting protein 106 KDa (OIP106), mRNA

<400> 78

gatgctgggc caggagcttt gtgtacaccc ctccacttca gctgagccag ggcatgtctg 60
cggcccaggc cagggcgcag tgtgtgccct gggggcccag gcctgcatgg ctcctctggg 120
tagggggtcg ggggcacccc caaggatggt cccttagggt gatgttttgg ctttggggtg 180
acttcagcaa tgtccctgcg agacaagggc ggggaagaag aatgttttga atacgactgc 240
caggatgaag agaggaagcc aacccacagg cagcatgaca cccaggacct cttggaagag 300
gttttatgtg ctgaaagagt tggccagatg actaagacat ataatgacat agatgctgtc 360
actcggcttc ttgaggagaa agagcgggat ttagaattgg ccgctcgcat cggccagtcg 420
ttgttgaaga agaacaagac cctaaccgag aggaacgagc tgctggagga gcaggtggaa 480
cacatcaggg aggaggtgtc tcagctccgg catgagctgt ccatgaagga tgagctgctt 540
cagttctaca ccagcgctgc ggaggagagt gagcccgagt ccgtttgctc aaccccgttg 600
aagaggaatg agtcgtcctc ctcagtccag aattactttc atttggattc tcttcaaaag 660
aagctgaaag accttgaaga ggagaatgtt gtacttcgat ccgaggccag ccagctgaag 720
acagagacca tcacctatga ggagaaggag cagcagctgg tcaatgactg cgtgaaggag 780
ctgagggatg ccaatgtcca gattgctagt atctcagagg aactggccaa gaagacggaa 840
gatgctgccc gccagcaaga ggagatcaca cacctgctat cgcaaatagt tgatttgcag 900
aaaaaggcaa aagcttgcgc agtggaaaat gaagaacttg tccagcatct gggggctgct 960
aaggatgccc agcggcagct cacagccgag ctgcgtgagc tggaggacaa gtacgcagag 1020
tgcatggaga tgctgcatga ggcgcaggag gagctgaaga acctccggaa caaaaccatg 1080
cccaatacca cgtctcggcg ctaccactca ctgggcctgt ttcccatgga ttccttggca 1140
gcagagattg agggaacgat gcgcaaggag ctgcagttgg aagaggccga gtctccagac 1200
atcactcacc agaagcgtgt ctttgagaca gtaagaaaca tcaaccaggt tgtcaagcag 1260
agatctctga ccccttctcc catgaacatc cccggctcca accagtcctc ggccatgaac 1320
tccctcctgt ccagctgcgt cagcaccccc cggtccagct tctacggcag cgacataggc 1380
aacgtcgtcc tcgacaacaa gaccaacagc atcattctgg aaacagaggc agccgacctg 1440
ggaaacgatg agcggagtaa gaagccgggg acgccgggca ccccaggctc ccacgacctg 1500
gagacggcgc tgaggcggct gtccctgcgc cgggagaact acctctcgga gaggaggttc 1560
tttgaggagg agcaagagag gaagctccag gagctggcgg agaagggcga gctgcgcagc 1620
ggctccctca cacccactga gagcatcatg tccctgggca cgcactcccg cttctccgag 1680
ttcaccggct tctctggcat gtccttcagc agccgctcct acctgcctga gaagctccag 1740
atcgtgaagc cgctggaagg ttccgccaca cttcaccact ggcagcagtt ggcccaacct 1800
caccttgggg gcatcctgga cccccggccc ggtgtggtca ccaagggctt ccggacgctg 1860

gatgttgacc tggacgaagt gtactgcctt aacgactttg aagaagatga cacaggtgac 1920
cacatttctc tcccacgcct agctacctcc actccagttc agcacccaga gacctcaggt 1980
gagaggtccc aagcacgtgt gactgtctca ggcagcagaa gttacccgag ccggcctcag 2040
gcttccccag aggagatgca ggagccgcca gcggccacgg aggaggagga ggaggaggag 2100
gaggaggagg ggtctggtga gggcaccacg ataagtcctg taaacttggc acctttcccg 2160
gaggcagagt tttgggccat tctcacctct gttccaggca ccatccgtag tggttctctg 2220
tctgtagctt ccgctcgtct gtgtgggtga tgattaaagc attctcattg cacagttctg 2280
tttttaaata cagagtctga tgcctcctat ttgtaacaat gggtgtagct cccctgccca 2340
tcttggaggt gcatggccca tcagggatct ttaaagtggg agcaggaaag gctgctaaaa 2400
aaaaaaaaaa aaaaagtggg cttttgggtc cctgaaaaca tcagtgccct tcttcctggt 2460
ctgggtgtct ccctgagtct aaggggaaga ttctcaagtc ccctggtgat ttccaagtgg 2520
agctgagcag ttttagggaa attgagtgct gggtcattca gaaggtaaat gagatcatct 2580
gttacctgta cgctgtatta aaatagaacc aggaaaggct caggatttca gacatttcgt 2640
cagccttttc actttcccag cttcaatgga ggtatatatg tcattttctt ttcagcttac 2700
acatgtgttc aaagtggatt tttaaaaagt gttttagcaa tactccttaa ccaaataaac 2760
cttcggagaa catcactaag cttttccaga gagaagaccc tagatgaagt tggaaaagag 2820
ctctggctga ctccacccac tgtccccaag cattaaaggt gtggccatga gtttacagaa 2880
ttacccacat tccagttgcc actgggatga aaagctttgt gctcagagct ctgggaatca 2940
tgggatcaca tggttactgt ttaccccaac agcatgctct gtctagactg gacctcccag 3000
ccccttgtgt tgggaaggca aagcttttgt ggagtcaggg gggagactga gaaaaaatga 3060
attaaccctg tgttgtcatc ctcatgacat tcctgaggat tcaccaggtt agaagtgagg 3120
acgtttatct ttgtgattca taattttcat ttgtgaaggc cacaacactc cccttgaaat 3180
acagggcagg aaagagctga ggttcttggt ggtgtctccc attcccctgg ctaatttcag 3240
acagctgtgg tcaagggatt ttacttggga tcaacttttc ctttttttcct tgacattaat 3300
tttagagaag tcatcaagtc atgtgatttg tttagcacat aggcttattt atggtttgat 3360
tttttttagg cagttatatt actagattaa gcttgtgagg gaatgaaaat gtttttttatt 3420
tgttatctac acactcgaaa aagagaaacc agctgcggta ctgtcccatt tttgtcatca 3480
gcaccagtgt ccgtcaggaa ggcaggtggt ggtgcagaaa catgatgcct ggctgatttt 3540
cgtggctaaa ggggtaggcc ttatgttgat tgggatgctc ccctacagcc ttacaggtag 3600
aatagaaggt gagttctgga agtgcaaaga agcaccatta agtgcatctt ctagaagttg 3660
tacagaagga ctaaagctta tcaagtcaac aaagaactta ccttggagga tagagagaga 3720
gaagggacca attatgagga ctgacatcct ggccactctc cttaaaataa acactgacat 3780
ttttcttgct tgttctctct gtactcaaac ctgtggcaaa ttcatcctag caacgttatt 3840

tgacgagggg catgaacatt tatagttgaa actgtagaaa gggtcaggtt ggaggtgtgt 3900
aataaaaaag aattacctag gttgccaaag gtaatttagg aagggtctga tcatttagat 3960
gagagttctt tggggcttat tttctgggta aggctcatct ttaaaaactg gcttcagagg 4020
ggagagggga gaacaatgaa ttggctctat tttctctatt gggaattaca ggaccatttt 4080
gattcttaga atgtaaaaag catatcgcta agtaaatcat cctggaggtc ccaagtagct 4140
ctatgcctgc aatcatggag acacaggcag acagataagc ttcatgggga aggcatgggg 4200
catcctctgt cttgggattt gtatccatgg tggtctggtc cctgcctttt aatccgtcct 4260
ctacgcttgg gcttttctgt taccaaacag cactatccca ggaactattg tctgcctggg 4320
aacactcagt agggagacac tttggagaca ggaggtgatg aacctttttta tgtgcagctg 4380
gtatgataga aggaaattgg gaaaacttgt atgctaggca cttttgtcca gagcctgctg 4440
tcccatggag aaaaagtttt aagcactgaa aaaatttgat taatgtattt aaatgtatta 4500
tttgaagcat cattcacttg ttgattttta caatcccatg tcttaaaaag gatgaatcca 4560
tgttattgta ttgtaaataa tttagattat taaaatggat tgtttaaaaa aaaaaaaaaa 4620
aaa 4623

<210> 79
<211> 2657
<212> DNA
<213> NM_017895.6| Homo sapiens DEAD (Asp-Glu-Ala-Asp) box polypeptide 27 (DDX27), mRNA

<400> 79

aagtgacgca tggtacttgc gcaaagacga cgaggaggct gcgaaaagtt aagggccgga 60
ccgcaggctg tgctcgcttc cggaagtggc ttctgcgaca acatgcttgc ggacctcggc 120
ttaatcggaa ccataggcga ggatgacgag gtgccggtgg agcccgagtc tgactccggg 180
gacgaggaag aggaggggcc cattgtgctg ggcagacgac aaaaagcttt ggggaagaac 240
cgcagtgctg atttcaaccc tgatttcgtt ttcactgaga aggaggggac gtacgatggc 300
agctgggccc tggctgatgt catgagccaa ctcaagaaga agagggcagc cactacatta 360
gatgagaaga ttgagaaagt tcgaaagaaa aggaaaacag aggataaaga agccaagtct 420
gggaagttgg aaaaggagaa agaagcaaag gaaggctctg aaccaaagga gcaggaagac 480
cttcaagaga atgatgagga aggctcagaa gatgaagcct cggagactga ctactcatca 540
gctgatgaga acatcctcac caaagcagat acactcaaag taaaggatcg gaagaagaag 600
aagaagaaag gacaggaagc aggaggattt tttgaagatg catctcagta cgatgaaaac 660
ctctcgttcc aggacatgaa cctttcccgc cctcttctga aggccattac agccatgggc 720
ttcaagcagc ccaccccgat ccagaaggcg tgcatacctg tgggtctatt ggggaaggac 780

atctgtgcct gtgcagccac tgggacaggt aaaactgccg cctttgccct gcctgttttg 840
gagcgtctga tttataaacc ccgccaggct ccagtcaccc gcgtgctggt gctagtgccc 900
acccgagagc tgggcatcca ggtgcactct gtcaccagac agctggccca gttctgcaac 960
atcaccacct gcctggctgt gggcggcttg gatgtgaagt ctcaggaagc agctcttcgg 1020
gcagcgcctg acatcctcat cgccacccca ggccggctca tcgatcacct ccacaactgc 1080
ccttccttcc acctgagcag catcgaggtg ctcatcctgg acgaggctga caggatgctg 1140
gatgagtact ttgaggagca gatgaaggag atcatccgaa tgtgttccca ccaccgccag 1200
accatgctct tctcggccac catgacagac gaggtgaaag atctggcttc tgtctccttg 1260
aagaatcctg tccggatatt tgtgaacagc aacacagatg tggctccctt cctgcggcag 1320
gagttcatcc ggatccggcc taatcgtgaa ggagaccggg aagccatcgt ggcagctttg 1380
ttgacgagga ccttcactga ccatgtgatg ctgttcacgc aaaccaagaa gcaggcccac 1440
cgcatgcaca tcctcctggg gctcatgggg ctgcaggtgg gtgagctcca tggcaacttg 1500
tcacagacgc agcggctgga ggccctccgg cgttttaagg atgaacagat tgacatcctc 1560
gtggccactg atgtggcagc ccgtggactt gacattgagg gggtcaaaac ggtaatcaac 1620
ttcacaatgc ctaataccat caaacattat gtccaccggg tggggcgaac agcacgtgct 1680
ggcagggctg ggcgctcagt ctctctggtg ggagaagatg agcggaagat gctgaaggag 1740
attgtaaaag ctgccaaggc ccctgtgaag gccaggatac ttccccaaga tgtcatcctc 1800
aaattccggg acaagattga gaaaatggag aaagatgtgt atgcagttct gcagctagag 1860
gcggaggaaa aagagatgca gcagtcagaa gcccagatca atacagcaaa gcggctcctg 1920
gagaagggga aggaggcagt ggtccaagag cccgagagga gctggttcca gaccaaagaa 1980
gagaggaaga aggagaaaat tgccaaagct ctgcaggaat ttgacttggc cttaagagga 2040
aagaagaaaa ggaagaagtt tatgaaggat gccaaaaaaa agggggagat gacagcagag 2100
gaaaggtctc agtttgaaat cctcaaggcg cagatgtttg ctgaacggct agcgaagagg 2160
aatcgcagag ccaagcgggc ccgagcaatg cccgaggagg agccagtgag aggtcctgcc 2220
aagaagcaaa agcaggggaa gaaatctgta tttgatgaag aactcaccaa cacaagcaag 2280
aaggccctga aacagtatcg agctagccct tcctttgaag aaaggaaaca gttgggcttg 2340
ccccaccaga gacgaggagg aaactttaaa tctaaatcca gatacaagag gaggaagtag 2400
ctgtcgtggc ctgaagaaat tcatgggggc agcccttaaa tcccttccct gtgggaagtc 2460
atcctggctg gtctgtcttt tctccatttg tttaaaaaaa aaacaaaaac aaaaaacaac 2520
actttggtgt ggtggtatgg tacgtagcta ttttcctaag catgtctgtc aatctccctt 2580
cttgctgatt agctttcata tgactatatt aaatggaagt atttttggga aaagagaaac 2640
caaaaaaaaa aaaaaaa 2657

<210> 80
<211> 3246
<212> DNA

<213> NM_018206.3| Homo sapiens vacuolar protein sorting 35 (yeast) (VPS35), mRNA

<400> 80

```
ctacgcgcgg ggcgggtgct gcttgctgca ggctctgggg agtcgccatg cctacaacac      60
agcagtcccc tcaggatgag caggaaaagc tcttggatga agccatacag gctgtgaagg     120
tccagtcatt ccaaatgaag agatgcctgg acaaaaacaa gcttatggat gctctaaaac     180
atgcttctaa tatgcttggt gaactccgga cttctatgtt atcaccaaag agttactatg     240
aactttatat ggccatttct gatgaactgc actacttgga ggtctacctg acagatgagt     300
ttgctaaagg aaggaaagtg gcagatctct acgaacttgt acagtatgct ggaaacatta     360
tcccaaggct ttacctttg atcacagttg gagttgtata tgtcaagtca tttcctcagt      420
ccaggaagga tattttgaaa gatttggtag aaatgtgccg tggtgtgcaa catcccttga     480
ggggtctgtt tcttcgaaat taccttcttc agtgtaccag aaatatctta cctgatgaag     540
gagagccaac agatgaagaa acaactggtg acatcagtga ttccatggat tttgtactgc     600
tcaactttgc agaaatgaac aagctctggg tgcgaatgca gcatcaggga catagccgag     660
atagagaaaa aagagaacga gaaagacaag aactgagaat tttagtggga acaaatttgg     720
tgcgcctcag tcagttggaa ggtgtaaatg tggaacgtta caaacagatt gttttgactg     780
gcatattgga gcaagttgta aactgtaggg atgctttggc tcaagaatat ctcatggagt     840
gtattattca ggttttccct gatgaatttc acctccagac tttgaatcct tttcttcggg     900
cctgtgctga gttacaccag aatgtaaatg tgaagaacat aatcattgct ttaattgata     960
gattagcttt atttgctcac cgtgaagatg gacctggaat cccagcggat attaaacttt    1020
ttgatatatt ttcacagcag gtggctacag tgatacagtc tagacaagac atgccttcag    1080
aggatgttgt atctttacaa gtctctctga ttaatcttgc catgaaatgt taccctgatc    1140
gtgtggacta tgttgataaa gttctagaaa caacagtgga gatattcaat aagctcaacc    1200
ttgaacatat tgctaccagt agtgcagttt caaaggaact caccagactt ttgaaaatac    1260
cagttgacac ttacaacaat attttaacag tcttgaaatt aaaacatttt cacccactct    1320
ttgagtactt tgactacgag tccagaaaga gcatgagttg ttatgtgctt agtaatgttc    1380
tggattataa cacagaaatt gtctctcaag accaggtgga ttccataatg aatttggtat    1440
ccacgttgat tcaagatcag ccagatcaac ctgtagaaga ccctgatcca gaagattttg    1500
ctgatgagca gagccttgtg ggccgcttca ttcatctgct gcgctctgag gaccctgacc    1560
agcagtactt gattttgaac acagcacgaa aacattttgg agctggtgga aatcagcgga    1620
ttcgcttcac actgccacct ttggtatttg cagcttacca gctggctttt cgatataaag    1680
agaattctaa agtggatgac aaatgggaaa agaaatgcca gaagattttt tcatttgccc    1740
```

accagactat cagtgctttg atcaaagcag agctggcaga attgccctta agactttttc 1800
ttcaaggagc actagctgct ggggaaattg gttttgaaaa tcatgagaca gtcgcatatg 1860
aattcatgtc ccaggcattt tctctgtatg aagatgaaat cagcgattcc aaagcacagc 1920
tagctgccat caccttgatc attggcactt ttgaaaggat gaagtgcttc agtgaagaga 1980
atcatgaacc tctgaggact cagtgtgccc ttgctgcatc caaacttcta aagaaacctg 2040
atcagggccg agctgtgagc acctgtgcac atctcttctg gtctggcaga aacacggaca 2100
aaaatgggga ggagcttcac ggaggcaaga gggtaatgga gtgcctaaaa aaagctctaa 2160
aaatagcaaa tcagtgcatg gacccctctc tacaagtgca gctttttata gaaattctga 2220
acagatatat ctatttttat gaaaaggaaa atgatgcggt aacaattcag gttttaaacc 2280
agcttatcca aaagattcga gaagacctcc cgaatcttga atccagtgaa gaaacagagc 2340
agattaacaa acattttcat aacacactgg agcatttgcg cttgcggcgg gaatcaccag 2400
aatccgaggg gccaatttat gaaggtctca tcctttaaaa aggaaatagc tcaccatact 2460
cctttccatg tacatccagt gagggtttta ttacgctagg tttcccttcc atagattgtg 2520
cctttcagaa atgctgaggt aggtttccca tttcttacct gtgatgtgtt ttacccagca 2580
cctccggaca ctcaccttca ggaccttaat aaaattattc acttggtaag tgttcaagtc 2640
tttctgatca ccccaagtag catgactgat ctgcaattta aaattcctgt gatctgtaaa 2700
aaaaaaaaaa aaaaaaaaaa caaaacccac aagcacttat cttggctact aatgaagctc 2760
tccttttttt tgtttgtttg tttgcttcat tgttgattgt gtattttctt cattcctggg 2820
gagtactaac ccaaaagcgt ctgtctcttg ttttctagtc cagtttgaga ttaatttaga 2880
agaaaggaat actgtatgtg aaattcatct tgggctttcc cctaaattgc aagataaggc 2940
catgtgtaag attttcccta aaactagaat atattaatgc atgtttgaga attttaaagc 3000
accatggtca aaaccagaag ctatattttg catatttgga ctcagccatc cattaagaac 3060
ccatgttgtc ctctggacat atttatcaat ataattgggt tttaaatagt ataaaagaaa 3120
acttgtgatc tatataattt atgtatcacc ttcattgtaa atttagcagg aaatgcatca 3180
caattatgat tttttttttt gcaccagtga aacaataaag atgctattaa caaaaaaaaa 3240
aaaaaa 3246

<210> 81
<211> 3182
<212> DNA
<213> NM_017583.3| Homo sapiens tripartite motif-containing 44 (TRIM44), mRNA

<400> 81

ggaggctgag cgggcggcgc gacgcggggg ccgacggggg cgccgggtgg ccgcgccgga 60

agtgccttgc gcggcagagg aagcgcaggg acagagcgga gcaggccgag ccggcggaaa 120
gggtctttgc tgctgcgccc gggcaggggc tgccgcggcc ccaggtcccg cttcgagacg 180
cggcgcggtc caggcgggag gcgactccct aggaagggac ccggggcggg aggaggaagt 240
gaggccgcgc ggaaggaagg cggcgagccc cggggccccg aggccttggc cgcgtcacag 300
cacccacatg gcctctggag tgggcgcggc cttcgaggaa ctgcctcacg acggcacgtg 360
tgacgagtgc gagcccgacg aggctccggg ggccgaggaa gtgtgccgag aatgcggctt 420
ctgctactgc cgccgccatg ccgaggcgca caggcagaag ttcctcagtc accatctggc 480
cgaatacgtc cacggctccc aggcctggac cccgccagct gacggagagg gggcggggaa 540
ggaagaagcg gaggtcaagg tggagcagga gagggagata gaaagcgagg caggggaaga 600
gagtgagtcg gaggaagaga gcgagtcaga ggaagagagc gagacagagg aagagagtga 660
ggatgagagc gatgaggaga gtgaagaaga cagcgaggaa gaaatggagg atgagcaaga 720
aagcgaggcc gaagaagaca accaagaaga aggggaatcc gaggcggagg gagaaactga 780
ggcagaaagt gaatttgacc cagaaataga aatggaagca gagagagtgg ccaagaggaa 840
gtgtccggac catgggcttg atttgagtac ctattgccag gaagataggc agctcatctg 900
tgtcctgtgt ccagtcattg gggctcacca gggccaccaa ctctccaccc tagacgaagc 960
ctttgaagaa ttaagaagca aagactcagg tggactgaag gccgctatga tcgaattggt 1020
ggaaaggttg aagttcaaga gctcagaccc taaagtaact cgggaccaaa tgaagatgtt 1080
tatacagcag gaatttaaga aagttcagaa agtgattgct gatgaggagc agaaggccct 1140
tcatctagtg gacatccaag aggcaatggc cacagctcat gtgactgaga tactggcaga 1200
catccaatcc cacatggata ggttgatgac tcagatggcc caagccaagg aacaacttga 1260
tacctctaat gaatcagctg agccaaaggc agagggcgat gaggaaggac ccagtggtgc 1320
cagtgaagaa gaggacacat gaaggcttgc taccccagt ggaaaatcat cccctcccct 1380
tgtgtgtatg tgacagcgtg tatgtaacgg cttctgattt ctgtgaaagc tgctcagcaa 1440
caaacgtact tccaccagat gtgtccccag atccacagca ggcacatatc tctccaaggg 1500
atgaccagtt ttatgcttac tgtgtgcttc tcatcccctg gttgtggtag gtcaaggaaa 1560
agagcccctt tgatccacca ggagcaatta agaaaggtcc ttcaggtaat ccctcaatgg 1620
ctgctttgaa cttactcagg aaagccagcc cccataatat tgtattacca aacagtatcg 1680
ctttgttagg aaggatctgg aataatcttg aagggaagtc agagttttct ccctgcctat 1740
taacaaaaac ccaattttgt tcatattgaa gcatgaaata aatgagagca aggtagggcc 1800
aaattaactc ttgtggacag tccctaaaag tccagttcta catttgtgaa aattgtggtg 1860
ccatgaatta agatggatga ctggaaaaag gtgttggaga aagagttaaa gatgaggaag 1920
agatattttt agtatatgaa gttatccagg acttgatatt cataattcag tgctgtggaa 1980
atgaaaaaaa tgattgaaga ggtggaacgg aaatgacctt agggggaaaa aaaaggacca 2040
aagaagtctg attaaaagtt gaaatcagta tttctgaatt caaattgctt gaatttccaa 2100

```
aatagtcagt aaaggatcta atagaaccag aattatttgg gtgaattctg caggttttat   2160
gggcttgtca caacgtgaag ggctggaatg tatattacca aatgggaatt tccattgtag   2220
gtttttgcta gtcccacccc cattttagcc taatttggct taaacgcagt atggggagaa   2280
ttgttcccat tccatgtgtt ctgaattcag ctcatctccc agcatataga tatatcctcc   2340
tttaactccg accagaaccc ttcttcctgt ggcactcccc acccatagac cttcagatca   2400
tctcccacac cctggatctc actctcctct tagtaacaga gacactcctg aggttggact   2460
tccttgcttt tctctacttc caaatcacaa tttcttacaa ccaagctttg tgctcccgag   2520
taagcaggga tgtactaggg gaatgtaaaa ctgcaaactt aaaaacctgc atcttcttga   2580
agcatcagtt ttacttacca aatggtttag agtcataaga tgacctattt ttatataaaa   2640
gttatattat agaataaaat gttcatacgc atagactgtt aagataaaaa aataggaatc   2700
ttgcaaggta attcttattt gcaagtgggt tatgtgttca ctctcctcta cctttatggt   2760
attttggtgt tcacttacga agcatacaac tagaaccata tccaagcaga ctctgggttg   2820
ctgttaaccc agggcctaga cttctagtgc ctctgaggca gaaccaaagg agcctgcact   2880
ggggaaaatc ccttttcctg cctgcctgtc tgcctgtgac ctgtgtacgt attacaggct   2940
ttaggaccag ctgattgtta tgcttgcagg atggttttga aacagaaaca atacttgttt   3000
actgtaggaa tcctatttat attatttttc agtcctgtga atgctgtgaa aagatttatt   3060
cctttgaggc caggaagctc ccaggcatat atgcttctag gttaggattg tcctgactca   3120
ctaaagatgc caggatattg ggctgaggg gagtttgagg tgttaaaaaa aaaaaaaaaa   3180
aa                                                                  3182
```

<210> 82
<211> 4930
<212> DNA
<213> NM_020182.3| Homo sapiens transmembrane, prostate androgen induced RNA (TMEPAI), transcript variant 1, mRNA

<400> 82

```
aaacccgatc tccttggact tgaatgagga ggaggaggcg gcggcggcgg cggcggcgga     60
ggcgctcggc tggggaaagc tagcggcaga ggctcagccc cggcggcagc gcgcgccccg    120
ctgccagccc attttccgga cgccacccgc gggcactgcc gacgcccccg gggctgccga    180
ggggaggccg ggggggcgca gcggagcgcg gtcccgcgca ctgagccccg cggcgccccg    240
ggaacttggc ggcgacccga gcccggcgag ccggggcgcg cctcccccgc cgcgcgcctc    300
ctgcatgcgg ggccccagct ccgggcgccg gccggagccc cccccggccg cccccgagcc    360
ccccgcgccc cgcgccgcgc cgccgcgccg tccatgcacc gcttgatggg ggtcaacagc    420
accgccgccg ccgccgccgg gcagcccaat gtctcctgca cgtgcaactg caaacgctct    480
```

ttgttccaga gcatggagat cacggagctg gagtttgttc agatcatcat catcgtggtg 540
gtgatgatgg tgatggtggt ggtgatcacg tgcctgctga gccactacaa gctgtctgca 600
cggtccttca tcagccggca cagccagggg cggaggagag aagatgccct gtcctcagaa 660
ggatgcctgt ggccctcgga gagcacagtg tcaggcaacg gaatcccaga gccgcaggtc 720
tacgccccgc ctcggcccac cgaccgcctg gccgtgccgc ccttcgccca gcgggagcgc 780
ttccaccgct tccagcccac ctatccgtac ctgcagcacg agatcgacct gccacccacc 840
atctcgctgt cagacgggga ggagccccca ccctaccagg gcccctgcac cctccagctt 900
cgggaccccg agcagcagct ggaactgaac cgggagtcgg tgcgcgcacc cccaaacaga 960
accatcttcg acagtgacct gatggatagt gccaggctgg gcggcccctg cccccccagc 1020
agtaactcgg gcatcagcgc cacgtgctac ggcagcggcg ggcgcatgga ggggccgccg 1080
cccacctaca gcgaggtcat cggccactac ccggggtcct ccttccagca ccagcagagc 1140
agtgggccgc cctccttgct ggaggggacc cggctccacc acacacacat cgcgccccta 1200
gagagcgcag ccatctggag caaagagaag gataaacaga aaggacaccc tctctagggt 1260
ccccaggggg gccgggctgg ggctgcgtag gtgaaaaggc agaacactcc gcgcttctta 1320
gaagaggagt gagaggaagg cggggggcgc agcaacgcat cgtgtggccc tcccctccca 1380
cctccctgtg tataaatatt tacatgtgat gtctggtctg aatgcacaag ctaagagagc 1440
ttgcaaaaaa aaaaagaaaa aagaaaaaaa aaaaccacgt ttctttgttg agctgtgtct 1500
tgaaggcaaa agaaaaaaaa tttctacagt agtctttctt gtttctagtt gagctgcgtg 1560
cgtgaatgct tattttcttt tgtttatgat aatttcactt aactttaaag acatatttgc 1620
acaaaacctt tgtttaaaga tctgcaatat tatatatata aatatatata agataagaga 1680
aactgtatgt gcgagggcag gagtattttt gtattagaag aggcctatta aaaaaaaaag 1740
ttgttttctg aactagaaga ggaaaaaaat ggcaattttt gagtgccaag tcagaaagtg 1800
tgtattacct tgtaaagaaa aaaattacaa agcaggggtt tagagttatt tatataaatg 1860
ttgagatttt gcactatttt ttaatataaa tatgtcagtg cttgcttgat ggaaacttct 1920
cttgtgtctg ttgagacttt aagggagaaa tgtcggaatt tcagagtcgc ctgacggcag 1980
agggtgagcc cccgtggagt ctgcagagag gccttggcca ggagcggcgg gctttcccga 2040
ggggccactg tccctgcaga gtggatgctt ctgcctagtg acaggttatc accacgttat 2100
atattcccta ccgaaggaga caccttttcc cccctgaccc agaacagcct ttaaatcaca 2160
agcaaaatag gaaagttaac cacggaggca ccgagttcca ggtagtggtt ttgcctttcc 2220
caaaaatgaa aataaactgt taccgaagga attagttttt cctcttcttt tttccaactg 2280
tgaaggtccc cgtggggtgg agcatggtgc cctcacaag ccgcagcggc tggtgcccgg 2340
gctaccaggg acatgccaga gggctcgatg acttgtctct gcagggcgct ttggtggttg 2400
ttcagctggc taaaggttca ccggtgaagg caggtgcggt aactgccgca ctggacccta 2460

ggaagcccca ggtattcgca atctgacctc ctcctgtctg tttcccttca cggatcaatt 2520
ctcacttaag aggccaataa acaacccaac atgaaaaggt gacaagcctg ggtttctccc 2580
aggataggtg aaagggttaa aatgagtaaa gcagttgagc aaacaccaac ccgagcttcg 2640
ggcgcagaat tcttcacctt ctcttcccct ttccatctcc tttccccgcg gaaacaacgc 2700
ttcccttctg gtgtgtctgt tgatctgtgt tttcatttac atctctctta gactccgctc 2760
ttgttctcca ggttttcacc agatagattt ggggttggcg ggacctgctg gtgacgtgca 2820
ggtgaaggac aggaaggggc atgtgagcgt aaatagaggt gaccagagga gagcatgagg 2880
ggtggggctt tgggacccac cggggccagt ggctggagct tgacgtcttt cctccccatg 2940
ggggtgggag ggcccccagc tggaagagca gactcccagc tgctaccccc tcccttccca 3000
tgggagtggc tttccatttt gggcagaatg ctgactagta gactaacata aaagatataa 3060
aaggcaataa ctattgtttg tgagcaactt ttttataact tccaaaacaa aaacctgagc 3120
acagttttga agttctagcc actcgagctc atgcatgtga aacgtgtgct ttacgaaggt 3180
ggcagctgac agacgtgggc tctgcatgcc gccagcctag tagaaagttc tcgttcattg 3240
gcaacagcag aacctgcctc tccgtgaagt cgtcagccta aaatttgttt ctctcttgaa 3300
gaggattctt tgaaaaggtc ctgcagagaa atcagtacag gttatcccga aaggtacaag 3360
gacgcacttg taaagatgat taaaacgtat ctttccttta tgtgacgcgt ctctagtgcc 3420
ttactgaaga agcagtgaca ctcccgtcgc tcggtgagga cgttcccgga cagtgcctca 3480
ctcacctggg actggtatcc cctcccaggg tccaccaagg gctcctgctt ttcagacacc 3540
ccatcatcct cgcgcgtcct caccctgtct ctaccaggga ggtgcctagc ttggtgaggt 3600
tactcctgct cctccaacct ttttttgcca aggtttgtac acgactccca tctaggctga 3660
aaacctagaa gtggaccttg tgtgtgtgca tggtgtcagc ccaaagccag gctgagacag 3720
tcctcatatc ctcttgagcc aaactgtttg ggtctcgttg cttcatggta tggtctggat 3780
ttgtgggaat ggctttgcgt gagaaagggg aggagagtgg ttgctgccct cagccggctt 3840
gaggacagag cctgtccctc tcatgacaac tcagtgttga agcccagtgt cctcagcttc 3900
atgtccagtg gatggcagaa gttcatgggg tagtggcctc tcaaaggctg ggcgcatccc 3960
aagacagcca gcaggttgtc tctggaaacg accagagtta agctctcggc ttctctgctg 4020
agggtgcacc ctttcctcta gatggtagtt gtcacgttat ctttgaaaac tcttggactg 4080
ctcctgagga ggccctcttt tccagtagga agttagatgg gggttctcag aagtggctga 4140
ttggaagggg acaagcttcg tttcaggggt ctgccgttcc atcctggttc agagaaggcc 4200
gagcgtggct ttctctagcc ttgtcactgt ctccctgcct gtcaatcacc acctttcctc 4260
cagaggagga aaattatctc ccctgcaaag cccggttcta cacagatttc acaaattgtg 4320
ctaagaaccg tccgtgttct cagaaagccc agtgtttttg caaagaatga aaagggaccc 4380
catatgtagc aaaaatcagg gctgggggag agccgggttc attccctgtc ctcattggtc 4440
gtccctatga attgtacgtt tcagagaaat tttttttcct atgtgcaaca cgaagcttcc 4500

agaaccataa aatatcccgt cgataaggaa agaaaatgtc gttgttgttg tttttctgga 4560
aactgcttga aatcttgctg tactatagag ctcagaagga cacagcccgt cctcccctgc 4620
ctgcctgatt ccatggctgt tgtgctgatt ccaatgcttt cacgttggtt cctggcgtgg 4680
gaactgctct cctttgcagc cccatttccc aagctctgtt caagttaaac ttatgtaagc 4740
tttccgtggc atgcggggcg cgcacccacg tccccgctgc gtaagactct gtatttggat 4800
gccaatccac aggcctgaag aaactgcttg ttgtgtatca gtaatcatta gtggcaatga 4860
tgacattctg aaaagctgca atacttatac aataaatttt acaattcttt ggaaaaaaaa 4920
aaaaaaaaaa 4930

<210> 83
<211> 702
<212> DNA
<213> NM_014183.2| Homo sapiens dynein, cytoplasmic, light polypeptide 2A (DNCL2A), transcript variant 1, mRNA

<400> 83

cgcagaaagg cacaggactc gctaagtgtt cgctacgcgg ggctaccgga tcggtcggaa 60
atggcagagg tggaggagac actgaagcga ctgcagagcc agaagggagt gcagggaatc 120
atcgtcgtga acacagaagg cattcccatc aagagcacca tggacaaccc caccaccacc 180
cagtatgcca gcctcatgca cagcttcatc ctgaaggcac ggagcaccgt gcgtgacatc 240
gacccccaga acgatctcac cttccttcga attcgctcca agaaaaatga aattatggtt 300
gcaccagata aagactattt cctgattgtg attcagaatc caaccgaata agccactctc 360
ttggctccct gtgtcattcc ttaatttaat gcccccaag aatgttaatg tcaatcatgt 420
cagtggacta gcacatggca gtcgcttgga acccactcac accaatccag tgaccgtgtg 480
tgggctggcg gctcttctcc cccaccaacg gaacccctgt gtgcaccaac cttccccaga 540
gctccggagc gccctctcct cacttccagg ttttggagca agagcttgca ggaagcccgc 600
acccagcttc cttctgacct tcagttcact ttgtcgccct tggagaaagc tgtttttctt 660
taactaaaaa taaccaaaat gcttaaaaaa aaaaaaaaaa aa 702

<210> 84
<211> 2100
<212> DNA
<213> NM_015907.2| Homo sapiens leucine aminopeptidase 3 (LAP3), mRNA

<400> 84

ctgcccatcc gtcccgcccc ctagacgcac gtccgctcgc ccggcgcccg agccagtccg 60

```
cgcgcacgcc gtctgcgccc cgaaagcccc gccccaaggc gcgcccgccc accgctctcc     120
acgtgctcgc tggagggcgg tgcgaggggc cgagccgaca agatgttctt gctgcctctt     180
ccggctgcgg ggcgagtagt cgtccgacgt ctggccgtga gacgtttcgg gagccggagt     240
ctctccaccg cagacatgac gaagggcctt gttttaggaa tctattccaa agaaaaagaa     300
gatgatgtgc cacagttcac aagtgcagga gagaattttg ataaattgtt agctggaaag     360
ctgagagaga ctttgaacat atctggacca cctctgaagg cagggaagac tcgaaccttt     420
tatggtctgc atcaggactt ccccagcgtg gtgctagttg gcctcggcaa aaaggcagct     480
ggaatcgacg aacaggaaaa ctggcatgaa ggcaaagaaa acatcagagc tgctgttgca     540
gcggggtgca ggcagattca agacctggag ctctcgtctg tggaggtgga tccctgtgga     600
gacgctcagg ctgctgcgga gggagcggtg cttggtctct atgaatacga tgacctaaag     660
caaaaaaaga agatggctgt gtcggcaaag ctctatggaa gtggggatca ggaggcctgg     720
cagaaaggag tcctgtttgc ttctgggcag aacttggcac gccaattgat ggagacgcca     780
gccaatgaga tgacgccaac cagatttgct gaaattattg agaagaatct caaaagtgct     840
agtagtaaaa ccgaggtcca tatcagaccc aagtcttgga ttgaggaaca ggcaatggga     900
tcattcctca gtgtggccaa aggatctgac gagcccccag tcttcttgga aattcactac     960
aaaggcagcc ccaatgcaaa cgaaccaccc ctggtgtttg ttgggaaagg aattaccttt    1020
gacagtggtg gtatctccat caaggcttct gcaaatatgg acctcatgag ggctgacatg    1080
ggaggagctg caactatatg ctcagccatc gtgtctgctg caaagcttaa tttgcccatt    1140
aatattatag gtctggcccc tctttgtgaa aatatgccca gcggcaaggc caacaagccg    1200
ggggatgttg ttagagccaa aaacgggaag accatccagg ttgataacac tgatgctgag    1260
gggaggctca tactggctga tgcgctctgt tacgcacaca cgtttaaccc gaaggtcatc    1320
ctcaatgccg ccaccttaac aggtgccatg gatgtagctt tgggatcagg tgccactggg    1380
gtctttacca attcatcctg gctctggaac aaactcttcg aggccagcat tgaaacaggg    1440
gaccgtgtct ggaggatgcc tctcttcgaa cattatacaa gacaggttgt agattgccag    1500
cttgctgatg ttaacaacat tggaaaatac agatctgcag gagcatgtac agctgcagca    1560
ttcctgaaag aattcgtaac tcatcctaag tgggcacatt tagacatagc aggcgtgatg    1620
accaacaaag atgaagttcc ctatctacgg aaaggcatga ctgggaggcc cacaaggact    1680
ctcattgagt tcttacttcg tttcagtcaa gacaatgctt agttcagata ctcaaaaatg    1740
tcttcactct gtcttaaatt ggacagttga acttaaaagg tttttgaata aatggatgaa    1800
aatcttttaa cggagacaaa ggatggtatt taaaaatgta gaacacaatg aaatttgtat    1860
gccttgattt tttttcatt tcacacaaag atttataaag gtaaagttaa tatcttactt    1920
gataaggatt tttaagatac tctataaatg attaaaattt ttagaacttc ctaatcactt    1980
ttcagagtat atgtttttca ttgagaagca aaattgtaac tcagatttgt gatgctagga    2040
```

acatgagcaa actgaaaatt actatgcact tgtcagaaac aataaatgca acttgttgtg 2100

<210> 85
<211> 1510
<212> DNA
<213> NM_018478.1| Homo sapiens chromosome 20 open reading frame 35 (C20orf35), mRNA

<400> 85

cgagtgtggc caagggtgcc ggaggcaggg ttcgggtgcg tagtcgttgc gtgggcgctg 60
cccaaaaggc gcagagcatc aagtgtgcgt gggcagaacc ggcgcgggcg cccgccgcgg 120
gtctgcgcgg ggcgggggcg cagcaagtgc atccgagcga gcggagacta gcgcaccggc 180
gtcggtggcg agggtggtgc agaggagtcc ggctgggcgg agggaggaag gatgggtgcg 240
ggtaactttt tgaccgcctt ggaagtacca gtagccgcgc tcgcaggggc tgcctccgac 300
cgccgggcga gctgcgagcg agtgagcccg ccaccgcccc tcccccactt ccgcctcggc 360
acgaggcctc ttcctcgttc ccggctccca gggcccgtgt ccaggccgga gccaggggcc 420
ccactgttgg gatgctggct gcagtggggc gccccaagcc caggtcccct ctgtcttctc 480
tttcgacttt gcagctgtac ttgttttgct cctctacccg caggagctga catggaccca 540
aatcctcggg ccgccctgga gcgccagcag ctccgccttc gggagcggca aaaattcttc 600
gaggacattt tacagccaga gacagagttt gtctttcctc tgtcccatct gcatctcgag 660
tcgcagagac cccccatagg tagtatctca tccatggaag tgaatgtgga cacactggag 720
caagtagaac ttattgacct tggggacccg gatgcagcag atgtgttctt gccttgcgaa 780
gatcctccac caacccccca gtcgtctggg gtggacaacc atttggagga gctgagcctg 840
ccggtggcta catcagacag gaccacatct aggacctcct cctcctcctc ctccgactcc 900
tccaccaacc tgcatagccc aaatccaagt gatgatggag cagatacgcc cttggcacag 960
tcggatgaag aggaggaaag gggtgatgga ggggcagagc ctggagcctg cagctagcag 1020
tgggcccctg cctacagact gaccacgctg gctattctcc acatgagacc acaggcccag 1080
ccagagcctg tcgggagaag accagactct ttacttgcag taggcaccag aggtgggaag 1140
gatggtggga ttgtgtacct ttctaagaat taaccctctc ctgctttact gctaattttt 1200
tcctgctgca accctcccac cagtttttgg cttactcctg agatatgatt tgcaaatgag 1260
gagagagaag atgaggttgg acaagatgcc actgcttttc ttagcactct tccctcccct 1320
aaaccatccc gtagtcttct aatacagtct ctcagacaag tgtctctaga tggatgtgaa 1380
ctccttaact catcaagtaa ggtggtactc aagccatgct gcctccttac atccttttg 1440
gaacagagca cggtataaat aataaactaa taataatatg ccaacaaaaa aaaaaaaaaa 1500
aaaaaaaaaa 1510

<210> 86
<211> 3105

<212> DNA
<213> NM_030674.2| Homo sapiens solute carrier family 38, member 1 (SLC38A1), mRNA

<400> 86

```
gcacgaggga ctggggcggc cacgcactcc gccagaaggt cgccaggagc ctccgccctt     60
caccttcctc ggaaatccgc caggccacgc aagctccctg cccaaccctt actgacgggg    120
gccacatttt cccggcctcc gcagccagac cttgacacaa aggacatcaa actgccgagg    180
gtaaaaaccc cggaagggcg gacacctcca catcgccttt tgccaccttt ccctttattt    240
ccggagatat ttattgagtg tctactgtgt gccaggcact atatctatgt gcatagaaaa    300
accctggaag gccatacaac aatatatata gagtgatcgt ctctgcttgc tgagctaaca    360
ggggtgtcaa gcttccattt tggtatctac ttctaaatac actcagaaca ggagaaattt    420
ggactaattt tcaaactaca gacactttct aatcatgatg catttcaaaa gtggactcga    480
attaactgag ttgcaaaaca tgacagtgcc cgaggatgat aacattagca atgactccaa    540
tgatttcacc gaagtagaaa atggtcagat aaatagcaag tttatttctg atcgtgaaag    600
tagaagaagt ctcacaaaca gccatttgga aaaaaagaag tgtgatgagt atattccagg    660
tacaacctcc ttaggcatgt ctgtttttaa cctaagcaac gccattatgg gcagtgggat    720
tttgggactc gcctttgccc tggcaaacac tggaatccta ctttttctgg tacttttgac    780
ttcagtgaca ttgctgtcta tatattcaat aaacctccta ttgatctgtt caaaagaaac    840
aggctgcatg gtgtatgaaa agctggggga acaagtcttt ggcaccacag ggaagttcgt    900
aatctttgga gccacctctc tacagaacac tggagcaatg ctgagctacc tcttcatcgt    960
aaaaaatgaa ctaccctctg ccataaagtt tctaatggga aaggaagaga cattttcagc   1020
ctggtacgtg gatggccgcg ttctggtggt gatagttacc tttggcataa ttctccctct   1080
gtgtctcttg aagaacttag ggtatcttgg ctatactagt ggattttcct tgagctgtat   1140
ggtttttttc ctaattgtgg ttatttacaa gaaatttcaa attccctgca ttgttccaga   1200
gctaaattca acaataagtg ctaattcaac aaatgctgac acgtgtacgc caaaatatgt   1260
taccttcaat tcaaagaccg tgtatgcttt acccaccatt gcatttgcat ttgtttgcca   1320
cccgtcagtc ctgccaattt acagtgagct taaagaccga tcacagaaaa aaatgcagat   1380
ggtttcaaac atctcctttt tcgccatgtt tgttatgtac ttcttgactg ccatttttgg   1440
ctacttgaca ttctatgaca acgtgcagtc cgacctcctt cacaaatatc agagtaaaga   1500
tgacattctc atcctgacag tgcggctggc tgtcattgtt gctgtgatcc tcacagtgcc   1560
ggtgttattt ttcacggatc gttcatcttt atttgaactg gctaagaaaa caaagtttaa   1620
```

tttatgtcgt cataccgtgg ttacctgcat actcttggtt gttatcaact tgttggtgat 1680
cttcataccc tccatgaagg atatttttgg agtcgtagga gttacatctg ctaacatgct 1740
tattttcatt cttccttcat ctctttattt aaaaatcaca accaggatgg agataaagga 1800
actcaaagaa tttgggctgc ccttttcttg ggcctggggg tgttgttctc cttggtcagc 1860
attcccttgg tcatctatga ctgggcctgc tcatcgagta atggtgaagg ccactgaaac 1920
ccgccgagaa aaagaaacat ccctgttgtc tgctcagtca agtccccaca catcagcaat 1980
ctctcaccac ttcttttgca agtttacaga agcaaacaga aatgtacagg atacttaaaa 2040
tggaataact ttttggttgc aaaacagaga catggttcta taatgcttca tgtccctcca 2100
agatttgaga tcaatttagg gattgtgaaa tttttttttc aaatttcata caatcatatt 2160
tcccagtact tttcacaatc atttttttacc catctaactc tatgttttgt ggcttcccgg 2220
tctcttagaa ctttgaaaac atgatataca ataatgttta tttattatac atccagattc 2280
tgaaataatt ttcctactga tgttcagctc acactatctg taccttttta gaagagaaaa 2340
gaatcttgaa ttgtatatat ttattttgct ttacagaaaa aaatggtttc gtaaataatt 2400
tgcctatttt gggtaacata gcacatggag ataatcatct gaaagttata gggcactgcc 2460
actgctgaat cagagcatgc ccaatatttg aggtggctct gatttcctgg cagctgaact 2520
cgggtagtcc agtggcctag ctggtaccac atctattccc atccagagac attctctggc 2580
aagtgttctc agctgaaaag tggttgggga tgattcttac cttggtaatt aaatgaagct 2640
acacatttgg gtaatctagc aaatgaagta ttttttccct cttggcaact tgtgtcagag 2700
ttactctggt ctgagtcaac tttcgctggg gaaaacctat ggaacctact gcaaaaagat 2760
tgtccaaaat gcctaagaaa atactcctct gatgcattta gccttcaacc ctacctgtct 2820
tgctgaaggg agaaaaatgt tttagtacat tataggccca gcagctttta ttcatgtcca 2880
ccagctagtt gcacagagaa tcatgtgtac ctaactaagg atgatctagg ataagtaact 2940
cctgttttat attgagtatt ttagggaagt ctttaaaaga cttgttttat atctataaat 3000
ctaggttatt acaaatacaa gaattttgta ccttaaataa gcctcatttc tatttcttct 3060
tcattaattc tccatctagt cttgtgaaaa aaaaaaaaaa aaaaa 3105

<210> 87
<211> 2711
<212> DNA
<213> NM_016028.4| Homo sapiens suppressor of variegation 4-20 homolog 1 (Drosophila) (SUV420H1), transcript variant 2, mRNA

<400> 87

ggtgctgcgg cccgcgccgc catcttggat tttactctcc atttttctct ggaattattt 60
ttggtgatta attttctggg ggggactggg acgcggggcc cggcggcgcg gccccgcatc 120

```
gcagcggccg ggcagcgggg cctgggacgc gccccgagga ggagcggggc ggcgcaggcg    180
gagagaacat tgaaagtatt ctctaagcta tttgaagaga gtgactaaat gcacctgggt    240
caggctgtct gtgggtatga agtggttggg agaatccaag aacatggtgg tgaatggcag    300
gagaaatgga ggcaagttgt ctaatgacca tcagcagaat caatcaaaat tacagcacac    360
ggggaaggac accctgaagg ctggcaaaaa tgcagtcgag aggaggtcga acagatgtaa    420
tggtaactcg ggatttgaag gacagagtcg ctatgtacca tcctctggaa tgtccgccaa    480
ggaactctgt gaaaatgatg acctagcaac cagtttggtt cttgatccct atttaggttt    540
tcaaacacac aaaatgaata ctagcgcctt tccttcgagg agctcaaggc atttttcaaa    600
atctgacagt ttttctcaca acaaccctgt gagatttagg cctattaaag gaaggcagga    660
agaactaaag gaagtaattg aacgttttaa gaaagatgaa cacttggaga aagccttcaa    720
atgtttgact tcaggcgaat gggcacggca ctattttctc aacaagaata aaatgcagga    780
gaaattattc aaagaacatg tatttattta tttgcgaatg tttgcaactg acagtggatt    840
tgaaatattg ccatgtaata gatactcatc agaacaaaat ggagccaaaa tagttgcaac    900
aaaagagtgg aaacgaaatg acaaaataga attactggtg ggttgtattg ccgaactttc    960
agaaattgag gagaacatgc tacttagaca tggagaaaac gacttcagtg tcatgtactc   1020
cacaaggaaa aactgtgctc aactctggct gggtcctgct gcgtttataa accatgattg   1080
cagacctaat tgtaagtttg tgtcaactgg tcgagataca gcatgtgtga aggctctaag   1140
agacattgaa cctggagaag aaatttcttg ttattatgga gatgggttct ttggagaaaa   1200
taatgagttc tgcgagtgtt acacttgcga aagacggggc actggtgctt ttaaatccag   1260
agtgggactg cctgcgcctg ctcctgttat caatagcaaa tatggactca gagaaacaga   1320
taaacgttta aataggctta aaaagttagg tgacagcagc aaaaattcag acagtcaatc   1380
tgtcagctct aacactgatg cagataccac tcaggaaaaa aacaatgcaa gtaagtaagg   1440
gagatttgat aagcatatct tttaaaagta ttttcacaca atttgcttta taaagtgtgc   1500
ttcagtagtt ttaaactttt aaatactcag agagactggg acttgtgagc tttggctgca   1560
cttcaaggct ctagacgtga tttgagtaga ggcacagtct gtatcccatc tctaacttca   1620
gtaccgtcct ctagactatt tttcttgaat accttggtaa ctggatatga gttcttcatc   1680
atatgttcca aggtcatcat atgttttaaa cattttcaag gtgttagaga ctgtgatgat   1740
gtcgctaagt cctgcaagaa gacaaaagga ctgagtagaa ttaaattaga ctctatacat   1800
tccagtgcct agccagtttg ttagaaaaga tgatggactt ggggaattca tagcttctgg   1860
ccttaaggct tccacctttt cattgcttgc tgaccttttt caaaacgaac tgactcagtt   1920
cagcagacca ccagtaccag actcagaatt gtgatagagg agcattttga acagtgccgt   1980
attgtgacat gctgtattgg ctactccaga aagtaggagt aaagatggaa aggagaaaga   2040
agcaacctct gagattccag tggtgtgtgg gggcaagatc tgatggaaac tgaaaaagag   2100
aacgaagact aaacaaagag aaaggaaaga gaagaaaccc taaatgggca aaggaaagca   2160
```

catcctgttt gcggagcttt gaaatattgg aaccatttct aattgctcct gtttttctgg 2220
gtaacaccag ttttctgtag ttgccactaa agcagtagac tcttgagtct cacttgtctc 2280
tgagagagac agaagttaga aagttttgac ttggcgattc cgaaagtatg cctttgttgg 2340
cacttaaatg tccagtgaga cttcttggca ccttagagcc ctctgagata ctgattattt 2400
taggttcttc tccctacttt cagatgtttt cagcccaaca ctgggtgctc tcttccacta 2460
cagagaatcc tgaagaaaag ggaaggtgtt tcccatgatg gtgaatgtca ctgccatgaa 2520
ttcctgaatc tacctgctgc tgggagtcag agtccaagca taacccgtgt agcataaaag 2580
cagcgctgta gccctattcc agtctttttc gttaatgtcc agagtgaaca acaagagtta 2640
gtcaatcatt aactgttgac tgttgattct cataataaat gcagcataac gacaaaaaaa 2700
aaaaaaaaaa a 2711

<210> 88
<211> 2977
<212> DNA
<213> NM_022105.2| Homo sapiens death associated transcription factor 1 (DATF1), transcript variant 1, mRNA

<400> 88

gggagcggga gggcaggcgc accggaggcc gcccctcagc acctctcgcg acagcaagag 60
agcgcgagag cgcgagccga tgaccaatga agcgcccccg cgagggggcg gggcggacgg 120
cctcccggaa gcgcggaacc tcagcttccg tacttgcgca gaactcccct cgcggcgacc 180
acgcactacg ggttggcgcc agagtcaaaa ggcgtcggcc ctctggcaag atggctgctg 240
cggaggcgtt ggagcgcgga aatctggaac cgggatggcg acgtctacac tgagtcggag 300
gcgaaggagc ttactccacg ggaacagcct ctagataatc tgagttgttg aaaatacgaa 360
gcctgttact cgtgaacagt ggctgacaac agtgttgttg tgagcctggc tgtctgcttg 420
gacccagagg tttcgtctgc cagggttttt ggttgtattt aggatttcag ggaaaagtgt 480
ccaagctttc agtgttggag caggtatgga cgacaaaggc gacccgagca atgaggaggc 540
acctaaggcc atcaaaccca ccagcaaaga gttcaggaaa acatggggtt ttcgaaggac 600
cactatcgcc aagcgagagg gcgcagggga cgcggaggct gacccactgg agccgccacc 660
cccacagcag cagctgggcc tgtccctgcg gcgcagtggg aggcagccca agcgcactga 720
gcgcgtggag cagttcctga ccattgcgcg gcgccgcggc aggaggagca tgcctgtctc 780
cctggaggat tctggtgagc ccacgtcctg ccccgccaca gacgccgaga cagcctccga 840
gggcagcgtg gaaagcgctt ctgagaccag aagcggcccc cagtctgctt ccacagctgt 900
gaaggaacga ccagcctctt ctgaaaaggt gaaaggaggg gatgaccacg atgacacctc 960
cgatagtgac agcgatggcc tgaccttgaa agagcttcag aatcgccttc gcaggaagcg 1020

ggaacaggag cccactgaga ggcccctgaa agggatccag agtcgcctgc ggaagaagcg 1080
ccgggaggag ggtcccgccg agactgtggg ctccgaggcc agtgacactg tggagggcgt 1140
cctgcccagt aagcaggagc ccgagaacga tcaggggggtt gtgtcccagg ctgggaaaga 1200
tgacagagag agtaagttgg agggaaaggc ggctcaggac atcaaagatg aggagcctgg 1260
agacttgggc cgaccgaagc ctgaatgtga gggttacgac cccaacgccc tgtattgcat 1320
ttgccgccag cctcacaaca acaggtttat gatttgctgt gaccgctgtg aagaatggtt 1380
tcatggcgat tgtgtgggca tttctgaggc tcgagggagg cttttggaaa ggaatgggga 1440
agactatatc tgcccaaact gcaccattct gcaagtgcag gatgagactc attcagaaac 1500
ggcagatcag caggaagcta aatggagacc tggagatgct gatggcaccg attgtacaag 1560
tataggaaca atagagcaga agtctagcga agaccaaggg ataaagggta gaattgagaa 1620
agctgcaaat ccaagtggca agaagaaact caagatcttc cagcctgtga tagaggcgcc 1680
tggtgcctca aaatgtattg gccccgggtg ctgtcacgtg gcgcagcccg actcggtgta 1740
ctgcagtaat gactgtatcc tcaaacacgc cgcagcgaca atgaagtttc taagctcagg 1800
taaagaacag aagccaaagc ctaaagaaaa gatgaagatg aagccagaga agcccagtct 1860
tccgaaatgc ggtgctcagg caggtattaa aatctcttct gtgcacaaga gaccagctcc 1920
agaaaaaaaa gagaccacag tgaagaaggc agtggtggtc cctgcgcgga gtgaagcact 1980
cgggaaggaa gcagcttgtg agagcagcac gccgtcgtgg gcgagcgatc acaattacaa 2040
tgcagtaaag ccagaaaaga ctgctgctcc ctcgccgtca ctgttgtata aatgtatgta 2100
tcacctaggg gttggcctcc tggacccctc ccgttctttc tggatagcca tcccctgggc 2160
ctgtccagga ctgggagttg cagctttgtg ttaagctgat cacagacacc ggctgcacca 2220
tcagcgggaa gcagagccca tgtccaggat gcctcctgct gccctgtgtc catccctagt 2280
ctgtcaggac ttcctgtcac tgttttccaa agctgtaaac ctcactggtg aacgttcacc 2340
ttaatgattg attctttaat ctctgttttc actctcaggc tctggtaagt attcgtattc 2400
tcttcatccc agtctgattg catagccaca ctgcccggca cgccacatcc acccctgtct 2460
gcacatgagt tgttctgaca acagcgctgt atacgcttca gtttttccac attgtccacg 2520
gccagcacat gaaagcatca cttctttttt atgttgtggg aatctttgca agttagtgtt 2580
gcatctgatt ttcaggtgta catttatttt tgactgggca gataggggat tttttttttt 2640
ccatgtccga ttcacacgct acacacccac atgaacacat tcgaacttcg aaggccacac 2700
actcctgctt cataggcccc acggtaagtg agttcacacc tagaacactg tcctgaccgc 2760
aggacgcgtg ccttggactt ggtattctac atgtgactgg ctttcttgcc ctcgtctctt 2820
gaatgtttag actcttaaga tcatatcctg ccccaaattt caaattaatg aaatgaagat 2880
atttcaaaca gatctttgaa acctcagatt ctgtggtgca attttaatgt tttcttgttt 2940
ctcagttttc tgctataaaa ctattttcaa ttcagtc 2977

<210> 89
<211> 1047

<212> DNA
<213> NM_018487.2| Homo sapiens hepatocellular carcinoma-associated antigen 112 (HCA112), mRNA

<400> 89

cccacttctc cagccagcgc cccagccctc ccgccgcccg ctcgcaggtc ccgaggagcg 60
cagactgtgt ccctgacaat gggaacagcc gacagtgatg agatggcccc ggaggcccca 120
cagcacaccc acatcgatgt gcacatccac caggagtctg ccctggccaa gctcctgctc 180
acctgctgct ctgcgctgcg gccccgggcc acccaggcca ggggcagcag ccggctgctg 240
gtggcctcgt gggtgatgca gatcgtgctg gggatcttga gtgcagtcct aggaggattt 300
ttctacatcc gcgactacac cctcctcgtc acctcgggag ctgccatctg gacaggggct 360
gtggctgtgc tggctggagc tgctgccttc atttacgaga aacggggtgg tacatactgg 420
gccctgctga ggactctgct aacgctggca gctttctcca cagccatcgc tgccctcaaa 480
ctttggaatg aagatttccg atatggctac tcttattaca acagtgcctg ccgcatctcc 540
agctcgagtg actggaacac tccagccccc actcagagtc cagaagaagt cagaaggcta 600
cacctatgta cctccttcat ggacatgctg aaggccttgt tcagaaccct tcaggccatg 660
ctcttgggtg tctggattct gctgcttctg gcatctctga cccctctgtg gctgtactgc 720
tggagaatgt tcccaaccaa agggaaaaga gaccagaagg aaatgttgga agtgagtgga 780
atctagccat gcctctcctg attattagtg cctggtgctt ctgcaccggg cgtccctgca 840
tctgactgct ggaagaagaa ccagactgag gaaaagaggc tcttcaacag ccccagttat 900
cctggcccca tgaccgtggc cacagccctg ctccagcagc acttgcccat tccttacacc 960
ccttccccat cctgctccgc ttcatgtccc ctcctgagta gtcatgtgat aataaactct 1020
catgttattg ttcccaggaa aaaaaaa 1047

<210> 90
<211> 2785
<212> DNA
<213> NM_014454.1| Homo sapiens sestrin 1 (SESN1), mRNA

<400> 90

gatccgccac catggctgaa ggagagaatg aagtgagatg ggatggactc tgcagcagag 60
attcaactac tagggagaca gcattggaaa acattaggca aaccattttg aggaaaaccg 120
agtatcttcg ttcggtgaaa gaaacacctc atcgtccatc agacgggctt tcaaataccg 180

agtcttcgga tgggttgaat aagctacttg ctcatctgct tatgctttct aagaggtgtc 240
ccttcaaaga tgtgagagag aaaagtgagt ttattctgaa gagcatccag gaacttggca 300
ttagaattcc tcgaccacta ggacagggac caagcagatt catcccagaa aaggagatcc 360
tccaagtggg gagtgaagac gcacagatgc atgctttatt tgcagattct tttgctgctt 420
tgggccgttt ggataacatt acgttagtga tggttttcca cccacaatat ttagaaagtt 480
tcttaaaaac tcagcactat ctactgcaaa tggatgggcc gttacccccta cattatcgtc 540
actacattgg aataatggct gcggcaagac atcagtgctc ctacttagtg aacctgcatg 600
taaatgattt ccttcatgtt ggtggggacc ccaagtggct caatggttta gagaatgctc 660
ctcaaaaact acagaattta ggagaactta acaaagtgtt agcccataga ccttggctta 720
ttaccaaaga acacattgag ggacttttaa aagctgaaga gcacagctgg tcccttgcgg 780
aattggtaca tgcagtagtt ttactcacac actatcattc tcttgcctca ttcacattcg 840
gctgtggaat cagtccagaa attcattgtg atggtggcca cacattcaga cctccttctg 900
ttagcaacta ctgcatctgt gacattacaa atggcaatca cagtgtggat gagatgccgg 960
tcaactcagc agaaaatgtt tctgtaagtg attctttctt tgaggttgaa gccctcatgg 1020
aaaagatgag gcagttacag gaatgtcgag atgaagaaga ggcaagtcag gaagagatgg 1080
cttcacgttt tgaaatagaa aaaagagaga gtatgtttgt cttctcttca gatgatgaag 1140
aagttacacc agcaagagct gtatctcgtc attttgagga tactagttat ggctataaag 1200
atttctctag acatgggatg catgttccaa catttcgtgt ccaggactat tgctgggaag 1260
atcatggtta ttctttggta aatcgccttt atccagatgt gggacagttg attgatgaaa 1320
aatttcacat tgcttacaat cttacttata atacaatggc aatgcacaaa gatgttgata 1380
cctcaatgct tagacgggca atttggaact atattcactg catgtttgga ataagatatg 1440
atgattatga ctatggtgaa attaaccagc tattggatcg tagctttaaa gtttatatca 1500
aaactgttgt ttgcactcct gaaaaggtta ccaaaagaat gtatgatagc ttctggaggc 1560
agttcaagca ctctgagaag gttcatgtta atctgcttct tatagaagct aggatgcaag 1620
cagaactcct ttatgctctg agagccatta cccgctatat gacctgatgc ctttccttca 1680
ttaaagatga ttctggaatg atcagcagat atagtctaca agggggaagg tactaagccc 1740
caggaccaat ggtagacaaa ataattcaga aatccattgt gccatgattc ctttagtttc 1800
tgctattttt ctgtggaaaa ccactgctgg cacaagcagt gactgtttgg cagcttcaag 1860
tttagagctg tgaagacagg ctgccattca cagtattttg ctttttgaca gtacaagatg 1920
ctgtgtaact gttttaatac agcaaatagt aactctccaa atcctgttgc ttttatgtta 1980
aataagataa caagaattgg agcatgcaaa gaatgggact tggataatga cttaagcttt 2040
atatgtaaag aattttagaa gatcttggtg ctgctattcc tgctggagga atgaatagat 2100
ggctgtttca gttaagctat tagtaataaa agtgaacatt gctactatct gagcctacat 2160
acataacttg tgtgatttca aattaaactt gcattatgtg ttaattttct tgcatctaaa 2220

aaagcataga attcctactc acacagctca gcaacaacca ttttgatggt aacagttaat 2280
ttctttcatt agtttttaa attcagggtt ctggatatta aattaaaatg gcattcttaa 2340
agattttctt caaaaagcaa tcctaaatga aagtgtgtaa attataagaa gctggcgatc 2400
ttttgatatg ctgtttcaca ggatcctgac actggagggc agctgtcttg tgcattactt 2460
gtgttcccag caccaaagtt gtgggacatg ttgctgtaga ctgctgcgca gtcctgggtg 2520
cattcagtct ctctgcctct gcctgcctcc tggtccccac tttaaaggct gtgcagctcc 2580
ttaaataata aagctggaaa atatttttag tcgggttatc aaatttgatt tacaaaaacg 2640
ctaactttgt ttgaaatgca aacaggtttg aaaatatgta ttaagtactt tgtattctgg 2700
aagcgtgaat tgcttttgaa gtctgtcagt attactggta tttttaaata aagaagaatt 2760
tttctccaaa aaaaaaaaaa aaaaa 2785

<210> 91
<211> 3802
<212> DNA
<213> NM_017763.1| Homo sapiens hypothetical protein FLJ20315 (FLJ20315), mRNA

<400> 91

aaaaaaaaaa aactttagag aaaggaaggg ccaaaactac gacttggctt tctgaaacgg 60
aagcataaat gttcttttcc tccatttgtc tggatctgag aacctgcatt tggtattagc 120
tagtggaagc agtatgtatg gttgaagtgc attgctgcag ctggtagcat gagtggtggc 180
caccagctgc agctggctgc cctctggccc tggctgctga tggctaccct gcaggcaggc 240
tttggacgca caggactggt actggcagca gcggtggagt ctgaaagatc agcagaacag 300
aaagctgtta tcagagtgat ccccttgaaa atggacccca caggaaaact gaatctcact 360
ttggaaggtg tgtttgctgg tgttgctgaa ataactccag cagaaggaaa attaatgcag 420
tcccacccac tgtacctgtg caatgccagt gatgacgaca atctggagcc tggattcatc 480
agcatcgtca agctggagag tcctcgacgg gcccccgcc cctgcctgtc actggctagc 540
aaggctcgga tggcgggtga gcgaggagcc agtgctgtcc tctttgacat cactgaggat 600
cgagctgctg ctgagcagct gcagcagccg ctggggctga cctggccagt ggtgttgatc 660
tggggtaatg acgctgagaa gctgatggag tttgtgtaca agaaccaaaa ggcccatgtg 720
aggattgagc tgaaggagcc cccggcctgg ccagattatg atgtgtggat cctaatgaca 780
gtggtgggca ccatctttgt gatcatcctg gcttcggtgc tgcgcatccg gtgccgcccc 840
cgccacagca ggccggatcc gcttcagcag agaacagcct gggccatcag ccagctggcc 900
accaggaggt accaggccag ctgcaggcag gcccggggtg agtggccaga ctcagggagc 960
agctgcagct cagcccctgt gtgtgccatc tgtctggagg agttctctga ggggcaggag 1020

ctacgggtca tttcctgcct ccatgagttc catcgtaact gtgtggaccc ctggttacat 1080
cagcatcgga cttgccccct ctgcgtgttc aacatcacag agggagattc attttcccag 1140
tccctgggac cctctcgatc ttaccaagaa ccaggtcgaa gactccacct cattcgccag 1200
catcccggcc atgcccacta ccacctccct gctgcctacc tgttgggccc ttcccggagt 1260
gcagtggctc ggcccccacg acctggtccc ttcctgccat cccaggagcc aggcatgggc 1320
cctcggcatc accgcttccc cagagctgca catccccggg ctccaggaga gcagcagcgc 1380
ctggcaggag cccagcaccc ctatgcacaa ggctggggaa tgagccacct ccaatccacc 1440
tcacagcacc ctgctgcttg cccagtgccc ctacgccggg ccaggccccc tgacagcagt 1500
ggatctggag aaagctattg cacagaacgc agtgggtacc tggcagatgg gccagccagt 1560
gactccagct cagggccctg tcatggctct tccagtgact ctgtggtcaa ctgcacggac 1620
atcagcctac agggggtcca tggcagcagt tctactttct gcagctccct aagcagtgac 1680
tttgaccccc tagtgtactg cagccctaaa ggggatcccc agcgagtgga catgcagcct 1740
agtgtgacct ctcggcctcg ttccttggac tcggtggtgc ccacagggga aacccaggtt 1800
tccagccatg tccactacca ccgccaccgg caccaccact acaaaaagcg gttccagtgg 1860
catggcagga agcctggccc agaaaccgga gtcccccagt ccaggcctcc tattcctcgg 1920
acacagcccc agccagagcc accttctcct gatcagcaag tcaccggatc caactcagca 1980
gccccttcgg ggcggctctc taacccacag tgccccaggg ccctccctga gccagcccct 2040
ggcccagttg acgcctccag catctgcccc agtaccagca gtctgttcaa cttgcaaaaa 2100
tccagcctct ctgcccgaca cccacagagg aaaaggcggg ggggtccctc cgagcccacc 2160
cctggctctc ggccccagga tgcaactgtg cacccagctt gccagatttt tccccattac 2220
acccccagtg tggcatatcc ttggtcccca gaggcacacc ccttgatctg tggacctcca 2280
ggcctggaca agaggctgct accagaaacc ccaggcccct gttactcaaa ttcacagcca 2340
gtgtggttgt gcctgactcc tcgccagccc ctggaaccac atccacctgg ggaggggcct 2400
tctgaatgga gttctgacac cgcagagggc aggccatgcc cttatccgca ctgccaggtg 2460
ctgtcggccc agcctggctc agaggaggaa ctcgaggagc tgtgtgaaca ggctgtgtga 2520
gatgttcagg cctagctcca accaagagtg tgctccagat gtgtttgggc cctacctggc 2580
acagagtcct gctcctggga aaggaaagga ccacagcaaa caccattctt tttgccgtac 2640
ttcctagaag cactggaaga ggactggtga tggtggaggg tgagagggtg ccgtttcctg 2700
ctccagctcc agaccttgtc tgcagaaaac atctgcagtg cagcaaatcc atgtccagcc 2760
aggcaaccag ctgctgcctg tggcgtgtgt gggctggatc ccttgaaggc tgagtttttg 2820
agggcagaaa gctagctatg ggtagccagg tgttacaaag gtgctgctcc ttctccaacc 2880
cctacttggt ttccctcacc ccaagcctca tgttcatacc agccagtggg ttcagcagaa 2940
cgcatgacac cttatcacct ccctccttgg gtgagctctg aacaccagct ttggcccctc 3000
cacagtaagg ctgctacatc aggggcaacc ctggctctat cattttcctt ttttgccaaa 3060

aggaccagta gcataggtga gccctgagca ctaaaaggag gggtccctga agctttccca 3120
ctatagtgtg gagttctgtc cctgaggtgg gtacagcagc cttggttcct ctgggggttg 3180
agaataagaa tagtggggag ggaaaaactc ctccttgaag atttcctgtc tcagagtccc 3240
agagaggtag aaaggaggaa tttctgctgg actttatctg ggcagaggaa ggatggaatg 3300
aaggtagaaa aggcagaatt acagctgagc ggggacaaca aagagttctt ctctgggaaa 3360
agttttgtct tagagcaagg atggaaaatg gggacaacaa aggaaaagca aagtgtgacc 3420
cttgggtttg gacagcccag aggcccagct ccccagtata agccatacag gccagggacc 3480
cacaggagag tggattagag cacaagtctg gcctcactga gtggacaaga gctgatgggc 3540
ctcatcaggg tgacattcac cccagggcag cctgaccact cttggcccct caggcattat 3600
cccatttgga atgtgaatgt ggtggcaaag tgggcagagg accccacctg ggaacctttt 3660
tccctcagtt agtggggaga ctagcaccta ggtacccaca tgggtattta tatctgaacc 3720
agacagacgc ttgaatcagg cactatgtta agaaatatat ttatttgcta atatatttat 3780
ccacaaaaaa aaaaaaaaaa aa 3802

<210> 92
<211> 1236
<212> DNA
<213> NM_017918.3| Homo sapiens hypothetical protein FLJ20647 (FLJ20647), RNA

<400> 92

caggcgctga cgaggagccc ggctgaggga ggatgcgccg ctgacgcctg cgggagccgc 60
gcgcctgggg cgggaggatg ctccagaggg gcctctggcc gtggcgcacg cggctgctgc 120
cgacccctgg cacctggcgc ccagcgcgcc cgtggccgct gccgcctccg ccccaggttt 180
tgcgtgtgaa gctgtgtgga aatgtgaaat actaccagtc acaccattat agtaccgtgg 240
tgccacctga tgaaataaca gttatttata gacatggcct tcccttggta acacttacct 300
tgccatctag aaaagaacgt tgtcaattcg tagtcaaacc aatgttgtca acagttggtt 360
cattccttca ggacctacaa aatgaagata agggtatcaa aactgcagcc atcttcacag 420
cagatggcaa catgatttca gcttctacct tgatggatat tttgctaatg aatgatttta 480
aacttgtcat taataaaata gcatatgatg tgcagtgtcc aaagagagaa aaaccaagta 540
atgagcacac tgctgagatg gaacacatga aatccttggt tcacagacta tttacaatct 600
tgcatttaga agagtctcag aaaaagagag agcaccattt actggagaaa attgaccacc 660
tgaaggaaca gctgcagccc cttgaacagg tgaaagctgg aatagaagct cattcggaag 720
ccaaaaccag tggactcctg tgggctggat tggcactgct gtccattcag ggtggggcac 780
tggcctggct cacgtggtgg gtgtactcct gggatatcat ggagccagtt acatacttca 840

tcacatttgc aaattctatg gtcttttttg catactttat agtcactcga caggattata 900
cttactcagc tgttaagagt aggcaatttc ttcagttctt ccacaagaaa tcaaagcaac 960
agcactttga tgtgcagcaa tacaacaagt taaaagaaga ccttgctaag gctaaagaat 1020
ccctgaaaca ggcgcgtcat tctctctgtt tgcaaatgca agtagaagaa ctcaatgaaa 1080
agaattaatc ttacagtttt aaatgtcgtc agattttcca ttatgtattg attttgcaac 1140
ttaggatgtt tttgagtccc atggttcatt ttgattgttt aatctttgtt attaaattct 1200
tgtaaaacag aaaaaaaaaa aaaaaaaaaa aaaaaa 1236

<210> 93
<211> 2096
<212> DNA
<213> NM_024792.1| Homo sapiens membrane protein expressed in epithelial-like lung adenocarcinoma (CT120), mRNA

<400> 93

agccacgcgg cgccagcgag gcggccggac ccgcagcccc gatgctgctg acgctggccg 60
ggggcgcgct cttcttcccg gggctcttcg cgctctgcac ctgggcgctg cgccgctccc 120
agcccggatg gagccgcacc gactgcgtga tgatcagcac caggctggtt tcctcggtgc 180
acgccgtgct ggccaccggc tcggggatcg tcatcattcg ctcctgcgac gacgtgatca 240
ccggcaggca ctggcttgcc cgggaatatg tgtggtttct gattccatac atgatctatg 300
actcgtacgc catgtacctc tgtgaatggt gccgaaccag agaccagaac cgtgcgccct 360
ccctcactct tcgaaacttc ctaagtcgaa accgcctcat gatcacacat catgcggtca 420
ttctctttgt ccttgtgcca gtcgcacaga ggctccgggg agaccttggg gacttctttg 480
tcggctgcat cttcacggca gaactgagca ctccgtttgt gtcgctgggc agggttctga 540
ttcagctaaa gcagcagcac acccttctgt acaaggtgaa tggaatcctc acgctggcca 600
ccttcctttc ctgccggatc cttctcttcc ccttcatgta ctggtcctat ggccgccagc 660
agggactaag cctgctccaa gtacccttca gcatcccatt ctactgcaac gtggccaatg 720
ccttcctcgt agctcctcag atctactggt tctgtctgct gtgcaggaag gcagtccggc 780
tctttgacac tccccaagcc aaaaaggatg gctaaatgct cctgggagtc aggcgcagcc 840
tcacaccagc tgcctcctcc actcagcatt ccatggacca aattgtgccc tgggtagcct 900
cagactttgg gtattgataa gccgatggat ttgagttttt ctaaagaata ttcatattac 960
ctccttcttc taacttgccc tatttgcaaa agcacttttg tagtaacaac tattgggtcc 1020
tgtcagacct ccacggacag caaagtggtt ttaatgcaag cccaaggatc cttcttaagg 1080
tcttatctca agagctctgg gaggtggaag catggggtgg gatcggtgga ccagggtggt 1140
aagtgtctgc acatctgcct gtccctgtat cagcggctac ccaccttcca aaccactcag 1200

```
gacagtaccc gtggcactgg gcccgcagaa gcaagggatg acttggttct tggaagtaat    1260
gtcgtcttgt gacattggcc tgggacaatc attgtgggta ggtagttatt gatcgtttac    1320
tagataaccc attggttctt tgcctcatcc tctcatccat gggtcagagt tgaattctta    1380
tgtctataga cttccaatca gaagtctcac tggtggggct gggggtgggg gcaggcagga    1440
ggcatggatg ggaacctgag taggtagtgt ggccaagaga tcagcacaac ctttgcaggc    1500
tgacttgcta agtctgacag tgacaaactt gtgagcttac tgcagtcagt cacagaggct    1560
gttctttttc acacacccct tcatgcccgg ctttccccat atccacatgc agagggcgag    1620
ctcataaaac tacagggaag cgtgaaatga tggctttggt agctgtttac tgggtaaccc    1680
cactgtgaca ctgtcctttt catgtgatgt ggaaacctac ttctgtcctc caaaccatga    1740
aatgtgtcat ctagactgca gagtacttga gtgctttgcc tcccgatatg ccagagcttg    1800
tggtccaaag cccattcctg tgtgtccgtc ctgccattta gccacagaag gctgcggagt    1860
gaggcggcag ctagcctggc cagtggctgt cccgtggacc gacacctgcg ccccttctg     1920
caagcaggat tttctggtgc caacactcat tcatcattcc cgatcaacta ggatgaattt    1980
aagactgtgc taccatgtgt tctcaagtgg tagtttaaaa agtggatttt taaagtgcct    2040
ttcaattgtc tgtgaacgtc taaaggactg atttgtctca aaaaaaaaaa aaaaaa        2096
```

<210> 94
<211> 4372
<212> DNA
<213> NM_014314.2| Homo sapiens DEAD (Asp-Glu-Ala-Asp) box polypeptide 58 (DDX58), mRNA

<400> 94

```
tagttattaa agttcctatg cagctccgcc tcgcgtccgg cctcatttcc tcggaaaatc      60
cctgctttcc ccgctcgcca cgccctcctc ctacccggct ttaaagctag tgaggcacag     120
cctgcgggga acgtagctag ctgcaagcag aggccggcat gaccaccgag cagcgacgca     180
gcctgcaagc cttccaggat tatatccgga agaccctgga ccctacctac atcctgagct     240
acatggcccc ctggtttagg gaggaagagg tgcagtatat tcaggctgag aaaaacaaca     300
agggcccaat ggaggctgcc acactttttc tcaagttcct gttggagctc caggaggaag     360
gctggttccg tggctttttg gatgccctag accatgcagg ttattctgga ctttatgaag     420
ccattgaaag ttgggatttc aaaaaaattg aaaagttgga ggagtataga ttacttttaa     480
aacgtttaca accagaattt aaaaccagaa ttatcccaac cgatatcatt tctgatctgt     540
ctgaatgttt aattaatcag gaatgtgaag aaattctaca gatttgctct actaagggga     600
tgatggcagg tgcagagaaa ttggtggaat gccttctcag atcagacaag gaaaactggc     660
ccaaaacttt gaaacttgct ttggagaaag aaaggaacaa gttcagtgaa ctgtggattg     720
```

tagagaaagg tataaaagat gttgaaacag aagatcttga ggataagatg gaaacttctg 780
acatacagat tttctaccaa gaagatccag aatgccagaa tcttagtgag aattcatgtc 840
caccttcaga agtgtctgat acaaacttgt acagcccatt taaaccaaga aattaccaat 900
tagagcttgc tttgcctgct atgaaaggaa aaaacacaat aatatgtgct cctacaggtt 960
gtggaaaaac ctttgtttca ctgcttatat gtgaacatca tcttaaaaaa ttcccacaag 1020
gacaaaaggg gaaagttgtc tttttgcga atcagatccc agtgtatgaa cagcagaaat 1080
ctgtattctc aaaatacttt gaaagacatg ggtatagagt tacaggcatt tctggagcaa 1140
cagctgagaa tgtcccagtg gaacagattg ttgagaacaa tgacatcatc attttaactc 1200
cacagattct tgtgaacaac cttaaaaagg gaacgattcc atcactatcc atctttactt 1260
tgatgatatt tgatgaatgc cacaacacta gtaaacaaca cccgtacaat atgatcatgt 1320
ttaattatct agatcagaaa cttggaggat cttcaggccc actgccccag gtcattgggc 1380
tgactgcctc ggttggtgtt ggggatgcca aaaacacaga tgaagccttg gattatatct 1440
gcaagctgtg tgcttctctt gatgcgtcag tgatagcaac agtcaaacac aatctggagg 1500
aactggagca agttgtttat aagccccaga agtttttcag gaaagtggaa tcacggatta 1560
gcgacaaatt taaatacatc atagctcagc tgatgaggga cacagagagt ctggcaaaga 1620
gaatctgcaa agacctcgaa aacttatctc aaattcaaaa tagggaattt ggaacacaga 1680
aatatgaaca atggattgtt acagttcaga aagcatgcat ggtgttccag atgccagaca 1740
aagatgaaga gagcaggatt tgtaaagccc tgtttttata cacttcacat ttgcggaaat 1800
ataatgatgc cctcattatc agtgagcatg cacgaatgaa agatgctctg gattacttga 1860
aagacttctt cagcaatgtc cgagcagcag gattcgatga gattgagcaa gatcttactc 1920
agagatttga agaaaagctg caggaactag aaagtgtttc cagggatccc agcaatgaga 1980
atcctaaact tgaagacctc tgcttcatct tacaagaaga gtaccactta aacccagaga 2040
caataacaat tctctttgtg aaaaccagag cacttgtgga cgctttaaaa aattggattg 2100
aaggaaatcc taaactcagt tttctaaaac ctggcatatt gactggacgt ggcaaaacaa 2160
atcagaacac aggaatgacc ctcccggcac agaagtgtat attggatgca ttcaaagcca 2220
gtggagatca caatattctg attgccacct cagttgctga tgaaggcatt gacattgcac 2280
agtgcaatct tgtcatcctt tatgagtatg tgggcaatgt catcaaaatg atccaaacca 2340
gaggcagagg aagagcaaga ggtagcaagt gcttccttct gactagtaat gctggtgtaa 2400
ttgaaaaaga acaaataaac atgtacaaag aaaaaatgat gaatgactct attttacgcc 2460
ttcagacatg ggacgaagca gtatttaggg aaaagattct gcatatacag actcatgaaa 2520
aattcatcag agatagtcaa gaaaaaccaa aacctgtacc tgataaggaa aataaaaaac 2580
tgctctgcag aaagtgcaaa gccttggcat gttacacagc tgacgtaaga gtgatagagg 2640
aatgccatta cactgtgctt ggagatgctt ttaaggaatg ctttgtgagt agaccacatc 2700
ccaagccaaa gcagttttca agttttgaaa aaagagcaaa gatattctgt gcccgacaga 2760

```
actgcagcca tgactgggga atccatgtga agtacaagac atttgagatt ccagttataa    2820
aaattgaaag ttttgtggtg gaggatattg caactggagt tcagacactg tactcgaagt    2880
ggaaggactt tcattttgag aagataccat ttgatccagc agaaatgtcc aaatgatatc    2940
aggtcctcaa tcttcagcta cagggaatga gtaactttga gtggagaaga aacaaacata    3000
gtgggtataa tcatggatcg cttgtacccc tgtgaaaata tattttttaa aaatatcttt    3060
agcagtttgt actatattat atatgcaaag cacaaatgag tgaatcacag cactgagtat    3120
tttgtaggcc aacagagctc atagtacttg ggaaaaatta aaaagcctca tttctagcct    3180
tctttttaga gtcaactgcc aacaaacaca cagtaatcac tctgtacaca ctgggataga    3240
tgaatgaatg gaatgttggg aatttttatc tccctttgtc tccttaacct actgtaaact    3300
ggcttttgcc cttaacaatc tactgaaatt gttcttttga aggttaccag tgactctggt    3360
tgccaaatcc actgggcact tcttaacctt ctatttgacc tctgcgcatt tggccctgtt    3420
gagcactctt cttgaagctc tccctgggct tctctctctt ctagttctat tctagtcttt    3480
ttttattgag tcctcctctt tgctgatccc ttccaagggt tcaatatata tacatgtata    3540
tactgtacat atgtatatgt aactaatata catacataca ggtatgtata tgtaatggtt    3600
atatgtactc atgttcctgg tgtagcaacg tgtggtatgg ctacacagag aacatgagaa    3660
cataaagcca tttttatgct tactactaaa agctgtccac tgtagagttg ctgtatgtag    3720
caatgtgtat ccactctaca gtggtcagct tttagtagag agcataaaaa tgataaaata    3780
cttcttgaaa acttagttta ctatacatct tgccctatta atatgttctc ttaacgtgtg    3840
ccattgttct ctttgaccat tttcctataa tgatgttgat gttcaacacc tggactgaat    3900
gtctgttctc agatcccttg gatgttacag atgaggcagt ctgactgtcc tttctacttg    3960
aaagattaga atatgtatcc aaatggcatt cacgtgtcac ttagcaaggt ttgctgatgc    4020
ttcaaagagc ttagtttgcg gtttcctgga cgtggaaaca agtatctgag ttccctggag    4080
atcaacggga tgaggtgtta cagctgcctc cctcttcatg caatctggtg agcagtggtg    4140
caggcgggga gccagagaaa cttgccagtt atataacttc tctttggctt ttcttcatct    4200
gtaaaacaag gataatactg aactgtaagg gttagtggag agtttttaat taaaagaatg    4260
tgtgaaaagt acatgacaca gtagttgctt gataatagtt actagtagta gtattcttac    4320
taagacccaa tacaaatgga ttatttaaac caaaaaaaaa aaaaaaaaaa aa            4372
```

<210> 95
<211> 2163
<212> DNA
<213> NM_015515.3| Homo sapiens keratin 23 (histone deacetylase inducible) (KRT23), transcript variant 1, mRNA

<400> 95

ggcagatgaa atataagatt catcaaccac atttgacagc ccatggcagg tttcctgttt 60
tccatcgtcc ctctgcaggt cacagacaca cagagcccag ccgtggcagg ctcagccggg 120
gtccggggct gctaacaacg gctacattcc tcccccaggg ccaagggaaa tcctgagcgc 180
aggccagggt tgtttggttt tgaggtgtgc tgggatgaaa ggcaccctgg aagtggaagg 240
taaatgaaca atggaaaaac ttcacggcaa gattagaaag atacctgagc ccaatacccg 300
cctgatgtcg tgggccacac ctccgggtta ccaggggaag ggaggaagca aactgtcata 360
ttgatgtggc tctaaacaac aacagtgtgc gaaggcccag gggcactttg ggattgacca 420
agaggaaaca caagttgcac aatgatacaa tcttgttggt acaattgtca gagaagggaa 480
ctcccacagc aaaggccata aaaccatcca gggcagtctg gggcggctca gttctgcggt 540
gccagggagt ggagcagagc tcagccccgt cccaaacaca gatgggacca tgaactccgg 600
acacagcttc agccagaccc cctcggcctc cttccatggc gccggaggtg gctggggccg 660
gcccaggagc ttccccaggg ctcccaccgt ccatggcggt gcggggggag cccgcatctc 720
cctgtccttc accacgcgga gctgcccacc ccctggaggg tcttggggtt ctggaagaag 780
cagcccccta ctaggcggaa atgggaaggc caccatgcag aatctcaacg accgcctggc 840
ctcctacctg gagaaggttc gcgccctgga ggaggccaac atgaagctgg aaagccgcat 900
cctgaaatgg caccagcaga gagatcctgg cagtaagaaa gattattccc agtatgagga 960
aaacatcaca cacctgcagg agcagatagt ggatggtaag atgaccaatg ctcagattat 1020
tcttctcatt gacaatgcca ggatggcagt ggatgacttc aacctcaagt atgaaaatga 1080
acactccttt aagaaagact tggaaattga agtcgagggc ctccgaagga ccttagacaa 1140
cctgaccatt gtcacaacag acctagaaca ggaggtggaa ggaatgagga aagagctcat 1200
tctcatgaag aagcaccatg agcaggaaat ggagaagcat catgtgccaa gtgacttcaa 1260
tgtcaatgtg aaggtggata caggtcccag ggaagatctg attaaggtcc tggaggatat 1320
gagacaagaa tatgagctta taataaagaa gaagcatcga gacttggaca cttggtataa 1380
agaacagtct gcagccatgt cccaggaggc agccagtcca gccactgtgc agagcagaca 1440
aggtgacatc cacgaactga agcgcacatt ccaggccctg gagattgacc tgcagacaca 1500
gtacagcacg aaatctgctt tggaaaacat gttatccgag acccagtctc ggtactcctg 1560
caagctccag gacatgcaag agatcatctc ccactatgag gaggaactga cgcagctacg 1620
ccatgaactg gagcggcaga acaatgaata ccaagtgctg ctgggcatca aaacccacct 1680
ggagaaggaa atcaccacgt accgacggct cctggaggga gagagtgaag ggacacggga 1740
agaatcaaag tcgagcatga aagtgtctgc aactccaaag atcaaggcca taacccagga 1800
gaccatcaac ggaagattag ttctttgtca agtgaatgaa atccaaaagc acgcatgaga 1860
ccaatgaaag tttccgcctg ttgtaaaatc tattttcccc caaggaaagt ccttgcacag 1920
acaccagtga gtgagttcta aaagataccc ttggaattat cagactcaga aacttttatt 1980

tttttttct gtaacagtct caccagactt ctcataatgc tcttaatata ttgcactttt 2040
ctaatcaaag tgcgagttta tgagggtaaa gctctacttt cctactgcag ccttcagatt 2100
ctcatcattt tgcatctatt ttgtagccaa taaaactccg cactagcaaa aaaaaaaaaa 2160
aaa 2163

<210> 96
<211> 2881
<212> DNA
<213> NM_007210.2| Homo sapiens UDP-N-acetyl-alpha-D-galactosamine:polypeptide N-acetylgalactosaminyl transferase 6 (GalNAc-T6) (GALNT6), mRNA

<400> 96

atgaggctcc tccgcagacg ccacatgccc ctgcgcctgg ccatggtggg ctgcgccttt 60
gtgctcttcc tcttcctcct gcatagggat gtgagcagca gagaggaggc cacagagaag 120
ccgtggctga agtccctggt gagccggaag gatcacgtcc tggacctcat gctggaggcc 180
atgaacaacc ttagagattc aatgcccaag ctccaaatca gggctccaga agcccagcag 240
actctgttct ccataaacca gtcctgcctc cctgggttct ataccccagc tgaactgaag 300
cccttctggg aacggccacc acaggacccc aatgcccctg gggcagatgg aaaagcattt 360
cagaagagca agtggacccc cctggagacc caggaaaagg aagaaggcta taagaagcac 420
tgtttcaatg cctttgccag cgaccggatc tccctgcaga ggtccctggg gccagacacc 480
cgaccacctg agtgtgtgga ccagaagttc cggcgctgcc ccccactggc caccaccagc 540
gtgatcattg tgttccacaa cgaagcctgg tccacactgc tgcgaacagt gtacagcgtc 600
ctacacacca cccctgccat cttgctcaag gagatcatac tggtggatga tgccagcaca 660
gaggagcacc taaaggagaa gctggagcag tacgtgaagc agctgcaggt ggtgagggtg 720
gtgcggcagg aggagcggaa ggggttgatc accgcccggc tgctgggggc cagcgtggca 780
caggcggagg tgctcacgtt cctggatgcc cactgtgagt gcttccacgg ctggctggag 840
cccctcctgg ctcgaatcgc tgaggacaag acagtggtgg tgagcccaga catcgtcacc 900
atcgacctta atacttttga gttcgccaag cccgtccaga ggggcagagt ccatagccga 960
ggcaactttg actggagcct gaccttcggc tgggaaacac ttcctccaca tgagaagcag 1020
aggcgcaagg atgaaacata ccccatcaaa tccccgacgt ttgctggtgg cctcttctcc 1080
atccccaagt cctactttga gcacatcggt acctatgata atcagatgga gatctgggga 1140
ggggagaacg tggaaatgtc cttccgggtg tggcagtgtg gggggccagct ggagatcatc 1200
ccctgctctg tcgtaggcca tgtgttccgg accaagagcc cccacacctt ccccaagggc 1260
actagtgtca ttgctcgcaa tcaagtgcgc ctggcagagg tctggatgga cagctacaag 1320
aagattttct ataggagaaa tctgcaggca gcaaagatgg cccaagagaa atccttcggt 1380

gacatttcgg aacgactgca gctgagggaa caactgcact gtcacaactt ttcctggtac 1440
ctgcacaatg tctacccaga gatgtttgtt cctgacctga cgcccacctt ctatggtgcc 1500
atcaagaacc tcggcaccaa ccaatgcctg gatgtgggtg agaacaaccg cggggggaag 1560
cccctcatca tgtactcctg ccacggcctt ggcggcaacc agtactttga gtacacaact 1620
cagagggacc ttcgccacaa catcgcaaag cagctgtgtc tacatgtcag caagggtgct 1680
ctgggccttg ggagctgtca cttcactggc aagaatagcc aggtccccaa ggacgaggaa 1740
tgggaattgg cccaggatca gctcatcagg aactcaggat ctggtacctg cctgacatcc 1800
caggacaaaa agccagccat ggcccctgc aatcccagtg acccccatca gttgtggctc 1860
tttgtctagg acccagatca tccccagaga gagcccccac aagctcctca ggaaacagga 1920
ttgctgatgt ctgggaacct gatcaccagc ttctctggag gccgtaaaga tggatttcta 1980
aacccactgg gtggcaaggc aggaccttcc taatccttgc aacaacattg ggcccatttt 2040
ctttccttca caccgatgga agagaccatt aggacatata tttagcctag cgttttcctg 2100
ttctagaaat agaggctccc aaagtaggga aggcagctgg gggagggttc agggcagcaa 2160
tgctgagttc aagaaaagta cttcaggctg ggcacagtgg ctcatgcctg aaatcctagc 2220
actttgggaa gacaatgtgg gagaatggct tgagcccagg agttcaagac cggcctgagc 2280
aacatagtga ggatcccatc tctacgccca ccctccccc ggcaaaaaaa aaagctgggt 2340
atggtggctt atgcctgtag tcgcagctac tcagaaggct gaggtgggag gattgcttgt 2400
tccccggagg ttgaagctac agtgagcctt gattgtgtca ctgcactcca gcctgggcaa 2460
caggtaagac tctgtctcaa aaaaaaaaca aaaaagaaga agaaaagtac ttctacagcc 2520
atgtcctatt ccttgatcat ccaaagcacc tgcagagtcc agtgaaatga tatattctgg 2580
ctgggcacag tggctcacac ctgtaatcct agcactttgg gaggccaagg caggtggatc 2640
acctgaggtc agaagtttga aaccagcctg gactacatgg tgaaactcca tctctactaa 2700
aagtacaaaa attagctggg catgatggca cgcacctgca gtcccagcta cttgggaggc 2760
tgaggcagga gaatcactcg aacccaggag gcagaggttg cagtgagcca agacagcacc 2820
attgcacccc agcctgagca acaagagcga aactccatct caggaaaaaa aaaaaaaaaa 2880
a 2881

<210> 97
<211> 1930
<212> DNA
<213> NM_020183.3| Homo sapiens aryl hydrocarbon receptor nuclear translocator-like 2 (ARNTL2), mRNA

<400> 97

gaccaagtgg ctcctgcgat ggcggcggaa gaggaggctg cggcgggagg taaagtgttg 60

agagaggaga accagtgcat tgctcctgtg gtttccagcc gcgtgagtcc agggacaaga 120
ccaacagcta tggggtcttt cagctcacac atgacagagt ttccacgaaa acgcaaagga 180
agtgattcag acccatccca gtcaggaatc atgacagaaa aagtggtgga aaagctttct 240
cagaatcccc ttacctatct tctttcaaca aggatagaaa tatcagcctc cagtggcagc 300
agagtggaag atggtgaaca ccaagttaaa atgaaggcct tcagagaagc tcatagccaa 360
actgaaaagc ggaggagaga taaaatgaat aacctgattg aagaactgtc tgcaatgatc 420
cctcagtgca accccatggc gcgtaaactg gacaaactta cagttttaag aatggctgtt 480
caacacttga gatctttaaa aggcttgaca aattcttatg tgggaagtaa ttatagacca 540
tcatttcttc aggataatga gctcagacat ttaatcctta agactgcaga aggcttctta 600
tttgtggttg gatgtgaaag aggaaaaatt ctcttcgttt ctaagtcagt ctccaaaata 660
cttaattatg atcaggctag tttgactgga caaagcttat ttgacttctt acatccaaaa 720
gatgttgcca aagtaaagga acaactttct tcttttgata tttcaccaag agaaaagcta 780
atagatgcca aaactggttt gcaagttcac agtaatctcc acgctggaag gacacgtgtg 840
tattctggct caagacgatc tttttctgt cggataaaga gttgtaaaat ctctgtcaaa 900
gaagagcatg gatgcttacc caactcaaag aagaaagagc acagaaaatt ctatactatc 960
cattgcactg gttacttgag aagctggcct ccaaatattg ttggaatgga agaagaaagg 1020
aacagtaaga aagacaacag taattttacc tgccttgtgg ccattggaag attacagcca 1080
tatattgttc cacagaacag tggagagatt aatgtgaaac caactgaatt tataacccgg 1140
tttgcagtga atggaaaatt tgtctatgta gatcaaaggg caacagcgat tttaggatat 1200
ctgcctcagg aacttttggg aacttcttgt tatgaatatt ttcatcaaga tgaccacaat 1260
aatttgactg acaagcacaa agcagttcta cagagtaagg agaaaatact tacagattcc 1320
tacaaattca gagcaaaaga tggctctttt gtaactttaa aaagccaatg gtttagtttc 1380
acaaatcctt ggacaaaaga actggaatat attgtatctg tcaacacttt agttttggga 1440
catagtgagc ctggagaagc atcattttta ccttgtagct ctcaatcatc agaagaatcc 1500
tctagacagt cctgtatgag tgtacctgga atgtctactg gaacagtact tggtgctggt 1560
agtattggaa cagatattgc aaatgaaatt ctggatttac agaggttaca gtcttcttca 1620
taccttgatg attcgagtcc aacaggttta atgaaagata ctcatactgt aaactgcagg 1680
agtatgtcaa ataaggagtt gtttccacca agtccttctg aaatggggga gctagaggct 1740
accaggcaaa accagagtac tgttgctgtc cacagccatg agccactcct cagtgatggt 1800
gcacagttgg atttcgatgc cctatgtgac aatgatgaca cagccatggc tgcatttatg 1860
aattacttag aagcagaggg gggcctggga gaccctgggg acttcagtga catccagtgg 1920
accctctagc 1930

<210> 98
<211> 2128
<212> DNA

<213> NM_014576.2| Homo sapiens apobec-1 complementation factor (ACF), transcript variant 1, mRNA

<400> 98

```
tttgatatga cgattagagc ataacccgag tgacacgttg aattcgccat aatcaaggaa      60
accttttccg ggtggggatc tctgaaatta ctcagataac agtgctgtgc caaaaacctg     120
tggattttct ctacaaaaat tattgagcaa ccctaattaa cctgattttt tgctgataat     180
cactctcaat ggaatcaaat cacaaatccg gggatggatt gagcggcact cagaaggaag     240
cagccctccg cgcactggtc cagcgcacag gatatagctt ggtccaggaa aatggacaaa     300
gaaaatatgg tggccctcca cctggttggg atgctgcacc ccctgaaagg ggctgtgaaa     360
tttttattgg aaaacttccc cgagaccttt ttgaggatga gcttatacca ttatgtgaaa     420
aaatcggtaa aatttatgaa atgagaatga tgatggattt taatggcaac aatagaggat     480
atgcatttgt aacattttca aataaagtgg aagccaagaa tgcaatcaag caacttaata     540
attatgaaat tagaaatggg cgcctcttag ggtttgtgc cagtgtggac aactgccgat      600
tatttgttgg gggcatccca aaaaccaaaa agagagaaga aatcttatcg gagatgaaaa     660
aggttactga aggtgttgtc gatgtcatcg tctacccaag cgctgcagat aaaaccaaaa     720
accgaggctt tgccttcgtg gagtatgaga gtcatcgagc agctgccatg gcgaggagga     780
aactgctacc aggaagaatt cagttatggg gacatggtat tgcagtagac tgggcagagc     840
cagaagtaga agttgatgaa gatacaatgt cttcagtgaa aatcctatat gtaagaaatc     900
ttatgctgtc tacctctgaa gagatgattg aaaaggaatt caacaatatc aaaccaggtg     960
ctgtggagag ggtgaagaaa attcgagact atgcttttgt gcacttcagt aaccgagaag    1020
atgcagttga ggctatgaaa gctttaaatg gcaaggtgct ggatggttcc cccattgaag    1080
tcaccctagc aaaaccagtg gacaaggaca gttatgttag gtatacccga ggcacaggtg    1140
gaaggggcac catgctgcaa ggagagtata cctactcttt gggccaagtt tatgatccca    1200
ccacaaccta ccttggagct cctgtcttct atgcccccca gacctatgca gcaattccca    1260
gtcttcattt cccagccacc aaaggacatc tcagcaacag agccattatc cgagcccctt    1320
ctgttagagg ggctgcggga gtgagaggac tgggcggccg tggctatttg gcatacacag    1380
gcctgggtcg aggataccag gtcaaaggag acaaaagaga agacaaactc tatgacattt    1440
tacctgggat ggagctcacc ccaatgaatc ctgtcacatt aaaaccccaa ggaattaaac    1500
tcgctcccca gatattagaa gagatttgtc agaaaaataa ctggggacag ccagtgtacc    1560
agctgcactc tgctattgga caagaccaaa gacagctatt cttgtacaaa ataactattc    1620
ctgctctagc cagccagaat cctgcaatcc accctttcac acctccaaag ctgagtgcct    1680
ttgtggatga agcaaagacg tatgcagccg aatacaccct gcagaccctg ggcatcccca    1740
```

ctgatggagg cgatggcacc atggctactg ctgctgctgc tgctactgct ttcccaggat 1800
atgctgtccc taatgcaact gcacccgtgt ctgcagccca gctcaagcaa gcggtaaccc 1860
ttggacaaga cttagcagca tatacaacct atgaggtcta cccaactttt gcagtgactg 1920
cccgagggga tggatatggc accttctgaa gatgcttttt taaatttaag aataagacac 1980
acaaaactct attaaaaaaa aaaaagaaat aaacctctaa ctcggtcccc aatgatcata 2040
aataatatgt ttcctaaaga aatgcctttc cagagactgt atagcttata ccaattatag 2100
aatcatgaag taaaaaaaaa aaaaaaa 2128

<210> 99
<211> 5730
<212> DNA
<213> NM_019008.4| Homo sapiens hypothetical protein FLJ20232 (FLJ20232), mRNA

<400> 99

cctccgcctc cactcgccct cgtgctccct tcagcccctt cgcagctccg tgcgcaaggt 60
cgtgtcccgg aagtgaaggg gccatgttga tgggtgaccc ggggagaggt acccggccag 120
aggcgagtcc tgcggagtgg tagcgcgcac ggcctgcggt gtgacaccca gcccctgcca 180
gtcccccatg gccccgtgga gccgagaggc ggtgctgagt ctctatcggg ctctgttgcg 240
ccagggccga cagcttcgct acactgatcg agacttctac tttgcctcca tccgccgtga 300
attccgaaaa aatcagaagc tagaggacgc tgaggcccgg gagaggcagc tggagaaggg 360
cctggtcttt ctcaacggca aattggggag gatcatttag gatcctccaa gggaaagagg 420
acaaaggtgc cttctgtaga cactcctgct ctcttccatc cccatcttac agatgtatta 480
agaagcctca gatgagcaat ggcaggcgct ggtgagcgca aaggcaagaa ggatgacaat 540
ggcattggca cggccattga ctttgtgctc tccaatgccc ggctggtgct gggggtgggt 600
ggagcggcca tgctgggcat cgccacgctg gcagttaagc ggatgtacga tcgggcgatc 660
agtgccccta ccagccccac ccgcctgagc cattcgggga aaaggagctg ggaagaaccc 720
aactggatgg gctccccacg actgctgaac agggacatga agacgggcct gagccggtcc 780
ttgcagaccc ttcccacaga ctcctccacc ttcgacacag atacattctg cccgccccgg 840
cccaagccag tggccaggaa gggccaggta gacttgaaga agtcacgact ccgcatgtcc 900
ctgcaggaga aacttcttac ttactaccgg aaccgggcag ccatccctgc tggagagcag 960
gctcgggcca agcaagctgc tgtggacata tgtgccgagc tccggagctt cctgcgggcc 1020
aagttgcctg acatgccgct tcgggacatg tacttgagtg gcagcctcta cgatgacctg 1080
caggtggtga cagctgacca catccaactc attgtgcccc ttgtgctgga gcagaacctg 1140
tggtcatgta ttcctggtga agacaccatc atgaatgtcc ctggcttctt cctggtgcgt 1200

cgtgagaatc cagagtactt tcctcgtggg agcagttact gggaccgctg tgtagtaggg 1260
ggctacctct ctccaaagac agtcgcagat acatttgaga aggtagtggc tggctccatc 1320
aattggccag ccatagggtc cctcttggac tatgtgatcc gcccggcccc acccccagaa 1380
gccctcacac tggaggtgca gtatgagcgt gacaaacatc tcttcattga cttcctgcca 1440
tcagtgaccc tcggtgacac agtcttggtg gccaaaccac accggctagc ccagtatgac 1500
aacctgtggc ggctgagcct gcgtcccgcg gagacggcac gcctgcgggc tctggaccag 1560
gctgactcgg gctgccgatc tctgtgcctc aagatcctca aggccatatg caagtccacc 1620
ccggctctgg gccacctcac tgccagccag ctaaccaatg tcatcctcca cttggcccag 1680
gaggaggctg actggtctcc ggatatgctg gccgaccgtt tcctgcaggc cttgagggga 1740
cttatcagct acttagaggc tggagtcctg cccagtgccc taaaccccaa ggtgaactta 1800
tttgcagagc tcacccctga agaaatagac gaattaggat acactctgta ttgctcattg 1860
tctgagccag aggtgctgct gcagacgtag ggcaggtgaa ggccaaagcg ggtgttggtg 1920
gtcaggccct ggattctccg ttagatacac ttggctacct agttggtgcc tcacagggtt 1980
cctgctgcct ggtgtcttgc tgatcatcac cctggtcact tcatgctgat tagaatgaca 2040
tctctttcgt ctcctatttt gttacccaac tcttcctatt tttgttacca atcactgtgc 2100
tctctgccgc cccctggctc caggctaatt tttctggaat gaattgagaa ggtggcgtgc 2160
tggcctgagc tgatggacca cttggtgttt tgcgttttgg cccatgtttg ctgcctctat 2220
ctggtctgcc ttgcccgttt gcctgttcct attcagtgtc ttttctattt tttcctctct 2280
cgttcatgcc ttctgttttg ctcttgtccc tggagcatat ctgcctaatt aagatgttgc 2340
cttttagttg aatgccactg aagagctgtg atagcatgtt tcaaagctga actctacaga 2400
gcgagtgctg agacagtatt tagggtttct gggagtgagg ctggtagaag agttggcctt 2460
tgaccacggt tcctggagta gaagtccatc ctccccccaa cctcctgacc cattcataaa 2520
tgctgagaat gtctctcatg ggaacactgt taatgaccca cacaggataa gctgaatgca 2580
aagttatttg caggttgaat ttcttggtgg ctattagcag aagtgcagag tagggaacca 2640
gagctggtta agggcctagt gaagggtttg tgtgcccagt gtctgctcgt catctgtggc 2700
tgcaggggtc agacagacaa ggatggggac tgccagggca ccacttcatc atgaatgctg 2760
gttttcacac cttttcctta ttttattgcc aatcaggaca aggccttgaa ggaacgcagc 2820
cttagacatc aggtgaggat gatggaggta gacagtcgac tgaatgtcag ctggaaaatc 2880
cagtcactag ttggggtttg gtggccatgt tttctaccca gacaggccct gcttttctag 2940
gatgtggcct tagagcaaga acagacccaa cagccagccc ttcatcctcc agcgtctgcc 3000
ataggaatgt gagaggggtg tttgctgagc gctccgggca cggccagagg gcaagtgagc 3060
atgcacggac ctcttccccc tgtcctgttt ctcacccagc acctggggag atcggtgcta 3120
ccaaggaaga gagcacacag ataagacaga ggggaggagg tgggcatttc ctacattcct 3180
ccttgtttgc cgctgctgag attgcagtat ttattgcaat gtaaatgtat cctgaaggtg 3240

gggaggaatg tttaatctac catgtccgtg tgtcatcttg gtttgtgttt ttccctgttt 3300
gtagcaagac tctgatgata attctgtttc tcatctgccc attcagtatt ttgttttcct 3360
tccgtcaagt tgtcttattt tttcaatgac tacctctcca tcattgaggt tctggtgaag 3420
ctctctgcag ctgtctcatt ccttcccaac gatagtaaca ggaaatgact ctttagcatc 3480
gatacctcaa catcaattta gggtagagat tcctgcccct cttttgtcac agattaggaa 3540
attgagaact agggttaacc ttgactatat ttagaggtct ttttgcctct tttcccctta 3600
acaaggattt cttatggtgg tttcagtttc atttgcataa aggtattgag agggaacaaa 3660
aaacataaag ctgagaatct tgagagagct catctaccct gtctgttggt cagactcaaa 3720
tgagagttaa aaaaaaaaaa aaaaatctgt atgcctgagt accatcctgg atgaatctag 3780
aaggtatggg gtagagcttg acagggttcc tgtgtaccca ctgggtatcc gttagaggta 3840
agggagagga gaggattgat agagtgttgc aaaagtatag attattcatt gagataaagg 3900
atttggtttc cctgccatga gtattaaaaa aatttaagtt ttcccaagct tgcatctctg 3960
accaaatttc acataaaaca ttggaaggag gctgggtgcg gtggctcatg cttgtaatcc 4020
cagcactggg aagctaaggc gggtggatca cttgaggtca ggagttcgag accagcctgg 4080
ccaacatggt gaaaccccgt ctccacgaaa aagataaaaa taagctgggc gtggtggcag 4140
gcgcctataa tcccagctac tcgggaggct gaggcaggag aataacttaa acccgggagg 4200
cggaggttac agtgagctga gatcgtgcca ctgcactcca gcctgggtga cagagtgaga 4260
ccctatttca aaaaaataaa aattggaaga agagcttaaa aaagataaga ttttaaagag 4320
tcccaagtta tttaagttga gtgtaattgt catttaagga aggcaaatga gtttatcatc 4380
cttcttaaag agcatctctt ttaactgttg gacaaaacca taactttgtc attttacaag 4440
gaagaacctc ttaagaagtc ctcagaacca gaagcaatgt gaactctcag cgctggtcct 4500
ggtgggtttg ctgaccatga ctgggcaagc cgttcttttt gctgccatct tcctcatcat 4560
aaagtgtgga acataggcaa ttgctttgag attcttggat agaagaggac aacattctgc 4620
acctgccccc ttttttaaat ctttggggaa agatgagtaa ctttccccac tactctgcct 4680
tcctgttcag taactcttac ttttgcctga agtaacagca tcttctactt ctccatctag 4740
agatttttgt gtgtgtgcca tcaaggttag caaactttat acgtagccta acacttaaaa 4800
aatgcactca ttatcttaaa cctaataaat tccagagttt attttggttc tcctctgttg 4860
cccttcctaa aaaatgagct gaagatgaca gtatttttct ttacatgctt ggttatgact 4920
tttaaagttt tatttaaata aatgttgaag ctcaagttta aagaagcgtt gcagaggccc 4980
acggtctcct gggtcccggc cacctgtcca tattccacat ttgctgactg tgctccctgc 5040
actccactca agttgagagt tcaaatagtc ttgaagggga atcagcttca ggatggaagg 5100
acccaggaga ggccccgagg tgggagggtt ctgtaaatac agactactgc gagtgtccag 5160
agctctctgc catgatactt ccttgggact gacttggctg agaacgtgtt ctgtcagagg 5220

atttgttaga actctgccct tttgtctgaa actcaaggcc aaggagaatg ataggagact 5280
taggacagag ctgacccttg caccaggctg ggaggctgca gccctttag atgccactta 5340
ctgtaagtgg ccagaatacc agagaggtgg gttccatggt caaatgcaca gtaggtgttt 5400
acctttacat ttggatcacc ttgtagtctt taaattcttg gtccctgagg ccaagtccac 5460
aacttgcctt ctagtcactt gcctgcccgc agtggtggtg gatgtgttag ctggtagatt 5520
tggaatcagt caccagtctt tctgtactgt cttggttagc tctatataag taggggcagc 5580
ttagccctga ggcccagaga cctgctgtcc tttttctcct tgagggagga aataaaactg 5640
cggaatacaa tgtccttcca tagcatggga agaagaaaat aaacatctcc tttccaacaa 5700
aaaaaaaaaa aaaaaaaaaa aaaaaaaaaa 5730

<210> 100
<211> 2545
<212> DNA
<213> NM_030882.2| Homo sapiens apolipoprotein L, 2 (APOL2), transcript variant alpha, mRNA

<400> 100

gtgctgggga gcagcgtgtt tactgtgctt ggtcatgagc tgctgggaag ttgtgacttt 60
cactttccct ttcgaattcc agggtatatc tgggaggccg gaggacgtgt ctggttatta 120
cacagatgca cagctggacg tgggatccac acagctcaga acagttggat cttgctcagt 180
ctctgtcaga ggaagatccc ttggacaaga ggaccctgcc ttggtgtgag agtgagggaa 240
gaggaagctg gaacgagggt taaggaaaac cttccagtct ggacagtgac tggagagctc 300
caaggaaagc ccctcggtaa cccagccgct ggcaccatga acccagagag cagtatcttt 360
attgaggatt accttaagta tttccaggac caagtgagca gagagaatct gctacaactg 420
ctgactgatg atgaagcctg gaatggattc gtggctgctg ctgaactgcc cagggatgag 480
gcagatgagc tccgtaaagc tctgaacaag cttgcaagtc acatggtcat gaaggacaaa 540
aaccgccacg ataaagacca gcagcacagg cagtggtttt tgaaagagtt tcctcggttg 600
aaaagggagc ttgaggatca cataaggaag ctccgtgccc ttgcagagga ggttgagcag 660
gtccacagag gcaccaccat tgccaatgtg gtgtccaact ctgttggcac tacctctggc 720
atcctgaccc tcctcggcct gggtctggca cccttcacag aaggaatcag ttttgtgctc 780
ttggacactg gcatgggtct gggagcagca gctgctgtgg ctgggattac ctgcagtgtg 840
gtagaactag taaacaaatt gcgggcacga gcccaagccc gcaacttgga ccaaagcggc 900
accaatgtag caaaggtgat gaaggagttt gtgggtggga acacacccaa tgttcttacc 960
ttagttgaca attggtacca agtcacacaa gggattggga ggaacatccg tgccatcaga 1020
cgagccagag ccaaccctca gttaggagcg tatgccccac ccccgcatat cattgggcga 1080

atctcagctg aaggcggtga acaggttgag agggttgttg aaggccccgc ccaggcaatg 1140
agcagaggaa ccatgatcgt gggtgcagcc actggaggca tcttgcttct gctggatgtg 1200
gtcagccttg catatgagtc aaagcacttg cttgaggggg caaagtcaga gtcagctgag 1260
gagctgaaga agcgggctca ggagctggag gggaagctca actttctcac caagatccat 1320
gagatgctgc agccaggcca agaccaatga ccccagagca gtgcagccac cagggcagaa 1380
atgccgggca caggccagga caaaatgcag actttttttt tttttttttt tttttttga 1440
gatggagtct cgctctatcg cccaggatgg agtgcagtgg ctcaatctcg gctcactgca 1500
aactccgcct cccgggttca caccattctc cggcctcagt ctcccgagta gctgggacta 1560
caggcacctg ccaccacgcc cggctaattt ttttgtattt tcactggaga cggggtttca 1620
ctgtgttagc cacgatggtc tccatctcct gacctcgtga tctgcccacc tcggcctccc 1680
aaagtgctgg gattacaggc gtgagccacc gcgcctggcc aaaatgcaga cattttatta 1740
gggggataag gagggcaagg taaagcttat ggaactgagt gttagtgact ttggcatttg 1800
tgtagctgag cacagcaagg gaggggttaa tgcagatggc aagtgcacca aggagaaggc 1860
aggaacactg gagcctgcaa taagggagga gagaggactg gagagtgtgg ggaatgggaa 1920
gaagtagttt actttggact aaagaatata ttgggcgaag aatagagggg gagcttgcag 1980
gaaccagcaa tgagaaggcc aggaaaagaa agagctgaaa atggagaaaa ccagagttag 2040
aactgttgga tacaggagaa gaaacagcag ctccactacc gacccccccc caggtttgat 2100
gtccttccaa gaataaagtc tttccctggt gatggtctct cgctctgtct ttccagcatc 2160
cactctccct tgtccttctg gggtgtatc acagtcagcc agtggcttct tcatgatggt 2220
ggttggggtg gttgtcatgt gacgggtccc ctccaggtta ctaaagggtg catgtcccct 2280
gcttgaaccc tgagaggcag gtggtaggcc atggccacaa tccccagctg aggagcaggt 2340
gtccctgaga acccaaactt cccagagagt atctgagaac caaccaatga aaacagtccc 2400
atcgctctta gccggtaagt aaacagtcag aagattagca tgaaagcagt ttagcattgg 2460
gaggaagcac agatctctag agctgtcctg tcgctgccca ggattgacct gtgtgtaagt 2520
cccaataaac tcacctactc accaa 2545

<210> 101
<211> 1429
<212> DNA
<213> NM_016612.1| Homo sapiens mitochondrial solute carrier protein (MSCP), mRNA

<400> 101

ccccctcccc tcctgcagcc tcctgcgccc cgccgagctg gcggatggag ctgcgcagcg 60
ggagcgtggg cagccaggcg gtggcgcgga ggatggatgg ggacagccga gatggcggcg 120

gcggcaagga cgccaccggg tcggaggact acgagaacct gccgactagc gcctccgtgt 180
ccacccacat gacagcagga gcgatggccg ggatcctgga gcactcggtc atgtacccgg 240
tggactcggt gaagacacga atgcagagtt tgagtccaga tcccaaagcc cagtacacaa 300
gtatctacgg agccctcaag aaaatcatgc ggaccgaagg cttctggagg cccttgcgag 360
gcgtcaacgt catgatcatg ggtgcagggc cggcccatgc catgtatttt gcctgctatg 420
aaaacatgaa aaggacttta aatgacgttt tccaccacca aggaaacagc cacctagcca 480
acgggatagc tgggagtatg gccaccctgc tccacgatgc ggtaatgaat ccagcagaag 540
tggtgaagca gcgcttgcag atgtacaact cgcagcaccg gtcagcaatc agctgcatcc 600
ggacggtgtg gaggaccgag gggttggggg ccttctaccg gagctacacc acgcagctga 660
ccatgaacat ccccttccag tccatccact tcatcaccta tgagttcctg caggagcagg 720
tcaaccccca ccggacctac aacccgcagt cccacatcat ctcaggcggg ctggccgggg 780
ccctcgccgc ggccgccacg acccccctgg acgtctgtaa gacccttctg aacactcagg 840
agaacgtggc cctctcgctg gccaacatca gcggccggct gtcgggtatg gccaatgcct 900
tccggacggt gtaccagctc aacggcctgc cggctacttc aaaggcatcc aggcgcgtgt 960
catctaccag atgccctcca ccgccatttc ttggtctgtc tatgagttct tcaagtactt 1020
tctcaccaag cgccagctgg aaaatcgagc tccatactaa aggaagggat catagaatct 1080
tttcttaaag tcattctctg cctgcatcca gccccttgcc ctctcctcac acgtagatca 1140
tttttttttt tgcagggtgc tgcctatggg ccctctgctc cccaatgcct tagagagagg 1200
aggggacggc acggccgctc accggaaggc tgtgtgcggg gacatccgag gtggtggtgg 1260
acaggaagga cttgggaagg ggagcgagaa attgcttttt ctcttcctcc ctgggcagaa 1320
tgtagctttt ctgcttcact gtggcagcct cctccctgga tccttagatc ccagaggagg 1380
gaagaaaatt tgcagtgact gaaaacagta aaaaaaaaaa aaaaaaaa 1429

<210> 102
<211> 2368
<212> DNA
<213> NM_017903.2| Homo sapiens hypothetical protein FLJ20618 (FLJ20618), mRNA

<400> 102

ccacgcgtcc gaaaatgttt gaacaattgg atttcaaaca ttttcgtttt gtggagtggt 60
gctcaccaag tggtacagcc ctaagcaagt gaacacaaac acatttaagt gtattttgtc 120
tgattagatg ttagccagtt atgctatttc attcaaatgt ctgaaaaaat caattgacta 180
ttccctttc ctaaagggca gagacagata atctcacttc cagagaaatg acttggagaa 240
aaaaaagtgt tggtcttttt gctcttttgt aattaaatcc ggatgtacct caaaagactt 300
aagactgtgg tgataagatg ctttcctcag cagaaaggag ggaaaaaaaa acaactggaa 360

ctcaaagctt gaaattctgt ggcaaaacat gagatgtcca ggattggagg ttgaaaagat 420
ttcactacag tgttctgcaa tagttggagc agataacttt cagtgtagcc acagccatgg 480
actccagatt tccagatttt caagacctgg acctggaacc cgaaagagct tgtcacgatg 540
cggcaggaac actggaggta gattttttt tatttttgaa ttttgggact gttgaccttg 600
ctgtgagaaa agagacaacg actgagcaag cactaccacc agcactgtta ctgggaatta 660
gaagacctga gtttctgtcc agaccctcag tgcaaactga ggatgctcca tccaaagtga 720
attatgtcct gtgcctcctg attgctgagt gttcacctgg accttctgac taccttccct 780
gtgctattcc atcagcctac agacctggta cctggatttt tgcccgagat gattcctacc 840
accttactac tgacgaagac acccattcca gtggaccact gtgacccagg aggcattcag 900
ccatcatgat gtggccttta cctccactcc tgtcttgttc tacccagatt cagcacagcc 960
ctttatagtg aagtcagagt cctcaagcca aatagctaaa gctgttttat cacaacaaag 1020
gcctagtttg ttccatgagt gtgcatttca tttcttcagt taaagccttc agagacacac 1080
aataaatttg gaccagggga ttttttagtt attaatgctc tctgaagaaa ggcaacatct 1140
ttttgagagc agcattggac cacaccccac aatctcaaat gattgaaatt catgaacatc 1200
taggatcccg tgaaggtcac tggaccctgt tttttctact tcaaatcctg tagtagccta 1260
ctgaatgaga aaacatattc tgacccattg ggatcaaatc aaaggcacag tgaactcctc 1320
atagcatctt ctttggaatt actcaggaac cagaactttt tacacaaatg taagaaattc 1380
taccaaggag tccccttacc taacagcatc tcacaaggct gcaccagatt ccagaaaagg 1440
cttctcttga tacatcaagc attttgtgac cgacttattc ttagatcatt ggttttccaa 1500
aggctttgtg gccatgaagc cctttgagtg aaaactgtgc agaagcccag agtaaaagtg 1560
aagctgctct ggatgaagta gtgaagcaag agtaggggcc tgaatcctgc tacaactatc 1620
ttcctttacc accgtggtga cacctaaggg gacttcctta caacaccttg aactcttccg 1680
aacacagttt gaaaaccact gccccagaca gcaatatgtt tgacctgaat ggcattccaa 1740
tcttttctgt acctccactc agcacagttc atgttcagta gatgctgaac attcttagaa 1800
atactgtgtg tgaacttaga aaagtgcaag aagacaggca tgtctttgac cccaggaatg 1860
atcatttgct gaagatggtg tcaagtgaac ctagattaac agccctccac tccagatgga 1920
tatccagtga ttcctagaat gggatatagc cagagaacaa ttctatgcac cctacactga 1980
cagactccct taagcaacac cagatgctct actggtactt gaagtacatg actttgaagt 2040
cttgaccctc catgaatacc tgaattatca gcaagcgggt tttgaagctg gtgcctcatt 2100
gaggccatat tagagcaact tgtacatttg acctcttgtt atcagccatg gtactctact 2160
tcgtgtgcaa gagataacta tgaaagccaa attcaaatac tggcaacatt tcctaaaggg 2220
gctcaatatc tatcattcgt cttcttttcc aaactacaca tcactgtatg actcaaccag 2280
tagcagttat attgcccctt ggtttttatt cagtttaact actgtttcca agataaatga 2340

gctaataagc tttaaaaaaa aaaaaaa 2368

<210> 103
<211> 2577
<212> DNA
<213> nm_003011.1 SET translocation (myeloid leukaemia-associated) Homo sapiens

<400> 103

cacatgtcgg cgcaggcggc caaagtcagt aaaaaggagc tcaactccaa ccacgacggg 60
gccgacgaga cctcagaaaa agaacagcaa gaagcgattg aacacattga tgaagtacaa 120
aatgaaatag acagacttaa tgaacaagcc agtgaggaga ttttgaaagt agaacagaaa 180
tataacaaac tccgccaacc atttttttcag aagaggtcag aattgatcgc caaaatccca 240
aatttttggg taacaacatt tgtcaaccat ccacaagtgt ctgcactgct tggggaggaa 300
gatgaagagg cactgcatta tttgaccaga gttgaagtga cagaatttga agatattaaa 360
tcaggttaca gaatagattt ttattttgat gaaaatcctt actttgaaaa taaagttctc 420
tccaaagaat ttcatctgaa tgagagtggt gatccatctt cgaagtccac cgaaatcaaa 480
tggaaatctg gaaaggattt gacgaaacgt tcgagtcaaa cgcagaataa agccagcagg 540
aagaggcagc atgaggaacc agagagcttc tttacctggt ttactgacca ttctgatgca 600
ggtgctgatg agttaggaga ggtcatcaaa gatgatattt ggccaaaccc attacagtac 660
tacttggttc ccgatatgga tgatgaagaa ggagaaggag aagaagatga tgatgatgat 720
gaagaggagg aaggattaga agatattgac gaagaagggg atgaggatga aggtgaagaa 780
gatgaagatg atgatgaagg ggaggaagga gaggaggatg aaggagaaga tgactaaata 840
gaacactgat ggattccaac cttccttttt ttaaattttc tccagtccct gggagcaagt 900
tgcagtcttt tttttttttt tttttttttt ccctcttgtg ctcagtcgcc ctgttcttga 960
ggtctctttt ctctactcca tggttctcaa tttatttggg gggaaatacc ttgagcagaa 1020
tacaatggga aaagagtctc taccccttc tgttcgaagt tcatttttat cccttcctgt 1080
ctgaacaaaa actgtatgga atcaacacca ccgagctctg tgggaaaaaa gaaaaacctg 1140
ctccctttgc tctgctggaa gctggagggt gctaggcccc tgtgtagtag tgtatagaat 1200
tctagctttt ttcctccttt ctctgtatat tgggctcaga gagtacactg tgtctctatg 1260
tgaatatgga cagttagcat ttaccaacat gtatctgtct actttctctt gtttaaaaaa 1320
agaaaaaaaa acttaaaaaa atggggttat agaaggtcag caaaggggtg gggtttgaga 1380
tgtttgggtg ggttagtggg cattttgaca acatggcttc tcctttggca tgtttaattg 1440
tgatatttga cagacatcct tgcagtttaa gatgacactt ttaaaataaa ttctctccta 1500
atgatgactt gagccctgcc actcaatggg agaatcagca gaacctgtag gatcttattt 1560

```
ggaattgaca ttctctattg taattttgtt cctgtttatt tttgggtttc tttttgtttc   1620
actggaaagg aaagatgatg ctcagtttta aacgttaaaa gtgtacaagt tgctttgtta   1680
caataaaact aaatgtgtac acaaaggatt tgatgctttt ctctcagcat aggtatgctt   1740
actatgacct tccaagtttg acttgtataa catcactgtc aaactttgtc accctaactt   1800
cgtatttttt gatacgcact tttgcaggat gacctcaggg ctatgtggat tgagtaatgg   1860
gatttgaatc aatgtattaa tatctccata gctgggaaac gtgggttcaa tttgccattg   1920
gtttctgaaa agtattcaca tcatttggga taccagatag ctcaatactc tctgagtaca   1980
ttgtgccctt gatttttatc tccaagtggc agtttttaaa attggccttt tacctggata   2040
taaattaatt gtgcctgcca ccaccatcca acagacctgg tgctctaatg ccaagttata   2100
cacgggacag ttgctggcat gtcttcattg gctctctaaa atgtggccaa gaagataggc   2160
tctcagtaag aagtctgatg gtgagcagta actgtccctg ctttctggta taaagctctc   2220
aaatgtgacc atgtgaatct gggtgggata atggactcag ctctgtctgc tcaatgccat   2280
tgtgcagaga agcaccctaa tgcataagct ttttaatgct gtaaaatata gtcgctgaaa   2340
ttaaatgcca ctttttcaga ggtgaattaa tggacagtct ggtgaacttc aaaagctttt   2400
tgatgtataa aacttgataa atggaactat tccatcaata ggcaaaagtg taacaaccta   2460
tctagatgga tagtatgtaa tttctgcaca ggtctctgtt tagtaaatac atcactgtat   2520
accgatcagg aatcttgctc caataaagga acataaagat ttaaaaaaaa aaaaaaa      2577
```

<210> 104
<211> 7577
<212> DNA
<213> XM_030577.9| PREDICTED: Homo sapiens ATPase, class II, type 9A (ATP9A), mRNA

<400> 104

```
atgacggaca acatcccgct gcagccggtg cgccagaaga agcggatgga cagcaggccc     60
cgcgccgggt gctgcgagtg gctgagatgc tgcggtggag gggaggccag gccccgcact    120
gtctggctgg ggcaccccga gaagagagac cagaggtatc ctcggaatgt catcaacaat    180
cagaagtaca atttcttcac ctttcttcct ggggtgctgt tcaaccagtt caaatacttt    240
ttcaacctct atttcttact tcttgcctgc tctcagtttg ttcccgaaat gagacttggt    300
gcactctata cctactgggt tcccctgggc ttcgtgctgg ccgtcactgt catccgtgag    360
gcggtggagg agatccgatg ctacgtgcgg gacaaggaag tcaactccca ggtctacagc    420
cggctcacag cacgaggcac agtgaaggtg aagagttcta acatccaagt tggagacctt    480
atcatcgttg aaaagaacca gcgggtccct gccgacatga tcttcctgag gacatcagaa    540
aaaaacgggt catgcttctt gcggacggat cagctggatg gggagacgga ctggaagctg    600
```

cggcttcccg tggcctgcac gcagaggctc cccacggccg ccgaccttct tcagattcga 660
tcgtatgtgt acgcagaaga gccaaatatt gacattcaca acttcgtggg aacttttacc 720
cgagaagaca gcgacccccc gatcagcgag agcctgagca tagagaacac gctgtgggct 780
ggcactgtgg tcgcatcagg tactgttgtg ggtgttgttc tttacactgg cagagaactc 840
cggagtgtca tgaatacctc aaatccccga agtaagatcg gcctgttcga cttggaagtg 900
aactgcctca ccaagatcct ctttggtgcc ctggtggtgg tctcgctggt catggttgcc 960
cttcagcact ttgcaggccg ttggtacctg cagatcatcc gcttcctcct cttgttttcc 1020
aacatcatcc ccattagttt gcgtgtgaac ctggacatgg gcaagatcgt gtacagctgg 1080
gtgattcgaa gggactcgaa aatccccggg accgtggttc gctccagcac gattcctgag 1140
cagctgggca ggatttcgta cttactcaca gacaagacag gcactcttac ccagaacgag 1200
atgattttca aacggctcca tctcggaaca gtagcctacg gcctcgactc aatggacgaa 1260
gtacaaagcc acattttcag catttacacc cagcaatccc aggacccacc ggctcagaag 1320
ggcccaacgc tcaccactaa ggtccggcgg accatgagca gccgcgtgca cgaagccgtg 1380
aaggccatcg cgctctgcca caacgtgact cccgtgtatg agtccaacgg tgtgactgat 1440
caggctgagg ccgagaagca gtacgaagac tcctgccgcg tataccaggc atccagcccc 1500
gatgaggtgg ccctggtaca gtggacggaa agtgtgggct taaccctggt gggccgagac 1560
cagtcttcca tgcagctgag gacccctggc gaccagatcc tgaacttcac catcctacag 1620
atcttccctt tcacctatga aagcaaacgt atgggcatca tcgtgcggga tgaatcaact 1680
ggagaaatta cgttttacat gaagggagca gatgtggtca tggctggcat tgtgcagtac 1740
aatgactggt tggaggaaga gtgtggcaac atggcccgag aagggctgcg ggtgctcgtg 1800
gtggcaaaga agtctcttgc agaggagcag tatcaggact ttgaagcccg ctacgtccag 1860
gccaagctga gtgtgcacga ccgctccctc aaagtggcca cggtgatcga gagcctggag 1920
atggagatgg aactgctgtg cctgacgggc gtggaggacc agctgcaggc agatgtgcgg 1980
cccacgctgg agaccctgag gaatgctggc atcaaggttt ggatgctgac aggggacaag 2040
ctggagacag ctacgtgcac agcgaagaat gcacatctgg tgaccagaaa ccaagacatc 2100
cacgtttttc ggctggtgac caaccgcggg gaggctcacc tcgagctgaa cgccttccgc 2160
aggaagcatg attgtgccct ggtcatctcg ggagactccc tggaggtttg cctcaagtac 2220
tatgagtacg agttcatgga gctggcctgc cagtgcccgg ccgtagtctg ctgccgatgt 2280
gcccccaccc agaaggccca gatcgtgcgc ctgcttcagg agcgcacggg caagctcacc 2340
tgtgcagtag gggacggagg caatgacgtc agcatgattc aggaatctga ctgcggcgtg 2400
ggagtggaag gaaaggaagg aaaacaggct tcgttggctg cagacttctc catcactcaa 2460
tttaagcatc ttggccggtt gcttatggtg catggccgga acagctacaa gcggtcagcc 2520
gccctcagcc agttcgtgat tcacaggagc ctctgtatca gcaccatgca ggctgtcttt 2580
tcctccgtgt tttactttgc ctccgtccct ctctatcaag gattcctcat cattgggtac 2640

```
tccacaattt acaccatgtt tcctgtgttt tctctggtcc tggacaaaga tgtcaaatcg   2700
gaagttgcca tgctgtatcc tgagctctac aaggatcttc tcaagggacg gccgttgtcc   2760
tacaagacat tcttaatatg ggttttgatt agcatctatc aagggagcac catcatgtac   2820
ggggcgctgc tgctgtttga gtcggagttc gtgcacatcg tggccatctc cttcacctcg   2880
ctgatcctca ccgagctgct catggtggcg ctgaccatcc agacctggca ctggctcatg   2940
acagtggcgg agctgctcag cctggcctgc tacatcgcct ccctggtgtt cttacacgag   3000
ttcatcgatg tgtacttcat cgccaccttg tcattcttgt ggaaagtctc cgtcatcact   3060
ctggtcagct gcctccccct ctatgtcctc aagtacctgc gaagacggtt ctctcccccc   3120
agctactcaa agctcacatc ataggccgtg cgttcgctgg agggggccct ggtcttggcg   3180
cttccctgat ggacagagct caagttccat ttatattaac cgccacctgt ggattttgca   3240
gtaattgcta acacatgcag ttttaatggg aagtggctct gcgcctaaac ggagtcctaa   3300
cgctgcatca acgggaggga gggtcctgaa agagacccat ctgggcctgt ctgaacccct   3360
cgttcttcat gtttaggtga atatgaatat gttaaagctg gtggctcagc tgggagattt   3420
atatgggtca ctgtgcgagc ttccttatga cttgaatttt gttgtcacat gataaaagtt   3480
tctgtgtagc tgaaggttgt agaaggcttg tgtgtgtgtg tgtgtgtgtg tgtgtgtgtg   3540
tgtgtgtttt taaagagtcc taatgtgtat gtactcttta tgtctttctt gctcttacaa   3600
agaggtgtca gaaaaataga aagctcttgg tgtcggtttg ggaggaaaag acagtgacat   3660
ttggtaaaaa gttatccaca caataatctc cattcggaaa tgctcagtat cgtctccagc   3720
cagccctgct tatccaggtt acactggatt cctgggatcg taaccagtaa atgagaggag   3780
agggagagag agtgtcctaa gtccaatctg ttatccttga tctgattcag catccatagt   3840
gtgtgagtta acttcacctg ccacctcgta aaagaatttc agaggtgtga tcccgcttta   3900
ttgggacctg gtaacaatca caaagccagt ggctgtttga gaaggacctc agacattttc   3960
agcagagttg ttttagcagg aaacgtgcca ctgaatggcc cctaaatgtg tcgacagtgt   4020
gataagagac tcaactaatt ctttaggcaa catggcagat gtgactcaga tcctccaaga   4080
ccaaagcgga aaggtcaggg ggctgggact cttctcttcc atagaagcct gtttcctgtt   4140
aggaggcata atggaagatg accccacaaa ggcagaggca tctttcggaa caacactggt   4200
ggcagctttc agaacaagga acccctggtg ggaggacgcc caagctacag cgttgggatc   4260
tgggatctgt tccactgccg gcagatttca aggggaactt gctgaaaggc agccagtggt   4320
gaagatttct cccctcccag gatggactac atgccggcat gtttcttata aagctgtggc   4380
tgcttgtttc agaggaaggg agtttgcagt cgcgggacgt ggtagagcaa ggcattcttg   4440
ggttttcaag ttgcttcttg cagaagccac atatgcatgc cataagggtt aagttggtgg   4500
atctttaaga gccaagtgtg gttgagatct tggatttgcg tttacttctt gatgaataca   4560
tatccttcaa accctctgcc tggcgctact tctgtgtgct tcagagatgt acatcacagc   4620
```

ctggtttctg atgcctacta actcctgctc ttggagagct ggagacacga ggatcagata 4680
gtcccttgcc tttggagcac tcttgataag cttttgtatt ttgtgttgtc cttttaaaat 4740
gttctagaat gactttacgt tgcaggtact ggttaattgg ctgttgacac cacatctatt 4800
ttgtcttatg attctgcagt tttgcagtac ttttctctat ctgattcagc catttctgcc 4860
agagggaaaa ggtcggcaga aaagatgtat tgagtgaata gttaaggata ggatctttgt 4920
ccaaaaattt cagaaagatt gagcaaatct gacgtattca ttgagtgagt ttctgtgttt 4980
tcaaaggtgg aggagaaatt tgtgctggaa gtttttaagc ctccgttttc ttggaaatca 5040
gtctgtaaca ctggcaagtc ttaagatagt cccatttaga ctttgcagat gctgaacctg 5100
gctctgtaac gctgggaagt cttaagatag tcctgtttag actttgcaaa ccctgtacct 5160
ggctttgctc ggagattcgg gatgctggct cctgcaggca gggcgtgtgg gagcctcgtc 5220
agaaagtttt agaggtttcc agcagaagca gaatgaagat ggtctccctg gccttttcct 5280
taattctcaa ttttgattga ggtgcacaag ttgactttta aagccaacgc ttaagatact 5340
gattgacatc ttcaagggag aatgctccca ggaggggctg aagaagccat agttggaagt 5400
ggaaggtact cgtcagtgtt ctccacaaac ctttttactc tgttgtctca gccgcactgg 5460
ggcggaggcg gtcaagggtg agaagtaccg acactcaagt gcaaactgcc acgtcgttgg 5520
cccatcccat cagtgggcag ctggctgacg ccattcactg gacggtccct gaacacctag 5580
gaatgcacac accgtgcttc tcagacactg gagacgcaaa ggcaggagga tgcagtccgg 5640
tgagaggaca cgatctttac ctgcacaatc agactgtaag cccagcagag aaccccaggg 5700
gcgcctgggt acttctcgga ggtcatctta gttgtggtgg ggaagacaaa gaaataagca 5760
aacaagaaac tagagttact atacaagaaa ctctcctgag tttgtaaacc ttaagcataa 5820
ggattcagtt gaccttttc ttggttcatc aatctggaaa gaacttacat aaagcgccat 5880
tgacactgtc acctgggagc tccatgggcc gtaagtcttt gacagccaat ttaatttgag 5940
gtcagagggc cttgaggtac acagtcagca ctgtttgaac acttttcctg aaagcaaaac 6000
tcacagctcc ctgcgccctc tgacaacact agctatttct gccagagtaa gaacttctat 6060
tactatttta ttattgttca tatgtctttt gatgatggtt gtgtgacagg gggaagcagg 6120
atctatttgg tttcttcccc tccccccacc ccttcctttt tgtctctctt tttttttctc 6180
taagaaaatc accagactag tttttccatc ttgagtaatt tcttatgtgg gacagttttg 6240
atcctcattt tgaaagcatg cgtgtgcaca tgtgtgttgc ctgtggtgcc aggtgagaca 6300
ggtggcacta actccagctg cttggaaggc atcccaaggg cgcatcttaa agttggagca 6360
gacctccctt ttccagcccc tggggccatt agaccacgtg ctggaactag cattgtaaaa 6420
ttcccatccc agttccactc ccctgaagtg aaacccttttt tttttgtga cagtaaatct 6480
taaaaatcat tgtctcttta tgaacatttc ctcagtttct tctctgctga aaatgtaagc 6540
catgctactt tttaatgtat tttgaatttt gtgctcattg gaaattgata tgctaatgcc 6600
tcccccaccc cccgccagac ttttcttttt atactttgtc ttgtttttac tggggtaggc 6660

tgggcatgcg tgcgtgcctt tagggcagca ttttaaacct ttgccaaaat tgcaaatggg 6720
acatgtacat tcttctgctc catcctactt aaacacctat cagctatttt tatctttaac 6780
cttttctgta tgtttgaagt gtgtgggggg tgtgtgtgtg tgtgaaagag cgagagaatg 6840
atgtcatcta aagtttttg aagaattatt tggttttcat tgcattaaaa ttctatcact 6900
cccagctttg ttttcattta aaaaaatata caaagagctt tgtaaataca acacatttta 6960
tttctccccc ttcttttaat gtacagcttt tttgccactt atatatactt aaaatattcc 7020
catgaattat gtccagttct tcttggaaaa aaatttggtt ttgaatgaac ctgcaaagca 7080
tcctgcagcg tgagcagctc ctccacctgg agctccgaag catcttctca ggccaaagcg 7140
gcattacccg tgaatctgtc ttctccgcca cagcatggtt tgaggcgcag tctgttaata 7200
tagctgggcc atgtcagtga ctgttgtgtt tgtggggtca ggtgggggc atggtatttg 7260
caaaaaaac aaattatggc taatttatta ttttgttgca gtggggttaa ctgtaaactc 7320
atgtaagagt ctgtgatttc ctcattggtt gatctctctc tctgtaatcc tcattgcaaa 7380
ttttcaccag gacagcgttt tttgattaga ggggagctct ggcacagtat gctttaattt 7440
agcaggaact tccagatgat ttaaattctc gatgctgtga tgacacacat atgatctttc 7500
gtgtttctga gcgactctac tttcattgtt tgccagcgtg gctcgttgct gttgcccaat 7560
aaagcttgtg tacgttc 7577

<210> 105
<211> 1672
<212> DNA
<213> NM_004503.2| Homo sapiens homeo box C6 (HOXC6), transcript variant 1, mRNA

<400> 105

ttttgtctgt cctggattgg agccgtccct ataaccatct agttccgagt acaaactgga 60
gacagaaata aatattaaag aaatcataga ccgaccaggt aaaggcaaag ggatgaattc 120
ctacttcact aacccttcct tatcctgcca cctcgccggg ggccaggacg tcctccccaa 180
cgtcgccctc aattccaccg cctatgatcc agtgaggcat ttctcgacct atggagcggc 240
cgttgcccag aaccggatct actcgactcc cttttattcg ccacaggaga atgtcgtgtt 300
cagttccagc cgggggccgt atgactatgg atctaattcc ttttaccagg agaaagacat 360
gctctcaaac tgcagacaaa acaccttagg acataacaca cagacctcaa tcgctcagga 420
ttttagttct gagcagggca ggactgcgcc ccaggaccag aaagccagta tccagattta 480
cccctggatg cagcgaatga attcgcacag tggggtcggc tacggagcgg accggaggcg 540
cggccgccag atctactcgc ggtaccagac cctggaactg gagaaggaat ttcacttcaa 600
tcgctaccta acgcggcgcc ggcgcatcga gatcgccaac gcgctttgcc tgaccgagcg 660

acagatcaaa atctggttcc agaaccgccg gatgaagtgg aaaaaagaat ctaatctcac 720
atccactctc tcggggggcg gcggaggggc caccgccgac agcctgggcg gaaaagagga 780
aaagcgggaa gagacagaag aggagaagca gaaagagtga ccaggactgt ccctgccacc 840
cctctctccc tttctccctc gctccccacc aactctcccc taatcacaca ctctgtattt 900
atcactggca caattgatgt gttttgattc cctaaaacaa aattagggag tcaaacgtgg 960
acctgaaagt cagctctgga ccccctccct caccgcacaa ctctctttca ccacgcgcct 1020
cctcctcctc gctcccttgc tagctcgttc tcggcttgtc tacaggccct tttccccgtc 1080
caggccttgg gggctcggac cctgaactca gactctacag attgccctcc aagtgaggac 1140
ttggctcccc cactccttcg acgcccccac ccccgccccc cgtgcagaga gccggctcct 1200
gggcctgctg gggcctctgc tccagggcct cagggcccgg cctggcagcc ggggagggcc 1260
ggaggcccaa ggagggcgcg ccttggcccc acaccaaccc ccagggcctc cccgcagtcc 1320
ctgcctagcc cctctgcccc agcaaatgcc cagcccaggc aaattgtatt taaagaatcc 1380
tgggggtcat tatggcattt tacaaactgt gaccgtttct gtgtgaagat ttttagctgt 1440
atttgtggtc tctgtattta tatttatgtt tagcaccgtc agtgttccta tccaatttca 1500
aaaaaggaaa aaaaagaggg aaaattacaa aaagagagaa aaaaagtgaa tgacgtttgt 1560
ttagccagta ggagaaaata aataaataaa taaatccctt cgtgttaccc tcctgtataa 1620
atccaacctc tgggtccgtt ctcgaatatt taataaaact gatattattt tt 1672

<210> 106
<211> 3394
<212> DNA
<213> NM_004764.2| Homo sapiens piwi-like 1 (Drosophila) (PIWIL1), mRNA

<400> 106

gttggcctcg ggctgaggtg caaggaccag gactagggcg agggcagcgg tccaagaaat 60
agaaaacaat gactgggaga gcccgagcca gagccagagg aagggcccgc ggtcaggaga 120
cagcgcagct ggtgggctcc actgccagtc agcaacctgg ttatattcag cctaggcctc 180
agccgccacc agcagagggg gaattatttg gccgtggacg gcagagagga acagcaggag 240
gaacagccaa gtcacaagga ctccagatat ctgctggatt tcaggagtta tcgttagcag 300
agagaggagg tcgtcgtaga gattttcatg atcttggtgt gaatacaagg cagaacctag 360
accatgttaa agaatcaaaa acaggttctt caggcattat agtaaggtta agcactaacc 420
atttccggct gacatcccgt ccccagtggg ccttatatca gtatcacatt gactataacc 480
cactgatgga agccagaaga ctccgttcag ctcttctttt tcaacacgaa gatctaattg 540
gaaagtgtca tgcttttgat ggaacgatat tatttttacc taaaagacta cagcaaaagg 600

ttactgaagt ttttagtaag acccggaatg gagaggatgt gaggataacg atcactttaa 660
caaatgaact tccacctaca tcaccaactt gtttgcagtt ctataatatt attttcagga 720
ggcttttgaa aatcatgaat ttgcaacaaa ttggacgaaa ttattataac ccaaatgacc 780
caattgatat tccaagtcac aggttggtga tttggcctgg cttcactact tccatccttc 840
agtatgaaaa cagcatcatg ctctgcactg acgttagcca taaagtcctt cgaagtgaga 900
ctgttttgga tttcatgttc aacttttatc atcagacaga agaacataaa tttcaagaac 960
aagtttccaa agaactaata ggtttagttg ttcttaccaa gtataacaat aagacataca 1020
gagtggatga tattgactgg gaccagaatc ccaagagcac ctttaagaaa gccgacggct 1080
ctgaagtcag cttcttagaa tactacagga agcaatacaa ccaagagatc accgacttga 1140
agcagcctgt cttggtcagc cagcccaaga gaaggcgggg ccctgggggg acactgccag 1200
ggcctgccat gctcattcct gagctctgct atcttacagg tctaactgat aaaatgcgta 1260
atgattttaa cgtgatgaaa gacttagccg ttcatacaag actaactcca gagcaaaggc 1320
agcgtgaagt gggacgactc attgattaca ttcataaaaa cgataatgtt caaagggagc 1380
ttcgagactg ggggtttgagc tttgattcca acttactgtc cttctcagga agaattttgc 1440
aaacagaaaa gattcaccaa ggtggaaaaa catttgatta caatccacaa tttgcagatt 1500
ggtccaaaga aacaagaggt gcaccattaa ttagtgttaa tccactagat aactggctgt 1560
tgatctatac gcgaagaaat tatgaagcag ccaattcatt gatacaaaat ctatttaaag 1620
ttacaccagc catgggcatg caaatgaaaa aagcaataat gattgaagtg gatgacagaa 1680
ctgaagccta cttaagagtc ttacagcaaa aggtcacagc agacacccag atagttgtct 1740
gtctgttgtc aagtaatcgg aaggacaaat acgatgctat taaaaaatac ccgtgtacag 1800
attgccctac cccaagtcag tgtgtggtgg cccgaacctt aggcaaacag caaactgtca 1860
tggccattgc tacaaagatt gccctacaga tgaactgcaa gatgggagga gagctctgga 1920
gggtggacat ccccctgaag ctcgtgatga tcgttggcat cgattgttac catgacatga 1980
cagctgggcg gaggtcaatc gcaggatttg ttgccagcat caatgaaggg atgacccgct 2040
ggttctcacg ctgcatattt caggatagag gacaggagct ggtagatggg ctcaaagtct 2100
gcctgcaagc ggctctgagg gcttggaata gctgcaatga gtacatgccc agccggatca 2160
tcgtgtaccg cgatggcgta ggagacggcc agctgaaaac actggtgaac tacgaagtgc 2220
cacagttttt ggattgtcta aaatccattg gtagaggtta caaccctaga ctaacggtaa 2280
ttgtggtgaa gaaaagagtg aacaccagat tttttgctca gtctggagga agacttcaga 2340
atccacttcc tggaacagtt attgatgtag aggttaccag accagaatgg tatgactttt 2400
ttatcgtgag ccaggctgtg agaagtggta gtgtttctcc cacacattac aatgtcatct 2460
atgacaacag cggcctgaag ccagaccaca tacagcgctt gacctacaag ctgtgccaca 2520
tctattacaa ctggccaggt gtcattcgtg ttcctgctcc ttgccagtac gcccacaagc 2580
tggcttttct tgttggccag agtattcaca gagagccaaa tctgtcactg tcaaaccgcc 2640

```
tttactacct ctaacctgca gaagacgatg cagccgcttt tctttttgaa atgactttgg   2700
gatttttta agcttttatt tactttttt ttaactgtta tctttctgga tgaaacttgg   2760
gaaggggatt aggagatcta gcattttatt tctagcattg ctattcaccg gcttccttat   2820
tttatacgta aaaattaaga ttttatattt tatcttcttg tttctcatag atattttgtg   2880
agcatttttt tgtttatttt gaagaaatgt ggataagata cttggtagta taaaacagac   2940
tctctgagag tatttgaaat gtgtttggag atttacttaa acgtactttc aggagtgagc   3000
aagtcctact tataaaccta tattaacttt atttttgaga tacctgtttt gaatttaaag   3060
gagataagag gcgtaaagta ggatgctcac tacaaccata ggtggggttt cagctcatat   3120
cttaaagata aaaggtacta ttatataacc tatacacaag atacaggaga aaatatgctt   3180
gatttttatt tggcaggggg gctaggttgt atgggagtaa aaaaaacatt gaaaattttt   3240
aaattgtcca aagaaacatt ttaagactct ttaacaaaaa aggccatgag taaatctcta   3300
tattaacatt actatttatt ttgttttgga actgggacat gattctattt gttataaaat   3360
aaaattgatg tgattgtcaa aaaaaaaaaa aaaa                              3394
```

<210> 107
<211> 2524
<212> DNA
<213> NM_000249.2| Homo sapiens mutL homolog 1, colon cancer, nonpolyposis type 2 (E. coli) (MLH1), mRNA

<400> 107

```
attggctgaa ggcacttccg ttgagcatct agacgtttcc ttggctcttc tggcgccaaa     60
atgtcgttcg tggcaggggt tattcggcgg ctggacgaga cagtggtgaa ccgcatcgcg    120
gcgggggaag ttatccagcg gccagctaat gctatcaaag agatgattga gaactgttta    180
gatgcaaaat ccacaagtat tcaagtgatt gttaaagagg gaggcctgaa gttgattcag    240
atccaagaca atggcaccgg gatcaggaaa gaagatctgg atattgtatg tgaaaggttc    300
actactagta aactgcagtc ctttgaggat ttagccagta tttctaccta tggctttcga    360
ggtgaggctt tggccagcat aagccatgtg gctcatgtta ctattacaac gaaaacagct    420
gatggaaagt gtgcatacag agcaagttac tcagatggaa aactgaaagc ccctcctaaa    480
ccatgtgctg gcaatcaagg gacccagatc acggtggagg acctttttta caacatagcc    540
acgaggagaa aagctttaaa aaatccaagt gaagaatatg ggaaaatttt ggaagttgtt    600
ggcaggtatt cagtacacaa tgcaggcatt agtttctcag ttaaaaaaca aggagagaca    660
gtagctgatg ttaggacact acccaatgcc tcaaccgtgg acaatattcg ctccatcttt    720
ggaaatgctg ttagtcgaga actgatagaa attggatgtg aggataaaac cctagccttc    780
aaaatgaatg gttacatatc caatgcaaac tactcagtga agaagtgcat cttcttactc    840
```

ttcatcaacc atcgtctggt agaatcaact tccttgagaa aagccataga aacagtgtat 900
gcagcctatt tgcccaaaaa cacacaccca ttcctgtacc tcagtttaga aatcagtccc 960
cagaatgtgg atgttaatgt gcaccccaca aagcatgaag ttcacttcct gcacgaggag 1020
agcatcctgg agcgggtgca gcagcacatc gagagcaagc tcctgggctc caattcctcc 1080
aggatgtact tcacccagac tttgctacca ggacttgctg gcccctctgg ggagatggtt 1140
aaatccacaa caagtctgac ctcgtcttct acttctggaa gtagtgataa ggtctatgcc 1200
caccagatgg ttcgtacaga ttcccgggaa cagaagcttg atgcatttct gcagcctctg 1260
agcaaacccc tgtccagtca gccccaggcc attgtcacag aggataagac agatatttct 1320
agtggcaggg ctaggcagca agatgaggag atgcttgaac tcccagcccc tgctgaagtg 1380
gctgccaaaa atcagagctt ggagggggat acaacaaagg ggacttcaga aatgtcagag 1440
aagagaggac ctacttccag caaccccaga aagagacatc gggaagattc tgatgtggaa 1500
atggtggaag atgattcccg aaaggaaatg actgcagctt gtaccccccg gagaaggatc 1560
attaacctca ctagtgtttt gagtctccag gaagaaatta atgagcaggg acatgaggtt 1620
ctccgggaga tgttgcataa ccactccttc gtgggctgtg tgaatcctca gtgggccttg 1680
gcacagcatc aaaccaagtt ataccttctc aacaccacca agcttagtga agaactgttc 1740
taccagatac tcatttatga ttttgccaat tttggtgttc tcaggttatc ggagccagca 1800
ccgctctttg accttgccat gcttgcctta gatagtccag agagtggctg gacagaggaa 1860
gatggtccca aagaaggact tgctgaatac attgttgagt ttctgaagaa gaaggctgag 1920
atgcttgcag actatttctc tttggaaatt gatgaggaag ggaacctgat tggattaccc 1980
cttctgattg acaactatgt gccccctttg gagggactgc ctatcttcat tcttcgacta 2040
gccactgagg tgaattggga cgaagaaaag gaatgttttg aaagcctcag taaagaatgc 2100
gctatgttct attccatccg gaagcagtac atatctgagg agtcgaccct ctcaggccag 2160
cagagtgaag tgcctggctc cattccaaac tcctggaagt ggactgtgga acacattgtc 2220
tataaagcct tgcgctcaca cattctgcct cctaaacatt tcacagaaga tggaaatatc 2280
ctgcagcttg ctaacctgcc tgatctatac aaagtctttg agaggtgtta aatatggtta 2340
tttatgcact gtgggatgtg ttcttctttc tctgtattcc gatacaaagt gttgtatcaa 2400
agtgtgatat acaaagtgta ccaacataag tgttggtagc acttaagact tatacttgcc 2460
ttctgatagt attcctttat acacagtgga ttgattataa ataaatagat gtgtcttaac 2520
ataa 2524

<210> 108
<211> 2928
<212> DNA
<213> NM_001313.2| Homo sapiens collapsin response mediator protein 1 (CRMP1), mRNA

<400> 108

```
ccgatccggg cggtgctggc agccggagcg gcggcgggcg ggccgagcag ccggggcagc     60
cgcgcgtggg catccacggg cgccgagcct ccgtccgtgt ctctatccct cccgggcctt    120
tgtcagcgcg cccgctggga gcggggccga gagcgccggt tccagtcaga cagccccgca    180
ggtcagcggc cgggccgagg gcgccagagg gggccatgtc gtaccagggc aagaagagca    240
tcccgcacat cacgagtgac cgactcctca tcaaaggtgg acggatcatc aacgatgacc    300
aatcccttta tgctgacgtc tacctggagg atggacttat caaacaaata ggagagaact    360
taatcgttcc tggtggagtg aagaccattg aagccaacgg gcggatggtt attcccggag    420
gtattgatgt caacacgtac ctgcagaagc cctcccaggg gatgactgcg gctgatgact    480
tcttccaagg gaccagggcg gcactggtgg gcgggaccac gatgatcatt gaccatgttg    540
ttcctgaacc tgggtccagc ctactgacct ctttcgagaa gtggcacgaa gcagctgaca    600
ccaaatcctg ctgtgattac tccctccacg tggacatcac aagctggtac gatggcgttc    660
gggaggagct ggaggtgctg gtgcaggaca aaggcgtcaa ttccttccaa gtctacatgg    720
cctataagga tgtctaccaa atgtccgaca gccagctcta tgaagccttt accttcctta    780
agggcctggg agctgtgatc ttggtccatg cagaaaatgg agatttgata gctcaggaac    840
aaaagcggat cctggagatg ggcatcacgg gtcccgaggg ccatgccctg agcagacctg    900
aagagctgga ggccgaggcg gtgttccggg ccatcaccat tgcgggccgg atcaactgcc    960
ctgtgtacat caccaaggtc atgagcaaga gtgcagccga catcatcgct ctggccagga   1020
agaaagggcc cctagttttt ggagagccca ttgccgccag cctggggacc gatggcaccc   1080
attactggag caagaactgg gccaaggctg cggcgttcgt gacttcccct cccctgagcc   1140
cggaccctac cacgcccgac tacttgacct ccctactggc ctgtggggac ttgcaggtca   1200
caggcagcgg ccactgtccc tacagcactg cccagaaggc ggtgggcaag gacaacttta   1260
ccctgatccc cgagggtgtc aacgggatag aggagcggat gacggtcgtc tgggacaagg   1320
cggtggctac tggcaaaatg gatgagaacc agtttgtcgc tgtcaccagc accaatgcag   1380
ccaagatctt taacctgtac ccaaggaaag ggcggattgc cgtgggctcg gatgccgacg   1440
tggtcatctg ggaccccgac aagttgaaga ccataacagc caaaagtcac aagtcggcgg   1500
tggagtacaa catcttcgag ggtatggagt gccacggctc cccactagtg gtcatcagcc   1560
agggcaagat cgtctttgaa gacggaaaca tcaacgtcaa caagggcatg ggccgcttca   1620
ttccgcggaa ggcgttcccg gagcacctgt accagcgcgt caaaatcagg aataaggttt   1680
ttggattgca aggggtttcc aggggcatgt atgacggtcc tgtgtacgag gtaccagcta   1740
cacccaaata tgcaactccc gctccttcag ccaaatcttc gccttctaaa caccagcccc   1800
cacccatcag aaacctccac cagtccaact tcagcttatc aggtgcccag atagatgaca   1860
```

acaatcccag gcgcaccggc caccgcatcg tggcgccccc tggtggccgc tccaacatca 1920
ccagcctcgg ttgaacgtgg atgcgcggag gagctagcct gaaggattct gggaatcatg 1980
tccatccctt ttcctgtcag tgtttttgaa acccacagtt ttagttggtg ctgatggagg 2040
gagggggaag tcgaaggatg ctctttccct tttctgttta ggaagaagtg gtactagtgt 2100
ggtgtgtttg cttggaaatt ccttgcccca cagttgtgtt catgctgaat ccacctcgga 2160
gcatggtgtt ttcattcccc cttcctagtg aaccacaggt tttagcattg tcttgttctg 2220
tcccttccac ttctaactcc actggctcca tgattctctg agtggtggtt cctttgcacc 2280
ctgtagatgt tctaggatag ttgatgcatg ttactaaatt acgtatgcaa gtctgtgagt 2340
gcgtctgagg ggacatcgcc aaggactgac tgagacacga tgccgagacc tcaagccctg 2400
aggggcagtc ccaaaaccct tacagtgaag atgtttactc attgccccca cctctggtcc 2460
acactagaaa gaagctcgcc ccacctccac ctgtgagatc cgtgaattct cggaatggca 2520
ggggaagcct tgcactaggt tgcagagaag catcctccac atcctgtgtc agaaaccctg 2580
gtctccgtgg cacttgtaac tcaccgtgct gtcttctggt ctgtgtgtgt tcttcaagcc 2640
agctctaggc ttcaggccga gccaggttca cactcagaaa gaggtctccc catccccatt 2700
cggggctgac gatgggggc tgatggctgc ccctgcgtgg cctgagtcct ggtccctctg 2760
aggcagttga cggggcagtc agatttttaa agttttgtac aaagttttcc tttgtaatca 2820
ctcccatttt tacttaacaa ccaacttgtt gtggctctta tttctgaatt caaagcttgt 2880
gaaaaaataa agaaaatgaa ctgcccactg aaaaaaaaaa aaaaaaa 2928

<210> 109
<211> 1609
<212> DNA
<213> NM_002145.2| Homo sapiens homeo box B2 (HOXB2), mRNA

<400> 109

atctccccct cccaaaatcg ctccattaca taaatcgggg ggggtgcagg aggggggtc 60
ccttccgatc ctccctcctg acgccccccc cagcagcccc ctcccccacc attgaaagcc 120
atgaattttg aatttgagag ggagattggg tttataaaca gccagccgtc gctcgccgag 180
tgtctgactt ccttccccgc tgtcttggag acatttcaaa cttcatcaat caaggagtcg 240
acattaattc ctcctcctcc tcctttcgag caaaccttcc ccagcctcca gcccggcgcc 300
tccacccttc agagacccag gagccaaaag cgagccgaag atgggcctgc tctgccgccg 360
ccaccgccgc cgccactccc cgctgccccc ccggcccccg agttcccttg gatgaaagag 420
aagaaatccg ccaagaaacc cagccaatcc gccacgtctc cttctccggc cgcctccgcc 480
gttccggcct ccggggtcgg atcgcctgca gatggcctgg gactgccgga ggctggtggc 540

ggcggggcgc gcaggctgcg cacggcttac accaacacgc agctgctgga actggagaag 600
gaattccact ttaataagta cctgtgccgg ccacgccgcg tcgagatcgc ggccttgctg 660
gacctcaccg aaaggcaggt caaagtctgg tttcagaacc ggcgcatgaa gcacaagcgg 720
cagacgcagc accgagagcc gccggatggg gagcctgcct gcccgggagc cctggaggac 780
atctgcgacc ctgccgagga acccgcggcc agcccgggcg gcccctccgc ctcgcgggcg 840
gcgtgggaag cctgctgtca cccgccggag gtggtgccgg gggccttaag cgcggacccc 900
cggcctttag ccgttcgctt agagggcgca ggcgcgtcga gtcccggctg cgcgctgcgc 960
ggggccggcg ggctggagcc cgggccattg ccagaagacg tcttctcggg gcgccaggat 1020
tcacctttcc ttcccgacct caacttcttc gcggccgact cctgtctcca gctatccgga 1080
ggcctctccc ctagcctaca gggttctctc gacagcccgg tccctttttc cgaggaagag 1140
ctggattttt tcaccagtac gctctgtgcc atcgacctgc agtttcccta acctgtttcc 1200
tcctcccggt cctttcgacc cccgcgctcc ttggccgtct actggaaaaa tcgagcctct 1260
cccaccctca gtcgcataga cttatgtgtt ttgctaaaat tcaggtatta ctgaattagc 1320
gtttaatcca cttcctttct tcttcttcta aaatattggg cactcggtta tcttttaaaa 1380
ttcacacaga aaaattccgt ttggtagact ccttccaatg aaatctcagg aataattaaa 1440
ctctaggggg actttcttaa aaataactag agggacctat tttcctcttt tttatgtttt 1500
agactgtaga ttatttatta aaattcttta ataataggaa aaggggaaag tatttattgt 1560
acattatttt catagattaa ataaatgtct ttataatacc aaaaaaaaa 1609

<210> 110
<211> 3262
<212> DNA
<213> NM_002860.2| Homo sapiens aldehyde dehydrogenase 18 family, member A1 (PYCS/ALDH18A1), mRNA

<400> 110

aggggcgaa ggcggcggtg gtgaggaaga tactttggtt agtgaccaca tcgcagcatg 60
ttgagtcaag tttaccgctg tgggttccag cccttcaacc aacatcttct gccctgggtc 120
aagtgtacaa ccgtcttcag atctcattgt atccagcctt cagtcatcag acatgttcgt 180
tcttggagca acatcccgtt tatcactgta cccctcagtc gtacacatgg aaagtccttc 240
gcccaccgca gtgagctgaa gcatgccaag agaatcgtgg tgaagctcgg cagtgccgtg 300
gtgacccgag gggatgaatg tggcctggcc ctggggcgct tggcatctat tgttgagcag 360
gtatcagtgc tgcagaatca gggcagagag atgatgctgg tgaccagtgg agccgtagcc 420
tttggcaaac aacgcttgcg ccatgagatc cttctgtctc agagcgtgcg gcaggccctc 480
cactcggggc agaaccagct gaaagaaatg gcaattccag tcttagaggc acgagcctgt 540

gcagctgccg gacagagtgg gctgatggcc ttgtatgagg ctatgtttac ccagtacagc 600
atctgtgctg cccagatttt ggtgaccaat ttggatttcc atgatgagca gaagcgccgg 660
aacctcaatg gaacacttca tgaactcctt agaatgaaca ttgtccccat tgtcaacaca 720
aatgatgctg ttgtccccc agctgagccc aacagtgacc tgcagggggt aaatgttatt 780
agtgttaaag ataatgatag cctggctgcc cgactggctg tggaaatgaa aactgatctc 840
ttgattgttc tttcagatgt agaaggcctt tttgacagcc ccccaggttc agatgatgca 900
aagcttattg atatatttta tcccggagat cagcagtctg tgacatttgg aaccaagtct 960
agagtgggaa tgggtggcat ggaagccaag gtgaaagcag ccctctgggc tttgcaaggt 1020
ggcacttctg ttgttattgc caatggaacc cacccaaagg tgtctgggca cgtcatcaca 1080
gacattgtgg aggggaagaa agttggtacc ttcttttcag aagtaaagcc tgcaggccct 1140
actgttgagc agcagggaga aatggcgcga tctggaggaa ggatgttggc caccttggaa 1200
cctgagcaga gagcagaaat tatccatcat ctggctgatc tgttgacgga ccagcgtgat 1260
gagatcctgt tagccaacaa aaaagacttg gaggaggcag aggggagact tgcagctcct 1320
ctgctgaaac gtttaagcct ctccacatcc aaattgaaca gcctggccat cggtctgcga 1380
cagatcgcag cctcctccca ggacagcgtg ggacgtgttt tgcgccgcac ccgaatcgcc 1440
aaaaacttgg aactggaaca agtgactgtc ccaattggag ttctgctggt gatctttgaa 1500
tctcgtcctg actgtctacc ccaggtggca gctttggcta tcgcaagtgg caatggcttg 1560
ttactcaaag gagggaagga ggctgcacac agcaaccgga ttctccacct cctgacccag 1620
gaggctctct caatccatgg agtcaaggag gccgtgcaac tggtgaatac cagagaagaa 1680
gttgaagatc tttgccgcct agacaaaatg atagatctga tcattccacg tggctcttcc 1740
cagctggtca gagacatcca gaaagctgct aaggggattc cagtgatggg gcacagcgaa 1800
gggatctgtc acatgtatgt ggattccgag gccagtgttg ataaggtcac caggctagtc 1860
agagactcta aatgtgaata tccagctgcc tgtaatgctt tggagacttt gttaatccac 1920
cgggatctgc tcaggacacc attatttgac cagatcattg atatgctgag agtggaacag 1980
gtaaaaattc atgcaggccc caaatttgcc tcctatctga ccttcagccc ctccgaagtg 2040
aagtcactcc gaactgagta tggggacctg gaattatgca ttgaagtagt ggacaacgtt 2100
caggatgcca ttgaccacat ccacaagtat ggcagctccc acacggatgt catcgtcaca 2160
gaggacgaaa acacagcgga gttcttcctg cagcacgtag acagtgcctg tgtgttctgg 2220
aatgccagca ctcgcttttc tgatggttac cgctttggac tgggagctga agtgggaatc 2280
agtacatcga gaatccacgc ccggggacca gtaggacttg agggactgct tactactaag 2340
tggctgctgc gagggaagga ccacgtggtc tcagatttct cagagcatgg aagtttaaaa 2400
tatcttcatg agaacctccc tattcctcag agaaacacca actgaaaaga gccaggaaaa 2460
cccgggaatt ttccaaaagg tcttcacgtt aaacttgtct tatctcagga gagagcccgc 2520
tcttgtctcc cagttcctgg tagggtctgc ctgttggaaa gtgtacctgg atgcttctgg 2580

gctccgtttg gcaatagcaa tcttggctga tgtgcacagt ctggctccca gctcaccctt 2640
ttttttaaa gtaagaaaat agttgctacc gatagggact ttgccaagtc caattatctt 2700
ctaggattga aaggtgcatt ttccccataa aaaaggcgag gaaaacccat ggctgctttg 2760
tgtcacctca gtgacttaca gtccccttc gcatttagtt ggtactagag ccagtcatcc 2820
ttaacaaatc ttttcgcgtt ttatttcttt cacatgtagt catcttcaaa aaggaaagat 2880
ttggaatttt agaaaagggg caactcttct ttttagcatt ctcatcagaa agtcacaaaa 2940
atcgatggaa tcatttccac tgggaagatt gaccttttgt atttatttgt ggggtaaatt 3000
aataagcatt ccagatgctt gcagcttcct gcatccagga gatgctgtgt cccccgtgat 3060
gcagctggaa cccaagctgc agcaggagat gcaagtttca ggatgttccc cactgagctg 3120
gaggaatatc tacagcagtg atgcttgaaa tttttgtatg aattattttg tcgtcctacc 3180
cttttcctcc aaaacaaaaa ttagaggatt attttaatac tttggattct tccccctttt 3240
ttgagaaata aagtttttta tg 3262

<210> 111
<211> 2899
<212> DNA
<213> NM_005655.1| Homo sapiens TGFB inducible early growth response (TIEG), mRNA

<400> 111

cagacggcgc tgagcgcggc ggcggcggga gcggcgtcga gtgtctccgt gcgcccgtct 60
gtggccaagc agccagcagc ctagcagcca gtcagcttgc cgccggcggc caagcagcca 120
accatgctca acttcggtgc ctctctccag cagactgcgg aggaaagaat ggaaatgatt 180
tctgaaaggc caaaagagag tatgtattcc tggaacaaaa ctgcagagaa aagtgatttt 240
gaagctgtag aagcacttat gtcaatgagc tgcagttgga agtctgattt taagaaatac 300
gttgaaaaca gacctgttac accagtatct gatttgtcag aggaagagaa tctgcttccg 360
ggaacacctg attttcatac aatcccagca ttttgtttga ctccacctta cagtccttct 420
gactttgaac cctctcaagt gtcaaatctg atggcaccag cgccatctac tgtacacttc 480
aagtcactct cagatactgc caaacctcac attgccgcac ctttcaaaga ggaagaaaag 540
agcccagtat ctgcccccaa actccccaaa gctcaggcaa caagtgtgat tcgtcataca 600
gctgatgccc agctatgtaa ccaccagacc tgcccaatga aagcagccag catcctcaac 660
tatcagaaca attcttttag aagaagaacc cacctaaatg ttgaggctgc aagaaagaac 720
ataccatgtg ccgctgtgtc accaaacaga tccaaatgtg agagaaacac agtggcagat 780
gttgatgaga aagcaagtgc tgcactttat gacttttctg tgccttcctc agagacggtc 840
atctgcaggt ctcagccagc ccctgtgtcc ccacaacaga agtcagtgtt ggtctctcca 900

cctgcagtat ctgcaggggg agtgccacct atgccggtca tctgccagat ggttcccctt 960
cctgccaaca accctgttgt gacaacagtc gttcccagca ctcctcccag ccagccacca 1020
gccgtttgcc cccctgttgt gttcatgggc acacaagtcc ccaaaggcgc tgtcatgttt 1080
gtggtacccc agcccgttgt gcagagttca aagcctccgg tggtgagccc gaatggcacc 1140
agactctctc ccattgcccc tgctcctggg ttttcccctt cagcagcaaa agtcactcct 1200
cagattgatt catcaaggat aaggagtcac atctgtagcc acccaggatg tggcaagaca 1260
tactttaaaa gttcccatct gaaggcccac acgaggacgc acacaggaga aaagcctttc 1320
agctgtagct ggaaaggttg tgaaaggagg tttgcccgtt ctgatgaact gtccagacac 1380
aggcgaaccc acacgggtga gaagaaattt gcgtgcccca tgtgtgaccg gcggttcatg 1440
aggagtgacc atttgaccaa gcatgcccgg cgccatctat cagccaagaa gctaccaaac 1500
tggcagatgg aagtgagcaa gctaaatgac attgctctac ctccaacccc tgctcccaca 1560
cagtgacaga ccggaaagtg aagagtcaga actaactttg gtctcagcgg gagccagtgg 1620
tgatgtaaaa atgcttccac tgcaagtctg tggccccaca acgtgggctt aaagcagaag 1680
ccccacagcc tggcacgaag gccccgcctg ggttaggtga ctaaaagggc ttcggccaca 1740
ggcaggtcac agaaaggcag gtttcatttc ttatcacata agagagatga gaaagctttt 1800
attcctttga atatttttg aaggtttcag atgaggtcaa cacaggtagc acagattttg 1860
aatctgtgtg catatttgtt actttacttt tgctgtttat acttgagacc aacttttcaa 1920
tgtgattctt ctaaagcact ggtttcaaga atatggaggc tggaaggaaa taaacattac 1980
ggtacagaca tggagatgta aaatgagttt gtattattac aaatattgtc atcttttct 2040
agagttatct tctttattat tcctagtctt tccagtcaac atcgtggatg tagtgattaa 2100
atatatctag aactatcatt tttacactat tgtgaatatt tggaattgaa cgactgtata 2160
ttgctaagag ggcccaaaga attggaatcc tccttaattt aattgctttg aagcatagct 2220
acaatttgtt tttgcatttt tgttttgaaa gtttaacaaa tgactgtatc taggcatttc 2280
attatgcttt gaactttagt ttgcctgcag tttcttgtgt agatttgaaa attgtatacc 2340
aatgtgtttt ctgtagactc taagatacac tgcactttgt ttagaaaaaa aactgaagat 2400
gaaatatata ttgtaaagaa gggatattaa gaatcttaga taacttcttg aaaaagatgg 2460
cttatgtcat cagtaaagta cctttatgtt atgaggatat aatgtgtgct ttattgaatt 2520
agaaaattag tgaccattat tcacaggtgg acaaatgttg tcctgttaat ttataggagt 2580
tttttgggga tgtggaggta gttgggtaga aaaattatta gaacattcac ttttgttaac 2640
agtatttctc ttttattctg ttatatagtg gatgatatac acagtggcaa aacaaaagta 2700
cattgcttaa aatatatagt gaaaaatgtc actatatctt cccatttaac attgtttttg 2760
tatattgggt gtagatttct gacatcaaaa cttggaccct tggaaaacaa aagttttaat 2820
taaaaaaaat ccttgtgact tacaatttgc acaatatttc ttttgttgta ctttatatct 2880

tgtttacaat aaagaattc 2899

<210> 112
<211> 3138
<212> DNA
<213> NM_018223.1| Homo sapiens checkpoint with forkhead and ring finger domains (CHFR), mRNA

<400> 112

ctcttgacag cggcggcggc gcagccggtt ccgggttcgg cgcggggcgg ggatgtgaat 60
cccgatggag cggcccgagg aaggcaagca gtcgccgccg ccgcagccct ggggacggct 120
cctgcgtctg ggcgcggagg agggcgagcc gcacgtcctc ctgaggaagc gggagtggac 180
catcgggcgg agacgaggtt gcgacctttc cttccccagc aataaactgg tctctggaga 240
tcactgtaga attgtagtgg atgaaaaatc aggtcaggtg acactggaag ataccagcac 300
cagtggaaca gtgattaaca agctgaaggt tgttaagaag cagacatgcc ctttacagac 360
tggggatgtc atctacttgg tgtacaggaa gaatgaaccg gaacacaacg tggcatacct 420
ctatgaatct ttaagtgaaa agcaaggcat gacacaagaa tcctttgaga tggtgccttg 480
ctgtgttgcc caggctggtc taaaactcct gggatcaagt gatcctccca ccttggcctc 540
ccaaagtatt gtgattacag ggtctggggg tggtggcatc tcccctaaag gaagtggtcc 600
ctctgtggca agtgatgaag tctccagctt tgcctcagct ctcccagaca gaaagactgc 660
gtccttttcg tcgttggaac cccaggatca ggaggatttg gagcccgaga agaagaaaat 720
gagaggagat ggggaccttg acctgaacgg gcagttgttg gtcgcacaac cgcgtagaaa 780
tgcccaaacc gtccacgagg acgtcagagc agcggctggg aagccagaca agatggagga 840
gacgctgaca tgcatcatct gccaggacct gctgcacgac tgcgtgagtt tgcagccctg 900
catgcacacg ttctgcgcgg cttgctactc gggctggatg gagcgctcgt ccctgtgtcc 960
tacctgccgc tgtcccgtgg agcggatctg taaaaaccac atcctcaaca acctcgtgga 1020
agcatacctc atccagcatc cagacaagag tcgcagtgaa gaagatgtgc aaagtatgga 1080
tgccaggaat aaaatcactc aagacatgct gcagcccaaa gtcaggcggt ctttttctga 1140
tgaagaaggg agttcagagg acctgctgga gctgtcagac gttgacagtg agtcctcaga 1200
cattagccag ccatacgtcg tgtgccggca gtgtcctgag tacagaaggc aggcggcgca 1260
gcctccccac tgcccagcac ccgagggcga gccaggagcc ccacaggccc tgggggatgc 1320
accccccacg tccgtcagcc tgacgacagc agtccaggat tacgtgtgcc ctctgcaagg 1380
aagccacgcc ctgtgcacct gctgcttcca gcccatgccc gaccggagag cggagcgcga 1440
gcaggacccg cgtgtcgccc ctcagcagtg tgcggtctgc ctgcagcctt tctgccacct 1500
gtactggggc tgcacccgga ccggctgcta cggctgcctg gccccgtttt gtgagctcaa 1560

```
cctgggtgac aagtgtctgg acggcgtgct gaacaacaac agctacgagt cagacatcct   1620
gaagaattac ctggcaacca gaggtttgac atggaaaaac atgttgaccg agagcctcgt   1680
ggctctccag cggggagtgt ttctgctgtc tgattacaga gtcacgggag acaccgttct   1740
gtgttactgc tgtggcctgc gcagcttccg tgagctgacc tatcagtatc agcagaacat   1800
tcctgcttcc gagttgccag tggccgtaac atcccgtcct gactgctact ggggccgtaa   1860
ctgccgcact caggtgaaag ctcaccacgc catgaaattc aatcatatct gtgaacagac   1920
aaggttcaaa aactaagcat ccagaggccc tgagcagctt tcagcactgg aggtgaagag   1980
agcgtgtttt taaaatacag aggcaagcac gtcaaggtgt tttcacagcc ccctgaggga   2040
agggacgcag ggtctccgac aggtgctctg gggtgactct tctgtggagc tttaccctct   2100
gagtgagacc ctccccagag ccccgggggc cgcagcccgc cctcctggtg agcgctgggc   2160
agggctcgtg gtggcatcag cagcagagac gaagcctttc tgtaacatgc ggccgtcctg   2220
ccgagagggg cagttttgct cttttgtaca ttttccgaaa ctacagttaa agcggaagtc   2280
tgttttcagg aaaagtttca agggagaagg gcaagtttat caaaaacatt gtttcaggag   2340
aagggagcat aagtttacag cctacaggac gtacacaata tcctgctgct gggaaaacca   2400
cagcatttta tctatttttt attttaatag gtttggtgct tatcttctaa taagatttaa   2460
atgtcacaaa ctgtagcaca aataatataa tttataattt acaaattgac taaaattggg   2520
tatagtatgg tatttgaaag aataagcata tgcttctgtt tattaaaaaa agaaaccttc   2580
caatgtccaa aactgctaac cctcgacgtg gccgccaagt tagtcgctcc ttgctaaccg   2640
gtgagtgacc gcggccccga gcctggggct ggacgcaggt cccaggacat gctgctccct   2700
tgtgtgagtg accgcggccc cgagcctggg gctggacgca ggtcccagga cgtgctgctc   2760
ccttgtgtga gtgaccacgg ccccaagccc agggctggag gcaggtccca ggacgcgccg   2820
ctccctcatg ctgcccgggc ccttcctcca agaccctaca gagcctgagg ggcaccttgg   2880
cttccgcctg tgctagcttt gccatgtcat ctggaataat acttgaaatt ttgattcttg   2940
gaaaaaaaag tttcttatct tttgttgaaa tcacctgtta tccttgtttg taaactgata   3000
actttttgc ttcttctcag gaatacagtt ttcaactgtt gtcttgctct tgatagaaac   3060
tgagaagcag caatctgtat ttgtggagga aagtcctctc ttttgcatat tctaataaat   3120
gagccgcgtt tgctcctc                                                 3138
```

<210> 113
<211> 2466
<212> DNA
<213> NM_024645.1| Homo sapiens hypothetical protein FLJ13842 (FLJ313842), mRNA

<400> 113

```
agttggtccg agctgccgaa aggtctggtc gcagagacag gaacgtgtaa tcctcagcgt     60
```

```
gctccagccc acagcttcgc tctactgctc ggcagggcag ctggcctctg ggcaccggcg    120
gcccctctgc ctcgcggaaa agcctgatga agtcctccga tattgatcag gatttattca    180
cagacagtta ctgcaaggtg tgcagtgcac agctgatctc cgaatcgcag cgtgtggccc    240
actacgagag tcgaaaacat gcaagcaaag tccgactgta ttacatgctt caccccaggg    300
atggagggtg tcctgccaag aggctccggt cagaaaatgg aagtgatgcc gacatggtgg    360
ataagaacaa gtgctgcaca ctctgcaaca tgtcattcac ttcagcggtg gtggccgatt    420
cccattatca aggcaaaatc cacgccaaga ggttaaaact cttgctagga gagaagaccc    480
cattaaagac cacagcaaca cccctgagcc cacttaagcc cccacggatg gacactgctc    540
cggtggtcgc atctccctat caaagaagag attcagacag atactgtggg ctctgtgcag    600
cctggtttaa taaccctctg atggcccagc aacattatga tggcaagaaa cacaaaaaga    660
atgcggcaag agttgctttg ttagaacaac tggggacaac cctggatatg gggaactga     720
gaggtctgag gcgcaattac agatgtacca tctgcagtgt ctccctaaac tcaatagaac    780
agtatcatgc ccatctgaaa ggatctaaac accagaccaa cctgaagaat aagtagtgaa    840
agcatcaatc aagacataag aacaaaacat tagcatttct ctgccgtgga gaattgctta    900
tcaaccacca gaggaggctt ctttcttgaa caataaacat ttcttataag gattcacaga    960
ttcacatacg actgatcttg atttttggaa atgaatgagg tttctttttt ctttttcctt   1020
tttttaattt tggggtaagt tatgatattt ggatggattt ttaaattctt tcctgataac   1080
atatttagca catgttctaa attataatcc tatagcaaac agttggagca ttattcaaac   1140
tgaaagtgga aaaatttaaa tttccaattt attctagatt tcctcagagc ataattattc   1200
tgttaaatcc tcaatgagtg tgatgtaaac cacctctatc cagaaatata cattcttttc   1260
tcatcatgtt ggacacagtt gagggtgaca tgcacagaac tggaacagat cactattagt   1320
ggaaaatacc aaatggacaa ataaatacca gtcgttttct ccgttctcca agcacaggag   1380
ccaggtttac catctgaaca atgaagacga agggagtaaa taaaggaaga attctcatct   1440
tttttcctga tcattcaaag aacagtttct caaggttaag ccaagtcctc cttgcaagtt   1500
gccaaataat agcttaggaa aagaattagt ctgcctgcat gatgatcttc ttaggcaaaa   1560
acgtcttcac agcccttgac cttggtgaat ttttttcccc aaaagcatcc aaaagaagaa   1620
ttataaaccc cagaacgaga tggaaataaa caagtatttt tttttatga tgtttggcct     1680
gaactgtggg ctttaattgg gggatactga tcgtttggaa agaagtgaga aaattctgaa   1740
gaaatggcgg ccttgggcta ggcggggtcc cctatttctt ctgtttctca ctgaagtcct   1800
actgctgagc caagactcag tcactctgga aagagcatga ccgataaaga aaacagttcc   1860
tttctgatgg ggagcgtctg agtgcagatc atgaggctct ttctctaggt ttaattcttt   1920
tccatggtga ccggacttgg tgtcttgtag cctggttacg aagtgggacg ttgagcttct   1980
actgacgatg ccctgcatgg accagctggg atctggctgg ggctgccctg tgtccctaac   2040
```

gaccataggc aatccatctt cttgtgtcag caatttctgg acacccactg ttttccacca 2100
agagctgagg tggcaacaac tcagtgagca ataaacaaaa tgacacagaa atgcacagtg 2160
ttgttatgaa ggagcctgtt tacctgtgtt caaaatctgg caccattccc ttgagcaggg 2220
cccgctcagg agggaccagg tctgccagtt tctgtgcctg cagagagacg aagccccacg 2280
agccacaccc tactctacaa gaggaaaggg ggttggatgg gaagaatcta ttttgctgtt 2340
ttggaaagca cacagccgac ctacaaacct cctgtgatgg tgtttcttcg gatgtgtaaa 2400
ataaggcttt atttgtcaat tccgctgtaa aataagcatt gtccgagtaa aaacagcagc 2460
aacaac 2466

<210> 114
<211> 3658
<212> DNA
<213> NM_025195.2| Homo sapiens tribbles homolog 1 (Drosophila) (TRIB1), mRNA

<400> 114

aaggaggagt gcactggccg ggatcggtgc agcgctcaca ctcactcaca gtcactctct 60
ctgagcgcgt ctcgctcgct ctcatacacg cccggagccc aggagcgctc aggatcccga 120
gcgccgcgaa aaagttcccc cggcttttgc tggagactca tcgttttggg aagtgcattt 180
gcttcgtggc tccgccgagc ctgctgaatc ctgtcctcgc ggcacgggac cccgggatcg 240
ctgaccgctg ccgccgccgc ctctgcctcc cggactatcg gcagcctcgg caacaatagt 300
ggcggccgcc cccagcgagg ctccgggagc ccttgcctgc gggggtccgg ggactcgagc 360
cggcctccgc ctcccggacg cacagccagc gtggtccccg cgtgcaacgc gagcgccggg 420
gagtggctcc tgctttgccc ctcgtggggg ccgagccaag accagtctgc aaactccatc 480
ccgccggctg gaagaagtcg cggagccggc accaaacccg cagcgtcttc ccgcgcggat 540
cccgggactt aaaaagccgg ggccaccccg gcccaggacg ggatgcgggt cggtccggtg 600
cgctctgcca tgagcggcgc ctcgcagccc cgcggcccgg ccctgctctt cccagccacc 660
cgaggcgtcc cggccaaacg cctgctggac gccgacgacg cggcggctgt ggcggccaag 720
tgcccgcgcc tctccgagtg ctccagcccc ccggactacc tcagcccccc cggctcgccc 780
tgcagcccgc agcccccgcc tgccgctccg ggggccggcg gaggctccgg gagcgcgccg 840
gggcccagcc gcatcgccga ctacctgctg ctgcccctag ccgagcgcga gcatgtgtcc 900
cgggcgctgt gcatccacac tggacgcgag ctgcgctgca aggtgtttcc cattaaacac 960
taccaggaca aaatcaggcc ttacatccag ctgccatcgc acagcaacat tactggcatt 1020
gtggaagtga tccttgggga aaccaaggcc tatgtcttct ttgagaagga ctttggggac 1080
atgcactcct atgtgcgaag ccggaagagg ctgcgggaag aggaagccgc ccggctcttc 1140
aagcagattg tctccgccgt cgcccactgc caccagtcag ccatcgtgct gggggacctg 1200

```
aagcttagga agttcgtctt ctccacggag gagagaaccc agcttagact agaaagtcta 1260
gaagacacac acataatgaa gggggaagat gatgctttgt cagacaaaca tggctgccca 1320
gcctacgtga gccctgagat cctcaacacc actgggacct actccggaaa ggctgcggac 1380
gtttggagcc tgggggtgat gctctacacc cttctggttg gacgataccc cttccatgac 1440
tcagacccca gtgccctttt ctccaaaatt cggcgtggac agttctgcat tcctgagcac 1500
atttccccca aagccaggtg cctcattcgc agcctcttga gacgggagcc ctccgagaga 1560
ctcactgccc ccgagatcct actgcacccc tggtttgagt ccgtcttgga acccgggtac 1620
atcgactcag aaataggaac ttcagaccag attgttccag agtaccagga ggacagtgac 1680
attagttcct tcttctgcta atccccaaaa cctcagaaac ctcataattc ttaacacctg 1740
gcatttccat ttctaaagat ggacaggccc tttggcgtgg taccaaccag ataatgactg 1800
catcaggatg aaagctgctg aactcggcat ggcgcctcct cttctctgtt gggatgagtg 1860
actttattga tttgagcagc atatgctgtg attggctgcc ctgcaaattt gtttccctta 1920
aggaaccctc accaactatc tctgctggat ttgggagttc cgcatctttt gtggagggca 1980
gagtatggac atcttacacc cggtggtcaa gtgtgtaata aacttgagca ttcgaatggg 2040
agaaaaagca aatcgcacaa tgacatattt tgagtaataa ccgtattttt cacagggtga 2100
caaattgggc caataaatct gccatctttg aactcatctt tggtggctag actgctacgg 2160
cagcttctct gatgggaaag ttcctttttt ggcttaacac tcaccctttc ttcacactca 2220
catttaccaa tgactctgct ccgtttttgg agcagactgt tttaagttgc tcaggagcct 2280
gatggaacca tgaaccgaga ctcttctctg tttcctgcca agacctcatc tgcactaatg 2340
ccttctccct gaccttgaca cttccccctt tagctataaa agcacttacc agccgaacgt 2400
ggaacagtat cacaaaagat tccatctccc aacgatttca gaactctgag ctcagagaga 2460
ctccagattt taaaaaataa tttgagtgct tggaaactat tagcttttta agttccttcc 2520
aaatatgtta gtacctaccc tttacttttt ccccaagacc atctcagggt ggagcattct 2580
gtctaagaga agaaagataa ggaggctccc acccacctct cccaagagca gacattaaac 2640
atctttgtgc tttgaagaga gtgaattttg gatagtcttg tgattctcag actaacttcc 2700
agaattatac tttaacccct cccagatatg gtccgccttt ggcattgtgt gtacatctgc 2760
agttttgcat ggtgggttgt taatatttca aatgtgtggt ttatgaatac gtctgtataa 2820
tcggcttctg gagtgaaaca gcaaacccca aatcttcaaa gttggaagga actttaaaaa 2880
tcatccggtc caatctcttt cctctttctg ccacctccca aggcagaaat cccctcttca 2940
gcttcttttg taggtgggaa tccagcctct gttagatatg tccagagatg gaaactcact 3000
cccctacaaa agatggagct taatggagaa attgcaactt tcattaaaaa acaaattcag 3060
atgaaatatc agtaactgtc ttggacagtg ctgaaatcag gtggttaaac gggtaaacaa 3120
aatatactgt attttgagaa atggcacaaa aacaggcagt catctttaag ggctatgcct 3180
```

aggcaaacta ctaacatgca ttgtgagaat gccgtgtata cctcacgtac tgtgtacttt 3240
gtacatatat tttacctttt atacctatgt tcgattttgt tttgttttgt tttgttctgg 3300
ctttgaggct tgttttgttg tctgtgtctg tctgaataac ctgcgtgtct aaaaccacgt 3360
gaaatgtgaa tgattattgg caatattacc ttgacagaat catgggactt tgagaagagg 3420
gaggacagag gcctctgtcg cactaacgct ctcgtggttg ctcgactgtt gtatctgtga 3480
tacattatcc gactaaggac tctgggctgg cagggccttc tgccgggaaa gctagaaaca 3540
ctaggttctt cctgtacata cgtgtatata tgtgaacagt gagatggccg tttctgactt 3600
gtagagaaat tttaataaac ctggtttcgt aaaaaaaaaa aaaaaaaaaa aaaaaaaa 3658

<210> 115
<211> 4624
<212> DNA
<213> NM_033331.1| Homo sapiens CDC14 cell division cycle 14 homolog B (S. cerevisiae) (CDC14B), transcript variant 2, mRNA

<400> 115

cacggaacag ccctcctggg gtccccacga gccgcgtcct gctgtgcccc ggcgcctacg 60
cagcagcggc cgcggccgcg gtgggcacgc acggttaccc cgggcagctc cggccgccag 120
ctgcagcccc gtcgcctcgg ccgcgccagc cggctgcggg cacctggggg cgggctgggg 180
gcgccggccg cggcaggagg cgctgtagcg agggctgcgg cgccggtcct gcggcggccg 240
cgggaggcag cggggcaggc gctgtgggcc gggctcctcc tccggctcct gcgcgaccgc 300
ctcccgccgg gctctgccgg cgcccgccgt ccccgcagcg ccgctctgcg cccgccgccc 360
cgagcgcccg cgcggggctg gcgggagcct cggcgggcgc gcgggcgcgc ggggccatgg 420
tcgtggcccc ctgacgggcc gcggccgcct ccatgaagcg gaaaagcgag cggcggtcga 480
gctgggccgc cgcgcccccc tgctcgcggc gctgctcgtc gacctcgccg ggtgtgaaga 540
agatccgcag ctccacgcag caagacccgc gccgccggga cccccaggac gacgtgtacc 600
tggacatcac cgatcgcctt tgttttgcca ttctctacag cagaccaaag agtgcatcaa 660
atgtacatta tttcagcata gataatgaac ttgaatatga gaacttctac gcagattttg 720
gaccactcaa tctggcaatg gtttacagat attgttgcaa gatcaataag aaattaaagt 780
ccattacaat gttaaggaag aaaattgttc attttactgg ctctgatcag agaaaacaag 840
caaatgctgc cttccttgtt ggatgctaca tggttatata tttggggaga accccagaag 900
aagcatatag aatattaatc tttggagaga catcctatat tcctttcaga gatgctgcct 960
atggaagttg caatttctac attacacttc ttgactgttt tcatgcagta aagaaggcaa 1020
tgcagtatgg cttccttaat ttcaactcat ttaaccttga tgaatatgaa cactatgaaa 1080
aagcagaaaa tggagattta aattggataa taccagaccg atttattgcc ttctgtggac 1140

ctcattcaag agccagactt gaaagtggtt accaccaaca ttctcctgag acttatattc 1200
aatattttaa gaatcacaat gttactacca ttattcgtct gaataaaagg atgtatgatg 1260
ccaaacgctt tacggatgct ggcttcgatc accatgatct ttctttgcg gatggcagca 1320
cccctactga tgccattgtc aaagaattcc tagatatctg tgaaaatgct gagggtgcca 1380
ttgcagtaca ttgcaaagct ggccttggtc gcacgggcac tctgatagcc tgctacatca 1440
tgaagcatta caggatgaca gcagccgaga ccattgcgtg ggtcaggatc tgcagacctg 1500
gctcggtgat tgggcctcag cagcagtttt tggtgatgaa gcaaaccaac ctctggctgg 1560
aaggggacta ttttcgtcag aagttaaagg ggcaggagaa tggacaacac agagcagcct 1620
tctccaaact tctctctggc gttgatgaca tttccataaa tggggtcgag aatcaagatc 1680
agcaagaacc cgaaccgtac agtgatgatg acgaaatcaa tggagtgaca caaggtgata 1740
gacttcgggc cttgaaaagc agaagacaat ccaaaacaaa cgctattcct ctcacagtaa 1800
ttcttcaatc cagtgttcag agctgtaaaa catctgaacc taacatttct ggcagtgcag 1860
gcattactaa aagaaccacc agatctgctt caaggaaaag cagtgttaaa agtctctcca 1920
tttcaaggac taaaacagtc ttgcgttaag taaaaacctg tgaccagagc tgaaggaaga 1980
ctctaggact gaaaactgca acagaaatta gcacaatttg aaaacaaaac aaaattgcaa 2040
aagccttagt tgctttttcc acctaagaag ttgatcaatg gagaaaatgt ccactggagt 2100
ttgaataatg aactttgagt ttgggtgcaa gcaaatgact cagagaaggg tccagctctc 2160
aagctgaatg acaaacatgc tgttgtaaat ttagtctcag gtgtaaatac ccaagccctc 2220
tggtacccag ggagctggct ggtctgtggt gcatgtgtgt ccctgtgatg gcaatcattg 2280
tagttgctgg ccttcagaag aattgaggat ctgatggagg tttttatgt atttattttc 2340
tgttcacctt gtgaccctgt gtcaaaattt ataaagatac aaaaggcatt actgaaatgg 2400
tactttctgt aatttgatac tatttggctt aatcatcttc acttgactat ttgtaatact 2460
gttgtaatgt taactctgtt aagtacccaa gctgcttgtc ttccaccaaa gagtgcttta 2520
ttaacaagaa tctgtgaaaa tcacatttaa acactgttgc atgttgtaag accaggtggt 2580
accttagtaa cctaaaactt gcaagagaat attaatggta gctttagaag actcaggagg 2640
agaaactgac ttcagagttg gaagatgttg caagtcgttc ctttttctgt ccttcaggga 2700
ctgaagaact gggaggctgc ccattgtttg gttgccagtc atacaaatta aaatcatatt 2760
tccttccatg aatggaagaa acacactatt ggtttttccc cttggaaaca gcaatcccaa 2820
ataatgtcgg cttacaaaaa aaaaaagtta ccactttttt agagtccttc cctgtaacat 2880
tggatttttt ttttccctta tgagatccac ctaaggccat tgacgtggcc tgcgatctca 2940
gtgacaatga tctgcttctg gatctcactg ttgcctttgg ttagggaaca caactagtaa 3000
ctctgcagag tgccttctcc cgcagcccta ctggaacaca gcagagtctg tgccatgaag 3060
cagttacaga aacagaattg atgtgctgcc aaaaaaaaaa aaaaaatggg gcccgaaata 3120
aaagaatata tagtactcac ctcagttcct tccataagaa gtgggtggtt taatgattgt 3180

taagccattt ttgcctgtgc cgggagcatg gagggctgag atgtcgacag gcagtgggaa 3240
acaaatgccc tcctaagcca caaggcgtgc gccagattag taggcaactc cattttaaga 3300
agctgccttt ttcacaaaac tggaagaaat aaaagcggtt ggaataaaca agttaaaagt 3360
ctttaatgca aaaagtaatt gaaaggcagt gcctccattt tggtgtactt tcttggaaga 3420
aagtataaaa ttgaccggca tcatgagaga cggaagatgc cgtgttctca gccaaacaag 3480
caactctttc cccgccaggc actgtcgggt ggggtcaggc cagcttttaa acactgggga 3540
ctggatcaca gaaaaacagt ggttttctgt ccctggaaat gaataggcac aaagacccac 3600
ttggctgtgg gcagactact cttcaataag atttgggtgg gaggaggaac attccttttg 3660
ctattttgag ctgagacaat ataaatattc aaactgtgcc atgcataaag cattgaattc 3720
tcagggcacc tcttcttccc cttacccctt ttaaggccat cccctccatt aataataatc 3780
caggtagttg tgaaaatcgt gcttctatct gatcccttct tagtttggct tttcatccca 3840
tcagaacaag taaacgtagg cgccacagct cttgtgagta ctgtctccct cacggtgaat 3900
gagcctcctg gtgtttcgtc caagaaaaga aagggtgtca ctggaaccac agcccttttt 3960
cattttataa actgcctctt catgttgcct gctcaagttt ccacctagaa ttgctatcac 4020
tgtggctctt tctaaaaatc tttctattta actggttcac tgaaattagt catagaaaac 4080
ttgtgatttg gtgaagaggc attccttgta ataaccaaat gacttgggat ggtgtgcata 4140
gcaagggcag tgttacactt atgaggactg tctctagcat ccaggaagtc tctgggtctg 4200
agggatggaa agttcttcct gctatgaatg agagtggact cttcccctca cccccaactg 4260
aaaccacaaa caaccagaat cttctggaat tctgacttag agtcgttgtt atagaagacc 4320
ttgttgctat ggaacatgaa actgtgtgtc agatggagag atccccttaa cctaagagcc 4380
ttaaatagcc ctgaaagtac actgggacgg tttgcgatgg aattaaaatt ggaagtgaat 4440
atttttaggt gctcttgaag ctttctgggg actcaaaatt atcaaaagtc agggacagtc 4500
cggaggaaga gcgtctgcaa aactgggttc ctagaagtat agacggactt agctttttgt 4560
agaatttggt gaggagcagc gcctcgtgag agcagaatgg cctggcgtgg ccagtgcttc 4620
ccgg 4624

<210> 116
<211> 3919
<212> DNA
<213> NM_201524.1| Homo sapiens G protein-coupled receptor 56 (GPR56), transcript variant 2, mRNA

<400> 116

cgcactagct gtctgccctg ccctgccgta ggagatgggc tgggagcctc ccacgctctc 60
cagctcactc ggcaggcagc ggggaccagg gctggcaggt taagcctctg ggggtggatc 120

ctgaaaggtg gtccagccgc ctggccctgc gtgggaccct ccacctggca gcagacaggg 180
tctcgctctg tcacacaggc tggagtgcag tggtgtgatc ttggctcatc gtaacctcca 240
cctcccgggt tcaagtgatt ctcatgcctc agcctcccga gtagctggga ttacaggtgg 300
tgacttccaa gagtgactcc gtcggaggaa aatgactccc cagtcgctgc tgcagacgac 360
actgttcctg ctgagtctgc tcttcctggt ccaaggtgcc cacggcaggg gccacaggga 420
agactttcgc ttctgcagcc agcggaacca gacacacagg agcagcctcc actacaaacc 480
cacaccagac ctgcgcatct ccatcgagaa ctccgaagag gccctcacag tccatgcccc 540
tttccctgca gcccaccctg cttcccgatc cttccctgac cccaggggcc tctaccactt 600
ctgcctctac tggaaccgac atgctgggag attacatctt ctctatggca agcgtgactt 660
cttgctgagt gacaaagcct ctagcctcct ctgcttccag caccaggagg agagcctggc 720
tcagggcccc ccgctgttag ccacttctgt cacctcctgg tggagccctc agaacatcag 780
cctgcccagt gccgccagct tcaccttctc cttccacagt cctccccaca cggccgctca 840
caatgcctcg gtggacatgt gcgagctcaa aagggacctc cagctgctca gccagttcct 900
gaagcatccc cagaaggcct caaggaggcc ctcggctgcc cccgccagcc agcagttgca 960
gagcctggag tcgaaactga cctctgtgag attcatgggg gacatggtgt ccttcgagga 1020
ggaccggatc aacgccacgg tgtggaagct ccagcccaca gccggcctcc aggacctgca 1080
catccactcc cggcaggagg aggagcagag cgagatcatg gagtactcgg tgctgctgcc 1140
tcgaacactc ttccagagga cgaaaggccg gagcggggag gctgagaaga gactcctcct 1200
ggtggacttc agcagccaag ccctgttcca ggacaagaat tccagccaag tcctgggtga 1260
gaaggtcttg gggattgtgg tacagaacac caaagtagcc aacctcacgg agcccgtggt 1320
gctcactttc cagcaccagc tacagccgaa gaatgtgact ctgcaatgtg tgttctgggt 1380
tgaagacccc acattgagca gcccggggca ttggagcagt gctgggtgtg agaccgtcag 1440
gagagaaacc caaacatcct gcttctgcaa ccacttgacc tactttgcag tgctgatggt 1500
ctcctcggtg gaggtggacg ccgtgcacaa gcactacctg agcctcctct cctacgtggg 1560
ctgtgtcgtc tctgccctgg cctgccttgt caccattgcc gcctacctct gctccaggag 1620
gaaacctcgg gactacacca tcaaggtgca catgaacctg ctgctggccg tcttcctgct 1680
ggacacgagc ttcctgctca gcgagccggt ggccctgaca ggctctgagg ctggctgccg 1740
agccagtgcc atcttcctgc acttctccct gctcacctgc ctttcctgga tgggcctcga 1800
ggggtacaac ctctaccgac tcgtggtgga ggtctttggc acctatgtcc ctggctacct 1860
actcaagctg agcgccatgg gctggggctt ccccatcttt ctggtgacgc tggtggccct 1920
ggtggatgtg gacaactatg gccccatcat cttggctgtg cataggactc cagagggcgt 1980
catctaccct tccatgtgct ggatccggga ctccctggtc agctacatca ccaacctggg 2040
cctcttcagc ctggtgtttc tgttcaacat ggccatgcta gccaccatgg tggtgcagat 2100

cctgcggctg cgcccccaca cccaaaagtg gtcacatgtg ctgacactgc tgggcctcag 2160
cctggtcctt ggcctgccct gggccttgat cttcttctcc tttgcttctg gcaccttcca 2220
gcttgtcgtc ctctaccttt tcagcatcat cacctccttc caaggcttcc tcatcttcat 2280
ctggtactgg tccatgcggc tgcaggcccg gggtggcccc tcccctctga agagcaactc 2340
agacagcgcc aggctcccca tcagctcggg cagcacctcg tccagccgca tctaggcctc 2400
cagcccacct gcccatgtga tgaagcagag attcggcctc gtcgcacact gcctgtggcc 2460
cccgagcccg gcccagcccc aggccagtca gccgcagact ttggaaagcc caacgaccat 2520
ggagagatgg gccgttgcca tggtggacgg actcccgggc tgggcttttg aattggcctt 2580
ggggactact cggctctcac tcagctccca cgggactcag aagtgcgccg ccatgctgcc 2640
tagggtactg tccccacatc tgtcccaacc cagctggagg cctggtctct ccttacaacc 2700
cctgggccca gccctcattg ctgggggcca ggccttggat cttgagggtc tggcacatcc 2760
ttaatcctgt gcccctgcct gggacagaaa tgtggctcca gttgctctgt ctctcgtggt 2820
caccctgagg gcactctgca tcctctgtca ttttaacctc aggtggcacc cagggcgaat 2880
ggggcccagg gcagaccttc agggccagag ccctggcgga ggagaggccc tttgccagga 2940
gcacagcagc agctcgccta cctctgagcc caggccccct ccctccctca gcccccagt 3000
cctccctcca tcttccctgg ggttctcctc ctctcccagg gcctccttgc tccttcgttc 3060
acagctgggg gtccccgatt ccaatgctgt tttttgggga gtggtttcca ggagctgcct 3120
ggtgtctgct gtaaatgttt gtctactgca caagcctcgg cctgcccctg agccaggctc 3180
ggtaccgatg cgtgggctgg gctaggtccc tctgtccatc tgggcctttg tatgagctgc 3240
attgcccttg ctcaccctga ccaagcacac gcctcagagg ggccctcagc ctctcctgaa 3300
gccctcttgt ggcaagaact gtggaccatg ccagtcccgt ctggtttcca tcccaccact 3360
ccaaggactg agactgacct cctctggtga cactggccta gggcctgaca ctctcctaag 3420
aggttctctc caagccccca aatagctcca ggcgccctcg gccgcccatc atggttaatt 3480
ctgtccaaca aacacacacg ggtagattgc tggcctgttg taggtggtag ggacacagat 3540
gaccgacctg gtcactcctc ctgccaacat tcagtctggt atgtgaggcg tgcgtgaagc 3600
aagaactcct ggagctacag ggacagggag ccatcattcc tgcctgggaa tcctggaaga 3660
cttcctgcag gagtcagcgt tcaatcttga ccttgaagat gggaaggatg ttctttttac 3720
gtaccaattc ttttgtcttt tgatattaaa aagaagtaca tgttcattgt agagaatttg 3780
gaaactgtag aagagaatca agaagaaaaa taaaaatcag ctgttgtaat cacctagcaa 3840
actggaaaaa aaaaaaaaaa aaaaaaaaaa aaaaaaaaaa aaaaaaaaaa aaaaaaaaaa 3900
aaaaaaaaaa aaaaaaaaa 3919

<210> 117
<211> 7401
<212> DNA
<213> NM_014615.1| Homo sapiens KIAA0182 protein (KIAA0182), mRNA

<400> 117

aagcagttta gacaaacact gggcgacggt ggctccagca tgtatcagcc gaggtggagc 60
tgcggggccc tggcatgaaa ggcatgagcc atgagcccaa gtccccttcg ctagggatgc 120
tttccaccgc gaccaggacc accgccaccg tcaacccccct caccccctcg ccgctcaatg 180
gcgccctggt gcccagcggc agccccgcca ccagcagcgc gctgtcggcc caggccgcgc 240
catcctccag ctttgccgcc gcgctgcgca agctcgccaa acaggcggag gagcccagag 300
ggtcctcact gagcagcgag tcgtcccccg tgtcctctcc ggccaccaac cacagctccc 360
ccgccagcac acccaagcgc gtgcccatgg gccctatcat cgtcccccct gggggccaca 420
gcgtgcccag caccccccccc gtggtgacca tcgctccaac caaaaccgtg aatggtgtct 480
ggaggagtga gagccggcag gatgccggct ccaggagcag cagtggaggt cgggaacgcc 540
tcattgtgga gcccccgctc cctcaggaga aggcaggggg accagccatc ccctcgcacc 600
tgctcagcac cccctacccc ttcggcctct cccccagctc agttgtgcag gattcccgct 660
tcccgccact caacctccag cggcccgtgc accacgtggt gccccccagt accgtgaccg 720
aggactacct gagaagcttc cggccctacc acaccaccga cgacctccgc atgtcctcac 780
tgcctcccct cggcctggac ccggccactg ctgcagccta ctaccacccc agctacctgg 840
ccccacaccc cttcccccac ccggccttca ggatggacga ctcctactgc ctgtctgccc 900
tgaggtcccc gttctacccc atccccaccc ccggctccct gcccccactg cacccatcag 960
cgatgcacct gcacctctct ggggtccgct accctcccga gctctcccac tcatccctgg 1020
cagcgctgca ctcggagcgc atgtctggcc tcagcgcgga gaggctgcag atggacgagg 1080
agctaaggcg ggagagggag cgcgagcgcg agcgcgagcg tgagcgtgag gctgaccgcg 1140
agcgggagaa ggaacgtgag cgcgaacgcg agaaggagcg cgagcaagag aaggagcgtg 1200
agcgtgagaa ggagcgcgag cgcgagctgg agcgccagcg ggagcagcgg gcccgggaga 1260
aggagctgct ggccgccaag gccctggagc ccagcttcct gcccgtggcc gagctgcatg 1320
ggctgcgtgg ccatgccact gaggagcggg gcaagccctc ggagcagctg accccaaccc 1380
gagcagagaa gctgaaggat gccggcctgc aggcgcccaa gcccgtccaa cacccccttgc 1440
atccggtgcc caccccacac cacacggtgc ccagcctcat ctccaaccat ggcatcttct 1500
ctctgcctag cagcagtgct gccacagccc tgctgatcca gcgcaccaat gaggaggaga 1560
agtggctggc gcggcagcgg cggctgcggc aggagaagga ggaccggcag tctcaggtgt 1620
ccgagttccg gcagcaggtg ctggagcagc acctggatat gggccggccc ccggtgccgg 1680
cggaggcaga gcacaggccg gagagcacca ccaggccagg accaaaccgt cacgagccag 1740
gtggccgtga ccctccgcag cactttgggg ggccaccacc tctgatttcg cccaagcccc 1800

agctccatgc tgcacccacg gccctctgga accccgtgtc cctgatggac aacaccttgg 1860
agacgcggcg ggccgaaagc cactctctgc acagccaccc ggctgcattt gagcccagcc 1920
gccaggcagc cgtgccgctg gtgaaggtgg agcgggtctt ctgcccggag aaagcagagg 1980
aggggccacg gaagcgtgag cctgcccctc tggacaagta ccagccacct ccgccgccac 2040
cacgagaggg agggagcctg gagcaccagc ccttcctgcc cgggcccggg cccttcctgg 2100
ctgagctcga gaagtccacc cagaccatcc tgggccagca gcgggcctcc ctcccacagg 2160
cggccacctt cggggagctc agcggacccc tgaagcctgg ctcgccctac cggcccccag 2220
tgccacgggc ccccgaccct gcctacatct atgatgagtt cctgcagcag cgccggaggc 2280
tggtcagcaa gctggacctg gaggagcgca ggcggcggga ggcccaggag aaagggtact 2340
actacgacct cgatgactct tacgacgaga gcgatgagga ggaggtcagg gcccacctcc 2400
gttgcgtggc cgagcagccg cccctcaaac tggacacgtc ctctgagaag ctagagtttt 2460
tgcaactttt tggcttgacc acccaacagc agaaggagga attggtggcc cagaagcgga 2520
ggaagcggcg gaggatgctg cgagagagaa gcccgtcgcc cccaacaatt cagagcaagc 2580
ggcagacgcc ttcaccgaga ctggcgctgt ctacccgcta cagccctgat gagatgaaca 2640
acagtcccaa cttcgaagaa aagaagaagt tcctgaccat cttcaacctg acccacatca 2700
gcgctgagaa gaggaaagac aaagagagac ttgttgaaat gctccgtgcc atgaagcaga 2760
aggcactgtc agcagcagtg gccgactcct tgacaaactc tccgagggac agtcctgccg 2820
tctccctgag tgaaccagcc acgcagcaag cctctctgga tgtggagaag ccggttggtg 2880
ttgctgcttc cttgtctgac atcccaaagg ccgcggagcc tgggaagctg gaacaggtcc 2940
ggccccagga gctgtcgaga gtccaggagc tagctcctgc cagcggggag aaggccaggc 3000
tgagcgaggc ccctggaggc aaaaagagtc tgagcatgct tcactatatc cggggcgctg 3060
cacccaagga cattcctgtg ccgctgtccc acagcaccaa tgggaagagc aagccgtggg 3120
agccctttgt ggcagaagag tttgcacatc agttccacga gtcagtgctg cagtccaccc 3180
agaaggccct gcagaagcat aaagggagcg tggctgtgct gtctgcagag cagaaccaca 3240
aggttgacac gtccgtccac tacaacattc ctgagctgca gtcctccagc cgcgcccctc 3300
caccccagca caatgggcag caggagcccc ccactgcaag gaagggcccc ccaacccagg 3360
agttggaccg ggactcggag gaggaggaag aggaggatga tgaagatgga gaagatgagg 3420
aggaagtccc caagcgcaag tggcaaggga tcgaggccgt ttttgaagct taccaggaac 3480
acatagaaga gcaaaatctg gagcggcagg tgttacagac acaatgtaga cgactggagg 3540
cccggcacta cagcctcagc ctgacggcag agcagctctc ccacagcgtg gcggagttga 3600
ggagccagaa acagaagatg gtctcagaaa gggagcggct ccaggcagaa ctggaccact 3660
tacgaaagtg ccttgccttg cctgcaatgc actggcctag ggctacctg aagggatatc 3720
ccaggtgacg gtttcccttg cactaggccg aacctatagt atagaaatat tatctatttt 3780
attaccttga atatttaata tttttcactg ggaggtttga agcttacaaa atgagaatgt 3840

gccatgcatg aagcaaagga ttccaggctc cagaaaaaat gaatgaactc accttgacgt 3900
caatgcaatt gaatcaccgt tgtcattcag cgagcaacca atgtaggatt gcccacagtt 3960
tttcttttta aaggtggttt tcgcccttcc tctcccacat tatttcttaa tctgaacatg 4020
aaggctccat tagcaacact aaaacttgat cattaacagc cccctgtgca tatgagtgga 4080
tcaaaccggt tctgttcttt cttgtgttgc catgttacta tgcctcaagc ccagtttgct 4140
tttgccgcag cgatggggcc agtctcattc ctccccagga gtgaaacttg cttcagctga 4200
aaaggttggg tgcattgtca gtaaaaaggg cttatttgtt tcattttact ttcctgcaaa 4260
attttcttca aagcaacaag tcctaggagc acacaaagca acccaaaggc ttttccctgg 4320
aaaagctctt tcttacctaa agataaaacc aattcacaaa ctgaaggtag ctttttatta 4380
ctccgtgggg agcatgtaca gagctctgtg tatacacagc ttcacaccca ccagattgtt 4440
actacagtgg gttgggtttt catacagacg taaattttga gagaaaagtc aaaggtgctt 4500
cagccttgta ctgtgtatat atattaaaaa aaaaacaaag ttttgtatgt ttttattact 4560
ttaactattg ttataaaaag cctgccattt ttaatatgtg gtttggggga tttttgtttg 4620
tttttcctgt ttgggggttt tgtttgttgt tttggttttt tttgggcaaa aaaaaaaaaa 4680
aaaccttgct tttagtgttt gtactgctgc tggtcaggac attaaaatat tgaagtgttt 4740
ttaaaaatta aagaagaaga aaagtaaaag agcttaccac tggcgcctat gcgatcactt 4800
catttttagt ttgagttgca ccagaagctg ccgtagaaag ccatgcgcta ctgcttacct 4860
cctccactcc ccctgcctgc ccccagcatc tggacaagct aatagcaaat attacccatt 4920
gctatcaagg gaggaggggg tagtctgtag aacccatgtg tgacagtcat gtgcacacat 4980
gggcgggggc ttttaaaaac ctttcaggaa gtcaatgatt tctgtgattg atataattct 5040
aaggtgtctg agagcaggta cagaatagga acttcagagg ctttgtttaa acgcaaagct 5100
ttgtaaaagc cacaaggtct gagctgaacc cctccttttt gaacttactg tgacaagcac 5160
aggaacggtc agaaactggg ctcatcacac caaggcaaag caacgggcga gtcttcctcc 5220
ttgtcctagt tactgcctat ggaggcagtg tttagatcaa gaaggcctct cttgctccca 5280
agggccctca ccagaggcca gggctgccag tcactggtct ggggggtgga ggcctgagct 5340
gagggcaggg tgcctgacct gtgtgccggc tgctcactgc tgtgaccagc agccgagccc 5400
ttggccctag cccttgctgc gcagaacagc ttgctggcag ctggcatcgt gtcgctttat 5460
ctgcccccgc acagtttgct ttgtacgtct gccaagaatc ttccagttat tagcaaactc 5520
agacgaatgt accgccagta ttatcagcag tcaacaagca ccttcctctc cacagaagca 5580
gctggaagag aactcgaggg gctgtgctgc aggcctcccc tcgaaagaca ctgggaggtc 5640
agcatgttcc acaggtgttc agagggagtc tgctacaaac tatcagggca aaatctcact 5700
ggatttctcc actgaaaacc tacttgaggt ttctggtctg aaggcttaag agtcacatct 5760
tagcacttcc gctctcaggc ctcctcctcc atcacagatg tctggatgct tttggaaatg 5820

gccttggcta aagtaaaagg gaaaagtaga tccgataact taaaaacgta gctcatccct 5880
taccatccaa ggggcactcc cttggttgga ttttctatga cagcacaggg gacaggtggc 5940
acaccatgag aggtctgccc agggtgggag cagtgtcact gtgctagcaa tagttggctt 6000
ctcccctgtc agtggaaacc ccacttctgc ccggcccttg agcttcttgc ccactgtctc 6060
cccatccttc cacctacttg tggcgatctg agtactctac tcttgctcaa gaagtaatac 6120
gacaatcaga atacaaacca gtaaggcaac acgaataaac taagaaaaag gtaagaactg 6180
tctcaaaaac gaaagcacac cacccaagac acagtaccca gtcatggttt ccccatccaa 6240
ctattagttt catactttga aaacttactt tcagattatt ctcaaagaac acagtagcac 6300
ctaaatctgt tttcaattgg gcttaaaaat tgacatgcaa tctcttaagt tttttgttca 6360
gctacttcac actgagtacc tcaaatctgc tctggagtcg attatgccac ctgtgtgtca 6420
ggatgcacct gaaagccctc ggctcggtcc ttagaccatc ttcctacatt acctggaagg 6480
gagctgccat ctgtccctct gcagagggat accttccaat agtaaattat ctggttcctc 6540
actgaaacaa gttatttttg cttcatatag tcagagtcag actgacatga taaaatatca 6600
tgttcctaat ctgttgtctc agataagtga ccaagacggg actttccaca ttttagtcta 6660
cattctaatc ttaaaggaat aaagcactga attgggacta acattctgat aggttgcacc 6720
cttaagagta ttcagagagc atcaaaagga gcccacacct tcagcagtga aggattctaa 6780
cacagggaat ctgcagtttg tagcagaatg gtattttcct caagtagctc ataatactgc 6840
caaatctcaa aagttaagct gaatttcaca ccagatccta ccccttttccc tgagccacat 6900
gtttcacaca agtgtagaaa atgccaggga tccaccacaa gatggagatg gtcagcacaa 6960
accgattctg ttcctcttta aagtgtatat tagccactta gcaatctcta tattctttca 7020
agtaaccaag ctgttgactt tcttactact tgcagtagcc tgtccccaac ttttccatcc 7080
agtgcttaac ctaaaaaact ccttaactct gccttgacct gaggaagacc atgctaactg 7140
gtgttatttt gtatgtaccc tgtgcttaat tctataacag taaaccccat acgcaggtgg 7200
gagggaggaa caccggtgcc tcggtcactc tgggggcagt ttagatgctg tgaaattaaa 7260
cctgttctaa gtgtacttgt ttgaattaat tgtattgtaa tattatttgt tgaatgtagt 7320
aattaggtat ttatgaatat attgctgtaa tttctgacaa catccaaaaa ataaaatctt 7380
cctaaattat gttaaaaaaa a 7401

<210> 118
<211> 2745
<212> DNA
<213> NM_033542.1| Homo sapiens chromosome 20 open reading frame 35 (C20orf35), mRNA

<400> 118

```
tttttccccg gaaacgtttc tttcctacgc agccgctcct gccgccgtgg tcgctggagc     60
tttgcctctc taggccggca gcgcctctcc tccatggtcc tgtctgtcag cgctgttttg    120
ggagcccgcc ggtgaggccg ggccacgctc agacacttcg atcgtcgagt ctgtcactgg    180
gcatggcggg tcagttccgc agctacgtgt gggacccgct gctgatcctg tcgcagatcg    240
tcctcatgca gaccgtgtat tacggctcgc tgggcctgtg gctggcgctg gtggacgggc    300
tagtgcgaag cagcccctcg ctggaccaga tgtttgacgc cgagatcctg ggcttttcca    360
cccctccagg ccggctctcc atgatgtcct tcatcctcaa cgccctcacc tgtgccctgg    420
gcttgctgta cttcatccgg cgaggaaagc agtgtctgga tttcactgtc actgtccatt    480
tctttcacct cctgggctgc tggttctaca gctcccgttt cccctcggcg ctgacctggt    540
ggctggtcca agccgtgtgc attgcactca tggctgtcat cggggagtac ctgtgcatgc    600
ggacggagct caaggagata cccctcaact cagcccctaa atccaatgtc tagaatcagg    660
ccctttggac atcctgctga cacttgggcc ccttaacacc ttgggctgct cagaccctcc    720
agatgaggtc cagcccagat ctgagaggaa ccctggaaat gtgaagtctc tgttggtttg    780
ggagagatag tgagggcctg tcaaagaagg caggtagcag tcagcatgac agctgcaaga    840
atgacctctg tctgttgaag ccttggtatc tgagaggtca ggaaggggac ctctttgagg    900
gtaataacag aattggaacc atgccactct tgagccacaa tacctgtcac cagcctgttg    960
ttttaagaga gaaaaaaaat caaggatatc tgattggagc aaaccacttc tttagtcatc   1020
tgtcttaccc ccctgggaca gctgttacct ttgcagtgtt gccgaatcac agcagttacc   1080
tttgcagtgt tgccgaatca cagcagttct gttggagaaa cgcttggttt ccggatccag   1140
agccacagaa agaaatgtag gtgtgaagta ttaggctgct gtcagggaga ggatggcaga   1200
tggaggcatc aagcacaagg aaaatgcaca acctgtgccc tgttatacac acgttcatgt   1260
gcacccaaga acctatgact ttcttccagt tccttctacc aggtccccat cctgctgcca   1320
gctctcaaca tagcaggcca taggacccag agaagaatcc cagcgttgct caaagtctaa   1380
ccatcataaa gacactgcct gtcttctagg aatgaccagg cacccagctc ccactggact   1440
ccaatttttt ttcctgcctt atttagaatt ctttggcggg aagggtatga tgggttccca   1500
gagacaagaa gcccaacctt ctggcctggg ctgtgctgat agtgctgagg gagataggaa   1560
tttgctgcta agatttttct ttggggtgga gtttcctctg tgaggggctt gcagctatcc   1620
ttcctgtgta tacaaataca gtattttcca tggttctgcc tgcacttact ttgtaatgcc   1680
acggttgaga ttgagagaga tcagcgcagc caggcaaggg aactttaaag aattattagg   1740
ccaccttctc cctttcctgg accccagagt cattcctcca tttggttaaa atactcagtg   1800
cagggaactc ttacatcctg tctccttcac ttgcagcgtc ccctgctatg cctcaggtga   1860
accacataat tcttgggttt ccgttcctac ttgctagtga tttctgaaca tgttcaatgg   1920
agcggcacac agtctagacc cacttccgca ttgaaacctt cactgttcct ctttggtttc   1980
ttcagagctt tcccaagaga gctgtcagtt ttcagctgtc agtaacacaa atgagtttat   2040
```

ggtaacacaa atgagttttg ctatctctct gagaagctca tctgacctcc tgactctcag 2100
ccctacagag tagggagttg atgctgacag gatgaagatt taggaataaa tatgcctggg 2160
aagagactgg gaaggttcta gggtgaggca cctcagtaac tcatggtacc ttggccaagt 2220
tggaaggaag cagtttgtta atgaggcaca gtaatcctgg ctgcagggtc taggaggtaa 2280
gaccagctgg gatgaccttc cctgggttaa tcaatttccc tctagacaac acaaactgca 2340
ggcatgtgac taactttgaa agaacaccca tcatgtggct gctgtcaccc ttgaccagcc 2400
gtggtggtgg ttactccatc tgtggttgga gcgcctcttt gggattcact tcaaggtctt 2460
gtgcctattt ttctgcatat cttctgtgat gacaaatctc tgtcccctga gtgttaattt 2520
gatttttaga aatggccaaa agtcacgtga tccaaacttt ttttcagtaa tatggagact 2580
gagctgcatg gtagttgggg atcaaaaata tgtgacctta atgagatttt tatgatttct 2640
aaagtaacaa taaaagcagc ttttagagtt gagttccaga gagggcaggg caatggcagt 2700
gacatgtttg tcattttaat aataaataac atctattgag tgctt 2745

<210> 119
<211> 2152
<212> DNA
<213> NM_138932.1| Homo sapiens apobec-1 complementation factor (ACF), transcript variant 2, mRNA

<400> 119

tttgatatga cgattagagc ataacccgag tgacacgttg aattcgccat aatcaaggaa 60
acctttttccg ggtggggatc tctgaaatta ctcagataac agtgctgtgc caaaaacctg 120
tggattttct ctacaaaaat tattgagcaa ccctaattaa cctgattttt tgctgataat 180
cactctcaat ggaatcaaat cacaaatccg gggatggatt gagcggcact cagaaggaag 240
cagccctccg cgcactggtc cagcgcacag gatatagctt ggtccaggaa aatggacaaa 300
gaaaatatgg tggccctcca cctggttggg atgctgcacc ccctgaaagg ggctgtgaaa 360
tttttattgg aaaacttccc cgagaccttt ttgaggatga gcttatacca ttatgtgaaa 420
aaatcggtaa aatttatgaa atgagaatga tgatggattt taatggcaac aatagaggat 480
atgcatttgt aacattttca aataaagtgg aagccaagaa tgcaatcaag caacttaata 540
attatgaaat tagaaatggg cgcctcttag ggtttgtgc cagtgtggac aactgccgat 600
tatttgttgg gggcatccca aaaaccaaaa agagagaaga aatcttatcg gagatgaaaa 660
aggttactga aggtgttgtc gatgtcatcg tctacccaag cgctgcagat aaaaccaaaa 720
accgaggctt tgccttcgtg gagtatgaga gtcatcgagc agctgccatg gcgaggagga 780
aactgctacc aggaagaatt cagttatggg gacatggtat tgcagtagac tgggcagagc 840
cagaagtaga agttgatgaa gatacaatgt cttcagtgaa aatcctatat gtaagaaatc 900

ttatgctgtc tacctctgaa gagatgattg aaaaggaatt caacaatatc aaaccaggtg 960
ctgtggagag ggtgaagaaa attcgagact atgcttttgt gcacttcagt aaccgagaag 1020
atgcagttga ggctatgaaa gctttaaatg gcaaggtgct ggatggttcc cccattgaag 1080
tcaccctagc aaaaccagtg gacaaggaca gttatgttag gtatacccga ggcacaggtg 1140
gaaggggcac catgctgcaa ggagagtata cctactcttt gggccaagtt tatgatccca 1200
ccacaaccta ccttggagct cctgtcttct atgcccccca gacctatgca gcaattccca 1260
gtcttcattt cccagccacc aaaggacatc tcagcaacag agccattatc cgagcccctt 1320
ctgttagaga aatttacatg aatgtacctg taggggctgc gggagtgaga ggactgggcg 1380
gccgtggcta tttggcatac acaggcctgg gtcgaggata ccaggtcaaa ggagacaaaa 1440
gagaagacaa actctatgac attttacctg ggatggagct caccccaatg aatcctgtca 1500
cattaaaacc ccaaggaatt aaactcgctc cccagatatt agaagagatt tgtcagaaaa 1560
ataactgggg acagccagtg taccagctgc actctgctat tggacaagac caaagacagc 1620
tattcttgta caaaataact attcctgctc tagccagcca gaatcctgca atccaccctt 1680
tcacacctcc aaagctgagt gcctttgtgg atgaagcaaa gacgtatgca gccgaataca 1740
ccctgcagac cctgggcatc cccactgatg gaggcgatgg caccatggct actgctgctg 1800
ctgctgctac tgctttccca ggatatgctg tccctaatgc aactgcaccc gtgtctgcag 1860
cccagctcaa gcaagcggta acccttggac aagacttagc agcatataca acctatgagg 1920
tctacccaac ttttgcagtg actgcccgag ggatggata tggcaccttc tgaagatgct 1980
tttttaaatt taagaataag acacacaaaa ctctattaaa aaaaaaaaag aaataaacct 2040
ctaactcggt ccccaatgat cataaataat atgtttccta aagaaatgcc tttccagaga 2100
ctgtatagct tataccaatt atagaatcat gaagtaaaaa aaaaaaaaaa aa 2152

<210> 120
<211> 3010
<212> DNA
<213> NM_145343.1| Homo sapiens apolipoprotein L, 1 (APOL1), transcript variant 2, mRNA

<400> 120

actttccctt tcgaattcct cggtatatct tggggactgg aggacctgtc tggttattat 60
acagacgcat aactggaggt gggatccaca cagctcagaa cagctggatc ttgctcagtc 120
tctgccaggg gaagattcct tgacttctgg ggtgatggag aagaaacagg ctgtgctgtg 180
tccctaatgg gaaacgtggc tgagacaggg gagtgagaag ggtgcgttgc agaatggtgc 240
ctgtggcatg atgccagctt tgcaatcatg agattcaaaa gccacactgt ggaattgagg 300
aggccctgca gcgacatgga gggagctgct ttgctgagag tctctgtcct ctgcatctgg 360

atgagtgcac ttttccttgg tgtgggagtg agggcagagg aagctggagc gagggtgcaa 420
caaaacgttc caagtgggac agatactgga gatcctcaaa gtaagcccct cggtgactgg 480
gctgctggca ccatggaccc agagagcagt atctttattg aggatgccat taagtatttc 540
aaggaaaaag tgagcacaca gaatctgcta ctcctgctga ctgataatga ggcctggaac 600
ggattcgtgg ctgctgctga actgcccagg aatgaggcag atgagctccg taaagctctg 660
gacaaccttg caagacaaat gatcatgaaa gacaaaaact ggcacgataa aggccagcag 720
tacagaaact ggtttctgaa agagtttcct cggttgaaaa gtgagcttga ggataacata 780
agaaggctcc gtgcccttgc agatggggtt cagaaggtcc acaaaggcac caccatcgcc 840
aatgtggtgt ctggctctct cagcatttcc tctggcatcc tgaccctcgt cggcatgggt 900
ctggcaccct tcacagaggg aggcagcctt gtactcttgg aacctgggat ggagttggga 960
atcacagccg ctttgaccgg gattaccagc agtaccatgg actacggaaa gaagtggtgg 1020
acacaagccc aagcccacga cctggtcatc aaaagccttg acaaattgaa ggaggtgagg 1080
gagtttttgg gtgagaacat atccaacttt ctttccttag ctggcaatac ttaccaactc 1140
acacgaggca ttgggaagga catccgtgcc ctcagacgag ccagagccaa tcttcagtca 1200
gtaccgcatg cctcagcctc acgcccccgg gtcactgagc caatctcagc tgaaagcggt 1260
gaacaggtgg agagggttaa tgaacccagc atcctggaaa tgagcagagg agtcaagctc 1320
acggatgtgg cccctgtaag cttctttctt gtgctggatg tagtctacct cgtgtacgaa 1380
tcaaagcact tacatgaggg ggcaaagtca gagacagctg aggagctgaa gaaggtggct 1440
caggagctgg aggagaagct aaacattctc aacaataatt ataagattct gcaggcggac 1500
caagaactgt gaccacaggg cagggcagcc accaggagag atatgcctgg caggggccag 1560
gacaaaatgc aaactttttt tttttctga gacagagtct tgctctgtcg ccaagttgga 1620
gtgcaatggt gcgatctcag ctcactgcaa gctctgcctc ccgtgttcaa gcgattctcc 1680
tgccttggcc tcccaagtag ctgggactac aggcgcctac caccatgccc agctaatttt 1740
tgtattttta atagagatgg ggtttcacca tgttggccag gatggtctcg atctcctgac 1800
ctcttgatct gcccaccttg gcctcccaaa gtgctgggat tacaggcgtg agccatcgct 1860
tttgacccaa atgcaaacat tttattaggg ggataaagag ggtgaggtaa agtttatgga 1920
actgagtgtt agggactttg gcatttccat agctgagcac agcaggggag gggttaatgc 1980
agatggcagt gcagcaagga gaaggcagga acattggagc ctgcaataag ggaaaaatgg 2040
gaactggaga gtgtggggaa tgggaagaag cagtttactt tagactaaag aatatattgg 2100
ggggccgggt gtagtggctc atgcctgtaa tccgagcact ttgggaggcc aaggcgggcg 2160
gatcacgagg tcaggagatc gagaccatcc tggctaacac agtgaaaccc cgtctctact 2220
aaaaatacaa aaaattagcc gggcatggtg gcgggcgcct gtagttccag ctaactgggc 2280
ggctgaggca ggagaatggc gtgaacctgg gaggtggagc ttgcagtgag ccgagatatc 2340

gccactgcac tccagcctgg gtgacagagc gagactccat ctcaaaaaaa aaaaaaaaaa 2400
gaatatattg acggaagaat agagaggagg cttgaaggaa ccagcaatga gaaggccagg 2460
aaaagaaaga gctgaaaatg gagaaagccc aagagttaga acagttggat acaggagaag 2520
aaacagcggc tccactacag acccagcccc aggttcaatg tcctccgaag aatgaagtct 2580
ttccctggtg atggtcccct gccctgtctt tccagcatcc actctccctt gtcctcctgg 2640
gggcatatct cagtcaggca gcggcttcct gatgatggtc attggggtgg ttgtcatgtg 2700
atgggtcccc tccaggttac taaagggtgc atgtcccctg cttgaacact gaagggcagg 2760
tggtgggcca tggccatggt ccccagctga ggagcaggtg tccctgagaa cccaaacttc 2820
ccagagagta tgtgagaacc aaccaatgaa aacagtccca tcgctcttac ccggtaagta 2880
aacagtcaga aaattagcat gaaagcagtt tagcattggg aggaagctca gatctctaga 2940
gctgtcttgt cgccgcccag gattgacctg tgtgtaagtc ccaataaact cacctactca 3000
tcaagctgga 3010

<210> 121
<211> 2759
<212> DNA
<213> NM_080796.1| Homo sapiens death associated transcription factor 1 (DATF1), transcript variant 2, mRNA

<400> 121

tctagccccg cgccatctcg gtggccgtcc gcccactccg cggcgttcgg ggaaatggct 60
gcgagacccct agaggcctgc ggcctgcgga gcttactcca cgggaacagc ctctagataa 120
tctgagttgt tgaaaatacg aagcctgtta ctcgtgaaca gtggctgaca acagtgttgt 180
tgtgagcctg gctgtctgct tggacccaga ggtttcgtct gccagggttt ttggttgtat 240
ttaggatttc agggaaaagt gtccaagctt tcagtgttgg agcaggtatg gacgacaaag 300
gcgacccgag caatgaggag gcacctaagg ccatcaaacc caccagcaaa gagttcagga 360
aaacatgggg ttttcgaagg accactatcg ccaagcgaga gggcgcaggg gacgcggagg 420
ctgacccact ggagccgcca cccccacagc agcagctggg cctgtccctg cggcgcagtg 480
ggaggcagcc caagcgcact gagcgcgtgg agcagttcct gaccattgcg cggcgccgcg 540
gcaggaggag catgcctgtc tccctggagg attctggtga gcccacgtcc tgccccgcca 600
cagacgccga gacagcctcc gagggcagcg tggaaagcgc ttctgagacc agaagcggcc 660
cccagtctgc ttccacagct gtgaaggaac gaccagcctc ttctgaaaag gtgaaaggag 720
gggatgacca cgatgacacc tccgatagtg acagcgatgg cctgaccttg aaagagcttc 780
agaatcgcct tcgcaggaag cgggaacagg agcccactga gaggcccctg aaagggatcc 840
agagtcgcct gcggaagaag cgccgggagg agggtcccgc cgagactgtg ggctccgagg 900

```
ccagtgacac tgtggagggc gtcctgccca gtaagcagga gcccgagaac gatcaggggg    960
ttgtgtccca ggctgggaaa gatgacagag agagtaagtt ggagggaaag gcggctcagg   1020
acatcaaaga tgaggagcct ggagacttgg gccgaccgaa gcctgaatgt gagggttacg   1080
accccaacgc cctgtattgc atttgccgcc agcctcacaa caacaggttt atgatttgct   1140
gtgaccgctg tgaagaatgg tttcatggcg attgtgtggg catttctgag gctcgaggga   1200
ggcttttgga aaggaatggg gaagactata tctgcccaaa ctgcaccatt ctgcaagtgc   1260
aggatgagac tcattcagaa acggcagatc agcaggaagc taaatggaga cctggagatg   1320
ctgatggcac cgattgtaca agtataggaa caatagagca gaagtctagc gaagaccaag   1380
ggataaaggg tagaattgag aaagctgcaa atccaagtgg caagaagaaa ctcaagatct   1440
tccagcctgt gatagaggcg cctggtgcct caaaatgtat tggccccggg tgctgtcacg   1500
tggcgcagcc cgactcggtg tactgcagta atgactgtat cctcaaacac gccgcagcga   1560
caatgaagtt tctaagctca ggtaaagaac agaagccaaa gcctaaagaa aagatgaaga   1620
tgaagccaga gaagcccagt cttccgaaat gcggtgctca ggcaggtatt aaaatctctt   1680
ctgtgcacaa gagaccagct ccagaaaaaa aagagaccac agtgaagaag gcagtggtgg   1740
tccctgcgcg gagtgaagca ctcgggaagg aagcagcttg tgagagcagc acgccgtcgt   1800
gggcgagcga tcacaattac aatgcagtaa agccagaaaa gactgctgct ccctcgccgt   1860
cactgttgta taaatgtatg tatcacctag gggttggcct cctggacccc tcccgttctt   1920
tctggatagc catcccctgg gcctgtccag gactgggagt tgcagctttg tgttaagctg   1980
atcacagaca ccggctgcac catcagcggg aagcagagcc catgtccagg atgcctcctg   2040
ctgccctgtg tccatcccta gtctgtcagg acttcctgtc actgttttcc aaagctgtaa   2100
acctcactgg tgaacgttca ccttaatgat tgattcttta atctctgttt tcactctcag   2160
gctctggtaa gtattcgtat tctcttcatc ccagtctgat tgcatagcca cactgcccgg   2220
cacgccacat ccacccctgt ctgcacatga gttgttctga caacagcgct gtatacgctt   2280
cagtttttcc acattgtcca cggccagcac atgaaagcat cacttctttt ttatgttgtg   2340
ggaatctttg caagttagtg ttgcatctga ttttcaggtg tacatttatt tttgactggg   2400
cagatagggg attttttttt ttccatgtcc gattcacacg ctacacaccc acatgaacac   2460
attcgaactt cgaaggccac acactcctgc ttcataggcc ccacggtaag tgagttcaca   2520
cctagaacac tgtcctgacc gcaggacgcg tgccttggac ttggtattct acatgtgact   2580
ggctttcttg ccctcgtctc ttgaatgttt agactcttaa gatcatatcc tgccccaaat   2640
ttcaaattaa tgaaatgaag atatttcaaa cagatctttg aaacctcaga ttctgtggtg   2700
caattttaat gttttcttgt ttctcagttt tctgctataa aactattttc aattcagtc    2759
```

<210> 122
<211> 781
<212> DNA
<213> NM_177953.1| Homo sapiens dynein, cytoplasmic, light polypeptide 2A (DNCL2A), transcript variant 2, mRNA

<400> 122

cgcagaaagg cacaggactc gctaagtgtt cgctacgcgg ggctaccgga tcggtcggaa 60
atggctgaag tggagagatc gcctgagccc aggaggtcaa ggctacagtg agccgtgact 120
gcaccactgc actccaccct gggcagaggt ggaggagaca ctgaagcgac tgcagagcca 180
gaagggagtg cagggaatca tcgtcgtgaa cacagaaggc attcccatca agagcaccat 240
ggacaacccc accaccaccc agtatgccag cctcatgcac agcttcatcc tgaaggcacg 300
gagcaccgtg cgtgacatcg acccccagaa cgatctcacc ttccttcgaa ttcgctccaa 360
gaaaaatgaa attatggttg caccagataa agactatttc ctgattgtga ttcagaatcc 420
aaccgaataa gccactctct tggctccctg tgtcattcct taatttaatg ccccccaaga 480
atgttaatgt caatcatgtc agtggactag cacatggcag tcgcttggaa cccactcaca 540
ccaatccagt gaccgtgtgt gggctggcgg ctcttctccc ccaccaacgg aacccctgtg 600
tgcaccaacc ttccccagag ctccggagcg ccctctcctc acttccaggt tttggagcaa 660
gagcttgcag gaagcccgca cccagcttcc ttctgacctt cagttcactt tgtcgccctt 720
ggagaaagct gtttttcttt aactaaaaat aaccaaaatg cttaaaaaaa aaaaaaaaaa 780
a 781

<210> 123
<211> 841
<212> DNA
<213> NM_022873.1| Homo sapiens interferon, alpha-inducible protein (clone IFI-6-16) (G1P3), transcript variant 3, mRNA

<400> 123

gaaccgttta ctcgctgctg tgcccatcta tcagcaggct ccgggctgaa gattgcttct 60
cttctctcct ccaaggtcta gtgacggagc ccgcgcgcgg cgccaccatg cggcagaagg 120
cggtatcgct ttcttgtgc tacctgctgc tcttcacttg cagtggggtg gaggcaggtg 180
agaatgcggg taaggatgca ggtaagaaaa agtgctcgga gagctcggac agcggctccg 240
ggttctggaa ggccctgacc ttcatggccg tcggaggagg actcgcagtc gccgggctgc 300
ccgcgctggg cttcaccggc gccggcatcg cggccaactc ggtggctgcc tcgctgatga 360
gctggtctgc gatcctgaat gggggcggcg tgcccgccgg ggggctagtg gccacgctgc 420
agagcctcgg ggctggtggc agcagcgtcg tcataggtaa tattggtgcc ctgatgggct 480
acgccaccca caagtatctc gatagtgagg aggatgagga gtagccagca gctcccagaa 540

cctcttcttc cttcttggcc taactcttcc agttaggatc tagaactttg cctttttttt 600
tttttttttt tttttttgag atgggttctc actatattgt ccaggctaga gtgcagtggc 660
tattcacaga tgcgaacata gtacactgca gcctccaact cctagcctca agtgatcctc 720
ctgtctcaac ctcccaagta ggattacaag catgcgccga cgatgcccag aatccagaac 780
tttgtctatc actctcccca acaacctaga tgtgaaaaca gaataaactt cacccagaaa 840
a 841

<210> 124
<211> 4652
<212> DNA
<213> NM_183047.1| Homo sapiens protein kinase C binding protein 1 (PRKCBP1), transcript variant 1, mRNA

<400> 124

gtgagaacta ggagcctgtc ctccatgttt tataagtatt gacattacac agtgttaaca 60
atgcatccac agagcttggc tgaagaggaa ataaaaacag aacaggaggt ggtagagggc 120
atggatatct ctactcgctc caaagatcct ggctctgcag agagaacagc ccagaaaaga 180
aagttcccca gccctccaca ttcttccaat ggccactcgc cgcaggacac atcaacaagc 240
cccattaaaa agaaaaagaa acctggctta ctgaacagta acaataagga gcagtcagaa 300
ctaagacatg gtccgtttta ctatatgaag cagccactca ccacagaccc tgttgatgtt 360
gtaccgcagg atggacggaa tgatttctac tgctgggttt gtcaccggga aggccaagtc 420
ctttgctgtg agctctgtcc ccgggtttat cacgctaagt gtctgagact gacatcggaa 480
ccagaggggg actggttttg tcctgaatgt gagaaaatta cagtagcaga atgcatcgag 540
acccagagta aagccatgac aatgctcacc attgaacagt tatcctacct gctcaagttt 600
gccattcaga aaatgaaaca gccagggaca gatgcattcc agaagcccgt tccattggaa 660
cagcaccctg actatgcgga atacatcttc catccaatgg acctttgtac attggaaaag 720
aatgcgaaaa agaaaatgta tggctgcaca gaagccttcc tggctgatgc aaagtggatt 780
ttgcacaact gcatcattta taatggggga aatcacaaat tgacgcaaat agcgaaagta 840
gtcatcaaaa tctgtgaaca tgagatgaat gaaatcgaag tatgtccaga atgttatcta 900
gctgcttgcc aaaaacgaga taactggttt tgtgagcctt gtagcaatcc acatcctttg 960
gtctgggcca aactgaaggg gtttccattc tggcctgcaa aagctctaag ggataaagac 1020
gggcaggtcg atgcccgatt ctttggacaa catgacaggg cctgggttcc aataaataat 1080
tgctacctca tgtctaaaga aattcctttt tctgtgaaaa agactaagag catcttcaac 1140
agtgccatgc aagagatgga ggtttacgtg gagaacatcc gcaggaagtt tggggttttt 1200
aattactctc catttaggac accctacaca cccaacagcc agtatcaaat gctgctcgat 1260

cccaccaacc ccagcgccgg cactgccaag atagacaagc aggagaaggt caagctcaac 1320
tttgacatga cggcatcccc caagatcctg atgagcaagc ctgtgctgag tgggggcaca 1380
ggccgccgga tttccttgtc ggatatgccg cgctccccca tgagcacaaa ctcttctgtg 1440
cacacgggct ccgacgtgga gcaggatgct gagaagaagg ccacgtcgag ccacttcagt 1500
gcgagcgagg agtccatgga cttcctggat aagagcacag cttcaccagc ctccaccaag 1560
acgggacaag cagggagttt atccggcagc ccaaagccct tctctcctca actgtcagct 1620
cctatcacga cgaaaacgga caaaacctcc accaccggca gcatcctgaa tcttaacctg 1680
gatcgaagca aagctgagat ggatttgaag gagctgagcg agtcggtcca gcaacagtcc 1740
acccctgttc ctctcatctc tcccaagcgc cagattcgta gcaggttcca gctgaatctt 1800
gacaagacca tagagagttg caaagcacaa ttaggcataa atgaaatctc ggaagatgtc 1860
tatacggccg tagagcacag cgattcggag gattctgaga agtcagatag tagcgatagt 1920
gagtatatca gtgatgatga gcagaagtct aagaacgagc cagaagacac agaggacaaa 1980
gaaggttgtc agatggacaa agagccatct gctgttaaaa aaaagcccaa gcctacaaac 2040
ccagtggaga ttaaagagga gctgaaaagc acgtcaccag ccagcgagaa ggcagaccct 2100
ggagcagtca aggacaaggc cagccctgag cctgagaagg acttttccga aaaggcaaaa 2160
ccttcacctc accccataaa ggataaactg aagggaaaag atgagacgga ttccccaaca 2220
gtccatttgg gcctggactc tgattcagag agcgaacttg tcatagattt aggagaagac 2280
cattctgggc gggagggtcg aaaaaataag aaggaaccca aagaaccatc tcccaaacag 2340
gatgttgtag gtaaaactcc accatccacg acggtgggca gccattctcc cccggaaaca 2400
ccggtgctca cccgctcttc cgcccaaact tccgcggctg gcgccacagc caccaccagc 2460
acgtcctcca cggtcaccgt cacggccccg gcccccgccg ccacaggaag cccagtgaaa 2520
aagcagaggc cgcttttacc gaaggagact gccccggccg tgcagcgggt cgtgtggaac 2580
tcatcaactg tccagcagaa ggagatcaca cagagcccat ccacgtccac catcaccctg 2640
gtgaccagca cacagtcatc gcccctggtc accagctcgg ggtccatgag caccccttgtg 2700
tcctcagtca acgctgacct gcccatcgcc actgcctcag ctgatgtcgc cgctgatatt 2760
gccaagtaca ctagcaaaat gatggatgca ataaaaggaa caatgacaga aatatacaac 2820
gatctttcta aaaacactac tggaagcaca atagctgaga ttcgcaggct gaggatcgag 2880
atagagaagc tccagtggct gcaccagcaa gagctctccg aaatgaaaca caacttagag 2940
ctgaccatgg cggagatgcg gcagagcctg gagcaggagc gggaccggct catcgccgag 3000
gtgaagaagc agctggagtt ggagaagcag caggcggtgg atgagaccaa gaagaagcag 3060
tggtgcgcca actgcaagaa ggaggccatc ttttactgct gttggaacac tagctactgt 3120
gactacccct gccagcaagc ccactggcct gagcacatga agtcctgcac ccagtcagct 3180
actgctcctc agcaggaagc ggatgctgag gtgaacacag aaacactaaa taagtcctcc 3240
caggggagct cctcgagcac acaatcagca ccttcagaaa cggccagcgc ctccaaagag 3300

aaggagacgt cagctgagaa aagcaaggag agtggctcga cccttgacct ttctggctcc 3360
agagagacgc cctcctccat tctcttaggc tccaaccaag gctctgttag caaaaggtgt 3420
gacaagcaac ctgcctatgc cccaaccacc acagaccacc agccgcaccc caactacccc 3480
gcccagaagt accattcccg gagtaataaa tccagttgga gcagcagtga tgagaagagg 3540
ggatcgacac gttccgatca caacaccagt accagcacga agagcctcct cccgaaagag 3600
tctcggctgg acaccttctg ggactagcag tgaatcggga cacaaaccac ccaccccatt 3660
gggagaaaaa cccagacgcc aggaaaagaa gaaacaacaa aggcaggaga acagccactt 3720
tcagacttga aaatgacaaa accctcagtt gagcctgagc cccggcgcg ggggctgcta 3780
cactacagga cacccagcat cggctttgac tgcagactgt tcacccacac gagccctgtg 3840
cttttggtgt aaataatgta caatttgtgg atgtcattga atctagagga ctttcccctt 3900
tttatatttg tattaacttt aacttattaa aaaaaaaaaa agaaaaagaa aaacgattta 3960
aaaaaaaaaa aaaaagcaac caaccccaac aacaaaaaag aatgttttgg tattggagaa 4020
gggatggtca gttagcctgt ctgtcacacg acggaatgga tactgggccc ggggaccact 4080
ttcatactca cgtcctcatc cttggatacc caggggaggg cgaaccgttt tcgctcgtgt 4140
gtctgtacgc agcatgttgg gatcgggagt ttcggcacag actatcccat caagccgttg 4200
gctcctttca gctactacgt taccacgttc ctaaaacgca agctctccgg accagacgga 4260
cacagggaga agctagtttc tttcatgtga ttgaaatgat gactctactc ctaaaaggga 4320
aaaaacaata tccttgttta cagaagagaa acaaacaagc cccactcagc tcagtcacag 4380
gagagaacac agaaagtctt aggatcatga actctgaaaa aaagagaaac cttatctttg 4440
ctttgtggtt cctttaaaca cactcacaca cacttggtca gagatgctgt gcttcttgga 4500
agcaaggact caaaggcaag gtgcacgcag aggacgtttg agtctgggat gaagcatgta 4560
cgtattattt atatgatgga atttcacgtt tttatgtaag catgaaacac aggcagtatg 4620
agagaaagca aggcccgtca tgctgtccgt ac 4652

<210> 125
<211> 3217
<212> DNA
<213> NM_017452.1| Homo sapiens staufen, RNA binding protein (Drosophila) (STAU), transcript variant T2, mRNA

<400> 125

acttcctgcc gggctgcggg cgcctgagcg ctcttcagcg tttgcgcggc ggctgcgcgt 60
ctctctcggc tcccgcttcc tttgaccgcc tcccccccc ggcccggcgg cgcccgcctc 120
ctccacggcc actccgcctc ttccctccct tcgtcccttc ttcctctccc ttttttcctt 180
cttccttccc ctcctcgccg ccaccgccca ggaccgccgg ccgggggacg agtccggagc 240

agcagccaga gtttattaac cacttaacct ctcagaactg aacaaagaca acattgttcc 300
tggaacgccc tctttttaaa aaagaaagca taacccctac tgtagaacta aatgcactgt 360
gcatgaaact tggaaaaaaa ccaatgtata agcctgttga cccttactct cggatgcagt 420
ccacctataa ctacaacatg agaggaggtg cttatccccc gaggtacttt tacccatttc 480
cagttccacc tttactttat caagtggaac tttctgtggg aggacagcaa tttaatggca 540
aaggaaagac aagacaggct gcgaaacacg atgctgctgc caaagcgttg aggatcctgc 600
agaatgagcc cctgccagag aggctggagg tgaatggaag agaatccgaa gaagaaaatc 660
tcaataaatc tgaaataagt caagtgtttg agattgcact taaacggaac ttgcctgtga 720
atttcgaggt ggcccgggag agtggcccac cccacatgaa gaactttgtg accaaggttt 780
cggttgggga gtttgtgggg gaaggtgaag ggaaaagcaa gaagatttca aagaaaaatg 840
ccgccatagc tgttcttgag gagctgaaga agttaccgcc cctgcctgca gttgaacgag 900
taaagcctag aatcaaaaag aaaacaaaac ccatagtcaa gccacagaca agcccagaat 960
atggccaggg gatcaatccg attagccgac tggcccagat ccagcaggca aaaaaggaga 1020
aggagccaga gtacacgctc ctcacagagc gaggcctccc gcgccgcagg gagtttgtga 1080
tgcaggtgaa ggttggaaac cacactgcag aaggaacggg caccaacaag aaggtggcca 1140
agcgcaatgc agccgagaac atgctggaga tccttggttt caaagtcccg cagcggcagc 1200
ccaccaaacc cgcactcaag tcagaggaga agacacccat aaagaaacca ggggatggaa 1260
gaaaagtaac ctttttgaa cctggctctg gggatgaaaa tgggactagt aataaagagg 1320
atgagttcag gatgccttat ctaagtcatc agcagctgcc tgctggaatt cttcccatgg 1380
tgcccgaggt cgcccaggct gtaggagtta gtcaaggaca tcacaccaaa gattttacca 1440
gggcagctcc gaatcctgcc aaggccacgg taactgccat gatagcccga gagttgttgt 1500
atgggggcac ctcgcccaca gccgagacca ttttaaagaa taacatctct tcaggccacg 1560
taccccatgg acctctcacg agaccctctg agcaactgga ctatctttcc agagtccagg 1620
gattccaggt tgaatacaaa gacttcccca aaaacaacaa gaacgaattt gtatctctta 1680
tcaattgctc ctctcagcca cctctgatca gccatggtat cggcaaggat gtggagtcct 1740
gccatgatat ggctgcgctg aacatcttaa agttgctgtc tgagttggac caacaaagta 1800
cagagatgcc aagaacagga aacggaccaa tgtctgtgtg tgggaggtgc tgaacctttt 1860
ctggccatga accattataa aatcccaaca tatatactga aaatactgaa actgctttga 1920
aaatttggaa tttctgatac ctccagtggg ccgagagaca cggtgggtaa aggatgtggg 1980
cagcagcagg gaagacaaca gaaacacaag gaggcggctg tggccggctg gactgtgctg 2040
gggtttgttg tgatggccac tcggtgacct ggcggtccct acgcaatagc agctgcctgt 2100
ggggaagaag ggctgcccag ccagctggtt ctcccgggac accagcagat ccacaccctg 2160
ggcacctccg tgtttggtct tttttttccc ctgtgtgaaa gaagaaacgg cacgacccct 2220

tctcaagctg gctcactcag acacattggg acaaaccctg gacagccatg ccagagagag 2280
gcctttgacc ggccccagag ctaaaagcac cagagaaaat caaatgcttc ctactcagcg 2340
tgacccaact tttctagtgt gccacggccc caccacctcc tgcagtaccc acaccatcac 2400
cactgctttc tcttccaaca gtgatctgta ttcttagttt cattattttc ttttgattga 2460
tatgacacta tataaaattt tcatttgaga atttctcaat tgtatctagt taaatagcac 2520
agtttggaaa cttgtctgag actgacttta tcaataatct aaccgacaaa gatcatatcc 2580
atgtgtatgt ggttagacat ttttatttca ttgactaacc caggacagtt tcagtgatgc 2640
aaattgtgtg ccctctggtt cagctgaaac agtcctggac tttcaaaaac cttgaataag 2700
tctcccacag ttgtataaat tggacaattt aggaatttta aactttagat gatcatttgg 2760
ttccattttt atttcatttt tatttttgtt aatgcaaaca ggacttaaat gaactttgat 2820
ctctgtttta aagattatta aaaaacattg tgtatctata catatggctc ttgaggactt 2880
agctttcact acactacagg atatgatctc catgtagtcc atataaacct gcagagtgat 2940
tttccagagt gctcgatact gttaattaca tctccattag ggctgaaaag aatgacctac 3000
gtttctgtat acagctgtgt tgcttttgat gttgtgttac tgtacacaga agtgtgtgca 3060
ctgaggctct gcgtgtggtc cgtatggaaa acctggtagc cctgcgagtt aagtactgct 3120
tccattcatt gtttacgctg gaatttttct ccccatggaa tgtaagtaaa acttaagtgt 3180
ttgtcatcaa taaatggtaa tactaaaaaa aaaaaaa 3217

<210> 126
<211> 3506
<212> DNA
<213> NM_017453.1| Homo sapiens staufen, RNA binding protein (Drosophila) (STAU), transcript variant T3, mRNA

<400> 126

acttcctgcc gggctgcggg cgcctgagcg ctcttcagcg tttgcgcggc ggctgcgcgt 60
ctctctcggc tcccgcttcc tttgaccgcc tccccccccc ggcccggcgg cgcccgcctc 120
ctccacggcc actccgcctc ttccctccct tcgtcccttc ttcctctccc ttttttcctt 180
cttccttccc ctcctcgccg ccaccgccca ggaccgccgg ccgggggacg agtccggagc 240
agcagccaga gtttattaac cacttaacct ctcagaactg aacaaagaca acattgttcc 300
tggaacgccc tctttttaaa aaaggtagaa ctttagactt catagcactg aattaacctg 360
cactgaaagc tgtttacctg catttgttca cttttgttga aagtgaccat gtctcaagtt 420
caagtgcaag ttcagaaccc atctgctgct ctctcaggga gccaaatact gaacaagaac 480
cagtctcttc tctcacagcc tttgatgagt attccttcta ctactagctc tctgccctct 540
gaaaatgcag gtagacccat tcaaaactct gctttaccct ctgcatctat tacatccacc 600

agtgcagctg cagaaagcat aacccctact gtagaactaa atgcactgtg catgaaactt 660
ggaaaaaaac caatgtataa gcctgttgac ccttactctc ggatgcagtc cacctataac 720
tacaacatga gaggaggtgc ttatccccg aggtactttt acccatttcc agttccacct 780
ttactttatc aagtggaact ttctgtggga ggacagcaat ttaatggcaa aggaaagaca 840
agacaggctg cgaaacacga tgctgctgcc aaagcgttga ggatcctgca gaatgagccc 900
ctgccagaga ggctggaggt gaatggaaga gaatccgaag aagaaaatct caataaatct 960
gaaataagtc aagtgtttga gattgcactt aaacggaact tgcctgtgaa tttcgaggtg 1020
gcccgggaga gtggcccacc ccacatgaag aactttgtga ccaaggtttc ggttggggag 1080
tttgtggggg aaggtgaagg gaaaagcaag aagatttcaa agaaaaatgc cgccatagct 1140
gttcttgagg agctgaagaa gttaccgccc ctgcctgcag ttgaacgagt aaagcctaga 1200
atcaaaaaga aaacaaaacc catagtcaag ccacagacaa gcccagaata tggccagggg 1260
atcaatccga ttagccgact ggcccagatc cagcaggcaa aaaaggagaa ggagccagag 1320
tacacgctcc tcacagagcg aggcctcccg cgccgcaggg agtttgtgat gcaggtgaag 1380
gttggaaacc acactgcaga aggaacgggc accaacaaga aggtggccaa gcgcaatgca 1440
gccgagaaca tgctggagat ccttggtttc aaagtcccgc agcggcagcc caccaaaccc 1500
gcactcaagt cagaggagaa gacacccata aagaaaccag gggatggaag aaaagtaacc 1560
ttttttgaac ctggctctgg ggatgaaaat gggactagta ataaagagga tgagttcagg 1620
atgccttatc taagtcatca gcagctgcct gctggaattc ttcccatggt gcccgaggtc 1680
gcccaggctg taggagttag tcaaggacat cacaccaaag attttaccag ggcagctccg 1740
aatcctgcca aggccacggt aactgccatg atagcccgag agttgttgta tgggggcacc 1800
tcgcccacag ccgagaccat tttaaagaat aacatctctt caggccacgt accccatgga 1860
cctctcacga gaccctctga gcaactggac tatctttcca gagtccaggg attccaggtt 1920
gaatacaaag acttccccaa aaacaacaag aacgaatttg tatctcttat caattgctcc 1980
tctcagccac ctctgatcag ccatggtatc ggcaaggatg tggagtcctg ccatgatatg 2040
gctgcgctga acatcttaaa gttgctgtct gagttggacc aacaaagtac agagatgcca 2100
agaacaggaa acggaccaat gtctgtgtgt gggaggtgct gaaccttttc tggccatgaa 2160
ccattataaa atcccaacat atatactgaa aatactgaaa ctgctttgaa aatttggaat 2220
ttctgatacc tccagtgggc cgagagacac ggtgggtaaa ggatgtgggc agcagcaggg 2280
aagacaacag aaacacaagg aggcggctgt ggccggctgg actgtgctgg ggtttgttgt 2340
gatggccact cggtgacctg gcggtcccta cgcaatagca gctgcctgtg gggaagaagg 2400
gctgcccagc cagctggttc tcccgggaca ccagcagatc cacaccctgg gcacctccgt 2460
gtttggtctt ttttttcccc tgtgtgaaag aagaaacggc acgacccctt ctcaagctgg 2520
ctcactcaga cacattggga caaaccctgg acagccatgc cagagagagg cctttgaccg 2580
gccccagagc taaaagcacc agagaaaatc aaatgcttcc tactcagcgt gacccaactt 2640

ttctagtgtg ccacggcccc accacctcct gcagtaccca caccatcacc actgctttct 2700
cttccaacag tgatctgtat tcttagtttc attattttct tttgattgat atgacactat 2760
ataaaatttt catttgagaa tttctcaatt gtatctagtt aaatagcaca gtttggaaac 2820
ttgtctgaga ctgactttat caataatcta accgacaaag atcatatcca tgtgtatgtg 2880
gttagacatt tttatttcat tgactaaccc aggacagttt cagtgatgca aattgtgtgc 2940
cctctggttc agctgaaaca gtcctggact ttcaaaaacc ttgaataagt ctcccacagt 3000
tgtataaatt ggacaattta ggaattttaa actttagatg atcatttggt tccattttta 3060
tttcattttt atttttgtta atgcaaacag gacttaaatg aactttgatc tctgttttaa 3120
agattattaa aaaacattgt gtatctatac atatggctct tgaggactta gctttcacta 3180
cactacagga tatgatctcc atgtagtcca tataaacctg cagagtgatt ttccagagtg 3240
ctcgatactg ttaattacat ctccattagg gctgaaaaga atgacctacg tttctgtata 3300
cagctgtgtt gcttttgatg ttgtgttact gtacacagaa gtgtgtgcac tgaggctctg 3360
cgtgtggtcc gtatggaaaa cctggtagcc ctgcgagtta agtactgctt ccattcattg 3420
tttacgctgg aatttttctc cccatggaat gtaagtaaaa cttaagtgtt tgtcatcaat 3480
aaatggtaat actaaaaaaa aaaaaa 3506

<210> 127
<211> 4538
<212> DNA
<213> NM_199169.1| Homo sapiens transmembrane, prostate androgen induced RNA (TMEPAI), transcript variant 2, mRNA

<400> 127

tggtcgtcct ccttgggttc gggtgaaagc gcttgggggt tcagtgggcc atgatccccg 60
agctgctgga gaactgaagg cggacagtct cctgcgaaac caggcaatgg cggagctgga 120
gtttgttcag atcatcatca tcgtggtggt gatgatggtg atggtggtgg tgatcacgtg 180
cctgctgagc cactacaagc tgtctgcacg gtccttcatc agccggcaca gccaggggcg 240
gaggagagaa gatgccctgt cctcagaagg atgcctgtgg ccctcggaga gcacagtgtc 300
aggcaacgga atcccagagc cgcaggtcta cgccccgcct cggcccaccg accgcctggc 360
cgtgccgccc ttcgcccagc gggagcgctt ccaccgcttc cagcccacct atccgtacct 420
gcagcacgag atcgacctgc cacccaccat ctcgctgtca gacggggagg agccccccacc 480
ctaccagggc ccctgcaccc tccagcttcg ggaccccgag cagcagctgg aactgaaccg 540
ggagtcggtg cgcgcacccc caaacagaac catcttcgac agtgacctga tggatagtgc 600
caggctgggc ggcccctgcc cccccagcag taactcgggc atcagcgcca cgtgctacgg 660
cagcggcggg cgcatggagg ggccgccgcc cacctacagc gaggtcatcg gccactaccc 720

ggggtcctcc ttccagcacc agcagagcag tgggccgccc tccttgctgg aggggacccg 780
gctccaccac acacacatcg cgcccctaga gagcgcagcc atctggagca aagagaagga 840
taaacagaaa ggacaccctc tctagggtcc ccaggggggc cgggctgggg ctgcgtaggt 900
gaaaaggcag aacactccgc gcttcttaga agaggagtga gaggaaggcg gggggcgcag 960
caacgcatcg tgtggccctc ccctcccacc tccctgtgta taaatattta catgtgatgt 1020
ctggtctgaa tgcacaagct aagagagctt gcaaaaaaaa aaagaaaaaa gaaaaaaaaa 1080
aaccacgttt ctttgttgag ctgtgtcttg aaggcaaaag aaaaaaaatt tctacagtag 1140
tctttcttgt ttctagttga gctgcgtgcg tgaatgctta ttttcttttg tttatgataa 1200
tttcacttaa ctttaaagac atatttgcac aaaacctttg tttaaagatc tgcaatatta 1260
tatatataaa tatatataag ataagagaaa ctgtatgtgc gagggcagga gtatttttgt 1320
attagaagag gcctattaaa aaaaaaagtt gttttctgaa ctagaagagg aaaaaaatgg 1380
caatttttga gtgccaagtc agaaagtgtg tattaccttg taaagaaaaa aattacaaag 1440
caggggttta gagttattta tataaatgtt gagattttgc actatttttt aatataaata 1500
tgtcagtgct tgcttgatgg aaacttctct tgtgtctgtt gagactttaa gggagaaatg 1560
tcggaatttc agagtcgcct gacggcagag ggtgagcccc cgtggagtct gcagagaggc 1620
cttggccagg agcggcgggc tttcccgagg ggccactgtc cctgcagagt ggatgcttct 1680
gcctagtgac aggttatcac cacgttatat attccctacc gaaggagaca ccttttcccc 1740
cctgacccag aacagccttt aaatcacaag caaaatagga aagttaacca cggaggcacc 1800
gagttccagg tagtggtttt gcctttccca aaaatgaaaa taaactgtta ccgaaggaat 1860
tagtttttcc tcttcttttt tccaactgtg aaggtccccg tggggtggag catggtgccc 1920
ctcacaagcc gcagcggctg gtgcccgggc taccagggac atgccagagg gctcgatgac 1980
ttgtctctgc agggcgcttt ggtggttgtt cagctggcta aaggttcacc ggtgaaggca 2040
ggtgcggtaa ctgccgcact ggaccctagg aagccccagg tattcgcaat ctgacctcct 2100
cctgtctgtt tcccttcacg gatcaattct cacttaagag gccaataaac aacccaacat 2160
gaaaaggtga caagcctggg tttctcccag gataggtgaa agggttaaaa tgagtaaagc 2220
agttgagcaa acaccaaccc gagcttcggg cgcagaattc ttcaccttct cttccccttt 2280
ccatctcctt tccccgcgga aacaacgctt cccttctggt gtgtctgttg atctgtgttt 2340
tcatttacat ctctcttaga ctccgctctt gttctccagg ttttcaccag atagatttgg 2400
ggttggcggg acctgctggt gacgtgcagg tgaaggacag gaaggggcat gtgagcgtaa 2460
atagaggtga ccagaggaga gcatgagggg tggggctttg ggacccaccg gggccagtgg 2520
ctggagcttg acgtctttcc tccccatggg ggtgggaggg cccccagctg gaagagcaga 2580
ctcccagctg ctaccccctc ccttcccatg ggagtggctt tccattttgg gcagaatgct 2640
gactagtaga ctaacataaa agatataaaa ggcaataact attgtttgtg agcaactttt 2700

ttataacttc caaaacaaaa acctgagcac agttttgaag ttctagccac tcgagctcat 2760
gcatgtgaaa cgtgtgcttt acgaaggtgg cagctgacag acgtgggctc tgcatgccgc 2820
cagcctagta gaaagttctc gttcattggc aacagcagaa cctgcctctc cgtgaagtcg 2880
tcagcctaaa atttgtttct ctcttgaaga ggattctttg aaaaggtcct gcagagaaat 2940
cagtacaggt tatcccgaaa ggtacaagga cgcacttgta aagatgatta aaacgtatct 3000
ttcctttatg tgacgcgtct ctagtgcctt actgaagaag cagtgacact cccgtcgctc 3060
ggtgaggacg ttcccggaca gtgcctcact cacctgggac tggtatcccc tcccagggtc 3120
caccaagggc tcctgctttt cagacacccc atcatcctcg cgcgtcctca ccctgtctct 3180
accagggagg tgcctagctt ggtgaggtta ctcctgctcc tccaaccttt ttttgccaag 3240
gtttgtacac gactcccatc taggctgaaa acctagaagt ggaccttgtg tgtgtgcatg 3300
gtgtcagccc aaagccaggc tgagacagtc ctcatatcct cttgagccaa actgtttggg 3360
tctcgttgct tcatggtatg gtctggattt gtgggaatgg ctttgcgtga gaaaggggag 3420
gagagtggtt gctgccctca gccggcttga ggacagagcc tgtccctctc atgacaactc 3480
agtgttgaag cccagtgtcc tcagcttcat gtccagtgga tggcagaagt tcatggggta 3540
gtggcctctc aaaggctggg cgcatcccaa gacagccagc aggttgtctc tggaaacgac 3600
cagagttaag ctctcggctt ctctgctgag ggtgcaccct ttcctctaga tggtagttgt 3660
cacgttatct ttgaaaactc ttggactgct cctgaggagg ccctcttttc cagtaggaag 3720
ttagatgggg gttctcagaa gtggctgatt ggaaggggac aagcttcgtt tcaggggtct 3780
gccgttccat cctggttcag agaaggccga gcgtggcttt ctctagcctt gtcactgtct 3840
ccctgcctgt caatcaccac ctttcctcca gaggaggaaa attatctccc ctgcaaagcc 3900
cggttctaca cagatttcac aaattgtgct aagaaccgtc cgtgttctca gaaagcccag 3960
tgtttttgca aagaatgaaa agggacccca tatgtagcaa aaatcagggc tgggggagag 4020
ccgggttcat tccctgtcct cattggtcgt ccctatgaat tgtacgtttc agagaaattt 4080
tttttcctat gtgcaacacg aagcttccag aaccataaaa tatcccgtcg ataaggaaag 4140
aaaatgtcgt tgttgttgtt tttctggaaa ctgcttgaaa tcttgctgta ctatagagct 4200
cagaaggaca cagcccgtcc tcccctgcct gcctgattcc atggctgttg tgctgattcc 4260
aatgctttca cgttggttcc tggcgtggga actgctctcc tttgcagccc catttcccaa 4320
gctctgttca agttaaactt atgtaagctt tccgtggcat gcggggcgcg cacccacgtc 4380
cccgctgcgt aagactctgt atttggatgc caatccacag gcctgaagaa actgcttgtt 4440
gtgtatcagt aatcattagt ggcaatgatg acattctgaa aagctgcaat acttatacaa 4500
taaattttac aattctttgg aaaaaaaaaa aaaaaaa 4538

<210> 128
<211> 4531
<212> DNA
<213> NM_199170.1| Homo sapiens transmembrane, prostate androgen induced RNA (TMEPAI), transcript variant 3, mRNA

<400> 128

tggtcgtcct ccttgggttc gggtgaaagc gcttgggggt tcagtgggcc atgatccccg 60
agctgctgga gaactgaagg cggacagtct cctgcgaaac cagcggagct ggagtttgtt 120
cagatcatca tcatcgtggt ggtgatgatg gtgatggtgg tggtgatcac gtgcctgctg 180
agccactaca agctgtctgc acggtccttc atcagccggc acagccaggg gcggaggaga 240
gaagatgccc tgtcctcaga aggatgcctg tggccctcgg agagcacagt gtcaggcaac 300
ggaatcccag agccgcaggt ctacgccccg cctcggccca ccgaccgcct ggccgtgccg 360
cccttcgccc agcgggagcg cttccaccgc ttccagccca cctatccgta cctgcagcac 420
gagatcgacc tgccacccac catctcgctg tcagacgggg aggagccccc accctaccag 480
ggcccctgca ccctccagct tcgggacccc gagcagcagc tggaactgaa ccgggagtcg 540
gtgcgcgcac cccccaaacag aaccatcttc gacagtgacc tgatggatag tgccaggctg 600
ggcggcccct gcccccccag cagtaactcg ggcatcagcg ccacgtgcta cggcagcggc 660
gggcgcatgg aggggccgcc gcccacctac agcgaggtca tcggccacta cccggggtcc 720
tccttccagc accagcagag cagtgggccg ccctccttgc tggaggggac ccggctccac 780
cacacacaca tcgcgcccct agagagcgca gccatctgga gcaaagagaa ggataaacag 840
aaaggacacc ctctctaggg tccccagggg ggccgggctg gggctgcgta ggtgaaaagg 900
cagaacactc cgcgcttctt agaagaggag tgagaggaag gcgggggcg cagcaacgca 960
tcgtgtggcc ctcccctccc acctccctgt gtataaatat ttacatgtga tgtctggtct 1020
gaatgcacaa gctaagagag cttgcaaaaa aaaaaagaaa aaagaaaaaa aaaaaccacg 1080
tttctttgtt gagctgtgtc ttgaaggcaa aagaaaaaaa atttctacag tagtctttct 1140
tgtttctagt tgagctgcgt gcgtgaatgc ttattttctt ttgtttatga taatttcact 1200
taactttaaa gacatatttg cacaaaacct ttgtttaaag atctgcaata ttatatatat 1260
aaatatatat aagataagag aaactgtatg tgcgagggca ggagtatttt tgtattagaa 1320
gaggcctatt aaaaaaaaaa gttgttttct gaactagaag aggaaaaaaa tggcaatttt 1380
tgagtgccaa gtcagaaagt gtgtattacc ttgtaaagaa aaaaattaca aagcaggggt 1440
ttagagttat ttatataaat gttgagattt tgcactattt tttaatataa atatgtcagt 1500
gcttgcttga tggaaacttc tcttgtgtct gttgagactt taagggagaa atgtcggaat 1560
ttcagagtcg cctgacggca gagggtgagc cccgtggag tctgcagaga ggccttggcc 1620
aggagcggcg ggctttcccg aggggccact gtccctgcag agtggatgct tctgcctagt 1680
gacaggttat caccacgtta tatattccct accgaaggag acaccttttc ccccctgacc 1740
cagaacagcc tttaaatcac aagcaaaata ggaaagttaa ccacggaggc accgagttcc 1800

aggtagtggt tttgcctttc ccaaaaatga aaataaactg ttaccgaagg aattagtttt 1860
tcctcttctt ttttccaact gtgaaggtcc ccgtggggtg gagcatggtg cccctcacaa 1920
gccgcagcgg ctggtgcccg ggctaccagg gacatgccag agggctcgat gacttgtctc 1980
tgcagggcgc tttggtggtt gttcagctgg ctaaaggttc accggtgaag gcaggtgcgg 2040
taactgccgc actggaccct aggaagcccc aggtattcgc aatctgacct cctcctgtct 2100
gtttcccttc acggatcaat tctcacttaa gaggccaata aacaacccaa catgaaaagg 2160
tgacaagcct gggtttctcc caggataggt gaaagggtta aaatgagtaa agcagttgag 2220
caaacaccaa cccgagcttc gggcgcagaa ttcttcacct tctcttcccc tttccatctc 2280
ctttccccgc ggaaacaacg cttcccttct ggtgtgtctg ttgatctgtg ttttcattta 2340
catctctctt agactccgct cttgttctcc aggttttcac cagatagatt tggggttggc 2400
gggacctgct ggtgacgtgc aggtgaagga caggaagggg catgtgagcg taaatagagg 2460
tgaccagagg agagcatgag gggtggggct ttgggaccca ccggggccag tggctggagc 2520
ttgacgtctt tcctccccat gggggtggga gggcccccag ctggaagagc agactcccag 2580
ctgctacccc ctcccttccc atgggagtgg ctttccattt tgggcagaat gctgactagt 2640
agactaacat aaaagatata aaaggcaata actattgttt gtgagcaact tttttataac 2700
ttccaaaaca aaaacctgag cacagttttg aagttctagc cactcgagct catgcatgtg 2760
aaacgtgtgc tttacgaagg tggcagctga cagacgtggg ctctgcatgc cgccagccta 2820
gtagaaagtt ctcgttcatt ggcaacagca gaacctgcct ctccgtgaag tcgtcagcct 2880
aaaatttgtt tctctcttga agaggattct ttgaaaaggt cctgcagaga aatcagtaca 2940
ggttatcccg aaaggtacaa ggacgcactt gtaaagatga ttaaaacgta tctttccttt 3000
atgtgacgcg tctctagtgc cttactgaag aagcagtgac actcccgtcg ctcggtgagg 3060
acgttcccgg acagtgcctc actcacctgg gactggtatc ccctcccagg gtccaccaag 3120
ggctcctgct tttcagacac cccatcatcc tcgcgcgtcc tcaccctgtc tctaccaggg 3180
aggtgcctag cttggtgagg ttactcctgc tcctccaacc tttttttgcc aaggtttgta 3240
cacgactccc atctaggctg aaaacctaga agtggacctt gtgtgtgtgc atggtgtcag 3300
cccaaagcca ggctgagaca gtcctcatat cctcttgagc caaactgttt gggtctcgtt 3360
gcttcatggt atggtctgga tttgtgggaa tggctttgcg tgagaaaggg gaggagagtg 3420
gttgctgccc tcagccggct tgaggacaga gcctgtccct ctcatgacaa ctcagtgttg 3480
aagcccagtg tcctcagctt catgtccagt ggatggcaga agttcatggg gtagtggcct 3540
ctcaaaggct gggcgcatcc caagacagcc agcaggttgt ctctggaaac gaccagagtt 3600
aagctctcgg cttctctgct gagggtgcac cctttcctct agatggtagt tgtcacgtta 3660
tctttgaaaa ctcttggact gctcctgagg aggccctctt ttccagtagg aagttagatg 3720
ggggttctca gaagtggctg attggaaggg gacaagcttc gtttcagggg tctgccgttc 3780

catcctggtt cagagaaggc cgagcgtggc tttctctagc cttgtcactg tctccctgcc 3840
tgtcaatcac cacctttcct ccagaggagg aaaattatct cccctgcaaa gcccggttct 3900
acacagattt cacaaattgt gctaagaacc gtccgtgttc tcagaaagcc cagtgttttt 3960
gcaaagaatg aaaagggacc ccatatgtag caaaaatcag ggctgggga gagccgggtt 4020
cattccctgt cctcattggt cgtccctatg aattgtacgt ttcagagaaa tttttttcc 4080
tatgtgcaac acgaagcttc cagaaccata aaatatcccg tcgataagga aagaaaatgt 4140
cgttgttgtt gtttttctgg aaactgcttg aaatcttgct gtactataga gctcagaagg 4200
acacagcccg tcctcccctg cctgcctgat tccatggctg ttgtgctgat tccaatgctt 4260
tcacgttggt tcctggcgtg ggaactgctc tcctttgcag ccccatttcc caagctctgt 4320
tcaagttaaa cttatgtaag ctttccgtgg catgcggggc gcgcacccac gtccccgctg 4380
cgtaagactc tgtatttgga tgccaatcca caggcctgaa gaaactgctt gttgtgtatc 4440
agtaatcatt agtggcaatg atgacattct gaaaagctgc aatacttata caataaattt 4500
tacaattctt tggaaaaaaa aaaaaaaaaa a 4531

<210> 129
<211> 2692
<212> DNA
<213> NM_152871.1| Homo sapiens tumor necrosis factor receptor superfamily, member 6 (TNFRSF6), transcript variant 2, mRNA

<400> 129

cctacccgcg cgcaggccaa gttgctgaat caatggagcc ctccccaacc cgggcgttcc 60
ccagcgaggc ttccttccca tcctcctgac caccggggct tttcgtgagc tcgtctctga 120
tctcgcgcaa gagtgacaca caggtgttca aagacgcttc tggggagtga gggaagcggt 180
ttacgagtga cttggctgga gcctcagggg cgggcactgg cacggaacac accctgaggc 240
cagccctggc tgcccaggcg gagctgcctc ttctcccgcg ggttggtgga cccgctcagt 300
acggagttgg ggaagctctt tcacttcgga ggattgctca acaaccatgc tgggcatctg 360
gaccctccta cctctggttc ttacgtctgt tgctagatta tcgtccaaaa gtgttaatgc 420
ccaagtgact gacatcaact ccaagggatt ggaattgagg aagactgtta ctacagttga 480
gactcagaac ttggaaggcc tgcatcatga tggccaattc tgccataagc cctgtcctcc 540
aggtgaaagg aaagctaggg actgcacagt caatggggat gaaccagact gcgtgccctg 600
ccaagaaggg aaggagtaca cagacaaagc ccatttttct tccaaatgca gaagatgtag 660
attgtgtgat gaaggacatg gcttagaagt ggaaataaac tgcacccgga cccagaatac 720
caagtgcaga tgtaaaccaa actttttttg taactctact gtatgtgaac actgtgaccc 780
ttgcaccaaa tgtgaacatg gaatcatcaa ggaatgcaca ctcaccagca acaccaagtg 840

```
caaagaggaa gtgaagagaa aggaagtaca gaaaacatgc agaaagcaca gaaaggaaaa     900
ccaaggttct catgaatctc caaccttaaa tcctgaaaca gtggcaataa atttatctga     960
tgttgacttg agtaaatata tcaccactat tgctggagtc atgacactaa gtcaagttaa    1020
aggctttgtt cgaaagaatg gtgtcaatga agccaaaata gatgagatca agaatgacaa    1080
tgtccaagac acagcagaac agaaagttca actgcttcgt aattggcatc aacttcatgg    1140
aaagaaagaa gcgtatgaca cattgattaa agatctcaaa aaagccaatc tttgtactct    1200
tgcagagaaa attcagacta tcatcctcaa ggacattact agtgactcag aaaattcaaa    1260
cttcagaaat gaaatccaaa gcttggtcta gagtgaaaaa caacaaattc agttctgagt    1320
atatgcaatt agtgtttgaa aagattctta atagctggct gtaaatactg cttggttttt    1380
tactgggtac attttatcat ttattagcgc tgaagagcca acatatttgt agatttttaa    1440
tatctcatga ttctgcctcc aaggatgttt aaaatctagt tgggaaaaca aacttcatca    1500
agagtaaatg cagtggcatg ctaagtaccc aaataggagt gtatgcagag gatgaaagat    1560
taagattatg ctctggcatc taacatatga ttctgtagta tgaatgtaat cagtgtatgt    1620
tagtacaaat gtctatccac aggctaaccc cactctatga atcaatagaa gaagctatga    1680
ccttttgctg aaatatcagt tactgaacag gcaggccact ttgcctctaa attacctctg    1740
ataattctag agattttacc atatttctaa actttgttta taactctgag aagatcatat    1800
ttatgtaaag tatatgtatt tgagtgcaga atttaaataa ggctctacct caaagacctt    1860
tgcacagttt attggtgtca tattatacaa tatttcaatt gtgaattcac atagaaaaca    1920
ttaaattata atgtttgact attatatatg tgtatgcatt ttactggctc aaaactacct    1980
acttctttct caggcatcaa aagcattttg agcaggagag tattactaga gctttgccac    2040
ctctccattt ttgccttggt gctcatctta atggcctaat gcacccccaa acatggaaat    2100
atcaccaaaa aatacttaat agtccaccaa aaggcaagac tgcccttaga aattctagcc    2160
tggtttggag atactaactg ctctcagaga aagtagcttt gtgacatgtc atgaacccat    2220
gtttgcaatc aaagatgata aaatagattc ttatttttcc cccacccccg aaaatgttca    2280
ataatgtccc atgtaaaacc tgctacaaat ggcagcttat acatagcaat ggtaaaatca    2340
tcatctggat ttaggaattg ctcttgtcat acccccaagt ttctaagatt taagattctc    2400
cttactacta tcctacgttt aaatatcttt gaaagtttgt attaaatgtg aattttaaga    2460
aataatattt atatttctgt aaatgtaaac tgtgaagata gttataaact gaagcagata    2520
cctggaacca cctaaagaac ttccatttat ggaggatttt tttgcccctt gtgtttggaa    2580
ttataaaata taggtaaaag tacgtaatta aataatgttt ttggtaaaaa aaaaaaaaaa    2640
aaaaaaaaaa aaaaaaaaaa aaaaaaaaaa aaaaaaaaaa aaaaaaaaaa aa            2692
```

<210> 130
<211> 2730
<212> DNA
<213> NM_152872.1| Homo sapiens tumor necrosis factor receptor superfamily, member 6 (TNFRSF6), transcript variant 3, mRNA

<400> 130

```
cctacccgcg cgcaggccaa gttgctgaat caatggagcc ctccccaacc cgggcgttcc      60
ccagcgaggc ttccttccca tcctcctgac caccggggct tttcgtgagc tcgtctctga     120
tctcgcgcaa gagtgacaca caggtgttca aagacgcttc tggggagtga gggaagcggt     180
ttacgagtga cttggctgga gcctcagggg cgggcactgg cacggaacac accctgaggc     240
cagccctggc tgcccaggcg gagctgcctc ttctcccgcg ggttggtgga cccgctcagt     300
acggagttgg ggaagctctt tcacttcgga ggattgctca acaaccatgc tgggcatctg     360
gaccctccta cctctggttc ttacgtctgt tgctagatta tcgtccaaaa gtgttaatgc     420
ccaagtgact gacatcaact ccaagggatt ggaattgagg aagactgtta ctacagttga     480
gactcagaac ttggaaggcc tgcatcatga tggccaattc tgccataagc cctgtcctcc     540
aggtgaaagg aaagctaggg actgcacagt caatggggat gaaccagact gcgtgccctg     600
ccaagaaggg aaggagtaca cagacaaagc ccatttttct tccaaatgca gaagatgtag     660
attgtgtgat gaaggacatg gcttagaagt ggaaataaac tgcacccgga cccagaatac     720
caagtgcaga tgtaaaccaa actttttttg taactctact gtatgtgaac actgtgaccc     780
ttgcaccaaa tgtgaacatg gaatcatcaa ggaatgcaca ctcaccagca acaccaagtg     840
caaagaggaa ggatccagat ctaacttggg gtggctttgt cttcttcttt tgccaattcc     900
actaattgtt tgggtgaaga gaaaggaagt acagaaaaca tgcagaaagc acagaaagga     960
aaaccaaggt tctcatgaat ctccaacctt aaatcctatg ttgacttgag taaatatatc    1020
accactattg ctggagtcat gacactaagt caagttaaag gctttgttcg aaagaatggt    1080
gtcaatgaag ccaaaataga tgagatcaag aatgacaatg tccaagacac agcagaacag    1140
aaagttcaac tgcttcgtaa ttggcatcaa cttcatggaa agaaagaagc gtatgacaca    1200
ttgattaaag atctcaaaaa agccaatctt tgtactcttg cagagaaaat tcagactatc    1260
atcctcaagg acattactag tgactcagaa aattcaaact tcagaaatga aatccaaagc    1320
ttggtctaga gtgaaaaaca acaaattcag ttctgagtat atgcaattag tgtttgaaaa    1380
gattcttaat agctggctgt aaatactgct tggtttttta ctgggtacat tttatcattt    1440
attagcgctg aagagccaac atatttgtag atttttaata tctcatgatt ctgcctccaa    1500
ggatgtttaa aatctagttg ggaaaacaaa cttcatcaag agtaaatgca gtggcatgct    1560
aagtacccaa ataggagtgt atgcagagga tgaaagatta agattatgct ctggcatcta    1620
acatatgatt ctgtagtatg aatgtaatca gtgtatgtta gtacaaatgt ctatccacag    1680
gctaacccca ctctatgaat caatagaaga agctatgacc ttttgctgaa atatcagtta    1740
ctgaacaggc aggccacttt gcctctaaat tacctctgat aattctagag attttaccat    1800
```

```
atttctaaac tttgtttata actctgagaa gatcatattt atgtaaagta tatgtatttg   1860
agtgcagaat ttaaataagg ctctacctca aagacctttg cacagtttat tggtgtcata   1920
ttatacaata tttcaattgt gaattcacat agaaaacatt aaattataat gtttgactat   1980
tatatatgtg tatgcatttt actggctcaa aactacctac ttctttctca ggcatcaaaa   2040
gcattttgag caggagagta ttactagagc tttgccacct ctccattttt gccttggtgc   2100
tcatcttaat ggcctaatgc acccccaaac atggaaatat caccaaaaaa tacttaatag   2160
tccaccaaaa ggcaagactg cccttagaaa ttctagcctg gtttggagat actaactgct   2220
ctcagagaaa gtagctttgt gacatgtcat gaacccatgt ttgcaatcaa agatgataaa   2280
atagattctt atttttcccc cacccccgaa aatgttcaat aatgtcccat gtaaaacctg   2340
ctacaaatgg cagcttatac atagcaatgg taaaatcatc atctggattt aggaattgct   2400
cttgtcatac ccccaagttt ctaagattta agattctcct tactactatc ctacgtttaa   2460
atatctttga aagtttgtat taaatgtgaa ttttaagaaa taatatttat atttctgtaa   2520
atgtaaactg tgaagatagt tataaactga agcagatacc tggaaccacc taaagaactt   2580
ccatttatgg aggatttttt tgccccttgt gtttggaatt ataaaatata ggtaaaagta   2640
cgtaattaaa taatgttttt ggtaaaaaaa aaaaaaaaaa aaaaaaaaaa aaaaaaaaaa   2700
aaaaaaaaaa aaaaaaaaaa aaaaaaaaaa                                    2730
```

<210> 131
<211> 2563
<212> DNA
<213> NM_152874.1| Homo sapiens tumor necrosis factor receptor superfamily, member 6 (TNFRSF6), transcript variant 8, mRNA

<400> 131

```
cctacccgcg cgcaggccaa gttgctgaat caatggagcc ctccccaacc cgggcgttcc     60
ccagcgaggc ttccttccca tcctcctgac caccggggct tttcgtgagc tcgtctctga    120
tctcgcgcaa gagtgacaca caggtgttca aagacgcttc tggggagtga gggaagcggt    180
ttacgagtga cttggctgga gcctcagggg cgggcactgg cacggaacac accctgaggc    240
cagccctggc tgcccaggcg gagctgcctc ttctcccgcg ggttggtgga cccgctcagt    300
acggagttgg ggaagctctt tcacttcgga ggattgctca acaaccatgc tgggcatctg    360
gaccctccta cctctggttc ttacgtctgt tgctagatta tcgtccaaaa gtgttaatgc    420
ccaagtgact gacatcaact ccaagggatt ggaattgagg aagactgtta ctacagttga    480
gactcagaac ttggaaggcc tgcatcatga tggccaattc tgccataagc cctgtcctcc    540
aggtgaaagg aaagctaggg actgcacagt caatggggat gaaccagact gcgtgccctg    600
ccaagaaggg aaggagtaca cagacaaagc ccatttttct tccaaatgca gaagatgtag    660
```

attgtgtgat gaaggacatg atgtgaacat ggaatcatca aggaatgcac actcaccagc 720
aacaccaagt gcaaagagga aggatccaga tctaacttgg ggtggctttg tcttcttctt 780
ttgccaattc cactaattgt ttggggaaac agtggcaata aatttatctg atgttgactt 840
gagtaaatat atcaccacta ttgctggagt catgacacta agtcaagtta aaggctttgt 900
tcgaaagaat ggtgtcaatg aagccaaaat agatgagatc aagaatgaca atgtccaaga 960
cacagcagaa cagaaagttc aactgcttcg taattggcat caacttcatg gaaagaaaga 1020
agcgtatgac acattgatta aagatctcaa aaaagccaat ctttgtactc ttgcagagaa 1080
aattcagact atcatcctca aggacattac tagtgactca gaaaattcaa acttcagaaa 1140
tgaaatccaa agcttggtct agagtgaaaa acaacaaatt cagttctgag tatatgcaat 1200
tagtgtttga aaagattctt aatagctggc tgtaaatact gcttggtttt ttactgggta 1260
cattttatca tttattagcg ctgaagagcc aacatatttg tagattttta atatctcatg 1320
attctgcctc caaggatgtt taaaatctag ttgggaaaac aaacttcatc aagagtaaat 1380
gcagtggcat gctaagtacc caaataggag tgtatgcaga ggatgaaaga ttaagattat 1440
gctctggcat ctaacatatg attctgtagt atgaatgtaa tcagtgtatg ttagtacaaa 1500
tgtctatcca caggctaacc ccactctatg aatcaataga agaagctatg accttttgct 1560
gaaatatcag ttactgaaca ggcaggccac tttgcctcta aattacctct gataattcta 1620
gagattttac catatttcta aactttgttt ataactctga gaagatcata tttatgtaaa 1680
gtatatgtat ttgagtgcag aatttaaata aggctctacc tcaaagacct ttgcacagtt 1740
tattggtgtc atattataca atatttcaat tgtgaattca catagaaaac attaaattat 1800
aatgtttgac tattatatat gtgtatgcat tttactggct caaaactacc tacttctttc 1860
tcaggcatca aaagcatttt gagcaggaga gtattactag agctttgcca cctctccatt 1920
tttgccttgg tgctcatctt aatggcctaa tgcaccccca aacatggaaa tatcaccaaa 1980
aaatacttaa tagtccacca aaaggcaaga ctgcccttag aaattctagc ctggtttgga 2040
gatactaact gctctcagag aaagtagctt tgtgacatgt catgaaccca tgtttgcaat 2100
caaagatgat aaaatagatt cttatttttc ccccaccccc gaaaatgttc aataatgtcc 2160
catgtaaaac ctgctacaaa tggcagctta tacatagcaa tggtaaaatc atcatctgga 2220
tttaggaatt gctcttgtca taccccccaag tttctaagat ttaagattct ccttactact 2280
atcctacgtt taaatatctt tgaaagtttg tattaaatgt gaattttaag aaataatatt 2340
tatatttctg taaatgtaaa ctgtgaagat agttataaac tgaagcagat acctggaacc 2400
acctaaagaa cttccattta tggaggattt ttttgcccct tgtgtttgga attataaaat 2460
ataggtaaaa gtacgtaatt aaataatgtt tttggtaaaa aaaaaaaaaa aaaaaaaaaa 2520
aaaaaaaaaa aaaaaaaaaa aaaaaaaaaa aaaaaaaaaa aaa 2563

<210> 132
<211> 2445
<212> DNA

<213> NM_152876.1| Homo sapiens tumor necrosis factor receptor superfamily, member 6 (TNFRSF6), transcript variant 6, mRNA

<400> 132

```
cctacccgcg cgcaggccaa gttgctgaat caatggagcc ctccccaacc cgggcgttcc     60
ccagcgaggc ttccttccca tcctcctgac caccggggct tttcgtgagc tcgtctctga    120
tctcgcgcaa gagtgacaca caggtgttca aagacgcttc tggggagtga gggaagcggt    180
ttacgagtga cttggctgga gcctcagggg cgggcactgg cacggaacac accctgaggc    240
cagccctggc tgcccaggcg gagctgcctc ttctcccgcg ggttggtgga cccgctcagt    300
acggagttgg ggaagctctt tcacttcgga ggattgctca acaaccatgc tgggcatctg    360
gaccctccta cctctggttc ttacgtctgt tgctagatta tcgtccaaaa gtgttaatgc    420
ccaagtgact gacatcaact ccaagggatt ggaattgagg aagactgtta ctacagttga    480
gactcagaac ttggaaggcc tgcatcatga tggccaattc tgccataagc cctgtcctcc    540
agatgtgaac atggaatcat caaggaatgc acactcacca gcaacaccaa gtgcaaagag    600
gaagtgaaga gaaaggaagt acagaaaaca tgcagaaagc acagaaagga aaaccaaggt    660
tctcatgaat ctccaacctt aaatcctgaa acagtggcaa taaatttatc tgatgttgac    720
ttgagtaaat atatcaccac tattgctgga gtcatgacac taagtcaagt taaaggcttt    780
gttcgaaaga atggtgtcaa tgaagccaaa atagatgaga tcaagaatga caatgtccaa    840
gacacagcag aacagaaagt tcaactgctt cgtaattggc atcaacttca tggaaagaaa    900
gaagcgtatg acacattgat taaagatctc aaaaaagcca atctttgtac tcttgcagag    960
aaaattcaga ctatcatcct caaggacatt actagtgact cagaaaattc aaacttcaga   1020
aatgaaatcc aaagcttggt ctagagtgaa aaacaacaaa ttcagttctg agtatatgca   1080
attagtgttt gaaaagattc ttaatagctg gctgtaaata ctgcttggtt ttttactggg   1140
tacattttat catttattag cgctgaagag ccaacatatt tgtagatttt taatatctca   1200
tgattctgcc tccaaggatg tttaaaatct agttgggaaa acaaacttca tcaagagtaa   1260
atgcagtggc atgctaagta cccaaatagg agtgtatgca gaggatgaaa gattaagatt   1320
atgctctggc atctaacata tgattctgta gtatgaatgt aatcagtgta tgttagtaca   1380
aatgtctatc cacaggctaa ccccactcta tgaatcaata gaagaagcta tgaccttttg   1440
ctgaaatatc agttactgaa caggcaggcc actttgcctc taaattacct ctgataattc   1500
tagagatttt accatatttc taaactttgt ttataactct gagaagatca tatttatgta   1560
aagtatatgt atttgagtgc agaatttaaa taaggctcta cctcaaagac ctttgcacag   1620
tttattggtg tcatattata caatatttca attgtgaatt cacatagaaa acattaaatt   1680
```

```
ataatgtttg actattatat atgtgtatgc attttactgg ctcaaaacta cctacttctt   1740
tctcaggcat caaaagcatt ttgagcagga gagtattact agagctttgc cacctctcca   1800
tttttgcctt ggtgctcatc ttaatggcct aatgcacccc caaacatgga aatatcacca   1860
aaaaatactt aatagtccac caaaaggcaa gactgccctt agaaattcta gcctggtttg   1920
gagatactaa ctgctctcag agaaagtagc tttgtgacat gtcatgaacc catgtttgca   1980
atcaaagatg ataaaataga ttcttatttt tcccccaccc ccgaaaatgt tcaataatgt   2040
cccatgtaaa acctgctaca aatggcagct tatacatagc aatggtaaaa tcatcatctg   2100
gatttaggaa ttgctcttgt catacccccca agtttctaag atttaagatt ctccttacta   2160
ctatcctacg tttaaatatc tttgaaagtt tgtattaaat gtgaatttta agaaataata   2220
tttatatttc tgtaaatgta aactgtgaag atagttataa actgaagcag atacctggaa   2280
ccacctaaag aacttccatt tatggaggat ttttttgccc cttgtgtttg gaattataaa   2340
atataggtaa aagtacgtaa ttaaataatg tttttggtaa aaaaaaaaaa aaaaaaaaaa   2400
aaaaaaaaaa aaaaaaaaaa aaaaaaaaaa aaaaaaaaaa aaaaa                   2445
```

<210> 133
<211> 2508
<212> DNA
<213> NM_152877.1| Homo sapiens tumor necrosis factor receptor superfamily, member 6 (TNFRSF6), transcript variant 7, mRNA

<400> 133

```
cctacccgcg cgcaggccaa gttgctgaat caatggagcc ctccccaacc cgggcgttcc     60
ccagcgaggc ttccttccca tcctcctgac caccggggct tttcgtgagc tcgtctctga    120
tctcgcgcaa gagtgacaca caggtgttca aagacgcttc tggggagtga gggaagcggt    180
ttacgagtga cttggctgga gcctcagggg cgggcactgg cacggaacac accctgaggc    240
cagccctggc tgcccaggcg gagctgcctc ttctcccgcg ggttggtgga cccgctcagt    300
acggagttgg ggaagctctt tcacttcgga ggattgctca acaaccatgc tgggcatctg    360
gaccctccta cctctggttc ttacgtctgt tgctagatta tcgtccaaaa gtgttaatgc    420
ccaagtgact gacatcaact ccaagggatt ggaattgagg aagactgtta ctacagttga    480
gactcagaac ttggaaggcc tgcatcatga tggccaattc tgccataagc cctgtcctcc    540
agatgtgaac atggaatcat caaggaatgc acactcacca gcaacaccaa gtgcaaagag    600
gaaggatcca gatctaactt ggggtggctt tgtcttcttc ttttgccaat tccactaatt    660
gtttgggtga agagaaagga agtacagaaa acatgcagaa agcacagaaa ggaaaaccaa    720
ggttctcatg aatctccaac cttaaatcct gaaacagtgg caataaattt atctgatgtt    780
gacttgagta aatatatcac cactattgct ggagtcatga cactaagtca agttaaaggc    840
```

tttgttcgaa agaatggtgt caatgaagcc aaaatagatg agatcaagaa tgacaatgtc 900
caagacacag cagaacagaa agttcaactg cttcgtaatt ggcatcaact tcatggaaag 960
aaagaagcgt atgacacatt gattaaagat ctcaaaaaag ccaatctttg tactcttgca 1020
gagaaaattc agactatcat cctcaaggac attactagtg actcagaaaa ttcaaacttc 1080
agaaatgaaa tccaaagctt ggtctagagt gaaaaacaac aaattcagtt ctgagtatat 1140
gcaattagtg tttgaaaaga ttcttaatag ctggctgtaa atactgcttg gttttttact 1200
gggtacattt tatcatttat tagcgctgaa gagccaacat atttgtagat ttttaatatc 1260
tcatgattct gcctccaagg atgtttaaaa tctagttggg aaaacaaact tcatcaagag 1320
taaatgcagt ggcatgctaa gtacccaaat aggagtgtat gcagaggatg aaagattaag 1380
attatgctct ggcatctaac atatgattct gtagtatgaa tgtaatcagt gtatgttagt 1440
acaaatgtct atccacaggc taaccccact ctatgaatca atagaagaag ctatgacctt 1500
ttgctgaaat atcagttact gaacaggcag gccactttgc ctctaaatta cctctgataa 1560
ttctagagat tttaccatat ttctaaactt tgtttataac tctgagaaga tcatatttat 1620
gtaaagtata tgtatttgag tgcagaattt aaataaggct ctacctcaaa gacctttgca 1680
cagtttattg gtgtcatatt atacaatatt tcaattgtga attcacatag aaaacattaa 1740
attataatgt ttgactatta tatatgtgta tgcattttac tggctcaaaa ctacctactt 1800
ctttctcagg catcaaaagc attttgagca ggagagtatt actagagctt tgccacctct 1860
ccatttttgc cttggtgctc atcttaatgg cctaatgcac ccccaaacat ggaaatatca 1920
ccaaaaaata cttaatagtc caccaaaagg caagactgcc cttagaaatt ctagcctggt 1980
ttggagatac taactgctct cagagaaagt agctttgtga catgtcatga acccatgttt 2040
gcaatcaaag atgataaaat agattcttat tttcccccca cccccgaaaa tgttcaataa 2100
tgtcccatgt aaaacctgct acaaatggca gcttatacat agcaatggta aaatcatcat 2160
ctggatttag gaattgctct tgtcataccc ccaagtttct aagatttaag attctcctta 2220
ctactatcct acgtttaaat atctttgaaa gtttgtatta aatgtgaatt ttaagaaata 2280
atatttatat ttctgtaaat gtaaactgtg aagatagtta taaactgaag cagatacctg 2340
gaaccaccta aagaacttcc atttatggag gatttttttg ccccttgtgt ttggaattat 2400
aaaatatagg taaaagtacg taattaaata atgtttttgg taaaaaaaaa aaaaaaaaaa 2460
aaaaaaaaaa aaaaaaaaaa aaaaaaaaaa aaaaaaaaaa aaaaaaaa 2508

<210> 134
<211> 2583
<212> DNA
<213> NM_152875.1| Homo sapiens tumor necrosis factor receptor superfamily, member 6 (TNFRSF6), transcript variant 5, mRNA

<400> 134

```
cctacccgcg cgcaggccaa gttgctgaat caatggagcc ctccccaacc cgggcgttcc      60
ccagcgaggc ttccttccca tcctcctgac caccggggct tttcgtgagc tcgtctctga     120
tctcgcgcaa gagtgacaca caggtgttca aagacgcttc tggggagtga gggaagcggt     180
ttacgagtga cttggctgga gcctcagggg cgggcactgg cacggaacac accctgaggc     240
cagccctggc tgcccaggcg gagctgcctc ttctcccgcg ggttggtgga cccgctcagt     300
acggagttgg ggaagctctt tcacttcgga ggattgctca acaaccatgc tgggcatctg     360
gaccctccta cctctggttc ttacgtctgt tgctagatta tcgtccaaaa gtgttaatgc     420
ccaagtgact gacatcaact ccaagggatt ggaattgagg aagactgtta ctacagttga     480
gactcagaac ttggaaggcc tgcatcatga tggccaattc tgccataagc cctgtcctcc     540
aggtgaaagg aaagctaggg actgcacagt caatggggat gaaccagact gcgtgccctg     600
ccaagaaggg aaggagtaca cagacaaagc ccattttttct tccaaatgca gaagatgtag     660
attgtgtgat gaaggacatg atgtgaacat ggaatcatca aggaatgcac actcaccagc     720
aacaccaagt gcaaagagga agtgaagaga aaggaagtac agaaaacatg cagaaagcac     780
agaaaggaaa accaaggttc tcatgaatct ccaaccttaa atcctgaaac agtggcaata     840
aatttatctg atgttgactt gagtaaatat atcaccacta ttgctggagt catgacacta     900
agtcaagtta aaggctttgt tcgaaagaat ggtgtcaatg aagccaaaat agatgagatc     960
aagaatgaca atgtccaaga cacagcagaa cagaaagttc aactgcttcg taattggcat    1020
caacttcatg gaaagaaaga agcgtatgac acattgatta aagatctcaa aaaagccaat    1080
ctttgtactc ttgcagagaa aattcagact atcatcctca aggacattac tagtgactca    1140
gaaaattcaa acttcagaaa tgaaatccaa agcttggtct agagtgaaaa acaacaaatt    1200
cagttctgag tatatgcaat tagtgtttga aaagattctt aatagctggc tgtaaatact    1260
gcttggtttt ttactgggta cattttatca tttattagcg ctgaagagcc aacatatttg    1320
tagattttta atatctcatg attctgcctc caaggatgtt taaaatctag ttgggaaaac    1380
aaacttcatc aagagtaaat gcagtggcat gctaagtacc caaataggag tgtatgcaga    1440
ggatgaaaga ttaagattat gctctggcat ctaacatatg attctgtagt atgaatgtaa    1500
tcagtgtatg ttagtacaaa tgtctatcca caggctaacc ccactctatg aatcaataga    1560
agaagctatg accttttgct gaaatatcag ttactgaaca ggcaggccac tttgcctcta    1620
aattacctct gataattcta gagattttac catatttcta aactttgttt ataactctga    1680
gaagatcata tttatgtaaa gtatatgtat ttgagtgcag aatttaaata aggctctacc    1740
tcaaagacct ttgcacagtt tattggtgtc atattataca atatttcaat tgtgaattca    1800
catagaaaac attaaattat aatgtttgac tattatatat gtgtatgcat tttactggct    1860
caaaactacc tacttctttc tcaggcatca aaagcatttt gagcaggaga gtattactag    1920
```

agctttgcca cctctccatt tttgccttgg tgctcatctt aatggcctaa tgcaccccca 1980
aacatggaaa tatcaccaaa aaatacttaa tagtccacca aaaggcaaga ctgcccttag 2040
aaattctagc ctggtttgga gatactaact gctctcagag aaagtagctt tgtgacatgt 2100
catgaaccca tgtttgcaat caaagatgat aaaatagatt cttatttttc ccccaccccc 2160
gaaaatgttc aataatgtcc catgtaaaac ctgctacaaa tggcagctta tacatagcaa 2220
tggtaaaatc atcatctgga tttaggaatt gctcttgtca taccccccaag tttctaagat 2280
ttaagattct ccttactact atcctacgtt taaatatctt tgaaagtttg tattaaatgt 2340
gaattttaag aaataatatt tatatttctg taaatgtaaa ctgtgaagat agttataaac 2400
tgaagcagat acctggaacc acctaaagaa cttccattta tggaggattt ttttgcccct 2460
tgtgtttgga attataaaat ataggtaaaa gtacgtaatt aaataatgtt tttggtaaaa 2520
aaaaaaaaaa aaaaaaaaaa aaaaaaaaaa aaaaaaaaaa aaaaaaaaaa aaaaaaaaaa 2580
aaa 2583

<210> 135
<211> 316
<212> DNA
<213> >gi|13310411|gb|AF333388.1|AF333388 Homo sapiens metallothionein 1H-like protein mRNA, complete cds

<400> 135

cctcttctct tctcgcttgg gaacgccggt ctcacctcgg cttgcaatgg accccaactg 60
ctcctgcgcc gctggaggct cctacgcctg cgccggctcc tgcaagtgca aaaagtgcaa 120
atgcacctcc tgcaagaaga gctgctgctc ctgttgcccc ctgggctgtg ccaagtgtgc 180
ccagggctgc atccgcaaag gggcttcgga aaagtgcagc tgctgtgcct gatgtcggga 240
ctgccctgct ctcggatgaa aacagaatga cacgtaaagt ccgggatttt tttttctaca 300
actccgactc atttgc 316

<210> 136
<211> 3145
<212> DNA
<213> NM_000251. Homo sapiens muts...[gi:4557760]

<400> 136

ggcgggaaac agcttagtgg gtgtggggtc gcgcattttc ttcaaccagg aggtgaggag 60
gtttcgacat ggcggtgcag ccgaaggaga cgctgcagtt ggagagcgcg gccgaggtcg 120
gcttcgtgcg cttctttcag ggcatgccgg agaagccgac caccacagtg cgccttttcg 180

accggggcga cttctatacg gcgcacggcg aggacgcgct gctggccgcc cgggaggtgt 240
tcaagaccca gggggtgatc aagtacatgg ggccggcagg agcaaagaat ctgcagagtg 300
ttgtgcttag taaaatgaat tttgaatctt ttgtaaaaga tcttcttctg gttcgtcagt 360
atagagttga agtttataag aatagagctg gaaataaggc atccaaggag aatgattggt 420
atttggcata taaggcttct cctggcaatc tctctcagtt tgaagacatt ctctttggta 480
acaatgatat gtcagcttcc attggtgttg tgggtgttaa aatgtccgca gttgatggcc 540
agagacaggt tggagttggg tatgtggatt ccatacagag gaaactagga ctgtgtgaat 600
tccctgataa tgatcagttc tccaatcttg aggctctcct catccagatt ggaccaaagg 660
aatgtgtttt acccggagga gagactgctg gagacatggg gaaactgaga cagataattc 720
aaagaggagg aattctgatc acagaaagaa aaaaagctga cttttccaca aaagacattt 780
atcaggacct caaccggttg ttgaaaggca aaaagggaga gcagatgaat agtgctgtat 840
tgccagaaat ggagaatcag gttgcagttt catcactgtc tgcggtaatc aagtttttag 900
aactcttatc agatgattcc aactttggac agtttgaact gactactttt gacttcagcc 960
agtatatgaa attggatatt gcagcagtca gagcccttaa cctttttcag ggttctgttg 1020
aagataccac tggctctcag tctctggctg ccttgctgaa taagtgtaaa acccctcaag 1080
gacaaagact tgttaaccag tggattaagc agcctctcat ggataagaac agaatagagg 1140
agagattgaa tttagtggaa gcttttgtag aagatgcaga attgaggcag actttacaag 1200
aagatttact tcgtcgattc ccagatctta accgacttgc caagaagttt caaagacaag 1260
cagcaaactt acaagattgt taccgactct atcagggtat aaatcaacta cctaatgtta 1320
tacaggctct ggaaaaacat gaaggaaaac accagaaatt attgttggca gtttttgtga 1380
ctcctcttac tgatcttcgt tctgacttct ccaagtttca ggaaatgata gaaacaactt 1440
tagatatgga tcaggtggaa aaccatgaat tccttgtaaa accttcattt gatcctaatc 1500
tcagtgaatt aagagaaata atgaatgact tggaaaagaa gatgcagtca acattaataa 1560
gtgcagccag agatcttggc ttggaccctg gcaaacagat taaactggat tccagtgcac 1620
agtttggata ttactttcgt gtaacctgta aggaagaaaa agtccttcgt aacaataaaa 1680
actttagtac tgtagatatc cagaagaatg gtgttaaatt taccaacagc aaattgactt 1740
ctttaaatga agagtatacc aaaaataaaa cagaatatga agaagcccag gatgccattg 1800
ttaaagaaat tgtcaatatt tcttcaggct atgtagaacc aatgcagaca ctcaatgatg 1860
tgttagctca gctagatgct gttgtcagct ttgctcacgt gtcaaatgga gcacctgttc 1920
catatgtacg accagccatt ttggagaaag gacaaggaag aattatatta aaagcatcca 1980
ggcatgcttg tgttgaagtt caagatgaaa ttgcatttat tcctaatgac gtatactttg 2040
aaaaagataa acagatgttc cacatcatta ctggccccaa tatgggaggt aaatcaacat 2100
atattcgaca aactggggtg atagtactca tggcccaaat tgggtgtttt gtgccatgtg 2160
agtcagcaga agtgtccatt gtggactgca tcttagcccg agtaggggct ggtgacagtc 2220

aattgaaagg agtctccacg ttcatggctg aaatgttgga aactgcttct atcctcaggt 2280
ctgcaaccaa agattcatta ataatcatag atgaattggg aagaggaact tctacctacg 2340
atggatttgg gttagcatgg gctatatcag aatacattgc aacaaagatt ggtgcttttt 2400
gcatgtttgc aacccatttt catgaactta ctgccttggc caatcagata ccaactgtta 2460
ataatctaca tgtcacagca ctcaccactg aagagacctt aactatgctt tatcaggtga 2520
agaaaggtgt ctgtgatcaa agttttggga ttcatgttgc agagcttgct aatttcccta 2580
agcatgtaat agagtgtgct aaacagaaag ccctggaact tgaggagttt cagtatattg 2640
gagaatcgca aggatatgat atcatggaac cagcagcaaa gaagtgctat ctggaaagag 2700
agcaaggtga aaaaattatt caggagttcc tgtccaaggt gaaacaaatg ccctttactg 2760
aaatgtcaga agaaaacatc acaataaagt taaaacagct aaaagctgaa gtaatagcaa 2820
agaataatag ctttgtaaat gaaatcattt cacgaataaa agttactacg tgaaaaatcc 2880
cagtaatgga atgaaggtaa tattgataag ctattgtctg taatagtttt atattgtttt 2940
atattaaccc tttttccata gtgttaactg tcagtgccca tgggctatca acttaataag 3000
atatttagta atattttact ttgaggacat tttcaaagat ttttattttg aaaaatgaga 3060
gctgtaactg aggactgttt gcaattgaca taggcaataa taagtgatgt gctgaatttt 3120
ataaataaaa tcatgtagtt tgtgg 3145

<210> 137
<211> 3239
<212> DNA
<213> NM_000534. Homo sapiens PMS1...[gi:53729349]

<400> 137

ctcgctgcca gcggattggc tgcgagcagc gccaatctca cgttgccccc gggcgaggcg 60
ggactcagtg ccgcgctctc tgcacccgct ctgccgcgcg cgtgcgtgct gggtgcgggt 120
gcgggtgcgg ggttgggcct gcgcatcggg tgagacgctg gctgcttgcg gctagtggat 180
ggtaattgcc tgcctcgcgc tagcaggaag ctgctctgtt aaaagcgaaa atgaaacaat 240
tgcctgcggc aacagttcga ctcctttcaa gttctcagat catcacttcg gtggtcagtg 300
ttgtaaaaga gcttattgaa aactccttgg atgctggtgc cacaagcgta gatgttaaac 360
tggagaacta tggatttgat aaaattgagg tgcgagataa cggggagggt atcaaggctg 420
ttgatgcacc tgtaatggca atgaagtact acacctcaaa aataaatagt catgaagatc 480
ttgaaaattt gacaacttac ggttttcgtg gagaagcctt ggggtcaatt tgttgtatag 540
ctgaggtttt aattacaaca agaacggctg ctgataattt tagcacccag tatgttttag 600
atggcagtgg ccacatactt tctcagaaac cttcacatct tggtcaaggt acaactgtaa 660

ctgctttaag attatttaag aatctacctg taagaaagca gttttactca actgcaaaaa 720
aatgtaaaga tgaaataaaa aagatccaag atctcctcat gagctttggt atccttaaac 780
ctgacttaag gattgtcttt gtacataaca aggcagttat ttggcagaaa agcagagtat 840
cagatcacaa gatggctctc atgtcagttc tggggactgc tgttatgaac aatatggaat 900
cctttcagta ccactctgaa gaatctcaga tttatctcag tggatttctt ccaaagtgtg 960
atgcagacca ctctttcact agtctttcaa caccagaaag aagtttcatc ttcataaaca 1020
gtcgaccagt acatcaaaaa gatatcttaa agttaatccg acatcattac aatctgaaat 1080
gcctaaagga atctactcgt ttgtatcctg ttttctttct gaaaatcgat gttcctacag 1140
ctgatgttga tgtaaattta acaccagata aaagccaagt attattacaa aataaggaat 1200
ctgttttaat tgctcttgaa aatctgatga cgacttgtta tggaccatta cctagtacaa 1260
attcttatga aaataataaa acagatgttt ccgcagctga catcgttctt agtaaaacag 1320
cagaaacaga tgtgcttttt aataaagtgg aatcatctgg aaagaattat tcaaatgttg 1380
atacttcagt cattccattc caaaatgata tgcataatga tgaatctgga aaaaacactg 1440
atgattgttt aaatcaccag ataagtattg gtgactttgg ttatggtcat tgtagtagtg 1500
aaatttctaa cattgataaa aacactaaga atgcatttca ggacatttca atgagtaatg 1560
tatcatggga gaactctcag acggaatata gtaaaacttg ttttataagt tccgttaagc 1620
acacccagtc agaaaatggc aataaagacc atatagatga gagtggggaa aatgaggaag 1680
aagcaggtct tgaaaactct tcggaaattt ctgcagatga gtggagcagg ggaaatatac 1740
ttaaaaattc agtgggagag aatattgaac ctgtgaaaat tttagtgcct gaaaaaagtt 1800
taccatgtaa agtaagtaat aataattatc caatccctga acaaatgaat cttaatgaag 1860
attcatgtaa caaaaaatca aatgtaatag ataataaatc tggaaaagtt acagcttatg 1920
atttacttag caatcgagta atcaagaaac ccatgtcagc aagtgctctt tttgttcaag 1980
atcatcgtcc tcagtttctc atagaaaatc ctaagactag tttagaggat gcaacactac 2040
aaattgaaga actgtggaag acattgagtg aagaggaaaa actgaaatat gaagagaagg 2100
ctactaaaga cttggaacga tacaatagtc aaatgaagag agccattgaa caggagtcac 2160
aaatgtcact aaaagatggc agaaaaaaga taaaacccac cagcgcatgg aatttggccc 2220
agaagcacaa gttaaaaacc tcattatcta atcaaccaaa acttgatgaa ctccttcagt 2280
cccaaattga aaaaagaagg agtcaaaata ttaaaatggt acagatcccc ttttctatga 2340
aaaacttaaa aataaatttt aagaaacaaa acaaagttga cttagaagag aaggatgaac 2400
cttgcttgat ccacaatctc aggtttcctg atgcatggct aatgacatcc aaaacagagg 2460
taatgttatt aaatccatat agagtagaag aagccctgct atttaaaaga cttcttgaga 2520
atcataaact tcctgcagag ccactggaaa agccaattat gttaacagag agtcttttta 2580
atggatctca ttatttagac gttttatata aaatgacagc agatgaccaa agatacagtg 2640
gatcaactta cctgtctgat cctcgtctta cagcgaatgg tttcaagata aaattgatac 2700

```
caggagtttc aattactgaa aattacttgg aaatagaagg aatggctaat tgtctcccat   2760
tctatggagt agcagattta aaagaaattc ttaatgctat attaaacaga aatgcaaagg   2820
aagtttatga atgtagacct cgcaaagtga taagttattt agagggagaa gcagtgcgtc   2880
tatccagaca attacccatg tacttatcaa aagaggacat ccaagacatt atctacagaa   2940
tgaagcacca gtttggaaat gaaattaaag agtgtgttca tggtcgccca ttttttcatc   3000
atttaaccta tcttccagaa actacatgat taaatatgtt taagaagatt agttaccatt   3060
gaaattggtt ctgtcataaa acagcatgag tctggtttta aattatcttt gtattatgtg   3120
tcacatggtt atttttttaaa tgaggattca ctgacttgtt tttatattga aaaaagttcc   3180
acgtattgta gaaaacgtaa ataaactaat atagactatt caaaaaaaaa aaaaaaaaa    3239
```

<210> 138
<211> 2771
<212> DNA
<213> NM_000535. Homo sapiens PMS2...[gi:11125773]

<400> 138

```
cgaggcggat cgggtgttgc atccatggag cgagctgaga gctcgagtac agaacctgct     60
aaggccatca aacctattga tcggaagtca gtccatcaga tttgctctgg gcaggtggta    120
ctgagtctaa gcactgcggt aaaggagtta gtagaaaaca gtctggatgc tggtgccact    180
aatattgatc taaagcttaa ggactatgga gtggatctta ttgaagtttc agacaatgga    240
tgtggggtag aagaagaaaa cttcgaaggc ttaactctga aacatcacac atctaagatt    300
caagagtttg ccgacctaac tcaggttgaa acttttggct ttcgggggga agctctgagc    360
tcactttgtg cactgagcga tgtcaccatt tctacctgcc acgcatcggc gaaggttgga    420
actcgactga tgtttgatca caatgggaaa attatccaga aaacccccta cccccgcccc    480
agagggacca cagtcagcgt gcagcagtta ttttccacac tacctgtgcg ccataaggaa    540
tttcaaagga atattaagaa ggagtatgcc aaaatggtcc aggtcttaca tgcatactgt    600
atcatttcag caggcatccg tgtaagttgc accaatcagc ttggacaagg aaaacgacag    660
cctgtggtat gcacaggtgg aagccccagc ataaaggaaa atatcggctc tgtgtttggg    720
cagaagcagt tgcaaagcct cattcctttt gttcagctgc cccctagtga ctccgtgtgt    780
gaagagtacg gtttgagctg ttcggatgct ctgcataatc tttttttacat ctcaggtttc    840
atttcacaat gcacgcatgg agttggaagg agttcaacag acagacagtt tttctttatc    900
aaccggcggc cttgtgaccc agcaaaggtc tgcagactcg tgaatgaggt ctaccacatg    960
tataatcgac accagtatcc atttgttgtt cttaacattt ctgttgattc agaatgcgtt   1020
gatatcaatg ttactccaga taaaaggcaa attttgctac aagaggaaaa gcttttgttg   1080
```

gcagttttaa agacctcttt gataggaatg tttgatagtg atgtcaacaa gctaaatgtc 1140
agtcagcagc cactgctgga tgttgaaggt aacttaataa aaatgcatgc agcggatttg 1200
gaaaagccca tggtagaaaa gcaggatcaa tccccttcat taaggactgg agaagaaaaa 1260
aaagacgtgt ccatttccag actgcgagag gccttttctc ttcgtcacac aacagagaac 1320
aagcctcaca gcccaaagac tccagaacca agaaggagcc ctctaggaca gaaaaggggt 1380
atgctgtctt ctagcacttc aggtgccatc tctgacaaag gcgtcctgag acctcagaaa 1440
gaggcagtga gttccagtca cggacccagt gaccctacgg acagagcgga ggtggagaag 1500
gactcggggc acggcagcac ttccgtggat tctgaggggt tcagcatccc agacacgggc 1560
agtcactgca gcagcgagta tgcggccagc tccccagggg acaggggctc gcaggaacat 1620
gtggactctc aggagaaagc gcctgaaact gacgactctt tttcagatgt ggactgccat 1680
tcaaaccagg aagataccgg atgtaaattt cgagttttgc ctcagccaac taatctcgca 1740
accccaaaca caaagcgttt taaaaaagaa gaaattcttt ccagttctga catttgtcaa 1800
aagttagtaa atactcagga catgtcagcc tctcaggttg atgtagctgt gaaaattaat 1860
aagaaagttg tgcccctgga cttttctatg agttctttag ctaaacgaat aaagcagtta 1920
catcatgaag cacagcaaag tgaaggggaa cagaattaca ggaagtttag ggcaaagatt 1980
tgtcctggag aaaatcaagc agccgaagat gaactaagaa aagagataag taaaacgatg 2040
tttgcagaaa tggaaatcat tggtcagttt aacctgggat ttataataac caaactgaat 2100
gaggatatct tcatagtgga ccagcatgcc acggacgaga agtataactt cgagatgctg 2160
cagcagcaca ccgtgctcca ggggcagagg ctcatagcac ctcagactct caacttaact 2220
gctgttaatg aagctgttct gatagaaaat ctggaaatat ttagaaagaa tggctttgat 2280
tttgttatcg atgaaaatgc tccagtcact gaaagggcta aactgatttc cttgccaact 2340
agtaaaaact ggaccttcgg accccaggac gtcgatgaac tgatcttcat gctgagcgac 2400
agccctgggg tcatgtgccg gccttcccga gtcaagcaga tgtttgcctc cagagcctgc 2460
cggaagtcgg tgatgattgg gactgctctt aacacaagcg agatgaagaa actgatcacc 2520
cacatggggg agatggacca cccctggaac tgtccccatg gaaggccaac catgagacac 2580
atcgccaacc tgggtgtcat ttctcagaac tgaccgtagt cactgtatgg aataattggt 2640
tttatcgcag atttttatgt tttgaaagac agagtcttca ctaacctttt ttgttttaaa 2700
atgaaacctg ctacttaaaa aaaatacaca tcacacccat ttaaaagtga tcttgagaac 2760
cttttcaaac c 2771

<210> 139
<211> 4264
<212> DNA
<213> NM_000179. Homo sapiens muts...[gi:4504190]

<400> 139

atttcccgcc agcaggagcc gcgcggtaga tgcggtgctt ttaggagctc cgtccgacag 60
aacggttggg ccttgccggc tgtcggtatg tcgcgacaga gcaccctgta cagcttcttc 120
cccaagtctc cggcgctgag tgatgccaac aaggcctcgg ccagggcctc acgcgaaggc 180
ggccgtgccg ccgctgcccc cggggcctct ccttccccag gcggggatgc ggcctggagc 240
gaggctgggc ctgggcccag gcccttggcg cgatccgcgt caccgcccaa ggcgaagaac 300
ctcaacggag ggctgcggag atcggtagcg cctgctgccc ccaccagttg tgacttctca 360
ccaggagatt tggtttgggc caagatggag ggttacccct ggtggccttg tctggtttac 420
aaccacccct ttgatggaac attcatccgc gagaaaggga aatcagtccg tgttcatgta 480
cagtttttttg atgacagccc aacaaggggc tgggttagca aaaggctttt aaagccatat 540
acaggttcaa aatcaaagga agcccagaag ggaggtcatt tttacagtgc aaagcctgaa 600
atactgagag caatgcaacg tgcagatgaa gccttaaata aagacaagat taagaggctt 660
gaattggcag tttgtgatga gccctcagag ccagaagagg aagaagagat ggaggtaggc 720
acaacttacg taacagataa gagtgaagaa gataatgaaa ttgagagtga agaggaagta 780
cagcctaaga cacaaggatc taggcgaagt agccgccaaa taaaaaaacg aagggtcata 840
tcagattctg agagtgacat tggtggctct gatgtggaat ttaagccaga cactaaggag 900
gaaggaagca gtgatgaaat aagcagtgga gtggggggata gtgagagtga aggcctgaac 960
agccctgtca aagttgctcg aaagcggaag agaatggtga ctggaaatgg ctctcttaaa 1020
aggaaaagct ctaggaagga aacgccctca gccaccaaac aagcaactag catttcatca 1080
gaaaccaaga atactttgag agctttctct gcccctcaaa attctgaatc ccaagcccac 1140
gttagtggag gtggtgatga cagtagtcgc cctactgttt ggtatcatga aactttagaa 1200
tggcttaagg aggaaaagag aagagatgag cacaggagga ggcctgatca ccccgatttt 1260
gatgcatcta cactctatgt gcctgaggat ttcctcaatt cttgtactcc tgggatgagg 1320
aagtggtggc agattaagtc tcagaacttt gatcttgtca tctgttacaa ggtggggaaa 1380
ttttatgagc tgtaccacat ggatgctctt attggagtca gtgaactggg gctggtattc 1440
atgaaaggca actgggccca ttctggcttt cctgaaattg catttggccg ttattcagat 1500
tccctggtgc agaagggcta taaagtagca cgagtggaac agactgagac tccagaaatg 1560
atggaggcac gatgtagaaa gatggcacat atatccaagt atgatagagt ggtgaggagg 1620
gagatctgta ggatcattac caagggtaca cagacttaca gtgtgctgga aggtgatccc 1680
tctgagaact acagtaagta tcttcttagc ctcaaagaaa aagaggaaga ttcttctggc 1740
catactcgtg catatggtgt gtgctttgtt gatacttcac tgggaaagtt tttcataggt 1800
cagttttcag atgatcgcca ttgttcgaga tttaggactc tagtggcaca ctatcccccca 1860
gtacaagttt tatttgaaaa aggaaatctc tcaaaggaaa ctaaaacaat tctaaagagt 1920

tcattgtcct gttctcttca ggaaggtctg atacccggct cccagttttg ggatgcatcc 1980
aaaactttga gaactctcct tgaggaagaa tattttaggg aaaagctaag tgatggcatt 2040
ggggtgatgt taccccaggt gcttaaaggt atgacttcag agtctgattc cattgggttg 2100
acaccaggag agaaaagtga attggccctc tctgctctag gtggttgtgt cttctacctc 2160
aaaaaatgcc ttattgatca ggagctttta tcaatggcta attttgaaga atatattccc 2220
ttggattctg acacagtcag cactacaaga tctggtgcta tcttcaccaa agcctatcaa 2280
cgaatggtgc tagatgcagt gacattaaac aacttggaga tttttctgaa tggaacaaat 2340
ggttctactg aaggaaccct actagagagg gttgatactt gccatactcc ttttggtaag 2400
cggctcctaa agcaatggct ttgtgcccca ctctgtaacc attatgctat taatgatcgt 2460
ctagatgcca tagaagacct catggttgtg cctgacaaaa tctccgaagt tgtagagctt 2520
ctaaagaagc ttccagatct tgagaggcta ctcagtaaaa ttcataatgt tgggtctccc 2580
ctgaagagtc agaaccaccc agacagcagg gctataatgt atgaagaaac tacatacagc 2640
aagaagaaga ttattgattt tctttctgct ctggaaggat tcaaagtaat gtgtaaaatt 2700
atagggatca tggaagaagt tgctgatggt tttaagtcta aaatccttaa gcaggtcatc 2760
tctctgcaga caaaaaatcc tgaaggtcgt tttcctgatt tgactgtaga attgaaccga 2820
tgggatacag cctttgacca tgaaaaggct cgaaagactg gacttattac tcccaaagca 2880
ggctttgact ctgattatga ccaagctctt gctgacataa gagaaaatga acagagcctc 2940
ctggaatacc tagagaaaca gcgcaacaga attggctgta ggaccatagt ctattggggg 3000
attggtagga accgttacca gctggaaatt cctgagaatt tcaccactcg caatttgcca 3060
gaagaatacg agttgaaatc taccaagaag ggctgtaaac gatactggac caaaactatt 3120
gaaaagaagt tggctaatct cataaatgct gaagaacgga gggatgtatc attgaaggac 3180
tgcatgcggc gactgttcta taactttgat aaaaattaca aggactggca gtctgctgta 3240
gagtgtatcg cagtgttgga tgttttactg tgcctggcta actatagtcg agggggtgat 3300
ggtcctatgt gtcgcccagt aattctgttg ccggaagata cccccccctt cttagagctt 3360
aaaggatcac gccatccttg cattacgaag actttttttg gagatgattt tattcctaat 3420
gacattctaa taggctgtga ggaagaggag caggaaaatg gcaaagccta ttgtgtgctt 3480
gttactggac caaatatggg gggcaagtct acgcttatga gacaggctgg cttattagct 3540
gtaatggccc agatgggttg ttacgtccct gctgaagtgt gcaggctcac accaattgat 3600
agagtgttta ctagacttgg tgcctcagac agaataatgt caggtgaaag tacatttttt 3660
gttgaattaa gtgaaactgc cagcatactc atgcatgcaa cagcacattc tctggtgctt 3720
gtggatgaat taggaagagg tactgcaaca tttgatggga cggcaatagc aaatgcagtt 3780
gttaaagaac ttgctgagac tataaaatgt cgtacattat tttcaactca ctaccattca 3840
ttagtagaag attattctca aaatgttgct gtgcgcctag gacatatggc atgcatggta 3900
gaaaatgaat gtgaagaccc cagccaggag actattacgt tcctctataa attcattaag 3960

```
ggagcttgtc ctaaaagcta tggctttaat gcagcaaggc ttgctaatct cccagaggaa   4020
gttattcaaa agggacatag aaaagcaaga gaatttgaga agatgaatca gtcactacga   4080
ttatttcggg aagtttgcct ggctagtgaa aggtcaactg tagatgctga agctgtccat   4140
aaattgctga ctttgattaa ggaattatag actgactaca ttggaagctt tgagttgact   4200
tctgaccaaa ggtggtaaat tcagacaaca ttatgatcta ataaacttta tttttaaaa   4260
atga                                                                4264
```

## Description

### Field of invention

The present invention relates to a method for classification of cancer in an individual, wherein the microsatellite status and a prognostic marker are determined by examining gene expression patterns. Various methods of treatment of cancer, a pharmaceutical composition for treatment of cancer, uses, and an assay for classification of cancer are also described.

### Background of invention

Studies of differential gene expression in diseased and normal tissues have been greatly facilitated by the building of large databases of the human genome sequences. Gene expression alterations are important factors in the progression from normal tissue to diseased tissue. In order to obtain a profile of transcriptional status in a certain cell type or tissue, array-based screening of thousands of genes simultaneously is an invaluable tool. Array-based screening even allows for the identification of key genes that alone, or in combination with other genes, regulate the behaviour of a cell or tissue. Candidate genes for future therapeutic intervention may thus also be identified.

Colorectal cancer generally occurs in 1 out of every 20 individuals at some point during their lifetime. In the United States alone about 150,000 new cases are diagnosed each year which amount to 15% of the total number of new cancer diagnoses. Unfortunately, colorectal cancer causes about 56,000 deaths a year in the United States.

The malignant transformation from normal tissue to cancer is believed to be a multistep process. Two molecular pathways are known to be involved in the development of colorectal cancer (Lengauer C, Kinzler KW, Vogelstein B., 1998) namely the microsatellite stable (MSS) pathway and the microsatellite instable (MSI) pathway. MSS is associated with high frequency of allelic losses, abnormalities of cytogenetic nature and abnormal tumor content of DNA. MSI however is associated with defects in the DNA mismatch repair system which leads to increased rate of point mutations and minor chromosomal insertions or deletions.

MSI tumors can be of hereditary or sporadic nature. Ninety percent of MSI tumours are of sporadic origin. Sporadic tumours are presumably MSI due to epigenetic hypermethylation of the MLH1 gene promoter. The hereditary tumours account for 10 % of the MSI tumors. Mutations of for example the MLH1 or MSH 2 genes are often the cause of hereditary tumor development.

The ability of being able to determine the sporadic or hereditary nature of a MSI tumor is highly valuable. In case a tumor is characterized as being MSI , and certain clinical criteria are fulfilled such as age below 50 or three first degree relatives with colon cancer, a screening programme of family members for early diagnosis and treatment of potential colon or endometrial cancer development is initiated. The human and economic costs in relation to screening programmes are severe. Consequently, a need for identifying colon cancers with a hereditary character exists. Further, these patients have a poor prognosis, as they have an increased risk of metachronous colon tumors and a highly increased risk of getting cancer in the endometrium (females), upper urinary tract and a number of other organs. Thus, one may regard the determination of a colon tumor as being sporadic or hereditary as determination of a prognostic factor.

Tumors appearing to be similar - morphologically, histochemically or microscopically - can be profoundly different. They can have different invasive and metastasizing properties, as well as respond differently to therapy. There is thus a need in the art for methods which distinguish tumors and tissues on different bases than are currently in use in the clinic. Determination of microsatellite status using an array-based methodology is faster than conventional DNA based methods, as it does not require microdissection, and forms a set of genes that can be combined with other sets of genes on a colon cancer array that can be used to determine microsatellite status as well as e.g. predict disease course by identifying hereditary cases or other prognostic important factors, and finally predict therapy response.

### Summary

In one aspect the present disclosure relates to a method of classifying cancer in an individual having contracted cancer comprising

in a sample from the individual having contracted cancer determining the microsatellite status of the tumor and

in a sample from the individual having contracted cancer, said sample comprising a plurality of gene expression products the presence and/or amount which forms a pattern, determining from said pattern a prognostic marker, wherein the microsatellite status and the prognostic marker is determined simultaneously or sequentially

classifying said cancer from the microsatellite status and the prognostic marker.

The cancer may be any cancer known to be microsatellite instable in at least a fraction of the cases, such as colon cancer, uterine cancer, ovary cancer, stomach cancer, cancer in the small intestine, cancer in the biliary system, urinary tract cancer, brain cancer or skin cancer. These cancers are part of the spectrum of cancers that belong to the hereditary non-polyposis colon cancer syndrome, but the invention is not limited to this syndrome.

Gene expression patterns may be formed by only a few genes, but it is also a preferred embodiment that a multiplicity of genes form the expression pattern whereby information for classification of cancer can be obtained.

Furthermore, the disclosure relates to a method for classification of cancer in an individual having contracted cancer, wherein the microsatellite status is determined by a method comprising the steps of

in a sample from the individual having contracted cancer, said sample comprising a plurality of gene expression products the presence and/or amount of which forms a pattern that is indicative of the microsatellite status of said cancer,

determining the presence and/or amount of said gene expression products forming said pattern,

obtaining an indication of the microsatellite status of said cancer in the individual based on the step above.

Yet another aspect of the disclosure relates to a method for classification cancer in an individual having contracted cancer, wherein the hereditary or sporadic nature is determined by a method comprising the steps of

in a sample from the individual having contracted cancer, said sample comprising a plurality of gene expression products the presence and/or amount of which forms a pattern that is indicative of the hereditary or sporadic nature of said cancer,

determining the presence and/or amount of said gene expression products forming said pattern,

obtaining an indication of the hereditary or sporadic nature of said cancer in the individual based on the step above.

The present disclosure further concerns a method for treatment of an individual comprising the steps of

selecting an individual having contracted a colon cancer, wherein the microsatellite status is stable, determined according to any of the methods as defined herein

treating the individual with anti cancer drugs.

Another aspect of the present disclosure relates to a method for treatment of an individual comprising the steps of

selecting an individual having contracted a colon cancer, wherein the microsatellite status is instable, determined according to any of the methods as defined herein

treating the individual with anti cancer drugs.

Yet another aspect of the present disclosure relates to a method for reducing malignancy of a cell, said method comprising

contacting a tumor cell in question with at least one peptide expressed by at least one gene selected from genes being expressed at least two-fold higher in tumor cells than the amount expressed in said tumor cell in question.

Additionally, the present disclosure concerns a method for reducing malignancy of a tumor cell in question comprising,

obtaining at least one gene selected from genes being expressed at least two fold lower in tumor cells than the amount expressed in normal cells

introducing said at least one gene into the tumor cell in question in a manner allowing expression of said gene(s).

The disclosure also relates to a method for reducing malignancy of a cell in question, said method comprising

obtaining at least one nucleotide probe capable of hybridising with at least one gene of a tumor cell in question, said at least one gene being selected from genes being expressed in an amount at least two-fold higher in tumor cells than the amount expressed in normal cells, and

introducing said at least one nucleotide probe into the tumor cell in question in a manner allowing the probe to hybridise to the at least one gene, thereby inhibiting expression of said at least one gene.

In a further aspect the disclosure relates to a method for producing antibodies against an expression product of a cell from a biological tissue, said method comprising the steps of

obtaining expression product(s) from at least one gene said gene being expressed as defined herein

immunising a mammal with said expression product(s) obtaining antibodies against the expression product.

The present disclosure also concerns a method for treatment of an individual comprising the steps of

selecting an individual having contracted a colon cancer, wherein the microsatellite status is stable, determined according to any of the methods as defined herein

introducing at least one gene into the tumor cell in a manner allowing expression of said gene(s).

The present disclosure further relates to a pharmaceutical composition for the treatment of a classified cancer comprising at least one antibody as defined herein.

In yet another aspect the disclosure concerns a pharmaceutical composition for the treatment of a classified cancer comprising at least one polypeptide as defined herein.

Further, the disclosure relates to a pharmaceutical composition for the treatment of a classified cancer comprising at least one nucleic acid and/or probe as defined herein.

In an additional aspect the present disclosure relates to an assay for classification of cancer in an individual having contracted cancer, comprising

at least one marker capable of determining the microsatellite status in a sample and

at least one marker in a sample determining the prognostic marker, wherein the microsatellite status and the prognostic marker is determined simultaneously or sequentially.

## Detailed description of the drawings

### Figure 1

**Unsupervised hierarchical clustering of colorectal tumors based on the 1239 genes with the highest variation across all tumors.**

The phylogenetic tree shows the spontaneous clustering of tumor samples and normal biopsies. Germline mutation indicates samples with hereditary mutations in either *MLH1* or *MSH2* genes. In columns referring to results of immunohistochemistry a plus indicates a positive antibody staining. Tumor location indicates right-sided or left-sided location in the colon of the tumor.

### Figure 2

**Summary of the performance of the microsatellite instability classifier based on microarray data.**

Panel A shows the number of classification errors as a function of the number of genes used. Panel B shows $\log_2$ of the ratio of the distance between a tumor to the centers of the microsatellite instable group and the microsatellite stable tumors. A value of +2 indicates that the distance of a tumor to the microsatellite instable group is 4 times the distance to the microsatellite stable group. Open bars are MSI tumors and solid bars are MSS tumors. Panel C shows the result of the permutation analysis for estimation of the stability of the classifier. This was estimated by generating one hundred new classifiers based on randomly chosen datasets from the 101 tumors each consisting of 30 microsatellite stable and 25 microsatellite instable samples. In each case the classifier was tested with the remaining 46 samples. The per formance for each set was evaluated and averaged over all 100 training and test sets.

### Figure 3

**Classification of MSI tumors as hereditary or sporadic cases based on two genes.**

Panel A shows the number of classification errors as a function of the number of genes used. In crossvalidation we found a minimum number of one error using two genes and adding more genes increased the number of errors to a maximum number of twelve. Both genes were used in at least 36 of the 37 crossvalidation loops. Panel B shows $\log_2$ of the ratio of the distance between a tumor to the centers of the sporadic microsatellite instable group and the hereditary microsatellite instable group. Panel C shows microarray signal values for *MLH1* and *PIWIL1* genes for all tumors. Asterisk indicates the misclassified tumor

### Figure 4

**Classification of microsatellite-instability status based on real-time PCR.**

Panel A shows a cluster analysis of 18 of the 101 tumors samples and 9 genes based on the microarray data and compared to real-time PCR data from same samples and genes. Dark colors indicate relative low expression and light/light grey color palette high expression. Panel B shows the result of 47 new independent samples based on PCR data from 7 of the 9 genes. Relative distances are explained in the legend to figure 2. The two misclassified tumors are indicated with an asterisk. For PCR primers and hybridization probes see supplement to methods.

### Figure 5

Kaplan-Meier estimates of crude survival among patient with Stage II and Stage III colorectal cancer according to microsatellite status of the tumor, determined by gene expression. Open triangles indicate censored samples. The patients left at risk are denoted in brackets. The P values were calculated with use of the log-rank test.

### Figure 6

Phylogenetic tree resulting from unsupervised hierarchical clustering. Clusteranalysis of colon specimens with associated clinicopathological features.

**Figure 7**

Multidimentional scaling plot showing distances between groups of tumors.

**Figure 8**

Performance of prediction of survival before and after separation in MSI-H and MSS

**Figure 9**

Performance of the classifier for identification of hereditary disease.

**Figure 10**

Kaplan Meier estimates of overall survival among patients with Dukes' B and Dukes' C colon cancer according to microsatellite-instability status of the tumor, determined by gene expression.

**Detailed description**

**Classification of cancer**

The present inventors have, using large-scale array-based screenings, found a pool of genes, the expression products of which may be used to classify cancer in an individual. The presence of expression products and level of expression products provides an expression pattern which is correlated to a specific status and/or prognostic marker of the cancer. Characterization of the genes or functional analysis of the gene expression products as such is not required to classify the cancer based on the present method. Thus, the expression products of the plurality of genes can be used as markers for the classification of disease.

One aspect of the present disclosure concerns a method for classifying cancer in an individual having contracted cancer by determining the microsatellite status and a prognostic marker in a sample. Determination of the microsatellite status and the prognostic marker may be performed simultaneously or sequentially. In one embodiment the microsatellite status is determined. The prognostic marker is determined in a sample, wherein the presence and/or the amount of a number of gene expression products form a pattern wherefrom the prognostic marker is determined. Based on the information gathered from the microsatellite status and the prognostic marker the cancer can be classified. In a preferred embodiment the prognostic marker is the hereditary or sporadic nature of the cancer. The hereditary or sporadic nature of the cancer can be determined through a number of steps comprising determining the presence and/or amount of gene expression products forming a pattern in a sample. The sample comprises a number of gene expression products the presence and/or amount of which forms a pattern that is indicative of the hereditary or sporadic nature of the cancer. Hereby, an indication of the hereditary or sporadic nature of the cancer is obtained.

In one embodiment the microsatellite status is determined using conventional analysis of microsatellite status as described elsewhere herein.

In another embodiment the microsatellite status is determined by gene expression patterns wherein the presence and/or the amount of the gene expression products form a pattern that is indicative of the microsatellite status.

Classification of cancer provides knowledge of the survival chances of an individual having contracted cancer. In case of cancer which according to the present invention has been classified as a hereditary cancer, screening programmes of family members to the individual having the classified cancer can be initiated. Such screening programmes can comprise conventional screening programmes employing sequencing and other methods as described elsewhere. Thus, individuals at risk of developing cancer may be identified and action taken accordingly to detect developing cancer at an early stage of the disease greatly improving the chances of successful intervention and thus survival rates.

Classification of cancer also provides insights on which sort of treatment should be offered to the individual having contracted cancer, thus providing an improved treatment response of the individual. Likewise, the individual may be spared treatment that is inefficient in treating the particular class of cancer and thus spare the individual severe side effects associated with treatment that may even not be suitable for the class of cancer.

**Microsatellite status**

The use of highly variable repetitive sequences found in microsatellite regions adjacent to genes or other areas of interest may be used as markers for linkage analysis, DNA fingerprinting, or other diagnostic application.

Microsatellites are defined as loci (or regions within DNA sequences) where short sequences of DNA are repeated in tandem repeats. This means that the sequences are repeated one right after the other. The lengths of sequences used most often are di-, tri-, or tetra-nucleotides. At the same location within the genomic DNA the number of times the sequence (ex. AC) is repeated often varies between individuals, within populations, and/or between species. Due to the many repeats the microsatellites are prone to alter if there is a reduced repair of mismatches in the genome. In the present disclosure the traditional method of determining microsatellite status by employing microsatellite markers is replaced by determination of gene expression patterns.

An important factor in multi-step carcinogenesis is genomic instability. The development of some cancer forms is known to follow two distinct molecular routes. One route is the microsatellite stable, MSS, (and chromosomal instable pathway) which is often associated with a high frequency of allelic losses, cytogenetic abnormalities and abnormal DNA tumor contents. The second route is the microsatellite instable pathway MSI that is characterized by defects in the DNA mismatch repair system which leads to a high rate of point mutations and small chromosomal insertions and deletions. The small chromosomal insertions and deletions can be detected as mono and dinucleotide repeats (Boland CR, Thibodeau SN, Hamilton SR, et al., Cancer Res 1998;58(22):5248-57).

Mori Y. et al. (Cancer Res. 63 (15): 4577-4582, 1 Aug. 2003) disclose markers (Table 2; Fig. 1c) that are capable of determining the microsatellite status in a sample.

One aspect of the present disclosure relates to the classification of cancer in an individual having contracted cancer by determining the microsatellite status and a prognostic marker. One embodiment relates to microsatellite status determined by conventional methods employing microsatellite analysis as described above. Another embodiment relates to establishing the microsatellite status by determining the presence and/or amount of gene expression products of a sample which comprises a plurality of gene expression products forming a pattern which is indicative of the microsatellite status.

The expression products of genes according to the present disclosure are not necessarily identical to the genes that are analysed by microsatellite markers in conventional methods of determining microsatellite status. The pattern of the gene expression products according to the present disclosure however correlates with information on microsatellite status that can be obtained using traditional methods.

The determination of the microsatellite status and the prognostic marker of the cancer may be performed sequentially. However, the determinations may also be performed simultaneously.

## Prognostic marker

Together with knowledge of the microsatellite status in a sample of an individual having contracted cancer a prognostic marker is employed for classifying the cancer. The prognostic marker may be any marker that provides knowledge of the cancer type when combined with knowledge of microsatellite status. Consequently the prognostic marker may provide additional information on the cancer type when the microsatellite status is stable and similarly when the microsatellite status is instable. In a preferred embodiment the prognostic marker is the hereditary or sporadic nature of a cancer given that the microsatellite status is instable. The prognostic marker may in another embodiment be a prognostic marker for any feature or trait that provides further possibilities of classifying cancer.
The prognostic marker is determined in a sample comprising a number of gene expression products wherein the presence and/or amounts of gene expression products form a pattern that is indicative of the prognostic marker.

## Hereditary and sporadic nature of cancer

Hereditary nonpolyposis colon cancer (HNPCC) is a hereditary cancer syndrome which carries a very high risk of colon cancer and an above-normal risk of other cancers (uterus, ovary, stomach, small intestine, biliary system, urinary tract, brain, and skin). The HNPCC syndrome is due to mutation in a gene in the DNA mismatch repair system, usually the MLH1 or MSH2 gene or less often the MSH6 or PMS2 genes. Families with HNPCC account for about 5% of all cases of colon cancer and typically have the following features (called the Amsterdam clinical criteria):

Three or more first relative family members with colorectal cancer; affected family members in two or more generations; and at least one person with colon cancer diagnosed before the age of 50.

The highest risk with HNPCC is for colon cancer. A person with HNPCC has about an 80% lifetime risk of colon cancer. Two-thirds of these tumors occur in the proximal colon. Women with HNPCC have a 20-60% lifetime risk of endometrial cancer. In HNPCC, the gastric cancer is usually intestinal-type adenocarcinoma. The ovarian cancer in HNPCC may be diagnosed before age 40. Other HNPCC-related cancers have characteristic features: the urinary tract cancers are transitional carcinoma of the ureter and renal pelvis; the small bowel cancer is most common in the duodenum and jejunum; and the most common type of brain tumor is glioblastoma. The diagnosis of HNPCC may be made on the basis of the Amsterdam clinical criteria (listed above) or on the basis of molecular genetic testing for mutations in a mismatch repair gene (MLH1, MSH2, MSH6 or PMS2). Mutations in MLH1 and MSH2 account for 90% of HNPCC. Mutations in MSH6 and PMS2 account for the rest. HNPCC is inherited in an autosomal dominant manner. Each child of an individual with HNPCC has a 50% chance of inheriting the mutation. Most people diagnosed with HNPCC have inherited the condition from a parent. However, not all individuals with an HNPCC gene mutation have a parent who had cancer. Prenatal diagnosis for pregnancies at increased risk for HNPCC is possible.

In tumors that are microsatellite instable it is often found that the DNA mismatch repair proteins that are encoded by the *MLH1* or *MSH2* genes are inactivated. In case of microsatellite instable hereditary non-polyposis colorectal cancers germline mutation in MLH1 and MSH2 and somatic loss of function of the normal allele has been found to be associated with the disease.

For most sporadic MSI tumors epigenetic hypermethylation of the MLH1 promoter can be found to be associated with the cancer (Cunningham JM, Christensen ER, Tester DJ, et al., Cancer Res 1998;58(15):3455-60., Kane MF, Loda M, Gaida GM, et al., Cancer Res 1997;57(5):808-11., Herman JG, Umar A, Polyak K, et al., Proc Natl Acad Sci U S A 1998;95(12):6870-5., Kuismanen SA, Holmberg MT, Salovaara R, de la Chapelle A, Peltomaki P., Am J Pathol 2000; 156(5):1773-9).

### Forms of cancer

Cancer leads to a change in the expression of one or more genes. The methods according to the disclosure may be used for classifying cancer according to the microsatellite status and/or the hereditary or sporadic nature of the cancer. Thus, the cancer may be any malignant condition in which genomic instability is involved in the development of cancer, such as cancers related to hereditary non-polyposis colorectal cancer, such as endometrial cancer, gastric cancer, small bowel cancer, ovarian cancer, kidney cancer, pelvic renal cancer or tumors of the nervous system, such as glioblastoma.

One particular form of cancer according to the present disclosure is that of the colon/rectum.

The cancer may be of any tumor type, such as an adenocarcinoma, a carcinoma, a teratoma, a sarcoma, and/or a lymphoma.

In relation to the gastro-intestinal tract, the biological condition may also be colitis ulcerosa, Mb. Crohn, diverticulitis, adenomas.

### Colorectal tumors

The data presented herein relates to colorectal tumors and therefore the description has focused on the gene expression level as one manner of identifying genes involved in the prediction of survival in cancer tissue. The malignant progression of cancer of colon or rectum may be described using Dukes stages where normal mucosa may progress to Dukes A superficial tumors to Dukes B, slightly invasive tumors, to Dukes C that have spread to lymphnodes and finally to Dukes D that have metastasized to other organs.

The grade of a tumor can also be expressed on a scale of I-IV. The grade reflects the cytological appearance of the cells. Grade I cells are almost normal, whereas grade II cells deviate slightly from normal. Grade III appear clearly abnormal, whereas grade IV cells are highly abnormal.

The phrase colon cancer is in this application meant to be equivalent to the phrase colorectal cancer. Colon cancers may be located in the right side of the colon, the left side of the colon, the transverse part of the colon and/or in the rectum.

### Samples

The samples according to the present disclosure may be any cancer tissue.
The sample may be in a form suitable to allow analysis by the skilled artisan, such as a biopsy of the tissue, or a superficial sample scraped from the tissue. In one embodiment it is preferred that the sample is from a resected colon cancer tumor. In another embodiment the sample may be prepared by forming a suspension of cells made from the tissue. The sample may, however, also be an extract obtained from the tissue or obtained from a cell suspension made from the tissue. The sample may be fresh or frozen, or treated with chemicals.

### Expression pattern

Expression of one gene or more genes in a sample forms a pattern that is characteristic of the state of the cell. In a sample from an individual having contracted cancer a plurality of gene expression products are present. By expression pattern is meant the presence of a combination of a number of expression products and/or the amount of expression products specific for a given biological condition, such as cancer. The pattern is produced by determining the expression products of selected genes that together reveals a pattern that is indicative of the biological condition. Thus, a selection of the genes that carry information about a specific condition is developed. Selection of the genes is achieved by analyzing large numbers of genes and their expression products to find the genes that will enable the desired differentiation between various conditions, such as microsatellite status (MSS or MSI) and/or prognostic marker, such as for example the sporadic or hereditary nature of a given cancer sample. The criteria for selection of the best genes for the pattern to be indicative of given biological conditions include confidence levels i.e. how accurate are the selected genes forming an expression pattern in giving correct information of the biological condition. Thus, in one aspect a specific pattern of gene expression profiles can be used to determine the microsatellite status in the sample. In a second aspect the microsatellite status is determined and a specific pattern of the presence of a plurality of gene expression products and/or amount wherefrom a prognostic marker is determined.

**Determination of the microsatellite status employing gene expression patterns**

One aspect of the disclosure specifically relates to a method for determining the microsatellite status in a sample of an individual having contracted cancer based on determination of the expression pattern of at least two genes, such as at least three genes, such as at least four genes, such as at least 5 genes, such as at least 6 genes, such as at least 7 genes, such as at least 8 genes, such as at least 9 genes, such as at least 10 genes, such as at least 15 genes, such as at least 20 genes, such as at least 30 genes, such as at least 40 genes, such as at least 50 genes, such as at least 60 genes, such as at least 70 genes, such as at least 80 genes, such as at least 90 genes, such as at least 126 genes selected from the group of genes listed in Table 1 below

Table 1

| Gene name | Ref seq | Gene symbol | SEQ ID NO.: |
|---|---|---|---|
| chemokine (C-C motif) ligand 5 | NM_002985 | CCL5 | 1 |
| tryptophanyl-tRNA synthetase | NM_004184 | WARS | 2 |
| proteasome (prosome, macropain) activator subunit 1 (PA28 alpha) | NM_006263 | PSME1 | 3 |
| bone marrow stromal cell antigen 2 | NM_004335 | BST2 | 4 |
| ubiquitin-conjugating enzyme E2L 6 | NM_004223 | UBE2L6 | 5 |
| A kinase (PRKA) anchor protein 1 | NM_003488 | AKAP1 | 6 |
| proteasome (prosome, macropain) activator subunit 2 (PA28 beta) | NM_002818 | PSME2 | 7 |
| carcinoembryonic antigen-related cell adhesion molecule 5 | NM_004363 | CEACAM5 | 8 |
| FERM, RhoGEF (ARHGEF) and pleckstrin domain protein 1 (chondrocyte-derived) | NM_005766 | FARP1 | 9 |
| myosin X | NM_012334 | MYO10 | 10 |
| heterogeneous nuclear ribonucleoprotein L | NM_001533 | HNRPL | 11 |
| autocrine motility factor receptor | NM_001144 | AMFR | 12 |
| dimethylarginine dimethylaminohydrolase 2 | NM_013974 | DDAH2 | 13 |
| tumor necrosis factor, alpha-induced protein 2 | NM_006291 | TNFAIP2 | 14 |
| mutL homolog 1, colon cancer, nonpolyposis type 2 (E. coli) | NM_000249 | MLH1 | 15 |
| thymidylate synthetase | NM_001071 | TYMS | 16 |
| intercellular adhesion molecule 1 (CD54), human rhinovirus receptor | NM_000201 | ICAM1 | 17 |
| general transcription factor IIA, 2, 12kDa | NM_004492 | GTF2A2 | 18 |
| Rho-associated, coiled-coil containing protein kinase 2 | NM_004850 | ROCK2 | 19 |
| ATP binding protein associated with cell differentiation | NM_005783 | TXNDC9 | 20 |
| NCK adaptor protein 2 | NM_003581 | NCK2 | 21 |
| phytanoyl-CoA hydroxylase (Refsum disease) | NM_006214 | PHYH | 22 |
| metastais-associated gene family, member 2 | NM_004739 | MTA2 | 23 |
| amiloride binding protein 1 (amine oxidase (copper-containing)) | NM_001091 | ABP1 | 24 |
| biliverdin reductase A | NM_000712 | BLVRA | 25 |
| phospholipase C, beta 4 | NM_000933 | PLCB4 | 26 |
| chemokine (C-X-C motif) ligand 9 | NM_002416 | CXCL9 | 27 |
| purine-rich element binding protein A | NM_005859 | PURA | 28 |
| quinolinate phosphoribosyltransferase (nicotinate-nucleotide pyrophosphorylase (carboxylating)) | NM_014298 | QPRT | 29 |

(continued)

| Gene name | Ref seq | Gene symbol | SEQ ID NO.: |
|---|---|---|---|
| retinoic acid receptor responder (tazarotene induced) 3 | NM_004585 | RARRES3 | 30 |
| chemokine (C-C motif) ligand 4 | NM_002984 | CCL4 | 31 |
| forkhead box 03A | NM_001455 | FOX03A | 32 |
| interferon, alpha-inducible protein (clone IFI-6-16) | NM_002038 | G1 P3 | 34 |
| | NM_022873 | | 123 |
| chemokine (C-X-C motif) ligand 10 | NM_001565 | CXCL10 | 35 |
| | NM_005950 | MT1G | 36 |
| metallothionein 1G | NM_005950 | | |
| | NM_000043 | TNFRSF6 | 37 |
| tumor necrosis factor receptor superfamily, | NM_152877 | | 133 |
| member 6 | NM_152876 | | 132 |
| | NM_152875 | | 134 |
| | NM_152872 | | 130 |
| | NM_152873 | | 33 |
| | NM_152871 | | 129 |
| | NM_152874 | | 131 |
| endothelial cell growth factor 1 (platelet-derived) | NM_001953 | ECGF1 | 38 |
| SCO cytochrome oxidase deficient homolog 2 (yeast) | NM_005138 | SCO2 | 39 |
| chemokine (C-X-C motif) ligand 13 (B-cell chemoattractant) | NM_006419 | CXCL13 | 40 |
| Granulysin | NM_006433 | GNLY | 41 |
| CD2 antigen (p50), sheep red blood cell receptor | NM_001767 | CD2 | 42 |
| splicing factor, arginine/serine-rich 6 | NM_006275 | SFRS6 | 43 |
| teratocarcinoma-derived growth factor 1 | NM_003212 | TDGF1 | 44 |
| metallothionein 1H | NM_005951 | MT1H | 45 |
| cytochrome P450, family 2, subfamily B, polypeptide 6 | NM_000767 | CYP2B6 | 46 |
| tumor necrosis factor (ligand) superfamily, member 9 | NM_003811 | TNFSF9 | 47 |
| | NM_006047 | RBM12 | 48 |
| RNA binding motif protein 12 | NM_006047 | | |
| heat shock 105kDa/110kDa protein 1 | NM_006644 | HSPH1 | 49 |
| staufen, RNA binding protein (Drosophila) | NM_004602 | STAU | 50 |
| | NM_017452 | | 125 |
| | NM_017453 | | 126 |
| lymphocyte antigen 6 complex, locus G6D | NM_021246 | LY6G6D | 51 |
| calcium binding protein P22 | NM_007236 | CHP | 52 |
| CDC14 cell division cycle 14 homolog B (S. cerevisiae) | NM_003671 | CDC14B | 53 |
| | NM_033331 | | 115 |
| epiplakin 1 | XM_372063 | EPPK1 | 54 |
| metallothionein 1X | NM_005952 | MT1X | 55 |
| transforming growth factor, beta receptor II (70/80kDa) | NM_003242 | TGFBR2 | 56 |
| protein kinase C binding protein 1 | NM 012408 | PRKCBP1 | 57 |
| | NM_183047 | | 124 |
| transmembrane 4 superfamily member 6 | NM_003270 | TM4SF6 | 58 |
| pleckstrin homology domain containing, family B (evectins) member 1 | NM_021200 | PLEKHB1 | 59 |
| apolipoprotein L, 1 | NM_003661 | APOL1 | 60 |
| | NM_145343 | | 120 |

(continued)

| Gene name | Ref seq | Gene symbol | SEQ ID NO.: |
|---|---|---|---|
| indoleamine-pyrrole 2,3 dioxygenase | NM_002164 | INDO | 61 |
| forkhead box A2 | NM_021784 | FOXA2 | 62 |
| granzyme H (cathepsin G-like 2, protein h-CCPX) | NM_033423 | GZMH | 63 |
| baculoviral IAP repeat-containing 3 | NM_001165 | BIRC3 | 64 |
| Homo sapiens metallothionein 1H-like protein | | AF333388 (Hs 382039) | 135 |
| KIAA0182 protein | NM_014615 | KIAA0182 | 117 |
| G protein-coupled receptor 56 | NM_005682 | GPR56 | 65 |
| | NM_201524 | | 116 |
| metallothionein 2A | NM_005953 | MT2A | 66 |
| F-box only protein 21 | NM_015002 | FBXO21 | 67 |
| erythrocyte membrane protein band 4.1-like 1 | NM_012156, NM_012156 | EPB41L1 | 68 |
| hypothetical protein MGC21416 | NM_173834 | MGC21416 | 69 |
| protein O-fucosyltransferase 1 | NM_015352, NM_015352 | POFUT1 | 70 |
| metallothionein 1 E (functional) | NM_175617 | MT1E | 71 |
| troponin T1, skeletal, slow | NM_003283 | TNNT1 | 72 |
| chimerin (chimaerin) 2 | NM_004067 | CHN2 | 73 |
| heterogeneous nuclear ribonucleoprotein H1 (H) | NM_005520 | HNRPH1 | 74 |
| ATP synthase, H+ transporting, mitochondrial F1 complex, alpha subunit, isoform 1, cardiac muscle | NM_004046 | ATP5A1 | 75 |
| eukaryotic translation initiation factor 5A | NM_001970 | EIF5A | 76 |
| perforin 1 (pore forming protein) | NM_005041 | PRF1 | 77 |
| OGT(O-Glc-NAc transferase)-interacting protein 106 KDa | NM_014965 | OIP106 | 78 |
| DEAD (Asp-Glu-Ala-Asp) box polypeptide 27 | NM_017895 | DDX27 | 79 |
| vacuolar protein sorting 35 (yeast) | NM_018206 | VPS35 | 80 |
| tripartite motif-containing 44 | NM_017583 | TRIM44 | 81 |
| transmembrane, prostate androgen induced | NM_020182 | TMEPAI | 82 |
| RNA | NM_199169 | | 127 |
| | NM_199170 | | 128 |
| dynein, cytoplasmic, light polypeptide 2A | NM_014183 | DNCL2A | 83 |
| | NM_177953 | | 122 |
| leucine aminopeptidase 3 | NM_015907 | LAP3 | 84 |
| chromosome 20 open reading frame 35 | NM_018478 | C20orf35 | 85 |
| | NM_033542 | | 118 |
| solute carrier family 38, member 1 | NM_030674 | SLC38A1 | 86 |

(continued)

| Gene name | Ref seq | Gene symbol | SEQ ID NO.: |
|---|---|---|---|
| CGI-85 protein | NM_016028 | CGI-85 | 87 |
| death associated transcription factor 1 | NM_022105, | DATF1 | 88 |
| | NM_080796 | | 121 |
| hepatocellular carcinoma-associated antigen 112 | NM_018487 | HCA112 | 89 |
| sestrin 1 | NM_014454 | SESN1 | 90 |
| hypothetical protein FLJ20315 | NM_017763 | FLJ20315 | 91 |
| hypothetical protein FLJ20647 | NM_017918 | FLJ20647 | 92 |
| membrane protein expressed in epithelial-like lung adenocarcinoma | NM_024792 | CT120 | 93 |
| DEAD/H (Asp-Glu-Ala-Asp/His) box polypeptide | NM_014314 | RIG-I | 94 |
| keratin 23 (histone deacetylase inducible) | NM_015515, | KRT23 | 95 |
| UDP-N-acetyl-alpha-D-galactosamine:polypeptide N- acetylgalactosaminyltransferase 6 (GaINAc-T6) | NM_007210 | GALNT6 | 96 |
| aryl hydrocarbon receptor nuclear translocator-like 2 | NM_020183 | ARNTL2 | 97 |
| apobec-1 complementation factor | NM_014576, | ACF | 98 |
| | NM_138932 | | 119 |
| hypothetical protein FLJ20232 | NM_019008 | FLJ20232 | 99 |
| apolipoprotein L, 2 | NM_030882, | APOL2 | 100 |
| | NM_145343 | | 120 |
| mitochondrial solute carrier protein | NM_016612 | MSCP | 101 |
| hypothetical protein FLJ20618 | NM_017903 | FLJ20618 | 102 |
| SET translocation (myeloid leukaemia-associated) | NM_003011.1 | SET | 103 |
| ATPase, class II, type 9a | Xm_030577.9 | ATP9a | 104 |

One embodiment concerning the determination of microsatellite status is based on the expression pattern of at least 2 genes, such as at least 3 genes, such as at least 4 genes, such as at least 5 genes, such as at least 6 genes, such as at least 7 genes, such as at least 8 genes, such as at least 9 genes, such as at least 10 genes, such as at least 15 genes, such as at least 20 genes, such as at least 25 genes selected from the group of genes listed in Table 2.

Table 2

| Gene name | Ref seq | Gene symbol | SEQ ID NO.: |
|---|---|---|---|
| chemokine (C-C motif) ligand 5 | NM_002985 | CCL5 | 1 |
| tryptophanyl-tRNA synthetase | NM_004184 | WARS | 2 |
| proteasome (prosome, macropain) activator subunit 1 (PA28 alpha) | NM_006263 | PSME1 | 3 |
| bone marrow stromal cell antigen 2 | NM_004335 | BST2 | 4 |
| ubiquitin-conjugating enzyme E2L 6 | NM_004223 | UBE2L6 | 5 |
| A kinase (PRKA) anchor protein 1 | NM_003488 | AKAP1 | 6 |
| proteasome (prosome, macropain) activator subunit 2 (PA28 beta) | NM_002818 | PSME2 | 7 |
| carcinoembryonic antigen-related cell adhesion molecules 5 | NM_004363 | CEACAM5 | 8 |

(continued)

| Gene name | Ref seq | Gene symbol | SEQ ID NO.: |
|---|---|---|---|
| FERM, RhoGEF (ARHGEF) and pleckstrin domain protein 1 (chondrocyte-derived) | NM_005766 | FARP1 | 9 |
| myosin X | NM_012334 | MYO10 | 10 |
| heterogeneous nuclear ribonucleoprotein L | NM_001533 | HNRPL | 11 |
| autocrine motility factor receptor | NM_001144 | AMFR | 12 |
| dimethylarglnine dimethylaminohydrolase 2 | NM_013974 | DDAH2 | 13 |
| tumor necrosis factor, alpha-induced protein 2 | NM_006291 | TNFAIP2 | 14 |
| mutL homolog 1, colon cancer, nonpolyposis type 2 (E. coli) | NM_000249 | MLH1 | 15 |
| thymidylate synthetase | NM_001071 | TYMS | 16 |
| intercellular adhesion molecule 1 (CD54), human rhinovirus receptor | NM_000201 | ICAM1 | 17 |
| general transcription factor IIA, 2, 12kDa | NM_004492 | GTF2A2 | 18 |
| Rho-associated, coiled-coil containing protein kinase 2 | NM_004850 | ROCK2 | 19 |
| ATP binding protein associated with cell differentiation | NM_005783 | APACD | 20 |
| metastais-associated gene family, member 2 | NM_004739 | MTA2 | 23 |
| chemokine (C-X-C motif) ligand 10 | NM_001565 | CXCL10 | 35 |
| splicing factor, arginine/serine-rich 6 | NM_006275 | SFRS6 | 43 |
| protein kinase C binding protein 1 | NM_012408 | PRKCBP1 | 57 |
| | NM_183047 | | 124 |
| hepatocellular carcinoma-associated antigen 112 | NM_018487 | HCA112 | 89 |
| hypothetical protein FLJ20618 | NM_017903 | FLJ20618 | 102 |
| SET translocation (myeloid leukaemia-associated ) | NM_003011.1 | SET | 103 |
| ATPase, class II, type 9a | Xm_030577.9 | ATP9a | 104 |

or from

Table 3

| Gene name | Ref seq | Gene symbol | SEQ ID NO.: |
|---|---|---|---|
| heterogeneous nuclear ribonucleoprotein L | NM_001533 | HNRPL | 11 |
| NCK adaptor protein 2 | NM_003581 | NCK2 | 21 |
| phytanoyl-CoA hydroxylase (Refsum disease) | NM_006214 | PHYH | 22 |
| metastais-associated gene family, member 2 | NM_004739 | MTA2 | 23 |
| amiloride binding protein 1 (amine oxidase (copper-containing)) | NM_001091 | ABP1 | 24 |
| biliverdin reductase A | NM_000712 | BLVRA | 25 |
| phospholipase C, beta 4 | NM_000933 | PLCB4 | 26 |
| chemokine (C-X-C motif) ligand 9 | NM_002416 | CXCL9 | 27 |
| purine-rich element binding protein A | NM_005859 | PURA | 28 |
| quinolinate phosphoribosyltransferase (nicotinate-nucleotide pyrophosphorylase (carboxylating)) | NM_014298 | QPRT | 29 |
| retinoic acid receptor responder (tazarotene induced) 3 | NM_004585 | RARRES3 | 30 |
| chemokine (C-C motif) ligand 4 | NM_002984 | CCL4 | 31 |
| forkhead box 03A | NM_001455 | FOX03A | 32 |
| metallothionein 1X | NM_005952 | MT1X | 55 |

(continued)

| Gene name | Ref seq | Gene symbol | SEQ ID NO.: |
|---|---|---|---|
| interferon, alpha-inducible protein (clone IFI-6-16) | NM_002038 | G1P3 | 34 |
| | NM_022873 | | 123 |
| chemokine (C-X-C motif) ligand 10 | NM_001565 | CXCL10 | 35 |
| | NM_005950, | MT1G | 36 |
| metallothionein 1G | NM_005950 | | |
| | NM_000043 | TNFRSF6 | 37 |
| tumor necrosis factor receptor superfamily, member 6 | NM_152877 | | 133 |
| | NM_152876 | | 132 |
| | NM_152875 | | 134 |
| | NM_152872 | | 130 |
| | NM_152873 | | 33 |
| | NM_152871 | | 129 |
| | NM_152874 | | 131 |
| endothelial cell growth factor 1 (platelet-derived) | NM_001953 | ECGF1 | 38 |
| SCO cytochrome oxidase deficient homolog 2 (yeast) | NM_005138 | SCO2 | 39 |
| chemokine (C-X-C motif) ligand 13 (B-cell chemoattractant) | NM_006419 | CXCL13 | 40 |
| | NM_006433 | GNLY | 41 |
| Granulysin | | | |
| splicing factor, arginine/serine-rich 6 | NM_006275 | SFRS6 | 43 |
| | NM_012408 | PRKCBP1 | 57 |
| | NM_183047 | | 124 |
| protein kinase C binding protein 1 | | | |
| hepatocellular carcinoma-associated antigen 112 | NM_018487 | HCA112 | 89 |
| hypothetical protein FLJ20618 | NM_017903 | FLJ20618 | 102 |
| SET translocation (myeloid leukaemia-associated) | NM_003011.1 | SET | 103 |
| ATPase, class II, type 9a | Xm_030577.9 | ATP9a | 104 |

or from

Table 4

| Gene name | Ref seq | Gene symbol | SEQ ID NO.: |
|---|---|---|---|
| heterogeneous nuclear ribonucleoprotein L | NM_001533 | HNRPL | 11 |
| metastais-associated gene family, member 2 | NM_004739 | MTA2 | 23 |
| chemokine (C-X-C motif) ligand 10 | NM_001565 | CXCL10 | 35 |
| CD2 antigen (p50), sheep red blood cell receptor | NM_001767 | CD2 | 42 |
| splicing factor, arginine/serine-rich 6 | NM_006275 | SFRS6 | 43 |
| teratocarcinoma-derived growth factor 1 | NM_003212 | TDGF1 | 44 |
| metallothionein 1H | NM_005951 | MT1H | 45 |
| cytochrome P450, family 2, subfamily B, polypeptide 6 | NM_000767 | CYP2B6 | 46 |
| tumor necrosis factor (ligand) superfamily, member 9 | NM_003811 | TNFSF9 | 47 |
| | NM_006047, | RBM12 | 48 |
| RNA binding motif protein 12 | NM_006047 | | |
| heat shock 105kDa/110kDa protein 1 | NM_006644 | HSPH1 | 49 |
| staufen, RNA binding protein (Drosophila) | NM_004602 | STAU | 50 |
| | NM_017452 | | 125 |

(continued)

| Gene name | Ref seq | Gene symbol | SEQ ID NO.: |
|---|---|---|---|
| | NM_017453 | | 126 |
| lymphocyte antigen 6 complex, locus G6D | NM_021246 | LY6G6D | 51 |
| calcium binding protein P22 | NM_007236 | CHP | 52 |
| CDC14 cell division cycle 14 homolog B (S. cerevisiae) | NM_003671 | CDC14B | 53 |
| | NM_033331 | | 115 |
| epiplakin 1 | XM_372063 | EPPK1 | 54 |
| metallothionein 1X | NM_005952 | MT1X | 55 |
| transforming growth factor, beta receptor II (70/80kDa) | NM_003242 | TGFBR2 | 56 |
| protein kinase C binding protein 1 | NM_012408 | PRKCBP1 | 57 |
| | NM_183047 | | 129 |
| transmembrane 4 superfamily member 6 | NM_003270 | TM4SF6 | 58 |
| pleckstrin homology domain containing, family B (evectins) member 1 | NM_021200 | PLEKHB1 | 59 |
| apolipoprotein L, 1 | NM_003661 | APOL1 | 60 |
| | NM_145343 | | 125 |
| indoleamine-pyrrole 2,3 dioxygenase | NM_002164 | INDO | 61 |
| | NM_021784 | FOXA2 | 62 |
| forkhead box A2 | NM_021784 | | |
| hepatocellular carcinoma-associated antigen 112 | NM_018487 | HCA112 | 89 |
| mitochondrial solute carrier protein | NM_016612 | MSCP | 101 |
| | NM_016612 | | |
| hypothetical protein FLJ20618 | NM_017903 | FLJ20618 | 102 |
| SET translocation (myeloid leukaemia-associated) | NM_003011.1 | SET | 103 |
| ATPase, class II, type 9a | Xm_030577.9 | ATP9a | 104 |

or from

Table 5

| Gene name | Ref seq | Gene symbol | SEQ ID NO.: |
|---|---|---|---|
| heterogeneous nuclear ribonucleoprotein L | NM_001533 | HNRPL | 11 |
| metastais-associated gene family, member 2 | NM_004739 | MTA2 | 23 |
| chemokine (C-X-C motif) ligand 10 | NM_001565 | CXCL10 | 35 |
| splicing factor, arginine/serine-rich 6 | NM_006275 | SFRS6 | 43 |
| protein kinase C binding protein 1 | NM_012408 | PRKCBP1 | 57 |
| | NM_183047 | | 124 |
| granzyme H (cathepsin G-like 2, protein h-CCPX) | NM_033423 | GZMH | 63 |
| | NM_001165 | BIRC3 | 64 |
| baculoviral IAP repeat-containing 3 | NM_001165 | | |
| | | AF333388 | 135 |

(continued)

| Gene name | Ref seq | Gene symbol | SEQ ID NO.: |
|---|---|---|---|
| Homo sapiens metallothionein 1H-like protein | | (Hs 382039) | |
| KIAA0182 protein | NM_014615 | KIAA0182 | 117 |
| | NM_005682 | GPR56 | 65 |
| G protein-coupled receptor 56 | NM_301524 | | 116 |
| metallothionein 2A | NM_005953 | MT2A | 66 |
| F-box only protein 21 | NM_015002 | FBX021 | 67 |
| erythrocyte membrane protein band 4.1-like 1 | NM_012156 | EPB41L1 | 68 |
| hypothetical protein MGC21416 | NM_173834 | MGC21416 | 69 |
| protein O-fucosyltransferase 1 | NM_015352 | POFUT1 | 70 |
| metallothionein 1 E (functional) | NM_175617 | MT1E | 71 |
| | NM_003283 | TNNT1 | 72 |
| troponin T1, skeletal, slow | | | |
| chimerin (chimaerin) 2 | NM_004067 | CHN2 | 73 |
| heterogeneous nuclear ribonucleoprotein H1 (H) | NM_005520 | HNRPH1 | 74 |
| ATP synthase, H+ transporting, mitochondrial F1 complex, alpha subunit, isoform 1, cardiac muscle | NM_004046 | ATP5A1 | 75 |
| eukaryotic translation initiation factor 5A | NM_001970 | EIF5A | 76 |
| perforin 1 (pore forming protein) | NM_005041 | PRF1 | 77 |
| OGT(O-Glc-NAc transferase)-interacting protein 106 KDa | NM_014965 | OIP106 | 78 |
| DEAD (Asp-Glu-Ala-Asp) box polypeptide 27 | NM_017895 | DDX27 | 79 |
| hepatocellular carcinoma-associated antigen 112 | NM_018487 | HCA112 | 89 |
| hypothetical protein FLJ20232 | NM_019008 | FLJ20232 | 99 |
| | NM_030882, | APOL2 | 100 |
| apolipoprotein L, 2 | NM_145343 | | 120 |
| hypothetical protein FLJ20618 | NM_017903 | FLJ20618 | 102 |
| SET translocation (myeloid leukaemia-associated) | NM_003011.1 | SET | 103 |
| ATPase, class II, type 9a | Xm_030577.9 | ATP9a | 104 |

or from

Table 6

| Gene name | Ref seq | Gene symbol | SEQ ID NO.: |
|---|---|---|---|
| heterogeneous nuclear ribonucleoprotein L | NM_001533 | HNRPL | 11 |
| metastais-associated gene family, member 2 | NM_004739 | MTA2 | 23 |
| chemokine (C-X-C motif) ligand 10 | NM_001565 | CXCL10 | 35 |
| metallothionein 1G | NM_005950 | MT1G | 36 |

(continued)

| Gene name | Ref seq | Gene symbol | SEQ ID NO.: |
|---|---|---|---|
| splicing factor, arginine/serine-rich 6 | NM_006275 | SFRS6 | 43 |
| protein kinase C binding protein 1 | NM_012408 | PRKCBP1 | 57 |
| | NM_183047 | | 129 |
| vacuolar protein sorting 35 (yeast) | NM_018206 | VPS35 | 80 |
| tripartite motif-containing 44 | NM_017583 | TRIM44 | 81 |
| | NM_020182 | TMEPAI | 82 |
| | NM_199169 | | 127 |
| transmembrane, prostate androgen induced RNA | NM_199170 | | 128 |
| dynein, cytoplasmic, light polypeptide 2A | NM_014183 | DNCL2A | 83 |
| | NM_177953 | | 122 |
| leucine aminopeptidase 3 | NM_015907 | LAP3 | 84 |
| chromosome 20 open reading frame 35 | NM_018478 | C20orf35 | 85 |
| | NM_033542 | | 118 |
| solute carrier family 38, member 1 | NM_030674 | SLC38A1 | 86 |
| CGI-85 protein | NM_016028 | CGI-85 | 87 |
| death associated transcription factor 1 | NM_022105, | DATF1 | 88 |
| | NM_080796 | | 121 |
| hepatocellular carcinoma-associated antigen 112 | NM_018487 | HCA112 | 89 |
| sestrin 1 | NM_014454 | SESN1 | 90 |
| hypothetical protein FLJ20315 | NM_017763 | FLJ20315 | 91 |
| hypothetical protein FLJ20647 | NM_017918 | FLJ20647 | 92 |
| membrane protein expressed in epithelial-like lung adenocarcinoma | NM_024792 | CT120 | 93 |
| DEAD/H (Asp-Glu-Ala-Asp/His) box polypeptide | NM_014314 | RIG-I | 94 |
| keratin 23 (histone deacetylase inducible) | NM_015515 | KRT23 | 95 |
| UDP-N-acetyl-alpha-D-galactosamine:polypeptide N-acetylgalactosaminyltransferase 6 (GalNAc-T6) | NM_007210 | GALNT6 | 96 |
| aryl hydrocarbon receptor nuclear translocator-like 2 | NM_020183 | ARNTL2 | 97 |
| apobec-1 complementation factor | NM_014576 | ACF | 98 |
| | NM_138932 | | 119 |
| hypothetical protein FLJ20618 | NM_017903 | FLJ20618 | 102 |
| SET translocation (myeloid leukaemia-associated) | NM_003011.1 | SET | 103 |
| ATPase, class II, type 9a | Xm_030577.9 | ATP9a | 104 |

Another embodiment concerning the determination of microsatellite status is based on the expression pattern of at least 2 genes, such as at least 3 genes, such as at least 4 genes, such as at least 5 genes, such as at least 6 genes, such as at least 7 genes, such as at least 8 genes, such as at least 9 genes selected from the group of genes listed in Table 7 below.

Table 7

| Gene name | Ref seq | Gene symbol | SEQ ID NO.: |
|---|---|---|---|
| heterogeneous nuclear ribonucleoprotein L | NM_001533 | HNRPL | 11 |
| metastais-associated gene family, member 2 | NM_004739 | MTA2 | 23 |
| chemokine (C-X-C motif) ligand 10 | NM_001565 | CXCL10 | 35 |
| splicing factor, arginine/serine-rich 6 | NM_006275 | SFRS6 | 43 |
| protein kinase C binding protein 1 | NM_012408<br>NM_183047 | PRKCBP1 | 57<br>124 |
| hepatocellular carcinoma-associated antigen 112 | NM_018487 | HCA112 | 89 |
| hypothetical protein FLJ20618 | NM_017903 | FLJ20618 | 102 |
| SET translocation (myeloid leukaemia-associated) | NM_003011.1 | SET | 103 |
| ATPase, class II, type 9a | Xm_030577.9 | ATP9a | 104 |

Another embodiment concerning the determination of microsatellite status is based on the expression pattern of at least 2 genes, such as at least 3 genes, such as at least 4 genes, such as at least 5 genes, such as at least 6 genes, such as at least 7 genes selected from the group of genes listed in Table 8 below.

Table 8

| Gene name | Ref seq | Gene symbol | SEQ ID NO.: |
|---|---|---|---|
| heterogeneous nuclear ribonucleoprotein L | NM_001533 | HNRPL | 11 |
| metastais-associated gene family, member 2 | NM_004739 | MTA2 | 23 |
| chemokine (C-X-C motif) ligand 10 | NM_001565 | CXCL10 | 35 |
| Splicing factor, arginine/serine-rich 6 | NM_006275 | SFRS6 | 43 |
| protein kinase C binding protein 1 | NM_012408<br>NM_183047 | PRKCBP1 | 57<br>124 |
| hepatocellular carcinoma-associated antigen 112 | NM_018487 | HCA112 | 89 |
| hypothetical protein FLJ20618 | NM_017903 | FLJ20618 | 102 |

One embodiment concerning the determination of microsatellite status is based on the expression pattern of at least one gene, for example two genes, for example 3 genes, such as 4 genes selected from the group of genes listed below in Table 9 and at least one gene, for example two genes, for example 3 genes, such as 4 genes, for example 5 genes selected from the group of genes listed below in Table 10

Table 9

| Gene name | Ref seq | Gene symbol | SEQ ID NO.: |
|---|---|---|---|
| heterogeneous nuclear ribonucleoprotein L | NM_001533 | HNRPL | 11 |
| metastais-associated gene family, member 2 | NM_004739 | MTA2 | 23 |
| chemokine (C-X-C motif) ligand 10 | NM_001565 | CXCL10 | 35 |
| Splicing factor, arginine/serine-rich 6 | NM_006275 | SFRS6 | 43 |

and

Table 10

| Gene name | Ref seq | Gene symbol | SEQ ID NO.: |
|---|---|---|---|
| protein kinase C binding protein 1 | NM_012408<br>NM_183047 | PRKCBP1 | 57<br>124 |
| hepatocellular carcinoma-associated antigen 112 | NM_018487 | HCA112 | 89 |
| hypothetical protein FLJ20618 | NM_017903 | FLJ20618 | 102 |
| SET translocation (myeloid leukaemia-associated) | NM_003011.1 | SET | 103 |
| ATPase, class II, type 9a | Xm_030577.9 | ATP9a | 104 |

Another embodiment relates to the determination of microsatellite status is based on the expression pattern of at least one gene, for example 2 genes, for example 3 genes, such as 4 genes selected from the group of genes that are down regulated in MSS colon cancers compared to MSI colon cancers listed below in Table 11, and at least one gene, for example 2 genes, for example 3 genes, such as 4 genes, for example 5 genes selected from the group of genes that are up-regulated in MSS colon cancers compared to MSI colon cancers listed below in Table 12.

Table 11

| Gene name | Ref seq | Gene symbol | SEQ ID NO.: |
|---|---|---|---|
| heterogeneous nuclear ribonucleoprotein L | NM_001533 | HNRPL | 11 |
| metastais-associated gene family, member 2 | NM_004739 | MTA2 | 23 |
| chemokine (C-X-C motif) ligand 10 | NM 001565 | CXCL10 | 35 |
| Splicing factor, arginine/serine-rich 6 | NM 006275 | SFRS6 | 43 |

Table 12

| Gene name | Ref seq | Gene symbol | SEQ ID NO.: |
|---|---|---|---|
| protein kinase C binding protein 1 | NM_012408<br>NM_183047 | PRKCBP1 | 57<br>124 |
| hepatocellular carcinoma-associated antigen 112 | NM_018487 | HCA112 | 89 |
| hypothetical protein FLJ20618 | NM_017903 | FLJ20618 | 102 |
| SET translocation (myeloid leukaemia-associated) | NM_003011.1 | SET | 103 |
| ATPase, class II, type 9a | Xm_030577.9 | ATP9a | 104 |

**Sporadic or hereditary classification using gene expression patterns**

One embodiment relates to a method of determining the hereditary or sporadic nature of cancer as the prognostic marker in an individual having contracted cancer based on determination of the expression pattern of at least 2 genes, such as at least 3 genes, such as at least 4 genes, such as at least 5 genes, such as at least 6 genes, such as at least 7 genes, such as at least 8 genes, such as at least 9 genes, such as at least 10 genes selected from the group of genes listed in Table 13.

Table 13

| Gene name | Ref seq | Gene symbol I | SEQ ID NO.: |
|---|---|---|---|
| Homeo box C6 | NM_004503 | HOXC6 | 105 |
| Piwi - like 1 | NM_004764.2 | PIWIL1 | 106 |
| Mut L homolog 1 | NM_00249.2 | MLH1 | 107 |
| Collapsin response mediator protein 1 | NM_001313.2 | CRMP1 | 108 |
| Homeo box B2 | NM_002145.2 | HOXB2 | 109 |
| Pyrroline-5-carboxylate synthetase (glutamate gamma-semialdehyd synthetase) | NM_002860.2 | PYCS/ADH A1 | 18110 |
| TGFB inducible early growth response | NM_005655.1 | TIEG | 111 |
| Checkpoint with forkhead and ring finger domains | NM_018223.1 | CHFR | 112 |
| Hypothetical protein FLJ13842 | NM_024645.1 | FLJ13842 | 113 |
| Phosphoprotein regulated by mitogenic pathways | NM_025195.1 | C8FW | 114 |

In a further embodiment the determination of the hereditary or sporadic nature of cancer is based on the expression pattern of at least 2 genes as listed in Table 14.

Table 14

| Gene name | Ref seq | Gene symbol I | SEQ ID NO.: |
|---|---|---|---|
| Piwi - like 1 | NM_004764.2 | pIWIL1 | 105 |
| Mut L homolog 1 | NM_00249.2 | MLH1 | 106 |

In one specific embodiment the MLH1 gene is down regulated in sporadic disease. In yet another embodiment the PIWIL1 is expressed in lower amounts in hereditary cancer.

**Gene names and definition**

The genes according to the present disclosure are identified by their gene name, gene symbol and a reference sequence number (RefSeq). Furthemore, the genes listed have been assigned a SEQ ID No and the sequence is submitted in the accompanying sequence listing. The reference sequence number refers to the Reference Sequence collection prepared by the the National Center for Biotechnologic Information (NCBI) and where a comprehensive set of sequences for major research organisms is provided, see http://www.ncbi.nlm.nih.gov/RefSeq.

**Sample preparation**

A number of procedures for the isolation of nucleic acids (DNA, RNA, mRNA) from a sample are available and well known to a person skilled in the art. Genomic DNA may be isolated for detection of mutations of the genome, or for detection of copy number of a gene or a number of other applications which will be appreciated by the skilled artisan. RNA and especially mRNA will be isolated when expression levels of a gene or several genes are to be detected. The sample may be from fresh or frozen tissue as defined elsewhere herein.

Before analyzing the sample by for example oligonucleotide arrays or quantitative PCR one or more preparations of the sample may be performed. Often, sample preparations include extraction of intracellular material for example extraction of nucleic acids from whole cell samples, viruses and the like, amplification of nucleic acids, fragmentation, transcription, labelling and/or extension reactions. One or more of these preparation features may be incorporated readily into the present disclosure.

**RNA extraction**

Methods of isolating total RNA and/or mRNA are well known to those skilled in the art. In one embodiment total RNA is isolated from a given sample by extraction using acidic guanidinium-phenol-chloroform extraction. mRNA may be isolated form RNA or directly for example based on the polyadenylated tail of mRNA using oligo dT column chromatography or by using (dT)n magnetic beads (see, e.g. Sambrook et al., Molecular Cloning: A laboratory Manual 2nd Ed.), Vols 1-3, Cold Spring Harbour Laboratory (1989), or Current Protocols in Molecular Biology, F.Ausubel et al., ed. Greene Publishing and Wiley-Interscience, New York (1987).

**PCR**

PCR (Polymerase Chain Reaction) is a key technique in molecular genetics that permits the analysis of any short sequence of DNA or RNA without having to clone the short sequence first. PCR is used to reproduce (amplify) selected sections of DNA or RNA. PCR amplification generally involves the use of one strand of the target nucleic acid sequence as a template for producing a large number of complements to that sequence. Generally, two primer sequences complementary to different ends of a segment of the complementary strands of the target sequence hybridize with their respective strands of the target sequence, and in the presence of polymerase enzymes and nucleoside triphosphates, the primers are extended along the target sequence. The extensions are melted from the target sequence and the process is repeated, this time with the additional copies of the target sequence synthesized in the preceding steps. PCR amplification typically involves repeated cycles of denaturation, hybridization and extension reactions to produce sufficient amounts of the target nucleic acid. The first step of each cycle of the PCR involves the separation of the nucleic acid duplex formed by the primer extension. Once the strands are separated, the next step in PCR involves hybridizing the separated strands with primers that flank the target sequence. The primers are then extended to form complementary copies of the target strands. For successful PCR amplification, the primers are designed so that the position at which each primer hybridizes along a duplex sequence is such that an extension product synthesized from one primer, when separated from the template (complement), serves as a template for the extension of the other primer. The cycle of denaturation, hybridization, and extension is repeated as many times as necessary to obtain the desired amount of amplified nucleic acid.

In PCR methods, strand separation is normally achieved by heating the reaction to a sufficiently high temperature for a sufficient time to cause the denaturation of the duplex but not to cause an irreversible denaturation of the polymerase. Typical heat denaturation involves temperatures ranging from about 80°C to 105° C for times ranging from seconds to minutes. Strand separation, however, can be accomplished by any suitable denaturing method including physical, chemical, or enzymatic means. Strand separation may be induced by a helicase, for example, or an enzyme capable of exhibiting helicase activity.

In addition to PCR reactions, the methods and devices of the present disclosure are also applicable to a number of other reaction types, e.g., reverse transcription, nick translation, and the like.

PCR may also be used to quantify the amount of transcripts of a particular gene. Typically, quantitative PCR also called real-time PCR is performed on cDNA synthesised from mRNA. Methods of "quantitative" amplification are well known to those of skill in the art. For example, quantitative PCR involves simultaneously co-amplifying a known quantity of a control sequence using the same primers. This provides an internal standard that may be used to calibrate the PCR reaction. The high density array may then include probes specific to the internal standard for quantification of the amplified nucleic acid.

Thus, in one embodiment, a method of detecting of the expression pattern formed by a number of genes is provided. Generally, this method involves providing a high density array containing a multiplicity of probes of one or more particular length(s) that are complementary to subsequences of the mRNA transcribed by the target gene. In one embodiment the high density array may contain every probe of a particular length that is complementary to a particular mRNA. The probes of the high density array are then hybridized with their target nucleic acid alone and then hybridized with a high complexity, high concentration nucleic acid sample that does not contain the targets complementary to the probes. Thus, for example, where the target nucleic acid is an RNA, the probes are first hybridized with their target nucleic acid alone and then hybridized with RNA made from a cDNA library (e.g., reverse transcribed polyA.sup.+ mRNA) where the sense of the hybridized RNA is opposite that of the target nucleic acid (to insu re that the high complexity sample does not contain targets for the probes). Those probes that show a strong hybridization signal with their target and little or no cross-hybridization with the high complexity sample are preferred probes for use in the high density arrays of this disclosure.

The method of measuring the level of expression of a number of genes forming a pattern is, however, not limited to the methods described herein, but includes any quantitative measurement.

**Fragmentation**

In addition, amplified sequences may be subjected to other post amplification treatments. For example, in some cases, it may be desirable to fragment the sequence prior to hybridization with an oligonucleotide array, in order to provide segments which are more readily accessible to the probes, which avoid looping and/or hybridization to multiple probes. Fragmentation of the nucleic acids may generally be carried out by physical, chemical or enzymatic methods that are known in the art.

## Hybridization

Following sample preparation, the sample can be subjected to one or more different analysis operations. A variety of analysis operations may generally be performed, including size based analysis using, e.g., microcapillary electrophoresis, and/or sequence based analysis using, e.g., hybridization to an oligonucleotide array.

In the latter case, the nucleic acid sample may be probed using an array of oligonucleotide probes. Oligonucleotide arrays generally include a substrate having a large number of positionally distinct oligonucleotide probes attached to the substrate. These arrays may be produced using mechanical or light directed synthesis methods which incorporate a combination of photolithographic methods and solid phase oligonucleotide synthesis methods.

## Detection

In the present context high density expression arrays may be used to determine the presence and/or amounts of gene expression products in a sample. Likewise, low density expression arrays may be used to determine the expression pattern in a sample.

While high density expression arrays can be used, other techniques are also contemplated. These include other techniques for assaying for specific mRNA species, including RT-PCR and Northern Blotting, as well as techniques for assaying for particular protein products, such as ELISA, Western blotting, and enzyme assays. Gene expression patterns according to the present disclosure are determined by measuring any gene product of a particular gene, including mRNA and protein. A pattern may be for two or more genes.

RNA or protein can be isolated and assayed from a test sample using any techniques known in the art. RNA or protein can for example be isolated from fresh or frozen biopsy, from formalin-fixed tissue.

Expression of genes may in general be detected by either detecting mRNA from the cells and/or detecting expression products, such as peptides and proteins.

## mRNA detection

The detection of mRNA may be a tool for determining the developmental stage of a cell type which is defined by its pattern of expression of messenger RNA. For example, in particular stages of cells, high levels of ribosomal RNA are found whereas relatively low levels of other types of messenger RNAs may be found. Where a pattern is shown to be characteristic of a stage, a stage may be defined by that particular pattern of messenger RNA expression. The mRNA population is a good determinant of developmental stage, will be correlated with other structural features of the cell. In this manner, cells at specific developmental stages will be characterized by the intracellular environment, as well as the extracellular environment. The present disclosure also allows the combination of definitions based, in part, upon antigens and, in part, upon mRNA expression.

In one embodiment, the two may be combined in a single incubation step. A particular incubation condition may be found which is compatible with both hybridization recognition and non-hybridization recognition molecules. Thus, e.g., an incubation condition may be selected which allows both specificity of antibody binding and specificity of nucleic acid hybridization. This allows simultaneous performance of both types of interactions on a single matrix. Again, where developmental mRNA patterns are correlated with structural features, or with probes which are able to hybridize to intracellular mRNA populations, a cell sorter may be used to sort specifically those cells having desired mRNA population patterns.

It is within the general scope of the present disclosure to provide methods for the detection of mRNA. Such methods often involve sample extraction, PCR amplification, nucleic acid fragmentation and labeling, extension reactions, transcription reactions and the like.

## DNA extraction

DNA extraction may be relevant in case possible mutations in the genes are to be determined in addition to the determination of expression of the genes.

For those embodiments where whole cells, or other tissue samples are being analyzed, it will typically be necessary to extract the nucleic acids from the cells or viruses, prior to continuing with the various sample preparation operations. Accordingly, following sample collection, nucleic acids may be liberated from the collected cells, viral coat, etc., into a crude extract, followed by additional treatments to prepare the sample for subsequent operations, e.g., denaturation of contaminating (DNA binding) proteins, purification, filtration, desalting, and the like.

Liberation of nucleic acids from the sample cells, and denaturation of DNA binding proteins may generally be performed by physical or chemical methods. For example, chemical methods generally employ lysing agents to disrupt the cells and extract the nucleic acids from the cells, followed by treatment of the extract with chaotropic salts such as guanidinium isothiocyanate or urea to denature any contaminating and potentially interfering proteins.

Alternatively, physical methods may be used to extract the nucleic acids and denature DNA binding proteins, such as physical protrusions within microchannels or sharp edged particles piercing cell membranes and extract their contents. Combinations of such structures with piezoelectric elements for agitation can provide suitable shear forces for lysis.

More traditional methods of cell extraction may also be used, e.g., employing a channel with restricted cross-sectional dimension which causes cell lysis when the sample is passed through the channel with sufficient flow pressure. Alternatively, cell extraction and denaturing of contaminating proteins may be carried out by applying an alternating electrical current to the sample. More specifically, the sample of cells is flowed through a microtubular array while an alternating electric current is applied across the fluid flow. Subjecting cells to ultrasonic agitation or forcing cells through microgeometry apertures, thereby subjecting the cells to high shear stress resulting in rupture are also possible extraction methods.

**Filtration**

Following extraction, it will often be desirable to separate the nucleic acids from other elements of the crude extract, e.g., denatured proteins, cell membrane particles, salts, and the like. Removal of particulate matter is generally accomplished by filtration, flocculation or the like. Further, where chemical denaturing methods are used, it may be desirable to desalt the sample prior to proceeding to the next step. Desalting of the sample, and isolation of the nucleic acid may generally be carried out in a single step, e.g., by binding the nucleic acids to a solid phase and washing away the contaminating salts or performing gel filtration chromatography on the sample, passing salts through dialysis membranes, and the like. Suitable solid supports for nucleic acid binding include, e.g., diatomaceous earth, silica (i.e., glass wool), or the like. Suitable gel exclusion media, also well known in the art, may also be readily incorporated into the devices of the present disclosure and is commercially available from, e.g., Pharmacia and Sigma Chemical.

Alternatively, desalting methods may generally take advantage of the high electrophoretic mobility and negative of DNA compared to other elements. Electrophoretic methods may also be utilized in the purification of nucleic acids from other cell contaminants and debris. Upon application of an appropriate electric field, the nucleic acids present in the sample will migrate toward the positive electrode and become trapped on the capture membrane. Sample impurities remaining free of the membrane are then washed away by applying an appropriate fluid flow. Upon reversal of the voltage, the nucleic acids are released from the membrane in a substantially purer form. Further, coarse filters may also be overlaid on the barriers to avoid any fouling of the barriers by particulate matter, proteins or nucleic acids, thereby permitting repeated use.

**Separation of contaminants by chromatography**

In a similar aspect, the high electrophoretic mobility of nucleic acids with their negative charges, may be utilized to separate nucleic acids from contaminants by utilizing a short column of a gel or other appropriate matrix or gel which will slow or retard the flow of other contaminants while allowing the faster nucleic acids to pass.

This disclosure provides nucleic acid affinity matrices that bear a large number of different nucleic acid affinity ligands allowing the simultaneous selection and removal of a large number of preselected nucleic acids from the sample. Methods of producing such affinity matrices are also provided. In general the methods involve the steps of a) providing a nucleic acid amplification template array comprising a surface to which are attached at least 50 oligonucleotides having different nucleic acid sequences, and wherein each different oligonucleotide is localized in a predetermined region of said surface, the density of said oligonucleotides is greater than about 60 different oligonucleotides per 1 cm.sup.2, and all of said different oligonucleotides have an identical terminal 3' nucleic acid sequence and an identical terminal 5' nucleic acid sequence. b) amplifying said multiplicity of oligonucleotides to provide a pool of amplified nucleic acids; and c) attaching the pool of nucleic acids to a solid support.

For example, nucleic acid affinity chromatography is based on the tendency of complementary, single-stranded nucleic acids to form a double-stranded or duplex structure through complementary base pairing. A nucleic acid (either DNA or RNA) can easily be attached to a solid substrate (matrix) where it acts as an immobilized ligand that interacts with and forms duplexes with complementary nucleic acids present in a solution contacted to the immobilized ligand. Unbound components can be washed away from the bound complex to either provide a solution lacking the target molecules bound to the affinity column, or to provide the isolated target molecules themselves. The nucleic acids captured in a hybrid duplex can be separated and released from the affinity matrix by denaturation either through heat, adjustment of salt concentration, or the use of a destabilizing agent such as formamide, TWEEN.TM.-20 denaturing agent, or sodium dodecyl sulfate (SDS).

Affinity columns (matrices) are typically used either to isolate a single nucleic acid typically by providing a single species of affinity ligand. Alternatively, affinity columns bearing a single affinity ligand (e.g. oligo dt columns) have been used to isolate a multiplicity of nucleic acids where the nucleic acids all share a common sequence (e.g. a polyA).

**Affinity matrices**

The type of affinity matrix used depends on the purpose of the analysis. For example, where it is desired to analyze mRNA expression levels of particular genes in a complex nucleic acid sample (e.g., total mRNA) it is often desirable to eliminate nucleic acids produced by genes that are constitutively overexpressed and thereby tend to mask gene products expressed at characteristically lower levels. Thus, in one embodiment, the affinity matrix can be used to remove a number of preselected gene products (e.g., actin, GAPDH, etc.). This is accomplished by providing an affinity matrix bearing nucleic acid affinity ligands complementary to the gene products (e.g., mRNAs or nucleic acids derived therefrom) or to subsequences thereof. Hybridization of the nucleic acid sample to the affinity matrix will result in duplex formation between the affinity ligands and their target nucleic acids. Upon elution of the sample from the affinity matrix, the matrix will retain the duplexes nucleic acids leaving a sample depleted of the overexpressed target nucleic acids.

The affinity matrix can also be used to identify unknown mRNAs or cDNAs in a sample. Where the affinity matrix contains nucleic acids complementary to every known gene (e.g., in a cDNA library, DNA reverse transcribed from an mRNA, mRNA used directly or amplified, or polymerized from a DNA template) in a sample, capture of the known nucleic acids by the affinity matrix leaves a sample enriched for those nucleic acid sequences that are unknown. In effect, the affinity matrix is used to perform a subtractive hybridization to isolate unknown nucleic acid sequences. The remaining "unknown" sequences can then be purified and sequenced according to standard methods.

The affinity matrix can also be used to capture (isolate) and thereby purify unknown nucleic acid sequences. For example, an affinity matrix can be prepared that contains nucleic acid (affinity ligands) that are complementary to sequences not previously identified, or not previously known to be expressed in a particular nucleic acid sample. The sample is then hybridized to the affinity matrix and those sequences that are retained on the affinity matrix are "unknown" nucleic acids. The retained nucleic acids can be eluted from the matrix (e.g. at increased temperature, increased destabilizing agent concentration, or decreased salt) and the nucleic acids can then be sequenced according to standard methods.

Similarly, the affinity matrix can be used to efficiently capture (isolate) a number of known nucleic acid sequences. Again, the matrix is prepared bearing nucleic acids complementary to those nucleic acids it is desired to isolate. The sample is contacted to the matrix under conditions where the complementary nucleic acid sequences hybridize to the affinity ligands in the matrix. The non-hybridized material is washed off the matrix leaving the desired sequences bound. The hybrid duplexes are then denatured providing a pool of the isolated nucleic acids. The different nucleic acids in the pool can be subsequently separated according to standard methods (e.g. gel electrophoresis).

As indicated above the affinity matrices can be used to selectively remove nucleic acids from virtually any sample containing nucleic acids (e.g., in a cDNA library, DNA reverse transcribed from an mRNA, mRNA used directly or amplified, or polymerized from a DNA template, and so forth). The nucleic acids adhering to the column can be removed by washing with a low salt concentration buffer, a buffer containing a destabilizing agent such as formamide, or by elevating the column temperature.

In one particularly preferred embodiment, the affinity matrix can be used in a method to enrich a sample for unknown RNA sequences (e.g. expressed sequence tags (ESTs)). The method involves first providing an affinity matrix bearing a library of oligonucleotide probes specific to known RNA (e.g., EST) sequences. Then, RNA from undifferentiated and/or unactivated cells and RNA from differentiated or activated or pathological (e.g., transformed) or otherwise having a different metabolic state are separately hybridized against the affinity matrices to provide two pools of RNAs lacking the known RNA sequences.

In a preferred embodiment, the affinity matrix is packed into a columnar casing. The sample is then applied to the affinity matrix (e.g. injected onto a column or applied to a column by a pump such as a sampling pump driven by an autosampler). The affinity matrix (e.g. affinity column) bearing the sample is subjected to conditions under which the nucleic acid probes comprising the affinity matrix hybridize specifically with complementary target nucleic acids. Such conditions are accomplished by maintaining appropriate pH, salt and temperature conditions to facilitate hybridization as discussed above.

For a number of applications, it may be desirable to extract and separate messenger RNA from cells, cellular debris, and other contaminants. As such, the device of the present disclosure may, in some cases, include an mRNA purification chamber or channel. In general, such purification takes advantage of the poly-A tails on mRNA. In particular and as noted above, poly- T oligonucleotides may be immobilized within a chamber or channel of the device to serve as affinity ligands for mRNA. Poly-T oligonucleotides may be immobilized upon a solid support incorporated within the chamber or channel, or alternatively, may be immobilized upon the surface(s) of the chamber or channel itself. Immobilization of oligonucleotides on the surface of the chambers or channels may be carried out by methods described herein including, e.g., oxidation and silanation of the surface followed by standard DMT synthesis of the oligonucleotides.

In operation, the lysed sample is introduced to a high salt solution to increase the ionic strength for hybridization, whereupon the mRNA will hybridize to the immobilized poly-T. The mRNA bound to the immobilized poly-T oligonucleotides is then washed free in a low ionic strength buffer. The poly-T oligonucleotides may be immobiliized upon porous surfaces, e.g., porous silicon, zeolites silica xerogels, scintered particles, or other solid supports.

**Light directed synthesis of oligonucleotide arrays**

The basic strategy for light directed synthesis of oligonucleotide arrays is as follows. The surface of a solid support, modified with photosensitive protecting groups is illuminated through a photolithographic mask, yielding reactive hydroxyl groups in the illuminated regions. A selected nucleotide, typically in the form of a 3'-O-phosphoramidite-activated deoxynucleoside (protected at the 5' hydroxyl with a photosensitive protecting group), is then presented to the surface and coupling occurs at the sites that were exposed to light. Following capping and oxidation, the substrate is rinsed and the surface is illuminated through a second mask, to expose additional hydroxyl groups for coupling. A second selected nucleotide (e.g., 5'-protected, 3'-O-phosphoramidite-activated deoxynucleoside) is presented to the surface. The selective deprotection and coupling cycles are repeated until the desired set of products is obtained. Since photolithography is used, the process can be readily miniaturized to generate high density arrays of oligonucleotide probes. Furthermore, the sequence of the oligonucleotides at each site is known. See, Pease, et al. Mechanical synthesis methods are similar to the light directed methods except involving mechanical direction of fluids for deprotection and addition in the synthesis steps.

For some embodiments, oligonucleotide arrays may be prepared having all possible probes of a given length. The hybridization pattern of the target sequence on the array may be used to reconstruct the target DNA sequence. Hybridization analysis of large numbers of probes can be used to sequence long stretches of DNA or provide an oligonucleotide array which is specific and complementary to a particular nucleic acid sequence. For example, in particularly preferred aspects, the oligonucleotide array will contain oligonucleotide probes which are complementary to specific target sequences, and individual or multiple mutations of these. Such arrays are particularly useful in the diagnosis of specific disorders which are characterized by the presence of a particular nucleic acid sequence.

Following sample collection and nucleic acid extraction, the nucleic acid portion of the sample is typically subjected to one or more preparative reactions. These preparative reactions include in vitro transcription, labeling, fragmentation, amplification and other reactions. Nucleic acid amplification increases the number of copies of the target nucleic acid sequence of interest. A variety of amplification methods are suitable for use in the methods and device of the present disclosure, including for example, the polymerase chain reaction method or (PCR), the ligase chain reaction (LCR), self sustained sequence replication (3SR), and nucleic acid based sequence amplification (NASBA).

The latter two amplification methods involve isothermal reactions based on isothermal transcription, which produce both single stranded RNA (ssRNA) and double stranded DNA (dsDNA) as the amplification products in a ratio of approximately 30 or 100 to 1, respectively. As a result, where these latter methods are employed, sequence analysis may be carried out using either type of substrate, i.e., complementary to either DNA or RNA.

Frequently, it is desirable to amplify the nucleic acid sample prior to hybridization. One of skill in the art will appreciate that whatever amplification method is used, if a quantitative result is desired, care must be taken to use a method that maintains or controls for the relative frequencies of the amplified nucleic acids.

**Labelling of nucleic acids**

The nucleic acids in a sample will generally be labeled to facilitate detection in subsequent steps. Labeling may be carried out during the amplification, in vitro transcription or nick translation processes. In particular, amplification, in vitro transcription or nick translation may incorporate a label into the amplified or transcribed sequence, either through the use of labeled primers or the incorporation of labeled dNTPs into the amplified sequence.

Hybridization between the sample nucleic acid and the oligonucleotide probes upon the array is then detected, using, e.g., epifluorescence confocal microscopy. Typically, sample is mixed during hybridization to enhance hybridization of nucleic acids in the sample to nucleoc acid probes on the array.

In some cases, hybridized oligonucleotides may be labeled following hybridization. For example, where biotin labeled dNTPs are used in, e.g., amplification or transcription, streptavidin linked reporter groups may be used to label hybridized complexes. Such operations are readily integratable into the systems of the present disclosure. Alternatively, the nucleic acids in the sample may be labeled following amplification. Post amplification labeling typically involves the covalent attachment of a particular detectable group upon the amplified sequences. Suitable labels or detectable groups include a variety of fluorescent or radioactive labeling groups well known in the art. These labels may also be coupled to the sequences using methods that are well known in the art.

Methods for detection depend upon the label selected. A fluorescent label is preferred because of its extreme sensitivity and simplicity. Standard labeling procedures are used to determine the positions where interactions between a sequence and a reagent take place. For example, if a target sequence is labeled and exposed to a matrix of different probes, only those locations where probes do interact with the target will exhibit any signal. Alternatively, other methods may be used to scan the matrix to determine where interaction takes place. Of course, the spectrum of interactions may be determined in a temporal manner by repeated scans of interactions which occur at each of a multiplicity of conditions. However, instead of testing each individual interaction separately, a multiplicity of sequence interactions may be simultaneously determined on a matrix.

Means of detecting labeled target (sample) nucleic acids hybridized to the probes of the high density array are known to those of skill in the art. Thus, for example, where a colorimetric label is used, simple visualization of the label is sufficient. Where a radioactive labeled probe is used, detection of the radiation (e.g with photographic film or a solid state detector) is sufficient.

In a preferred embodiment, however, the target nucleic acids are labeled with a fluorescent label and the localization of the label on the probe array is accomplished with fluorescent microscopy. The hybridized array is excited with a light source at the excitation wavelength of the particular fluorescent label and the resulting fluorescence at the emission wavelength is detected. In a particularly preferred embodiment, the excitation light source is a laser appropriate for the excitation of the fluorescent label.

The target polynucleotide may be labeled by any of a number of convenient detectable markers. A fluorescent label is preferred because it provides a very strong signal with low background. It is also optically detectable at high resolution and sensitivity through a quick scanning procedure. Other potential labeling moieties include, radioisotopes, chemiluminescent compounds, labeled binding proteins, heavy metal atoms, spectroscopic markers, magnetic labels, and linked enzymes. Another method for labeling may bypass any label of the target sequence. The target may be exposed to the probes, and a double strand hybrid is formed at those positions only. Addition of a double strand specific reagent will detect where hybridization takes place. An intercalative dye such as ethidium bromide may be used as long as the probes themselves do not fold back on themselves to a significant extent forming hairpin loops. However, the length of the hairpin loops in short oligonucleotide probes would typically be insufficient to form a stable duplex.

Suitable chromogens will include molecules and compounds which absorb light in a distinctive range of wavelengths so that a color may be observed, or emit light when irradiated with radiation of a particular wave length or wave length range, e.g., fluorescers. Biliproteins, e.g., phycoerythrin, may also serve as labels.

A wide variety of suitable dyes are available, being primarily chosen to provide an intense color with minimal absorption by their surroundings. Illustrative dye types include quinoline dyes, triarylmethane dyes, acridine dyes, alizarine dyes, phthaleins, insect dyes, azo dyes, anthraquinoid dyes, cyanine dyes, phenazathionium dyes, and phenazoxonium dyes. A wide variety of fluorescers may be used either on their own or in conjunction with quencher molecules. Fluorescers of interest fall into a number of categories having certain primary functionalities. These primary functionalities include 1- and 2-aminonaphthalene, p,p'-diaminostilbenes, pyrenes, quaternary phenanthridine salts, 9-aminoacridines, p,p'-diaminobenzophenone imines, anthracenes, oxacarbocyanine, merocyanine, 3-aminoequilenin, perylene, bis-benzoxazole, bis-p-oxazolyl benzene, 1,2-benzophenazin, retinol, bis-3-aminopyridinium salts, hellebrigenin, tetracycline, sterophenol, benzimidzaolylphenylamine, 2-oxo-3-chromen, indole, xanthen, 7-hydroxycoumarin, phenoxazine, salicylate, strophanthidin, porphyrins, triarylmethanes and flavin. Individual fluorescent compounds which have functionalities for linking or which can be modified to incorporate such functionalities include, e.g., dansyl chloride; fluoresceins such as 3,6-dihydroxy-9-phenylxanthhydrol; rhodamineisothiocyanate; N-phenyl 1-amino-8-sulfonatonaphthalene; N-phenyl 2-amino-6-sulfonatonaphthalene; 4-acetamido-4-isothiocyanato-stilbene-2,2'-disulfonic acid; pyrene-3-sulfonic acid; 2-toluidinonaphthalene-6-sulfonate; N-phenyl, N-methyl 2-aminoaphthalene-6-sulfonate; ethidium bromide; stebrine; auromine-0,2-(9'-anthroyl)palmitate; dansyl phosphatidylethanolamine; N,N'-dioctadecyl oxacarbocyanine; N,N'-dihexyl oxacarbocyanine; merocyanine, 4-(3'pyrenyl)butyrate; d-3-aminodesoxy-equilenin; 12-(9'-anthroyl)stearate; 2-methylanthracene; 9-vinylanthracene; 2,2'-(vinylene-p-phenylene)bisbenzoxazole; p-bis>2-(4-methyl-5-phenyl-oxazolyl)!benzene; 6-dimethylamino-1,2-benzophenazin; retinol; bis(3'-aminopyridinium) 1,10-decandiyl diiodide; sulfonaphthylhydrazone of hellibrienin; chlorotetracycline; N-(7-dimethylamino-4-methyl-2-oxo-3-chromenyl)maleimide; N->p-(2-benzimidazolyl)-phenyl!maleimide; N-(4-fluoranthyl)maleimide; bis(homovanillic acid); resazarin; 4-chloro-7-nitro-2,1,3-benzooxadiazole; merocyanine 540; resorufin; rose bengal; and 2,4-diphenyl-3(2H)-furanone.

Desirably, fluorescers should absorb light above about 300 nm, preferably about 350 nm, and more preferably above about 400 nm, usually emitting at wavelengths greater than about 10 nm higher than the wavelength of the light absorbed. It should be noted that the absorption and emission characteristics of the bound dye may differ from the unbound dye. Therefore, when referring to the various wavelength ranges and characteristics of the dyes, it is intended to indicate the dyes as employed and not the dye which is unconjugated and characterized in an arbitrary solvent.

Fluorescers are generally preferred because by irradiating a fluorescer with light, one can obtain a plurality of emissions. Thus, a single label can provide for a plurality of measurable events.

Detectable signal may also be provided by chemiluminescent and bioluminescent sources. Chemiluminescent sources include a compound which becomes electronically excited by a chemical reaction and may then emit light which serves as the detectible signal or donates energy to a fluorescent acceptor. A diverse number of families of compounds have been found to provide chemiluminescence under a variety of conditions. One family of compounds is 2,3-dihydro-1,-4-phthalazinedione. The most popular compound is luminol, which is the 5-amino compound. Other members of the family include the 5-amino-6,7,8-trimethoxy- and the dimethylamino>ca!benz analog. These compounds can be made to luminesce with alkaline hydrogen peroxide or calcium hypochlorite and base. Another family of compounds is the 2,4,5-triphenylimidazoles, with lophine as the common name for the parent product. Chemiluminescent analogs include para-dimethylamino and - methoxy substituents. Chemiluminescence may also be obtained with oxalates, usually oxalyl active esters, e.g., p-nitrophenyl and a peroxide, e.g., hydrogen peroxide, under basic conditions. Alternatively, luciferins may be used in conjunction with luciferase or lucigenins to provide bioluminescence.

Spin labels are provided by reporter molecules with an unpaired electron spin which can be detected by electron spin resonance (ESR) spectroscopy. Exemplary spin labels include organic free radicals, transitional metal complexes, particularly vanadium, copper, iron, and manganese, and the like. Exemplary spin labels include nitroxide free radicals.

### Expression products

The present disclosure relates to the classification of cancer in a tissue sample, based on the expression pattern formed by expression products such as mRNA as described above. Furthermore, the disclosure also relates to determining expression products such as peptides and proteins. The expression products, peptides and proteins, may be detected by any suitable technique known to the person skilled in the art.

In a preferred embodiment the expression products are detected by means of specific antibodies directed to the various expression products, such as immunofluorescent and/or immunohistochemical staining of the tissue.

Immunohistochemical localization of expressed proteins may be carried out by immunostaining of tissue sections from the single tumors to determine which cells expressed the protein encoded by the transcript in question. The transcript levels were used to select a group of proteins supposed to show variation from sample to sample, making possible a rough correlation between level of protein detected and intensity of the transcript on the microarray.

For example tissue sections may be cut from paraffin-embedded tissue blocks, mounted, and deparaffinized by incubation at 80 C° for 10 min, followed by immersion in heated oil at 60 C for 10 min (Estisol 312, Estichem A/S, Denmark) and rehydration. Antigen retrieval is achieved in TEG (TrisEDTA-Glycerol) buffer using microwaves at 900 W. The tissue sections are cooled in the buffer for 15 min before a brief rinse in tap water. Endogenous peroxidase activity is blocked by incubating the sections with 1% H202 for 20 min, followed by three rinses in tap water, 1 min each. The sections are subsequently soaked in PBS buffer for 2 min. The following steps are modified from the descriptions given by Oncogene Science Inc., in the Mouse Immunohistochemistry Detection System, XHCO1 (UniTect, Uniondale, NY, USA). Briefly, the tissue sections are incubated overnight at 4 C with primary antibody, followed by three rinses in PBS buffer for 5 min each. Afterwards, the sections are incubated with biotinylated secondary antibody for 30 min, rinsed three times with PBS buffer and subsequently incubated with ABC (avidin-biotinlylated horseradish peroxidase complex) for 30 min, followed by three rinses in PBS buffer. Staining is performed by incubation with AEC (3-amino-ethylcarbazole) for 10 min. The tissue sections are counter stained with Mayers hematoxylin, washed in tap water for 5 min. and mounted with glycerol-gelatin. Positive and negative controls may be included in each staining round with all antibodies.

In yet another embodiment the expression products may be detected by means of conventional enzyme assays, such as ELISA methods.

Furthermore, the expression products may be detected by means of peptide/protein chips capable of specifically binding the peptides and/or proteins assessed. Thereby an expression pattern may be obtained.

### Levels of expression

In the present disclosure the pattern formed by the expression profiles of genes is used for classifying cancer. The presence and/or amount of a plurality of gene expression products are/is consequently determined. The level of expression of selected genes is compared between different cancer cells.

The expression level of a particular gene in one cell type may be determined to be at least one fold higher than the expression level in a second cell type. By a one fold higher expression level is meant that the level of expression is doubled, i.e. an expression level is determined to be of the value 2 in one cell type and thus a one fold higher expression level is 4. By the term two-fold is meant the value is tripled, i.e. an increase from 2 to 6.

### Treatment

MSS occurs in 85% of the diagnosed colon cancers, whereas MSI occurs in 15 % of the diagnoses. The choice of treatment in relation to chemotherapeutic agents seems to be related to the microsatellite status of a given colon cancer. MSS cells are affected by treatment using fluorouracil-based drugs (Carethers JM, Hawn MT, Chauhan DP, et al., J Clin Invest 1996;98(1):199-206.; Carethers JM, Chauhan DP, Fink D, et al. Gastroenterology 1999;117(1):123-31; Koi M, Umar A, Chauhan DP, et al. Cancer Res 1994;54(16):4308-12., Hawn MT, Umar A, Carethers JM, et al. Cancer Res 1995;55(17):3721-5). MSI cells are hypersensitive to for example irinotecan and CPT (Hsiang YH, Lihou MG, Liu LF. Cancer Res 1989;49(18):5077-82; Jacob S, Aguado M, Fallik D, Praz F. Cancer Res 2001;61(17):6555-62). Thus, the ability to determine the microsatellite status of a colon cancer can facilitate the selection of a chemotherapeutic agent that will be effective in treatment of that particular colon cancer type.

One aspect relates to treatment with anti cancer drugs following the determination of the microsatellite status of a cancer in a sample to be microsatellite stable (MSS) and a prognostic marker. Typically, the MSS status has been determined using the methods of the present disclosure. A preferred embodiment is treatment using fluorouracil-based drugs, such as 5-fluorouracil, N-methy-N'-nitro-N-nitrosoguanidine or 6-thioguanine. Yet another preferred embodiment is treatment with non-fluorouracil-based anti cancer drugs. Another preferred embodiment is treatment using anti cancer drugs used in any combination. However; the treatment may also be a combination of treatment using for example fluorouracil-based anti cancer drugs and/or non-fluorouracil-based anti cancer drugs in combination with other sorts of treatment such as surgical intervention, radiation therapy, radiofrequency ablation, immuno therapy, gene therapy.

**Anti cancer drugs**

In one embodiment of the method for treatment of an individual comprising the steps of selecting an individual having contracted colon cancer, wherein the microsatellite status is stable and treating the individual with anti cancer drugs that are suitable for the diagnosed nature of the cancer. Fluorouracil-based anti cancer drugs prevent cells from synthesizing DNA and RNA by interfering with the synthesis of nucleic acids, thus disrupting the growth of the cancer cells. The drugs are therefore also called antimetabolites. The group of fluorouracil-based anti cancer drugs comprises a large number of drugs. One of the drugs currently used in the clinic is 5-fluorouracil that is used to treat several types of cancer including colon cancer. Another preferred drug of the same group is N-methy-N'-nitro-N-nitrosoguanidine, and in particular 6-thioguanine. The drugs belonging to the group of fluorouracil-based anti cancer drugs are not limited to the ones described above, but comprise all fluorouracil-based anti cancer drugs.

A preferred embodiment for the treatment of MSI cells is the use of non-fluorouracil-based drugs, for example Topoisomerase I-inhibitors such as irinotecan, such as CPT(CPT-II, Camptothecin).

Another embodiment regards the use of anti cancer drugs that are suitable for cancer treatment but are not fluorouracil-based drugs. The non-fluorouracil based group of drugs is a heterogenous group of drugs that comprises for example antibodies, chemotherapeutic drugs known as antineoplastic drugs, and antidote drugs such as folate. One preferred drug of the group is Cetuximab (Erbitux) which is used in the clinic to treat patients with advanced colorectal cancer that has spread to other parts of the body. Erbitux is a monoclonal antibody approved to treat this type of cancer. Another preferred embodiment is a combination treatment of Erbitux to be given intravenously with Irinotecan, another drug approved to fight colorectal cancer. Irinotecan and oxaliplatin are chemotherapy drugs that are given as a treatment for cancer in the colon or rectum and exemplify yet other preferred embodiments. Irinotecan and oxaliplatin belong to the group of antineoplastics. Leucovorin is another preferred drug belonging to the group of non-fluorouracil-based drugs, where leucovorin is the active form of the B complex vitamin, folate, and is used as an antidote to drugs that decrease levels of folic acid. Some treatments require what is called leucovorin rescue, because the drug used to treat the cancer has had an adverse effect on folic acid levels.

In a third embodiment is the sequential or combined use of drugs from either of the two groups of drugs.

**Assay**

A further aspect relates to an assay for classifying cancer in an individual having contracted cancer. At least one marker capable of detecting the microsatellite status in a sample is included in the assay together with at least one marker determining the prognostic marker. The determination of said microsatellite status marker and the determination of the prognostic marker may in the assay be determined sequentially or simultaneously.

In a preferred embodiment the assay comprises at least two markers, one for the microsatellite status and one for the prognostic marker.

The marker (s) is/are preferably specifically detecting a gene as identified herein, in particular the genes of the tables in the examples and as discussed above. However, the marker of microsatellite status may be determined by conventional microsatellite analysis as described elsewhere herein.

As discussed above the marker may be any nucleotide probe, such as a DNA, RNA, PNA, or LNA probe capable of hybridising to mRNA indicative of the expression level. The hybridisation conditions are preferably as described below for probes.

In another embodiment the marker is an antibody capable of specifically binding the expression product in question.

**Detection of expression pattern**

Patterns can be compared manually by a person or by a computer or other machine. An algorithm can be used to detect similarities and differences. The algorithm may score and compare, for example, the genes which are expressed and the genes which are not expressed. Alternatively, the algorithm may look for changes in intensity of expression of a particular gene and score changes in intensity between two samples. Similarities may be determined on the basis of genes which are expressed in both samples and genes which are not expressed in both samples or on the basis of genes whose intensity of expression are numerically similar.

Generally, the detection operation will be performed using a reader device external to the diagnostic device. However, it may be desirable in some cases, to incorporate the data gathering operation into the diagnostic device itself.

The detection apparatus may be a fluorescence detector, or a spectroscopic detector, or another detector.

Although hybridization is one type of specific interaction which is clearly useful for use in this mapping embodiment, antibody reagents may also be very useful.

**Data gathering and analysis**

Gathering data from the various analysis operations, e.g., oligonucleotide and/or microcapillary arrays, will typically be carried out using methods known in the art. For example, the arrays may be scanned using lasers to excite fluorescently labeled targets that have hybridized to regions of probe arrays mentioned above, which can then be imaged using charged coupled devices ("CCDs") for a wide field scanning of the array. Alternatively, another particularly useful method for gathering data from the arrays is through the use of laser confocal microscopy which combines the ease and speed of a readily automated process with high resolution detection.

Following the data gathering operation, the data will typically be reported to a data analysis operation. To facilitate the sample analysis operation, the data obtained by the reader from the device will typically be analyzed using a digital computer. Typically, the computer will be appropriately programmed for receipt and storage of the data from the device, as well as for analysis and reporting of the data gathered, i.e., interpreting fluorescence data to determine the sequence of hybridizing probes, normalization of background and single base mismatch hybridizations, ordering of sequence data in SBH applications, and the like.

It is an object of the present disclosure to provide a biological sample which may be classified or characterized by analyzing the pattern of specific interactions mentioned above. This may be applicable to a cell or tissue type, to the messenger RNA population expressed by a cell to the genetic content of a cell, or to virtually any sample which can be classified and/or identified by its combination of specific molecular properties.

**Pharmaceutical composition**

The disclosure also relates to a pharmaceutical composition for treating the classified cancer, such as colorectal tumors.

In one aspect the pharmaceutical composition comprises one or more of the peptides being expression products as defined above. In a preferred embodiment, the peptides are bound to carriers. The peptides may suitably be coupled to a polymer carrier, for example a protein carrier, such as BSA. Such formulations are well-known to the person skilled in the art.

The peptides may be suppressor peptides normally lost or decreased in tumor tissue administered in order to stabilise tumors towards a less malignant stage. In another embodiment the peptides are onco-peptides capable of eliciting an immune response towards the tumor cells.

In another aspect the pharmaceutical composition comprises genetic material, either genetic material for substitution therapy, or for suppressing therapy as discussed below.

In a third aspect the pharmaceutical composition comprises at least one antibody produced as described above.

In the present context the term pharmaceutical composition is used synonymously with the term medicament. The medicament of the disclosure comprises an effective amount of one or more of the compounds as defined above, or a composition as defined above in combination with pharmaceutically acceptable additives. Such medicament may suitably be formulated for oral, percutaneous, intramuscular, intravenous, intracranial, intrathecal, intracerebroventricular, intranasal or pulmonal administration. For most indications a localised or substantially localised application is preferred.

Strategies in formulation development of medicaments and compositions based on the compounds of the present disclosure generally correspond to formulation strategies for any other protein-based drug product. Potential problems and the guidance required to overcome these problems are dealt with in several textbooks, e.g. "Therapeutic Peptides and Protein Formulation. Processing and Delivery Systems", Ed. A.K. Banga, Technomic Publishing AG, Basel, 1995.

Injectables are usually prepared either as liquid solutions or suspensions, solid forms suitable for solution in, or suspension in, liquid prior to injection. The preparation may also be emulsified. The active ingredient is often mixed with excipients which are pharmaceutically acceptable and compatible with the active ingredient. Suitable excipients are, for example, water, saline, dextrose, glycerol, ethanol or the like, and combinations thereof. In addition, if desired, the preparation may contain minor amounts of auxiliary substances such as wetting or emulsifying agents, pH buffering agents, or which enhance the effectiveness or transportation of the preparation.

Formulations of the compounds of the disclosure can be prepared by techniques known to the person skilled in the art. The formulations may contain pharmaceutically acceptable carriers and excipients including microspheres, liposomes, microcapsules, nanoparticles or the like.

The preparation may suitably be administered by injection, optionally at the site, where the active ingredient is to exert its effect. Additional formulations which are suitable for other modes of administration include suppositories, and, in some cases, oral formulations. For suppositories, traditional binders and carriers include polyalkylene glycols or triglycerides. Such suppositories may be formed from mixtures containing the active ingredient(s) in the range of from 0.5% to 10%, preferably 1-2%. Oral formulations include such normally employed excipients as, for example, pharmaceutical grades of mannitol, lactose, starch, magnesium stearate, sodium saccharine, cellulose, magnesium carbonate, and the like. These compositions take the form of solutions, suspensions, tablets, pills, capsules, sustained release formulations or powders and generally contain 10-95% of the active ingredient(s), preferably 25-70%.

The preparations are administered in a manner compatible with the dosage formulation, and in such amount as will be therapeutically effective. The quantity to be administered depends on the subject to be treated, including, e.g. the weight and age of the subject, the disease to be treated and the stage of disease. Suitable dosage ranges are of the order of several hundred μg active ingredient per administration with a preferred range of from about 0.1 μg to 1000 μg, such as in the range of from about 1 μg to 300 μg, and especially in the range of from about 10 μg to 50 μg. Administration may be performed once or may be followed by subsequent administrations. The dosage will also depend on the route of administration and will vary with the age and weight of the subject to be treated. A preferred dosis would be in the interval 30 mg to 70 mg per 70 kg body weight.

Some of the compounds of the present disclosure are sufficiently active, but for some of the others, the effect will be enhanced if the preparation further comprises pharmaceutically acceptable additives and/or carriers. Such additives and carriers will be known in the art. In some cases, it will be advantageous to include a compound, which promotes delivery of the active substance to its target.

In many instances, it will be necessary to administrate the formulation multiple times. Administration may be a continuous infusion, such as intraventricular infusion or administration in more doses such as more times a day, daily, more times a week, weekly, etc.

**Therapy**

The disclosure further relates to a method of treating individuals suffering from a classified cancer, in particular for treating a colorectal tumor. In one embodiment the tumor cell in question is a microsatellite stable (MSS) tumor cell. In a second embodiment the tumor cell in question is a microsatellite instable (MSI) tumor cell.

In one embodiment relates to a method of substitution therapy, i.e. administration of genetic material generally expressed in MSI cells, but lost or decreased in classified MSS cancer cells. Thus, the disclosure relates to a method for reducing malignancy of a MSS tumor cell of the Dukes B class, said method comprising

obtaining at least one gene selected from genes being expressed at least one-fold higher in MSI cells than the amount expressed in said MSS tumor cell

introducing said at least one gene into the MSS tumor cell in a manner allowing expression of said gene(s)

The at least one gene is preferably selected individually from genes comprising a sequence as identified below

| Gene name | Ref seq | Gene symbol | SEQ ID NO.: |
|---|---|---|---|
| heterogeneous nuclear ribonucleoprotein L | NM_001533 | HNRPL | 11 |
| metastais-associated gene family, member 2 | NM_004739 | MTA2 | 23 |
| chemokine (C-X-C motif) ligand 10 | NM_001565 | CXCL10 | 35 |
| splicing factor, arginine/serine-rich 6 | NM_006275 | SFRS6 | 43 |

In a preferred embodiment at least two different genes are introduced into the MSS tumor cell.

By the term one-fold is meant that the value is doubled, i.e. an expression level is 2 - a one-fold higher expression level is 4.

By the term two-fold is meant that the value is tripled, i.e. an expression level is 2 - a two-fold higher expression level is 6.

Another embodiment relates to a method of substitution therapy, i.e. administration of genetic material generally expressed in MSS cells, but lost or decreased in classified MSI cancer cells. Thus, the disclosure relates to a method for reducing malignancy of a MSI tumor cell of the Dukes C class, said method comprising
obtaining at least one gene selected from genes being expressed at least one-fold higher in MSS cells than the amount expressed in said MSI tumor cell
introducing said at least one gene into the MSI tumor cell in a manner allowing expression of said gene(s).

The at least one gene is preferably selected individually from genes comprising a sequence as identified below

| Gene name | Ref seq | Gene symbol | SEQ ID NO.: |
|---|---|---|---|
| protein kinase C binding protein 1 | NM_012408<br>NM_183047 | PRKCBP1 | 57<br>129 |
| hepatocellular carcinoma-associated antigen 112 | NM_018487 | HCA112 | 89 |
| hypothetical protein FLJ20618 | NM _017903 | FLJ20618 | 102 |
| SET translocation (myeloid leukaemia-associated) | NM_003011.1 | SET | 103 |
| ATPase, class II, type 9a | Xm_030577.9 | ATP9a | 104 |

In a preferred embodiment at least two different genes are introduced into the MSI tumor cell.

The disclosure also relates to a method of treating individuals having contracted colon cancer, wherein the microsatellite status has been determined to be stable and the cancer has been determined to be hereditary in nature. Preferably, the determination of microsatellite status and the hereditary nature of the cancer has been determined according to the present disclosure, analyzing expression patterns as described herein. The method of treating individuals involves introducing at least one gene into the tumor cell, whereby the gene is being expressed. The at least one gene is selected individually from the group of genes listed below

| Gene name | Ref seq | Gene symbol | SEQ ID NO.: |
|---|---|---|---|
| Homo sapiens mutS homolog 2, colon cancer, nonpolyposis type 1 (E. coli) | NM_000251 | MSH2 | 136 |
| Mut L homolog 1 | NM_00249.2 | MLH1 | 107 |
| Homo sapiens PMS1 postmeiotic segregation increased 1 (S. cerevisiae) | NM_000534 | PMS1 | 137 |
| Homo sapiens PMS2 postmeiotic segregation increased 2 (S. cerevisiae) (PMS2), mRNA | NM_000535 | PMS2 | 138 |
| Homo sapiens mutS homolog 6 (E. coli) | NM_000179 | MSH6 | 139 |

In a preferred embodiment at least two different genes are introduced into the tumor cell.

The disclosure further relates to a method of treating individuals suffering from a classified cancer, in particular for treating a colorectal tumor. In one embodiment of the present invention the tumor cell in question is a microsatellite stable (MSS) tumor cell. In a second embodiment the tumor cell in question is a microsatellite instable (MSI) tumor cell.

Yet another embodiment relates to a method of administration of peptides to the tumor cell in question. The peptide is expressed by at least one gene selected from genes being expressed at least two-fold higher in tumor cells than in the amount expressed in the tumor cells in question. The tumor cell in question is MSI tumor cell or MSI tumor cell.

Thus, the disclosure relates to a method for reducing cell malignancy of a MSS tumor cell of the Dukes B class, said method comprising
contacting a a MSS tumor cell with at least one peptide expressed by at least one gene selected from genes being expressed in at least two-fold higher in MSI tumor cells than in the amount expressed in said MSS tumor cell.
The at least one peptide is selected individually from genes as listed below

| Gene name | Ref seq | Gene symbol | SEQ ID NO.: |
|---|---|---|---|
| heterogeneous nuclear ribonucleoprotein L | NM_001533 | HNRPL | 11 |
| metastais-associated gene family, member 2 | NM_004739 | MTA2 | 23 |
| chemokine (C-X-C motif) ligand 10 | NM_001565 | CXCL10 | 35 |
| splicing factor, arginine/serine-rich 6 | NM_006275 | SFRS6 | 43 |

A preferred embodiment relates to a method for reducing cell malignancy of a MSI tumor cell of the Dukes C class, said method comprising
contacting a MSI tumor cell with at least one peptide expressed by at least one gene selected from genes being expressed in at least two-fold higher in MSS tumor cells than in the amount expressed in said MSI tumor cell.
The at least one peptide is selected individually from genes as listed below

| Gene name | Ref seq | Gene symbol | SEQ ID NO.: |
|---|---|---|---|
| protein kinase C binding protein 1 | NM_012408 | PRKCBP1 | 57 |
| | NM_183047 | | 129 |
| hepatocellular carcinoma-associated antigen 112 | NM_018487 | HCA112 | 89 |
| hypothetical protein FLJ20618 | NM_017903 | FLJ20618 | 102 |
| SET translocation (myeloid leukaemia-associated) | NM_003011.1 | SET | 103 |
| ATPase, class II, type 9a | Xm_030577.9 | ATP9a | 104 |

Another aspect relates to a therapy whereby genes generally correlated to disease are inhibited by one or more of the following methods:
One embodiment relates to a method for reducing malignancy of a MSS cell of the Dukes B class, said method comprising
obtaining at least one nucleotide probe capable of hybridising with at least one gene of a MSS tumor cell , said at least one gene being selected from genes being expressed in an amount at least one-fold lower in MSI tumor cells than the amount expressed in said MSS tumor cell, and
introducing said at least one nucleotide probe into the MSS tumor cell in a manner allowing the probe to hybridise to the at least one gene, thereby inhibiting expression of said at least one gene.
The probes are preferably selected from probes capable of hybridising to a nucleotide sequence comprising a sequence as identified below

| Gene name | Ref seq | Gene symbol | SEQ ID NO.: |
|---|---|---|---|
| protein kinase C binding protein 1 | NM_012408 | PRKCBP1 | 57 |
| | NM_183047 | | 129 |
| hepatocellular carcinoma-associated antigen 112 | NM_018487 | HCA112 | 89 |
| hypothetical protein FLJ20618 | NM_017903 | FLJ20618 | 102 |
| SET translocation (myeloid leukaemia-associated) | NM_003011.1 | SET | 103 |
| ATPase, class II, type 9a | Xm_030577.9 | ATP9a | 104 |

Another embodiment relates to a method for reducing malignancy of a MSI tumor cell of the Dukes B class, said method comprising
obtaining at least one nucleotide probe capable of hybridising with at least one gene of a MSI tumor cell , said at least one gene being selected from genes being expressed in an amount at least one-fold lower in MSS tumor cells than the amount expressed in said MSI tumor cell, and
introducing said at least one nucleotide probe into the MSI tumor cell in a manner allowing the probe to hybridise to the at least one gene, thereby inhibiting expression of said at least one gene.

The probes are preferably selected from probes capable of hybridising to a nucleotide sequence comprising a sequence as identified below

| Gene name | Ref seq | Gene symbol | SEQ ID NO.: |
|---|---|---|---|
| heterogeneous nuclear ribonucleoprotein L | NM_001533 | HNRPL | 11 |
| metastais-associated gene family, member 2 | NM_004739 | MTA2 | 23 |
| chemokine (C-X-C motif) ligand 10 | NM_001565 | CXCL10 | 35 |
| splicing factor, arginine/serine-rich 6 | NM_006275 | SFRS6 | 43 |

These methods are preferably based on anti-sense technology, whereby the hybridisation of said probe to the gene leads to a down-regulation of said gene.

The down-regulation may of course also be based on a probe capable of hybridising to regulatory components of the genes in question, such as promoters.

In yet another embodiment the probes consists of the sequences identified above.

The hybridization may be tested in vitro at conditions corresponding to in vivo conditions. Typically, hybridization conditions are of low to moderate stringency. These conditions favour specific interactions between completely complementary sequences, but allow some non-specific interaction between less than perfectly matched sequences to occur as well. After hybridization, the nucleic acids can be "washed" under moderate or high conditions of stringency to dissociate duplexes that are bound together by some non-specific interaction (the nucleic acids that form these duplexes are thus not completely complementary).

As is known in the art, the optimal conditions for washing are determined empirically, often by gradually increasing the stringency. The parameters that can be changed to affect stringency include, primarily, temperature and salt concentration. In general, the lower the salt concentration and the higher the temperature, the higher the stringency. Washing can be initiated at a low temperature (for example, room temperature) using a solution containing a salt concentration that is equivalent to or lower than that of the hybridization solution. Subsequent washing can be carried out using progressively warmer solutions having the same salt concentration. As alternatives, the salt concentration can be lowered and the temperature maintained in the washing step, or the salt concentration can be lowered and the temperature increased. Additional parameters can also be altered. For example, use of a destabilizing agent, such as formamide, alters the stringency conditions.

In reactions where nucleic acids are hybridized, the conditions used to achieve a given level of stringency will vary. There is not one set of conditions, for example, that will allow duplexes to form between all nucleic acids that are 85% identical to one another, hybridization also depends on unique features of each nucleic acid. The length of the sequence, the composition of the sequence (for example, the content of purine-like nucleotides versus the content of pyrimidine-like nucleotides) and the type of nucleic acid (for example, DNA or RNA) affect hybridization. An additional consideration is whether one of the nucleic acids is immobilized (for example, on a filter).

An example of a progression from lower to higher stringency conditions is the following, where the salt content is given as the relative abundance of SSC (a salt solution containing sodium chloride and sodium citrate; 2X SSC is 10-fold more concentrated than 0.2X SSC). Nucleic acids are hybridized at 42°C in 2X SSC/0.1 % SDS (sodium dodecylsulfate; a detergent) and then washed in 0.2X SSC/0.1 % SDS at room temperature (for conditions of low stringency); 0.2X SSC/0.1 % SDS at 42°C (for conditions of moderate stringency); and 0.1X SSC at 68°C (for conditions of high stringency). Washing can be carried out using only one of the conditions given, or each of the conditions can be used (for example, washing for 10-15 minutes each in the order listed above). Any or all of the washes can be repeated. As mentioned above, optimal conditions will vary and can be determined empirically.

In another aspect a method of reducing tumorigenicity relates to the use of antibodies against an expression product of a cell from the biological tissue. The antibodies may be produced by any suitable method, such as a method comprising the steps of
obtaining expression product(s) from at least one gene said gene being expressed as defined below,
immunising a mammal with said expression product(s) obtaining antibodies against the expression product.

Use

The methods described above may be used for producing an assay for classifying cancer in animal tissue.

Furthermore, the disclosure relates to the use of a peptide as defined above for preparation of a pharmaceutical composition for the treatment of a classified cancer in animal tissue.

Furthermore, the disclosure relates to the use of a gene as defined above for preparation of a pharmaceutical composition for the treatment of a classified cancer in animal tissue.

Also, the disclosure relates to the use of a probe as defined above for preparation of a pharmaceutical composition for the treatment of a biological condition in animal tissue.

**Gene delivery therapy**

The genetic material discussed above may be any of the described genes or functional parts thereof. The constructs may be introduced as a single DNA molecule encoding all of the genes, or different DNA molecules having one or more genes. The constructs may be introduced simultaneously or consecutively, each with the same or different markers.

The gene may be linked to the complex as such or protected by any suitable system normally used for transfection such as viral vectors or artificial viral envelope, liposomes or micellas, wherein the system is linked to the complex.

Numerous techniques for introducing DNA into eukaryotic cells are known to the skilled artisan. Often this is done by means of vectors, and often in the form of nucleic acid encapsidated by a (frequently virus-like) proteinaceous coat. Gene delivery systems may be applied to a wide range of clinical as well as experimental applications.

Vectors containing useful elements such as selectable and/or amplifiable markers, promoter/enhancer elements for expression in mammalian, particularly human, cells, and which may be used to prepare stocks of construct DNAs and for carrying out transfections are well known in the art. Many are commercially available.

Various techniques have been developed for modification of target tissue and cells in vivo. A number of virus vectors, discussed below, are known which allow transfection and random integration of the virus into the host. See, for example, Dubensky et al. (1984) Proc. Natl. Acad. Sci. USA 81:7529-7533; Kaneda et al., (1989) Science 243:375-378; Hiebert et al. (1989) Proc. Natl. Acad. Sci. USA 86:3594-3598; Hatzoglu et al., (1990) J. Biol. Chem. 265:17285-17293; Ferry et al. (1991) Proc. Natl. Acad. Sci. USA 88:8377-8381. Routes and modes of administering the vector include injection, e.g intravascularly or intramuscularly, inhalation, or other parenteral administration.

Advantages of adenovirus vectors for human gene therapy include the fact that recombination is rare, no human malignancies are known to be associated with such viruses, the adenovirus genome is double stranded DNA which can be manipulated to accept foreign genes of up to 7.5 kb in size, and live adenovirus is a safe human vaccine organisms.

Another vector which can express the DNA molecule of the present disclosure, and is useful in gene therapy, particularly in humans, is vaccinia virus, which can be rendered non-replicating (U.S. Pat. Nos. 5,225,336; 5,204,243; 5,155,020; 4,769,330).

Based on the concept of viral mimicry, artificial viral envelopes (AVE) are designed based on the structure and composition of a viral membrane, such as HIV-1 or RSV and used to deliver genes into cells in vitro and in vivo. See, for example, U.S. Pat. No. 5,252,34.8, Schreier H. et al., J. Mol. Recognit., 1995, 8:59-62; Schreier H et al., J. Biol. Chem., 1994, 269:9090-9098; Schreier, H., Pharm. Acta Helv. 1994, 68:145-159; Chander, R et al. Life Sci., 1992, 50: 481-489, which references are hereby incorporated by reference in their entirety. The envelope is preferably produced in a two-step dialysis procedure where the "naked" envelope is formed initially, followed by unidirectional insertion of the viral surface glycoprotein of interest. This process and the physical characteristics of the resulting AVE are described in detail by Chander et al., (supra). Examples of AVE systems are (a) an AVE containing the HIV-1 surface glycoprotein gp160 (Chander et al., supra; Schreier et al., 1995, supra) or glycosyl phosphatidylinositol (GPI)-linked gp120 (Schreier et al., 1994, supra), respectively, and (b) an AVE containing the respiratory syncytial virus (RSV) attachment (G) and fusion (F) glycoproteins (Stecenko, A. A. et al., Pharm. Pharmacol. Lett. 1:127-129 (1992)). Thus, vesicles are constructed which mimic the natural membranes of enveloped viruses in their ability to bind to and deliver materials to cells bearing corresponding surface receptors.

AVEs are used to deliver genes both by intravenous injection and by instillation in the lungs. For example, AVEs are manufactured to mimic RSV, exhibiting the RSV F surface glycoprotein which provides selective entry into epithelial cells. F-AVE are loaded with a plasmid coding for the gene of interest, (or a reporter gene such as CAT not present in mammalian tissue).

The AVE system described herein in physically and chemically essentially identical to the natural virus yet is entirely "artificial", as it is constructed from phospholipids, cholesterol, and recombinant viral surface glycoproteins. Hence, there is no carry-over of viral genetic information and no danger of inadvertant viral infection. Construction of the AVEs in two independent steps allows for bulk production of the plain lipid envelopes which, in a separate second step, can then be marked with the desired viral glycoprotein, also allowing for the preparation of protein cocktail formulations if desired.

Another delivery vehicle for use in the present disclosure are based on the recent description of attenuated Shigella as a DNA delivery system (Sizemore, D. R. et al., Science 270:299-302 (1995), which reference is incorporated by reference in its entirety). This approach exploits the ability of Shigellae to enter epithelial cells and escape the phagocytic vacuole as a method for delivering the gene construct into the cytoplasm of the target cell. Invasion with as few as one to five bacteria can result in expression of the foreign plasmid DNA delivered by these bacteria.

A preferred type of mediator of nonviral transfection in vitro and in vivo is cationic (ammonium derivatized) lipids. These positively charged lipids form complexes with negatively charged DNA, resulting in DNA charged neutralization and compaction. The complexes endocytosed upon association with the cell membrane, and the DNA somehow escapes the endosome, gaining access to the cytoplasm. Cationic lipid:DNA complexes appear highly stable under normal conditions. Studies of the cationic lipid DOTAP suggest the complex dissociates when the inner layer of the cell membrane is destabilized and anionic lipids from the inner layer displace DNA from the cationic lipid. Several cationic lipids are available commercially. Two of these, DMRI and DC-cholesterol, have been used in human clinical trials. First generation cationic lipids are less efficient than viral vectors. For delivery to lung, any inflammatory responses accompanying the liposome administration are reduced by changing the delivery mode to aerosol administration which distributes the dose more evenly.

The following are non-limiting examples illustrating the present invention.

## Examples

In the following two complete studies have been performed. The type of experiments performed in Study 1 is described in examples 1 to 6. Study 2 describes an earlier, additional study.

## Study I

101 colorectal tumors were tested for microsatellite instability and their global gene transcription was measured using high-density oligonucleotide microarrays. Unsupervised and supervised classification methods were applied to visualize tumor classes and define sets of genes for classification. Real-time PCR was used to validate the microarray data and to investigate platform independency on an independent set of 47 tumors.

### Patients and biopsy specimens

A broad spectrum of patients representing different common groups of colorectal cancers were included (Table 15, Fig.1).

**Table 15**

**Summary of clinicopathological and microsatellite features of colon cancer samples.**

| Patient group (Danish,Finish) | N | Median age (range) | Localization in colon<br>right (Darish,Finish) | left (Danish,Finnish) | Tumour stage N (Danish,Finnish)<br>0[a] | II | III | IHC negative stain N (n tested)<br>MLH1 | MSH2 |
|---|---|---|---|---|---|---|---|---|---|
| All cases | 118 (44,75) | 62.0 (32-87) | 44 (7,37) | 74 (36,38) | 17 (6,11) | 36 (14,22) | 65 (23,42) | 12 (56) | 1 (56) |
| Sporadic microsatellite instable tumors[b] | 20 (9,16) | 66.8 (44-87) | 15 (3,12) | 9 (6,4) | - | 8 (2,6) | 12 (2,10) | 6 (11) | 0 (11) |
| Hereditary microsatellite instable tumors[b,c] | 17 (4,13) | 49.6 (32-75) | 9 (2,7) | 8 (2,6) | - | 10 (2,8) | 5 (1,4) | 5 (7) | 1 (7) |
| Microsatellite stable tumors | 61 (30,36) | 61.0 (36-85) | 11 (0,11) | 55 (30,25) | - | 18 (10,8) | 48 (20,28) | 0 (38) | 0 (38) |

[a]nomial biopsy taken from the resection edge of a tumor
[b]according to microsatellite analysis
[c]according to the Amsterdam criteria
[c]DK is Denmark, SF is Finland)

MSI and MSS tumors both from the right and left colon were used and the MSI tumors represent both sporadic and hereditary cases. Hereditary cases included both missense and truncating germline mutations. All specimens were verified by histology and contained more than 50% tumor tissue. The tumors were resected at 15 different clinics in Denmark and Finland. Patients with stage III disease received adjuvant chemotherapy in addition to curative surgery. None of the patients received pre-operative radiation or chemotherapy. Informed consent was obtained from patients to use their specimens and clinical and pathological data for research purposes and the local ethic committees approved the study.

**Microsatellite-instability analysis**

DNA was extracted from microdissected cancer tissue. Control DNA was extracted from blood samples when available, otherwise normal epithelium from the oral resection edge was used. Samples positive for markers BAT25 and BAT26 were scored as microsatellite instable whereas samples positive for only one of these markers were tested for further markers and scored as low-frequency MSI if none of these tested positive. Tumors with low-frequency MSI have similar clinical features as microsatellite stable tumors and were considered as such in this study. For determining the MSI status of the 47-tumors in the test set a pentaplex polymerase chain reaction with five quasimonomorphic mononucleotide repeats was used ( Suraweera N, Duval A, Reperant M, et al.. Gastroenterology 2002;123(6):1804-11).

**RNA purification**

Colon specimens were obtained fresh from surgery and were either immediately snap frozen in liquid nitrogen (Denmark) or transferred to -70°C freezers with as little delay as possible (Finland). The Finnish samples were stored as dry tissue, whereas the Danish samples were either embedded in OCD-compound or stored in a SDS/guadinium thiocyanate solution. Total RNA was isolated using Trizol (Invitrogen) or GenElute Kits (Sigma) following the manufacturers' instructions.

**Gene expression analysis**

Labeling of RNA, hybridization and scanning was performed as described elsewhere (Dyrskjot L, Thykjaer T, Kruhoffer M, et al. Nat Genet 2003;33(1):90-6) Biotin labeled cRNA was prepared from 10μg of total RNA and hybridized to the Human Genome U133A GeneChip (Affymetrix) containing 22.289 probesets. The readings from the quantitative scanning were analyzed by the Affymetrix Software MAS 5.0 and normalized using the quantile normalization procedure implemented in RMA (robust multiarray analysis (Irizarry RA, Bolstad BM, Collin F, Cope LM, Hobbs B, Speed TP. Nucleic Acids Res 2003;31 (4):e15., Bolstad BM, Irizarry RA, Astrand M, Speed TP, Bioinformatics 2003;19(2):185-93)).

**Statistical testing**

The 101 samples were divided into four groups according to country of origin and MS status. To study if there was any systematic difference between samples from the two countries, a test statistic $S_1/S_2$ was used. $S_1$ is the sum of squared deviations when a gene effect, country effect and microsatellite effect is observed, and $S_2$ is the same sum where a gene effect and microsatellite effect is present only. The significance is obtained by calculating $S_1/S_2$ of data achieved after one hundred random permutations of the country labels. The above test was also performed for each gene separately by considering the number of genes with a test value $S_1(g)/S_2(g)$ below a given threshold. The same method was used to evaluate differences between the MSI and MSS groups. These calculations were based on the expression level of 5.082 genes with a variance over all tumor samples larger than 0.2.

**Microsatellite status classifier**

The MSI classifier was based on the 5.082 genes defined above. A normal distribution was used with the mean dependent on the gene and the group. For each gene, the variation between the groups and the variation within the groups was calculated to select genes with a high ratio between these. To classify a sample, the sum over the genes of the squared distance from the sample value to the group mean was calculated, standardized by the variance, and assigned the sample to the nearest group. The sample to be classified was excluded when calculating group means and variances.

**Real-time PCR**

The procedures were performed as described ( Birkenkamp-Demtroder K, Christensen LL, Olesen SH, et al.

Cancer Res 2002;62(15):4352-63) except that short LNA (Locked Nucleic Acid) enhanced probes from a Human Probe Library (Exiqon™) was used. All samples were normalized to GAPDH as this gene has been reported to be constantly expressed in colorectal cancer samples (Andersen CL, Ledet JL, Omtoft TF, Cancer Res 2004;In press).

Classification of new independent test samples based on real-time PCR

To translate the microarray-defined classifier to a PCR-platform, the nine-classifier genes were analyzed by quantitative PCR on a subset of 18 of the 101 tumor samples. The average for each gene and group of the microarray data were multiplied with a constant so that the total average was equal to the average of the corresponding log (Loeb LA., Cancer Res 1991;51(12):3075-9) transformed PCR values. This translation can be made because the normalized PCR values are expected to be proportional to the normalized array values, and on a log scale this becomes an additive difference. The difference is gene specific and is therefore estimated for each gene separately. Thus, the variation obtained from the microarray data, and used in the classifier, can be used directly on the PCR platform.

**Example 1**

**Hierarchical clustering**

The phylogenetic tree resulting from hierarchical clustering is shown with relevant clinical and laboratory data (Fig. 1). The cluster analysis based on 1239 genes with a variation across all samples larger than 0.5 reveals that the samples are mainly separated in accordance to the microsatellite status. On the upper trunk we find two clusters represented mainly by normal biopsies (14/20) and MSS tumors (21/25), respectively. The six tumors among the normal samples were labeled in four different batches, excluding a labeling batch effect.

The lower trunk is divided into a MSI cluster (30/36) and a second MSS cluster (37/37). The MSI cluster contains three morphologically normal tissue specimens. Notably, there is no sign of separation between sporadic and hereditary MSI samples and right sided and left sided tumors are interspersed among each other. The two MSS clusters are either dominated by Danish samples (19/25) or by Finnish samples (26/37), which is likely to be due to differences in sampling and preparation in the two countries.

Based on these observations, we performed a series of statistical tests to evaluate if the observed separation of tumors into MSS and MSI groups as well as into Danish and Finnish groups were significant. Our test value $S_1/S_2$ was 0.914 between Danish and Finnish and 0.908 between MSI and MSS groups, as compared to minimum values of 0.966 and 0.963, respectively, in 100 permutations, thus demonstrating a very clear separation between the groups. This clear distinction of the groups are caused by many genes even at very strict criteria, i.e. low test statistic $S_1(g)/S_2(g)$ values (See Table 16).

**Table 16**

**Permutation test of groups**

| Pseudo group | S1/S2 from data | Smaller values in 100 permutations | Minimum in 100 permutations |
|---|---|---|---|
| DK-SF | 0.9146 | 0 | 0.9660 |
| MSI-MSS | 0.9084 | 0 | 0.9631 |

**Permutation test of genes**

| Pseudo group | | $S_1(j)/S_2(j)$ < 0.6 | < 0.7 | < 0.8 | < 0.9 |
|---|---|---|---|---|---|
| DK-SF | number of genes | 22 | 114 | 425 | 1536 |
| | max in 100 permutations | 0 | 1 | 4 | 132 |
| MSI-MSS | number of genes | 49 | 151 | 461 | 1600 |
| | max in 100 permutations | 0 | 0 | 13 | 251 |

## Example 2

### Construction of a classifier for microsatellite instability status

To construct the classifier the expression profiles from 101 stage II and III tumors were employed. A maximum likelihood classifier was built in order to select a low number of genes resulting in the best possible separation of the two groups. The performance of the classifier was tested using 1-5082 genes and found to be stable showing 2-5 errors when using 4 to several hundred genes (Fig. 2A). In the final classifier the 9 genes (Table 17) that were most frequently used in the crossvalidation were used which resulted in 3 errors (Fig. 2B). The stability of the classifier was tested by randomly chosen datasets (Fig.2C).

**Table 17. Genes used for the classification of microsatellite status**

| Genechip Probe_ID | Gene name | Gene Symbol | Array signal* | Array signal* |
|---|---|---|---|---|
| | | | Microsatellite Stable tumors | Microsatellite instable tumors |
| 202072 | heterogeneous nuclear ribonucleoprotein L | HNRPL | 208 ±73 | 776 ±340 |
| 203444 | metastasis-associated 1-like 1 | MTA1L1 | 45 ±13 | 104 ±36 |
| 206108 | splicing factor, arginine/serine-rich 6 | SFRS6 | 74 ±56 | 478 ±242 |
| 204533 | chemokine (C-X-C motif) ligand 10 | CXCL10 | 111 ±80 | 315 ±535 |
| 212062 | ATPase, class II, type 9a | ATP9a | 588 ±222 | 208 ±114 |
| 218345 | hepatocellular carcinoma-associated antigen 112 | HCA112 | 1261 ±603 | 446 ±271 |
| 222444 | hypothetical protein FLJ20618 | FLJ20618 | 776 ±193 | 338 ±168 |
| 210047 | SET translocation (myeloid leukemia-associated) | SET | 1351±298 | 478 ±201 |
| 209048 | protein kinase C binding protein 1 | PRKCBP1 | 294 ±113 | 158 ±79 |

* Array signal are median signal intensity values ±standard deviation.

The gene MTA1L1 shown in figures and tables in the examples section is in the context of the present disclosure identical to MTA2 and has been assigned SEQ ID NO.: 23.

## Example 3

### Stability of classification

The mean error rate for MSS tumors was 1.62% and for MSI tumors 6.0% for microsatellite instable tumors. More than 50% of the errors were related to three tumors (marked with an asterisk in figure 2B) of which two were wrongly classified in all permutation and one in 94%. The remaining errors were mainly caused by four tumors (marked + in figure 2B) with error rates of 40-47%. In terms of sensitivity and specificity, the classification of MSS tumors could be made with a sensitivity of 98.4% and with a specificity of 94.0% (Table 18). After correction for prevalence of MSS tumors, the positive predictive value of MSS was calculated to be almost 98.5% and the negative predictive value to 91 %.

**Table 18**
**Permutation analysis of the microsatellite instability classifier**

| | Trainings set | Test set |
|---|---|---|
| | Errors in crossvalidation | Test errors |
| Microsatellite instable tumors | 6.48% (n=25, range 0-4) | 6.0% (n=9, range 0-3) |
| Microsatellite stable tumors | 1.67% (n=30, range 0-3) | 1.62% (n=37, range 0-4) |
| All | 3.85% (n=55, range 0-5) | 2.48% (n=46, range 0-4) |

## Example 4

### Construction of a classifier for sporadic versus hereditary microsatellite instable tumors

In order to identify a gene set for identification of hereditary MSI tumors the 19 sporadic MSI samples and 18 HNPCC MSI samples in the data set were subjected to supervised classification as described above (Fig.3A, B). The mismatch repair gene *MLH1* show a general downregulation in sporadic disease whereas *PIWIL1* is lower expressed in hereditary cases (Fig. 3C). Using these two genes only one error occurred: a sporadic MSI tumor was classified as hereditary. Based on t-test 500 permutations were performed to test the significance of these two genes as marker genes and found both genes highly significant with estimated p-values < 0.005.

In addition to identifying MSI tumors, we were also able to classify these as being sporadic or inherited based on the expression of only two genes, *MLH1* and *PIWIL1.* The classification only showed one misclassification out of 37. This single case did not have any family history of CRC but the family is very small and the patient was diagnosed at the age of 32 and may thus represent a missed HNPCC case. A majority of sporadic and about half of hereditary microsatellite instable colorectal cancers are caused by inactivation of *MLH1.* In sporadic tumors this is mostly caused by biallelic promotor hypermethylation whereas somatic mutation or loss of heterozygosity of the wild-type allele is significant mechanisms in hereditary tumors. As a result, the *MLH1* expression level in sporadic disease in strongly compromised whereas one or two alleles of *MLH1* in HNPCC are transcribed although encoding a mutated protein. *PIWIL1* is a member of the human Argonaute family that contain a conserved RNA-binding PAZ domain and may be involved in the development and maintenance of stem cells through the RNA-mediated gene-quelling mechanisms associated with DICER ( Yuan Z, Sotsky Kent T, Weber TK., Oncogene 2003;22(40):6304-10., Sasaki T, Shiohama A, Minoshima S, Shimizu N., Genomics 2003;82(3):323-30. The association of this gene with hereditary MSI tumors is unknown and needs further investigation.

## Example 5

### Cross platform classification

The gene expression level by was measured real-time PCR of the nine classifier genes in a subset of 18 MSS and MSI samples. Median centered and scaled PCR data gave the same overall picture as clustered array data from the 18 samples (Fig. 4A). As *SET* and *ATP9a* did not work well in the PCR reaction, and only seven of the nine classifier genes *(HNRPL, MTA1L1, SFR6, CXCL10, HCA112, FLJ20618* and *PRKCBP1*) were used and quantified the transcripts from these genes by real-time PCR in a new independent sample set of 47 tumors containing 35 MSS and 12 MSI. The classifier correctly classified 45 of 47 tumors with only two MSI tumors being classified as MSS (Fig. 4B).

## Example 6

### Relation between microsatellite-instability status, stage and survival

The 9-gene classifier was used to classify 36 patients with Stage II tumors and found that 17 were MSI and 19 MSS. The overall survival was highly significantly related to the classification since 10 of 11 patients that died within five years belonged to the MSS group (P=0.0014) (Fig. 5A). Thus, as expected the classifier clearly proved to be a strong predictor of survival in stage II disease and probably can be used to select those MSS patients who may benefit from adjuvant chemotherapy.

Among 65 patients with Stage III tumors receiving adjuvant chemotherapy, 16 were classified as MSI tumors and 49 as MSS tumors. As 6 MSI and 30 MSS patients died within five years of follow-up there was no significant difference in overall survival between these groups (P=0.55) (Fig. 5B). A trend was that the patients with MSI tumors showed a poorer short-term survival than those with MSS tumors, contrary to stage II patients. This difference may be in accordance with a recent large study which showed that chemotherapy only benefit the MSS tumor patients ( Ribic CM, Sargent DJ, Moore MJ, et al., N Engl J Med 2003;349(3):247-57).

**Study 2**

**Background**

Colon cancers microsatellite instability status is a better marker for response to adjuvant chemotherapy with fluorouracil than tumour stage II and III. The majority of hereditary colorectal cancer cases are microsatellite instable. We investigated the possibility of classifying colon tumors based on gene expression in crude biopsies and correlated these to crude survival and investigated if the gene expression profile can also identify hereditary cases from sporadic cases.

**Methods**

Gene transcripts from tumour specimens were quantified using microarray technology. The tumors were clustered using unsupervised and supervised classification algorithms. Sets of genes were defined for classification of microsatellite instability status and sporadic verses hereditary microsatellite instable tumors. Real-time PCR was used to validate microarray data and to investigate platform dependency in a new independent set of 47 colorectal tumors.

**Results**

Unsupervised hierarchical clustering revealed that tumors were essentially separated according to microsatellite instability status. Supervised classification of the 97 tumor samples using a maximum likelihood classifier with a cross-validation loop resulted in tree misclassification as compared to microsatellite analysis using from 106 genes and down to only seven genes. The stability of Classification of colon tumors in relation to microsatellite status was tested by permutation analysis. The sensitivity for diagnosis of microsatellite stable tumors exceeded 99% with a specificity exceeding 96%. The positive and negative predictive values exceeded 95% and 98%, respectively. The classifier was demonstrated not to be platform dependent as it could successfully be reproduced by real-time PCR. This was further verified as the classifier also correctly classified 95.7% of a new independent set of 47 colorectal tumors using real-time PCR.

Based on microarray data we identified ten genes that were highly correlated with hereditary disease. Using down to two of these genes 36 of 37 microsatellite instable tumors could be correctly separated into sporadic and hereditary MSI-H colorectal tumors.

Crude survival according to microsatellite status as determined by the classifiers, revealed that stage II colon receiving no adjuvant chemotherapy, that patient displaying microsatellite instability had significantly longer overall survival than patient exhibiting microsatellite stable tumors (P=0.0014).

By contrast, the patient with Dukes' C tumors displaying microsatellite instability did not have a significant increase in overall survival as compared to patient exhibiting microsatellite stable tumors (P=0.55).

**Conclusion**

Colon cancer can be stratified into two molecular distinct groups by quantification of the transcripts of 106 genes or even down to seven genes. The two groups are highly correlated with microsatellite stable (MSS) and microsatellite instable (MSI) tumors. The 7-gene classifier clearly proved to be a strong predictor of survival in Dukes B and it can be used to select patients who need adjuvant chemotherapy, namely those classified as MSS. We demonstrate that this classification is also valid when performed by real-time PCR analysis allowing a fast diagnosis in a clinical setting. Finally, sporadic from hereditary cases in tumors exhibiting microsatellite instability can be identified based on gene expression monitoring.

**Introduction**

Colon is the fourth most frequently diagnosed malignancy and the second most common cause of cancer death in the western world. The standard treatment of colon cancer is advised according to tumor stage. Patient with Dukes' C colon cancer receives a flurouracil-based adjuvant systemic chemotherapy in addition to surgical resection of the tumor, whereas the treatment for Dukes' B patients is based alone on surgical resection.

There is accumulating evidence that these cancers belong to two distinct molecular types according to genetic alterations. The mutator phenotype featuring tumors with microsatellite instability (MSI) and the suppressor pathway displaying chromosomal instability and microsatellite stable (MSS). MSI has been defined as a change of any length due to either insertions or deletions of repeating units in a microsatellite within a tumor compared to normal tissue and is caused by an underlying defect in the mismatch repair (MMR) system. (Boland et al, CR 1998, 58:5248). The MSI pathway may either be sporadic or hereditary (HNPCC) and whereas the disruption of the MMR system in sporadic MSI tumors is most often caused by somatic methylation of the MLH1 gene promoter more that 90% of HNPCC cancers are caused by germline mutations in MLH1 or MSH2.

The MSS pathway to cancer begins with the inactivation of tumor suppressor genes, such as APC/β-catenin genes, followed by activation of oncogenes and inactivation of additional tumor suppressor genes, commonly with a high frequency of allelic losses and cytogenetic abnormalities and abnormal DNA tumor content. Many studies have defined the pathoclinical trait of MSI and MSS tumors and found that MSI positive cancers are most frequently found in the right side of the colon, they tend to be of less differentiated, they tend to be larger in size, are often mucinous and often exhibit extensive infiltration by lymphocytes.

Crude survival data suggest that patients with HNPCC have a better prognosis than those with sporadic disease [48,49,50] and studies have also shown that MSI is an independent indicator of good prognosis [35,52,53]. Recently it was shown that MSS benefit from 5-FU treatment/leucovin treatment (New England J Med, august 2nd, 2003) whereas MSI cancer patients gained no advantage in survival.

Gene expression profiling has become an increasing used method for classification, outcome prediction, prediction of response (for a review see Dyrskjøt, expert opinion). Most such studies dealing with colon cancer have dealt with the identification of general tumor markers (Alon U; Levine AJ PNAS (1999); Kitahara O; Tsunoda T., Cancer Res (2001); Notterman DA; Levine AJ, Cancer Res (2001); Yanagava R; Nakamura (2001); Zou TT; Meltzer SJ, Oncogene (2002), Demtroeder CR (2002)), markers for benign adenomas versus adenocarcinomas (Lin YM; Nakamura Y, Oncogene (2002); Williams NS; Becerra C., Clin Cancer Res (2003)), staging (Frederiksen CM; Omtoft TF, J Cancer Res Clin Oncol (2003)), or liver metastasis (Takemasa I; Matsubara K., Biochem Biophys Res Commun (2001); Yanagawa R; Nakamura, Neoplasia (2001); Agrawal D; Yeatman T, J Natl Cancer Inst (2002)). One study has addressed the separation of low-frequency microsatellite instability tumors (MSI-L) from MSI and MSS (PCA) (Mori Y; Meltzer SJ, Cancer Res (2003)). The aim of this study was to build a general applicable and robust classifier based on gene expression to separate MSS from MSI tumors. To achieve such robustness the tumors for this study were collecting from 14-16 different clinics, RNA was isolated using different methods and labelled in several batches. Gene expression was measured by DNA microarrays of 101 Danish and Finnish tumors from primary colon cancer patients along with 17 normal biopsies.

## Methods

**Biological material** From the Danish and Finnish CRC tissue banks 101 primary colon cancers and 17 macroscopically normal colon epithelium samples from the oral resection edge were chosen. Only adenocarcinomas from Dukes' stage B and C were included, however, these represented a broad spectrum of tumors in relation to location, heredity, microsatellite instability status, and origin of the patient. All tumors were collected in the period from 1994 to 2002, 68 tumor samples were collected at nine different clinics in Finland and 33 samples were collected at four different clinics in Denmark, 36 were Dukes' B, 67 Dukes' C, 41 were sporadic microsatellite highly instable (MSI-H) of which were 17 HNPCC, and 59 were sporadic microsatellite stable (MSS) (table 19). None of the patients received pre-operative radiation or chemotherapy.

Table 19

Summary of clinocopathological and microsatellite features of colon samples

| Patient group n (DK,SF) | | Median age range | Localization in colon | | Dukes' Stage n (DK,SF) | | | IHC negative stain N (n tested) | |
|---|---|---|---|---|---|---|---|---|---|
| | | | right (DK,SF) | left (DK, SF) | N[a] | B | C | MLH1 | MSH2 |
| All cases | 119 (44,75) | 62.0 | 45 (8,37) | 74 (36,38) | 17 (6,11) | 36 (14,22) | 66 (20,46) | 12 (56) | 1 (56) |

(continued)

Summary of clinocopathological and microsatellite features of colon samples

| Patient group n (DK,SF) | | Median age range | Localization in colon | | | Dukes' Stage n (DK,SF) | | IHC negative stain N (n tested) | |
|---|---|---|---|---|---|---|---|---|---|
| | | | right (DK,SF) | left (DK, SF) | N[a] | B | C | MLH1 | MSH2 |
| MSi-H[b] | 24 (9,16) | 67.0 | 15 (3,12) | 9 (6,4) | - | 10 (3,7) | 14 (5,9) | 6 (11) | 0 (11) |
| HNPCC[c] | 17(4,13) | 45.0 | 9 (2,7) | 8 (2,6) | - | 10 (2,8) | 7 (2,5) | 6 (8) | 1 (8) |
| MSS | 60 (25,35) | 63.0 | 11 (0,11) | 49 (25,24) | - | 16 (9,7) | 44 (16,28) | 0 (37) | 0 (37) |

[a]normal biopsy taken from the resection edge of a tumor
[b]according to microsatellite analysis
[c]all tumors MSI-H[b]

**Microsatellite-instability analysis.** From all tumor samples available as paraffin blocks, ten sections were cut at 10μm and stained with haematoxylin. The first and last section was cut at 4 μm and stained with haematoxylin. These two sections were used for the identification of tumor and normal cells from each sample. Regions enriched in tumor cells (more than 90%) were microdissected from these sections and DNA was extracted using a Puregene DNA extraction kit (Gentra Systems, Minneapolis, MN). DNA from blood samples was used as control when available, otherwise normal tissue was microdissected from the tissue sections. The samples were analysed for microsatellite instability according to the NCI guidelines (Boland et al). Samples positive for markers BAT25 and BAT26 were scored as MSI-H. Samples positive for only one of these markers were tested for further markers and scored as MSI-L if none of these tested positive. Since MSI-L has similar clinical features as MSS these samples were considered as MSS in this study. In addition to microsatellite analysis all tumors from which paraffin blocks were available were tested for the presence of MLH1 and MSH2 protein by immunohistochemistry. None of the samples scored MSS were negative for either protein whereas six of the MSI scored samples were positive for both (Table 19).

**RNA purification** Colon specimens were obtained fresh from surgery and were immediately snap frozen in liquid nitrogen either as was, in OCD-compound or in a SDS/guadinium thiocyanate solution. Total RNA was isolated using RNAzol (WAK-Chemie Medical) or spin column technology (Sigma) following the manufactures' instructions.

**Gene expression analysis** These procedures were performed at described in detail elsewhere (Dyrskødt et al). Briefly, ten μg of total RNA was used as starting material for the target preparation as described. First and second strand cDNA synthesis was performed using the SuperScript II System (Invitrogen) according to the manufacturers' instructions except using an oligo-dT primer containing a T7 RNA polymerase promoter site. Labelled aRNA was prepared using the BioArray High Yield RNA Transcript Labelling Kit (Enzo) using Biotin labelled CTP and UTP (Enzo) in the reaction together with unlabeled NTP's. Unincorporated nucleotides were removed using RNeasy columns (Qiagen). Fifteen μg of cRNA was fragmented, loading onto the Affymetrix HG_U133A probe array cartridge and hybridized for 16h. The arrays were washed and stained in the Affymetrix Fluidics Station and scanned using a confocal laser-scanning microscope (Hewlett Packard GeneArray Scanner G2500A). The readings from the quantitative scanning were analyzed by the Affymetrix Gene Expression Analysis Software (MAS 5.0) and normalized using RMA (robust multi array normalisation, Irizarry et al. 2002) in the statistical application R. Redundant probesets (as defined form Unigene build 168) with high correlation (>0.5) over all samples were removed, which reduced the dataset to approximately 14.400 probesets. This dataset was used a source for all further calculations in this manuscript.

**Unsupervised agglomerative hierarchical clustering**

For hierarchical cluster analysis 1239 genes with a variation across all samples greater than 0.5 were median-centred to a magnitude of 1. Samples and genes were then clustered using average linkage clustering with a modified Person correlation as similarity metric (Eisen et al., PNAS 95: 14863-14868, 1998). The cluster dendrogram was visualized with TreeView (Eisen).

## Group testing

We make a statistical test where the p-value is evaluated through permutations. For each group and gene we calculate the average and the sum of squared deviations from the average. We then sum these over the genes and the groups:

$$S_1 = \sum_{groups} \sum_{genes} (X_{ij} - \bar{X}_{gr(i)j})^2$$

This expression is calculated for joining DK with SF and MSI with MSS such that we end up with two groups. The sum of squared deviations is denoted $S_2$. As a test statistic we use $S_1/S_2$. A small value indicates that there is a real reduction in the deviations when going from 2 to 4 groups and thus the groups have a real significance. To judge if a value is significantly small we use permutations. For each of the four groups left when joining DK and SF we randomly allocate the members to a pseudo DK and pseudo SF in such a way that the number of members in each group are as in the original data.

To get an understanding of this separation we performed a test to see if this is caused by few genes or if many genes are involved. For this test we calculated $S_1 = \Sigma_{genes}$ S1(gene) and similarly with $S_2 = \Sigma_{genes} S_2$(gene). For each gene j we used the test statistic $S_1(j)/S_2(j)$ (Table 3).

## Multidimentional scaling

We carried out multidimentional scaling on median-centered and normalized data using CMD-scale in the statistical application R and visualized in a two-dimentional plot.

## Microsatellite status classifier

The readings from the quantitative scanning were analyzed by the Affymetrix Gene Expression Analysis Software (MAS 5.0) and normalized using RMA (robust multi array normalisation, Irizarry et al. 2002) in the statistical application R. Redundant probesets (as defined form Unigene build 168) with high correlation (>0.5) over all samples were removed, which reduced the dataset to approximately 14.400 probesets.

The microsatellite instability status classifier was based on a dataset of 4.266 genes. These genes result from the removal of genes with a variance over all tumor samples smaller than 0.2 and genes that separate Danish from Finnish samples with a t-value numerically greater than 2. We used a normal distribution with the mean dependent on the gene and the group (MSI, MSS). For each gene, we calculated the variation between the groups and the variation within the groups to select genes with a high ratio between these. To classify a sample, we calculated the sum over the genes of the squared distance from the sample value to the group mean, standardized by the variance and assigned the sample to the nearest group. The sample to be classified was excluded when calculating group means and variances.

## Estimation of classifier stability

We validated the performance of the classifier by permutation. One hundred datasets consisting of 30 MSS samples and 25 MSI samples were randomly chosen by permutation for training of the classifier with the remaining samples in each case being assign to a testset. Averages over the 100 data sets of the number of errors in the cross-validation of the training set and in the test set were used as a measure of the precision of the classifier.

**Real-time PCR (RT-PCR).** The procedures were as described (Birkenkamp-Demtroder) except that we used short LNA (Locked Nucleic Acid) enhanced probes from a Human Probe Library (Exiqon™). In short, cDNA was synthesized from single samples some of which were previously analyzed on GeneChips. Reverse transcription was performed using Superscript II RT (Invitrogen). Real-time PCR analysis was performed on selected genes using the primers (DNA Technology) and probes (Exiqon, DK) described in figure legend X. All samples were normalized to GAPDH as described previously (Birkenkamp-Demtroder et. al. Cancer Res., 62: 4352-4363, 2002).

## Rebuilding of Classifier based on Real-Time PCR

The 79 tumors samples that were not analysed by real-time PCR were transformed into log ratios using one of the tumor samples as reference and used for training of the classifier. Then 23 samples of which 18 were also analyzed on arrays were equally transformed into log ratios using the same tumor sample as above as reference and tested. The idea behind this translation is that we expect the normalized PCR values to be proportional to the normalized array values, and on a log scale this becomes an additive difference. The difference is gene specific and is therefore estimated for each gene separately. The variation obtained from the microarray data, and used in the classifier, can be used directly on the PCR platform.

## Results

### Hierarchical clustering

The clinical specimens used in this study were collected in two different countries from 14 different clinics in the period 1994 to 2001. The samples were selected to keep a balanced representation of microsatellite instable (MSI) and microsatellite stable (MSS) tumors from both the right- and left-sided colon. The MSI class was represented both by sporadic MSI and hereditary MSI (HNPCC) tumors. Only Dukes' B and Dukes' C tumor samples were included were selected (table 19). Before any attempt to divide a diverse sample collection into distinct classes analyzed the data for systematic bias that may have been introduces during the experimental procedures. A fast and easy way to discover both true distinct classes as well as systematic biases in the data is to perform a hierarchical clustering.

The phylogenetic tree resulting from hierarchical clustering on 1239 genes (Fig. 6) reveals that the main separating factor is microsatellite status. On the upper trunk we find two clusters represented mainly by normal biopsies (14/21) and MSS tumors (18/25), respectively. The lower trunk is divided into a MSI cluster (30/36) and a second MSS cluster (MSS2-cluster) (34/37). A closer inspection of the two MSS clusters unveil that one is dominated by Danish samples (19/25) and one by Finnish samples (26/37 check). Also, it is worth to notice that the MSI cluster contains a vast majority of Finnish samples (32/36) and that the sporadic MSI samples are interspersed among the hereditary samples. The normal biopsies cluster tight together with a slight tendency to separation according to origin. Tree normal samples cluster within the MSI cluster indicating that resection of these samples may have been to close to the tumor lesion.

Inspection of the gene cluster dendrogram shows that the two groups of MSS tumors are mainly separated by a large cluster of genes being upregulated in the Danish samples (data not shown) indicating that a systematic difference between Danish and Finnish samples.

### Significance of observed groups

Based on these observations, we performed a series of test to evaluate if the observed separation of tumors into MSS and MSI as well as DK and SF are significant. For these tests the tumor samples were grouped into four virtual tumor-groups labelled, i.e. Danish MSI (MSI-DK), Danish MSS (MSS-DK), Finnish MSI (MSI-SF) and Finnish MSS (MSS-SF). Based on 5082 genes with a variance above 0.2, we tested if all four groups are significant or if some of the groups can be joined. We considered the two possibilities of joining DK and SF, and of joining MSI and MSS and made a statistical test where the p-value is evaluated through permutations. In 100 permutations of each group combination our test value S1/S2 is considerably smaller than in all permutation (Table 20) demonstrating a very clear separation between DK and SF and between MSI and MSS.

Table 20

Permutation test of groups

| Pseudo group | S1/S2 from data | Smaller values in 100 permutations | Minimum in 100 permutations |
|---|---|---|---|
| DK-SF | 0.9072795 | 0 | 0.962269 |
| I-S | 0.9166195 | 0 | 0.9583325 |

Such a clear distinction between groups may rely on a few highly separating genes or a general difference in the gene expression profile including many genes. For both the DK-SF and MSI-MSS the effect are caused by many genes even at very criteria, i.e. low test statistic $S_1(j)/S_2(j)$ values (Table 21).

Table 21

Permutation test of genes

| | | $S_1(j)/S_2(j)$ | | | |
|---|---|---|---|---|---|
| Pseudo group | | < 0.6 | < 0.7 | < 0.8 | < 0.9 |
| DK-SF | number of genes | 36 | 136 | 522 | 1785 |

(continued)

Permutation test of genes

| | | $S_1(j)/S_2(j)$ | | | |
|---|---|---|---|---|---|
| Pseudo group | | < 0.6 | < 0.7 | < 0.8 | < 0.9 |
| | max in 100 permutations | 0 | 0 | 2 | 225 |
| MSI-MSS | number of genes | 17 | 103 | 399 | 1507 |
| | max in 100 permutations | 0 | 1 | 8 | 250 |

When a property is present that influences a large proportion of the genes this may obscure separation of clinical relevant features in unsupervised clustering. To visualize the effect of such properties, we calculated distances by multidimensional scaling between samples with and without of 816 genes separating DK from SF with a t-value numerically greater than 2 (Fig 7). We see an improved separation of MSI and MSS with Danish and Finnish cases mixed. The MSI-DK samples are not completely separated as they are found both between the MSI-SF and the MSS samples. (These plots are not entirely unsupervised since the groups have been used to remove gene).

**Construction of an MSI-MSS classifier**

For the construction of a classifier we used the expression profiles from 97 tumors for which no ambiguity had been identified in relation to microsatellite status. The 816 genes separating DK from SF were excluded, as these would be unreliable for MS classification. We built a maximum likelihood classifier in order to select a minimum of genes giving the largest possible separation of the two groups. We tested the performance of the classifier using 1-1000 genes and found that it was stable showing 3-6 errors when using 4 - 400 genes. Of these 106 genes were especially suited for discrimination of MSS from MSI (table 22).

Table 22

| ***AFFYID*** | ***SYMBOL*** | ***LOCUS LINK*** | ***OMIM*** | ***REFSEQ*** | ***GENENAME*** |
|---|---|---|---|---|---|
| 1405_I_at | CCL5 | 6352 | 187011 | NM 002985 | chemokine (C-C motif) ligand 5 |
| 200628_s_at | WARS | 7453 | 191050 | NM 004184 | tryptophanyl-tRNA synthetase |
| 200814_at | PSME1 | 5720 | 600654 | NM_006263 | proteasome (prosome, macropain) activator subunit 1 (PA28 alpha) |
| 201641_at | BST2 | 684 | 600534 | NM 004335 | bone marrow stromal cell antigen 2 |
| 201649_at | UBE2L6 | 9246 | 603890 | NM_004223 | ubiquitin-conjugating enzyme E2L 6 |
| 201674_s_at | AKAP1 | 8165 | 602449 | NM_003488 | A kinase (PRKA) anchor protein 1 |
| 201762_s_at | PSME2 | 5721 | 602161 | NM_002818 | proteasome (prosome, macropain) activator subunit 2 (PA28 beta) |
| 201884_at | CEACAM5 | 1048 | 114890 | NM 004363 | carcinoembryonic antigen-related cell adhesion molecule 5 |
| 201910_at | FARP1 | 10160 | 602654 | NM 005766 | FERM, RhoGEF (ARHGEF) and pleckstrin domain protein 1 (chondrocyte-derived) |
| 201976_s_at | MY010 | 4651 | 601481 | NM 012334 | myosin X |
| 202072_at | HNRPL | 3191 | 603083 | NM 001533 | heterogeneous nuclear ribonucleoprotein L |
| 202203_s_at | AMFR | 267 | 603243 | NM_001144 | autocrine motility factor receptor |

(continued)

| AFFYID | SYMBOL | LOCUS LINK | OMIM | REFSEQ | GENENAME |
|---|---|---|---|---|---|
| 202262_x_at | DDAH2 | 23564 | 604744 | NM 013974 | dimethylarginine dimethylaminohydrolase 2 |
| 202510_s_at | TNFAIP2 | 7127 | 603300 | NM 006291 | tumor necrosis factor, alpha-induced protein 2 |
| 202520_s_at | MLH1 | 4292 | 120436 | NM 000249 | mutL homolog 1, colon cancer, nonpolyposis type 2 (E. coil) |
| 202589_at | TYMS | 7298 | 188350 | NM 001071 | thymidylate synthetase |
| 202637_s_at | ICAM1 | 3383 | 147840 | NM 000201 | intercellular adhesion molecule 1 (CD54), human rhinovirus receptor |
| 202678_at | GTF2A2 | 2958 | 600519 | NM 004492 | general transcription factor IIA, 2.12KDa |
| 202762_at | ROCK2 | 9475 | 604002 | NM 004850 | Rho-associated, coiled-coil containing protein kinase 2 |
| 203008_x_at | APACD | 10190 | | NM 005783 | ATP binding protein associated with cell differentiation |
| 203315_at | NCK2 | 8440 | 604930 | NM 003581 | NCK adaptor protein 2 |
| 203335_at | PHYH | 5264 | 602026 | NM 006214 | phytanoyl-CoA hydroxylase (Refsum disease) |
| 203444_s_at | MTA2 | 9219 | 603947 | NM 004739 | metastals-associated gene family, member 2 |
| 203559_s_at | ABP1 | 26 | 104610 | NM 001091 | amiloride binding protein 1 (amine oxidase (copper-containing)) |
| 203773_x_at | BLVRA | 644 | 109750 | NM 000712 | biliverdin reductase A |
| 203896_s_at | PLCB4 | 5332 | 600810 | NM_000933 | phospholipase C, beta 4 |
| 203915_at | CXCL9 | 4283 | 601704 | NM 002416 | chemokine (C-X-C motif) ligand 9 |
| 204020_at | PURA | 5813 | 600473 | NM 005859 | purine-rich element binding protein A |
| 204044_at | QPRT | 23475 | 606248 | NM_014298 | quinolinate phosphoribosyltransferase (nicotinate-nucleotide pyrophosphorylase (carboxylating)) |
| 204070_at | RARRES3 | 5920 | 605092 | NM 004585 | retinoic acid receptor responder (tazarotene induced) 3 |
| 204103_at | CCL4 | 6351 | 182284 | NM 002984 | chemokine (C-C motif) ligand 4 |
| 204131_s_at | FOXO3A | 2309 | 602681 | NM 001455 | forkhead box O3A |
| 204326_x_at | MT1X | 4501 | 156359 | NM 005952 | metallothionein 1X |
| 204415_at | G1P3 | 2537 | 147572 | NM_002038, NM_022873 | interferon, alpha-inducible protein (clone IFI-6-16) |
| 204533_at | CXCL10 | 3627 | 147310 | NM 001565 | chemokine (C-X-C motif) ligand 10 |

(continued)

| AFFYID | SYMBOL | LOCUS LINK | OMIM | REFSEQ | GENENAME |
|---|---|---|---|---|---|
| 204745_x_at | MT1G | 4495 | 156353 | NM_005950, NM_005950 | metallothionein 1G |
| 204780_s_at | TNFRSF6 | 355 | 134637 | NM_000043, NM_152877, NM_152876, NM_52875, NM_152872, NM_152873, NM_152871 | tumor necrosis factor receptor superfamily, member 6 |
| 204858_s_at | ECGF1 | 1890 | 131222 | NM 001953 | endothelial cell growth factor 1 (platelet-derived) |
| 205241_at | SCO2 | 9997 | 604272 | NM 005138 | SCO cytochrome oxidase deficient homolog 2 (yeast) |
| 205242_at | CXCL13 | 10563 | 605149 | NM 006419 | chemokine (C-X-C motif) ligand 13 (B-cell chemoat-tractant) |
| 205495_s_at | GNLY | 10578 | 188855 | NM_006433, NM_006433 | granulysin |
| 205831_at | CD2 | 914 | 186990 | NM 001767 | CD2 antigen (p50), sheep red blood cell receptor |
| 206108_s_at | SFRS6 | 6431 | 601944 | NM 006275 | splicing factor, arginine/serine-rich 6 |
| 206286_s_at | TDGF1 | 6997 | 187395 | NM 003212 | teratocarcinoma-derived growth factor 1 |
| 206461_x_at | MT1H | 4496 | 156354 | NM 005951 | metallothionein 1H |
| 20654_s_at | CYP2B6 | 1555 | 123930 | NM 000767 | cytochrome P450, family 2, subfamily B, polypeptide 6 |
| 20607_at | TNFSF9 | 8744 | 606182 | NM 003811 | tumor necrosis factor (ligand) superfamily, member 9 |
| 206918_s_at | RBM12 | 10137 | 607179 | NM_006047, NM_006047 | RNA binding motif protein 12 |
| 206976_s_at | HSPH1 | 10808 | | NM 006644 | heat shock 105kDa/110kDa protein 1 |
| 207320_x_at | STAU | 6780 | 601716 | NM_004602, NM_004602, NM_017452, NM_017453 | staufen, RNA binding protein (Drosophila) |
| 207457_s_ at | LY6G6D | 58530 | 606038 | NM_021246 | lymphocyte antigen 6 complex, locus G6D |
| 207993_s_at | CHP | 11261 | 606988 | NM_007236 | calcium binding protein P22 |
| 208022_s_at | CDC14B | 8555 | 603505 | NM_003671, NM_003671, NM_033331 | CDC14 cell division cycle 14 homolog B (S. cerevisiae) |
| 208156_x_at | EPPK1 | 83481 | | | epiplakin 1 |

(continued)

| AFFYID | SYMBOL | LOCUS LINK | OMIM | REFSEQ | GENENAME |
|---|---|---|---|---|---|
| 208581_x_at | MT1X | 4501 | 156359 | NM_005952 | metallothionein 1X |
| 208944_at | TGFBR2 | 7048 | 190182 | NM_003242 | transforming growth factor, beta receptor II (70/80kDa) |
| 209048_s_at | PRKCBP1 | 23613 | | NM_012408, NM_012408, NM_183047 | protein kinase C binding protein 1 |
| 209108_at | TM4SF6 | 7105 | 300191 | NM_003270 | transmembrane 4 superfamily member 6 |
| 209504_s_at | PLEKHB1 | 58473 | 607651 | NM_021200 | pleckstrin homology domain containing, family B (evectins) member 1 |
| 209546_s_at | APOL1 | 8542 | 603743 | NM_003661, NM_003661, NM_145343 | apolipoprotein L, 1 |
| 210029_at | INDO | 3620 | 147435 | NM_002164 | indoleamine-pyrrole 2,3 dioxygenase |
| 210103_s_at | FOXA2 | 3170 | 600288 | NM_021784, NM_021784 | forkhead box A2 |
| 210321_at | GZMH | 2999 | 116831 | NM_033423 | granzyme H (cathepsin G-like 2, protein h-CCPX) |
| 210538_s_at | BIRC3 | 330 | 601721 | NM_001165, NM_001165 | baculoviral IAP repeat-containing 3 |
| 211456_x_at | AF333388 | | | | |
| 212057_at | KIAA0182 | 23199 | | NM_050495 | KIAA0182 protein |
| 212070_at | GPR56 | 9289 | 604110 | NM_005682 | G protein-coupled receptor 56 |
| 212185_x_at | MT2A | 4502 | 156360 | NM_005953 | metallothionein 2A |
| 212229_s_at | FBXO21 | 23014 | | NM_015002, NM_015002 | F-box only protein 21 |
| 212336_at | EPB41L1 | 2036 | 602879 | NM_012156, NM_012156 | erythrocyte membrane protein band 4.1-like 1 |
| 212341_at | MGC21416 | 286451 | | NM_173834 | hypothetical protein MGC21416 |
| 212349_at | POFUT1 | 23509 | 607491 | NM_015352, NM_015352 | protein O-fucosyltransferase 1 |
| 212859_x_at | MT1E | 4493 | 156351 | NM_175617 | metallothionein 1E (functional) |
| 213201_s_at | TNNT1 | 7138 | 191041 | NM_003283, NM_003283, XM_352926 | troponin T1, skeletal, slow |
| 213385 at | CHN2 | 1124 | 602857 | NM_004067 | chimerin (chimaerin) 2 |
| 213470_s_at | HNRPH1 | 3187 | 601035 | NM_005520 | heterogeneous nuclear ribonucleoprotein H1 (H) |

(continued)

| AFFYID | SYMBOL | LOCUS LINK | OMIM | REFSEQ | GENENAME |
|---|---|---|---|---|---|
| 213738_s_ at | ATP5A1 | 498 | 164360 | NM_004046 | ATP synthase, H+ transporting, mitochondrial F1 complex, alpha subunit, isoform 1, cardiac muscle |
| 213757_at | EIF5A | 1984 | 600187 | NM_001970 | eukaryotic translation initiation factor 5A |
| 214617_at | PRF1 | 5551 | 170280 | NM_005041 | perforin 1 (pore forming protein) |
| 214924_s_at | OIP106 | 22906 | 608112 | NM_014965 | OGT(O-Glc-NAc transferase)-interacting protein 106 KDa |
| 215693_x_at | DDX27 | 55661 | | NM_017895 | DEAD (Asp-Glu-Ala-Asp) box polypeptide 27 |
| 215780_s_at | Hs.382039 | | | | |
| 216336_x_at | AL031602 | | | | |
| 217727_x_at | VPS35 | 55737 | 606931 | NM_018206 | vacuolar protein sorting 35 (yeast) |
| 217759_at | TRIM44 | 54765 | | NM_017583 | tripartite motif-containing 44 |
| 217875_s_at | TMEPAI | 56937 | 606564 | NM_020182, NM_020182, NM_199169, NM_199170 | transmembrane, prostate androgen induced RNA |
| 217917_s_at | DNCL2A | 83658 | 607167 | NM_014183, NM_014183, NM_177953 | dynein, cytoplasmic, light polypeptide 2A |
| 217933_s_at | LAP3 | 51056 | 170250 | NM_015907 | leucine aminopeptidase 3 |
| 218094_s_at | C20orf35 | 55861 | | NM_018478, NM_018478 | chromosome 20 open reading frame 35 |
| 218237_s_ at | SLC38A1 | 81539 | | NM_030674 | solute carrier family 38, member 1 |
| 218242_s_at | CGI-85 | 51111 | | NM_016028, NM_016028 | CGI-85 protein |
| 218325_s_at | DATF1 | 11083 | 604140 | NM_022105, NM_022105, NM_080796 | death associated transcription factor 1 |
| 218345 at | HCA112 | 55365 | | NM_018487 | hepatocellular carcinoma-associated antigen 112 |
| 218346_s_at | SESN1 | 27244 | 606103 | NM_014454 | sestrin 1 |
| 218704_at | FLJ20315 | 54894 | | NM_017763 | hypothetical protein FLJ20315 |
| 218802_at | FLJ20647 | 55013 | | NM_017918 | hypothetical protein FLJ20647 |
| 218898_at | CT120 | 79850 | | NM_024792 | membrane protein expressed in epithelial-like lung adenocarcinoma |

(continued)

| AFFYID | SYMBOL | LOCUS LINK | OMIM | REFSEQ | GENENAME |
|---|---|---|---|---|---|
| 218943_s_at | RIG-I | 23586 | | NM_014314 | DEAD/H (Asp-Glu-Ala-Asp/His) box polypeptide |
| 218963_s_at | KRT23 | 25984 | 606194 | NM_015515, NM_015515 | keratin 23 (histone deacetylase inducible) |
| 219956 at | GALNT6 | 11226 | 605148 | NM_007210 | UDP-N-acetyl-alpha-D-galactosamine:polypeptide N-acetylgalactosaminyltransferase 6 (GalNAc-T6) |
| 220658_s_at | ARNTL2 | 56938 | | NM_020183 | aryl hydrocarbon receptor nuclear translocator-like 2 |
| 220951_s_at | ACF | 29974 | | NM_014576, NM_014576, NM_138932 | apobec-1 complementation factor |
| 221516_s_at | FLJ20232 | 54471 | | NM_019008 | hypothetical protein FLJ20232 |
| 221653_x_at | APOL2 | 23780 | 607252 | NM_030882, NM_030882 | apolipoprotein L, 2 |
| 221920_s_ at | MSCP | 51312 | | NM_016612, NM_016612 | mitochondrial solute carrier protein |
| 222244_s_at | FLJ20618 | 55000 | | NM_017903 | hypothetical protein FLJ20618 |

The minimum of three errors was found even using only 7 genes (Table 23).

Table 23. Genes used for the classification of MSS vs MSI tumors

| Name | Symbol | Unigene | MSS | MSI |
|---|---|---|---|---|
| hepatocellular carcinoma-associated antigen 112 | HCA112 | Hs.12126 | 1261 | 653 |
| metastasis-associated 1-like 1 | MTA1L1 | Hs.173043 | 45 | 91 |
| chemokine (C-X-C motif) ligand 10 | CXCL10 | Hs.2248 | 104 | 274 |
| heterogeneous nuclear ribonucleoprotein L | HNRPL | Hs.2730 | 194 | 630 |
| hypothetical protein FLJ20618 | FLJ20618 | Hs.52184 | 776 | 388 |
| splicing factor, arginine/serine-rich 6 | SFRS6 | Hs.6891 | 74 | 446 |
| protein kinase C binding protein 1 | PRKCBP1 | Hs.75871 | 294 | 168 |

**Classification of ambiguous samples**

Application of the 7-gene classifier to the four samples showing ambiguity in the microsatellite analyses assigns all four to be microsatellite stable tumor class. Notably, all four showed expression levels of *Tumor Growth Factor* β *induced protein* (TFGBI), MLH1 and thymidylate synthase (TYMS) that are atypical for MSI tumors. Furthermore, these tumors were all from the left colon. Thus the misclassified tumors are clearly truly MSS or they belong to a yet undefined class of MSI tumors.

**Stability of classification**

To estimate the stability of the classifier based on all 97 tumor samples, we generated one hundred new classifiers based on randomly chosen datasets consisting of 30 MSS and 25 MSI samples. In each case the classifiers were tested with the remaining samples. The performance for each set was evaluated and averaged over all 100 training and test sets (Table 24). The mean error rate for MSS tumors was 0.52% and 1.38% for MSI tumors. The seven genes defined above were found to be those genes that were most frequently used in the crossvalidation loop. More than 50% of the errors were related to three tumors of which two were wrongly classified in all permutation and one in 94%. The remaining errors were mainly caused by four tumors with error rates of 40-47% showing that the former three samples are truly assigned contradictory to result from the microsatellite analysis and that four samples could not be assigned with confidence too any of the classes.

Table 24 Performance of the classifier

| | Trainings set<br>Errors in crossvalidation | Test set<br>Test errors |
|---|---|---|
| MSI | 2.8% (n=25, range 0-6) | 1.4% (n=10, range 0-4) |
| MSS | 0.70% (n=30, range 0-3) | 0.52% (n=29, range 0-2) |
| All | 1.7% (n=55, range 1-7) | 1.9% (n=39, range 0-5) |

Table 25

| Sensitivity, Specificity, and Predictive Value of Test for MSS based on the eight gene Classifier | | |
|---|---|---|
| Positive for MSS<br>Negative for MSS | True = (0.9948*29)=28,8492<br>False = (0.0052*29)= 0.1508 | False = (0.138*10)= 1.38<br>True = (0.962*10)= 9.62 |
| Sensitivity<br>Specificity<br>Positive predictive value<br>Negative predictive value | 28.9507/29 =<br>9.62/10 =<br>28.8492/30.2292 =<br>9.62/9.7708 = | 99.5%<br>96.2%<br>95.4%<br>98.5% |
| *Based on a prevalence for MSS of 85% | | |

**Survival classifier**

Using the same classification methods described above, we build classifiers for survival based on either all samples or the above defined groups of MSI-H and MSS. As seen in figure 10 a distinction of patient with good prognosis (>5 year survival) from patient with bad prognosis (< 5 years survival) can be achieved with higher precision and using only a fraction of the genes by first separating into MSI-H and MSS groups.

**Construction of a classifier for sporadic versus hereditary microsatellite instable tumors**

In order to identify a gene set for identification of hereditary microsatellite instable tumors we applied 19 sporadic microsatellite instable samples and 18 microsatellite instable samples to supervised classification as described above. We found ten genes we high scored for separation of sporadic MSI-H from hereditary MSI-H tumours (Table 26). In crossvalidation we found a minimum number of one error using two genes (Fig 9A) and were used in at least 36 of the 37 crossvalidation loops. The genes were: the mismatch repair gene MLH1 that show a general downregulation in sporadic disease and PIWIL1 that is lower expressed in hereditary cases (Fig 9B). Using these two genes only one error occurred: a sporadic microsatellite instable was classified as hereditary. Based on T-test we performed 500 permutations to test the significance of these two genes for marker genes and found both genes highly significant with p-values < 0.005.

Table 26

| *AFFYID* | *SYMBOL* | **LOCUSLINK** | *OMIM* | *REFSEQ* | *AFFYDESCRIPTION* |
|---|---|---|---|---|---|
| 206194_at | HOXC6 | 3223 | 142972 | NM_004503 | Homeo box C4 |
| 214868_at | PIWIL1 | 9271 | 605571 | NM_004764.2 | Piwi (Drosophila)-like 1 |
| 202520_s_at | MLH1 | 4292 | 120436 | NM_000249.2 | MutL (E. coli) homolog 1 (colon cancer, nonpolyposis type 2) |

(continued)

| AFFYID | SYMBOL | LOCUSLINK | OMIM | REFSEQ | AFFYDESCRIPTION |
|---|---|---|---|---|---|
| 202517_at | CRMP1 | 1400 | 602462 | NM_001313.2 | Collapsin response mediator protein 1 |
| 205453_at | HOXB2 | 3212 | 142967 | NM_002145.2 | Homeo box B2 (HOXB2) |
| 217791_s_at | PYCS/ADH 18A1 | 5832 | 138250 | NM_002860.2 | Pyrroline-5-carboxylate synthetase (glutamate gamma-semialdehyde synthetase) (/ PYCS/ADH18A1) |
| 202393_s_at | TIEG | 7071 | 601878 | NM_005655.1 | TGFB inducible early growth response (TIEG) |
| 218803_at | CHFR | 55743 | 605209 | NM_018223.1 | Checkpoint with forkhead and ring finger domains (CHFR) |
| 219877_at | FLJ13842 | 79698 | | NM_024645.1 | Hypothetical protein FLJ13842 (FLJ13842) |
| 202241_at | C8FW | 10221 | | NM_025195.2 | Phosphoprotein regulated by mitogenic pathways (C8FW) |

**Cross platform classification**

Real time PCR was applied both to verify the array data and examine if the 7-gene classifier would also perform on this platform. We chose 23 samples of which 18 were also analyzed on arrays. The correlation between the two platforms was high (data not shown). In order to test the performance of classification using PCR data we re-build our classifier with a 79 samples array dataset including only those tumors that were not analyzed with PCR. Two samples were classified in discordance with the microsatellite instability test of which one of them was ambiguously classified by the 7-gene array classifier.

**Relation between microsatellite-instability status, stage and survival**

Based on the 7-gene classifier, Classification of 36 patients with Dukes' B tumors receiving no adjuvant chemotherapy, 18 were classified as MSI tumors and 18 as MSS tumors. The overall survival was highly significantly related to the classification since all nine patients that died within five years of follow-up were belonged to the MSS group (P=0.0014) (Fig. 10A). Thus, the 7-gene classifier clearly proved to be a strong predictor of survival in Dukes B and it can be used to select patients who need adjuvant chemotherapy, namely those classified as MSS.

Among 65 patients with Dukes' C tumors receiving adjuvant chemotherapy, 17 were classified as MSI tumors and as 48 MSS tumors. Of these, 6 MSI and 27 MSS patients died within five years of follow-up meaning no significant difference in overall survival between these groups (P=0.55) (Fig. 10B). A trend was that the MSI showed a poorer short-term survival than the MSS, contrary to Dukes B patients. This difference can be attributed to the fact that a recent large study has shown that chemotherapy only benefit the MSS tumor patients, thus improving their survival to a level comparable to that which is characteristic of MSI tumor patients.

**Clinical application of the discovery**

In the clinic the 106 or less genes described can be used for predicting outcome of colorectal cancer when examined at the RNA level and also on the protein level as each gene identified is the project is transcribed to RNA that is further translated into protein. The genes can also be used determine which patient should be treated with chemotherapy as only non-microsatellite instable tumors will respond to 5-FU based therapy. Building classifiers can achieve a further stratification of patient with god and bad prognosis after stratification into microsatellite instable and stable tumors. The genes used to identify hereditary disease can be used to decide which patient should enter into sequencing analysis of mismatch repair genes.

The RNA determination can be made in any form using any method that will quantify RNA. The proteins can be measured with any method quantification method that can determine the level of proteins.

**References**

Agrawal D, Chen T, Irby R, Quackenbush J, Chambers AF, Szabo M, Cantor A, Coppola D, Yeatman TJ. Osteopontin identified as lead marker of colon cancer progression, using pooled sample expression profiling. J Natl Cancer Inst. 2002 Apr 3;94(7):513-21.

Birkenkamp-Demtroder K, Christensen LL, Olesen SH, Frederiksen CM, Laiho P, Aaltonen LA, Laurberg S, Sorensen FB, Hagemann R, ORntoft TF. Gene expression in colorectal cancer. Cancer Res. 2002 Aug 1;62(15):4352-63.

Boland CR, Thibodeau SN, Hamilton SR, Sidransky D, Eshleman JR, Burt RW, Meltzer SJ, Rodriguez-Bigas MA, Fodde R, Ranzani GN, Srivastava S. A National Cancer Institute Workshop on Microsatellite Instability for cancer detection and familial predisposition: development of international criteria for the determination of microsatellite instability in colorectal cancer. Cancer Res. 1998 Nov 15;58(22):5248-57. Review.

Chapusot C, Martin L, Bouvier AM, Bonithon-Kopp C, Ecarnot-Laubriet A, Rageot D, Ponnelle T, Laurent Puig P, Faivre J, Piard F. Microsatellite instability and intratumoural heterogeneity in 100 right-sided sporadic colon carcinomas. Br J Cancer. 2002 Aug 12;87(4):400-4.

Dyrskjot L, Thykjaer T, Kruhoffer M, Jensen JL, Marcussen N, Hamilton-Dutoit S, Wolf H, Orntoft TF. Identifying distinct classes of bladder carcinoma using microarrays. Nat Genet. 2003 Jan;33(1):90-6.

Frederiksen CM, Knudsen S, Laurberg S, Orntoft TF. Classification of Dukes' B and C colorectal cancers using expression arrays. J Cancer Res Clin Oncol. 2003 May;129(5):263-71.

Huang J, Qi R, Quackenbush J, Dauway E, Lazaridis E, Yeatman T. Effects of ischemia on gene expression.J Surg Res. 2001 Aug;99(2):222-7.

Irizarry RA, Bolstad BM, Collin F, Cope LM, Hobbs B, Speed TP. Summaries of Affymetrix GeneChip probe level data. Nucleic Acids Res. 2003 Feb 15;31(4):e15.

Loukola A, Eklin K, Laiho P, Salovaara R, Kristo P, Jarvinen H, Mecklin JP, Launonen V, Aaltonen LA. Microsatellite marker analysis in screening for hereditary nonpolyposis colorectal cancer (HNPCC). Cancer Res. 2001 Jun 1;61 (11):4545-9.

Markowitz S, Hines JD, Lutterbaugh J, Myeroff L, Mackay W, Gordon N, Rustum Y, Luna E, Kleinerman J.Mutant K-ras oncogenes in colon cancers Do not predict Patient's chemotherapy response or survival. Clin Cancer Res. 1995 Apr;1 (4):441-5.

Mori Y, Selaru FM, Sato F, Yin J, Simms LA, Xu Y, Olaru A, Deacu E, Wang S, Taylor JM, Young J, Leggett B, Jass JR, Abraham JM, Shibata D, Meltzer SJ. The impact of microsatellite instability on the molecular phenotype of colorectal tumors. Cancer Res. 2003 Aug 1;63(15):4577-82.

Ribic CM, Sargent DJ, Moore MJ, Thibodeau SN, French AJ, Goldberg RM, Hamilton SR, Laurent-Puig P, Gryfe R, Shepherd LE, Tu D, Redston M, Gallinger S. Tumor microsatellite-instability status as a predictor of benefit from fluorouracil-based adjuvant chemotherapy for colon cancer.N Engl J Med. 2003 Jul 17;349(3):247-57.

## Claims

**1.** A method for classification of colon cancer in an individual having contracted colon cancer comprising

i) in a sample from the individual having contracted colon cancer determining the microsatellite status of the tumor and
ii) in a sample from the individual having contracted colon cancer, said sample comprising a plurality of gene expression products the presence and/or amount of which forms a pattern that is indicative of the hereditary or sporadic nature of said cancer,
iii) determining, the presence and/or amount of said plurality of gene expression products forming said pattern, wherein at least one of said gene expression products is selected from the group consisting of

| Gene name | Ref seq | Gene symbol I | SEQ ID NO.: |
|---|---|---|---|
| Homeo box C6 | NM_004503 | HOXC6 | 105 |
| Piwi - like 1 | NM_004764.2 | PIWIL1 | 106 |
| Mut L homolog 1 | NM_00249.2 | MLH1 | 107 |
| Collapsin response mediator protein 1 | NM_001313.2 | CRMP1 | 108 |
| Homeo box B2 | NM_002145.2 | HOXB2 | 109 |
| Pyrroline-5-carboxylate synthetase (glutamate gamma-semialdehyd synthetase) | NM_002860.2 | PYCS/ADH18 A1 | 110 |
| TGFB inducible early growth response | NM_005655.1 | TIEG | 111 |
| Checkpoint with forkhead and ring finger domains ?? | NM_018223.1 | CHFR | 112 |
| Hypothetical protein FLJ13842 | NM_024645.1 | FLJ13842 | 113 |
| Phosphoprotein regulated by mitogenic pathways | NM_025195.1 | C8FW | 114 |

and
iv) obtaining from said pattern an indicator of the hereditary or sporadic nature of said cancer in the individual,
v) classifying said colon cancer from the microsatellite status and said indicator.

**2.** The method of claim 1, wherein the determination of microsatellite status comprises the steps of

i) in a sample from the individual having contracted cancer, said sample comprising a plurality of gene expression products the presence and/or amount of which forms a pattern that is indicative of the microsatellite status of said cancer,
ii) determining the presence and/or amount of said gene expression products forming said pattern,
iii) obtaining an indication of the microsatellite status of said cancer in the individual based on step ii).

**3.** The method of any of the preceding claims, wherein a plurality of gene expression products are analysed using solid support, having binding partners (hybridisation partners) for said plurality of gene expression products forming a pattern.

**4.** The method of any of the preceding claims, wherein a plurality of gene expression products are analysed using binding partners (hybridisation partners) for said plurality of gene expression products forming a pattern.

**5.** The method of claim 1 or 3, wherein at least two of said plurality of gene expression products forming a pattern used to determine said microsatellite status are selected individually from a group of genes indicative of microsatellite status.

**6.** The method of claim 1 or 3, wherein at least two of said plurality of gene expression products forming a pattern used to determine said microsatellite status are selected individually from the group consisting of the genes listed below

| Gene name | Ref seq | Gene symbol | SEQ ID NO.: |
|---|---|---|---|
| chemokine (C-C motif) ligand 5 | NM_002985 | CCL5 | 1 |
| Tryptophanyl-tRNA synthetase | NM_004184 | WARS | 2 |
| Proteasome (prosome, macropain) | NM_006263 | PSME1 | 3 |
| activator subunit 1 (PA28 alpha) | | | |
| Bone marrow stromal cell antigen 2 | NM_004335 | BST2 | 4 |
| ubiquitin-conjugating enzyme E2L 6 | NM_004223 | UBE2L6 | 5 |
| A kinase (PRKA) anchor protein 1 | NM_003488 | AKAP1 | 6 |
| Proteasome (prosome, macropain) | NM_002818 | PSME2 | 7 |
| activator subunit 2 (PA28 beta) carcinoembryonic antigen-related cell | NM_004363 | CEACAM5 | 8 |
| adhesion molecule 5 | | | |
| FERM, RhoGEF (ARHGEF) and | | | 9 |
| pleckstrin domain protein 1 (chondrocyte-derived) | NM_005766 | FARP1 | |
| myosin X | NM_012334 | MYO10 | 10 |
| heterogeneous nuclear ribonucleoprotein L | NM_001533 | HNRPL | |
| Autocrine motility factor receptor | NM_001144 | AMFR | 12 |
| dimethylarginine dimethylaminohydrolase 2 | NM_013974 | DDAH2 | 13 |
| tumor necrosis factor, alpha-induced protein 2 | NM_006291 | TNFAIP2 | 14 |
| mutL homolog 1, colon cancer, | NM_000249 | MLH1 | 15 |
| nonpolyposis type 2 (E. coli) | | | |
| thymidylate synthetase | NM_001071 | TYMS | 16 |
| intercellular adhesion molecule 1 (CD54), human rhinovirus receptor | NM_000201 | ICAM1 | 17 |
| general transcription factor IIA, 2, 12kDa | NM_004492 | GTF2A2 | 18 |
| Rho-associated, coiled-coil containing | NM_004850 | ROCK2 | 19 |
| protein kinase 2 | | | |
| ATP binding protein associated with cell | NM_005783 | TXNDC9 | 20 |
| differentiation | | | |
| NCK adaptor protein 2 | NM_003581 | NCK2 | 21 |
| phytanoyl-CoA hydroxylase (Refsum disease) | NM_006214 | PHYH | 22 |
| metastais-associated gene family, | | | 23 |
| member 2 | NM_004739 | MTA2 | |
| amiloride binding protein 1 (amine | NM_001091 | ABP1 | 24 |
| oxidase (copper-containing)) | | | |
| Biliverdin reductase A | NM_000712 | BLVRA | 25 |
| phospholipase C, beta 4 | NM_000933 | PLCB4 | 26 |
| chemokine (C-X-C motif) ligand 9 | NM_002416 | CXCL9 | 27 |
| purine-rich element binding protein A | NM_005859 | PURA | 28 |
| quinolinate phosphoribosyltransferase | NM_014298 | QPRT | 29 |
| (nicotinate-nucleotide pyrophosphorylase | | | |
| (carboxylating)) | | | |

(continued)

| Gene name | Ref seq | Gene symbol | SEQ ID NO.: |
|---|---|---|---|
| retinoic acid receptor responder (tazarotene induced) 3 | NM_004585 | RARRES3 | 30 |
| chemokine (C-C motif) ligand 4 | NM_002984 | CCL4 | 31 |
| forkhead box O3A | NM_001455 | FOXO3A | 32 |
| interferon, alpha-inducible protein (clone | NM_002038 | G1P3 | 34 |
| IFI-6-16) | NM_022873 | | 123 |
| chemokine (C-X-C motif) ligand 10 | NM_001565 | CXCL10 | 35 |
| | NM_005950 | MT1G | 36 |
| metallothionein 1G | NM_005950 | | |
| tumor necrosis factor receptor | NM_000043 | TNFRSF6 | 37 |
| superfamily, | NM_152877 | | 133 |
| member 6 | NM_152876 | | 132 |
| | NM_152875 | | 134 |
| | NM_152872 | | 130 |
| | NM_152873 | | 33 |
| | NM_152871 | | 129 |
| | NM_152874 | | 131 |
| endothelial cell growth factor 1 (platelet-derived) | NM_001953 | ECGF1 | 38 |
| SCO cytochrome oxidase deficient homolog 2 (yeast) | NM_005138 | SCO2 | 39 |
| chemokine (C-X-C motif) ligand 13 (B-cell chemoattractant) | NM_006419 | CXCL13 | 40 |
| Granulysin | NM_006433 | GNLY | 41 |
| CD2 antigen (p50), sheep red blood cell | | | 42 |
| receptor | NM_001767 | CD2 | |
| splicing factor, arginine/serine-rich 6 | NM 006275 | SFRS6 | 43 |
| Teratocarcinoma-derived growth factor 1 | NM_003212 | TDGF1 | 44 |
| metallothionein 1H | NM_005951 | MT1H | 45 |
| cytochrome P450, family 2, subfamily B, polypeptide 6 | NM_000767 | CYP2B6 | 46 |
| tumor necrosis factor (ligand) superfamily, member 9 | NM_003811 | TNFSF9 | 47 |
| | NM_006047 | RBM12 | 48 |
| RNA binding motif protein 12 | NM_006047 | | |
| heat shock 105kDa/110kDa protein 1 | NM_006644 | HSPH1 | 49 |
| staufen, RNA binding protein (Drosophila) | NM_004602 | STAU | 50 |
| | NM_017452 | | 125 |
| | NM_017453 | | 126 |
| lymphocyte antigen 6 complex, locus G6D | NM_021246 | LY6G6D | 51 |
| calcium binding protein P22 | NM_007236 | CHP | 52 |
| CDC14 cell division cycle 14 homolog B | NM_003671 | CDC14B | 53 |
| (S. cerevisiae) | NM_033331 | | 115 |
| Epiplakin 1 | XM_372063 | EPPK1 | 54 |
| metallothionein 1X | NM_005952 | MT1X | 55 |
| Transforming growth factor, beta receptor | NM 003242 | TGFBR2 | 56 |

(continued)

| Gene name | Ref seq | Gene symbol | SEQ ID NO.: |
|---|---|---|---|
| II (70/80kDa) | | | |
| protein kinase C binding protein 1 | NM_012408<br>NM_183047 | PRKCBP1 | 57<br>124 |
| Transmembrane 4 superfamily member 6 | NM_003270 | TM4SF6 | 58 |
| pleckstrin homology domain containing, family B (evectins) member 1 | NM_021200 | PLEKHB1 | 59 |
| apolipoprotein L, 1 | NM_003661<br>NM_145343 | APOL1 | 60<br>120 |
| Indoleamine-pyrrole 2,3 dioxygenase | NM_002164 | INDO | 61 |
| forkhead box A2 | NM_021784 | FOXA2 | 62 |
| granzyme H (cathepsin G-like 2, protein h-CCPX) | NM_033423 | GZMH | 63 |
| baculoviral IAP repeat-containing 3 | NM_001165 | BIRC3 | 64 |
| Homo sapiens metallothionein 1H-like protein | | AF333388 (Hs 382039) | 135 |
| KIAA0182 protein | NM_014615 | KIAA0182 | 117 |
| G protein-coupled receptor 56 | NM_005682<br>NM_201524 | GPR56 | 65<br>116 |
| metallothionein 2A | NM_005953 | MT2A | 66 |
| F-box only protein 21 | NM_015002 | FBXO21 | 67 |
| erythrocyte membrane protein band 4.1-like 1 | NM_012156,<br>NM_012156 | EPB41L1 | 68 |
| hypothetical protein MGC21416 | NM_173834 | MGC21416 | 69 |
| protein O-fucosyltransferase 1 | NM_015352,<br>NM_015352 | POFUT1 | 70 |
| metallothionein 1E (functional) | NM_175617 | MT1 E | 71 |
| troponin T1, skeletal, slow | NM_003283 | TNNT1 | 72 |
| chimerin (chimaerin) 2 | NM_004067 | CHN2 | 73 |
| heterogeneous nuclear ribonucleoprotein H1 (H) | NM_005520 | HNRPH1 | 74 |
| ATP synthase, H+ transporting, mitochondrial F1 complex, alpha subunit, isoform 1, cardiac muscle | NM_004046 | ATP5A1 | 75 |
| eukaryotic translation initiation factor 5A | NM_001970 | EIF5A | 76 |
| perforin 1 (pore forming protein) | NM_005041 | PRF1 | 77 |
| OGT(O-Glc-NAc transferase)-interacting protein 106 KDa | NM_014965 | OIP106 | 78 |
| DEAD (Asp-Glu-Ala-Asp) box polypeptide 27 | NM_017895 | DDX27 | 79 |
| vacuolar protein sorting 35 (yeast) | NM_018206 | VPS35 | 80 |
| tripartite motif-containing 44 | NM_017583 | TRIM44 | 81 |

(continued)

| Gene name | Ref seq | Gene symbol | SEQ ID NO.: |
|---|---|---|---|
| transmembrane, prostate androgen induced | NM_020182 | TMEPAI | 82 |
| RNA | NM_199169 | | 127 |
| | NM_199170 | | 128 |
| dynein, cytoplasmic, light polypeptide 2A | NM_014183 | DNCL2A | 83 |
| | NM_177953 | | 122 |
| leucine aminopeptidase 3 | NM_015907 | LAP3 | 84 |
| Chromosome 20 open reading frame 35 | NM_018478 | C20orf35 | 85 |
| | NM_033542 | | 118 |
| solute carrier family 38, member 1 | NM_030674 | SLC38A1 | 86 |
| CGI-85 protein | NM_016028 | CGI-85 | 87 |
| death associated transcription factor 1 | N_022105, | DATF1 | 88 |
| | NM_080796 | | 121 |
| hepatocellular carcinoma-associated antigen 112 | NM_018487 | HCA112 | 89 |
| sestrin 1 | NM_014454 | SESN1 | 90 |
| hypothetical protein FLJ20315 | NM_017763 | FLJ20315 | 91 |
| hypothetical protein FLJ20647 | NM_017918 | FLJ20647 | 92 |
| membrane protein expressed in epithelial-like lung adenocarcinoma | NM_024792 | CT120 | 93 |
| DEAD/H (Asp-Glu-Ala-Asp/His) box | | | 94 |
| polypeptide | NM_014314 | RIG-I | |
| keratin 23 (histone deacetylase inducible) | NM_015515, | KRT23 | 95 |
| UDP-N-acetyl-alpha-D-galactosamine:polypeptide N-acetylgalactosaminyltransferase 6 (GalNAc-T6) | NM_007210 | GALNT6 | 96 |
| aryl hydrocarbon receptor nuclear translocator-like 2 | NM_020183 | ARNTL2 | 97 |
| apobec-1 complementation factor | NM_014576, | ACF | 98 |
| | NM_138932 | | 119 |
| hypothetical protein FLJ20232 | NM_019008 | FLJ20232 | 99 |
| apolipoprotein L, 2 | NM_030882, | APOL2 | 100 |
| | NM_145343 | | 120 |
| mitochondrial solute carrier protein | NM_016612 | MSCP | 101 |
| hypothetical protein FLJ20618 | NM_017903 | FLJ20618 | 102 |
| SET translocation (myeloid leukaemia-associated) | NM_003011.1 | SET | 103 |
| ATPase, class II, type 9a | Xm_030577.9 | ATP9a | 104 |

**7.** The method of claims 1 or 3, wherein at least two of said plurality of gene expression products forming a pattern used to determine said microsatellite status are selected individually from the group consisting of the genes listed below

| Gene name | Ref seq | Gene symbol | SEQ ID NO.: |
|---|---|---|---|
| heterogeneous nuclear ribonucleoprotein L | NM_001533 | HNRPL | 11 |

(continued)

| Gene name | Ref seq | Gene symbol | SEQ ID NO.: |
|---|---|---|---|
| metastais-associated gene family, member 2 | NM_004739 | MTA2 | 23 |
| chemokine (C-X-C motif) ligand 10 | NM_001565 | CXCL10 | 35 |
| splicing factor, arginine/serine-rich 6 | NM_006275 | SFRS6 | 43 |
| protein kinase C binding protein 1 | NM_012408 | PRKCBP1 | 57 |
| | NM_183047 | | 124 |
| hepatocellular carcinoma-associated antigen 112 | NM_018487 | HCA112 | 89 |
| hypothetical protein FLJ20618 | NM_017903 | FLJ20618 | 102 |
| SET translocation (myeloid leukaemia-associated) | NM_003011.1 | SET | 103 |
| ATPase, class II, type 9a | Xm_030577.9 | ATP9a | 104 |

**8.** The method of claims 1 or 3, wherein

i) at least one of said plurality of gene expression products forming a pattern used to determine said microsatellite status is selected from the group of genes consisting of

| Gene name | Ref seq | Gene symbol | SEQ ID NO.: |
|---|---|---|---|
| heterogeneous nuclear ribonucleoprotein L | NM_001533 | HNRPL | 11 |
| metastais-associated gene family, member 2 | NM_004739 | MTA2 | 23 |
| Chemokine (C-X-C motif) ligand 10 | NM_001565 | CXCL10 | 35 |
| splicing factor, arginine/serine-rich 6 | NM_006275 | SFRS6 | 43 |

and
ii) at least one of said plurality of gene expression products forming a pattern used to determine said microsatellite status is selected from the group of genes consisting of

| Gene name | Ref seq | Gene symbol | SEQ ID NO.: |
|---|---|---|---|
| protein kinase C binding protein 1 | NM_012408 | PRKCBP1 | 57 |
| | NM_183047 | | 124 |
| hepatocellular carcinoma-associated antigen 112 | NM_018487 | HCA112 | 89 |
| hypothetical protein FLJ20618 | NM 017903 | FLJ20618 | 102 |
| SET translocation (myeloid leukaemia-associated) | NM 003011.1 | SET | 103 |
| ATPase, class II, type 9a | Xm 030577.9 | ATP9a | 104 |

**9.** The method of claims 1 or 3, wherein

i) at least one of said plurality of gene expression products forming a pattern used to determine said microsatellite status is selected from the group of genes that are down regulated in MSS colon cancers compared to MSI colon cancers consisting of

| Gene name | Ref seq | Gene symbol | SEQ ID NO.: |
|---|---|---|---|
| heterogeneous nuclear ribonucleoprotein L | NM_001533 | HNRPL | 11 |
| metastais-associated gene family, member 2 | NM_004739 | MTA2 | 23 |
| chemokine (C-X-C motif) ligand 10 | NM_001565 | CXCL10 | 35 |
| Splicing factor, arginine/serine-rich 6 | NM_006275 | SFRS6 | 43 |

and
ii) at least one of said plurality of gene expression products forming a pattern used to determine said microsatellite status is selected from the group of genes that are up regulated in MSS colon cancers compared to MSI colon cancers consisting of

| Gene name | Ref seq | Gene symbol | SEQ ID NO.: |
|---|---|---|---|
| protein kinase C binding protein 1 | NM_012408 | PRKCBP1 | 57 |
| | NM_183047 | | 124 |
| hepatocellular carcinoma-associated antigen 112 | NM_018487 | HCA112 | 89 |
| hypothetical protein FLJ20618 | NM_017903 | FLJ20618 | 102 |
| SET translocation (myeloid leukaemia-associated) | NM_003011.1 | SET | 103 |
| ATPase, class II, type 9a | Xm_030577.9 | ATP9a | 104 |

**10.** The method of claim 9, wherein the difference in the level of the gene expression products forming a pattern is at least one-fold.

**11.** The method of claim 9, wherein the difference of the level of the gene expression products forming a pattern is at least 1.5 fold.

**12.** The method of claim 1 or 3, wherein at least two of said plurality of gene expression products forming a pattern used to determine said hereditary or sporadic nature of colon cancer are the two genes as listed below

| Gene name | Ref seq | Gene symbol l | SEQ ID NO.: |
|---|---|---|---|
| Piwi - like 1 | NM_004764.2 | PIWIL1 | 106 |
| Mut L homolog 1 | NM_00249.2 | MLH1 | 107 |

**13.** The method according to claims 1 or 3, wherein the microsatellite status in an individual having contracted colon cancer is microsatellite instable.

**14.** The method according to any of the preceding claims, wherein said colon cancer is of Duke's B or Duke's C stage.

**15.** The method according to any of the preceding claims, wherein said colon cancer is an adenocarcinoma, a carcinoma, a teratoma, a sarcoma, and/or a lymphoma.

**16.** The method according to any of the preceding claims, wherein the sample is a biopsy of the tissue.

**17.** The method according to any of the preceding claims, wherein the expression level is determined by determining mRNA of the sample.

**18.** The method according to any of the preceding claims, wherein the expression level is determined by determining expression products, such as peptides and/or protein in the sample.

**19.** The method according to any of the preceding claims, wherein the microsatellite status of the colon cancer in an individual has been determined prior to the determination of the presence and/or amount of gene expression products

**20.** The method according to any of the preceding claims, wherein the sporadic or hereditary nature of a colon cancer has been determined prior to the determination of the presence and/or amount of gene expression products.

**21.** A method for classification of colon cancer in an individual having contracted colon cancer, wherein the hereditary or sporadic nature of said cancer is determined by a method comprising the steps of

i) in a sample from the individual having contracted colon cancer, said sample comprising a plurality of gene expression products the presence and/or amount of which forms a pattern that is indicative of the hereditary or sporadic nature of said cancer, determining the presence and/or amount of said gene expression products forming said pattern, wherein at least one of said gene expression products is selected from the group consisting of:

| Gene name | Ref seq | Gene symbol I | SEQ ID NO.: |
|---|---|---|---|
| Homeo box C6 | NM_004503 | HOXC6 | 105 |
| Piwi - like 1 | NM_004764.2 | PIWIL1 | 106 |
| Mut L homolog 1 | NM_00249.2 | MLH1 | 107 |
| Collapsin response mediator protein 1 | NM_001313.2 | CRMP1 | 108 |
| Homeo box B2 | NM_002145.2 | HOXB2 | 109 |
| Pyrroline-5-carboxylate synthetase (glutamate gamma-semialdehyd synthetase) | NM_002860.2 | PYCS/ADH18 A1 | 110 |
| TGFB inducible early growth response | NM_005655.1 | TIEG | 111 |
| Checkpoint with forkhead and ring finger domains ?? | NM_018223.1 | CHFR | 112 |
| Hypothetical protein FLJ13842 | NM_024645.1 | FLJ13842 | 113 |
| Phosphoprotein regulated by mitogenic pathways | NM_025195.1 | C8FW | 114 |

and
ii) obtaining from said patttern an indicator of the hereditary or sporadic nature of said cancer in the individual.

**22.** The method according to claim 21, wherein the microsatellite status of said cancer is determined simultaneously or sequentially therewith.

**Patentansprüche**

**1.** Verfahren zum Klassifizieren eines Kolonkarzinoms in einem Individuum mit stenosierendem Kolonkarzinom, das umfasst

(i) Bestimmen in einer Probe des Individuums mit stenosierendem Kolonkarzinom den Mikrosatellitenstatus des Tumors, und
(ii) Bestimmen in einer Probe des Individuums mit stenosierendem Kolonkarzinom, wobei die Probe mehrere Genexpressionsprodukte umfasst deren Anwesenheit und/oder Menge ein Muster bildet, das die erbliche oder die sporadische Beschaffenheit des Krebses anzeigt, der Anwesenheit und/oder der Menge der mehreren musterbildenden Genexpressionsprodukte bestimmt, wobei mindestens eines der Genexpressionsprodukte ausgewählt ist aus der Gruppe bestehend aus

| **Genname** | **Ref.-seq.** | **Gensymbol** | **SEQ ID Nr:** |
|---|---|---|---|
| Homeobox C6 | NM_004503 | HOXC6 | 105 |
| Piwi-like 1 | NM_004764.2 | PIWIL1 | 106 |
| Mut L Homolog 1 | NM_00249.2 | MLH1 | 107 |
| Collapsin Antwort Mediator Protein 1 | NM_001313.2 | CRMP1 | 108 |
| Homeobox B2 | NM_002145.2 | HOXB2 | 109 |
| Pyrrolin-5-Carboxylat-Synthetase (Glutamat gamma-Semialdehyd-Synthetase) | NM_002860.2 | PYCS/ADH 18 A1 | 110 |
| TGFB induzierbare frühe Wachstumsantwort | NM_005655.1 | TIEG | 111 |
| Checkpoint mit Forkhead und Ringfinger Domänen?? | NM_018223.1 | CHFR | 112 |
| Hypothetisches Protein FLJ13842 | NM_024645.1 | FLJ13842 | 113 |
| Phosphoprotein reguliert durch Mitogenwege | NM_025195.1 | C8FW | 114 |

und
iii) Erhalten eines Hinweises aus dem Muster auf die erbliche oder sporadische Beschaffenheit des Karzinoms in dem Individuum,

iv) Klassifizieren des Kolonkarzinoms aus dem Mikrosatellitenstatus und dem Hinweis.

**2.** Verfahren nach Anspruch 1, wobei die Bestimmung des Mikrosatellitenstatus die Schritte umfasst

i) in einer Probe des Individuums mit stenosierendem Karzinom, wobei die Probe mehrere musterbildende Genexpressionsprodukte umfasst, deren Anwesenheit und/oder Menge davon ein Muster bildet, das auf den Mirkosatellitenstatus des Krebses hinweist,

ii) Bestimmen der Anwesenheit und/oder Menge der Genexpressionsprodukte, die das Muster bilden,

iii) Erhalten eines Hinweises über den Mikrosatellitenstatus des Karzinoms in dem Individuum basierend auf Schritt ii).

**3.** Verfahren nach einem der vorstehenden Ansprüche, wobei mehrere Genexpressionsprodukte unter Verwendung eines soliden Trägers analysiert werden, der Bindungspartner (Hybridisierungspartner) für die mehreren musterbildenden Genexpressionsprodukte aufweist.

**4.** Verfahren nach einem der vorstehenden Ansprüche, wobei mehrere Genexpressionsprodukte unter Verwendung von Bindungspartnern (Hybridisierungspartnern) für die mehreren musterbildenden Genexpressionsprodukte analysiert werden.

**5.** Verfahren nach einem der Ansprüche 1 oder 3, wobei mindestens zwei der mehreren musterbildenden Genexpressionsprodukte, die verwendet werden, um den Mikrosatellitenstatus zu bestimmen, einzeln aus einer Gruppe von Genen ausgewählt sind, die auf den Mikrosatellitenstatus hinweisen.

**6.** Verfahren nach einem der Ansprüche 1 oder 3, wobei mindestens zwei der mehreren musterbildenden Genexpressionsprodukte, die verwendet werden, um den Mikrosatellitenstatus zu bestimmen, einzeln aus der nachstehend aufgelisteten Gruppe von Genen ausgewählt sind

| Genname | Ref.-seq. | Gensymbol | SEQ ID Nr: |
|---|---|---|---|
| Chemokin (C-C Motiv) Ligand 5 | NM_002985 | CCL5 | 1 |
| Tryptophanyl-tRNA Synthetase | NM_004184 | WARS | 2 |
| Proteasom (Prosom, Macropain) Aktivator Untereinheit (PA28 alpha) | NM_006263 | PSME1 | 3 |
| Knochenmarks-Stromazellantigen 2 | NM_004335 | BST2 | 4 |
| Ubiquitin-konjugierendes Enzym E2L 6 | NM_004223 | UBE2L6 | 5 |
| A Kinase PRKA Ankerprotein 1 | NM_003488 | AKAP1 | 6 |
| Proteasom (Prosom, Macropain) Aktivatoruntereinheit 2 (PA28 beta) | NM_002818 | PSME2 | 7 |
| Karzinoembryonales Antigen bezogenes Zelladhäsions-Molekül 5 | NM_004363 | CEACAM5 | 8 |
| FERM, RhoGEF (ARHGEF) und Pleckstrin-Domänenprotein 1 (Chondrozyten-abgeleitet) | NM_005766 | FARP1 | 9 |
| Myosin X | NM_012334 | MYO10 | 10 |
| Heterogenes nukleäres Ribonukleoprotein L | NM_001533 | HNRPL | 11 |
| Autokriner Motilitäts-Faktor-Rezeptor | NM_001144 | AMFR | 12 |
| Dimethylarginin-Dimethylamino-Hydrolase 2 | NM_013974 | DDAH2 | 13 |
| Tumor-Nekrose-Faktor, alpha induziertes Protein 2 | NM_006291 | TNFAIP2 | 14 |
| mutL Homolog 1, Kolonkrebs, nicht Polypose Typ 2 (E. coli) | NM_000249 | MHL1 | 15 |
| Thymidylat-Synthetase | NM_001071 | TYMS | 16 |
| Interzelluläres Adhäsions-Molekül 1 (CD54) | NM_000201 | ICAM1 | 17 |

(fortgesetzt)

| Genname | Ref.-seq. | Gensymbol | SEQ ID Nr: |
|---|---|---|---|
| Humanes Rhinovirus-Rezeptor genereller Transkriptionsfaktor IIA, 2, 12kDa | NM_004492 | GTF2A2 | 18 |
| Rho assoziiertes, superspiralisierte α-Helix beinhaltende Protein Kinase 2 | NM_004850 | ROCK2 | 19 |
| ATP Bindungsprotein assoziiert mit Zelldifferenzierung | NM_005783 | TXNDC9 | 20 |
| NCK Adapter Protein 2 | NM_003581 | NCK2 | 21 |
| Phytanoyl-CoA Hydroxylase (Refsum-Krankheit) | NM_006214 | PHYH | 22 |
| Metastasen assoziierte Gen-Familie, Mitglied 2 | NM_004739 | MTA2 | 23 |
| Amilorid Bindungsprotein 1 (Amin- Oxidase (Kupfer enthaltend)) | NM_001091 | ABP1 | 24 |
| Biliverdin Reduktase A | NM_000712 | BLVRA | 25 |
| Phospholipase C, beta 4 | NM_000933 | PLCB4 | 26 |
| Chemokin (C-X-C Motiv) Ligand 9 | NM_002416 | CXCL9 | 27 |
| Purin reiches Element Bindungsprotein A | NM_005859 | PURA | 28 |
| Quinolinat-Phosphoribosyltransferase (Nikotinat-Nukleotid-Pyrophosphorylase (carboxylierend)) | NM_014298 | QPRT | 29 |
| Retinolsäure-Rezeptor Responder (Tazaoroten induziert) 3 | NM_004585 | RARRES3 | 30 |
| Chemokin (C-C Motiv) Ligand 4 | NM_002984 | CCL4 | 31 |
| Forkhead Box O3A | NM_001455 | FOXO3A | 32 |
| Interferon, alpha induzierbares Protein | NM_002038 | G1P3 | 34 |
| (Klon IFI-6-16) | NM_022873 | | 123 |
| Chemokin (C-X-C Motiv) Ligand 10 | NM_001565 | CXCL10 | 35 |
| Metallothionein 1G | NM_005950 | MT1G | 36 |
| Tumor Nekrose Faktor Rezeptor | NM_000043 | TNFRSF6 | 37 |
| Superfamilie, | NM_152877 | | 133 |
| Mitglied 6 | NM_152876 | | 132 |
| | NM_152875 | | 134 |
| | NM_152872 | | 130 |
| | NM_152873 | | 33 |
| | NM_152871 | | 129 |
| | NM_152874 | | 131 |
| Endothelzell-Wachstums-Faktor 1 (Thrombozyten abgeleitet) | NM_001953 | ECGF1 | 38 |
| SCO Cytochrom-Oxidase defizientes Homolog 2 (Hefe) | NM_005138 | SCO2 | 39 |
| Chemokin (C-X-C Motiv) Ligand 13 (B-Zell chemotaktisch) | NM_006419 | CXCL13 | 40 |
| Granulysin | NM_006433 | GNLY | 41 |
| CD2 Antigen (p50), Schafserythrozyten-Rezeptor | NM_001767 | CD2 | 42 |
| Splicing Faktor, Arginin-/Serin-reich 6 | NM_006275 | SFRS6 | 43 |
| Teratokarzinom abgeleiteter Wachstums-Faktor 1 | NM_003212 | TDGF1 | 44 |
| Metallothinein 1H | NM_005951 | MT1H | 45 |
| Cytochrom P450, Familie 2, Unterfamilie B, Polypeptid 6 | NM_000767 | CYP2B6 | 46 |
| Tumor Nekrose Faktor (Ligand) Superfamilie, Mitglied 9 | NM_003811 | TNFSF9 | 47 |
| RNA Bindungsmotiv Protein 12 | NM_006047 | RBM12 | 48 |
| Hitzeschock 105kDa/110kDa Protein 1 | NM_006644 | HSPH1 | 49 |
| Staufen, RNA Bindungsprotein (Drosophila) | NM_004602 | STAU | 50 |
| | NM_017452 | | 125 |

(fortgesetzt)

| Genname | Ref.-seq. | Gensymbol | SEQ ID Nr: |
|---|---|---|---|
| | NM_017453 | | 126 |
| Lymphozyten-Antigen 6 Komplex, Lokus G6D | NM_021246 | LY6G6D | 51 |
| Calcium Bindungsprotein P22 | NM_007236 | CHP | 52 |
| CDC14 Zellteilungszyklus 14 Homolog B | NM_003671 | CDC14B | 53 |
| (*S. cerevisiae*) | NM_033331 | | 115 |
| Epiplakin 1 | XM_372063 | EPPK1 | 54 |
| Metallothionein 1X | NM_005952 | MT1X | 55 |
| Transformierender Wachstums-Faktor, beta Rezeptor II (70/80kDa) | NM_003242 | TGFBR2 | 56 |
| Protein-Kinase C Bindungsprotein 1 | NM_012408 | PRKCBP1 | 57 |
| | NM_183047 | | 124 |
| Transmembran 4 Superfamilienmitglied 6 | NM_003270 | TM4SF6 | 58 |
| Pleckstrin Homolgiedomäne enthaltende, Familie B (Evectine) Mitglied 1 | NM_021200 | PLEKHB1 | 59 |
| Apolipoprotein L,1 | NM_003661 | APOL1 | 60 |
| | NM_145343 | | 120 |
| Indolamin-pyrrol 2,3 Dioxygenase | NM_002164 | INDO | 61 |
| Forkhead Box A2 | NM_021784 | FOXA2 | 62 |
| Granzym H (Cathepsin G-ähnlich 2, Protein h-CCPX) | NM_033423 | GZMH | 63 |
| Baculovirale IAP Wiederholung-enthaltend 3 | NM_001165 | BIRC3 | 64 |
| Homo sapiens Metallothionein 1H-like Protein | AF333388 (Hs 382039) | | 135 |
| KIAA0182 Protein | NM_014615 | KIAA0182 | 117 |
| G-Protein gekoppelter Rezeptor 56 | NM_005682 | GPR56 | 65 |
| | NM_201524 | | 116 |
| Metallothionein 2A | NM_005953 | MT2A | 66 |
| F-Box only Protein 21 | NM_015002 | FBXO21 | 67 |
| Erythrozytenmembranprotein Band 4.1-like 1 | NM_012156 | EPB41L1 | 68 |
| Hypothetisches Protein MGC21416 | NM_173834 | MGC21416 | 69 |
| Protein O-Fucosyltransferase 1 | NM_015352 | POFUT1 | 70 |
| Metallothionein 1E (funktional) | NM_175617 | MT1E | 71 |
| Troponin T1, skelttartig, langsam | NM_003283 | TNNT1 | 72 |
| Chimerin (Chimaerin) 2 | NM_004067 | CHN2 | 73 |
| Heterogenes Kernribonukleoprotein H1 (H) | NM_005520 | HNRPH1 | 74 |
| ATP-Synthase, H+ transportirender, mitochon-drialer F1 Komplex, alpha Untereinheit, Isoform 1, Herzmuskel | NM_004046 | ATP5A1 | 75 |
| Eukaryotischer Translations-Initiations-Faktor5A | NM_001970 | EIF5A | 76 |
| Perforin 1 (Poren formendes Protein) | NM_005041 | PRF1 | 77 |
| OGT (O-Glc-NAc Transferase) interagierendes Protein 106 kDa | NM_014965 | OIP106 | 78 |
| DEAD (Asp-Glu-Ala-Asp) Box Polypeptid 27 | NM_017895 | DDX27 | 79 |
| vakuoläre Proteinsortierung 35 (Hefe) | NM_018206 | VPS35 | 80 |
| Dreier-Motiv enthaltendes 44 | NM_017583 | TRIM44 | 81 |
| Transmembran, Prostataandrogen induzierte | NM_020182 | TMEPAI | 82 |
| RNA | NM_199169 | | 127 |
| | NM_199170 | | 128 |
| Dynein, zytoplasmatisch, leichtes Polypeptid 2A | NM_014183 | DNCL2A | 83 |
| | NM_177953 | | 122 |
| Leucin Aminopeptidase 3 | NM_015907 | LAP3 | 84 |
| Chromosom 20 offener Leserahmen 35 | NM_018478 | C20orf35 | 85 |

(fortgesetzt)

| Genname | Ref.-seq. | Gensymbol | SEQ ID Nr: |
|---|---|---|---|
| | NM_033542 | | 118 |
| Solute Carrier Familie 38, Mitglied 1 | NM_030674 | SLC38A1 | 86 |
| CGI-85 Protein | NM_016028 | CGI-85 | 87 |
| Death assoziierter Transkriptionsfaktor 1 | NM_022105 | DATF1 | 88 |
| | NM_080796 | | 121 |
| Leberzellkarzinom assoziiertes Antigen 112 | NM_018487 | HCA112 | 89 |
| Sestrin 1 | NM_014454 | SESN1 | 90 |
| Hypothetisches Protein FLJ20315 | NM_017763 | FLJ20315 | 91 |
| Hypothetisches Protein FLJ20647 | NM_017918 | FLJ20647 | 92 |
| Membranprotein exprimiert in epithelähnlichem Lungenadenokarzinom | NM_024792 | CT120 | 93 |
| DEAD/H (Asp-Glu-Ala-Asp/His) Box Polypeptid | NM_014314 | RIG-1 | 94 |
| Keratin 23 (Histon-Deacetylase induzierbar) | NM_015515 | KRT23 | 95 |
| UDP-N-Acetyl alpha-D-galactosamin: Polypeptid N-Acetylgalactosaminyltransferase 6 (GalNAc-T6) | NM_007210 | GALNT6 | 96 |
| Aryl-Kohlenwasserstoff Rezeptor Kern-Translokator-like 2 | NM_020183 | ARNTL2 | 97 |
| apobec-1 Komplementierungsfaktor | NM_014576 | ACF | 98 |
| | NM_138932 | | 119 |
| Hypothetisches Protein FLJ20232 | NM_019008 | FLJ20232 | 99 |
| Apolipoprotein L, 2 | NM_030882 | APOL2 | 100 |
| | NM_145343 | | 120 |
| Mitochondriales Solute Carrier Protein | NM_016612 | MSCP | 101 |
| Hypothetisches Protein FLJ20618 | NM_017903 | FLJ20618 | 102 |
| SET Translokation (myeloische Leukämie assoziiert) | NM_003011 | SET | 103 |
| ATPase, Klasse II Typ 9a | Xm_030577.9 | ATP9a | 104 |

**7.** Verfahren nach einem der Ansprüche 1 oder 3, wobei mindestens zwei der mehreren musterbildenden Genexpressionsprodukte, die verwendet werden, um den Mikrosatellitenstatus zu bestimmen, einzeln aus der Gruppe ausgewählt werden bestehend aus den nachfolgend aufgelisteten Genen

| Genname | Ref.-seq. | Gensymbol | SEQ ID Nr: |
|---|---|---|---|
| Heterogenes nukleäres Ribonukleoprotein L | NM_001533 | HNRPL | 11 |
| Metastasen assoziierte Gen-Familie, Mitglied 2 | NM_004739 | MTA2 | 23 |
| Chemokin (C-X-C Motiv) Ligand 10 | NM_001565 | CXCL10 | 35 |
| Splicing Faktor, Arginin-/Serin-reich 6 | NM_006275 | SFRS6 | 43 |
| Protein Kinase C bindendes Protein 1 | NM_012408 | PRKCBP1 | 57 |
| | NM_183047 | | 124 |
| Leberzellkarzinom assoziiertes Antigen 112 | NM_018487 | HCA112 | 89 |
| Hypothetisches Protein FLJ20618 | NM_017903 | FLJ20618 | 102 |
| SET Translokation (myeloische Leukämie assoziiert) | NM_003011.1 | SET | 103 |
| ATPase, Klasse II Typ 9a | Xm_030577.9 | ATP9a | 104 |

**8.** Verfahren nach Ansprüchen 1 oder 3, wobei

i) mindestens eines der mehreren musterbildenden Genexpressionsprodukte, die zum Bestimmen des Mikrosatellitenstatus verwendet werden, ausgewählt ist aus der Gruppe bestehend aus den Genen

| Genname | Ref.-seq. | Gensymbol | SEQ ID Nr: |
|---|---|---|---|
| Heterogenes nukleäres Ribonukleoprotein L | NM_001533 | HNRPL | 11 |

(fortgesetzt)

| Genname | Ref.-seq. | Gensymbol | SEQ ID Nr: |
|---|---|---|---|
| Metastasen assoziierte Gen-Familie, Mitglied 2 | NM_004739 | MTA2 | 23 |
| Chemokin (C-X-C Motiv) Ligand 10 | NM_001565 | CXCL10 | 35 |
| Splicing Faktor, Arginin-/Serin-reich 6 | NM_006275 | SFRS6 | 43 |

und
ii) mindestens eines der mehreren musterbildenden Genexpressionsprodukte, die zum Bestimmen des Mikrosatellitenstatus verwendet werden, ausgewählt ist aus der Gruppe bestehend aus den Genen

| Genname | Ref.-seq. | Gensymbol | SEQ ID Nr: |
|---|---|---|---|
| Protein Kinase C bindendes Protein 1 | NM_012408 | PRKCBP1 | 57 |
| | NM_183047 | | 124 |
| Leberzellkarzinom assoziiertes Antigen 112 | NM_018487 | HCA112 | 89 |
| Hypothetisches Protein FLJ20618 | NM_017903 | FLJ20618 | 102 |
| SET Translokation (myeloische Leukämie assoziiert) | NM_003011.1 | SET | 103 |
| ATPase, Klasse II Typ 9a | Xm_030577.9 | ATP9a | 104 |

**9.** Verfahren nach einem der Ansprüche 1 oder 3, wobei

i) mindestens eines der mehreren musterbildenden Genexpressionsprodukte, die zum Bestimmen des Mikrosatellitenstatus verwendet werden, aus der Gruppe von Genen ausgewählt ist, die verglichen zu MSI Kolonkarzinomen in MSS Kolonkarzinomen herunterreguliert sind

| Genname | Ref.-seq. | Gensymbol | SEQ ID Nr: |
|---|---|---|---|
| Heterogenes nukleäres Ribonukleoprotein L | NM_001533 | HNRPL | 11 |
| Metastasen assoziierte Gen-Familie, Mitglied 2 | NM_004739 | MTA2 | 23 |
| Chemokin (C-X-C Motiv) Ligand 10 | NM_001565 | CXCL10 | 35 |
| Splicing Faktor, Arginin-/Serin-reich 6 | NM_006275 | SFRS6 | 43 |

und
ii) mindestens eines der mehreren musterbildenden Genexpressionsprodukte, die zum Bestimmen des Mikrosatellitenstatus verwendet werden, aus der Gruppe ausgewählt ist, die verglichen zu MSI Kolonkarzinomen in MSS Kolonkarzinomen hochreguliert sind,

| Genname | Ref.-seq. | Gensymbol | SEQ ID Nr: |
|---|---|---|---|
| Protein Kinase C bindendes Protein 1 | NM_012408 | PRKCBP1 | 57 |
| | NM_183047 | | 124 |
| Leberzellkarzinom assoziiertes Antigen 112 | NM_018487 | HCA112 | 89 |
| Hypothetisches Protein FLJ20618 | NM_017903 | FLJ20618 | 102 |
| SET Translokation (myeloische Leukämie assoziiert) | NM_003011.1 | SET | 103 |
| ATPase, Klasse II Typ 9a | Xm_030577.9 | ATP9a | 104 |

**10.** Verfahren nach Anspruch 9, wobei der Unterschied in dem Grad der Expression von musterbildenden Genprodukten mindestens einfach ist.

**11.** Verfahren nach Anspruch 9, wobei der Unterschied in dem Grad, indem die musterbildenden Genprodukte exprimiert werden, mindestens 1,5-fach ist.

**12.** Verfahren nach Anspruch 1 oder 3, wobei mindestens zwei der mehreren musterbildenden Genexpressionsprodukte, die verwendet werden, um die erbliche oder sporadische Beschaffenheit eines Kolonkarzinoms zu bestimmen, die zwei nachfolgend aufgelisteten Gene sind

| Genname | Ref.-seq. | Gensymbol | SEQ ID Nr: |
|---|---|---|---|
| Piwi-like 1 | NM_004764.2 | PIWIL1 | 106 |
| Mut L Homolog 1 | NM_00249.2 | MLH1 | 107 |

**13.** Verfahren nach einem der Ansprüche 1 oder 3, wobei der Mikrosatellitenstatus in einem Individuum mit stenosierendem Kolonkarzinoms Mikrosatelliten-instabil ist.

**14.** Verfahren nach einem der vorstehenden Ansprüche, wobei das Kolonkarzinom einem Duke's B oder Duke's C Stadium entspricht.

**15.** Verfahren nach einem der vorstehenden Ansprüche, wobei das Kolonkarzinom ein Adenokarzinom, ein Karzinom, ein Teratom, ein Sarkom und/oder ein Lymphom ist.

**16.** Verfahren nach einem der vorstehenden Ansprüche, wobei die Probe eine Biopsie des Gewebes ist.

**17.** Verfahren nach einem der vorstehenden Ansprüche, wobei der Expressionsgrad durch Bestimmen von mRNA der Probe bestimmt wird.

**18.** Verfahren nach einem der vorstehenden Ansprüche, wobei der Expressionsgrad durch Bestimmen der Expressionsprodukte, wie beispielsweise Peptide und/oder Protein, in der Probe bestimmt wird.

**19.** Verfahren nach einem der vorstehenden Ansprüche, wobei der Mikrosatellitenstatus des Kolonkarzinoms in einem Individuum vor der Bestimmung der Anwesenheit und/oder Menge von Genexpressionsprodukten bestimmt wurde.

**20.** Verfahren nach einem der der vorstehenden Ansprüche, wobei die sporadische oder erbliche Beschaffenheit eines Kolonkarzinoms vor der Bestimmung der Anwesenheit und/oder der Menge von Genexpressionsprodukten bestimmt wurde.

**21.** Verfahren zum Klassifizieren eines Kolonkarzinom in einem Individuum mit stenosierendem Kolonkarzinom, wobei die erbliche oder sporadische Beschaffenheit des Krebses durch ein Verfahren bestimmt wird, das die Schritte umfasst

i) Bestimmen in einer Probe des Individuums mit stenosierendem Kolonkarzinom, wobei die Probe mehrere Genexpressionsprodukte umfasst deren Anwesenheit und/oder Menge ein Muster bildet, das die erbliche oder die sporadische Beschaffenheit des Krebses anzeigt, der Anwesenheit und/oder der Menge der mehreren musterbildenden Genexpressionsprodukte bestimmt, wobei mindestens eines der Genexpressionsprodukte ausgewählt ist aus der Gruppe bestehend aus

| Genname | Ref.-seq. | Gensymbol | SEQ ID Nr: |
|---|---|---|---|
| Homeobox C6 | NM_004503 | HOXC6 | 105 |
| Piwi-like 1 | NM_004764.2 | PIWIL1 | 106 |
| Mut L Homolog 1 | NM_00249.2 | MLH1 | 107 |
| Collapsin Antwort-Mediator-Protein 1 | NM_001313.2 | CRMP1 | 108 |
| Homeobox B2 | NM_002145.2 | HOXB2 | 109 |
| Pyrrolin-5-carboxylat Synthetase (Glutamat gamma-Semialdehyd Synthetase) | NM_002860.2 | PYCS/ADH18 A1 | 110 |
| TGFB induzierbare frühe Wachstumsantwort | NM_005655.1 | TIEG | 111 |
| Checkpoint mit Forkhead und Ringfinger-Domänen?? | NM_018223.1 | CHFR | 112 |
| Hypothetisches Protein FLJ13842 | NM_024645.1 | FLJ13842 | 113 |
| Phosphoprotein reguliert durch Mitogenwege | NM_025195.1 | C8FW | 114 |

und
ii) Erhalten eines Hinweises aus dem Muster auf die erbliche oder sporadische Beschaffenheit des Krebses in dem Individuum.

**22.** Verfahren nach Anspruch 21, wobei der Mikrosatellitenstatus des Krebses gleichzeitig oder danach bestimmt wird.

## Revendications

**1.** Procédé de classification du cancer du côlon chez un individu ayant contracté le cancer du côlon, qui comprend :

i) la détermination, dans un échantillon de l'individu ayant contracté le cancer du côlon, du statut microsatellite de la tumeur, et
ii) dans un échantillon de l'individu ayant contracté le cancer du côlon, ledit échantillon comprenant une pluralité de produits d'expression génique dont la présence et/ou la quantité forment un motif qui indique la nature héréditaire ou sporadique dudit cancer,
iii) la détermination de la présence et/ou de la quantité de ladite pluralité de produits d'expression génique formant ledit motif, ledit ou lesdits produits d'expression génique étant choisis dans le groupe constitué par

| Nom du gène | Réf. de la séq. | Symbole du gène | SEQ ID NO : |
|---|---|---|---|
| Homeo box C6 | NM_004503 | HOXC6 | 105 |
| Piwi-like 1 | NM_004764.2 | PIWIL1 | 106 |
| Homologue 1 de Mut L | NM_00249.2 | MLH1 | 107 |
| Protéine médiatrice de la réponse à la collapsine 1 | NM_001313.2 | CRMP1 | 108 |
| Homeo box B2 | NM_002145.2 | HOXB2 | 109 |
| Pyrroline-5-carboxylate synthétase (glutamate gamma-semialdéhyde synthétase) | NM_002860.2 | PYCS/ADH18 A1 | 110 |
| réponse de croissance précoce inductible par le TGFB | NM_005655.1 | TIEG | 111 |
| Checkpoint with forkhead and ring finger domains ?? | NM_018223.1 | CHFR | 112 |
| Protéine hypothétique FLJ13842 | NM_024645.1 | FLJ 13842 | 113 |
| Phosphoprotéine régulée par les voies mitogéniques | NM_025195.1 | C8FW | 114 |

et
iv) l'obtention dudit motif d'un indicateur de la nature héréditaire ou sporadique dudit cancer chez l'individu,
v) la classification dudit cancer du côlon en se basant sur le statut microsatellite et sur ledit indicateur.

**2.** Procédé selon la revendication 1, dans lequel la détermination du statut microsatellite comprend les étapes consistant à

i) dans un échantillon de l'individu ayant contracté le cancer, ledit échantillon comprenant une pluralité de produits d'expression génique dont la présence et/ou la quantité forment un motif qui indique le statut microsatellite dudit cancer,
ii) déterminer la présence et/ou la quantité desdits produits d'expression génique formant ledit motif,
iii) obtenir une indication du statut microsatellite dudit cancer chez l'individu en se basant sur l'étape ii).

**3.** Procédé selon l'une quelconque des revendications précédentes, dans lequel une pluralité de produits d'expression génique sont analysés en utilisant un support solide, ayant des partenaires de liaison (partenaires d'hybridation) pour ladite pluralité de produits d'expression génique formant un motif.

**4.** Procédé selon l'une quelconque des revendications précédentes, dans lequel une pluralité de produits d'expression génique sont analysés à l'aide de partenaires de liaison (partenaires d'hybridation) pour ladite pluralité de produits d'expression génique formant un motif.

**5.** Procédé selon les revendications 1 ou 3, dans lequel au moins deux produits d'expression génique de ladite pluralité de produits d'expression génique formant un motif utilisé pour déterminer ledit statut microsatellite sont choisis individuellement dans un groupe de gènes indiquant le statut microsatellite.

**6.** Procédé selon les revendications 1 ou 3, dans lequel au moins deux produits d'expression génique de ladite pluralité de produits d'expression génique formant un motif utilisé pour déterminer ledit statut microsatellite sont choisis individuellement dans le groupe constitué par les gènes dont la liste est proposée ci-dessous

| Nom du gène | Réf. de la séq. | Symbole du gène | SEQ ID NO : |
|---|---|---|---|
| ligand 5 de la chimiokine (motif C-C) | NM_002985 | CCL5 | 1 |
| tryptophanyl-tRNA synthétase | NM_004184 | WARS | 2 |
| sous-unité 1 activatrice du protéasome (prosome, macropain) (PA28 alpha) | NM_006263 | PSME1 | 3 |
| antigène 2 des cellules stromales de la moelle osseuse | NM_004335 | BST2 | 4 |
| enzyme de conjugaison de l'ubiquitine E2L 6 | NM_004223 | UBE2L6 | 5 |
| protéine d'ancrage de la kinase A (PRKA) 1 | NM_003488 | AKAP1 | 6 |
| sous-unité 2 de l'activateur du protéasome (prosome, macropain) (PA28 bêta) | NM_002818 | PSME2 | 7 |
| molécule d'adhésion cellulaire apparentée à l'antigène carcinoembryonnaire 5 | NM_004363 | CEACAM5 | 8 |
| FERM, RhoGEF (ARHGEF) et protéine 1 du domaine de la pleckstrine (dérivé des chondrocytes) | NM_005766 | FARP1 | 9 |
| myosine X | NM_012334 | MYO10 | 10 |
| ribonucléoprotéine nucléaire hétérogène L | NM_001533 | HNRPL | 11 |
| récepteur du facteur de motilité autocrine | NM_001144 | AMFR | 12 |
| diméthylarginine diméthylaminohydrolase 2 | NM_013974 | DDAH2 | 13 |
| protéine 2 induite par le facteur onconécrosant alpha | NM_006291 | TNFAIP2 | 14 |
| homologue de mutL 1, cancer du côlon, sans polypose type 2 (E. coli) | NM_000249 | MLH1 | 15 |
| thymidylate synthétase | NM_00107 | TYMS | 16 |
| molécule d'adhésion intracellulaire 1 (CD54), récepteur des rhinovirus humains | NM_000201 | ICAM1 | 17 |
| facteur de transcription général IIA, 2, 12 kDa | NM_004492 | GTF2A2 | 18 |
| protéine kinase 2 en forme d'hélice enroulée associée à Rho | NM_004850 | ROCK2 | 19 |
| protéine de liaison à l'ATP associée à la différenciation cellulaire | NM_005783 | TXNDC9 | 20 |
| protéine adaptateur NCK 2 | NM_003581 | NCK2 | 21 |
| phytanoyl-CoA hydroxylase (maladie de Refsum) | NM_006214 | PHYH | 22 |
| famille des gènes associés à la métastase, membre 2 | NM_004739 | MTA2 | 23 |
| protéine 1 de liaison à l'amiloride (amine oxydase (contenant du cuivre)) | NM_001091 | ABP1 | 24 |
| biliverdin réductase A | NM_000712 | BLVRA | 25 |
| phospholipase C, bêta 4 | NM_000933 | PLCB4 | 26 |

(suite)

| Nom du gène | Réf. de la séq. | Symbole du gène | SEQ ID NO : |
|---|---|---|---|
| ligand 9 de la chimiokine (motif C-X-C motif) | NM_002416 | CXCL9 | 27 |
| protéine A liant les éléments riches en purine | NM_005859 | PURA | 28 |
| quinolinate phosphoribosyltransférase (nicotinate-nucléotide pyrophosphorylase (carboxylante)) | NM_014298 | QPRT | 29 |
| répondeur récepteur de l'acide rétinoïque3 (induit par le tazarotène) | NM_004585 | RARRES3 | 30 |
| ligand 4 des chimiokines (motif C-C) | NM_002984 | CCL4 | 31 |
| Forkhead box O3A | NM_001455 | FOX03A | 32 |
| protéine inductible par l'interféron alpha (clone IFI-6-16) | NM_002038 | G1P3 | 34 |
| | NM_022873 | | 123 |
| ligand 10 des chimiokines (motif C-X-C) | NM_001565 | CXCL10 | 35 |
| | NM_005950 | MT1G | 36 |
| métallothionéine 1G | NM_005950 | | |
| récepteur du facteur onconécrosant | NM_000043 | TNFRSF6 | 37 |
| Superfamille | NM_152877 | | 133 |
| membre 6 | NM_152876 | | 132 |
| | NM_152875 | | 134 |
| | NM_152872 | | 130 |
| | NM_152873 | | 33 |
| | NM_152871 | | 129 |
| | NM_152874 | | 131 |
| facteur de croissance des cellules endothéliales 1 (dérivé des plaquettes) | NM_001953 | ECGF1 | 38 |
| Homologue 2 déficitaire en cytochrome oxydase SCO (levure) | NM_005138 | SCO2 | 39 |
| Ligand 13 des chimiokines (motif C-X-C) (chimioattractant des lymphocytes B) | NM_006419 | CXCL13 | 40 |
| Granulysine | NM_006433 | GNLY | 41 |
| | | | |
| Antigène CD2 (p50), récepteur des globules rouges de mouton | NM_001767 | CD2 | 42 |
| Facteur d'épissage, riche en arginine/sérine 6 | NM_006275 | SFRS6 | 43 |
| Facteur de croissance dérivé de tératocarcinome 1 | NM_003212 | TDGF1 | 44 |
| Métallothionéine 1H | NM_005951 | MT1H | 45 |
| Cytochrome P450, famille 2, sous-famille B, polypeptide 6 | NM_000767 | CYP2B6 | 46 |
| Superfamille du facteur onconécrosant (ligand), membre 9 | NM_003811 | TNFSF9 | 47 |
| | NM_006047 | RBM12 | 48 |
| RNA binding motif protein 12 | NM_006047 | | |

(suite)

| Nom du gène | Réf. de la séq. | Symbole du gène | SEQ ID NO : |
|---|---|---|---|
| Protéine de choc thermique 1 105 kDa/110 kDa | NM_006644 | HSPH1 | 49 |
| | | | |
| Staufen, protéine de liaison à l'ART (Drosophile) | NM_004602 | STAU | 50 |
| | NM_017452 | | 125 |
| | NM_017453 | | 126 |
| complexe de l'antigène lymphocytaire 6, locus G6D | NM_021246 | LY6G6D | 51 |
| protéine de liaison au calcium P22 | NM_007236 | CHP | 52 |
| Homologue B du cycle 14 de division cellulaire CDC14 (S. cerevisiae) | NM_003671 | CDC14B | 53 |
| | NM_03333 | | 115 |
| épiplakine 1 | XM_372063 | EPPK1 | 54 |
| métallothionéine 1X | NM_005952 | MT1X | 55 |
| facteur de croissance transformant, récepteur bêta II (70/80 kDa) | NM_003242 | TGFBR2 | 56 |
| protéine de liaison 1 à la protéine C | NM_012408 | PRKCBP1 | 57 |
| | NM_183047 | | 124 |
| élément 6 de la superfamille transmembranaire 4 | NM_003270 | TM4SF6 | 58 |
| domaine d'homologie de la pleckstrine contenant le membre 1 de la famille B (évectines) | NM_021200 | PLEKHB1 | 59 |
| apolipoprotéine L, 1 | NM_003661 | APOL1 | 60 |
| | NM_145343 | | 120 |
| indoleamine-pyrrole 2,3 dioxygénase | NM_002164 | INDO | 61 |
| forkhead box A2 | NM_021784 | FOXA2 | 62 |
| granzyme H (cathepsin G-like 2, protein h-CCPX) | NM_033423 | GZMH | 63 |
| baculoviral IAP repeat-containing 3 | NM_001165 | BIRC3 | 64 |
| protéine de type métallothionéine 1H d'Homo sapiens | | AF333388 (Hs 382039) | 135 |
| protéine KIAA0182 | NM_014615 | KIAA0182 | 117 |
| récepteur 56 couplé à la protéine G | NM_005682 | GPR56 | 65 |
| | NM_20152 | | 116 |
| métallothionéine 2A | NM_005953 | MT2A | 66 |
| F-box only protein 21 | NM_015002 | FBX021 | 67 |
| erythrocyte membrane protein band 4.1-like 1 | NM_012156 | EPB41L1 | 68 |
| | NM_012156 | | |
| protéine hypothétique MGC21416 | NM_173834 | MGC21416 | 69 |
| protéine O-fucosyltransférase 1 | NM_015352 | POFUT1 | 70 |
| | NM_015352 | | |
| métallothionéine 1E (fonctionnelle) | NM_175617 | MT1E | 71 |
| troponine T1, squelettique, lente | NM_003283 | TNNT1 | 72 |

(suite)

| Nom du gène | Réf. de la séq. | Symbole du gène | SEQ ID NO : |
|---|---|---|---|
| chimérine (chimaérine) 2 | NM_004067 | CHN2 | 73 |
| ribonucléoprotéine nucléaire hétérogène H1 (H) | NM_005520 | HNRPH1 | 74 |
| ATP synthase, H+ complexe mitochondrial F1 de transport, sous-unité alpha, isoforme 1, facteur d'initiation de la traduction eucaryote dans le muscle cardiaque 5A | NM_004046 | ATP5A1 | 75 |
| | NM_001970 | EIF5A | 76 |
| perforine 1 (protéine de formation des pores) | NM_005041 | PRF1 | 77 |
| protéine d'interaction avec OGT(O-Glc-NAc transférase) 106 KDa | NM_014965 | OIP106 | 78 |
| D E A D (A s p-Glu-Ala-Asp) box polypeptide 27 | NM_017895 | DDX27 | 79 |
| tri des protéines vacuolaires 35 (levure) | NM_018206 | VPS35 | 80 |
| transmembranaire à motif tripartite tripartite 44, ARN induit par les androgènes de la prostate | NM_017583 | TRIM44 | 81 |
| | NM_020182 | TMEPAI | 82 |
| | NM_199169 | | 127 |
| | NM_199170 | | 128 |
| dynéine, cytoplasmique, polypeptide 2A léger | NM_014183 | DNCL2A | 83 |
| | NM_177953 | | 122 |
| leucine aminopeptidase 3 | NM_015907 | LAP3 | 84 |
| cadre de lecture ouvert 35 du chromosome 20 | NM_018478 | C20orf35 | 85 |
| | NM_033542 | | 118 |
| famille 38 de transport de soluté, membre 1 | NM_030674 | SLC38A1 | 86 |
| protéine CGI-85 | NM_016028 | CGI-85 | 87 |
| facteur de transcription associé à la mort 1 | NM_022105 | DATF1 | 88 |
| | NM_080796 | | 121 |
| antigène 112 associé au carcinome hépatocellulaire | NM_018487 | HCA112 | 89 |
| sestrine 1 | NM_014454 | SESN1 | 90 |
| protéine hypothétique FLJ20315 | NM_017763 | FLJ20315 | 91 |
| protéine hypothétique FLJ20647 | NM_017918 | FLJ20647 | 92 |
| protéine membranaire exprimée dans un adénocarcinome pulmonaire de type épithélial | NM_024792 | CT120 | 93 |
| DEAD/H (Asp-Glu-Ala-Asp/His) box polypeptide | NM_014314 | RIG-I | 94 |
| kératine 23 (inductible par l'histone désacétylase) | NM_015515 | KRT23 | 95 |
| UDP-N-acétyl-alpha-D-galactosamine:polypeptide N-acétylgalactosaminyltransférase 6 (GalNAc-T6) | NM_007210 | GALNT6 | 96 |
| aryl hydrocarbon receptor nuclear translocator- like 2 | NM_020183 | ARNTL2 | 97 |
| facteur de complémentation d'apobec-1 | NM_014576, | ACF | 98 |
| | NM_138932 | | 119 |
| protéine hypothétique FLJ20232 | NM_019008 | FLJ20232 | 99 |

(suite)

| Nom du gène | Réf. de la séq. | Symbole du gène | SEQ ID NO : |
|---|---|---|---|
| apolipoprotéine L, 2 | NM_030882 | APOL2 | 100 |
| | NM_145343 | | 120 |
| protéine mitochondriale de transport de soluté | NM_016612 | MSCP | 101 |
| protéine hypothétique FLJ20618 | NM_017903 | FLJ20618 | 102 |
| translocation de SET (associé à la leucémie myéloïde) | NM_003011.1 | SET | 103 |
| ATPase, classe II, type 9a | Xm_030577.9 | ATP9a | 104 |

**7.** Procédé selon les revendications 1 ou 3, dans lequel au moins deux produits d'expression génique de ladite pluralité de produits d'expression génique formant un motif utilisé pour déterminer ledit statut microsatellite sont choisis individuellement dans le groupe constitué par les gènes dont la liste est proposée ci-dessous

| Nom du gène | Réf. de la séq. | Symbole du gène | SEQ ID NO : |
|---|---|---|---|
| ribonucléoprotéine nucléaire hétérogène L | NM_001533 | HNRPL | 11 |
| famille des gènes associés à la métastase, membre 2 | NM_004739 | MTA2 | 23 |
| ligand 10 des chimiokines (motif C-X-C) | NM_001565 | CXCL10 | 35 |
| facteur d'épissage, riche en arginine/sérine 6 | NM_006275 | SFRS6 | 43 |
| protéine 1 de liaison à la protéine kinase C | NM_012408 | PRKCBP1 | 57 |
| | NM_183047 | | 124 |
| antigène 112 associé au carcinome hépatocellulaire | NM_018487 | HCA112 | 89 |
| protéine hypothétique FLJ20618 | NM_017903 | FLJ20618 | 102 |
| translocation de SET (associé à la leucémie myéloïde) | NM_003011.1 | SET | 103 |
| ATPase, classe II, type 9a | Xm 030577.9 | ATP9a | 104 |

**8.** Procédé selon les revendications 1 ou 3, dans lequel

i) au moins un produit d'expression génique de ladite pluralité de produits d'expression génique formant un motif utilisé pour déterminer ledit statut microsatellite est choisi dans le groupe de gènes constitué par

| Nom du gène | Réf. de la séq. | Symbole du gène | SEQ ID NO : |
|---|---|---|---|
| ribonucléoprotéine nucléaire hétérogène L | NM_001533 | HNRPL | 11 |
| famille des gènes associés à la métastase, membre 2 | NM_004739 | MTA2 | 23 |
| ligand 10 des chimiokines (motif C-X-C) | NM_001565 | CXCL10 | 35 |
| facteur d'épissage, riche en arginine/sérine 6 | NM_006275 | SFRS6 | 43 |

et

ii) au moins un produit d'expression génique de ladite pluralité de produits d'expression génique formant un motif utilisé pour déterminer ledit statut microsatellite est choisi dans le groupe de gènes constitué par

| Nom du gène | Réf. de la séq. | Symbole du gène | SEQ ID NO : |
|---|---|---|---|
| protéine 1 de liaison à la protéine kinase C | NM_012408 | PRKCBP1 | 57 |

(suite)

| Nom du gène | Réf. de la séq. | Symbole du gène | SEQ ID NO : |
|---|---|---|---|
| | NM_183047 | | 124 |
| antigène 112 associé au carcinome hépatocellulaire | NM_018487 | HCA112 | 89 |
| protéine hypothétique FLJ20618 | NM_017903 | FLJ20618 | 102 |
| translocation de SET (associé à la leucémie myéloïde) | NM_003011.1 | SET | 103 |
| ATPase, classe II, type 9a | Xm 030577.9 | ATP9a | 104 |

**9.** Procédé selon les revendications 1 ou 3, dans lequel

i) au moins un produit d'expression génique de ladite pluralité de produits d'expression génique formant un motif utilisé pour déterminer ledit statut microsatellite est choisi dans le groupe de gènes qui sont régulés à la baisse dans les cancers du côlon MSS comparés aux cancers du côlon MSI, constitué par

| Nom du gène | Réf. de la séq. | Symbole du gène | SEQ ID NO : |
|---|---|---|---|
| ribonucléoprotéine nucléaire hétérogène L | NM_001533 | HNRPL | 11 |
| famille des gènes associés à la métastase, membre 2 | NM_004739 | MTA2 | 23 |
| ligand 10 des chimiokines (motif C-X-C) | NM_001565 | CXCL10 | 35 |
| facteur d'épissage, riche en arginine/sérine 6 | NM_006275 | SFRS6 | 43 |

et

ii) au moins un produit d'expression génique de ladite pluralité de produits d'expression génique formant un motif utilisé pour déterminer ledit statut microsatellite est choisi dans le groupe de gènes qui sont régulés à la hausse dans les cancers du côlon MSS comparés aux cancers du côlon MSI, constitué par

| Nom du gène | Réf. de la séq. | Symbole du gène | SEQ ID NO : |
|---|---|---|---|
| protéine 1 de liaison à la protéine kinase C | NM_012408 | PRKCBP1 | 57 |
| | NM_183047 | | 124 |
| antigène 112 associé au carcinome hépatocellulaire | NM_018487 | HCA112 | 89 |
| protéine hypothétique FLJ20618 | NM_017903 | FLJ20618 | 102 |
| translocation de SET (associé à la leucémie myéloïde) | NM_003011.1 | SET | 103 |
| ATPase, classe II, type 9a | Xm 030577.9 | ATP9a | 104 |

**10.** Procédé selon la revendication 9, dans lequel la différence du niveau des produits d'expression génique formant un motif est d'au moins une fois.

**11.** Procédé selon la revendication 9, dans lequel la différence du niveau des produits d'expression génique formant un motif est d'au moins 1,5 fois.

**12.** Procédé selon la revendication 1 ou 3, dans lequel au moins deux produits d'expression génique de ladite pluralité de produits d'expression génique formant un motif utilisé pour déterminer ladite nature héréditaire ou sporadique du cancer du côlon sont les deux gènes proposés dans la liste ci-dessous

| Nom du gène | Réf. de la séq. | Symbole du gène | SEQ ID NO : |
|---|---|---|---|
| Piwi-like 1 | NM_004764.2 | PIWIL1 | 106 |

(suite)

| Nom du gène | Réf. de la séq. | Symbole du gène | SEQ ID NO : |
|---|---|---|---|
| Homologue 1 de Mut L | NM_00249.2 | MLH1 | 107 |

**13.** Procédé selon les revendications 1 ou 3, dans lequel le statut microsatellite chez un individu ayant contracté le cancer du côlon est microsatellite instable.

**14.** Procédé selon l'une quelconque des revendications précédentes, dans lequel ledit cancer du côlon est au stade B de Duke ou au stade C de Duke.

**15.** Procédé selon l'une quelconque des revendications précédentes, dans lequel ledit cancer du côlon est un adénocarcinome, un carcinome, un tératome, un sarcome, et/ou un lymphome.

**16.** Procédé selon l'une quelconque des revendications précédentes, dans lequel l'échantillon est une biopsie du tissu.

**17.** Procédé selon l'une quelconque des revendications précédentes, dans lequel le niveau d'expression est déterminé par détermination de l'ARNm de l'échantillon.

**18.** Procédé selon l'une quelconque des revendications précédentes, dans lequel le niveau d'expression est déterminé par détermination des produits d'expression, tels que les peptides et/ou les protéines dans l'échantillon.

**19.** Procédé selon l'une quelconque des revendications précédentes, dans lequel le statut microsatellite du cancer du côlon chez un individu a été déterminé avant la détermination de la présence et/ou de la quantité des produits d'expression génique.

**20.** Procédé selon l'une quelconque des revendications précédentes, dans lequel la nature sporadique ou héréditaire d'un cancer du côlon a été déterminée avant la détermination de la présence et/ou de la quantité de produits d'expression génique.

**21.** Procédé de classification du cancer du côlon chez un individu ayant contracté le cancer du côlon, la nature héréditaire ou sporadique dudit cancer étant déterminée par un procédé qui comprend les étapes consistant à

i) dans un échantillon de l'individu ayant contracté le cancer du côlon, ledit échantillon comprenant une pluralité de produits d'expression génique dont la présence et/ou la quantité forment un motif indiquant la nature héréditaire ou sporadique dudit cancer, déterminer la présence et/ou la quantité desdits produits d'expression génique formant ledit motif, au moins un desdits produits d'expression génique étant choisi dans le groupe constitué par :

| Nom du gène | Réf. de la séq. | Symbole du gène | SEQ ID NO : |
|---|---|---|---|
| Homeo box C6 | NM_004503 | HOXC6 | 105 |
| Piwi-like 1 | NM_004764.2 | PIWIL1 | 106 |
| Homologue 1 de Mut L | NM_00249.2 | MLH1 | 107 |
| Protéine médiatrice de la réponse à la collapsine 1 | NM_001313.2 | CRMP1 | 108 |
| Homeo box B2 | NM_002145.2 | HOXB2 | 109 |
| Pyrroline-5-carboxylate synthétase (glutamate gamma-semialdéhyde synthétase) | NM_002860.2 | PYCS/ADH18 A1 | 110 |
| réponse de croissance précoce inductible par le TGFB | NM_005655.1 | TIEG | 111 |
| Checkpoint with forkhead and ring finger domains ?? | NM_018223.1 | CHFR | 112 |
| Protéine hypothétique FLJ13842 | NM_024645.1 | FLJ 13842 | 113 |
| Phosphoprotéine régulée par les voies mitogéniques | NM_025195.1 | C8FW | 114 |

et
ii) obtenir dudit motif un indicateur de la nature héréditaire ou sporadique dudit cancer chez l'individu.

**22.** Procédé selon la revendication 21, dans lequel le statut microsatellite dudit cancer est déterminé simultanément ou séquentiellement avec cela.

# Fig. 1

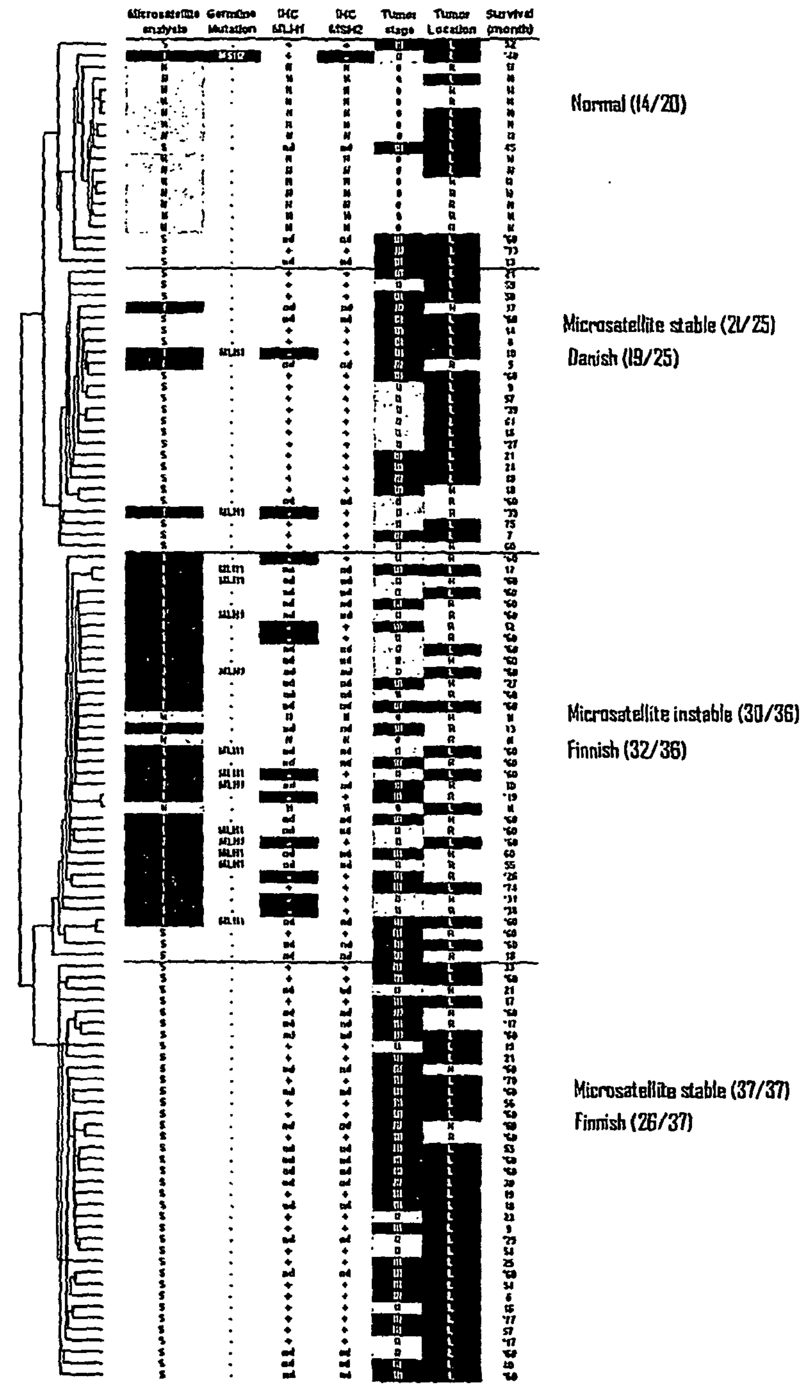
Microsatellite analysis
Germline Mutation
IHC MLH1
IHC MSH2
Tumor stage
Tumor Location
Survival (month)
Normal (14/20)
Microsatellite stable (21/25)
Danish (19/25)
Microsatellite instable (30/36)
Finnish (32/36)
Microsatellite stable (37/37)
Finnish (26/37)

Fig. 2

A

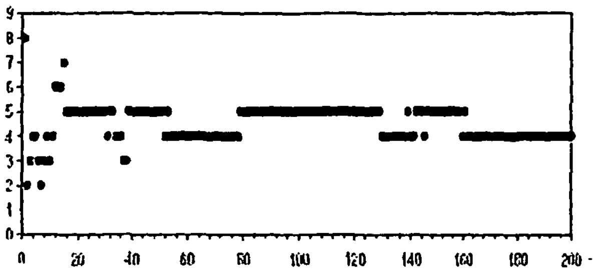


B

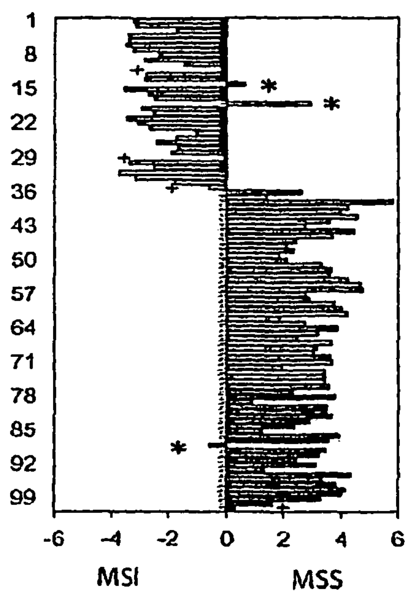


C

| | Trainings set<br>Errors in crossvalidation | Test set<br>Test errors |
|---|---|---|
| MSI | 6.48% (n=25, range 0-4) | 6.0% (n=9, range 0-3) |
| MSS | 1.67% (n=30, range 0-3) | 1.62% (n=37, range 0-4) |
| All | 3.85% (n=55, range 0-5) | 2.48% (n=46, range 0-4) |

Fig. 3

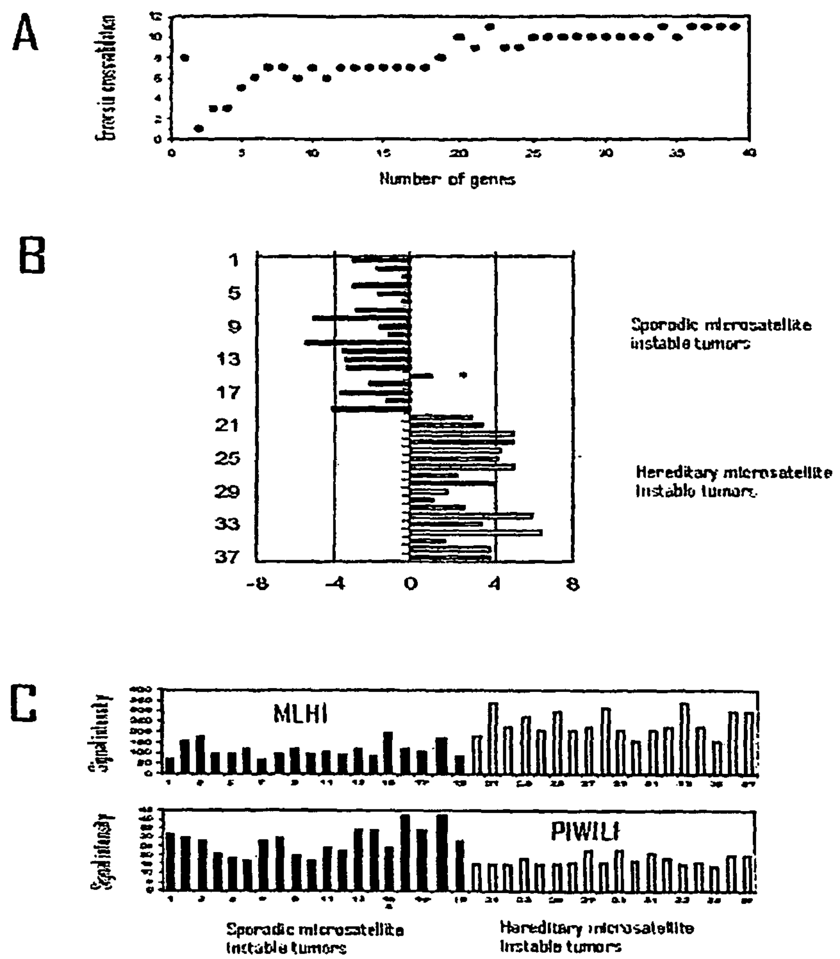
A
Number of genes
B
Sporadic microsatellite instable tumors
Hereditary microsatellite instable tumors
C
MLHI
PIWIL1
Signal intensity
Sporadic microsatellite instable tumors
Hereditary microsatellite instable tumors

Fig. 4

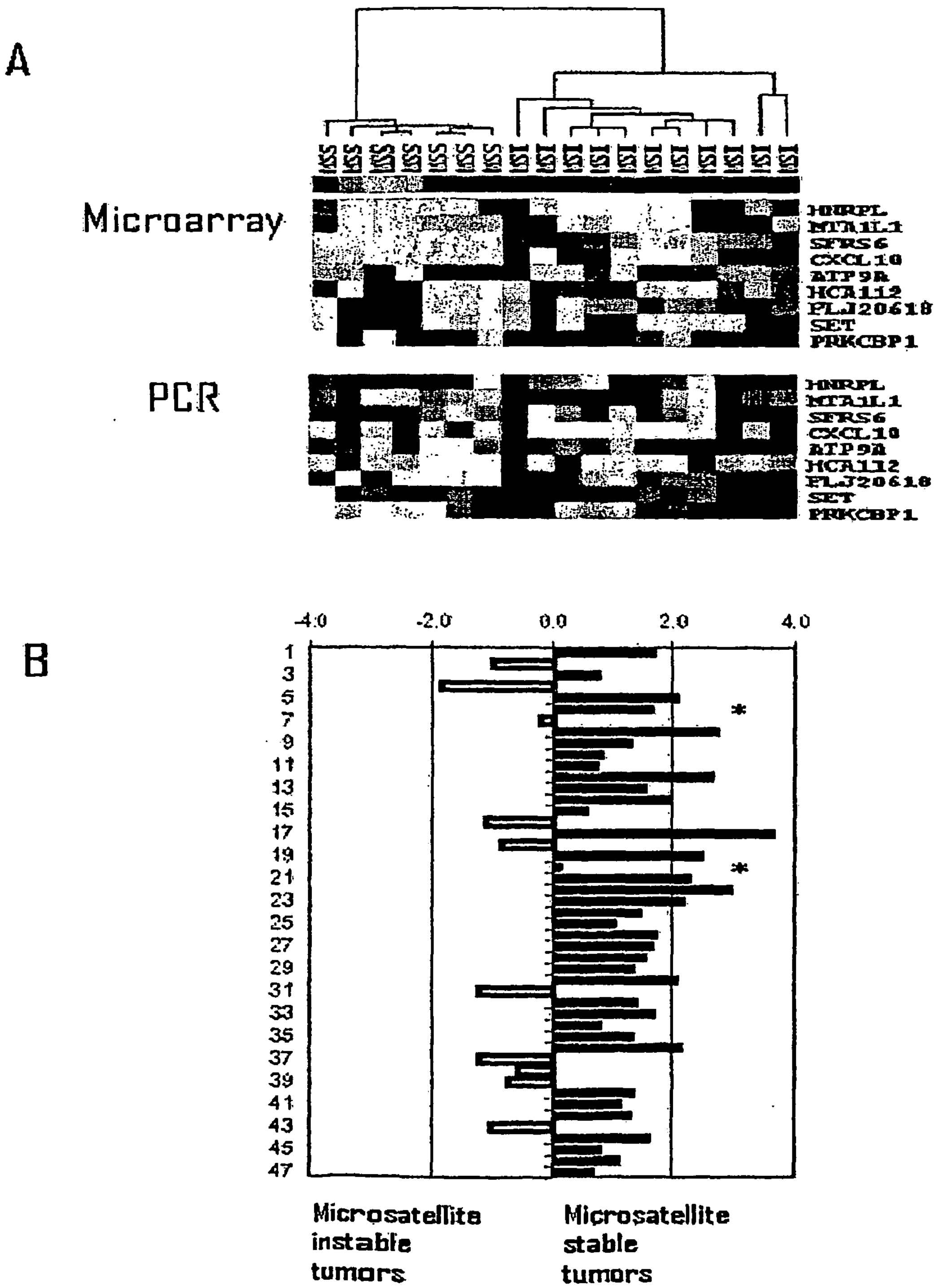
A
Microarray
PCR
HNRPL
MTA1L1
SFRS6
CXCL10
ATP9A
HCA112
FLJ20618
SET
PRKCBP1
B
-4.0
-2.0
0.0
2.0
4.0
Microsatellite instable tumors
Microsatellite stable tumors

Fig. 5

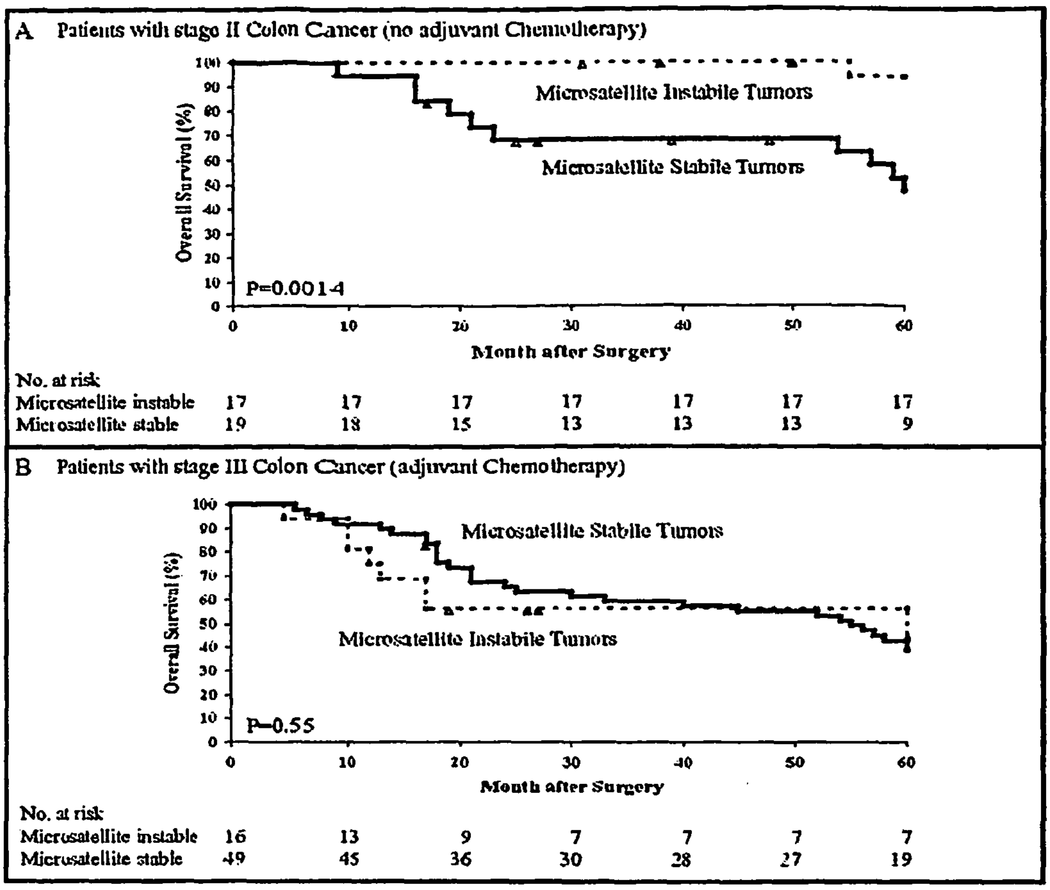
A Patients with stage II Colon Cancer (no adjuvant Chemotherapy)
Overall Survival (%)
Microsatellite Instabile Tumors
Microsatellite Stabile Tumors
P=0.0014
Month after Surgery
No. at risk
Microsatellite instable 17 17 17 17 17 17 17
Microsatellite stable 19 18 15 13 13 13 9
B Patients with stage III Colon Cancer (adjuvant Chemotherapy)
Overall Survival (%)
Microsatellite Stabile Tumors
Microsatellite Instabile Tumors
P=0.55
Month after Surgery
No. at risk
Microsatellite instable 16 13 9 7 7 7 7
Microsatellite stable 49 45 36 30 28 27 19

Fig. 6

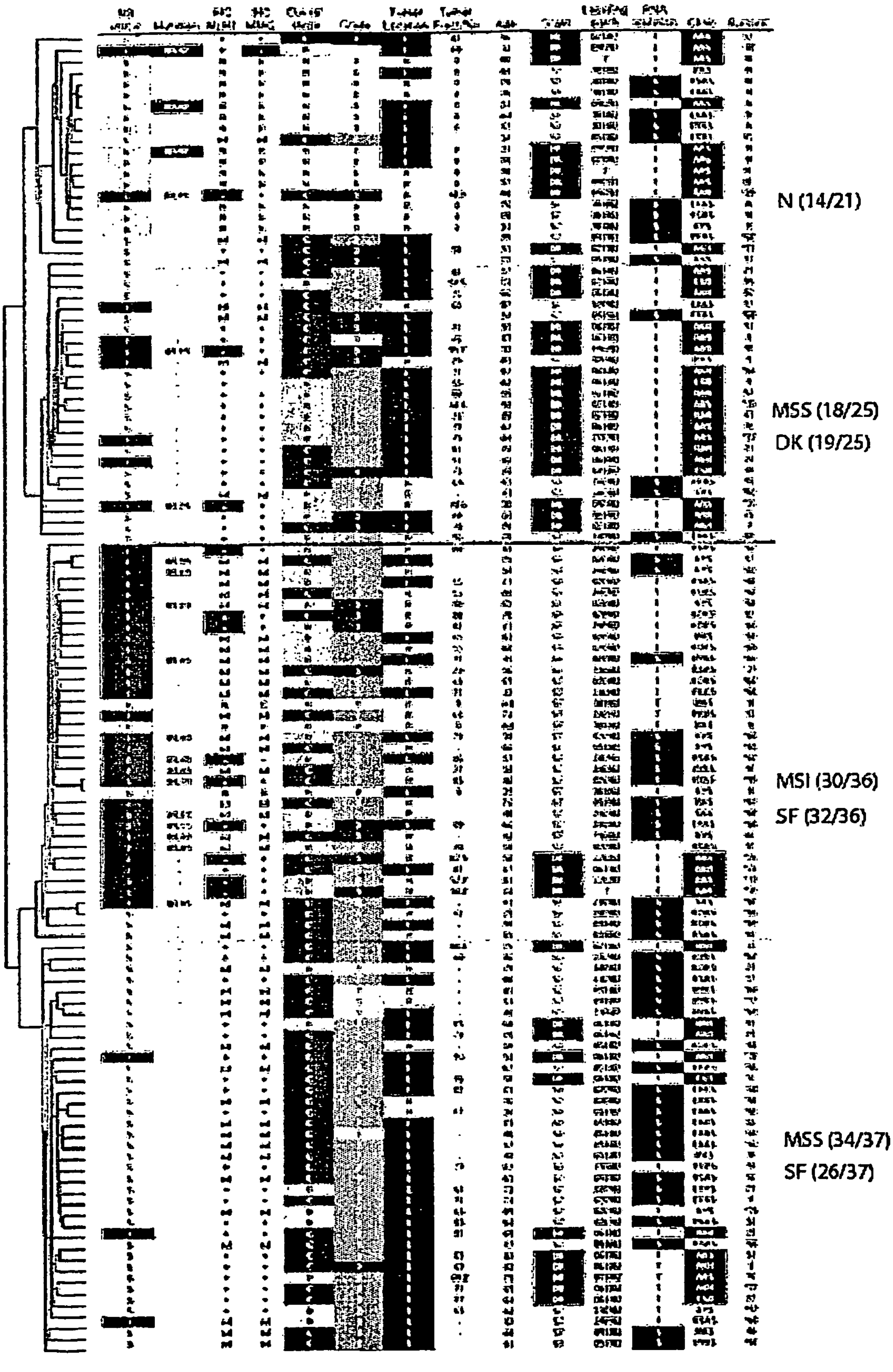

Fig. 7

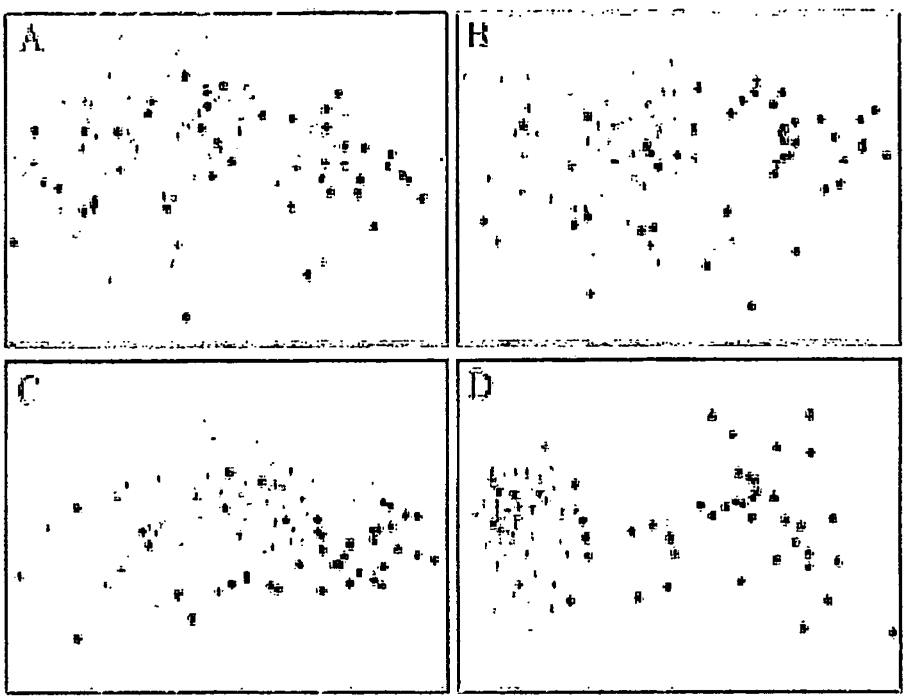

Fig. 8

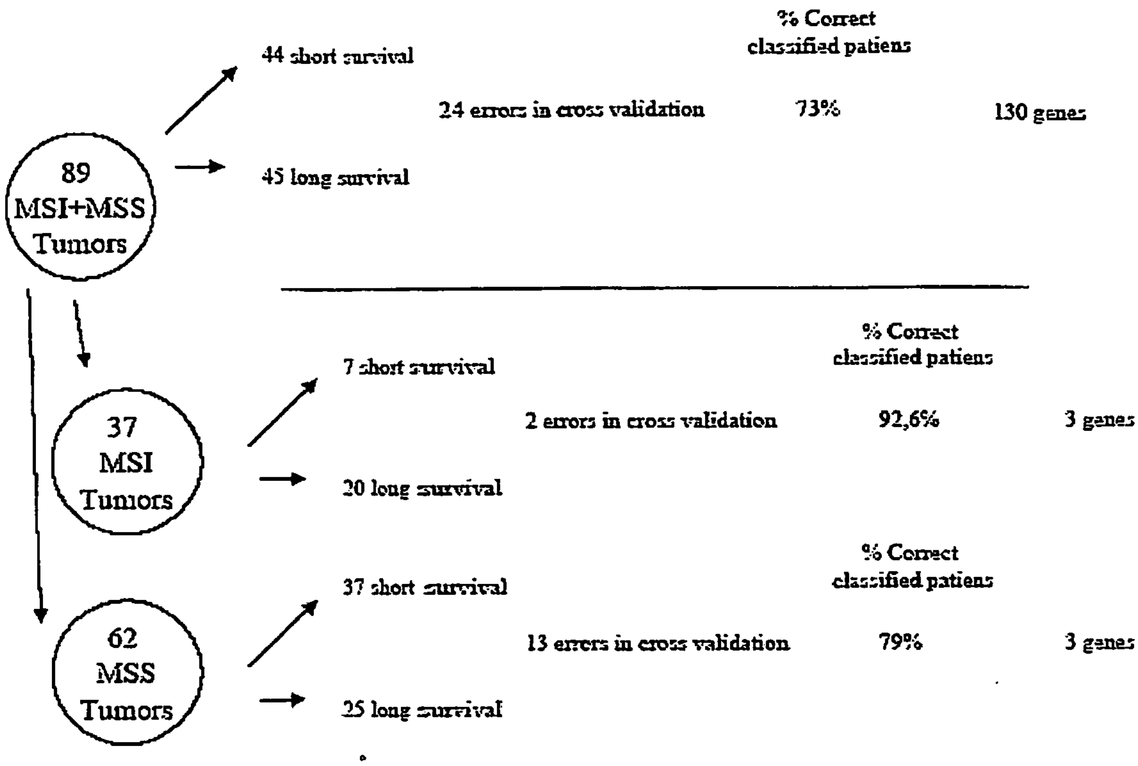
89 MSI+MSS Tumors
44 short survival
45 long survival
24 errors in cross validation
% Correct classified patiens
73%
130 genes
37 MSI Tumors
7 short survival
20 long survival
2 errors in cross validation
% Correct classified patiens
92,6%
3 genes
62 MSS Tumors
37 short survival
25 long survival
13 errors in cross validation
% Correct classified patiens
79%
3 genes

Fig. 9

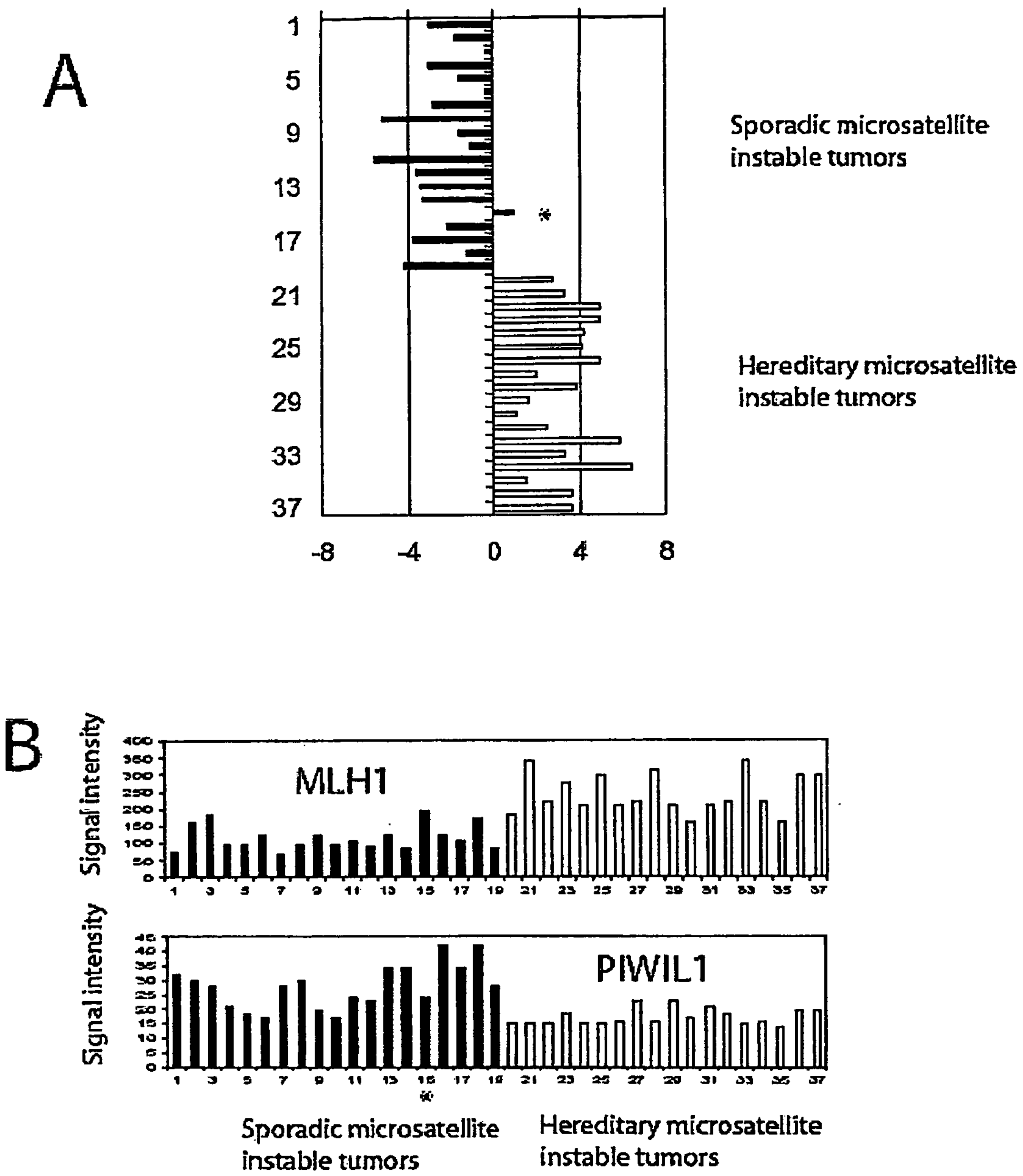
A
1
5
9
13
17
21
25
29
33
37
-8
-4
0
4
8
*
Sporadic microsatellite instable tumors
Hereditary microsatellite instable tumors
B
Signal intensity
MLH1
PIWIL1
Sporadic microsatellite instable tumors
Hereditary microsatellite instable tumors

Fig. 10

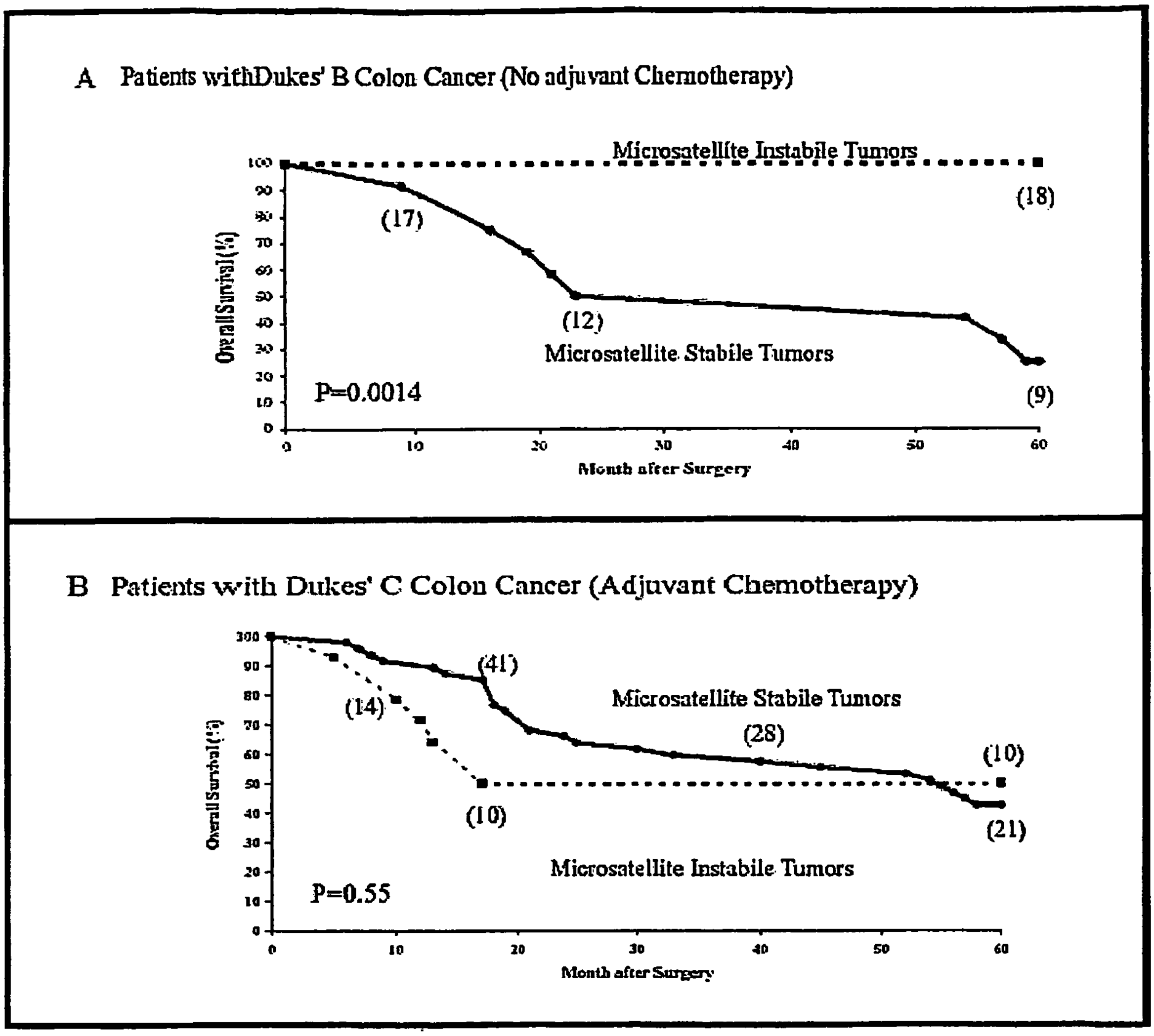
A Patients withDukes' B Colon Cancer (No adjuvant Chemotherapy)
Microsatellite Instabile Tumors
Overall Survival (%)
(18)
(17)
(12)
Microsatellite Stabile Tumors
P=0.0014
(9)
Month after Surgery
B Patients with Dukes' C Colon Cancer (Adjuvant Chemotherapy)
(41)
(14)
Microsatellite Stabile Tumors
(28)
(10)
(10)
(21)
Overall Survival (%)
Microsatellite Instabile Tumors
P=0.55
Month after Surgery

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5225336 A
- US 5204243 A
- US 5155020 A
- US 4769330 A
- US 525234 A


### Non-patent literature cited in the description


- **Boland CR ; Thibodeau SN ; Hamilton SR et al.** *Cancer Res,* 1998, vol. 58 (22), 5248-57
- **Mori Y. et al.** *Cancer Res.,* 01 August 2003, vol. 63 (15), 4577-4582
- **Tester DJ et al.** *Cancer Res,* 1998, vol. 58 (15), 3455-60
- **Kane MF ; Loda M ; Gaida GM et al.** *Cancer Res,* 1997, vol. 57 (5), 808-11
- **Herman JG ; Umar A ; Polyak K et al.** *Proc Natl Acad Sci U S A,* 1998, vol. 95 (12), 6870-5
- **Kuismanen SA ; Holmberg MT ; Salovaara R ; de la Chapelle A ; Peltomaki P.** *Am J Pathol,* 2000, vol. 156 (5), 1773-9
- **Sambrook et al.** Molecular Cloning: A laboratory Manual. Cold Spring Harbour Laboratory, 1989, vol. 1-3
- Current Protocols in Molecular Biology. Greene Publishing and Wiley-Interscience, 1987
- **Carethers JM ; Hawn MT ; Chauhan DP et al.** *J Clin Invest,* 1996, vol. 98 (1), 199-206
- **Carethers JM ; Chauhan DP ; Fink D et al.** *Gastroenterology,* 1999, vol. 117 (1), 123-31
- **Koi M ; Umar A ; Chauhan DP et al.** *Cancer Res,* 1994, vol. 54 (16), 4308-12
- **Hawn MT ; Umar A ; Carethers JM et al.** *Cancer Res,* 1995, vol. 55 (17), 3721-5
- **Hsiang YH ; Lihou MG ; Liu LF.** *Cancer Res,* 1989, vol. 49 (18), 5077-82
- **Jacob S ; Aguado M ; Fallik D ; Praz F.** *Cancer Res,* 2001, vol. 61 (17), 6555-62
- Therapeutic Peptides and Protein Formulation. Processing and Delivery Systems. Technomic Publishing AG, 1995
- **Dubensky et al.** *Proc. Natl. Acad. Sci. USA,* 1984, vol. 81, 7529-7533
- **Kaneda et al.** *Science,* 1989, vol. 243, 375-378
- **Hiebert et al.** *Proc. Natl. Acad. Sci. USA,* 1989, vol. 86, 3594-3598
- **Hatzoglu et al.** *J. Biol. Chem.,* 1990, vol. 265, 17285-17293
- **Ferry et al.** *Proc. Natl. Acad. Sci. USA,* 1991, vol. 88, 8377-8381
- **Schreier H. et al.** *J. Mol. Recognit.,* 1995, vol. 8, 59-62
- **Schreier H et al.** *J. Biol. Chem.,* 1994, vol. 269, 9090-9098
- **Schreier, H.** *Pharm. Acta Helv.,* 1994, vol. 68, 145-159
- **Chander, R et al.** *Life Sci.,* 1992, vol. 50, 481-489
- **Stecenko, A. A. et al.** *Pharm. Pharmacol. Lett.,* 1992, vol. 1, 127-129
- **Sizemore, D. R. et al.** *Science,* 1995, vol. 270, 299-302
- **Suraweera N ; Duval A ; Reperant M et al.** *Gastroenterology,* 2002, vol. 123 (6), 1804-11
- **Dyrskjot L ; Thykjaer T ; Kruhoffer M et al.** *Nat Genet,* 2003, vol. 33 (1), 90-6
- **Irizarry RA ; Bolstad BM ; Collin F ; Cope LM ; Hobbs B ; Speed TP.** *Nucleic Acids Res,* 2003, vol. 31 (4), e15
- **Bolstad BM ; Irizarry RA ; Astrand M ; Speed TP.** *Bioinformatics,* 2003, vol. 19 (2), 185-93
- **Birkenkamp-Demtroder K ; Christensen LL ; Olesen SH et al.** *Cancer Res,* 2002, vol. 62 (15), 4352-63
- **Andersen CL ; Ledet JL ; Omtoft TF.** *Cancer Res,* 2004
- **Loeb LA.** *Cancer Res,* 1991, vol. 51 (12), 3075-9
- **Yuan Z ; Sotsky Kent T ; Weber TK.** *Oncogene,* 2003, vol. 22 (40), 6304-10
- **Sasaki T ; Shiohama A ; Minoshima S ; Shimizu N.** *Genomics,* 2003, vol. 82 (3), 323-30
- **Ribic CM ; Sargent DJ ; Moore MJ et al.** *N Engl J Med,* 2003, vol. 349 (3), 247-57
- **Boland et al.** *CR,* 1998, vol. 58, 5248
- *New England J Med,* 02 August 2003
- **Alon U ; Levine AJ.** *PNAS,* 1999
- **Kitahara O ; Tsunoda T.** *Cancer Res,* 2001
- **Notterman DA ; Levine AJ.** *Cancer Res,* 2001

- **Yanagava R.** *Nakamura,* 2001
- **Zou TT ; Meltzer SJ.** *Oncogene,* 2002
- **Demtroeder.** *CR,* 2002
- **Lin YM ; Nakamura Y.** *Oncogene,* 2002
- **Williams NS ; Becerra C.** *Clin Cancer Res,* 2003
- **Frederiksen CM ; Omtoft TF.** *J Cancer Res Clin Oncol,* 2003
- **Takemasa I ; Matsubara K.** *Biochem Biophys Res Commun,* 2001
- **Yanagawa R.** *Nakamura, Neoplasia,* 2001
- **Agrawal D ; Yeatman T.** *J Natl Cancer Inst,* 2002
- **Mori Y ; Meltzer SJ.** *Cancer Res,* 2003
- **Eisen et al.** *PNAS,* 1998, vol. 95, 14863-14868
- **Birkenkamp-Demtroder.** *Cancer Res.,* 2002, vol. 62, 4352-4363
- **Agrawal D ; Chen T ; Irby R ; Quackenbush J ; Chambers AF ; Szabo M ; Cantor A ; Coppola D ; Yeatman TJ.** Osteopontin identified as lead marker of colon cancer progression, using pooled sample expression profiling. *J Natl Cancer Inst.,* 03 April 2002, vol. 94 (7), 513-21
- **Birkenkamp-Demtroder K ; Christensen LL ; Olesen SH ; Frederiksen CM ; Laiho P ; Aaltonen LA ; Laurberg S ; Sorensen FB ; Hagemann R ; ORntoft TF.** Gene expression in colorectal cancer. *Cancer Res.,* 01 August 2002, vol. 62 (15), 4352-63
- **Boland CR ; Thibodeau SN ; Hamilton SR ; Sidransky D ; Eshleman JR ; Burt RW ; Meltzer SJ ; Rodriguez-Bigas MA ; Fodde R ; Ranzani GN.** A National Cancer Institute Workshop on Microsatellite Instability for cancer detection and familial predisposition: development of international criteria for the determination of microsatellite instability in colorectal cancer. *Cancer Res.,* 15 November 1998, vol. 58 (22), 5248-57
- **Chapusot C ; Martin L ; Bouvier AM ; Bonithon-Kopp C ; Ecarnot-Laubriet A ; Rageot D ; Ponnelle T ; Laurent Puig P ; Faivre J ; Piard F.** Microsatellite instability and intratumoural heterogeneity in 100 right-sided sporadic colon carcinomas. *Br J Cancer,* 12 August 2002, vol. 87 (4), 400-4
- **Dyrskjot L ; Thykjaer T ; Kruhoffer M ; Jensen JL ; Marcussen N ; Hamilton-Dutoit S ; Wolf H ; Orntoft TF.** Identifying distinct classes of bladder carcinoma using microarrays. *Nat Genet.,* January 2003, vol. 33 (1), 90-6
- **Frederiksen CM ; Knudsen S ; Laurberg S ; Orntoft TF.** Classification of Dukes' B and C colorectal cancers using expression arrays. *J Cancer Res Clin Oncol.,* May 2003, vol. 129 (5), 263-71
- **Huang J ; Qi R ; Quackenbush J ; Dauway E ; Lazaridis E ; Yeatman T.** Effects of ischemia on gene expression. *J Surg Res.,* August 2001, vol. 99 (2), 222-7
- **Irizarry RA ; Bolstad BM ; Collin F ; Cope LM ; Hobbs B ; Speed TP.** Summaries of Affymetrix GeneChip probe level data. *Nucleic Acids Res.,* 15 February 2003, vol. 31 (4), e15
- **Loukola A ; Eklin K ; Laiho P ; Salovaara R ; Kristo P ; Jarvinen H ; Mecklin JP ; Launonen V ; Aaltonen LA.** Microsatellite marker analysis in screening for hereditary nonpolyposis colorectal cancer (HNPCC). *Cancer Res.,* 01 June 2001, vol. 61 (11), 4545-9
- **Markowitz S ; Hines JD ; Lutterbaugh J ; Myeroff L ; Mackay W ; Gordon N ; Rustum Y ; Luna E ; Kleinerman J.** Mutant K-ras oncogenes in colon cancers Do not predict Patient's chemotherapy response or survival. *Clin Cancer Res.,* April 1995, vol. 1 (4), 441-5
- **Mori Y ; Selaru FM ; Sato F ; Yin J ; Simms LA ; Xu Y ; Olaru A ; Deacu E ; Wang S ; Taylor JM.** The impact of microsatellite instability on the molecular phenotype of colorectal tumors. *Cancer Res.,* 01 August 2003, vol. 63 (15), 4577-82
- **Ribic CM ; Sargent DJ ; Moore MJ ; Thibodeau SN ; French AJ ; Goldberg RM ; Hamilton SR ; Laurent-Puig P ; Gryfe R ; Shepherd LE.** Tumor microsatellite-instability status as a predictor of benefit from fluorouracil-based adjuvant chemotherapy for colon cancer. *N Engl J Med.,* 17 July 2003, vol. 349 (3), 247-57